# EUROPEAN PATENT SPECIFICATION

(11) **EP 1 082 463 B1**
(45) Date of publication and mention of the grant of the patent: **03.01.2007**
(21) Application number: 99923223.4
(22) Date of filing: 14.05.1999
(51) Int. Cl.: C12Q 1/68, C07K 14/47, C12N 15/10

(54) **MECHANICAL STRESS INDUCED GENES, EXPRESSION PRODUCTS THEREFROM, AND USES THEREOF**
DURCH MECHANISCHEN STRESS INDUZIERTE GENE, DEREN EXPRESSIONSPRODUKTE UND DEREN VERWENDUNG.
GENES INDUITS PAR LA CONTRAINTE MECANIQUE, PRODUITS D'EXPRESSION DE CES GENES ET UTILISATIONS ASSOCIEES

(30) Priority: 15.05.1998 US 85673 P
(43) Date of publication of application: 14.03.2001
(73) Proprietor: Quark Biotech, Inc., Pleasanton, CA 94566 (US)
(72) Inventor: EINAT, Paz, Ness-Ziona (IL); MOR, Orna, Ganei Ilan, Kiryat Ono (IL); SKALITER, Rami, Ness-Ziona (IL); FEINSTEIN, Elena, Rehovot (IL); FAERMAN, Alexander, Bnei Aiish (IL)
(74) Representative: Maschio, Antonio
(86) International application number: PCT/US1999/011066
(87) International publication number: WO 1999/060164

(56) References cited:
- US-A- 5 445 941
- US-A- 5 599 708
- US-A- 5 763 416
- US-A- 5 861 249
- US-A- 5 882 925
- MASON ET AL.: "Mechanically regulated expression of a neural glutamate transporter in bone: a role for excitatory amino acids as osteotrophic agents" BLOOD, vol. 20, no. 3, 1997, pages 199-205, XP002220095
- ASAHINA ET AL.: "Human osteogenic protein- 1 induces both chondroblastic and osteoblastic differentiation of osteoprogenitor cells derived from newborn rat calvaria." J. OF CELL BIOLOGY, vol. 123, no. 4, 1993, pages 921-933, XP002220096
- FANCIULLI ET AL.: "Cloning of a novel human RNA polymerase II subunit down regulated by doxorubicin: new potential mechanisms of drug related toxicity" FEBS LETTERS, 1996, pages 48-52, XP002220097
- SCHENA ET AL.: "Parallel human genome analysis: Microarray-based expression monitoring of 1000 genes" P.N.A.S., vol. 93, 1996, pages 10614-10619, XP002220098
- DAHL ET AL.: "Effects of Normal Mouse Serum on the lL-3-Induced Proliferation of Bone Marrow Cells" BLOOD, vol. 73, no. 3, 1989, pages 700-705, XP002220099
- NAGASE ET AL.: "Prediction of the Coding Sequences of Unidentified Human Genes. V. The Coding Sequences of 40 New Genes (KIAA0161-KIAA0200) Deduced by analysis of cDNA Clones from Human Cell Line KG-1" DNA RESEARCH, vol. 3, 1996, pages 17-24, XP002037754
- SEKI ET AL.: "Characterization of cDNA Clones in Size-Fractionated eDNA Libraries from Human Brain" DNA RESEARCH, vol. 4, 1997, pages 345-349, XP000870411
- LABEIT ET AL.: "Titins: giant proteins in charge of the muscle ultrastructure and elasticity" SCIENCE, vol. 270, no. 5234, 1995, pages 293-296, XP002220102

## Description

### RELATED APPLICATION

This application is based upon and claims priority from US 6603046.

Reference is also made to US 6171652.

### FIELD OF THE INVENTION

This invention relates to osteoporosis. Moreover, the invention relates to mechanical stress induced genes, probes therefor, tests to identify such genes, expression products of such genes, uses for such genes and expression products, e.g., in diagnosis (for instance risk determination), treatment, prevention, or control, of osteoporosis or factors or processes which lead to osteoporosis; and, to diagnostic, treatment, prevention, or control methods or processes, as well as compositions therefor and methods or processes for making and using such compositions.

The present application also relates to a method for identifying genes that are regulated at the RNA level. More specifically, the present application relates to the rapid isolation of differentially expressed or developmentally regulated gene sequences through analysis of mRNAs obtained from specific cellular compartments. By comparing changes in the relative abundance of the mRNAs found in these compartments occurring as a result of application of a cue or stimulus to the tested biological sample, genes that are differentially expressed can be characterized.

The present invention especially relates to such methods with respect to bone cells and/or the stimulus being mechanical stress.

These and other areas to which the invention relates will be apparent from the following text.

### BACKGROUND OF THE INVENTION

Bone is composed of a collagen-rich organic matrix impregnated with mineral, largely calcium and phosphate. Two major forms of bone exist, compact cortical bone forms the external envelopes of the skeleton and trabecular or medullary bone forms plates that traverse the internal cavities of the skeleton. The responses of these two forms to metabolic influences and their susceptibility to fracture differ.

Bone undergoes continuous remodeling (turnover, renewal) throughout life. Mechanical and electrical forces, hormones and local regulatory factors influence remodeling. Bone is renewed by two opposing activities that are coupled in time and space (Parfitt 1979). These activities - resorption and formation - are contained within a temporary anatomic structure known as a bone remodeling unit (Parfitt 1981). Within a given bone remodeling unit, old bone is resorbed by osteoclasts. The resorbed cavity created by the osteoclasts is subsequently filled with new bone by osteoblasts, which synthesize the organic matrix of bone.

Peak bone mass is mainly genetically determined, though dietary factors and physical activity can have positive effects. Peak bone mass is attained at the point when skeletal growth ceases, after which time bone loss starts.

In contrast to the positive balance that occurs during growth, in osteporosis, the resorbed cavity is not completely refilled by bone (Parfitt 1988). Osteoporosis, or porous bone, is a progressive and chronic disease characterized by low bone mass and structural deterioration of bone tissue, leading to bone fragility and an increased susceptibility to fractures of the hip, spine, and wrist (diminishing bone strength).

Bone loss occurs without symptoms. The Consensus Development Conference (Am J Med 94:646-50, 1993) defined osteoporosis as "a systemic skeletal disease characterized by low bone mass and microarchitectural deterioration of bone tissue, with a consequent increase in bone fragility and susceptibility to fracture."

Common types of osteoporosis include postmenopausal osteoporosis; and senile osteoporosis, which generally occurs in later life, e.g., 70+ years; *see, e.g.,* U.S. Patent No. 5,691,153 and documents cited therein and during its prosecution, all incorporated herein by reference.

Osteoporosis is estimated to affect more than 25 million people in the United States (Rosen 1997); and, at least one estimate asserts that osteoporosis affects 1 in 3 women (Keen et al. 1997). However, life expectancy has increased, and in the western world, 17% of women are now over 50 years of age; and, a woman can expect to live a third (1/3) of her life after menopause. Thus, some estimate that 1 out of every 2 women and 1 out of 5 men will eventually develop osteoporosis; and, that 75 million people in the US, Japan and Europe have osteoporosis. The World Summit of Osteoporosis Societies estimates that more than 200 million people world-wide are afflicted with the disease. The actual incidence of the disease is difficult to estimate since the condition is often asymptotic until a bone fracture occurs. It is believed that there are over 1.5 million osteoporosis-associated bone fractures per year in the U.S. of which 300,000 are hip fractures that usually require hospitalization and surgery and may result in lengthy or permanent disability or even death. (*See* Spangler et al. "The Genetic Component of Osteoporosis Mini-review;
http://www.csa.com.osteointro.html)

Further, there is a 20-30% mortality rate related to hip fractures in elderly women (U.S. Patent No. 5,691,153); and, it is reported that such a patient with a hip fracture has a 10-15% greater chance of dying than others of the same age. Further, it is reported that although men suffer fewer hip injuries than women, men are 25% more likely than women to die within one year of the injury. *See* Sprangler et al., *supra.* Also, about 20% of the patients who were living independently before a hip fracture still remain confined in a long-term health care facility one year later; and, the treatment of ostepososis and related fractures can cost over $10 billion annually.

Accordingly, osteoporosis is a major health problem in virtually all societies (Eisman 1996; Wark 1996; U.S. Patent No. 5,834,200 and the documents cited therein, being hereby incorporated herein by reference).

Treatment for osteoporosis helps stop further bone loss and fractures, such as HRT (hormone replacement therapy), bisphosphonates, e.g., alendronate (Fosamax), as well as, estrogen and estrogen receptor modulators, progestin, calcitonin, and vitamin D.
While there may be numerous factors that determine whether any particular person will develop osteoporosis, a step towards prevention, control or treatment of osteoporosis is determining whether one is at risk for osteoporosis. Genetic factors are said to play an important role in the pathogenesis of osteoporosis (Ralston 1997; *see also* Keen et al. 1997; Eisman 1996; Rosen 1997; Cole 1998, Johnston et al. 1995; Gong et al. 1996; Wasnich 1996 *inter alia*).
Some attribute 50-60% of total bone variation (Bone Mineral Density; BMD), depending upon the bone area, to genetic effects (Livshits et al. 1996). However, up to 85%-90% of the variance in bone mineral density may be genetically determined.

For instance, as studies have shown from family histories, twin studies, and racial factors, there may be a predisposition for osteoporosis (*see, e.g.,* Jouanny et al. 1995; Garnero et al. 1996; Cummings 1996; Lonzer et al. 1996). Several candidate genes may be involved in this, most probably multigenic process.

Association between vitamin D receptor gene (VDR) allelic variation and BMD has been suggested.Restriction fragment length polymorphisms (RFLPs) at the vitamin D receptor (VDR) gene locus have been recently correlated to bone mineral density (BMD) and rate of bone loss (*see, e.g.,* Tokita et al. 1996; Cole et al. 1998; Eisman 1996; Keen et al. Ralston 1997; Fujita 1996; Houston et al. 1996; Riggs et al. 1995; Fleet et al. 1995; Krall et al. 1995).

Collagen type I alpha gene has been implicated (*see, e.g.,* Dalgleish 1997; Pereira et al. 1995). The COLIA1 and COLIA2 genes encode type I collagen, a key bone protein, and, therefore, may play a role in the genetic control of bone mass.

Mutation of the estrogen receptor (ER) gene may be implicated in some cases of osteoporosis (polymorphism of the ER gene has been correlated with BMD in some populations) (*see* Sano et al. 1995; *see also* U.S. Patent No. 5,834,200). Interleukin 1 (IL-1) and tumor necrosis factor alpha (TNF-alpha) have also been implicated in the pathogenesis of osteoporosis in recent studies. These proinflammatory cytokines induce both cyclooxygenase (COX) and nitric oxide synthase (NOS) with the release of prostaglandin (PG) and NO, respectively. Cytokines have been shown to be powerful regulators of bone resorption and formation, though under superior control from oestrogen/testosterone, parathyroidhormone and 1,25(OH)2D3. Some of the cytokines primarily enhance osteoclastic bone resorption e.g. IL-1 (Interleukin-1), TNF (Tumor Necrosis Factor) and IL-6 (Interleukin-6), while others primarily stimulate bone formation e.g. TGF-beta (Transforming Growth Factor), IGF (Insulin-like Growth Factor) and PDGF (Platelet Derived Growth Factor).

There is need for clinical and epidemiological research to further explore and extend the current potential for practical control, prevention and treatment of the disease. A deeper knowledge of factors controlling bone cell activity and regulation of bone mineral and matrix formation and remodeling is desired.

Further, while certain genetic may be useful for detecting high bone mass or predisosition to low or high bone mineral density (*see* U.S. Patents Nos. 5,691,153 and 5,834,200), there is a need for further tests to determine risk for osteoporosis; and, there is a need for new treatments, preventatives, or means to control osteoporosis or factors or processes which lead to osteoporosis.

### OBJECTS AND SUMMARY OF THE INVENTION

An object of the invention can include any one or any combination or all of: advancing clinical and epidemiological research and/or further exploring and extending the current potential for practical control, prevention and treatment of the disease; providing further knowledge of factors controlling bone cell activity and regulation of bone mineral and matrix formation and remodeling; providing further tests to determine risk for osteoporosis; and/or new treatments, preventatives, or means to control osteoporosis or factors or processes which lead to osteoporosis.

The present invention provides mechanical stress induced genes, probes therefor, a test to identify such genes, expression products of such genes, uses for such genes and expression products, e.g., in diagnosis (for instance risk determination), treatment, prevention, control, or osteoporosis or factors or processes which lead to osteoporosis. Thus, the invention further provides diagnostic, treatment, prevention, control methods or processes as well as compositions.

There is disclosed a method for identifying genes whose expression is regulated at the RNA level in an organism including the steps of selectively stimulating translation of an unknown target mRNA with a stress inducing element, the target mRNA being part of a larger sample of mRNA, dividing the sample of mRNA into pools of translated and untranslated mRNA and differentially analyzing the pools of mRNA to identify genes translationally regulated by the stress inducing element. The stress inducing element can include pathologic, environmental including chemical and physical stressors or other stimulus that induces mRNA translation. The stress inducing element can comprise mechanical stress. The sample can comprise bone cells that retain being bone cells in a culture, e.g., calvaria cells.

According to the present application, methods are disclosed for identifying genes that may be regulated on a number of possible regulatory levels. Such methods include the steps of exposing cells or tissue to a cue or stimulus such as mechanical, chemical, toxic, pharmaceutical or other stress, hormones, physiological disorders or disease; fractionating the cells into compartments such as polysomes, nuclei, cytoplasm and spliceosomes; extracting the mRNA from these fractions, and subjecting the mRNA to differential analysis using accepted methodologies, such as gene expression array (GEM).

For instance, the application discloses the use of RNA isolation from nuclei for isolating genes whose steady state levels show only minor changes, but which show high differential expression when detected by nuclear RNA probe. Most such genes are regulated at the transcriptional level. One type of regulation is shown using polysomes isolated from cells/tissues to identify genes whose mRNA steady state levels do not change, but are highly increased in the polysomes after application of a stress cue. Such genes are regulated strictly on the translation level. A subgroup of genes regulated on the translational level involves the existence of internal ribosome entry sites. A method is disclosed for identification of such genes, which includes inhibiting 5'cap-dependant mRNA translation in a cell, collecting a pool of mRNA from the cells, and differentially analyzing the pool of mRNA to identify genes with sequences coding for internal ribosome entry sites.

Thus, the application discloses a method or process for identifying genes whose expression is responsive to a specific cue or cues including the steps of:
(a) applying a cue to an organism or tissue or cells;
(b) isolating specific cellular fractions from the tissues or cells subjected to the cue;
(c) extracting the mRNA from the cellular fractions; and
(d) differentially analyzing the mRNA samples in comparison with control samples not subjected to the cue to identify genes that have responded to the cue.

The cells or tissues can be bone cells which retain the nature of being bone cells when in a culture and the cue can be mechanical stress or a lack thereof.

The cue can be a toxin or a chemical, or a pharmaceutical, or a mechanical stress, or an electric current, or a pathogen or a pathological condition, or a hormone, or a specific protein. The cue can be further defined as chemically treating the cells, or irradiating the cells, or depriving the cells of oxygen. The cue can be further defined as a stress-inducing element of unknown relationship to gene translation.

The genes can be identified at the translation level; genes regulated at the transcription level; genes regulated by RNA stability; genes regulated by mRNA transport rate between the nucleus and cytoplasm; genes regulated by differential splicing; and genes regulated by antisense RNA.

The mRNA samples can be further fractionated into mRNA subfractions which are subjected to differential analysis to identify genes responsive to the cue at all levels of expression regulation as herein defined and to determine the abundance and direction of the response. The mRNA sample can be fractionated into one or more subfractions from the group consisting essentially of cytoplasmic, nuclear, polyribosomal, sub polyribosomal, microsomal or rough endoplasmic reticulum, mitochondrial and splicesome associated mRNA.

The differential analysis step can be selected from the group consisting of differential display, representational differential analysis (RDA), suppressive subtraction hybridization (SSH), serial analysis of gene expression (SAGE), gene expression microarray (GEM), nucleic acid chip technology, oligonucleotide chip technology; DNA membrane arrays; direct sequencing and variations and combinations of these methods. The differential analysis step can be further defined as identifying and measuring the genes regulated at the translation level. The differential analysis step can also be further defined as identifying and measuring the genes regulated at the transcription level. The differential analysis step can also be further defined as identifying and measuring the genes regulated by RNA stability. The differential analysis step can additionally be further defined as identifying and measuring the genes regulated by mRNA transport rate between the nucleus and the cytoplasm. The differential analysis step can also be further defined as identifying and measuring the genes regulated by differential splicing. The differential analysis step additionally can be further defined as identifying and measuring the genes encoding secreted and membrane proteins. The differential analysis step can also be further defined as identifying and measuring the genes encoding for nuclear proteins.

The application further discloses a method for determining risk of developing a physiological or disease state based upon absence or decrease from normal cells of mRNA or protein from a gene shown to be down regulated in a mammal by an inventive or disclosed method comprising:
(a) determining the level or status of mRNA in cells of said mammal; and/or
(b) determining the level or status of corresponding protein in cells of said mammal; and
(c) comparing said level or status of mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein, and from the comparing determining an absence or decrease from normal cells of mRNA or protein and thus the risk of developing a physiological or disease state.

The application further discloses a method for determining risk of physiological or disease state based upon presence or an increase from normal cells of mRNA or protein from a gene shown to be upregulated by an inventive or disclosed method in a mammal comprising:
(a) determining the level or status of mRNA in cells of said mammal; and/or
(b) determining the level or status of corresponding protein in cells of said mammal; and
(c) comparing said level or status of mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein, and from the comparing determining a presence or increase from normal cells of mRNA or protein and thus risk.

These "determining" methods can be diagnostic methods; e.g., methods for diagnosing a physiological or disease state.

The application further discloses a method for testing a medicament for or a gene therapy approach to a physiological or disease state or other factors causing or contributing thereto or to symptoms thereof based on absence or decrease from normal cells or presence or increase from normal cells of mRNA or protein of identified genes comprising an inventive or disclosed method additionally comprising: (a') administering the medicament or the gene therapy; and from the comparing determining an absence or decrease from normal cells or presence or increase from normal cells of the relevant mRNA or protein and thus efficacy of the medicament or the gene therapy.

The application still further discloses a method for treating, preventing or controlling a physiological or disease state comprising an inventive or disclosed method and additionally comprising administering a medicament or treatment therefor or for a cause thereof or a symptom thereof.

The medicament or treatment can comprise the protein, a functional portion thereof, a vector expressing the protein or a functional portion thereof, or an inhibitor of the protein or of a functional portion thereof, or an inhibitor of a nucleic acid encoding the protein or a functional portion thereof.

Inventive or disclosed methods can further comprise:
(d) determining the level or status of a second gene mRNA in cells of said mammal; and/or
(e) determining the level or status of protein expressed by a second gene product in cells of said mammal; and
(f) comparing said level or status of that mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, which may bear a mutation, deletion or any other modifications which would affect the overall activity of the gene when compared to the wild-type normal gene product, including post-translational modifications of the protein, and from the comparing determining risk.

Steps (a) and/or (b) and optionally (d) and/or (e) can be carried out *in vivo* and/or steps (a) and/or (b) and optionally (d) and/or (e) can be carried out *in vitro.* The determination in step (a) and optionally in step (d) can be effected by employing
(i) a nucleic acid sequence corresponding to at least a part of the gene encoding at least part of the protein and optionally a second nucleic acid sequence corresponding to at least a part of the second gene encoding at least part of the second protein;
(ii) a nucleic acid sequence complementary to the nucleic acid sequence(s) of (i); or
(iii) a primer or a primer pair hybridizing to the nucleic acid sequence(s) of (i) or (ii).

The determination in step (b) and optionally of step (e) can be effected by employing an antibody or a fragment thereof that specifically binds to the protein and optionally by employing a second antibody or a fragment thereof which specifically binds to the second protein.

In inventive methods, the stimulus can be mechanical stress or a lack thereof and the sample comprises bone cells which retain their characteristic thereof in cultures.

The invention further provides a gene identification process comprising: preparation of probes from a model system; analysis of DNA chip hybridization; sequencing of clones showing differential expression; and optionally full-length cloning of clones of interest.

The model system can comprise bone cells which retain their characteristic thereof in cultures which have mechanical stress or a lack thereof applied thereto. The bone cells can comprise a calvaria primary culture.

The invention further provides a method for determining risk of developing osteoporosis or low or high bone density or other factors causing or contributing to osteporosis or other conditions involving mechanical stress or a lack thereof, based upon absence or decrease from normal cells of mRNA or protein from a gene shown to be down regulated by an inventive method comprising:
(a) determining the level or status of mRNA in bone cells of said mammal; and/or
(b) determining the level or status of corresponding protein in bone cells of said mammal; and
(c) comparing said level or status of mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein, and from the comparing determining an absence or decrease from normal cells of mRNA or protein and thus risk.

The invention still further provides a method for determining risk of developing osteoporosis or low or high bone density or other factors causing or contributing to osteporosis or other conditions involving mechanical stress or a lack thereof, based upon presence or increase from normal cells of mRNA or protein from a gene shown to be upregulated by an inventive method in a mammal comprising:
(a) determining the level or status of mRNA in bone cells of said mammal; and/or
(b) determining the level or status of corresponding protein in bone cells of said mammal; and
(c) comparing said level or status of mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein, and from the comparing determining a presence or increase from normal cells of mRNA or protein and thus risk.

The invention also provides a method for determining risk of developing osteoporosis or low or high bone density or other factors causing or contributing to osteporosis or lower levels of osteoblasts and chondrocytes or other conditions involving mechanical stress or a lack thereof, based upon absence or decrease from normal cells of mRNA or protein from 608 comprising:
(a) determining the level or status of mRNA in bone cells of said mammal; and/or
(b) determining the level or status of corresponding protein in bone cells of said mammal; and
(c) comparing said level or status of mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein, and from the comparing determining a presence or increase from normal cells of mRNA or protein and thus risk.

Further still, the invention provides a method for determining risk of developing osteoporosis or low or high bone density or other factors causing or contributing to osteporosis or of imbalance as to osteogenic and chondrogenic cells or other conditions involving mechanical stress or a lack thereof, based upon absence or decrease from normal cells or presence or increase from normal cells of mRNA or protein from 405 in a mammal comprising:
(a) determining the level or status of mRNA in bone cellsof said mammal; and/or
(b) determining the level or status of corresponding protein in bone cells of said mammal; and
(c) comparing said level or status of mRNA and/or protein with the corresponding level in normal cells; wherein the term "level" denotes the amount ofmRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein, and from the comparing determining an absence or decrease from normal cells of mRNA or protein and thus risk.

Even further, the invention provides a method for determining risk of developing osteoporosis or low or high bone density or other factors causing or contributing to osteporosis or of being susceptible to environmental factors or other than genetic factors of osteoporosis or of predisposition of bones towards susceptibility to environmental factors, or less lymphoid cells, or osteopososis, or other conditions involving mechanical stress or a lack thereof, based upon presence or increase from normal cells or absence or decrease from normal cells of mRNA or protein from 274 in a mammal comprising:
(a) determining the level or status of mRNA in bone cells of said mammal; and/or
(b) determining the level or status of corresponding protein in bone cells of said mammal; and
(c) comparing said level or status of mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein, and from the comparing determining an absence or decrease from normal cells of mRNA or protein and thus risk.

These "determining" methods can be diagnostic methods; e.g., methods for diagnosing osteoporosis or for diagnosing other conditions recited in the preamble of these "determining" methods.

Also, the invention further provides a method for testing a medicament for or gene therapy approach to osteoporosis or bone density or other factors causing or contributing to osteporosis or symptoms thereof or other conditions involving mechanical stress or a lack thereof, based on absence or decrease from normal cells or presence or increase from normal cells of mRNA or protein comprising a method according to any one of the foregoing inventive methods and additionally comprising: (a') administering the medicament or the gene therapy; and from the comparing determining an absence or decrease from normal cells or presence or increase from normal cells of the relevant mRNA or protein and thus efficacy of the medicament or the gene therapy.

The invention also comprehends analogous methods with respect to other genes identified by inventive processes, e.g., CMF2-224, CMF2-45.

Similarly, the invention additionally provides a method for treating, preventing or controlling osteporosis or other conditions involving mechanical stress or a lack thereof, comprising a method according to any one of the foregoing inventive methods and further comprising administering a medicament or treatment for osteoporosis or a cause thereof or a symptom thereof.

Still further, the invention provides a composition comprising a gene or portion thereof or a protein or portion thereof expressed by the gene or portion thereof or an antibody or portion thereof which binds to the protein or portion thereof, wherein the gene is identified by an inventive method.

Even further still, the invention provides an osteoporosis or mechanical stress or lack thereof model comprising bone cells which retain their characteristic thereof in culture with mechanical stress applied thereto or an absence of mechanical stress applied thereto.

The invention additionally provides an isolated nucleic acid molecule encoding the herein identified protein 608 or a functional portion thereof or a polypeptide which is at least substantially homologous or identical thereto.

Further, the invention provides an isolated nucleic acid molecule encoding the herein identified protein 405 or a functional portion thereof or a polypeptide which is at least substantially homologous or identical thereto.

Also, the invention provides an isolated nucleic acid molecule encoding the herein identified protein 274 or a functional portion thereof or a polypeptide which is at least substantially homologous or identical thereto.

The invention comprehends an isolated nucleic acid molecule encoding human protein 608 or a functional portion thereof. The invention further comprehends an isolated nucleic acid molecule encoding human protein 405 or a functional portion thereof. And, the invention comrpehends the isolated nucleic acid molecule encoding human protein 274 or a functional portion thereof. In particular embodiments, the invention provides the isolated nucleic acid molecules identified herein by sequence numbers, as well as functional portions thereof.

The invention further encompasses a vector comprising an inventive isolated nucleic acid molecule, a composition comprising such a vector, a probe or primer which specifically hybridizes to such an isolated nucleic acid molecule, and an expression product of such an isolated nucleic acid molecule.

The invention still further provides an isolated polypeptide herein identified as protein 608 or a functional portion thereof or a polypeptide which is at least substantially homologous or identical thereto.

The invention also provides an isolated polypeptide herein identified as protein 405 or a functional portion thereof or a polypeptide which is at least substantially homologous or identical thereto.

And, the invention provides an isolated polypeptide herein identified as protein 274 or a functional portion thereof or a polypeptide which is at least substantially homologous or identical thereto.

The invention comprehends an isolated polypeptide which is human protein 608 or a functional portion thereof, as well as an isolated polypeptide which is human protein 405 or a functional portion thereof, and an isolated polypeptide which is human protein 274 or a functional portion thereof. The invention further comprehends polypeptides identified by sequence identification numbers, as well as polypeptides from expression of nucleic acid molecules identified by sequence identification numbers; and, functional portions thereof. Further still, the invention comprehends compositions comprising an inventive polypeptide or portion thereof. Even further, the invention envisions an antibody elicited by an inventive polypeptide or a functional portion thereof, as well as a functional portion of such an antibody; and, compositions comprising such an antibody or portion thereof.

The invention further encompasses methods for preventing, treating or controlling osteoporosis or bone density or other factors causing or contributing to osteporosis or symptoms thereof or other conditions involving mechanical stress or a lack thereof, comprising administering an inventive polypeptide or portion thereof; and accordingly, the invention comprehends uses of polypeptides in preparing a medicament or therapy for such prevention, treatment or control.

The invention even further encompasses a method for preventing, treating or controlling osteoporosis or bone density or other factors causing or contributing to osteoporosis or symptoms thereof or other conditions involving mechanical stress or a lack thereof, comprising administering an inventive vector or inventive nucleic acid molecules; and accordingly, the invention comprehends uses of such vectors or nucleic acid molecules in preparing a medicament or therapy for such prevention, treatment or control.

The invention also comprehends a method for preventing, treating or controlling osteoporosis or bone density or other factors causing or contributing to osteporosis or symptoms thereof or other conditions involving mechanical stress or a lack thereof, comprising administering a composition comprising a gene or functional portion thereof identified in inventive methods or an inventive model or an expression product thereof or an antibody or portion thereof elicited by such an expression product or portion thereof; and, the invention thus further comprehends uses of such genes, expression products, antibodies, portions thereof, in the preparation of a medicament or thereapy for such control, prevention or treatment.

The invention yet further provides methods for preparing a polypeptide comprising expressing the polypeptide from inventive vectors or from inventive genes or genes identified in inventive methods or models, or portions of such genes.

Further still, the invention envisions advancing research in or studies of bone development and/or osteopososis comprising the inventive methods, materials/products, and/or models.

The invention comprehends genes differentially expressed under the influence of (a) mechanical force applied to a calvaria primary cell culture and (b) treatment of PGE2 applied to the same culture. In addition the invention involves the effect of calcium depletion. The genes that are differentially expressed are thus demonstrated to be involved in the processes that lead to osteoporosis or other mechanical stress or lack thereof related conditions.

Certain genes identifed by the methods herein respond to estrogen. From the methods disclosed herein one can identify compounds to which genes idenfied by herein methods respond. Thus, the invention comprehends a method for affecting a gene identified by any one of the herein methods comprising contacting cells containing the gene with a compound to which the gene responds; e.g., administering the compound as a composition or formulation as herein described. Thus, for instance, with respect to genes which respond to estrogen, the invention envisions a method for affecting (e.g., stimulating expression, inhibiting expression, and the like) the gene comprising contacting a cell containing the gene with estrogen or a derivative or precursor thereof, e.g., 17-Beta estradiol and the like.

It is noted that in this disclosure, the word "comprises" can have the meaning attributed to it in U.S. Patent law; e.g., it can mean "includes".

These and other embodiments are disclosed or are obvious from and encompassed by, the following Detailed Description.

### BRIEF DESCRIPTION OF FIGURES

The following Detailed Description, given by way of example, but not intended to limit the invention to specific embodiments described, may be understood in conjunction with the accompanying Figures, incorporated herein by reference, in which:
Figure 1 A shows an absorbance profile of a fractionation of cytoplasmic RNA on a sucrose density gradient wherein the absorbance (at 254nm) is plotted against the sedimentation rate of the cytoplasmic RNA;
Figure 1B shows a purified RNA electrophoresed on an agarose gel and stained with ethidium bromide illustrating the fractionation of RNA;
Figure 2 shows a representation of DNA chip hybridization results comparing probes of total RNA (Tot) to probes derived from nuclear RNA (STP);
Figure 2A shows a table of genes identified by inventive methods, and sequences therefor or sequences of ESTs thereof;
Figure 3 shows DNA and amino acid sequences for inventive nucleic acid molecule 608 and the expression product therefrom with this Figure differing from other 608 sequences herein in that Figure 3 shows additional protein sequences towards the 5' end (compare Figure 3 from about position 1025 with other 608 sequence figures herein);
Figure 4 shows the results of a 5' fragment probe of inventive nucleic acid molecule 608 on target mRNA in normal and mechanically stressed cells;
Figure 5 shows DNA and amino acid sequences for inventive nucleic acid molecule 608 and the expression product therefrom;
Figure 6 shows Clustal X (1.64b) Multiple Sequence Alignment with respect to inventive nucleic acid molecule 608 and probes therefor;
Figures 7 shows the results of a probe of human 405 on the target total RNA of human k562;
Figure 8 shows the results of a probe of human 405 on the target rat cmf RNA;
Figures 9 and 10 show the DNA and amino acid sequences for inventive nucleic acid molecule 405 and the expression product therefrom;
Figure 11 shows Clustal X (1.64b) Multiple Sequence Alignment with respect to inventive nucleic acid molecule 405 and probes therefor;
Figure 12 shows the results of a probe of 8 KB of human 274 on the target rat bone, rat testes and human cell line NB4 total RNA sources;
Figure 13 shows the DNA and amino acid sequences for inventive nucleic acid molecule 274 and the expression product therefrom;
Figure 14 shows the DNA and amino acid sequences for inventive nucleic acid molecule 274 and the expression product therefrom.
(Markings on sequence figures, e.g., sequence figures such those for 608, such as dots and plus/minus signs may indicate repeats, such as IgG repeats, that may appear in many proteins; there are approximately 20 such IgG repeats in the 608 sequence listing.)

### DETAILED DESCRIPTION

As discussed, disclosed herein is a method for identifying genes whose expression is regulated at the RNA level in an organism.

More in particular, disclosed herein is a method of identifying genes whose expression is regulated at least in part at the mRNA level by selectively stimulating an unknown target mRNA with a stress inducing element, the target mRNA being part of a larger sample. The organism may be any organism which provides suitable mRNA. The mRNA sample is derived from cellular compartments based on expression regulation and protein localization which are differentially analyzed to identify genes which are translationally regulated by the stress inducing element. This method is designed for identifying and cloning genes which are responsive to specific cues. That is, the present method is designed for identifying and cloning genes which are either up- or down- regulated responsive to a specific pathology, stress, physiological condition, and so on, and in generally to any factor that can influence cells or organisms to alter their gene expression.

This disclosure provides a novel approach to the identification and cloning of genes that are involved in fundamental cellular functions and which are regulated at any level in an organism. The basic underlying theory for this method relies on the knowledge that the regulation of gene expression can be controlled at different levels (modes) and that each different regulation level(s) is manifested by some difference in the distribution of the specific mRNAs in the cell. In genes that are regulated by translation, the mRNA is stored in the cell in an inactive form and will not be found on polysomes. Following the appropriate external cue, the mRNA is incorporated into the polysomes and translated, and the encoded protein quickly appears. By comparing mRNA populations that are "active" or "non-active" at a given time, genes that are regulated by a mechanism referred to as the "shift mechanism" can be identified.

Genes whose main regulatory level is the active transport of mRNA from the nucleus to the cytoplasm are stored in the nucleus and at the appropriate cue the mRNA is transported to the cytoplasm. Comparison of mRNA isolated from the nucleus and cytoplasm before and after the cue can lead to the discovery of genes controlled in this way. The comparison of mRNA derived from the nucleus also allows direct analysis of the transcription activity of many genes. For most transcriptionally activated genes a basal level of mRNA exists in the cell even when the basal transcription activity is low. Thus, increased transcription (up to five-fold) is often obscured when total cellular RNA is used for differential analysis of gene expression. The use of nuclear RNA allows direct measurement of transcription activity of many genes, since the basal mRNA is found in the cytoplasm. The result is a major increase in sensitivity for the detection of differential expression.

In the case of mRNA stability regulation, it is expected that such mRNA would be similarly transcribed before and after cue administration, resulting in a similar abundance in nuclear mRNA pools. However, if the mRNA is stabilized following the cue, its abundance in the cytoplasm would become higher. In the case of mRNA transport regulation, such mRNA is expected to exist at a high level in the nucleus and a low level in the cytoplasm prior to the cue, which situation would be reversed after administration of the cue. It is thus easy to differentiate between the two regulatory modes.

The method of the invention includes the identification of genes regulated at the translational level; genes regulated at the transcription level; genes regulated by RNA stability; genes regulated by mRNA transport rate between the nucleus and the cytoplasm; and genes regulated by differential splicing. That is, genes whose expression is at least partly controlled or regulated at the mRNA level can be identified.

The method will identify genes encoding secreted and membrane proteins; genes encoding for nuclear proteins; genes encoding for mitochondrial proteins; and genes encoding for cytoskeletal proteins. In addition, any other gene whose expression can be controlled at the mRNA level can be identified by this method.

As used herein, RNA refers to RNA isolated from cell cultures, cultured tissues or cells or tissues isolated from organisms which are stimulated, differentiated, exposed to a chemical compound, are infected with a pathogen or otherwise stimulated. As used herein, translation is defined as the synthesis of protein on an mRNA template.

As used herein, stimulation of translation, transcription, stability or transportation of unknown target mRNA or stimulating element, includes chemically, pathogenically, physically, or otherwise inducing or repressing an mRNA population from genes which can be derived from native tissues and/or cells under pathological and/or stress conditions. In other words, stimulating the expression of a gene's mRNA with a stress inducing element or "stressor" can include the application of an external cue, stimulus, or stimuli which stimulates or initiates translation of a mRNA stored as untranslated mRNA in the cells from the sample. The stressor may cause an increase in stability of certain mRNAs, or induce the transport of specific mRNAs from the nucleus to the cytoplasm. The stressor may also induce gene transcription. In addition to stimulating translation of mRNA from genes in native cells/tissues, stimulation can include induction and/or repression of genes under pathological and/or stress conditions. The present method utilizes a stimulus or stressor to identify unknown target genes which are regulated at the various possible levels by the stress inducing element or stressor.

The method synergistically integrates methodologies which were not previously used together.

One methodology comprises the division of cellular mRNA into separate pools of mRNA derived from polysomes, nucleus, cytoplasm or spliceosomes.

Another methodology comprises the simultaneous comparison of the relative abundance of the mRNA species found in the separate pools by a method of differential analysis such as differential display, representational difference analysis (RDA), gene expression microarray (GEM), suppressive subtraction hybridization (SSH) (Diatchenko et al., 1996), and oligonucleotide chip techniques such as the chip technology exemplified by United States Patent No. 5,545,531 to Rava et al. assigned to Affymax Technologies N.V. and direct sequencing exemplified by WO 96/17957 patent application to Hyseq, Inc.

Briefly, subtractive hybridization is defined as subtraction of mRNA by hybridization in solution. RNAs that are common to the two pools form a duplex that can be removed, enriching for RNAs that are unique or more abundant in one pool. Differential Display is defined as reverse transcription of mRNA into cDNA and PCR amplification with degenerated primers. Comparison of the amounts amplification products (by electrophoresis) from two pools indicate transcript abundance. RDA, GEM, SSH, SAGE are described herein above.

The specific cells/tissues which are to be analyzed in order to identify translationally regulated genes, can include any suitable cells and/or tissues. Any cell type or tissue can be used, whether an established cell line or culture or whether directly isolated from an exposed organism.

The cells/tissues to be analyzed under the present method are selectively stimulated or "stressed" utilizing a physiological, chemical, environmental and/or pathological stress inducing element or stressor, in order to stimulate the translation of mRNA within the sample tissue and identify genes whose expression is regulated at least in part at the mRNA level. Stimulation can cause up or down regulation. Following stimulation, RNA is isolated or extracted from the cells/tissues. The isolation of the RNA can be performed utilizing techniques which are well known to those skilled in the art and are described, for example, in "Molecular Cloning; A Laboratory Manual" (Cold Springs Harbor Laboratory Press, Cold Spring Harbor, New York, 1989). Other methods for the isolation and extraction of RNA from cells/tissue can be used and will be known to those of ordinary skill in the art. (Mach et al., 1986, Jefferies et al., 1994). However, may variations of these methodologies have been published. The methods described herein were carefully selected after many trials.

The mRNAs which are actively engaged in translation and those which remain untranslated can be separated utilizing a procedure such as fractionation on a sucrose density gradient, high performance gel filtration chromatography, or polyacrylamide gel matrix separation (Ogishima et al., 1984, Menaker et al., 1974, Hirama et al., 1986, Mechler, 1987, and Bharucha and Murthy, 1992), since mRNAs that are being translated are loaded with ribosomes and, therefore, will migrate differently on a density gradient than ribosome-free untranslated mRNAs. By comparing mRNA populations that are active or non-active in translation at a given time, genes that are regulated by the "shift mechanism" can be identified.

Polysomal fractionation and specific analysis can be facilitated by treatment of target cell/tissue with drugs that will specifically inhibit or modulate transcription or translation. Examples of such drugs are actinomycin D and cyclohexamide, respectively.

The fractionation can be completed to create polysomal subdivisions. The subdivisions can be made to discriminate between total polyribosomes or membrane bound ribosomes by methods known in the art (Mechler, 1987). Further, the mRNA sample can additionally be fractionated into one or more of at least the following subsegments or fractions: cytoplasmatic, nuclear, polyribosomal, sub polyribosomal, microsomal or rough endoplasmic reticulum, mitochondrial and splicesome associated mRNA by methods known in the art.

More specifically, nuclear fractions can be obtained using the method set forth in the article entitled Abundant Nuclear Ribonucleoprotein Form of CAD RNA (Sperling, 1984) as set forth in the Examples, thus allowing nuclear RNA to be utilized for a method of identifying genes which are regulated or responsive to stress conditions. Further, antisense RNA can be utilized as a method for identifying genes which are responsive to specific pathology or stress conditions. Antisense RNA can be isolated using the methods described by Dimitrijevic, whose abstract details the methods utilized for obtaining and isolating antisense RNA from a sample. Additionally, microsomal fractions may be obtained using the methods of the present invention as set forth in the Experimental Section which are modifications of the methods disclosed by Walter and Blobel in 1983.

Following isolation and division of the total mRNA population into separate expression regulation and protein localization pools of mRNA, the relative abundance of the many mRNA species found in these pools are simultaneously compared using a differential analysis technique such as differential display, oligonucleotide chips, representational difference analysis (RDA), GEM-Gene Expression Microarrays (Schena et al., 1995, Aiello et al., 1994, Shen et al., 1995, Bauer et al., 1993, Liang and Pardee, 1992, Liang and Pardee, 1995, Liang et al., 1993, Braun et al., 1995, Hubank and Schatz, 1994) and suppressive subtraction hybridization (SSH). The RNA isolated from the fractions can be further purified into mRNA without the ribosomal RNA by poly A selection. It should be noted that multiple pools can be analyzed utilizing this method. That is, different cell aliquots subjected to different stressors can be compared with each other as well as with the reference sample.

Labeled nucleic acid probes (in a cDNA, PCR product or rRNA transcribed from the cDNA) made from RNA derived from polysomal, non-polysomal, mRNPs, nuclear, cytoplasmic, or spliceosome fractions can be used as probes, to identify clones of cDNA, genomic clones, and mRNA species that are fixed onto a solid matrix-like microarrays such as (GEM), that shown in United States Patent Number 5,545,531 to Rava et al. and WO96/17957 to Hyseq, Inc., and membranes of any kind where clones can be either blotted after electrophoresis or directly loaded (dot blot) onto the membrane. The label can be radioactive, fluorescent, or incorporating a modified base such as digoxigenin and biotin.

Comparison between the fractions derived from the polysomal or polyribosomal fraction or other fractions to the total unfractionated material is essential to discriminate between differentials in expression levels that are the result of transcription modulation from those that result from modulation of translation per se. The polysomal fractions or groups can include membrane bound polysomes, loose or tight polysomes, or free unbound polysome groups.

The importance of utilizing the polysomal sub-population in order to identify differentially (translationally) expressed genes is shown in Example 1 where a number of genes were not detected as translationally expressed under heat shock inducement when total mRNA was used as the detection probe but, however, when polysomal mRNA was used as a probe, a number of genes were identified as differentially expressed. As shown in Example 1, a number of genes under heat shock inducement with total mRNA derived probe were detected when probed with polysomal mRNA fractions. Heat shock, being a model for acute diseases such as ischemic diseases, reveal the importance of the polysomal probe. Cells store critical mRNAs in an inactive form so that in an acute situation they can be quickly loaded onto polysomes (without the need to wait for their production by transcription) and translated to produce the proteins the cells require for their survival under stress.

The present method for identifying translationally regulated genes is not limited by the source of the mRNA pools. Therefore, the present method can be utilized to clone genes from native cells/tissue under pathological and/or stress conditions that are regulated by the "shift mechanism," as well as genes that are induced/repressed under pathological and/or stress conditions. Pathologies can include disease states including those diseases caused by pathogens and trauma. Stress conditions can also include disease states, physical and psychological trauma, and environmental stresses. Following analysis by the selected method of differential analysis, the genes which have been identified as being regulated by translation can be cloned by any suitable cloning methodologies known to those skilled in the art. (Lisitsyn and Wigler, 1993).

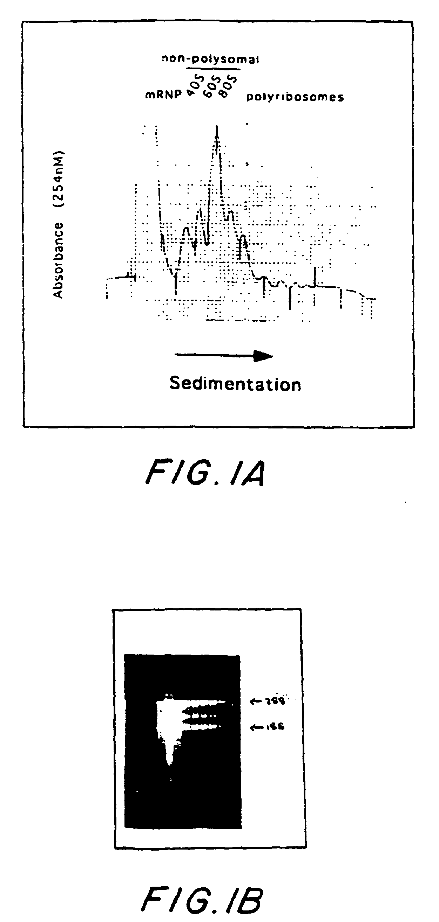

Differential comparisons can be made of all possible permutations of polysomal vs. non-polysomal RNA where the definition of the fraction type is done, for example, by absorbance profile at 254nm, density of the sucrose gradient as shown in Figure 1A (or another size standard if high pressure liquid chromatography or gel systems are used) and types of RNA that are stained with ethidium bromide after electrophoresis of the fractions on agarous gels are completed, as shown in Figure 1B. In Figure 1A, the polysomal fractions are those that have mRNA with more than two ribosomes loaded. The materials and methods for this comparison are set forth below in the experimental section.

Differential comparisons can also include polysomal vs. non-polysomal fractions in each condition. By "condition" it is meant that cells from the same source, such as a cell line, a primary cell, or a tissue that undergoes different treatment or has been modified to have different features or to express different sets of genes. For example, this can be accomplished by differentiation, transformation, application of the stress such as oxygen deprivation, chemical treatment, or radiation. Permutations can include, for example:
1. polysomal fractions between conditions individually (migrating in the same density) or in a pool;
2. non-polysomal fractions between conditions individually (migrating in the same density) or in a pool;
3. non-polysomal to polysomal between conditions and within each condition individually (migrating in the same density) or in a pool; and
4. each of the fractions being polysomal and non-polysomal individually (migrating in the same density) or in a pool that can be compared to total RNA that is unfractionated.

The method described above for the identification of genes regulated on the translational level has a number of applications. A particular application for this method is its use for the detection of changes in the pattern of mRNA expression in cells/tissue associated with any physiological or pathological change. By comparing the translated versus untranslated mRNAs, the effect of the physiological or pathological cue or stress on the change of the pattern of mRNA expression in the cell/tissue can be observed and/or detected. This method can be used to study the effects of a number of cues, stimuli, or stressors to ascertain their effect or contribution to various physiological and pathological activities of the cell/tissue. In particular, the present method can be used to analyze the results of the administrations of pharmaceuticals (drugs) or other chemicals to an individual by comparing the mRNA pattern of a tissue before and after the administration of the drug or chemical. This analysis allows for the identification of drugs, chemicals, or other stimuli which affect cells/tissue at the level of translational regulation. Utilizing this method, it is possible to ascertain if particular mRNA species are involved in particular physiological or disease states and, in particular, to ascertain the specific cells/tissue wherein the external stimulus, i.e., a drug, affects a gene which is regulated at the translational level.

The identification of a subgroup of genes regulated on the translational level involved a method for identifying gene sequences coding for internal ribosome entry sites (IRES), including the general steps of inhibiting 5'cap-dependant mRNA translation in a cell, collecting a pool of mRNA from the cells, and differentially analyzing the pool of mRNA to identify genes with sequences coding for internal ribosome entry sites. The inhibiting step can be further defined as selecting for non-5'-cap dependent mRNA translation or by incorporating a gene, such as a gene coding for a protease such as poliovirus 2A protease. The method can include the step of controlling the expression of the gene. The analyzing step can be further defined as differential display analysis, or as representational difference analysis, or as performing a gene expression microarray analysis. The method can include the further step of cloning genes identified as being translationally regulated. The analyzing step can distinguish between polysomal fractions that migrate in the same density individually or in a pool. The analyzing step can distinguish between nonpolysomal fractions individually or as a pool. The analyzing step can distinguish between stimulated polysomal and nonpolysomal fractions individually or in a pool. And, the analyzing step can distinguish between each of the polysomal and nonpolysomal fractions individually or in a pool compared to an unfractionated total RNA pool.

Utilizing these methods, it is possible to ascertain if particular mRNA species are involved in particular physiological or disease states and, in particular, to ascertain the specific cells/tissue wherein the external stimulus, e.g., a drug, affects a gene which is regulated at the translational level.

Accordingly, in an aspect, the application also discloses a method for determining risk of developing a physiological or disease state based upon absence or decrease from normal cells of mRNA or protein from a gene shown to be down regulated by the inventive or herein disclosed methods in a mammal comprising:
(a) determining the level or status of mRNA in cells, e.g., bone cells (for instance, osteoblasts and/or osteoclasts) of said mammal; and/or
(b) determining the level or status of corresponding protein in cells of said mammal; and
(c) comparing said level or status of mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein, and from the comparing determining an absence or decrease from normal cells of mRNA or protein and thus risk.

In another aspect, the disclosure herein provides a method for determining risk of physiological or disease state based upon presence or increase from normal cells of mRNA or protein from a gene shown to be upregulated by the inventive or herein disclosed methods in a mammal comprising:
(a) determining the level or status of mRNA in cells, e.g., bone cells (for instance, osteoblasts and/or osteoclasts) of said mammal; and/or
(b) determining the level or status of corresponding protein in cells of said mammal; and
(c) comparing said level or status of mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein, and from the comparing determining a presence or increase from normal cells of mRNA or protein and thus risk.

The foregoing methods can be employed in inventive methods for testing a medicament for or a gene therapy approach to a physiological or disease state or other factors causing or contributing thereto or to symptoms thereof based on absence or decrease from normal cells or presence or increase from normal cells of mRNA or protein of identified genes additionally comprising: (a') administering the medicament or the gene therapy; and from the comparing determining an absence or decrease from normal cells or presence or increase from normal cells of the relevant mRNA or protein and thus efficacy of the medicament or the gene therapy.

Similarly, in still further aspects, the disclosure herein provides methods for treating, preventing or controlling a physiological or disease state comprising administering a medicament or treatment therefor or for a cause thereof or a symptom thereof, including the foregoing detection methods. For instance, from the comparing one determines an absence or decrease from normal cells or presence or increase from normal cells of particular mRNA or protein and thus risk and administers a the medicament or treatment.

The methods can additionally comprise using the steps in conjunction with another test method akin to those described above, e.g., having a same or similar preamble recitation and comprising:
(d) determining the level or status of a second gene mRNA in cells of said mammal; and/or
(e) determining the level or status of protein expressed by a second gene product in cells of said mammal; and
(f) comparing said level or status of that mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein.

The absence or decrease or presence or increase may be correlated to risk.

Thus, the second gene can be identified by methods of the invention or as disclosed herein. Or alternatively or additionally, the second gene and/or the additional steps can be determined in accordance with other methods, e.g., other methods for determining the risk of the physiological or disease state or a condition or factor associated therewith. Thus, such methods can be used in conjunction with methods herein to advance or improve diagnostic or detection methodologies.

In the methods, steps (a) and/or (b) and optionally (d) and/or (e) are carried out *in vivo* and/or steps (a) and/or (b) and optionally (d) and/or (e) are carried out *in vitro.*

The determination in step (a) and optionally in step (d) can be effected by employing
(i) a nucleic acid sequence corresponding to at least a part of the gene encoding at least part of the protein and optionally a second nucleic acid sequence corresponding to at least a part of the second gene encoding at least part of the second protein;
(ii) a nucleic acid sequence complementary to the nucleic acid sequence(s) of (i); or
(iii) a primer or a primer pair hybridizing to the nucleic acid sequence(s) of (i) or (ii).

The determination in step (b) and optionally of step (e) can be effected by employing an antibody or a fragment thereof that specifically binds to the protein and optionally by employing a second antibody or a fragment thereof which specifically binds to the second protein.

Cells may be considered "normal" in the methods by having an absence of the physiological or disease being tested for; or by any other standard definition recognized in the art.

The medicament or treatment can be any conventional medicament or treatment for the physiological or disease. Alternatively or additionally, the medicament or treatment can be the particular protein of the gene detected in the inventive methods or a functional portion thereof, or that which inhibits that protein, e.g., binds to it. Similarly, additionally or alternatively, the medicament or treatment can be a vector which expresses the protein of the gene detected in the inventive methods or a functional portion thereof or that which inhibits expression of that gene; again, for instance, that which can bind to it and/or otherwise prevents its transcription or translation. The selection of administering a protein or that which expresses it, or of administering that which inhibits the protein or the gene expression, can be done without undue experimentation, e.g., based on down regulation or up regulation as determined by inventive methods (e.g., in the osteoporosis model).

In an aspect of the invention, the stimulus in inventive methods is mechanical stress or a lack thereof, e.g., with respect to bone cells which retain their characteristic thereof in cultures.

In a further aspect, the invention provides an application of inventive methods with respect to osteoporosis, a major health problem; and provides inventive products and uses therefor. As discussed, osteoporosis or porous bone, is a progressive and chronic disease characterized by low bone mass and structural deterioration of bone tissue, with bone loss being possibly without symptoms, leading to bone fragility and an increased susceptibility to fractures of the hip, spine, and wrist (diminishing bone strength).

Osteoporosis is histologically, biochemical and kinetically heterogeneous. Data points to causes such as: deficiency of estrogen and deficiency of calcium.

Calcium is an essential nutrient that is involved in most metabolic processes and the phosphate salts of which provide mechanical rigidity to the bones and teeth, where 99% of the body's calcium resides. The calcium in the skeleton has the additional role of acting as a reserve supply of calcium to meet the body's metabolic needs in states of calcium deficiency. Calcium deficiency is easily induced because of the obligatory losses of calcium via the bowel, kidneys, and skin. Calcium deficiency delays the consolidation of the skeleton, may cause mobilization of bones and has been shown in animals to lead to osteoporosis.

Further, bone is composed of a collagen-rich organic matrix impregnated with mineral, largely calcium and phosphate. Two major forms of bone exist, compact cortical bone forms the external envelopes of the skeleton and trabecular or medullary bone forms plates that traverse the internal cavities of the skeleton. The responses of these two forms to metabolic influences and their susceptibility to fracture differ. Bone undergoes continuous remodeling (turnover) throughout life. Osteoclasts are the cells in the skeleton that responsible for breaking down bones, osteoblasts on the other hand, are capable of forming new bones. Mechanical and electrical forces, hormones and local regulatory factors influence remodeling. Peak bone mass is mainly genetically determined, though dietary factors and physical activity can have positive effects. Peak bone mass is attained at the point when skeletal growth ceases, after which time bone loss starts. Bone mass declines throughout life due to an imbalance in this process.

It is noted that the World Health Organization (WHO Technical Report Series: 843, 1994) characterizes "normal ", e.g., as to women, as bone mineral density (BMD) or bone mineral content (BMC) that is greater than or equal to 1 standard deviation (SD) below the young adult reference range; "low bone mass" as BMD or BMC 1-2.5 SD below the mean of young healthy adults, e.g., women; "osteoporosis" as BMD or BMC greater than 2.5 SD below the mean of young healthy adults, e.g., women; and "severe osteoporosis" as BMD or BMC greater than 2.5 SD below the mean of young healthy adults, e.g., women and the presence of one or more fragility fractures. From this information and the knowledge in the art, the skilled artisan can determine and employ "normal" cells, without any undue experimentation.

Osteoblasts are particularly sensitive to aging phenomena--more sensitive than are osteoclasts--so the negative bone balance increases with increasing age. Age-dependent bone loss is aggravated by reduced calcium absorption, a mutation in the collagen gene and polymorphism in TGF-beta and estrogen receptor proteins.

Cells bind to ECM (extracellular matrix) via specific cell surface receptors such as integrins. When engaging with ECM ligands, these receptors can activate signal transduction pathways within the cells and may act as mechanochemical transducers. Thus, interaction of cells with ECM can modulate gene expression. Among the genes that are, in part, controlled by cell-ECM interactions are those for certain ECM components themselves. Bone cells, remodel their matrix and reorient bone trabeculae in response to mechanical strain.

Accordingly, the nature of the bone cell response can relate to the state of differentiation. Furthermore, evidence shows that prostaglandins are likely to play an important role in the physiologic and pathologic responses of bone tissue. Prostaglandins can stimulate and inhibit bone resorption and formation.
Prostaglandins mediate bone loss due to immobilization, but prostaglandin E2 (PGE2) stimulates bone formation *in vivo.* Prostaglandin production by bone cells is highly regulated by mechanical forces, cytokines, growth factors and systemic hormones. Mechanical stimulation applied to cultured bone cells results in increased production of several prostaglandins including PGE2, prostaglandin 12 (PGI2), and prostaglandin F2a. Addition of indomethacin, which blocks endogenous prostaglandin production, neutralizes the effect of mechanical stress treatment.

Cells isolated from calvaria bone maintain their osteoblastic phenotype in culture. Genetics factors play an important role in the pathogenesis of osteoporosis. It is suggested that up to 85%-90% of the variance in bone mineral density is genetically determined. Thus, calvaria bone cells were used in methods of the invention. Genes differentially expressed under the influence of (a) mechanical force applied to a calvaria primary cell culture and (b) treatment of PGE2 applied to the same culture. In addition, the effect of calcium depletion is also shown. The genes that result differentially expressed are thus demonstrated to be involved in the processes that lead to osteoporosis, and ergo osteoporosis.

It is well accepted that the main process that is characteristic of osteoporosis - enhanced bone resorption - takes place not only in conditions of low estrogen production (menopause women), but also in some other conditions, like treatment with glucocorticoids or bone immobilization. Therefore, it was reasoned that application of mechanical force is stimulatory for bone formation. To model this process, as discussed in the Examples, e.g., Example 2, primary rat calvaria cells grown on elastic membranes and stretched together with this membrane for 20 minutes. Genes expression patterns were compared before and after the application of mechanical force. Particular genes were found to be differentially regulated and/or differentially expressed following mechanical stimulation, validating the osteoporosis model; and, showing that the inventive methods can be used to identify genes, expression products therefrom, probes/primers for such genes, as well as uses for such genes, expression products, probes/primers, *inter alia.*

In an aspect the invention provides a gene identification process. Steps involved in the gene identification process comprise one or more or all of the following: Preparation of probes from the model system (mechanical force, calvaria primary culture); analysis of DNA chip hybridization; sequencing of clones showing differential expression; and full-length cloning of clones of interest (cloning can be by a variety of known methodologies).

In yet another aspect the invention provides an osteoporosis model or a model for other conditions caused by mechanical stress or force, e.g., bone mass formation, comprising rat calvaria cells or another cell which retains osteoblast or osteoclast nature in cell cultures, being subjected to mechanical or other bone growth/formation inducing stress or stimuli or bone loss inducing stress or stimuli.

With respect to mechanical stress and osteoporosis, it is well documented that exercise has very beneficial effects on bone mass. The effect of the zero of gravity on astronauts and their need to do a lot of exercise is also believed well known.
However, as far as the inventors know, efforts to isolate genes involved in the biological interpretation of mechanical stress signals into increase in bone mass have not heretofore been done. And, mention is made of: Binderman I, Duksin D, Harell A, Katzir E, Sachs L Formation of bone tissue in culture from isolated bone cells. J Cell Biol 1974 May;61(2):427-39; Harell A, Dekel S, Binderman I Biochemical effect of mechanical stress on cultured bone cells. Calcif Tissue Res 1977 May;22 Suppl:202-7; Somjen D, Binderman I, Berger E, Harell A Bone remodelling induced by physical stress is prostaglandin E2 mediated. Biochim Biophys Acta 1980 Jan 3;627(1):91-100; Shimshoni Z, Binderman I, Fine N, Somjen D Mechanical and hormonal stimulation of cell cultures derived from young rat mandible condyle. Arch Oral Biol 1984;29(10):827-31; Binderman I, Shimshoni Z, Somjen D Biochemical pathways involved in the translation of physical stimulus into biological message. Calcif Tissue Int 1984;36 Suppl 1:S82-5; Binderman I, Zor U, Kaye AM, Shimshoni Z, Harell A, Somjen D The transduction of mechanical force into biochemical events in bone cells may involve activation of phospholipase A2. Calcif Tissue Int 1988 Apr;42(4):261-6; Binderman I, Berger E, Fine N, Shimshoni Z, Harell A, Somjen D Calvaria derived osteogenic cells: phenotypic expression in culture. Connect Tissue Res 1989;20(1-4):41-7.

The inventive osteoporosis model or model for other conditions caused by mechanical stress or force or lack thereof, encompasses inventive methods and products of inventive methods employed under conditions of little or no gravity, e.g., the results of performing inventive methods, such as those exemplified or analogous to those exemplified, for instance without applying mechanical stress and under conditions of little or no gravity such as on a space vehicle such as a Space Shuttle or a space station as is being constructed.

The invention is still further aspects provides CMF274, expression products therefrom, probes/primers therefor, and uses for such gene, expression products and primers/probes, as well as of functional portions of the gene or of the expression product.

The invention is still further aspects provides CMF405, expression products therefrom, probes/primers therefor, and uses for such gene, expression products and primers/probes, as well as of functional portions of the gene or of the expression product.

The invention is still further aspects provides CMF608, expression products therefrom, probes/primers therefor, and uses for such gene, expression products and primers/probes, as well as of functional portions of the gene or of the expression product.

Species of origin of the sequences: all initial sequences (short fragments of ~500bp) were rat. For 405 a homologue in the form of a partially characterized mRNA was found but there is no published information on its expression in bones. The study in rats was done to prove its function and possible uses in humans (directly or indirectly) for therapeutics. Once the rat sequence is known the isolation of the human homologues can be within the ambit of the skilled artisan; and thus, this disclosure is intended to cover the human homologues as well because these homologues fall within a degree of homology included within the present invention.

More in particular, respect to the herein mentioned nucleic acid molecules and polypeptides therefrom, e.g., the aforementioned nucleic acid molecules (608, 405, 274) and polypeptides expressed from them, the invention further comprehends isolated and/or purified nucleic acid molecules and isolated and/or purified polypeptides having at least about 70%, preferably at least about 75% or about 77% identity or homology ("substantially homologous or identical"), advantageously at least about 80% or about 83%, such as at least about 85% or about 87% homolgy or identity ("significantly homologous or identical"), for instance at least about 90% or about 93% identity or homology ("highly homologous or identical"), more advantageously at least about 95%, e.g., at least about 97%, about 98%, about 99% or even about 100% identity or homology ("very highly homologous or identical" to "identical"; or from about 84-100% identity considered "highly conserved"). The invention also comprehends that these nucleic acid molecules and polypeptides can be used in the same fashion as the herein or aforementioned nucleic acid molecules and polypeptides.

Nucleotide sequence homology can be determined using the "Align" program of Myers and Miller, ("Optimal Alignments in Linear Space", CABIOS 4, 11-17, 1988, incorporated herein by reference) and available at NCBI. Alternatively or additionally, the term "homology" or "identity", for instance, with respect to a nucleotide or amino acid sequence, can indicate a quantitative measure of homology between two sequences. The percent sequence homology can be calculated as (N*_{ref}* - N*_{dif}*)* 100/N*_{ref}*, wherein N*_{dif}* is the total number of non-identical residues in the two sequences when aligned and wherein N*_{ref}* is the number of residues in one of the sequences. Hence, the DNA sequence AGTCAGTC will have a sequence similarity of 75% with the sequence AATCAATC (N*_{ref}* = 8; N*_{dif}*=2).

Alternatively or additionally, "homology" or "identity" with respect to sequences can refer to the number of positions with identical nucleotides or amino acids divided by the number of nucleotides or amino acids in the shorter of the two sequences wherein alignment of the two sequences can be determined in accordance with the Wilbur and Lipman algorithm (Wilbur and Lipman, 1983 PNAS USA 80:726, incorporated herein by reference), for instance, using a window size of 20 nucleotides, a word length of 4 nucleotides, and a gap penalty of 4, and computer-assisted analysis and interpretation of the sequence data including alignment can be conveniently performed using commercially available programs (e.g., Intelligenetics ^{™} Suite, Intelligenetics Inc. CA). When RNA sequences are said to be similar, or have a degree of sequence identity or homology with DNA sequences, thymidine (T) in the DNA sequence is considered equal to uracil (U) in the RNA sequence (*see also* alignment used in Figures).

RNA sequences within the scope of the invention can be derived from DNA sequences, by thymidine (T) in the DNA sequence being considered equal to uracil (U) in RNA sequences.

Additionally or alternatively, amino acid sequence similarity or identity or homology can be determined using the BlastP program (Altschul *et al.,* Nucl. Acids Res. 25, 3389-3402, incorporated herein by reference) and available at NCBI. The following references (each incorporated herein by reference) provide algorithms for comparing the relative identity or homology of amino acid residues of two proteins, and additionally or alternatively with respect to the foregoing, the teachings in these references can be used for determining percent homology or identity: Needleman SB and Wunsch CD, "A general method applicable to the search for similarities in the amino acid sequences of two proteins," J. Mol. Biol. 48:444-453 (1970); Smith TF and Waterman MS, "Comparison of Bio-sequences," Advances in Applied Mathematics 2:482-489 (1981); Smith TF, Waterman MS and Sadler JR, "Statistical characterization of nucleic acid sequence functional domains," Nucleic Acids Res., 11:2205-2220 (1983); Feng DF and Dolittle RF, "Progressive sequence alignment as a prerequisite to correct phylogenetic trees," J. of Molec. Evol., 25:351-360 (1987); Higgins DG and Sharp PM, "Fast and sensitive multiple sequence alignment on a microcomputer," CABIOS, 5: 151-153 (1989); Thompson JD, Higgins DG and Gibson TJ, "ClusterW: improving the sensitivity of progressive multiple sequence alignment through sequence weighing, positions-specific gap penalties and weight matrix choice, Nucleic Acid Res., 22:4673-480 (1994); and, Devereux J, Haeberlie P and Smithies O, "A comprehensive set of sequence analysis program for the VAX," Nucl. Acids Res., 12: 387-395 (1984).

In this fashion, by comprehending nucleic acid molecules and polypeptides having such homology to the particular sequences disclosed, it is envisioned that the invention encompasses human and other homologues to the disclosed sequences, within the herein terms. Identification and/or isolation of corresponding human sequences can be any suitable method, for instance, by analysis of hybridization of herein defined genes (such as genes identified herein and/or identified by inventive methods herein) or suitable portions thereof, e.g., primers/probes derived from herein defined genes; for instance, in PCR amplification of portions of the human genome by such primers/probes and/or labeled hybridization analysis of herein defined genes or portions thereof to portions of the human genome (*see also* discussions *infra,* e.g., concerning PCR, hybridization, *inter alia*).

Furthermore, by comprehending proteins having homology to the gene products of genes identified herein (e.g., 405, 608, 274) as well as of genes identified by the methods disclosed herein, the invention comprehends proteins which are "functional" proteins derived from gene products identified herein, as well as from gene products of genes identified by the methods disclosed herein; e.g., truncated forms of proteins identified herein or expressed by genes identified by the methods disclosed herein.

As to uses, the inventive genes and expression products as well as genes identified by the herein disclosed methods and expression products thereof (including "functional" variations of such expression products, and ergo truncated portions of herein defined genes such as portions of herein defined genes which encode a functional porton of an expression product) are useful in treating, preventing or controlling or diagnosing or observing or studying osteoporosis or processes thereof or mechanical stress conditions or absence or reduced mechanical stress conditions. They may aid in bone density. They may be useful for diagnostic purposes. They may be used for determining predisposition to high or low bone density or for determining gene association or other factors associated with high bone mass or low bone mass.

For instance, 608 expression causes cells to differentiate into osteoblasts and chondrocytes. The expression product of 608, or if cells or vectors expressing 608 may cause cells to selectively differentiate and thereby increase or alter bone density. Detecting levels of 608 mRNA or expression and comparing it to "normal" non-osteopathic levels may allow one to detect who may be at risk for osteoporosis or lower levels of osteoblasts and chondrocytes.

405 expression impacts upon bone density by being characteristic for osteogenic and chondrogenic cells in their differentiation predeeding matrix calcification. The expression product of 405 or cells or vectors expressing it may cause cells to differentiate into osteogenic and chondrogenic cells and thereby increase matrix calcification and bone density. One may detect a risk of low bone density or low matrix calcification or osteoporosis by determining levels of expression of 405 or of mRNA and compare it to "normal" levels.

274 is implicated in lymphoid precursors in bone marrow. Under expression may lead to less lymphoid cells and bones that are more susceptible to environmental factors or other than genetic factors of osteoporosis, e.g., cancer causes of osteoporosis. One may detect a risk of predisposition of bones towards susceptibility to environmental factors, or less lymphoid cells, or osteopososis by determining levels of expression of 405 or of mRNA and comparing it to "normal" levels.

Further, genes which were upregulated and identified by the method of the present invention are of interest. That which may inhibit these genes and/or the expression products therefrom or portions thereof, e.g., antibodies or functional portions thereof or other compounds which bind thereto, may be useful in preventing, controlling or treating osteoporosis or factors leading thereto or causing osteoporosis or other conditions involving mechanical stress or a lack thereof, and the genes may be targets for anti-osteoporosis treatment or therapy, as well as for study of osteoporosis or factors leading thereto or causes thereof, e.g., determining predisposition to high or low bone density or for determining gene association or other factors associated with high bone mass or low bone mass.

Among these, three identified upregulated RGD-containing proteins, ADAMTS-1 and complement 3 itself (potential prevention of osteoclast attraction) and two proteins of the SARP family (secreted apoptosis related proteins) as potential modifiers of programmed cell death in bone formation were identified.

Similarly, genes which were downregulated and identified by the method of the present invention are interesting. These genes and/or the expression products therefrom and/or a functional portion thereof may be useful in preventing, controlling or treating osteoporosis or factors leading thereto or causing osteoporosis, or other conditions involving mechanical stress or a lack thereof, and the genes may be targets for anti-osteoporosis treatment or therapy, as well as for study of osteoporosis or factors leading thereto or causes thereof, e.g., determining predisposition to high or low bone density or for determining gene association or other factors associated with high bone mass or low bone mass.

Accordingly, in an aspect, the invention provides a method for determining risk of developing osteoporosis or low or high bone density or other factors causing or contributing to osteporosis or other conditions involving mechanical stress or a lack thereof, based upon absence or decrease from normal cells of mRNA or protein from a gene shown to be down regulated by the inventive methods in a mammal comprising:
(a) determining the level or status of mRNA in cells, e.g., bone cells (for instance, osteoblasts and/or osteoclasts) of said mammal; and/or
(b) determining the level or status of corresponding protein in cells of said mammal; and
(c) comparing said level or status of mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein, and from the comparing determining an absence or decrease from normal cells of mRNA or protein and thus risk.

In another aspect, the invention provides a method for determining risk of developing osteoporosis or low or high bone density or other factors causing or contributing to osteporosis or other conditions involving mechanical stress or a lack thereof, based upon presence or increase from normal cells of mRNA or protein from a gene shown to be upregulated by the inventive methods in a mammal comprising:
(a) determining the level or status of mRNA in cells, e.g., bone cells (for instance, osteoblasts and/or osteoclasts) of said mammal; and/or
(b) determining the level or status of corresponding protein in cells of said mammal; and
(c) comparing said level or status of mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein, and from the comparing determining a presence or increase from normal cells of mRNA or protein and thus risk.

As mentioned, 608 expression causes cells to differentiate into osteoblasts and chondrocytes. Thus, in a further aspect, the invention provides a method for determining risk of developing osteoporosis or low or high bone density or other factors causing or contributing to osteporosis or lower levels of osteoblasts and chondrocytes or other conditions involving mechanical stress or a lack thereof, based upon absence or decrease from normal cells of mRNA or protein from 608 comprising:
(a) determining the level or status of mRNA in cells, e.g., bone cells (for instance, osteoblasts and/or osteoclasts) of said mammal; and/or
(b) determining the level or status of corresponding protein in cells of said mammal; and
(c) comparing said level or status of mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein, and from the comparing determining a presence or increase from normal cells of mRNA or protein and thus risk.

As discussed herein, 405 expression impacts upon bone density by being characteristic for osteogenic and chondrogenic cells in their differentiation predeeding matrix calcification. Accordingly, in a still further aspect, the invention provides a method for determining risk of developing osteoporosis or low or high bone density or other factors causing or contributing to osteporosis or of imbalance as to osteogenic and chondrogenic cells or other conditions involving mechanical stress or a lack thereof, based upon absence or decrease from normal cells or presence or increase from normal cells, e.g., absence or decrease from normal cells of mRNA or protein from 405 in a mammal comprising:
(a) determining the level or status of mRNA in cells, e.g., bone cells (for instance, osteoblasts and/or osteoclasts) of said mammal; and/or
(b) determining the level or status of corresponding protein in cells of said mammal; and
(c) comparing said level or status of mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein, and from the comparing determining an absence or decrease from normal cells of mRNA or protein and thus risk.

Likewise, as discussed herein, 274 is implicated in lymphoid precursors in bone marrow.
Therefore, in yet another aspect, the invention provides a method for determining risk of developing osteoporosis or low or high bone density or other factors causing or contributing to osteporosis or of being susceptible to environmental factors or other than genetic factors of osteoporosis, e.g., cancer causes of osteoporosis or of predisposition of bones towards susceptibility to environmental factors, or less lymphoid cells, or osteopososis, or other conditions involving mechanical stress or a lack thereof, based upon presence or increase from normal cells or absence or decrease from normal cells, e.g., absence or decrease from normal cells of mRNA or protein from 274 in a mammal comprising:
(a) determining the level or status of mRNA in cells, e.g., bone cells (for instance, osteoblasts and/or osteoclasts) of said mammal; and/or
(b) determining the level or status of corresponding protein in cells of said mammal; and
(c) comparing said level or status of mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein, and from the comparing determining an absence or decrease from normal cells of mRNA or protein and thus risk.

The foregoing methods, in still further aspects of the invention, can be employed in inventive methods for testing a medicament for or a gene therapy approach to osteoporosis or bone density or other factors causing or contributing to osteporosis or symptoms thereof or other conditions involving mechanical stress or a lack thereof, based on absence or decrease from normal cells or presence or increase from normal cells of mRNA or protein of identified genes additionally comprising: (a') administering the medicament or the gene therapy; and from the comparing determining an absence or decrease from normal cells or presence or increase from normal cells of the relevant mRNA or protein and thus efficacy of the medicament or the gene therapy.

Similarly, in still further aspects, the invention provides methods for treating, preventing or controlling osteporosis or other conditions involving mechanical stress or a lack thereof, comprising administering a medicament or treatment for osteoporosis or a cause thereof or a symptom thereof, including the foregoing detection methods. For instance, from the comparing one determines an absence or decrease from normal cells or presence or increase from normal cells of particular mRNA or protein and thus risk and administers a the medicament or treatment.

The cells in the inventive methods can be *in vitro* or *in vivo* or from any suitable mammal, e.g., a human, a domesticated animal, for instance a companion animal or livestock, or a laboratory animal, such as a rat, mouse or the like; and, the cells can be from any stage of the mammal's development, such as embryonic, mature or adult, immature or child, newborn, or elderly, and the like.

It is noted that as to CMF608, the inventors did not see any differences in its expression between normal and ovariectomized rats suggesting it may not necessarily *per se* be a marker for bone intensity. Similarly, for CMF405 there was no change in expression after ovariectomy. In addition, expression in a few non-bone tissues suggests it may not necessarily *per se* be a marker.

Thus, inventive methods can additionally comprise using the steps in conjunction with another test method akin to those described herein, e.g., having a same or similar preamble recitation and comprising:
(d) determining the level or status of a second gene mRNA in bone cells of said mammal; and/or
(e) determining the level or status of protein expressed by a second gene product in bone cells of said mammal; and
(f) comparing said level or status of that mRNA and/or protein with the corresponding level in normal cells; wherein the term "level" denotes the amount of mRNA or protein produced; and, the term "status" includes that the gene, mRNA, protein or a transcription control element, including a promoter/enhancer sequence, may bear a mutation, deletion or any other modifications which would affect the overall activity of the gene when compared to the wild-type normal gene product, including post-translational modifications of the protein.

The absence or decrease or presence or increase detected may be correlated to risk. Thus, the second gene can be identified by methods of the invention. Or, alternatively or additionally, the second gene and/or the additional steps can be determined in accordance with other methods, e.g., as in U.S. Patents Nos. 5,834,200 and/or 5,691,153.

Likwise, it is within the invention that the inventive genes or genes identified by inventive methods herein or portions thereof can be the subject of other or analogous methods, such as a method for determining predisposition to high or low bone density comprising detecting the under or over expression of the gene or abnormalities in a receptor for a gene product or polymorphysim; *see, e.g.,* U.S. Patent Nos. 5,834,200 and 5,691,153; for instance, the inventive genes or genes identified by inventive methods herein can be used in methods analogous to those of U.S. Patent Nos. 5,834,200 and 5,691,153.

In the inventive methods steps (a) and/or (b) and optionally (d) and/or (e) are carried out *in vivo* and/or steps (a) and/or (b) and optionally (d) and/or (e) are carried out *in vitro.*

The determination in step (a) and optionally in step (d) can be effected by employing
(i) a nucleic acid sequence corresponding to at least a part of the gene encoding at least part of the protein and optionally a second nucleic acid sequence corresponding to at least a part of the second gene encoding at least part of the second protein;
(ii) a nucleic acid sequence complementary to the nucleic acid sequence(s) of (i); or
(iii) a primer or a primer pair hybridizing to the nucleic acid sequence(s) of (i) or (ii).
(Note also the discussion herein, e.g., infra, concerning primers/probes and PCR and hybridization.)

The determination in step (b) and optionally of step (e) can be effected by employing an antibody or a fragment thereof that specifically binds to the protein and optionally by employing a second antibody or a fragment thereof which specifically binds to the second protein. (Note also the discussion herein, e.g., infra, concerning antibodies and methods for making and uses thereof.)

The medicament or treatment can be any conventional medicament or treatment for osteoporosis. Alternatively or additionally, the medicament or treatment can be the particular protein of the gene detected in the inventive methods, or that which inhibits that protein, e.g., binds to it. Similarly, additionally or alternatively, the medicament or treatment can be a vector which expresses the protein of the gene detected in the inventive methods or that which inhibits expression of that gene; again, for instance, that which can bind to it and/or otherwise prevents its transcription or translation. The selection of administering a protein or that which expresses it, or of administering that which inhibits the protein or the gene expression, can be done without undue experimentation, e.g., based on down regulation or up regulation as determined by inventive methods (e.g., in the osteoporosis model).

In the practice of the invention, one can employ general methods in molecular biology: Standard molecular biology techniques known in the art and not specifically described are generally followed as in Sambrook et al., Molecular Cloning: A Laboratory Manual, Cold Spring Harbor Laboratory, New York (1989, 1992), and in Ausubel et al., Current Protocols in Molecular Biology, John Wiley and Sons, Baltimore, Maryland (1989).

To determine the absence or decrease from normal cells or presence or increase from normal cells of a nucleic acid molecule, or to amplify it, e.g., in using probes or primers described herein or derived from nucleic acid molecules disclosed herein, the polymerase chain reaction (PCR) may be used and is conveniently carried out generally as in PCR Protocols: A Guide To Methods And Applications, Academic Press, San Diego, CA (1990). Reactions and manipulations involving other nucleic acid techniques, unless stated otherwise, are performed as generally described in Sambrook et al., 1989, Molecular Cloning: A Laboratory Manual, Cold Spring Harbor Laboratory Press, and methodology as set forth in United States patents 4,666,828; 4,683,202; 4,801,531; 5,192,659 and 5,272,057 and incorporated herein by reference. *In-situ* (In-cell) PCR in combination with Flow Cytometry can be used for detection of cells containing specific DNA and mRNA sequences (Testoni et al, 1996, Blood 87:3822.)

In PCR, as well as in hybridization, it is preferred that the primers (or probes) bind specifically to the gene of interest, e.g., an inventive gene disclosed herein such as 608, 405 or 274, or a gene identified by methods disclosed herein, or a corresponding human homolog being detected by primer(s) or probe(s) derived from a herein defined gene. One way to ensure this is to select primers from the gene sequence that are not generally found in other known sequences.

The invention accordingly in yet a further aspect provides an isolated nucleic acid molecule, e.g., DNA comprising a sequence encoding a herein defined gene or encoding a herein defined a polypeptide (e.g., an expression product of a herein defined gene) comprising at least about 12 nucleotides in length, for instance, at least about 15, about 18, about 21, about 24 or about 27 nucleotides in length, such as at least about 30, about 33, about 36, about 39 or about 42 nucleotides in length, for example, a nucleic acid molecule of at least about 12 nucleotides in length such as about 12 to about 30, about 12 to about 50 or about 12 to about 60, or about 12 to about 75 or about 12 to about 100 or more nucleotides in length. Nucleic acid molecules of these lengths may be useful in hybridization; and, the invention further comprehends vectors or plasmids containing and/or expressing such a nucleic acid molecule, as well as uses of such nucleic acid molecules, e.g., for expression thereof either *in vitro* or *in vivo,* or for amplifying or detecting a herein defined gene or a homolog thereof, e.g., a human homolog, in a sample, for instance by a polymerase chain reaction.

A probe or primer can be any stretch of at least 8, preferably at least 10, more preferably at least 12, 13, 14, or 15, such as at least 20, e.g., at least 23 or 25, for instance at least 27 or 30 nucleotides in a herein defined gene which are unique thereto. As to PCR or hybridization primers or probes and optimal lengths therefor, reference is also made to Kajimura et al., GATA 7(4):71-79 (1990). The invention will thus be understood to provide oligonucleotides, such as, pairs of oligonucleotides, for use as primers for the *in vitro* amplification of DNA samples and fragments thereof, or for use in expressing a portion of DNA, either *in vitro* or *in vivo.* The oligonucleotides preferably specifically hybridize to sequences flanking a nucleic acid to be amplified, wherein the oligonucleotides hybridize to different and opposite strands of the double-stranded DNA target. The oligonucleotides of the invention are preferably derived from the nucleic acid molecules, e.g., a herein defined gene, and teachings herein. As used in the practice of this invention, the term "derived from" is intended to encompass the development of such oligonucleotides from the nucleic acid molecules and herein defined gene(s) and teachings disclosed herein, from which a multiplicity of alternative and variant oligonucleotides can be prepared.

The term "specific hybridization" will be understood to mean that the nucleic acid probes of the invention are capable of stable, double-stranded hybridization to gene-derived DNA or RNA under conditions of high stringency, as the term "high stringency" would be understood by those with skill in the art (see, for example, Sambrook et al., 1989, Molecular Cloning: A Laboratory Manual, Cold Spring Harbor Laboratory Press, Cold Spring Harbor, N.Y. and Hames and Higgins, eds., 1985, Nucleic Acid Hybridization, IRL Press, Oxford, U.K.). Hybridization will be understood to be accomplished using well-established techniques, including but not limited to Southern blot hybridization, Northern blot hybridization, *in situ* hybridization and, most preferably, Southern hybridization to PCR-amplified DNA fragments.

The nucleic acid hybridization probe of the invention may be obtained by use of the polymerase chain reaction (PCR) procedure, using appropriate pairs of PCR oligonucleotide primers as provided herein or derived from the gene sequence(s) provided herein. See U.S. Pat. Nos. 4,683,195 to Mullis et al. and 4,683,202 to Mullis. The invention in a still further aspect provides oligonucleotides for *in vitro* amplification using any of a variety of amplification protocols known in the art. Preferably, the invention provides oligonucleotides for performing polymerase chain reaction (PCR). See U.S. Pat. Nos. 4,683,195 to Mullis et al. and 4,683,202 to Mullis.

The invention will thus be understood to provide oligonucleotides, specifically, pairs of oligonucleotides, for use as primers for the *in vitro* amplification of genes as disclosed herein, e.g., of DNA samples and fragments thereof. In the practice of this invention, the pairs of oligonucleotides herein provided will be understood to comprise two oligonucleotides, comprising from about 8 to about 30 nucleotide residues apiece, said oligonucleotides specifically hybridizing to sequences flanking a nucleic acid to be amplified, wherein the oligonucleotides hybridize to different and opposite strands of the DNA target. The oligonucleotides of the invention are preferably derived from the nucleic acid primers discussed below or from the gene(s) disclosed herein. As used in the practice of this invention, the term "derived from" is intended to encompass the development of such oligonucleotides from the nucleic acid sequence of the gene(s) or the primers herein disclosed, from which a multiplicity of alternative and variant oligonucleotides can be prepared. In particular, the invention provides oligonucleotides having a sequence that is substantially complementary to the corresponding sequence of the nucleic acid hybridization probe. As used herein, the term "substantially corresponding to" is intended to encompass oligonucleotides comprising sequence additions, deletions and mismatches, wherein certain nucleotide residues of the oligonucleotide sequence are not optimally complementary (e.g., A-C or G-T) or are non-complementary (e.g., A-G or T-C) to the corresponding sequence of the nucleic acid hybridization probe, provided that such oligonucleotides retain the capacity to specifically amplify the gene(s).

Nucleic acids, e.g., 405, 608 or 274, and oligonucleotides therefrom, such as primers disclosed herein and derivable from the sequences of the present invention (e.g., portions of each disclosed gene which are about 8 to 30 or more nucleotides in length and bind with sufficient specificity to the gene are useful as diagnostic tools for detecting the existence of a osteoporosis or conditions or factors of osteoporosis. Such diagnostic or detection reagents comprise nucleic acid hybridization probes of the invention and encompass paired oligonucleotide PCR primers, as described above.

Methods provided by the invention include blot hybridization, *in situ* hybridization and *in vitro* amplification techniques for detecting osteoporosis or conditions or factors of osteoporosis in a sample such as a biological sample. Appropriate biological samples advantageously screened using the methods described herein include blood, serum, saliva and other body fluids, and other potential sources of infection.

In the detection methods of the invention, production of a specific DNA fragment produced by *in vitro* amplification of a template DNA sample is detected by agarose gel electrophoresis, ethidium bromide staining and ultraviolet transillumination of ethidium bromide stained gels, performed using conventional techniques (Sambrook et al., *supra*), or detection by sequence detection systems using fluorogenic or other labeled probes that rely on automatic or automated detection instrumentation. In instances where a greater degree of specificity is required, hybridization of such agarose gels probed with a detectably-labeled nucleic acid hybridization probe of the invention is performed using standard techniques (Sambrook et al., *supra*)*.* In each of these embodiments of the methods of the invention, a sufficient amount of a specific PCR-amplified DNA fragment is produced to be readily detected. For the purposes of this invention, the term "a sufficient amount of a specific PCR-amplified DNA fragment" is defined as that amount required to be detected, either by visualization of ethidium bromide-stained agarose gels or autoradiographic or other development of a blot hybridized with a detectably-labeled probe.

It will be understood that a sufficient quantity of a specific PCR amplified DNA fragment is prepared in PCR amplification reactions by performing a number of cycles required to produce said sufficient amount of the specific DNA fragment. The number of cycles in each PCR required to produce said sufficient amount of a specific DNA fragment will be understood to depend on the oligonucleotide primers, buffers, salts and other reaction components, the amount of template DNA and the PCR cycling times and temperatures. It will also be understood that the optimization of these parameters are within the skill of the worker of ordinary skill to achieve with no more than routine experimentation.

Detectably-labeled probes as provided by the invention are labeled with biotin, a radioisotope (including ³H, ¹⁴C, ³⁵S and ³²P), a fluorescent label (including fluorescein isothiocyanate), and an antigenic label. The detectable label is incorporated into the probe during synthetic preparation of the probe, whereby the probe is alternatively end-labeled or labeled by the incorporation of labeled nucleotides into the synthesized probe.

The invention also provides a PCR-based method for preparing a nucleic acid hybridization probe of the invention. In these embodiments, template DNA comprises a recombinant genetic construct of the invention. A detectably-labeled nucleic acid hybridization probe is prepared by performing PCR amplification using a pair of oligonucleotide primers specific for sequences flanking the position of the nucleic acid insert. Detectable label is incorporated into the nucleic acid hybridization probe by direct end-labeling of PCR primers or incorporation of detectably-labeled nucleotide triphosphates into the probe nucleic acid.

PCR comprising the methods of the invention is performed in a reaction mixture comprising an amount, typically between <10 ng-200 ng template nucleic acid; 50-100 pmoles each oligonucleotide primer; 1-1.25 mM each deoxynucleotide triphosphate; a buffer solution appropriate for the polymerase used to catalyze the amplification reaction; and 0.5-2 Units of a polymerase, most preferably a thermostable polymerase (e.g., Taq polymerase or Tth polymerase).

The invention thus provides in further aspects diagnostic assays for the specific detection of osteoporosis or genes associated therewith. These diagnostic assays include nucleic acid hybridization assays, using the nucleic acids of the invention or specifically-hybridizing fragments thereof, for sensitive detection of fungal genomic DNA and/or RNA. Such assays include various blot assays, such as Southern blots, Northern blots, dot blots, slot blots and the like, as well as *in vitro* amplification assays, such as the polymerase chain reaction assay (PCR), reverse transcription-polymerase chain reaction assay (RT-PCR), ligase chain reaction assay (LCR), and others known to those skilled in the art. Specific restriction endonuclease digestion of diagnostic fragments detected using any of the methods of the invention, analogous to restriction fragment linked polymorphism assays (RFLP) are also within the scope of this invention.

These PCR techniques can be used in conjunction with or in the practice of other methods disclosed herein, or other conditions associated with or correlated to mechanical stress or a lack thereof.

Accordingly, the invention relates to compositions and methods for detecting and/or diagnosing osteoporosis or conditions or factors associated therewith, including genetic factors associated therewith.

Similarly, in the practice of the invention, e.g., protein detection, general methods in immunology may be employed. Standard methods in immunology known in the art and not specifically described are generally followed as in Stites et al.(eds), Basic and Clinical Immunology (8th Edition), Appleton & Lange, Norwalk, CT (1994) and Mishell and Shiigi (eds), Selected Methods in Cellular Immunology, W.H. Freeman and Co., New York (1980). Immunoassays such as RIA and ELISA can be employed to assess a specimen for the presence of specific proteins or other compounds of interest where appropriate as known in the art. Both polyclonal and monoclonal antibodies can be used in the assays. Available immunoassays are extensively described in the patent and scientific literature. See, for example, United States patents 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521 as well as Sambrook et al, Molecular Cloning: A Laboratory Manual, Cold Springs Harbor, New York, 1989

Antibodies may be used in various aspects of the invention, e.g., in detection or treatment or prevention methods. Antibodies may be either monoclonal, polyclonal or recombinant to be used in the immunoassays or other methods of analysis necessary for the practice of the invention. Conveniently, the antibodies may be prepared against the immunogen or portion thereof for example a synthetic peptide based on the sequence, or prepared recombinantly by cloning techniques or the natural gene product and/or portions thereof may be isolated and used as the immunogen. The genes are identified as set forth in the present invention and the gene product identified. Immunogens can be used to produce antibodies by standard antibody production technology well known to those skilled in the art as described generally in Harlow and Lane, Antibodies: A Laboratory Manual, Cold Spring Harbor Laboratory, Cold Spring Harbor, NY, 1988 and Borrebaeck, Antibody Engineering - A Practical Guide, W.H. Freeman and Co., 1992. Antibody fragments may also be prepared from the antibodies and include Fab, F(abl)2, and Fv by methods known to those skilled in the art.

For producing polyclonal antibodies a host, such as a rabbit or goat, is immunized with the immunogen or immunogen fragment, generally with an adjuvant and, if necessary, coupled to a carrier; antibodies to the immunogen are collected from the sera. Further, the polyclonal antibody can be absorbed such that it is monospecific. That is, the sera can be absorbed against related immunogens so that no cross-reactive antibodies remain in the sera rendering it monospecific.

For producing monoclonal antibodies the technique involves hyperimmunization of an appropriate donor with the immunogen, generally a mouse, and isolation of splenic antibody producing cells. These cells are fused to a cell having immortality, such as a myeloma cell, to provide a fused cell hybrid which has immortality and secretes the required antibody. The cells are then cultured, in bulk, and the monoclonal antibodies harvested from the culture media for use.

For producing recombinant antibody (see generally Huston et al, 1991; Johnson and Bird, 1991; Mernaugh and Mernaugh, 1995), messenger RNAs from antibody producing Blymphocytes of animals, or hybridoma are reverse -transcribed to obtain complimentary DNAs (cDNAs). Antibody cDNA, which can be full or partial length, is amplified and cloned into a phage or a plasmid. The cDNA can be a partial length of heavy and light chain cDNA, separated or connected by a linker. The antibody, or antibody fragment, is expressed using a suitable expression system to obtain recombinant antibody. Antibody cDNA can also be obtained by screening pertinent expression libraries.

The antibody can be bound to a solid support substrate or conjugated with a detectable moiety or be both bound and conjugated as is well known in the art. (For a general discussion of conjugation of fluorescent or enzymatic moieties see Johnstone & Thorpe, Immunochemistry in Practice, Blackwell Scientific Publications, Oxford, 1982.) The binding of antibodies to a solid support substrate is also well known in the art. (see for a general discussion Harlow & Lane Antibodies: A Laboratory Manual, Cold Spring Harbor Laboratory Publications, New York, 1988 and Borrebaeck, Antibody Engineering - A Practical Guide, W.H. Freeman and Co., 1992) The detectable moieties contemplated with the present invention can include, but are not limited to, fluorescent, metallic, enzymatic and radioactive markers such as biotin, gold, ferritin, alkaline phosphatase, Beta-galactosidase, peroxidase, urease, fluorescein, rhodamine, tritium, ¹³C and iodination.

Antibodies can also be used as an active agent in a therapeutic composition and such antibodies can be humanized, for instance, to enhance their effects. *See, e.g.,* Huls et al., "A recombinant, fully human monoclonal antibody with antitumor activity constructed from phage-displayed antibody fragments," Nature Biotechnology Vol. 17, No. 3, March 1999, and documents cited therein, incorporated herein by reference.

Accordingly, antibodies from expression products of genes identified herein or by inventive methods disclosed herein are useful in immunodiagnostics, as well as in drugs or other commercial uses such as in research.

Simply, the expression product from the gene or portions thereof can be useful for generating antibodies such as monoclonal or polyclonal antibodies which are useful for diagnostic purposes or to block activity of expression products or portions thereof or of genes or a portion thereof, e.g., as a therapeutic. Monoclonal antibodies are immunoglobulins produced by hybridoma cells. A monoclonal antibody reacts with a single antigenic determinant and provides greater specificity than a conventional, serum-derived antibody. Furthermore, screening a large number of monoclonal antibodies makes it possible to select an individual antibody with desired specificity, avidity and isotype. Hybridoma cell lines provide a constant, inexpensive source of chemically identical antibodies and preparations of such antibodies can be easily standardized. Methods for producing monoclonal antibodies are well known to those of ordinary skill in the art, e.g., Koprowski, H. et al., U.S. Pat. No. 4,196,265, issued Apr. 1, 1989, incorporated herein by reference, and other documents cited herein, e.g., *supra.*

Uses of monoclonal antibodies are known. One such use is in David, G. and Greene, H., U.S. Pat. No. 4,376,110, issued Mar. 8, 1983, incorporated herein by reference; *see also* documents cited herein, e.g., *supra.* Monoclonal antibodies have also been used to recover materials by immunoadsorption chromatography, *see, e.g.* Milstein, C., 1980, Scientific American 243:66, 70, incorporated herein by reference; and documents cited herein, such as *supra.* Thus, products expressed from genes identified herein or by methods herein or portions thereof are useful in therapeutics, immunoadsorption chromatography, as well as for generating antibodies for diagnostic or detection purposes. Furthermore, the expression products can be used in assays for detecting the presence of antibodies. For instance, the antibodies or expressed products can be used in assays analogous to those disclosed in U.S. Patents Nos. 5,591,645, 4,861,711, 5,861,319, 5,858,804, and 5,863,720, as well as in WO 86/04683, EP 154 749, WO 86/03839, and EP 186 799. Antibodies in the practice of the invention can include fragments thereof which are functional, e.g., a fragment that at least statistically significantly retains some (for instance a majority) or all of binding as compared with the entire antibody; for instance, antibodies comprehends a fragment comprising a binding domain.

Protein purification, including recombinant protein purification in the practice of the invention can be in accordance with or analogous to Marshak et al, "Strategies for Protein Purification and Characterization. A laboratory course manual. " CSHL Press, 1996.

With respect to transgenic and knockout methods, the present invention comprehends transgenic gene and polymorphic gene animal and cellular (cell lines) models as well as for knockout models for the genes identified in the present invention. These models are constructed using standard methods known in the art and as set forth in United States Patents 5,487,992, 5,464,764, 5,387,742, 5,360,735, 5,347,075, 5,298,422, 5,288,846, 5,221,778, 5,175,385, 5,175,384,5,175,383, 4,736,866 as well as Burke and Olson (1991), Capecchi (1989), Davies et al. (1992), Dickinson et al. (1993), Duff and Lincoln (1995), Huxley et al. (1991), Jakobovits et al. (1993), Lamb et al. (1993), Pearson and Choi (1993), Rothstein (1991), Schedl et al. (1993), Strauss et al. (1993). Further, patent applications WO 94/23049, WO 93/14200, WO 94/06908, WO 94/28123 also provide information.

Thus, for instance, the inventive methods of the invention can be used to determine a gene of interest with respect to a physiological or disease state, e.g., osteoporosis or other conditions caused by mechanical stress for instance 608 or 405 or 274 and using the information herein and in the art (such as documents cited herein), knockout or trangenic animals such as mice or rat, can be prepared, to generate animals prone to the physiological or disease state, osteoporosis or other conditions caused by mechanical stress, to thereby test treatments or medicaments therefor; or, to test theories and thus advance research pertaining to the physiological or disease state, e.g., to test the functions of identified genes such as 405, 608 and 274, *inter alia.* Accordingly, from this disclosure and the knowledge in the art, no undue experimentation is needed to prepare knockout or transgenic animals, such as mice or rats or rodents; and, such animals have great value and utility.

Moreover, the genes of the present invention or a portion thereof, e.g., a portion thereof which expresses a protein which function the same as or analogously to the full length protein, or genes identified by the methods herein can be expressed recombinantly, e.g., in *E. coli* or in another vector or plasmid for either *in vivo* expression or *in vitro* expression. The methods for making and/or administering a vector or recombinant or plasmid for expression of gene products of genes of the invention or identified by the invention or a portion thereof either *in vivo* or *in vitro* can be any desired method, e.g., a method which is by or analogous to the methods disclosed in: U.S. Patent Nos. 4,603,112,4,769,330, 5,174,993, 5,505,941, 5,338,683, 5,494,807, 4,722,848, WO 94/16716, WO 96/39491, Paoletti, "Applications of pox virus vectors to vaccination: An update," PNAS USA 93:11349-11353, October 1996, Moss, "Genetically engineered poxviruses for recombinant gene expression, vaccination, and safety," PNAS USA 93:11341-11348, October 1996, Smith et al., U.S. Patent No. 4,745,051 (recombinant baculovirus), Richardson, C.D. (Editor), Methods in Molecular Biology 39, "Baculovirus Expression Protocols" (1995 Humana Press Inc.), Smith et al., "Production of Huma Beta Interferon in Insect Cells Infected with a Baculovirus Expression Vector," Molecular and Cellular Biology, Dec., 1983, Vol. 3, No. 12, p. 2156-2165; Pennock et al., "Strong and Regulated Expression of *Escherichia coli* B-Galactosidase in Infect Cells with a Baculovirus vector," Molecular and Cellular Biology Mar. 1984, Vol. 4, No. 3, p. 399-406; EPA0 370 573, U.S. application Serial No. 920,197, filed October 16, 1986, EP Patent publication No. 265785, U.S. Patent No. 4,769,331 (recombinant herpesvirus), Roizman, "The function of herpes simplex virus genes: A primer for genetic engineering of novel vectors," PNAS USA 93:11307-11312, October 1996, Andreansky et al., "The application of genetically engineered herpes simplex viruses to the treatment of experimental brain tumors," PNAS USA 93:11313-11318, October 1996, Robertson et al. "Epstein-Barr virus vectors for gene delivery to B lymphocytes," PNAS USA 93:11334-11340, October 1996, Frolov et al., "Alphavirus-based expression vectors: Strategies and applications," PNAS USA 93:11371-11377, October 1996, Kitson et al., J. Virol. 65, 3068-3075, 1991; U.S. Patent Nos. 5,591,439, 5,552,143 (recombinant adenovirus), Grunhaus et al., 1992, "Adenovirus as cloning vectors," Seminars in Virology (Vol. 3) p. 237-52, 1993, Ballay et al. EMBO Journal, vol. 4, p. 3861-65, Graham, Tibtech 8, 85-87, April, 1990, Prevec et al., J. Gen Virol. 70,429-434, PCT WO91/11525, Felgner et al. (1994), J. Biol. Chem. 269,2550-2561, Science, 259:1745-49,1993 and McClements et al., "Immunization with DNA vaccines encoding glycoprotein D or glycoprotein B, alone or in combination, induces protective immunity in animal models of herpes simplex virus-2 disease," PNAS USA 93:11414-11420, October 1996, and U.S. Patents Nos 5,591,639, 5,589,466, and 5,580,859 relating to DNA expression vectors, *inter alia.* See also WO 98/33510; Ju et al., Diabetologia, 41:736-739, 1998 (lentiviral expression system); Sanford et al., U.S. Patent No. 4,945,050 (method for transporting substances into living cells and tissues and apparatus therefor); Fischbach et al. (Intracel), WO 90/01543 (method for the genetic expression of heterologous proteins by cells transfected); Robinson et al., seminars in IMMUNOLOGY, vol. 9, pp.271-283 (1997) (DNA vaccines); Szoka et al., U.S. Patent No. 4,394,448 (method of inserting DNA into living cells); and McCormick et al., U.S. Patent No. 5,677,178 (use of cytopathic viruses for therapy and prophylaxis of neoplasia).

The expression product generated by vectors or recombinants in this invention optionally can also be isolated and/or purified from infected or transfected cells; for instance, to prepare compositions for administration to patients. However, in certain instances, it may be advantageous to not isolate and/or purify an expression product from a cell; for instance, when the cell or portions thereof enhance the effect of the polypeptide.

An inventive vector or recombinant expressing a gene identifed herein or from a method herein or a portion thereof can be administered in any suitable amount to achieve expression at a suitable dosage level, e.g., a dosage level analogous to the herein mentioned dosage levels (wherein the gene product is directly present). The inventive vector or recombinant can be administered to a patient or infected or transfected into cells in an amount of about at least 10³ pfu; more preferably about 10⁴ pfu to about 10¹⁰ pfu, e.g., about 10⁵ pfu to about 10⁹ pfu, for instance about 10⁶ pfu to about 10⁸ pfu. In plasmid compositions, the dosage should be a sufficient amount of plasmid to elicit a response analogous to compositions wherein gene product or a portion thereof is directly present; or to have expression analogous to dosages in such compositions; or to have expression analogous to expression obtained *in vivo* by recombinant compositions. For instance, suitable quantities of plasmid DNA in plasmid compositions can be 1 ug to 100 mg, preferably 0.1 to 10 mg, e.g., 500 micrograms, but lower levels such as 0.1 to 2 mg or preferably 1-10 ug may be employed. Documents cited herein regarding DNA plasmid vectors may be consulted for the skilled artisan to ascertain other suitable dosages for DNA plasmid vector compositions of the invention, without undue experimentation.

Compositions for administering vectors can be as in or analogous to such compositions in documents cited herein or as in or analogous to compositions herein described, e.g., pharmaceutical or therapeutic compositions and the like (e.g., *see infra*).

Thus, the invention comprehends *in vivo* gene expression which is sometimes termed "gene therapy". Gene therapy can refer to the transfer of genetic material (e.g DNA or RNA) of interest into a host to treat or prevent a genetic or acquired disease or condition phenotype. The particular gene that is to be used or which has been identified as the target gene is identified as set forth herein. The genetic material of interest encodes a product (e.g. a protein, polypeptide, peptide or functional RNA) whose production in vivo is desired. For example, the genetic material of interest can encode a hormone, receptor, enzyme, polypeptide or peptide of therapeutic value. For a review see, in general, the text "Gene Therapy" (Advances in Pharmacology 40, Academic Press, 1997).

Two basic approaches to gene therapy have evolved: (1) *ex vivo* and (2) *in vivo* gene therapy. In *ex vivo* gene therapy cells are removed from a patient, and while being cultured are treated *in vitro.* Generally, a functional replacement gene is introduced into the cell via an appropriate gene delivery vehicle/method (transfection, homologous recombination, etc.) and, an expression system as needed and then the modified cells are expanded in culture and returned to the host/patient. These genetically reimplanted cells have been shown to produce the transfected gene product *in situ.* In *in vivo* gene therapy, target cells are not removed from the subject rather the gene to be transferred is introduced into the cells of the recipient organism in situ, that is within the recipient. Alternatively, if the host gene is defective, the gene is repaired *in situ* (Culver, 19981. These genetically altered cells have been shown to produce the transfected gene product *in situ.*

The gene expression vehicle is capable of delivery/transfer of heterologous nucleic acid into a host cell. The expression vehicle may include elements to control targeting, expression and transcription of the nucleic acid in a cell selective manner as is known in the art. It should be noted that often the 5'UTR and/or 3'UTR of the gene may be replaced by the 5' UTR and/or 3'UTR of the expression vehicle. Therefore as used herein the expression vehicle may, as needed, not include the 5'LTTR and/or 3'UTR shown in sequences herein and only include the specific amino acid coding region.

The expression vehicle can include a promotor for controlling transcription of the heterologous material and can be either a constitutive or inducible promotor to allow selective transcription. Enhancers that may be required to obtain necessary transcription levels can optionally be included. Enhancers are generally any non-translated DNA sequence which works contiguously with the coding sequence (in cis) to change the basal transcription level dictated by the promoter. The expression vehicle can also include a selection gene as described herein.

Vectors can be introduced into cells or tissues by any one of a variety of known methods within the art. Such methods can be found generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Maryland (1989), Chang et al., Somatic Gene Therapy, CRC Press, Ann Arbor, MI (1995), Vega et al., Gene Targeting, CRC Press, Ann Arbor, MI (1995), Vectors: A Survey of Molecular Cloning Vectors and Their Uses, Butterworths, Boston MA (1988) and Gilboa et al (1986), as well as other documents cited herein (*see supra*) and include, for example, stable or transient transfection, lipofection, electroporation and infection with recombinant viral vectors. In addition, see United States patent 4,866,042 for vectors involving the central nervous system and also United States patents 5,464,764 and 5,487,992 for positive-negative selection methods.

Introduction of nucleic acids by infection offers several advantages over the other listed methods. Higher efficiency can be obtained due to their infectious nature. Moreover, viruses are very specialized and typically infect and propagate in specific cell types. Thus, their natural specificity can be used to target the vectors to specific cell types in vivo or within a tissue or mixed culture of cells. Viral vectors can also be modified with specific receptors or ligands to alter target specificity through receptor mediated events.

A specific example of DNA viral vector for introducing and expressing recombinant sequences is the adenovirus derived vector Adenop53TK. This vector expresses a herpes virus thymidine kinase (TK) gene for either positive or negative selection and an expression cassette for desired recombinant sequences. This vector can be used to infect cells that have an adenovirus receptor which includes most cancers of epithelial origin as well as others. This vector as well as others that exhibit similar desired functions,can be used to treat a mixed population of cells and can include, for example, an *in vitro* or *ex vivo* culture of cells, a tissue or a human subject.

Additional features can be added to the vector to ensure its safety and/or enhance its therapeutic efficacy. Such features include, for example, markers that can be used to negatively select against cells infected with the recombinant virus. An example of such a negative selection marker is the TK gene described above that confers sensitivity to the antibiotic gancyclovir. Negative selection is therefore a means by which infection can be controlled because it provides inducible suicide through the addition of antibiotic. Such protection ensures that if, for example, mutations arise that produce altered forms of the viral vector or recombinant sequence, cellular transformation will not occur. Features that limit expression to particular cell types can also be included. Such features include, for example, promoter and regulatory elements that are specific for the desired cell type.

In addition, recombinant viral vectors are useful for *in vivo* expression of a desired nucleic acid because they offer advantages such as lateral infection and targeting specificity. Lateral infection is inherent in the life cycle of, for example, retrovirus and is the process by which a single infected cell produces many progeny virions that bud off and infect neighboring cells. The result is that a large area becomes rapidly infected, most of which was not initially infected by the original viral particles. This is in contrast to vertical-type of infection in which the infectious agent spreads only through daughter progeny. Viral vectors can also be produced that are unable to spread laterally. This characteristic can be useful if the desired purpose is to introduce a specified gene into only a localized number of targeted cells.

As described above, viruses are very specialized infectious agents that have evolved, in many cases, to elude host def ense mechanisms. Typically, viruses infect and propagate in specific cell types. The targeting specificity of viral vectors utilizes its natural specificity to specifically target predetermined cell types and thereby introduce a recombinant gene into the infected cell. The vector to be used in the methods of the invention will depend on desired cell type to be targeted and will be known to those skilled in the art. For example, if breast cancer is to be treated then a vector specific for such epithelial cells would be used. Likewise, if diseases or pathological conditions of the hematopoietic system are to be treated, then a viral vector that is specific for blood cells and their precursors, preferably for the specific type of hematopoietic cell, would be used.

Retroviral vectors can be constructed to function either as infectious particles or to undergo only a single initial round of infection. In the former case, the genome of the virus is modified so that it maintains all the necessary genes, regulatory sequences and packaging signals to synthesize new viral proteins and RNA. Once these molecules are synthesized, the host cell packages the RNA into new viral particles which are capable of undergoing further rounds of infection. The vector's genome is also engineered to encode and express the desired recombinant gene. In the case of non-infectious viral vectors, the vector genome is usually mutated to destroy the viral packaging signal that is required to encapsulate the RNA into viral particles. Without such a signal, any particles that are formed will not contain a genome and therefore cannot proceed through 'subsequent rounds of infection. The specific type of vector will depend upon the intended application. The actual vectors are also known and readily available within the art or can be constructed by one skilled in the art using well-known methodology.

The recombinant vector can be administered in several ways. If viral vectors are used, for example, the procedure can take advantage of their target specificity and consequently, do not have to be administered locally at the diseased site. However, local administration can provide a quicker and more effective treatment, administration can also be performed by, for example, intravenous or subcutaneous injection into the subject. Injection of the viral vectors into a spinal fluid can also be used as a mode of administration, especially in the case of neurodegenerative diseases. Following injection, the viral vectors will circulate until they recognize host cells with the appropriate target specificity for infection.

An alternate mode of administration can be by direct inoculation locally at the site of the disease or pathological condition or by inoculation into the vascular system supplying the site with nutrients or into the spinal fluid. Local administration is advantageous because there is no dilution effect and, therefore, a smaller dose is required to achieve expression in a majority of the targeted cells. Additionally, local inoculation can alleviate the targeting requirement required with other forms of administration since a vector can be used that infects all cells in the inoculated area. If expression is desired in only a specific subset of cells within the inoculated area, then promoter and regulatory elements that are specific for the desired subset can be used to accomplish this goal. Such non-targeting vectors can be, for example, viral vectors, viral genome, plasmids, phagemids and the like. Transfection vehicles such as liposomes can also be used to introduce the non-viral vectors described above into recipient cells within the inoculated area. Such transfection vehicles are known by one skilled within the art.

Inventive vectors can comprise a herein defined gene, as well as a regulatory element operative linked thereto, e.g., a promoter, for expression; and, the regulatory element or promoter can be tissue or cell specific; for instance, the regulatory element or promoter can be for expression in a cell or precursor thereto employed in an inventive or herein-described or herein-cited test, e.g., the regulatory element or promoter can be for expression in a bone cell such as an osteoblast or an osteoclast or a precursor thereto.

Delivery of gene products (products from herein defined genes: genes identified herein or by inventive methods or portions thereof) and/or antibodies or portions thereof and/or agonists or antagonists (collectively or individually "therapeutics"), and compositions comprising the same, as well as of compositions comprising a vector expressing gene products, can be done without undue experimentation from this disclosure and the knowledge in the art.

The present invention provides compositions comprising an expression vector comprising a herein defined gene, or a portion thereof, e.g., which codes for a functional portion thereof, as well as therapeutics based on the genes identified herein, e.g., compositions comprising expression products or a functional portion thereof or antibodies thereto or a functional portion thereof and/or agonists or antagonists. (Thus, a herein defined gene can comprehend a portion thereof which expresses a functional portion of a full length expression product.). The therapeutics and vectors of the present invention are administered and dosed in accordance with good medical practice, taking into account the clinical condition of the individual patient, the site and method of administration, scheduling of administration, patient age, sex, body weight, species of the patient, and other factors known to those skilled in the pharmaceutical or veterinary arts.

The pharmaceutically "effective amount" for purposes herein is thus determined by such considerations as are known in the art. The amount must be effective to achieve improvement including but not limited to improved survival rate or more rapid recovery, or improvement or elimination of symptoms and other indicators, e.g., of osteopososis, for instance, improvement in bone density, as are selected as appropriate measures by those skilled in the art.

Where appropriate the therapeutics of the present invention are pharmaceuticals and as such can be administered in various ways. It should be noted that these therapeutics can be administered as the expression product and/or portion thereof and/or antibody and/or portion thereof or as pharmaceutically acceptable salt and can be administered alone or as an active ingredient in combination with pharmaceutically acceptable carriers, diluents, adjuvants and vehicles, as well as other active ingredients (e.g., other expression products, portions thereof, antibodies, portions thereof, from inventive methods, and/or other therapies, such as those discussed herein). The compounds can be administered orally, subcutaneously or parenterally including intravenous, intraarterial, intramuscular, intraperitoneally, and intranasal administration as well as intrathecal and infusion techniques.

Implants of the therapeutics and/or of vectors expressing the herein defined genes are also useful. The patient being treated is a warm-blooded animal and, in particular, mammals including man. The pharmaceutically acceptable carriers, diluents, adjuvants and vehicles as well as implant carriers generally refer to inert, non-toxic solid or liquid fillers, diluents or encapsulating material not reacting with the active ingredients of the invention. The implant can be placed near bone, to stimulate bone growth or increase bone density. With respect to implants or slow release systems that can be used in the practice of the invention with respect to therapeutics, or vectors expressing the herein defined genes, mention is made of U.S. Patents Nos. 4,150,108, 4,329,332, 4,331,652, 4,333,919, 4,389,330, 4,489,055, 4,526,938, 4,530,840, 4,542,025, 4,563,489, 4,675,189, 4,677,191, 4,683,288, 4,758,435, 4,857,335, 4,931,287, 5,178,872, 5,252,701, 5,275,820, 5,478,564, 5,540,912, 5,447,725, 5,599,852, 5,607,686, 5,609,886, 5,631,015, 5,654,010, 5,700,485, 5,702,717, 5,711,968, 5,733,566, 4,938,763, 5,077,049, 5,278,201, 5,278,202, 5,288,496, 5,324,519, 5,324,520, 5,340,849, 5,368,859, 5,401,507, 5,419,910, 5,427,796, 5,487,897, 5,599,552, 5,632,727, 5,643,595, 5,660,849, 5,686,092, 5,702,716, 5,707,647, 5,717,030, 5,725,491, 5,733,950, 5,736,152, 5,744,153, 5,759,563, and 5,780,044, European Patent Application 0537559, Shah et al (J. Controlled Release, 1993, 27:139-147), Lambert and Peck (J. Controlled Release, 1995, 33:189-195), and Shivley et al (J. Controlled Release, 1995, 33:237-243).

It is noted that humans are treated generally longer than the mice or other experimental animals exemplified herein which treatment has a length proportional to the length of the disease process and drug effectiveness. The doses may be single doses or multiple doses over a period of several days, but single doses are preferred. Thus, one can scale up from animal experiments, e.g., rats, mice, and the like, to humans, by techniques from this disclosure and the knowledge in the art, without undue experimentation.

The doses may be single doses or multiple doses over a period of several days. The treatment generally has a length proportional to the length of the disease process and drug effectiveness and the patient species being treated.

When administering a therapeutic or vector of the present invention parenterally, it will generally be formulated in a unit dosage injectable form (solution, suspension, emulsion). The pharmaceutical formulations suitable for injection include sterile aqueous solutions or dispersions and sterile powders for reconstitution into sterile injectable solutions or dispersions. The carrier can be a solvent or dispersing medium containing, for example, water, ethanol, polyol (for example, glycerol, propylene glycol, liquid polyethylene glycol, and the like), suitable mixtures thereof, and vegetable oils.

Proper fluidity can be maintained, for example, by the use of a coating such as lecithin, by the maintenance of the required particle size in the case of dispersion and by the use of surfactants. Nonaqueous vehicles such a cottonseed oil, sesame oil, olive oil, soybean oil, corn oil, sunflower oil, or peanut oil and esters, such as isopropyl myristate, may also be used as solvent systems for compound compositions

Additionally, various additives which enhance the stability, sterility, and isotonicity of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. In many cases, it will be desirable to include isotonic agents, for example, sugars, sodium chloride, and the like. Prolonged absorption of the injectable pharmaceutical form can be brought about by the use of agents delaying absorption, for example, aluminum monostearate and gelatin. According to the present invention, however, any vehicle, diluent, or additive used would have to be compatible with the compounds.

Sterile injectable solutions can be prepared by incorporating the compounds utilized in practicing the present invention in the required amount of the appropriate solvent with various of the other ingredients, as desired.

A pharmacological formulation of the present invention, e.g., comprising a therapeutic and/or vector, can be administered to the patient in an injectable formulation containing any compatible carrier, such as various vehicle, adjuvants, additives, and diluents; or the compounds utilized in the present invention can be administered parenterally to the patient in the form of slow-release subcutaneous implants or targeted delivery systems such as monoclonal antibodies, vectored delivery, iontophoretic, polymer matrices, liposomes, and microspheres. Examples of delivery systems useful in the present invention include: 5, 225, 182; 5, 169, 383; 5, 167,616; 4,959,217; 4,925,678; 4,487,603; 4,486,194; 4,447,233; 4,447,224; 4,439,196; and 4,475,196. Many other such implants, delivery systems, and modules are well known to those skilled in the art (*See also* documents cited herein, e.g., *supra*).

A pharmacological formulation of the compound utilized in the present invention can be administered orally to the patient. Conventional methods such as administering the compounds in tablets, suspensions, solutions, emulsions, capsules, powders, syrups and the like are usable. Known techniques which deliver it orally or intravenously and retain the biological activity are preferred.

In one embodiment, a formulation of the present invention can be administered initially, and thereafter maintained by further administration. For instance, a formulation of the invention can be administered in one type of composition and thereafter further administered in a different or the same type of composition. For example, a formulaiton of the invention can be administered by intravenous injection to bring blood levels to a suitable level. The patient's levels are then maintained by an oral dosage form, although other forms of administration, dependent upon the patient's condition and as indicated above, can be used.

The quantity to be administered will vary for the patient being treated and will vary from about 100 ng/kg of body weight to 100 mg/kg of body weight per day and preferably will be from 10 pg/kg to 10 mg/kg per day. For instance, dosages can be readily ascertained by those skilled in the art from this disclosure and the knowledge in the art. Thus, the skilled artisan can readily determine the amount of gene product and optional additives, vehicles, carrier and/or adjuvant in compositions and to be administered in methods of the invention. Typically, an adjuvant or additive is commonly used as 0.001 to 50 wt% solution in phosphate buffered saline, and the gene product or active ingredient is present on the order of micrograms to milligrams, such as about 0.0001 to about 5 wt%, preferably about 0.0001 to about 1 wt%, most preferably about 0.0001 to about 0.05 wt% or about 0.001 to about 20 wt%, preferably about 0.01 to about 10 wt%, and most preferably about 0.05 to about 5 wt%. Of course, for any composition to be administered to an animal or human, including the components thereof, and for any particular method of administration, it is preferred to determine therefor: toxicity, such as by determining the lethal dose (LD) and LD₅₀ in a suitable animal model e.g., rodent such as mouse; and, the dosage of the composition(s), concentration of components therein and timing of administering the composition(s), which elicit a suitable response, such as by titrations of sera and analysis thereof, e.g., by ELISA and/or RFFIT analysis. Such determinations do not require undue experimentation from the knowledge of the skilled artisan, this disclosure and the documents cited herein. And, the time for sequential administrations can be ascertained without undue experimentation.

Examples of compositions comprising a therapeutic and/or vector of the invention include liquid preparations for orifice, e.g., oral, nasal, anal, vaginal, peroral, intragastric, mucosal (e.g., perlingual, alveolar, gingival, olfactory or respiratory mucosa) etc., administration such as suspensions, syrups or elixirs; and, preparations for parenteral, subcutaneous, intradermal, intramuscular or intravenous administration (e.g., injectable administration), such as sterile suspensions or emulsions. Such compositions may be in admixture with a suitable carrier, diluent, or excipient such as sterile water, physiological saline, glucose or the like. The compositions can also be lyophilized. The compositions can contain auxiliary substances such as wetting or emulsifying agents, pH buffering agents, gelling or viscosity enhancing additives, preservatives, flavoring agents, colors, and the like, depending upon the route of administration and the preparation desired. Standard texts, such as "REMINGTON'S PHARMACEUTICAL SCIENCE", 17th edition, 1985, incorporated herein by reference, may be consulted to prepare suitable preparations, without undue experimentation.

Compositions of the invention, are conveniently provided as liquid preparations, e.g., isotonic aqueous solutions, suspensions, emulsions or viscous compositions which may be buffered to a selected pH. If digestive tract absorption is preferred, compositions of the invention can be in the "solid" form of pills, tablets, capsules, caplets and the like, including "solid" preparations which are time-released or which have a liquid filling, e.g., gelatin covered liquid, whereby the gelatin is dissolved in the stomach for delivery to the gut. If nasal or respiratory (mucosal) administration is desired, compositions may be in a form and dispensed by a squeeze spray dispenser, pump dispenser or aerosol dispenser. Aerosols are usually under pressure by means of a hydrocarbon. Pump dispensers can preferably dispense a metered dose or, a dose having a particular particle size.

Compositions of the invention can contain pharmaceutically acceptable flavors and/or colors for rendering them more appealing, especially if they are administered orally. The viscous compositions may be in the form of gels, lotions, ointments, creams and the like (e.g., for transdermal administration) and will typically contain a sufficient amount of a thickening agent so that the viscosity is from about 2500 to 6500 cps, although more viscous compositions, even up to 10,000 cps may be employed. Viscous compositions have a viscosity preferably of 2500 to 5000 cps, since above that range they become more difficult to administer. However, above that range, the compositions can approach solid or gelatin forms which are then easily administered as a swallowed pill for oral ingestion.

Liquid preparations are normally easier to prepare than gels, other viscous compositions, and solid compositions. Additionally, liquid compositions are somewhat more convenient to administer, especially by injection or orally, to animals, children, particularly small children, and others who may have difficulty swallowing a pill, tablet, capsule or the like, or in multi-dose situations. Viscous compositions, on the other hand, can be formulated within the appropriate viscosity range to provide longer contact periods with mucosa, such as the lining of the stomach or nasal mucosa.

Obviously, the choice of suitable carriers and other additives will depend on the exact route of administration and the nature of the particular dosage form, e.g., liquid dosage form (e.g., whether the composition is to be formulated into a solution, a suspension, gel or another liquid form), or solid dosage form (e.g., whether the composition is to be formulated into a pill, tablet, capsule, caplet, time release form or liquid-filled form).

Solutions, suspensions and gels, normally contain a major amount of water (preferably purified water) in addition to the antigen, lipoprotein and optional adjuvant. Minor amounts of other ingredients such as pH adjusters (e.g., a base such as NaOH), emulsifiers or dispersing agents, buffering agents, preservatives, wetting agents, jelling agents, (e.g., methylcellulose), colors and/or flavors may also be present. The compositions can be isotonic, i.e., it can have the same osmotic pressure as blood and lacrimal fluid.

The desired isotonicity of the compositions of this invention may be accomplished using sodium chloride, or other pharmaceutically acceptable agents such as dextrose, boric acid, sodium tartrate, propylene glycol or other inorganic or organic solutes. Sodium chloride is preferred particularly for buffers containing sodium ions.

Viscosity of the compositions may be maintained at the selected level using a pharmaceutically acceptable thickening agent. Methylcellulose is preferred because it is readily and economically available and is easy to work with. Other suitable thickening agents include, for example, xanthan gum, carboxymethyl cellulose, hydroxypropyl cellulose, carbomer, and the like. The preferred concentration of the thickener will depend upon the agent selected. The important point is to use an amount which will achieve the selected viscosity. Viscous compositions are normally prepared from solutions by the addition of such thickening agents.

As mentioned herein, a pharmaceutically acceptable preservative can be employed to increase the shelf-life of the compositions. Benzyl alcohol may be suitable, although a variety of preservatives including, for example, parabens, thimerosal, chlorobutanol, or benzalkonium chloride may also be employed. A suitable concentration of the preservative will be from 0.02% to 2% based on the total weight although there may be appreciable variation depending upon the agent selected.

Those skilled in the art will recognize that the components of the compositions should be selected to be chemically inert with respect to the gene product and optional adjuvant or additive. This will present no problem to those skilled in chemical and pharmaceutical principles, or problems can be readily avoided by reference to standard texts or by simple experiments (not involving undue experimentation), from this disclosure and the documents cited herein.

The inventive compositions of this invention are prepared by mixing the ingredients following generally accepted procedures. For example the selected components may be simply mixed in a blender, or other standard device to produce a concentrated mixture which may then be adjusted to the final concentration and viscosity by the addition of water or thickening agent and possibly a buffer to control pH or an additional solute to control tonicity. Generally the pH may be from about 3 to 7.5. Compositions can be administered in dosages and by techniques well known to those skilled in the medical and veterinary arts taking into consideration such factors as the age, sex, weight, and condition of the particular patient or animal, and the composition form used for administration (e.g., solid vs. liquid). Dosages for humans or other mammals can be determined without undue experimentation by the skilled artisan, from this disclosure, the documents cited herein, the Examples below.

Suitable regimes for initial administration and booster doses or for sequential administrations also are variable, may include an initial administration followed by subsequent administrations; but nonetheless, may be ascertained by the skilled artisan, from this disclosure, the documents cited herein, and the Examples below.

Accordingly, the invention comprehends, in further aspects, methods for preparing therapeutic compositions including a gene product or functional fragment thereof of a gene identified herein or a gene identified in an inventive method herein, as well as to methods for increasing bone density, treating, preventing or controlling osteporosis, or otherwise alleviating a condition caused by mechanical stress or inducing bone growth, comprising administering an inventive composition, or a gene product, or functional fragment thereof of a gene identified herein or a gene identified in an inventive method herein, or a vector expressing such a gene.

In this context and as used throughout this specification, "functional" means a protein having part or all of the primary structural conformation of the protein gene product of a gene identified herein or of a gene identified by the methods herein, and possessing the biological property of contributing to the development of bone cells in the same or an analogous fashion to the full length protein gene product, said protein gene product being either isolated from a natural source or being the product of procaryotic or eukaryotic expression or of protein synthesis methods. The protein can have an amino acid sequence comprising an amino acid sequence of a sequence disclosed herein or of a gene product of a gene identified by a method herein or any fragment or derivative thereof by way of amino acid deletion, substitution, insertion, addition and/or replacement of the amino acid sequence. Also comprised by the term "functional" protein is the capability of said protein or part thereof to generate a specific immune response such as an antibody response; e.g., to bind to antibodies elicited by the full length protein.

Moreover, the present invention and embodiments thereof provide advances in and assist to further research and knowledge with respect to osteoporosis and conditions caused by or having as a factor mechanical stress or a lack thereof and provide an insight into development and maintenance of bone tissue. The present invention and embodiments thereof also provide advances in and assist to further clinical and epidemiological research, e.g., to allow others to further explore and extend the current potential for practical prevention and treatment. Further still, the present invention and embodiments thereof provide a deeper knowledge of factors controlling bone cell activity and regulation of bone mineral and matrix formation and remodeling contribute ultimately to the understanding of the etiology of osteoporosis or other conditions involving mechanical stress or a lack thereof. For example, the present invention provides osteporosis or mechanical stress or lack thereof models for *in vitro* studies. This understanding will permit a more rational choice and evaluation of therapies, even as current treatments are evaluated clinically. Moreover, the present invention, for instance, via the inventive models, allows for the discovery of genes involved in processes of osteoporosis and/or one growth or formation or bone cell activity, *inter alia.* Every new gene discovered sheds more light on the complex molecular events that govern all aspects of life. The elucidation of the function of the gene and its place and role in this intricate network of pathways and structures resolves another piece in the puzzle of life. Thus, the educational and research implications are very clear. Sometimes genes may have much more benefit in this respect than in the therapeutics/diagnostics fields.

A better understanding of the present invention and of its many advantages will be had from the following examples, given by way of illustration and as a further description of the invention.

### EXAMPLES/RESULTS

### Example/Result 1: ANALYSYS OF GENES AT A TRANSCRIPTIONAL LEVEL USING NUCLEAR mRNA PROBES

### DIFFERENTIAL TRANSLATION

### MATERLALS/METHODS (WHICH MAY APPLY IN WHOLE OR PART TO SOME OR ALL EXAMPLES)

### General Scheme

a. Total mRNA organic extraction of all RNA from the source tissue or cell. (additional selection for polyA+ mRNA can be included).
b. Nuclear RNA-lysis of cells (from a tissue or a cell line) by homogenization in hypotonic buffer. Collection of nuclei by centrifugation and organic extraction of the RNA.
c. Cytoplasmic RNA - Organic extraction of the RNA from the supernatant from b above.
d. Potyribosomal/subpolyribosomal fractionation. Lysis of cells by homogenization hypotonic buffer, removal of nuclei and fractionation of polyribosome on linear sucrose gradients and organic extraction of the RNA from each fraction of the gradient.
e. Secreted and membrane encoding transcripts.
   1. Isolation of RER on Percol gradients (after homogenization of cells).
   2. Preparation of microsomes containing the RER
   3. Isolation of membrane-bound polyribosomes by successive treatment of cells with detergents.
f. Nuclear proteins. Isolation of cytoskeletal associated polyribosomes by treating cells lyzates with different detergents.
g. Mitochondrial genes. Isolation of mitochondria on Percoll gradients.
h. Alternative splicing. Separation of nuclei and isolation of splicsosome (proteins and RNA complex) on linear sucrose gradients.

Preparation of cell extracts: Cells were centrifuged. The pellet was washed with PBS and recentrifuged. The cells were resuspended in 4x of one packed cell volume (PCV) with hypotonic lysis buffer (HLB: 20mM TrisHCL pH=7.4; 10mM NaCl; 3mM MgCl₂). The cells were incubated five minutes on ice. 1xPCV ofHLB containing 1.2% Triton X-100 and 0.2M sucrose was added. The cells were homogenized with a Dounce homogenizer (five strokes with B pestle). The cell lysate was centrifuged at 2300g for ten minutes at 4°C. The supernatant was transferred to a new tube. HLB containing 10mg/ml heparin was added to a final concentration of 1mg/ml heparin. NaCl was added to a final concentration of 0.15M. The supernatant was frozen at -70°C after quick freezing in liquid N₂ or used immediately.

Sucrose gradient fractionation: A linear sucrose gradient from O.5M to 1.5M sucrose in HLB was prepared. Polyallomer tubes (14X89mm) were used. 0.5 to 1.0ml of cell extract was loaded on the gradient. The cells were centrifuged at 36,000 RPM for 110 minutes at 4°C. An ISCO Density Fractionator was used to collect the fractions and record the absorbance profile.

RNA purification: SDS was added to 0.5% and Proteinase K to 0.1mg/ml and incubated at 37°C for 30 minutes. Extract with an equal volume of phenol+chloroform (1:1). The aqueous phase was extracted with one volume of chloroform and the RNA was precipitated by adding Na-Acetate to 0.3M and 2.5 volumes of ethanol and incubating at -20°C overnight. Centrifuged ten minutes, the supernatant was aspirated and the RNA pellet was dissolved in sterile, diethylpyrocarbonate (hereinafter referred to as "DEPC") DEPC-treated water.

Preparation of Microsomes: When possible fresh tissues and cells are used, without freezing. Tissues were powdered in liquid nitrogen with mortar and pestle and then homogenized using 4ml of buffer A/l gr tissue (Buffer A is 250mM sucrose, 50mM TEA, 50mM KOAc pH7.5, 6mM Mg(Oac)₂, 1mM EDTA, 1mM DTT, 0.5mM PMSF. PMSF was made in ethanol before making the buffer and added in drops to buffer while being stirred. This was stirred for 15 minutes and then DTT was added). Fresh organs were washed in Buffer A a few times, and then cut into pieces and homogenized. Approximately 5ml buffer A/5x10⁸ cells were added and homogenized. This was then homogenized on ice for 5-10 times, or as needed with the individual tissue. The mixture was transferred to 50ml tubes, then centrifuged for 10 minutes, at 4°C in a swinging bucket rotor machine. Next, the supernatant was transferred, avoiding the pellet as much as possible, to a Sorvall tube, the pellet was washed again with 1ml buffer and centrifuge as before. The two pellets were combined, thus establishing the nuclear fraction. The combination was dissolved and treated the pellet with Tri-reagent (usually 2ml of Tri-reagent when sample is from cells) to extract the nuclear RNA. The combined 1st and 2nd supernatants were centrifuged for 10 minutes at 10000g at 4°C. Again, the supernatant was transferred to a tube and kept on ice. The pellet was washed again with 1ml buffer and centrifuged for 10 minutes at 10000g and the two pellets were combined as before, thus establishing the Mitochondrial pellet. Again, the pellet was treated with Tri-reagent (usually 1ml with cells) and the Mitochondrial RNA was extracted. Next, cold ultracentrifuge tubes were prepared containing a sucrose cushion made of: buffer A + 1.3M sucrose. The volume of the cushion was approximately 1/3 of the supernatant. The supernatant was loaded on the cushion in a 1:3 ratio of cushion to supernatant. A pair of tubes was weighed for balancing, a 20-30mg difference is allowable. The tubes were centrifuged 2.5 hours at 140,000g, 4°C with a Ti60.2 rotor (45,000 rpm). When two phases of supernatant were visible, then the red phase only was transferred (if possible), as the cytoplasmic fraction, to a sorvall tube. The clear supernatant was aspirated. When not possible to separate or phase distinction not visible, all the supernatant was taken as cytoplasmic fraction and dilute sucrose with TE (10mM Tris-HCl pH 8.0, 1mM EDTA). In the pellet were the microsomes which were visible and were clear or yellowish. For the RNA extraction, the cytoplasmic fraction was treated with 1% SDS, 0.1mg/ml proteinase K, for 30 minutes, at 37°C. After this, freezing at -80°C was possible. The RNA was extracted with a phenol:chloroform combination and precipitate with 0.3M Na-acetate, 1µl glycogen, and equal volume of isopropanol. O'N precipitation was possible and can be accomplished at 30 minutes on ice. The extract was spun at 10000g, for 20 minutes, then the RNA pellet was washed with 70% ethanol. The pellet was dried and then dissolved in H₂O. The microsomes were then dissolved with 0.1M NaCl/1% SDS solution (1ml is usually sufficient for a small pellet) and extracted with a phenol:chloroform combination (no proteinase K treatment). Then the precipitation of the RNA was done in the same way as for the cytoplasmic fraction but without the requirement of adding salt.

Preparation of Nuclear and Cytoplasmic RNA: Subconfluent plates were washed with 125 mM KCI-30 mM Tris-hydrochloride (pH 7.5)-5 mM magnesium acetate-1 mM 2-mercaptoethanol-2 mM ribonucleoside vanadyl complex (2)-0.15 mM spermine-0.05 mM spermidine at 4°C, and cells scraped from the plates were washed twice with the same buffer. Approximately 10⁸ cells were allowed to swell for 10 minutes in 2.5 ml of swelling buffer (same as wash buffer except the KCI concentration was 10 mM) lysed with 20 strokes of a Dounce homogenizer (B pestle), overlaid on an equal volume of swelling buffer containing 25% glycerol, and centrifuged for 5 min. at 400 x g and 4°C. The upper layer of the supernatant, which contained 90% of the CAD sequences released by lysis, was designated the cytoplasmic fraction. The nuclear pellet was washed once with 2 ml of swelling buffer-25% glycerol-0.5% Triton X-100 and once with 2 ml of swelling buffer.

Nuclear RNP. Nuclei from 10⁸ cells, prepared as described above, were suspended in 1 ml of 10 mM Tris-hydrochloride (pH 8.0)-100 mM NaCl-2 mM MgCl₂-1 mM 2-mercapthoethanol-0.15 mM spermine-0.05 mM spermidine-10 mM ribonucleoside vanadyl complex (2)-100 U of placental RNase inhibitor (Amersham Corp.) per ml and sonicated at the maximum power setting of a Konres micro-ultrasonic cell disrupter for 20 g at 4°C. Bacterial tRNA (2 mg) was added, to adsorb basic proteins (9), and the mixture was centrifuged for 1 minute (Eppendorf microcentrifuge). The supernatant was applied to a 15 to 45% sucrose gradient in mM Tris-hydrochloride-100 mM NaCl-2 mM MgCl₂-2 mM ribonucleoside vanadyl complex and centrifuged in a Beckman SW41 rotor for 90 minutes at 40,000 rpm and 4°C. RNA was recovered from gradient fractions by the addition of sodium dodecyl sulfate to 0.5%, treatment with proteinase K (200 µg/ml) for 2 hours at 37°C, extraction with phenol, and precipitation with ethanol.

Preparation of Antisense RNA: Total cellular RNA is extracted. Part of the RNA pool is immobilized on a membrane, another part converted into cDNA after ligation of oligodeoxynucletides to the 3'-ends. The use of biotinylated, complementary oligos for cDNA synthesis allows immobilization of a "minus" strand to streptavidin-coated magnetic beads. A second set of oligos is ligated to the cDNA at the previous 5'-end of the RNA. Plus strands are eluted from the bound strands and hybridized to the membrane-bound RNA. Since the cDNA strand used has the same polarity of the RNAs, only cDNA sequences that can bind to complementary RNAs should be retained. PCR amplification and subsequent cloning of PCR-fragments is followed by sequence analysis. To test whether cloned sequences are correctly identified, probes are generated in sense and antisense direction. Positive clones will be structurally and functionally characterized. In order to work out this method, we started using a bacterial strain (Escherichia coli), containing plasmid R1 that regulates its copy number by antisense RNA. Previous work has identified both antisense (CopA) and target RNA (CopT) of R1 intracellularly. This procedure, if feasible, will then be used to screen for antisense RNA systems in other organisms.

### DIFFERENTIAL ANALYSIS

Differential display: Reverse transcription: 2µg of RNA were annealed with 1pmol of oligo dT primer (dT)₁₈ in a volume of 6.5µl by heating to 70°C for five minutes and cooling on ice. 2µl reaction buffer (x5), 1µl of 10mM dNTP mix, and 0.5µl of SuperScript II reverse transcriptase (GibcoBRL) was added. The reaction was carried out for one hour at 42°C. The reaction was stopped by adding 70µl TE (10mM Tris pH=8; 0.1mM EDTA). Oligonucleotides used for Differential display: The oligonucleotides were essentially those described in the Delta RNA Fingerprinting kit (Clonetech Labs. Inc.). There were 9 "T" oligonucleotides of the structure: 5' CATTATGCTGAGTGATATCTTTTTTTTTXY 3'. The 10 "P" oligonucleotides were of the structure: 3' ATTAACCCTCACTAAA "TGCTGGGGA" 3' where the 9 or 10 nucleotides between the parenthesis represent an arbitrary sequence and there are 10 different sequences, one for each "P" oligo.

Amplification reactions: each reaction is done in 20µl and contains 50µM dNTP mix, 1µM from each primer, 1x polymerase buffer, 1 unit expand Polymerase (Beohringer Mannheim), 2µCi [α-³²P]dATP and 1µl cDNA template. Cycling conditions were: three minutes at 95°C, then three cycles of two minutes at 94°C, five minutes at 40°C, five minutes at 68°C. This was followed by 27 cycles of one minute at 94°C, two minutes at 60°C, two minutes at 68°C. Reactions were terminated by a seven minute incubation at 68°C and addition of 20µl sequencing stop solution (95% formamide, 10mM NaOH, 0.025% bromophenol blue, 0.025% xylene cyanol).

Gel analysis: 3-4µl were loaded onto a 5% sequencing polyacrylamide gel and samples were electrophoresed at 2000 volts/40 milliamperes until the slow dye (xylene cyanol) was about 2 cm from the bottom. The gel was transferred to a filter paper, dried under vacuum and exposed to x-ray film.

Recovery of differential bands: bands showing any a differential between the various pools were excised out of the dried gel and placed in a microcentrifuge tube. 50µl of sterile H₂O were added and the tubes heated to 100°c for five minutes. 1µl was added to a 49µl PCR reaction using the same primers used for the differential display and the samples were amplified for 30 cycles of: one minute at 94°C, one minute at 60°C and one minute at 68°C. 10µl was analyzed on agarous gel to visualize and confirm successful amplification.

### REPRESENTATIONAL DIFFERENCE ANALYSIS

Reverse transcription: as above but with 2µg polyA+ selected mRNA. Preparation of double stranded cDNA: cDNA from previous step was treated with alkali to remove the mRNA, precipitated and dissolved in 20µl H₂O. 5µl buffer, 2µl 10mM dATP, H₂O to 48µl and 2µl terminal deoxynucleotide transferase (TdT) were added. The reaction was incubated 2-4 hours at 37°C. 5µl oligo dT (1µg/µl) was added and incubated at 60°C for 5 minutes. 5µl 200 mM DTT, 10 µl 10x section buffer (100mM Mg Cl₂, 900 mM Hepes, pH 6.6) 16 µl dNTPs (1 mM), and 16 U of Klenow were added and the mixture was incubated overnight at room temperature to generate ds cDNA. 100µl TE was added and extracted with phenol/chloroform. The DNA was precipitated and dissolved in 50µl H₂O.

Generation of representations: cDNA with DpnII was digested by adding 3µl DpnII reaction buffer 20 V and DpnII to 25µl cDNA and incubated five hours at 37°C. 50µl TE was added and extracted with phenol/chloroform. cDNA was precipitated and dissolved to a concentration of 10ng/µl.

The following oligonucleotides are used in this procedure:
R-Bg1-12 5' GATCTGCGGTGA 3'
R-Bg1-24 5' AGCACTCTCCAGCCTCTCACCGCA 3'
J-Bg1-12 5' GATCTGTTCATG 3'
J-Bg1-24 5' ACCGACGTCGACTATCCATGAACA 3'
N-Bg1-12 5' GATCTTCCCTCG 3'
N-Bgl-24 5' AGGCAACTGTGCTATCCGAGGGAA 3'
R-Bgl-12 and R-Bgl-24 oligos were ligated to Tester and Driver: 1.2µg DpnII digested cDNA. 4µl from each oligo and 5µl ligation buffer X10 and annealed at 60°C for ten minutes. 2µl ligase was added and incubated overnight at 16°C. The ligation mixture was diluted by adding 140µl TE. Amplification was carried out in a volume of 200µl using R-Bg1-24 primer and 2µl ligation product and repeated in twenty tubes for each sample. Before adding Taq DNA polymerase, the tubes were heated to 72°C for three minutes. PCR conditions were as follows: five minutes at 72°C, twenty cycles of one minute at 95°C and three minutes at 72°C, followed by ten minutes at 72°C.

Every four reactions were combined, extracted with phenol/chloroform and precipitated. Amplified DNA was dissolved to a concentration of 0.5µg/µl and all samples were pooled.

Subtraction: Tester DNA (20µg) was digested with DpnII as above and separated on a 1.2% agarous gel. The DNA was extracted from the gel and 2µg was ligated to J-Bgl-12 and J-Bg124 oligos as described above for the R-oligos. The ligated Tester DNA was diluted to 10ng/µl with TE. Driver DNA was digested with DpnII and repurified to a final concentration of 0.5µg/µl. Mix 40µg of Driver DNA with 0.4µg of Tester DNA. Extraction was carried out with phenol/chloroform and precipitated using two washes with 70% ethanol, resuspended DNA in 4µl of 30mM EPPS pH=8.0, 3mM EDTA and overlayed with 35µl mineral oil. Denatured at 98°C for five minutes, cool to 67°C and 1µl of 5M NaCl was added to the DNA. Incubated at 67°C for twenty hours. Diluted DNA by adding 400µl TE.

Amplification: Amplification of subtracted DNA in a final volume of 200µl as follows: Buffer, nucleotides and 20µl of the diluted DNA were added, heated to 72°C, and Taq DNA polymerase was added. Incubated at 72°C for five minutes and added J-Bg1-24 oligo. Ten cycles of one minute at 95°C, three minutes at 70°C were performed. Incubated ten minutes at 72°C. The amplification was repeated in four separate tubes. The amplified DNA was extracted with phenol/chloroform, precipitated and all four tubes were combined in 40µl 0.2XTE, Digested with Mung Bean Nuclease as follows: To 20µl DNA 4µl buffer, 14µl H₂O and 2µl Mung Bean Nuclease (10 units/µl) was added. Incubated at 30°C for thirty-five minutes + First Differential Product (DPI).

Repeat subtraction hybridization and PCR amplification at driver: differential ratio of 1:400 (DPII) and 1:40,000 (DPIII) using N-Bgl oligonucleotides and J-Bgl oligonucleotides, respectively. Differential products were cloned into a Bluescript vector at the BAM HI site for analysis of the individual clones.

The experimental cells were grown alternatively under normal conditions, for 4 hours under hypoxia (<1% oxygen) and for 16 hours under hypoxia. The cells were harvested and RNA was extracted either from nuclei that were prepared from the cells (nuclear RNA) or from extracts of unfractionated cells (total cellular RNA).

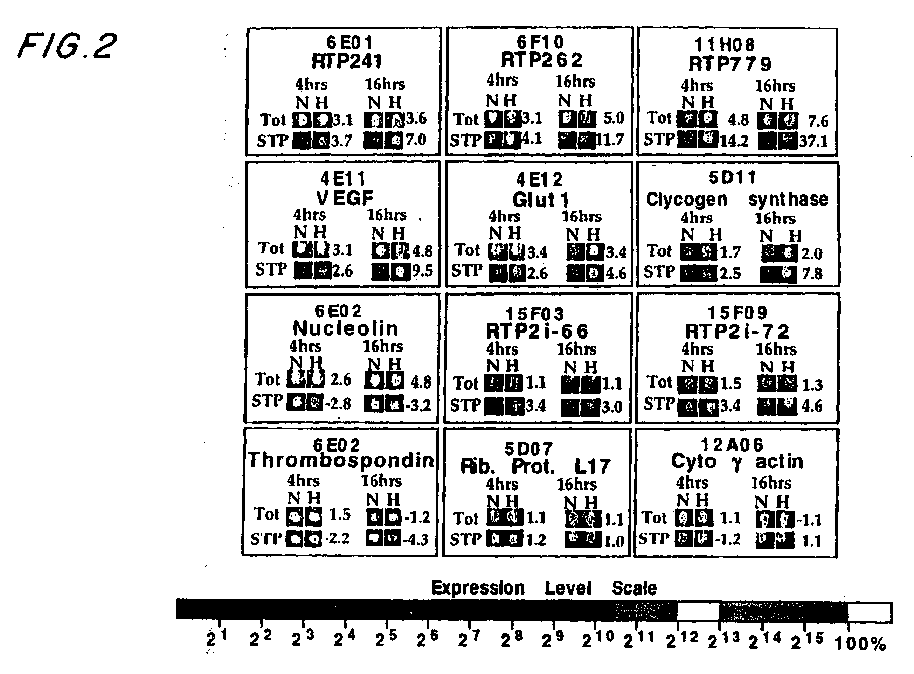

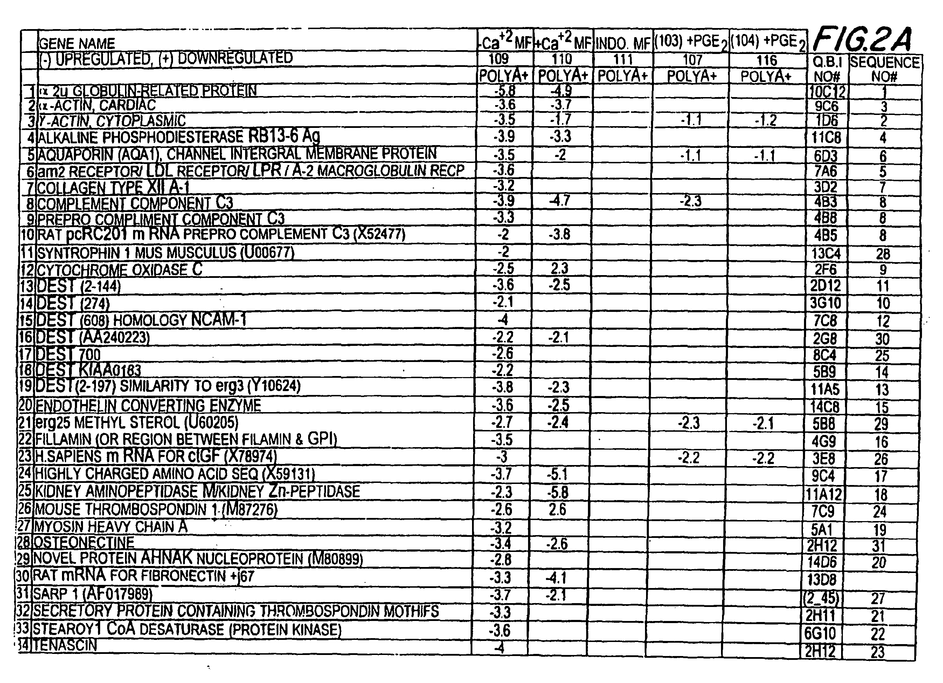

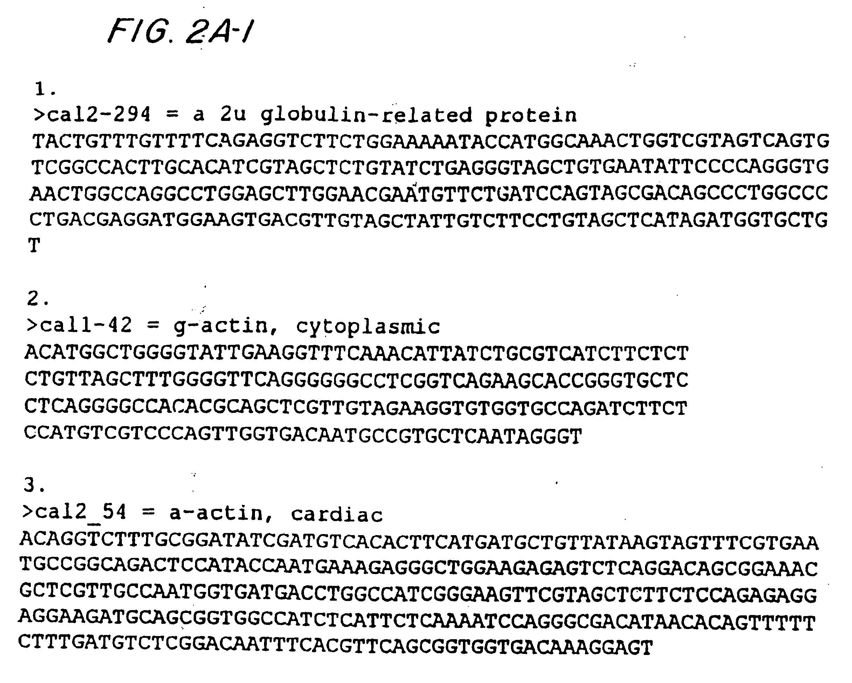

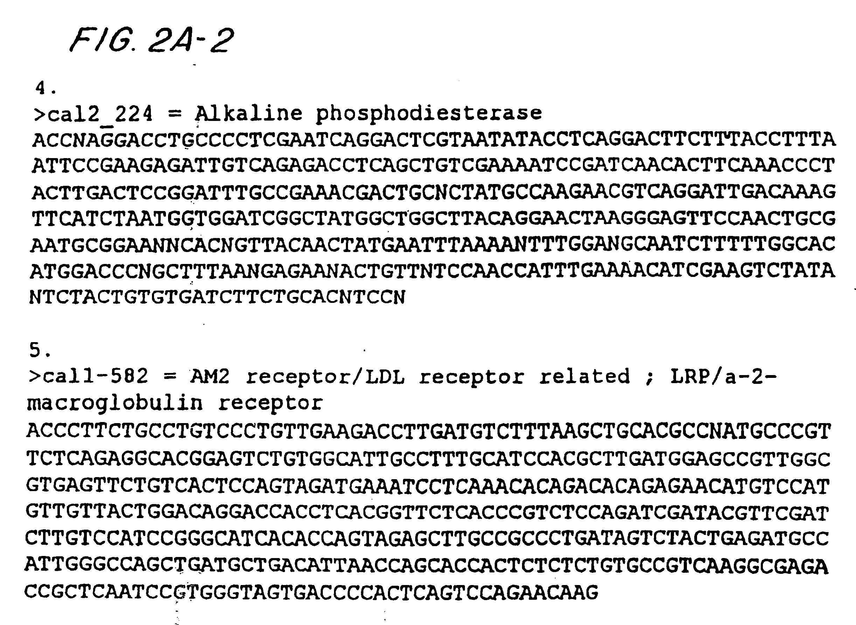

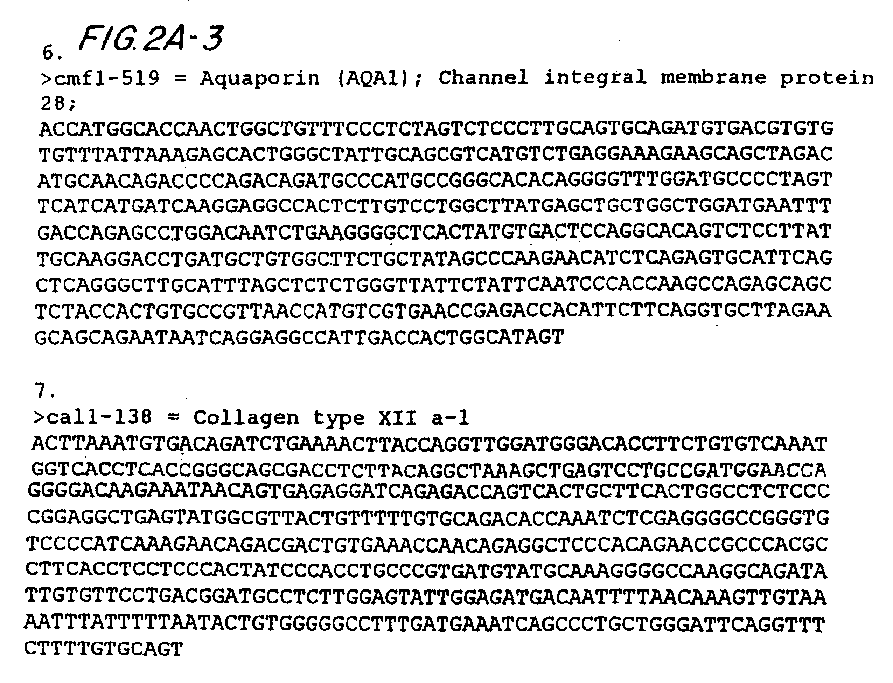

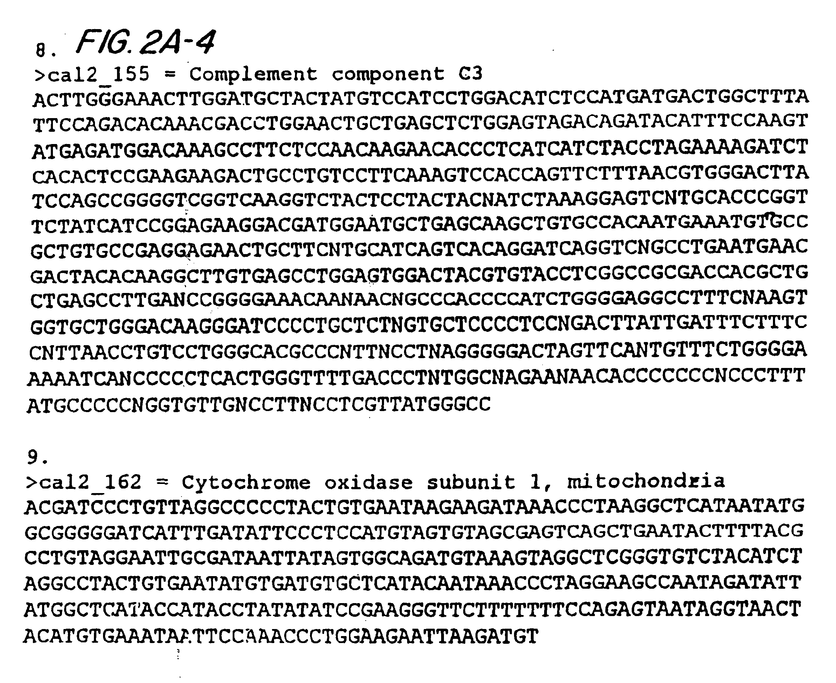

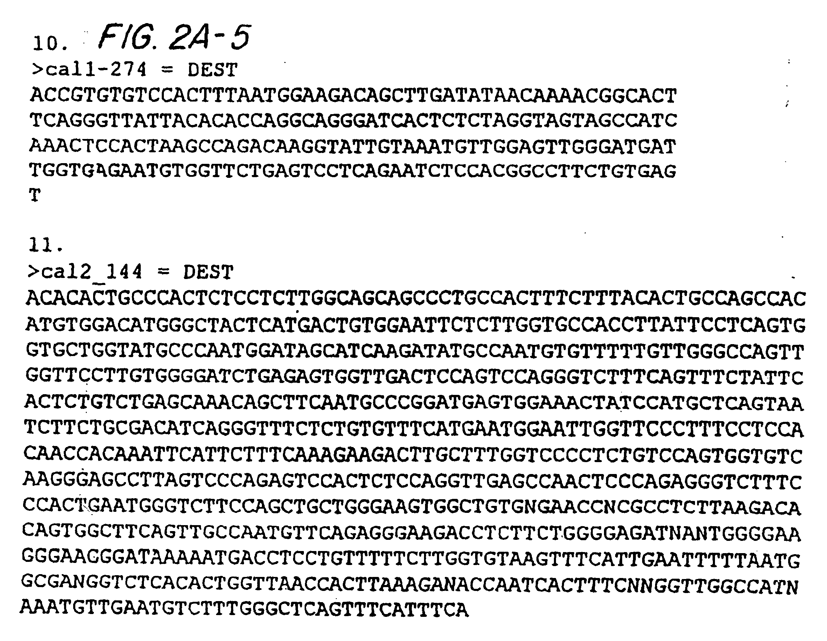

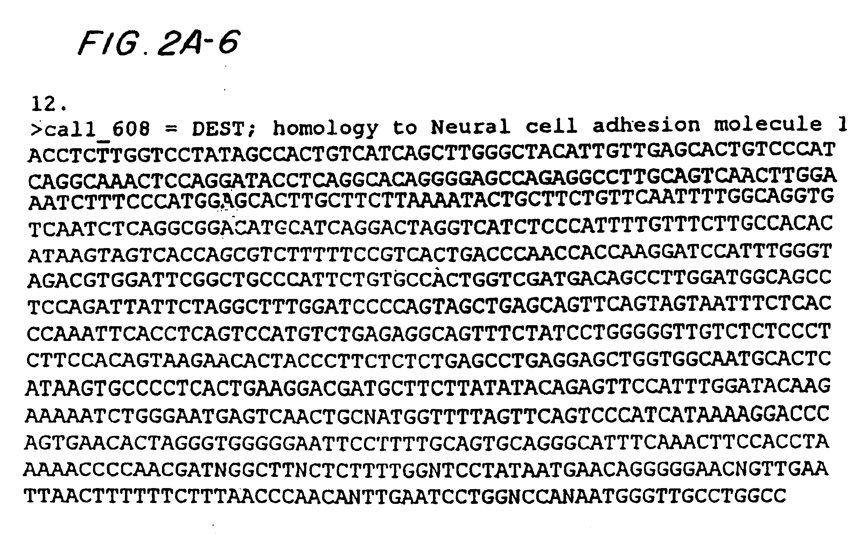

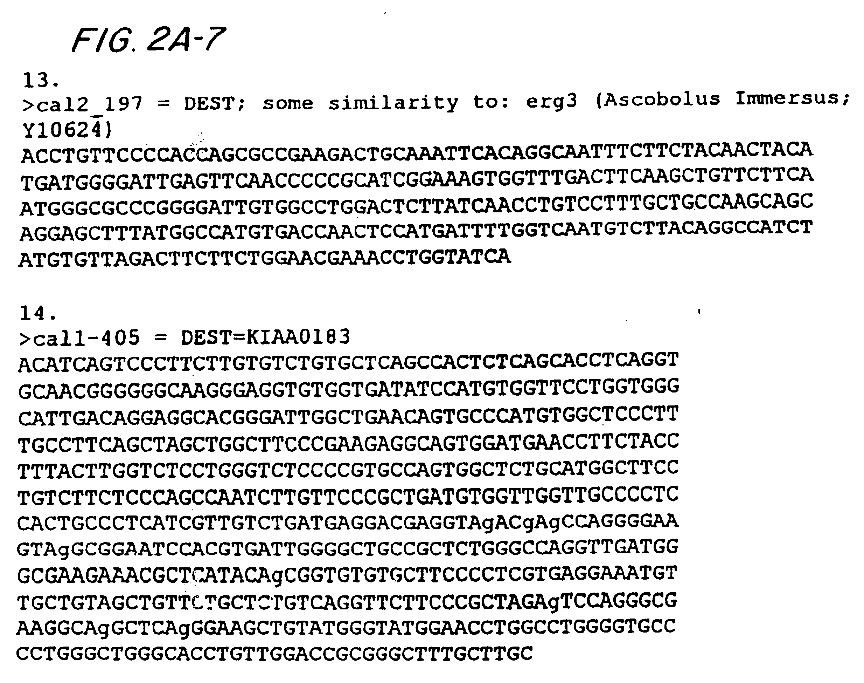

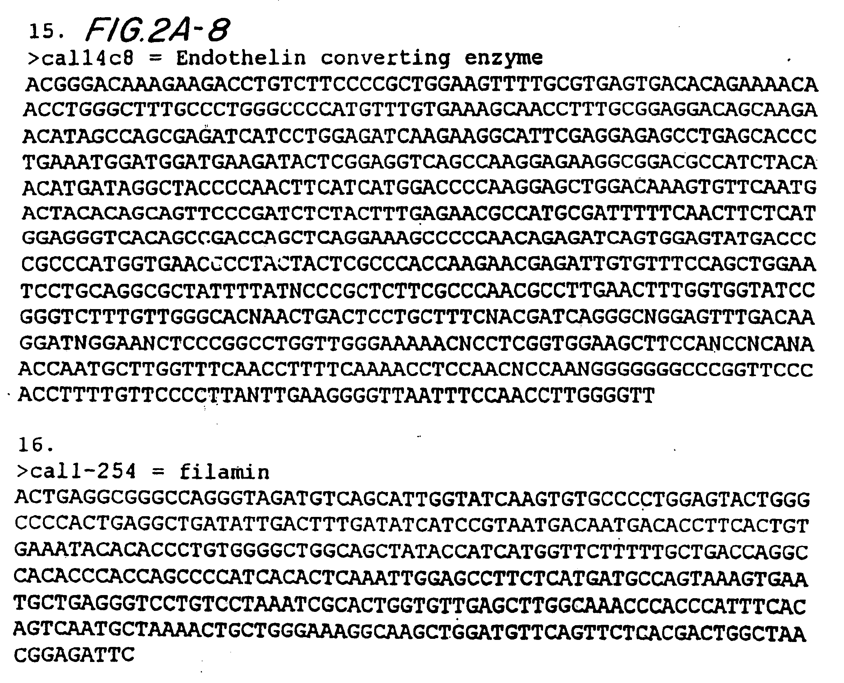

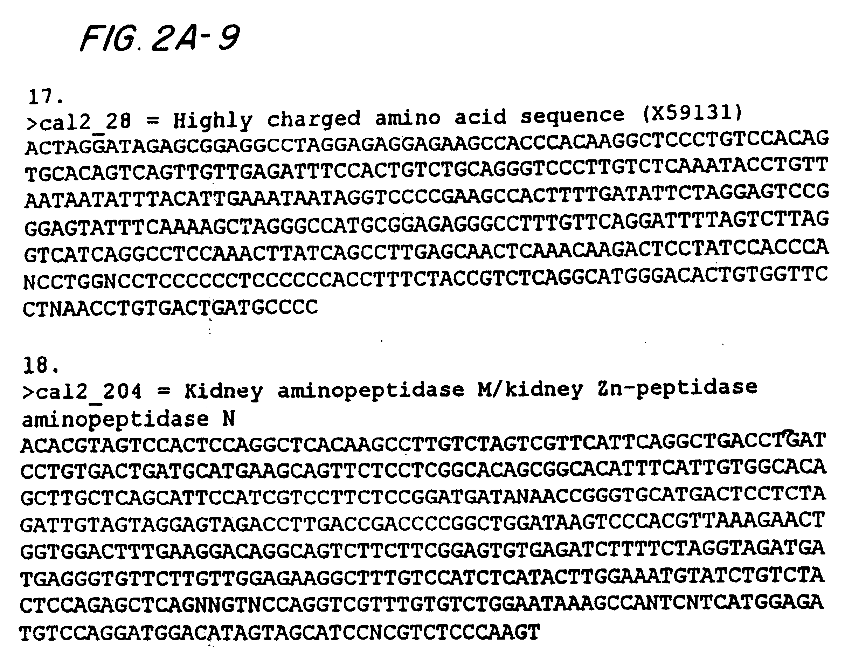

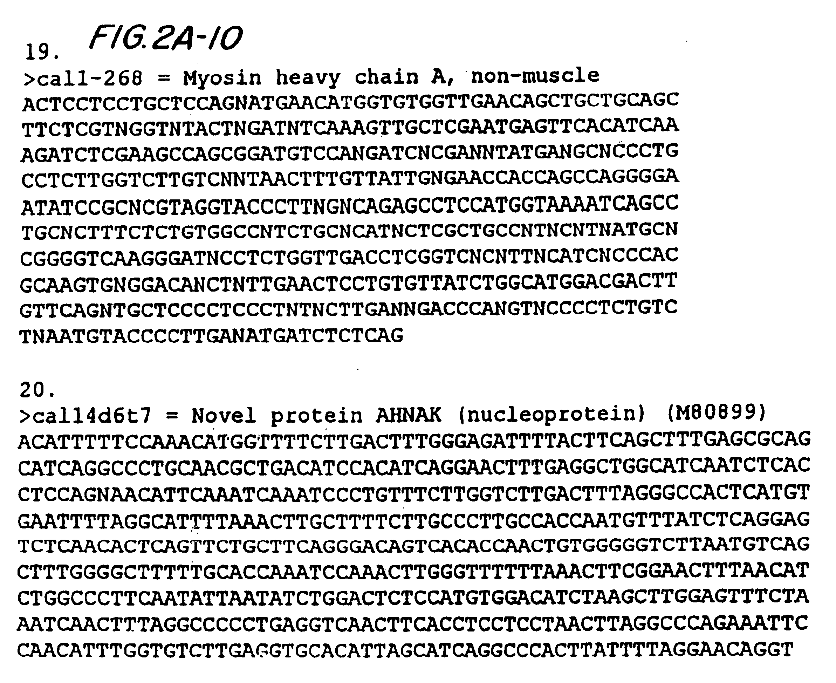

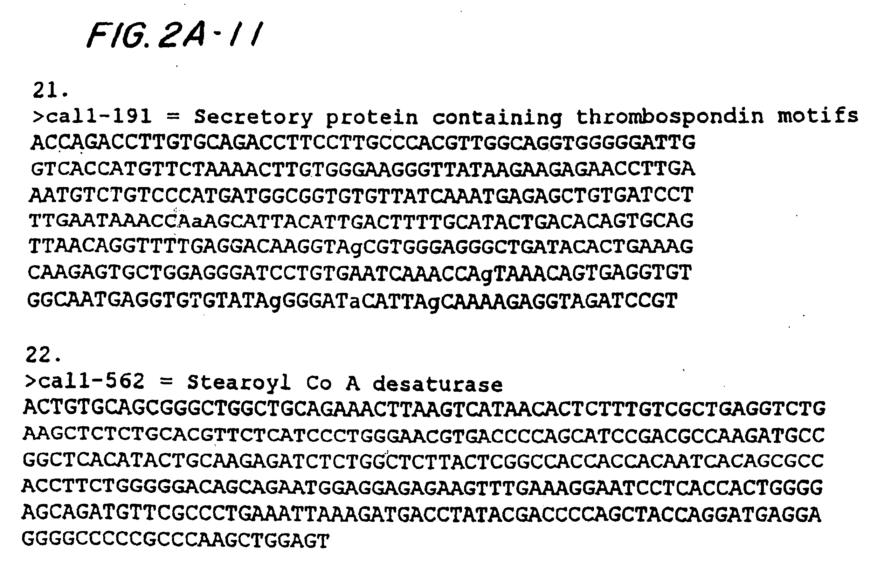

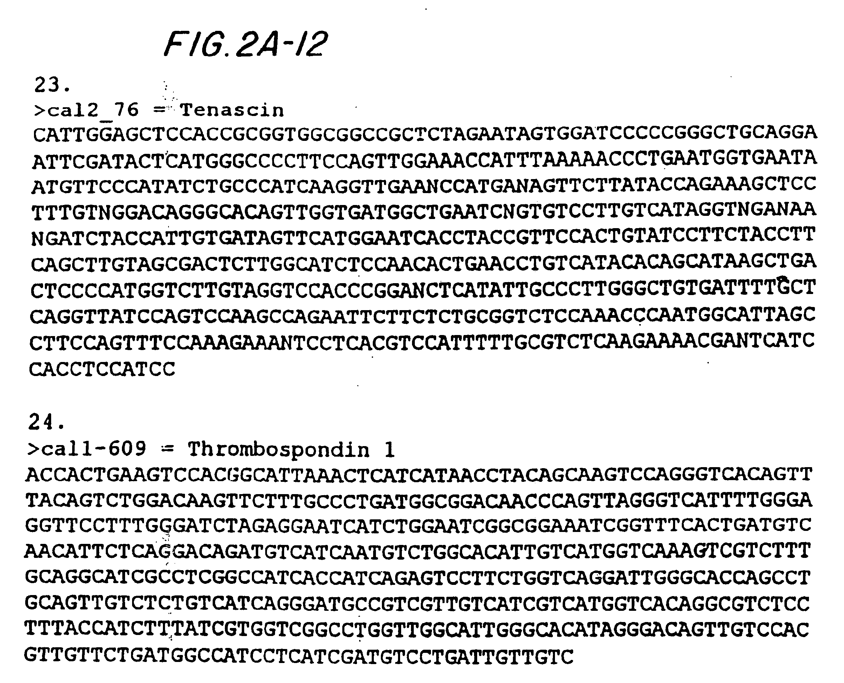

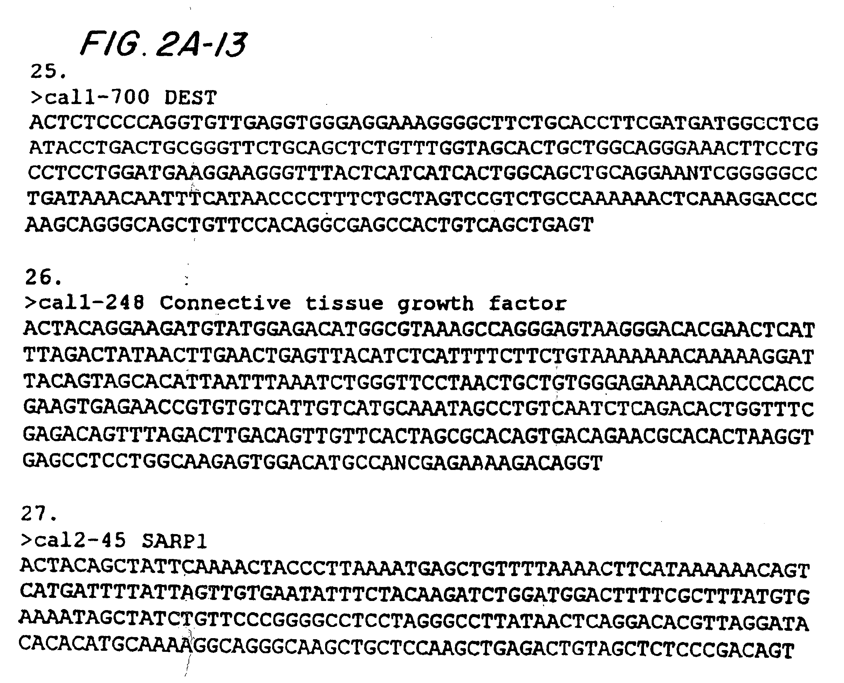

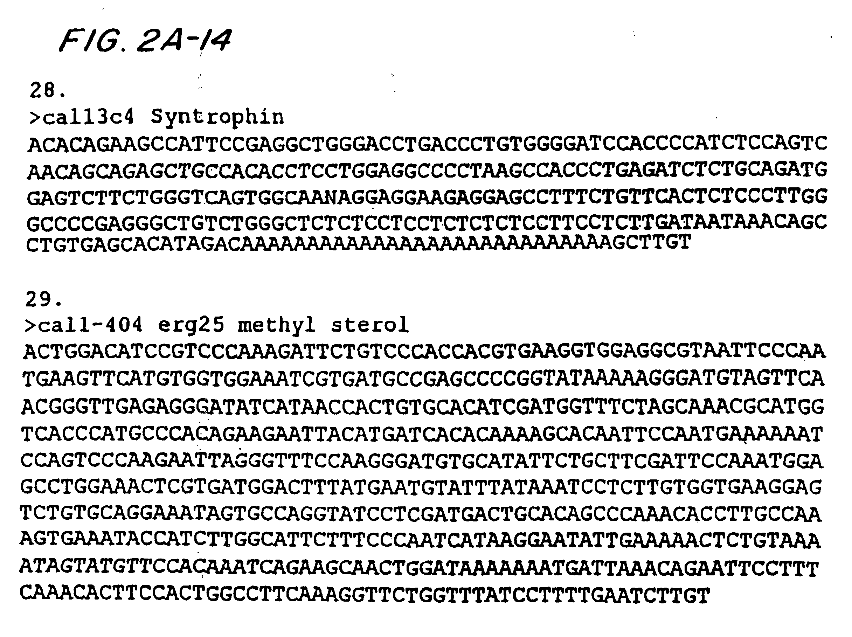

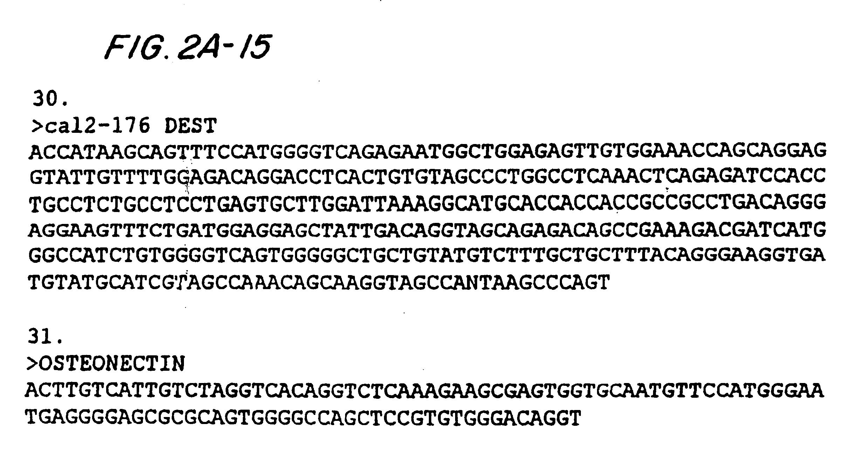

Figure 2 demonstrates how the probes prepared from the nuclear RNA (STP) give a higher differential expression than the total cellular RNA probe (Tot). The control genes encoding VEGF (vascular endothelial growth factor), Glut1 (glucose transporter 1) and glycogen synthase are known to be induced by the hypoxia stress. The level of induction observed in the nuclear probe is much higher than that seen in the total probe and much closer to the actual know level of induction. The three new genes RTP 241, RTP 262 and RTP 779 show marked induction by hypoxia. Again, the induction level seen with the nuclear probe is much higher, up to five-fold higher, as seen for RTP779. When the induction of these genes was analyzed by the Northern blot method, it was found that the nuclear probe was once again much closer to the actual situation, while the total probe gives a marked underestimation.

The genes RTPi-66 and RTP2I-72 demonstrate the ability of the nuclear probe to detect differentially expressed genes that do not appear differentially with the total probe.

The genes for Nucleolin and Thrombospondin show that also for down-regulated mRNAs the nuclear probe is much more sensitive and gives much high levels of differential expression values.

Lastly, the genes for ribosomal protein L17 and cytoplasmic ganuna-actin are known as genes that do not respond to hypoxia stress. The nuclear probe and the total probe both show that no induction occurs.

### Example/Result 2: DIFFERENTIAL EXPRESSION PROFILING

Chip: The microarray (Chip) used was prepared as follows. Subtraction experiments were carried out on rat osteoblasts (Calvaria) using CLONTECH SSH kit (K 1804- 1). Cells were subjected to 20 minutes of mechanical stress and compared to "normal" cells not subjected to mechanical force. 767 induced sequences and 606 reduced sequences were selected and printed on a chip.
Probe: Total RNA
Cells: Primary Calvaria cultures derived from 17-19 days old rat embryos.

### List of Analyses:

| CHIP no | Experiment | Analysis |
|---|---|---|
| 109 | 3 | Compare the system with and without application of mechanical force in absence of Ca in culture medium. Find genes differentially expressed under the influence of mechanical force. |
| | -Ca | |
| | +/-mechanical force | |
| 110 | 2 | Compare the system with and without application of mechanical force in presence of Ca in culture medium. Find genes differentially expressed under the influence of mechanical force. |
| | +Ca | |
| | +/-mechanical force | |
| 111 | 1 | Compare the system with and without application of mechanical force. Find genes differentially expressed under the influence of mechanical force (with prostaglandin synthesis inhibited by indomethacin). |
| | +Indomethacin | |
| | +/- mechanical force | |
| 107 | 4 | Compare the system with and without PGE2 treatment (to mimic mechanical force) |
| | +/- PEG2 | |
| 116 | 5 | Compare the system with and without PGE2 treatment (to mimic mechanical force) |
| | +/- PEG2 | |

### Calvaria treated with indomethacin and mechanical force

Primary cell cultures derived from 17-19 days old rat embryos. The cultures were prepared by trypsin - EDTA digestion of Calvaria including the periosteum. The cell cultures were grown in MEM medium with 10% FCS for 5-6 days to reach confluency.

At this time 10 microliter which contains 20ug of indomethacin were added to culture dishes which had 4ml of medium. 20 minutes later the dishes were activated mechanically. The mechanical activation is carried out e.g. by expanding an orthodontic expansion screw which is attached to two pieces of solid acrylic resin glued to the outer surface of the cell culture dish. The expansion deforms the dish irreversibly. Same cultures which were not treated with indomethacin and activated mechanically were as positive control.

The rationale is because mechanical activation stimulates *de novo* synthesis of prostaglandins. Indomethacin inhibits synthesis of prostaglandins.

### Calvaria. grown in the presence of Ca activated by mechanical force

Calvaria grown in the presence of Ca were activated by mechanical activating devices at confluency. The cultures were prepared as described above. The cells were grown in MEM medium which normally includes 1 mM of calcium: - from the seeding of the cells until confluency and mechanical activation.

### Calvaria grown in absence of Ca treated with mechanical force

The cultures were grown in MEM medium which was calcium free. The calcium in this medium was 0.25 mM because it consisted of 10% FCS (serum contains 2.5 mM of calcium). After 3-4 days the medium was replaced by regular MEM which included normal calcium concentration.

The rationale of this experiment is mainly because transudation mechanism of mechanical activation like prostaglandin synthesis and action is calcium dependent. The cultures in low calcium suppress the proliferation of fibroblasts and allow growth and differentiation of osteoblasts in culture. Therefore, the strategy is to start with low calcium medium and after 3-4 days to booster growth by switching to normal calcium medium (1 mM Ca).

### Calvaria treated with prostaglandin PGE2

Primary Calvaria cells were treated with PGE2 in both experiments (both are similar primary cultures prepared at different date, and treated identically).

PGE2 treatment was performed on cultures which reached confluency by adding (treating with) 10 microliter of PGE2 which consists of total of 500ng of PGE2. After 30 minutes the cells were scraped and stored in -70°C.

The rationale: The PGE2 treatment is supposed to mimic the mechanical activation effect.

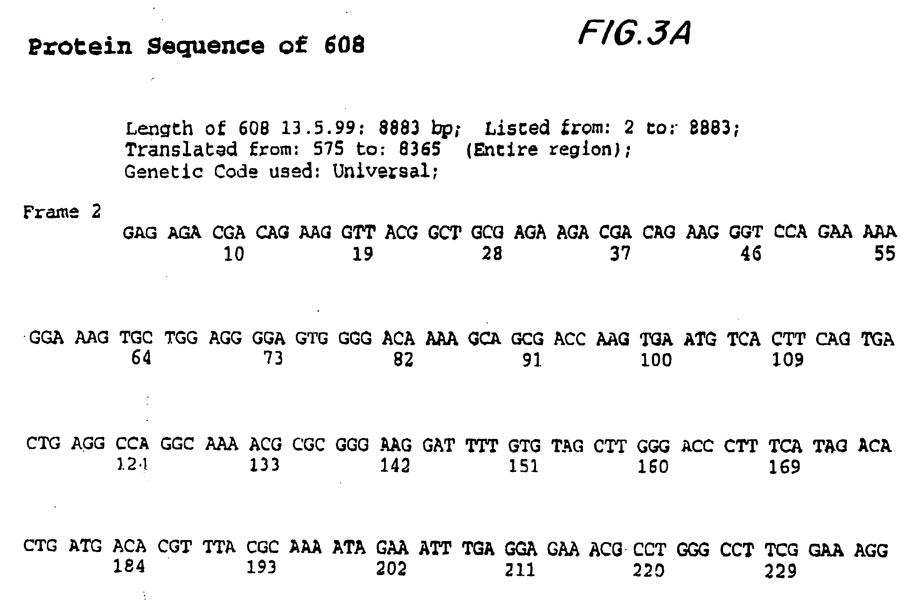

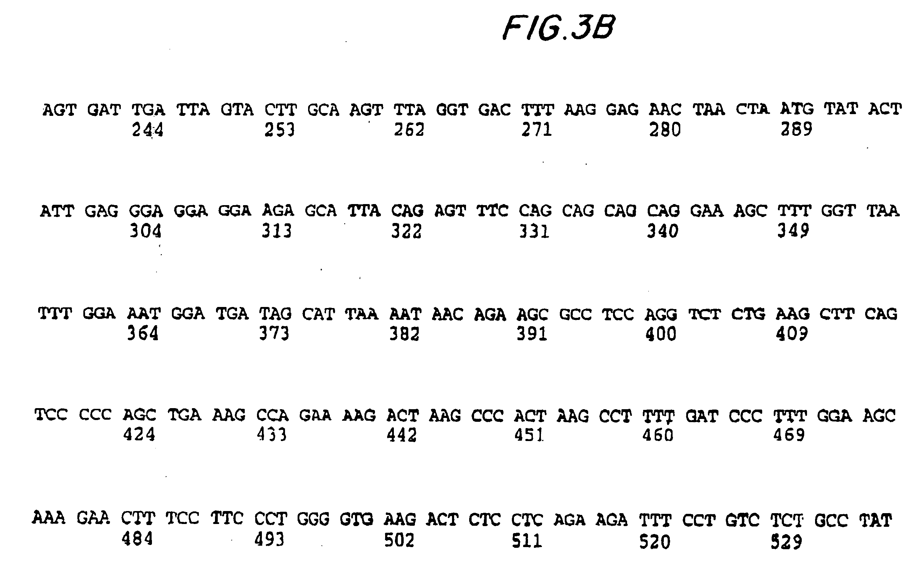

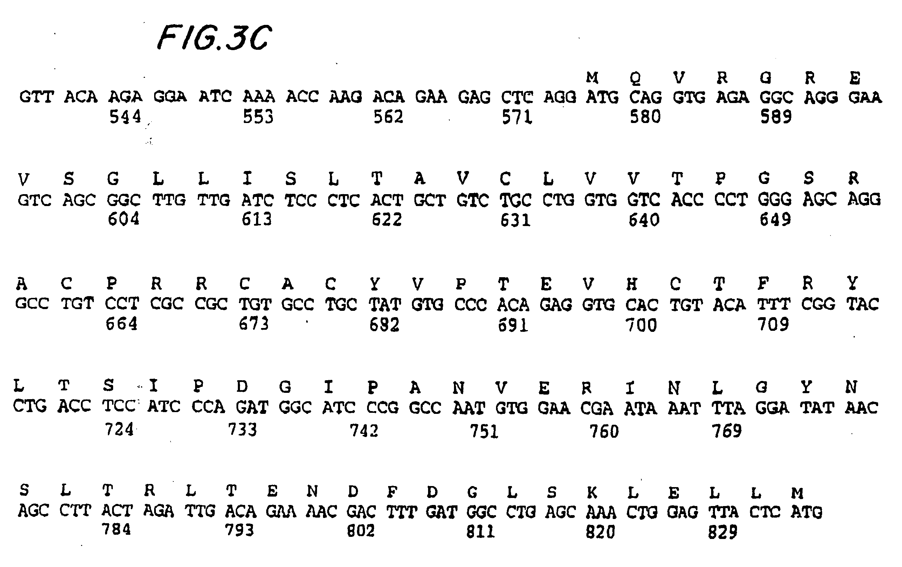

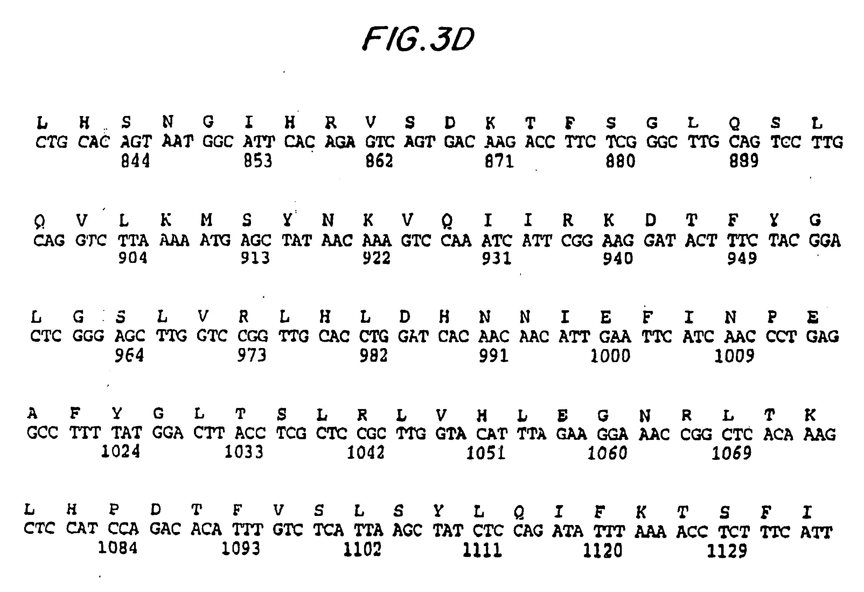

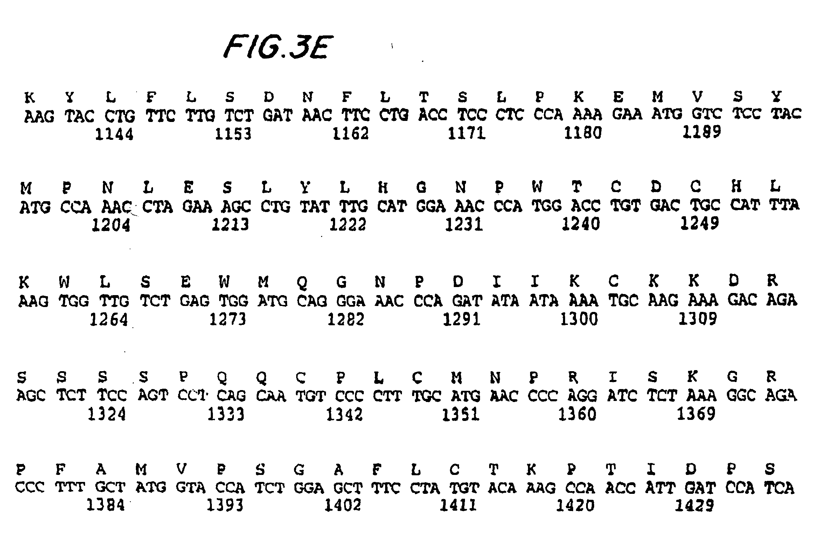

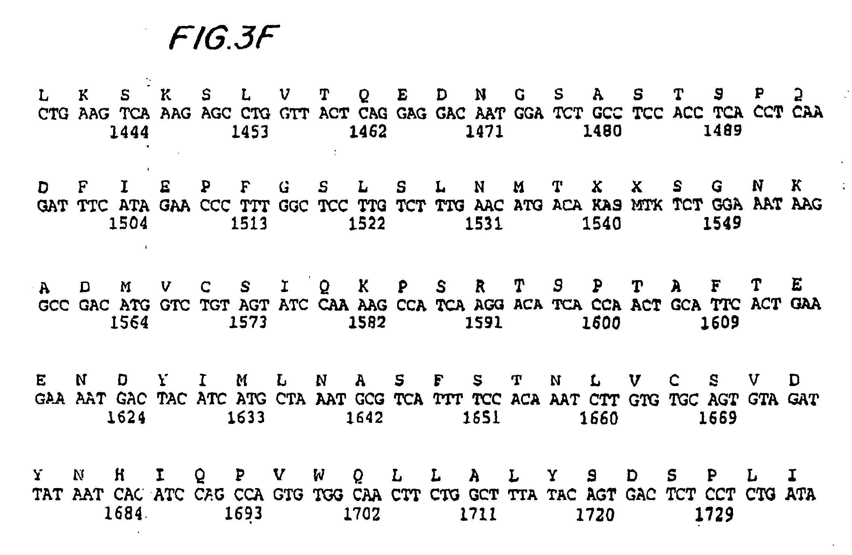

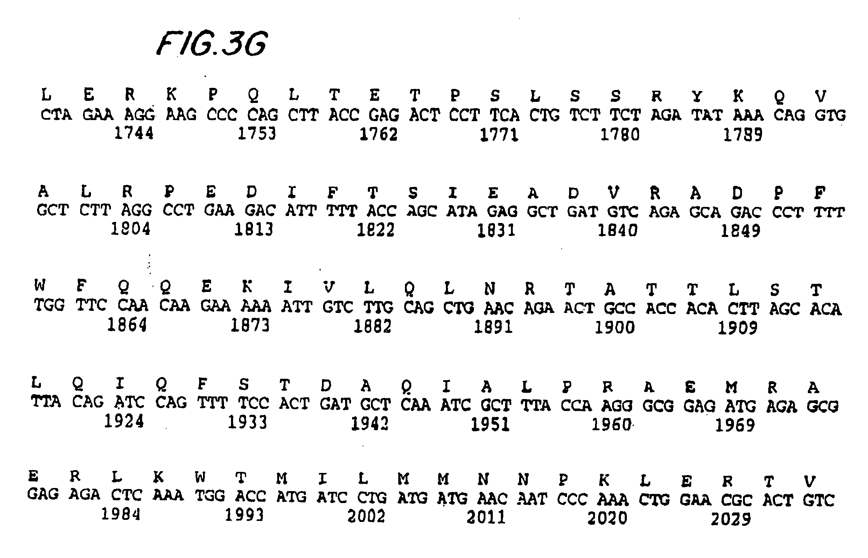

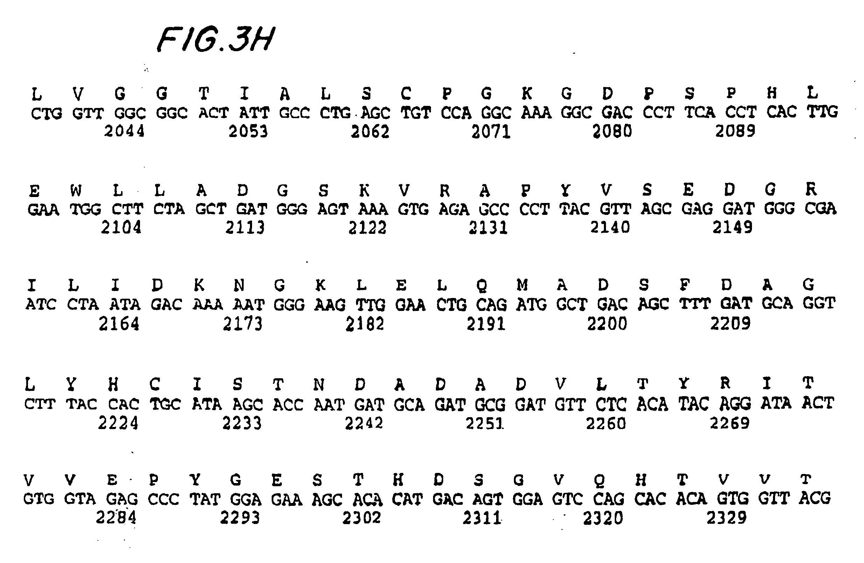

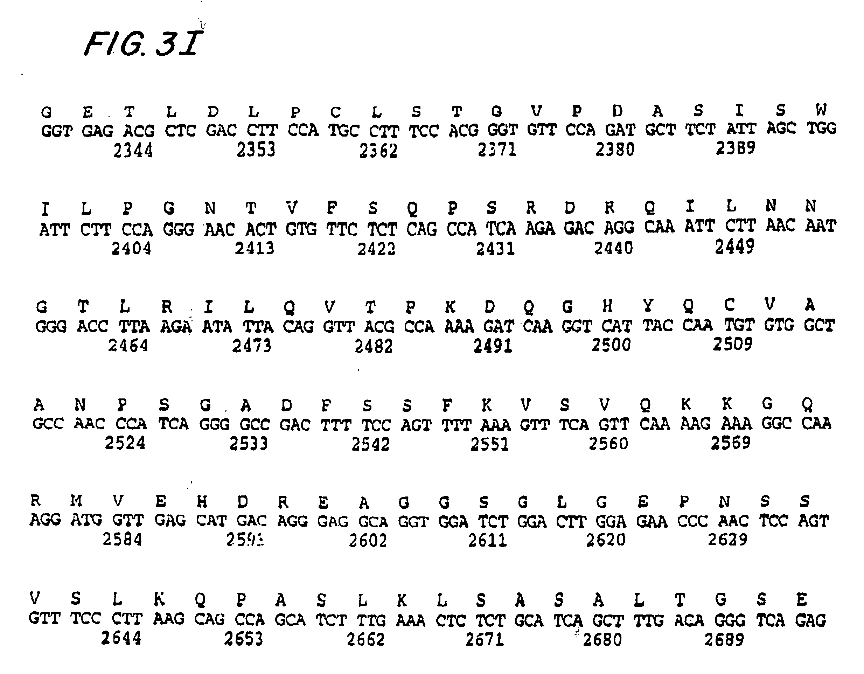

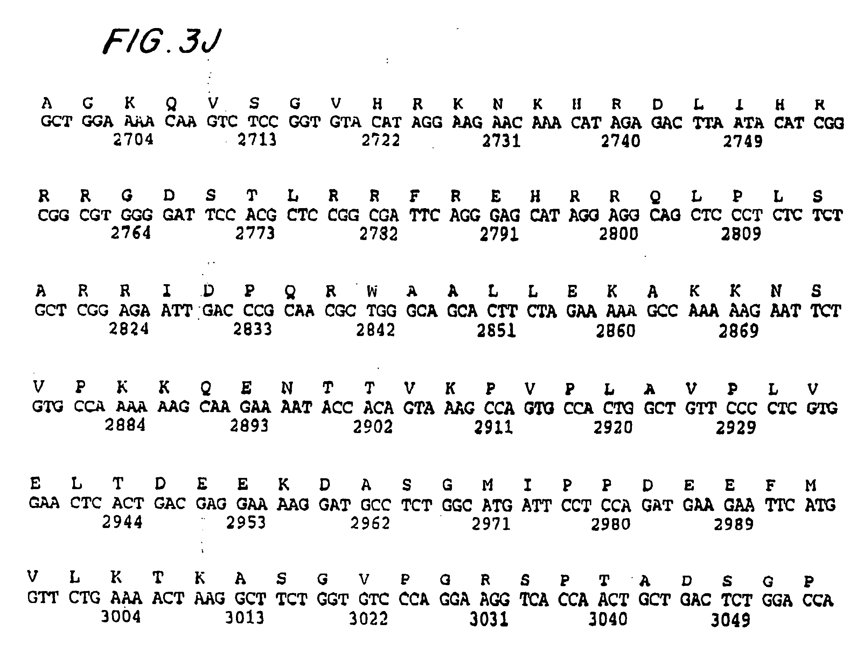

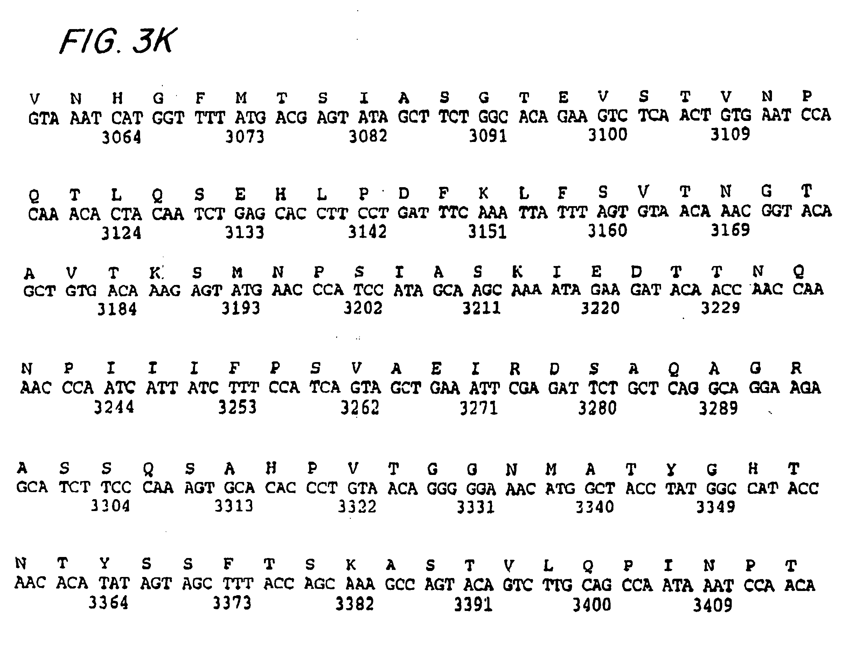

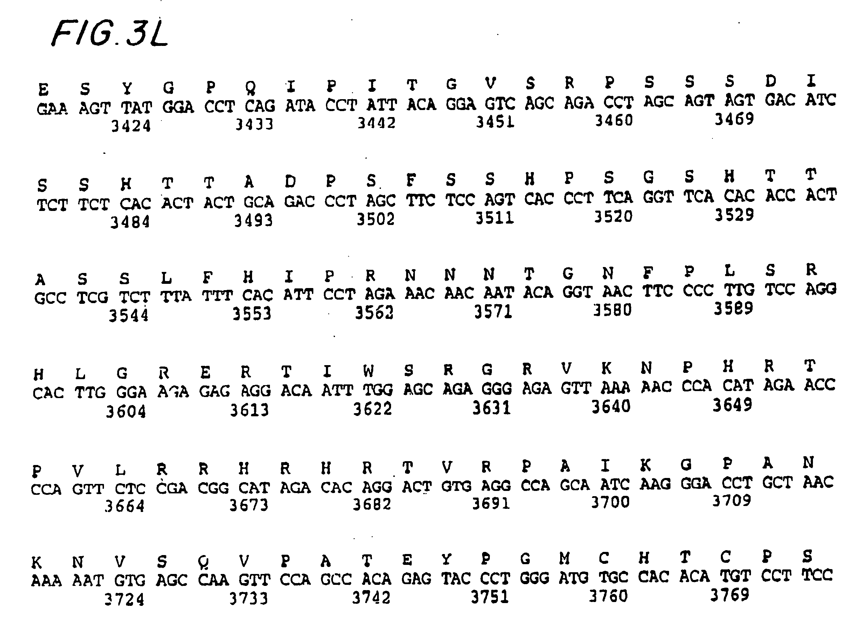

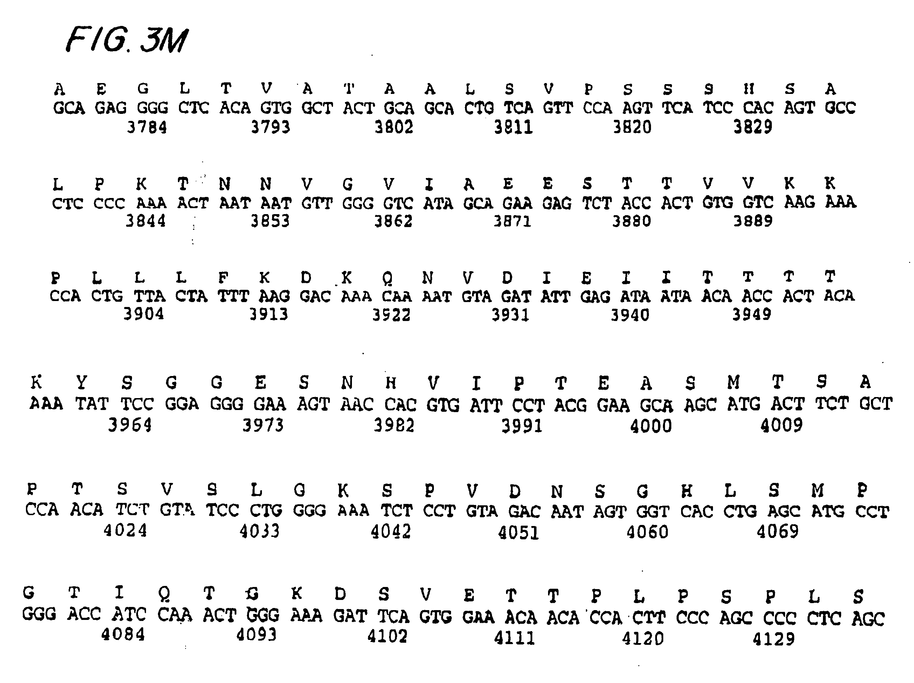

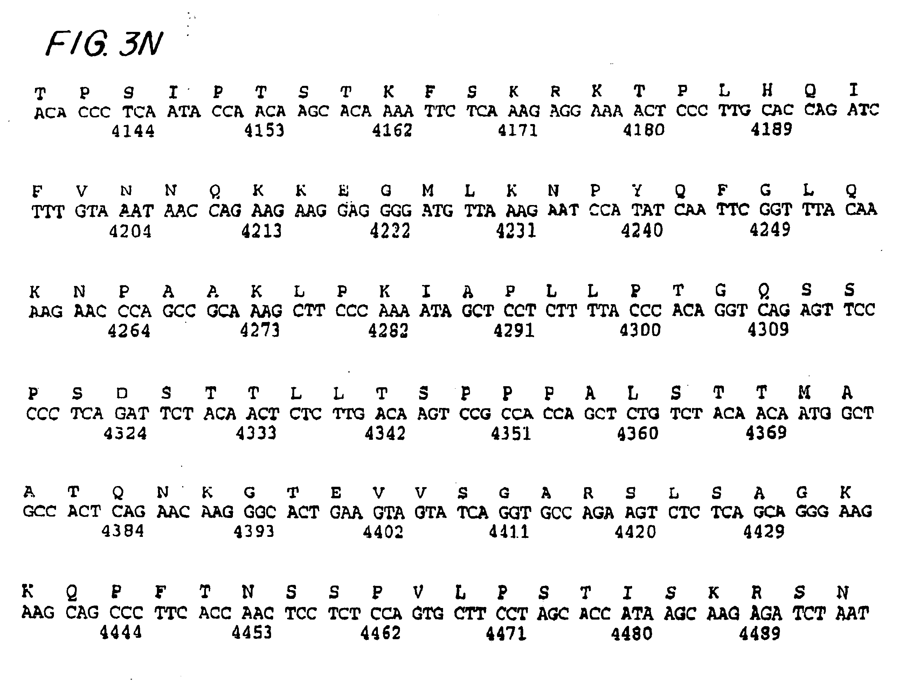

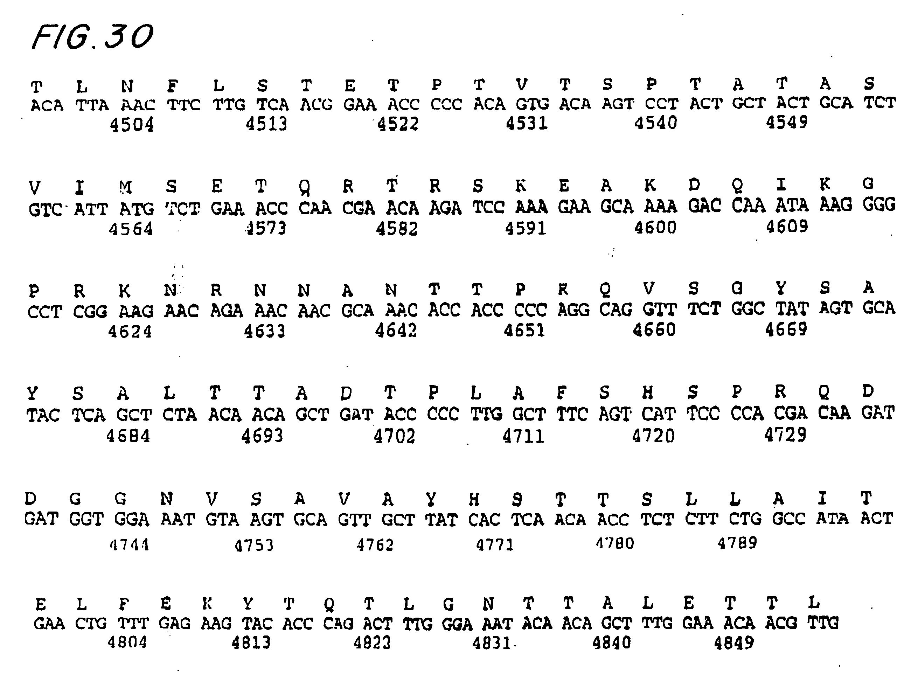

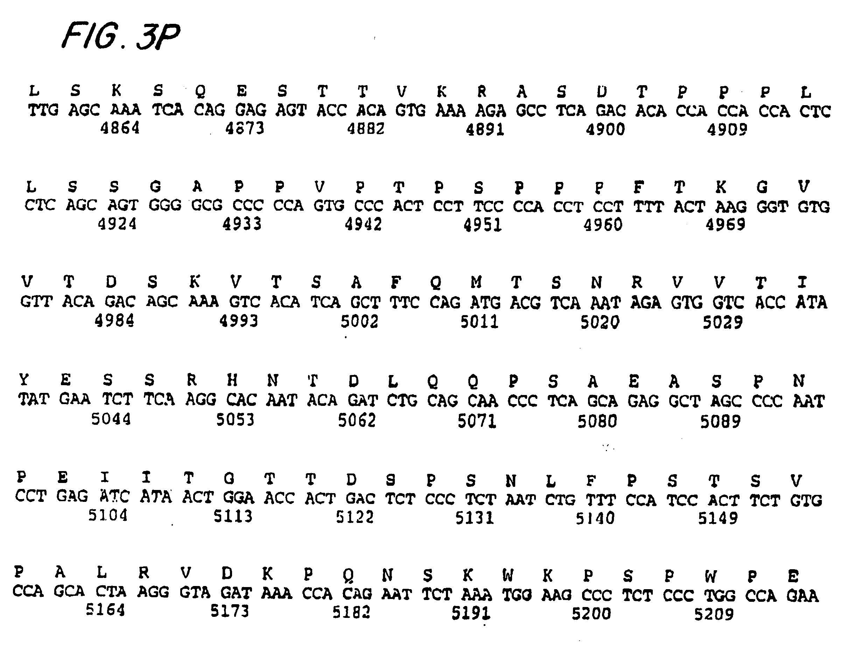

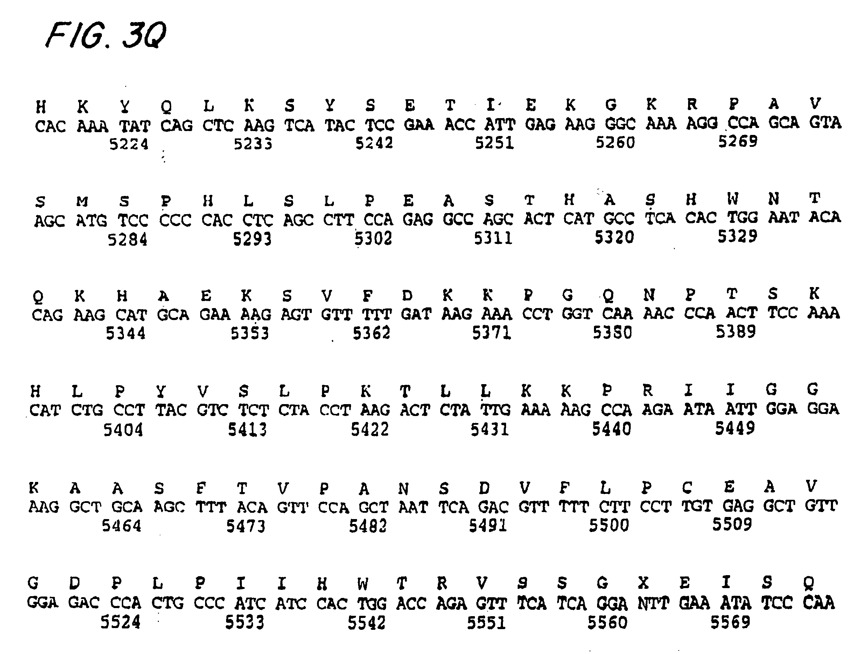

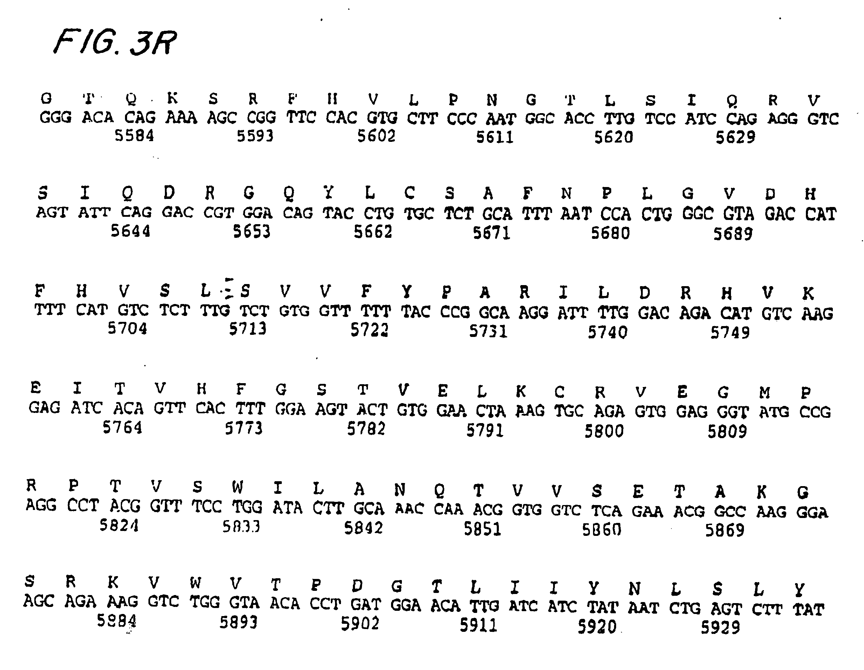

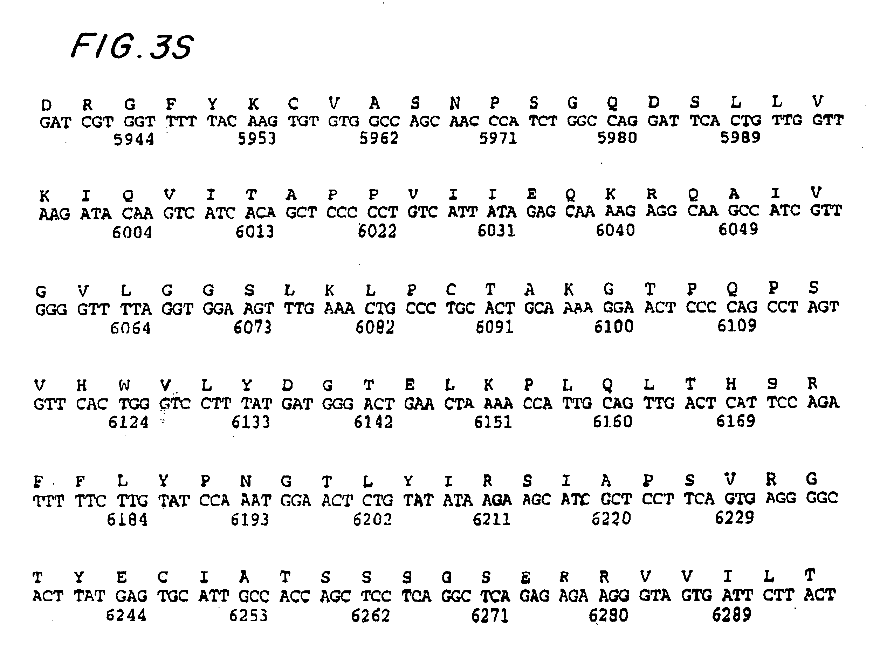

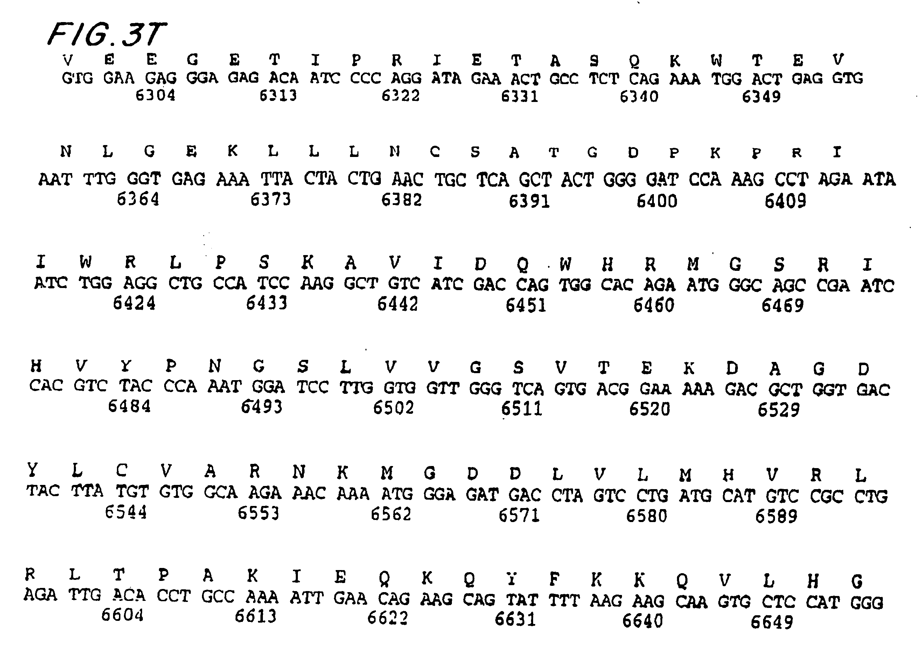

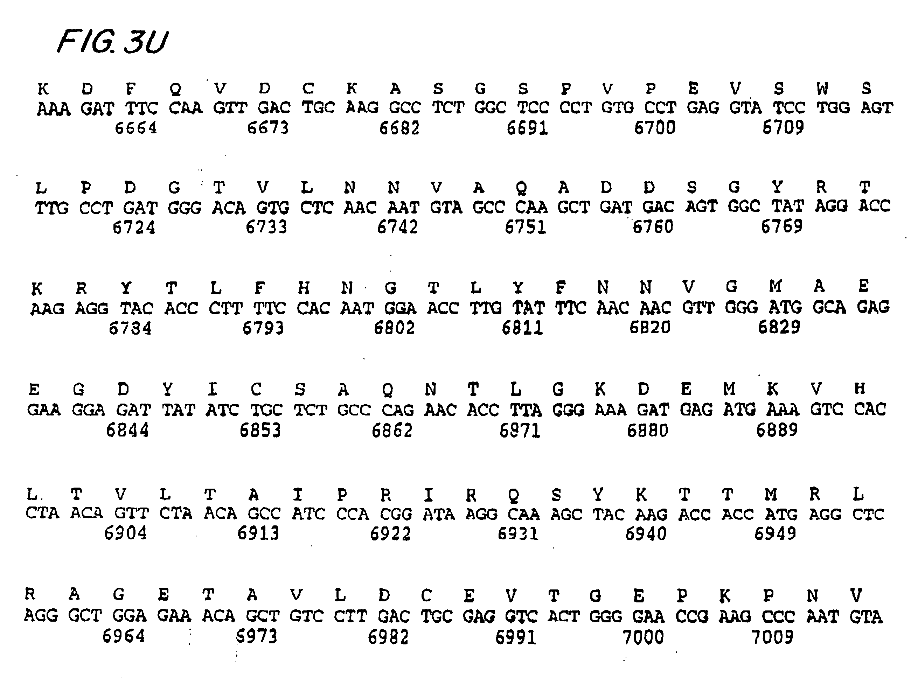

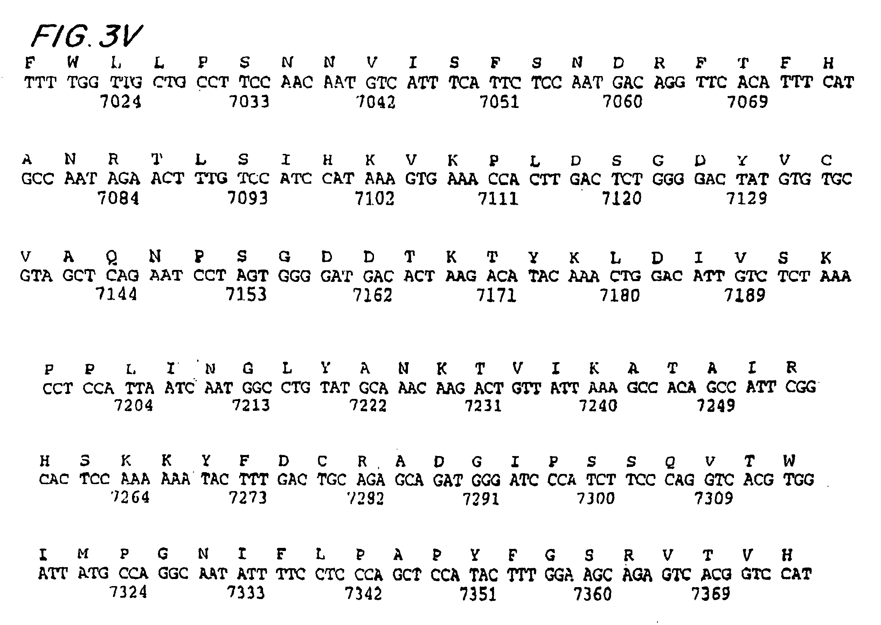

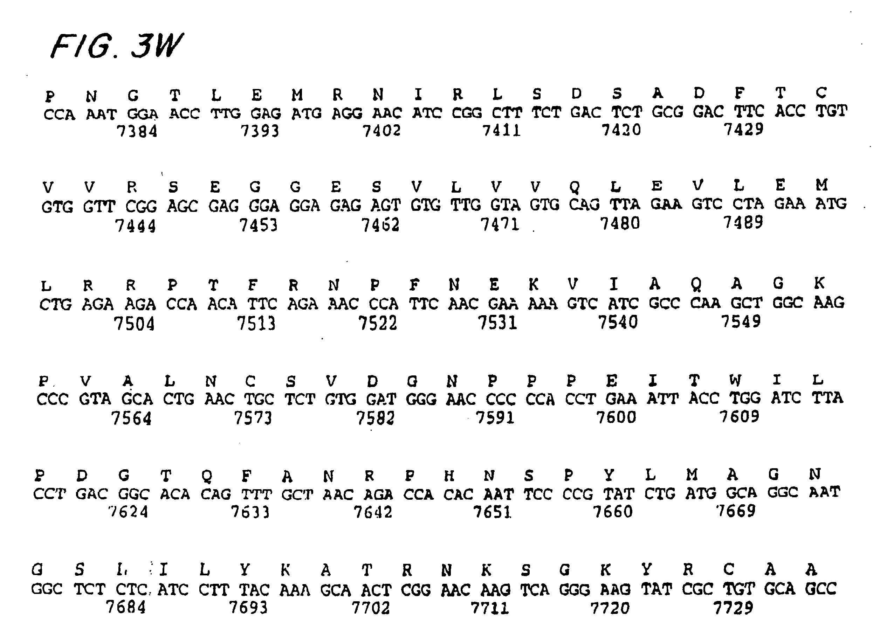

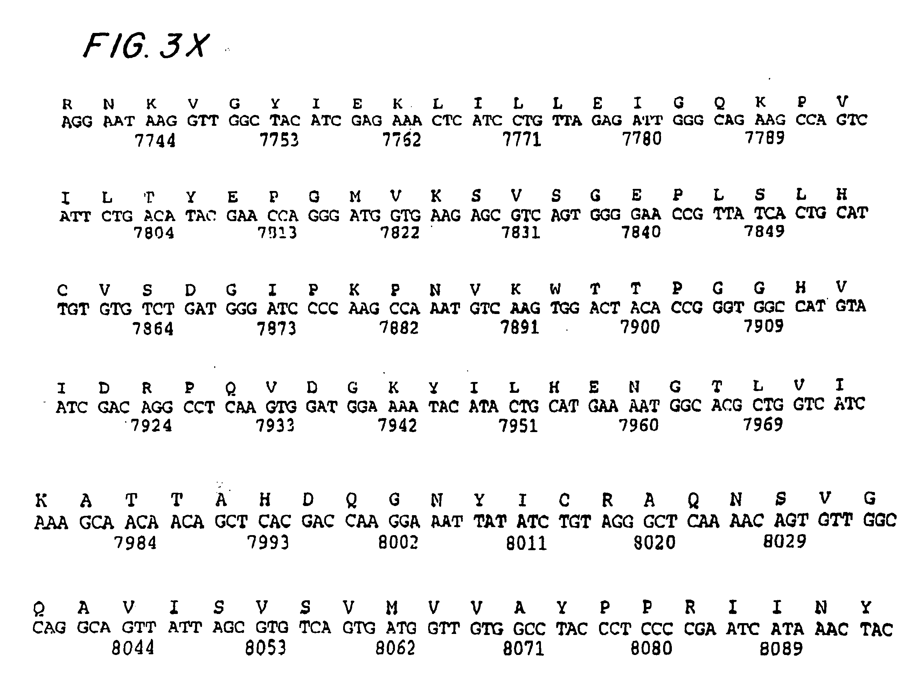

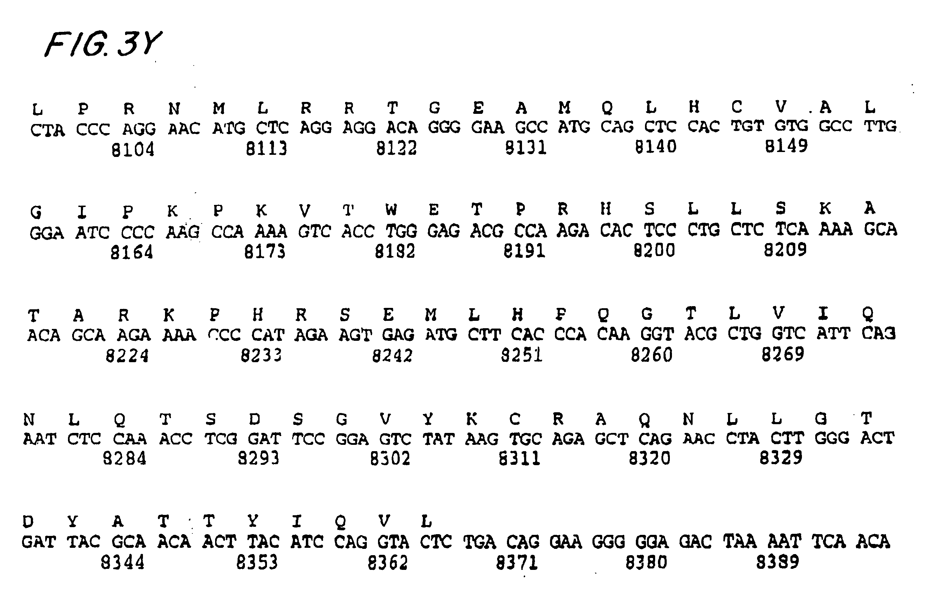

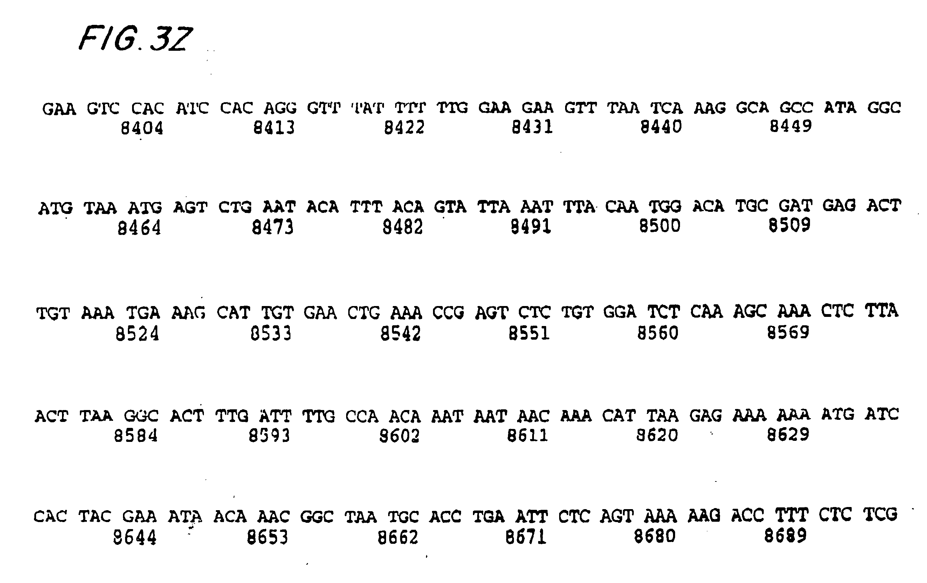

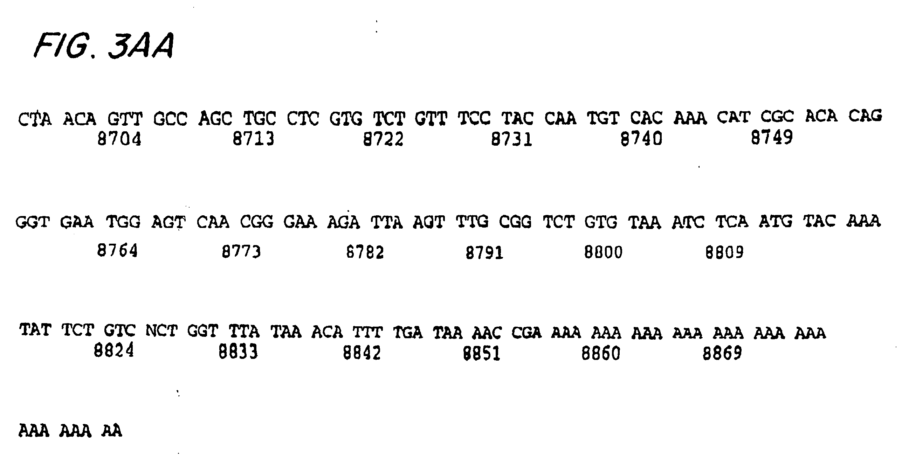

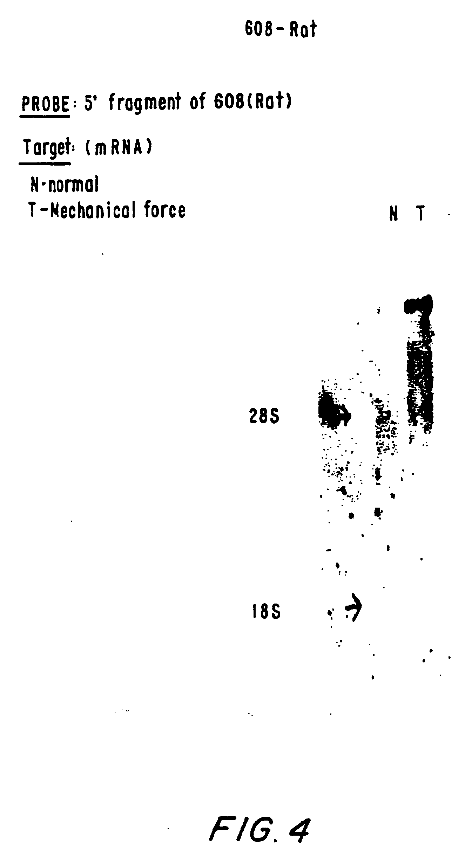

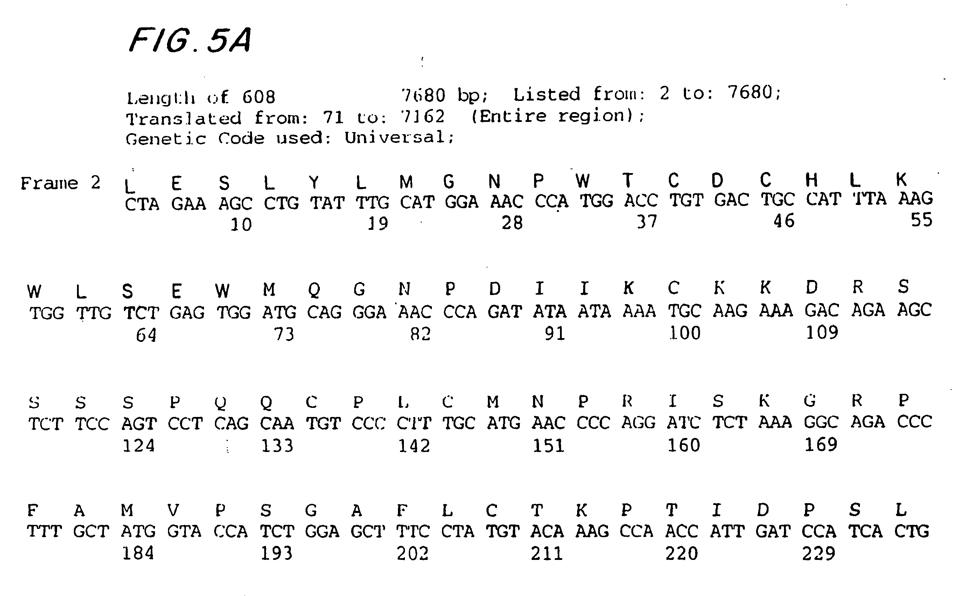

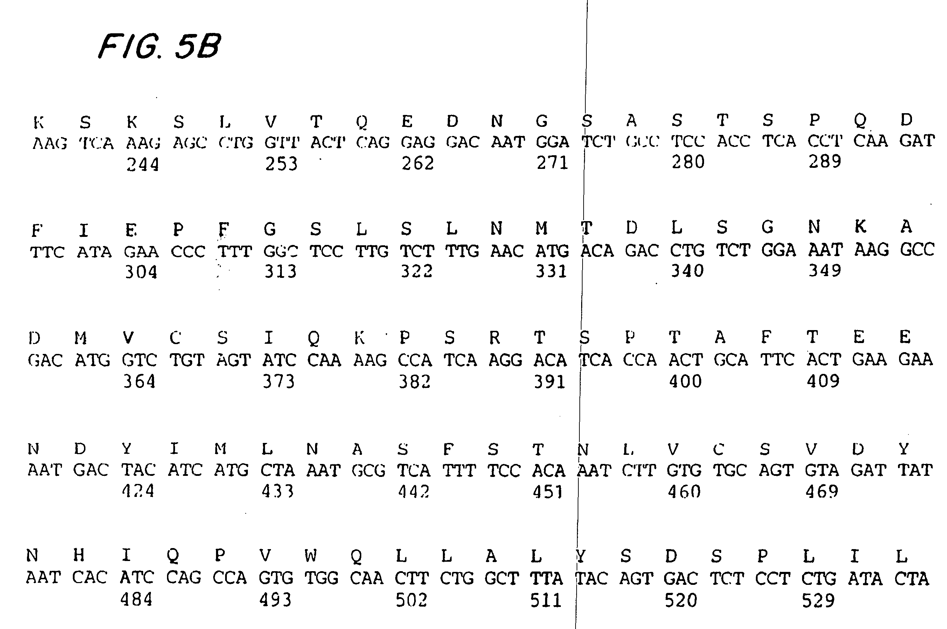

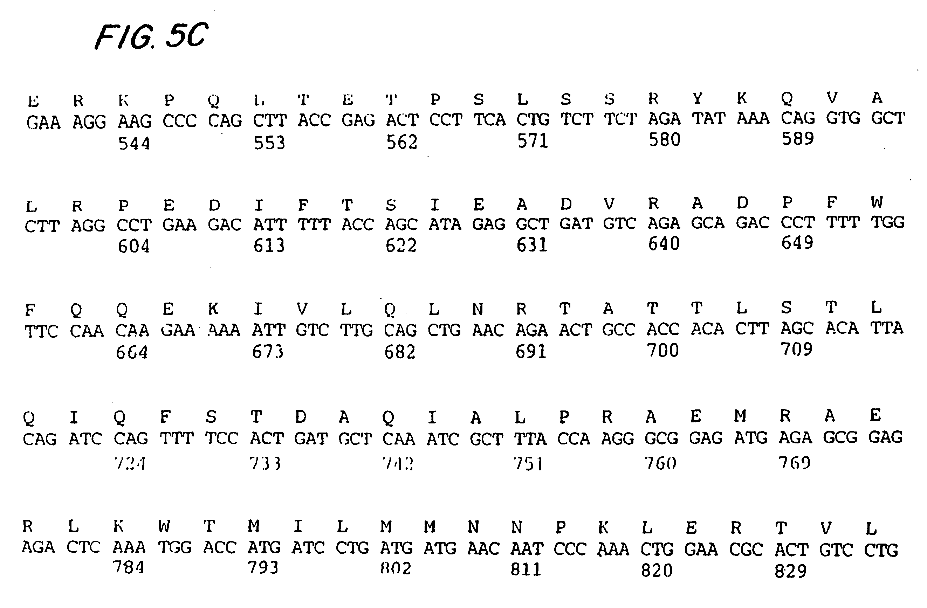

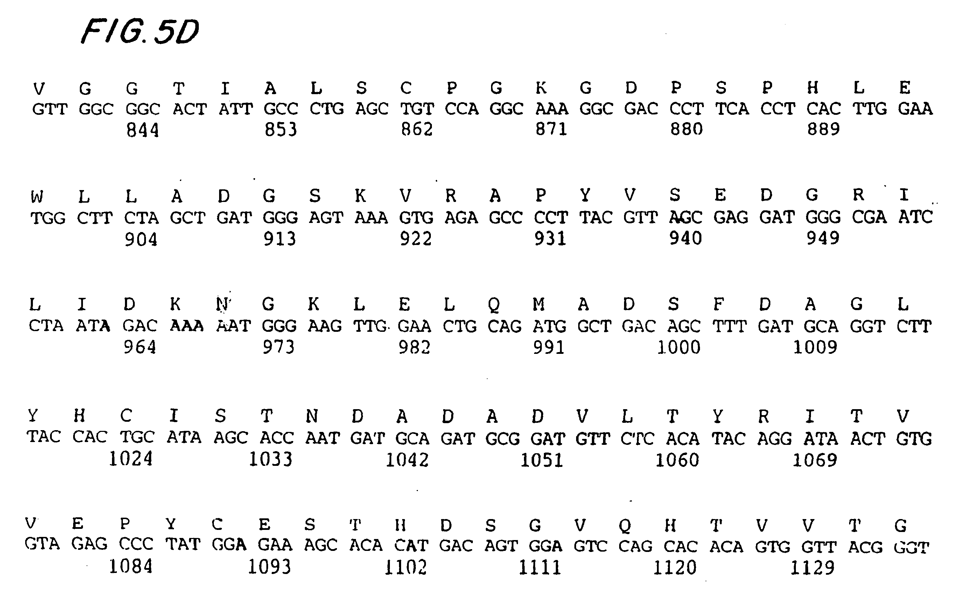

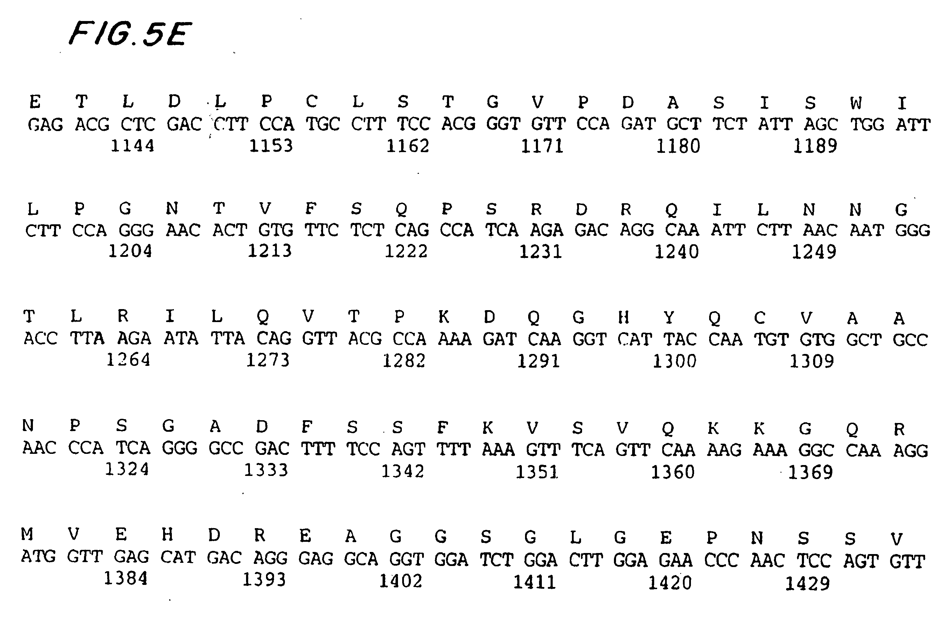

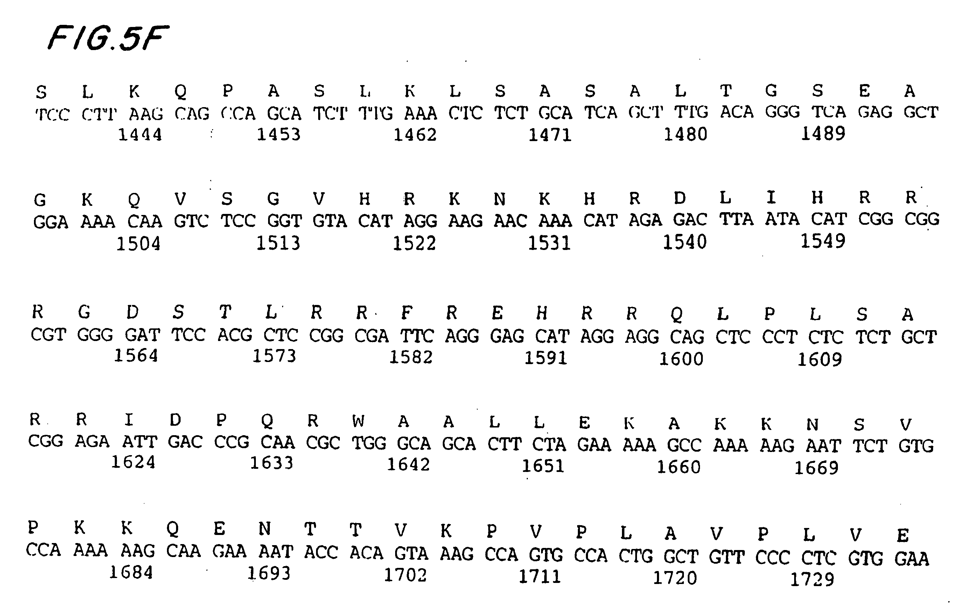

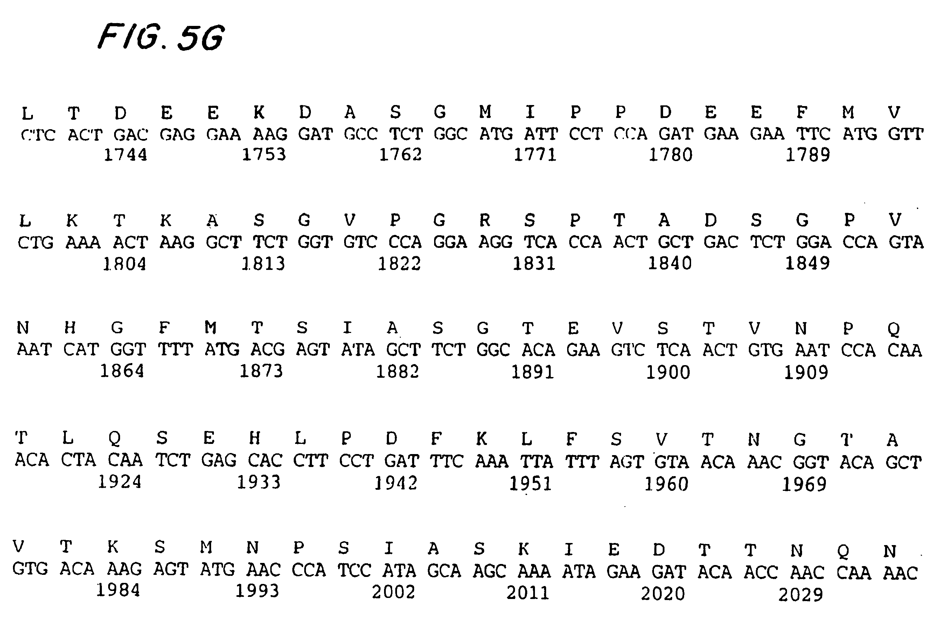

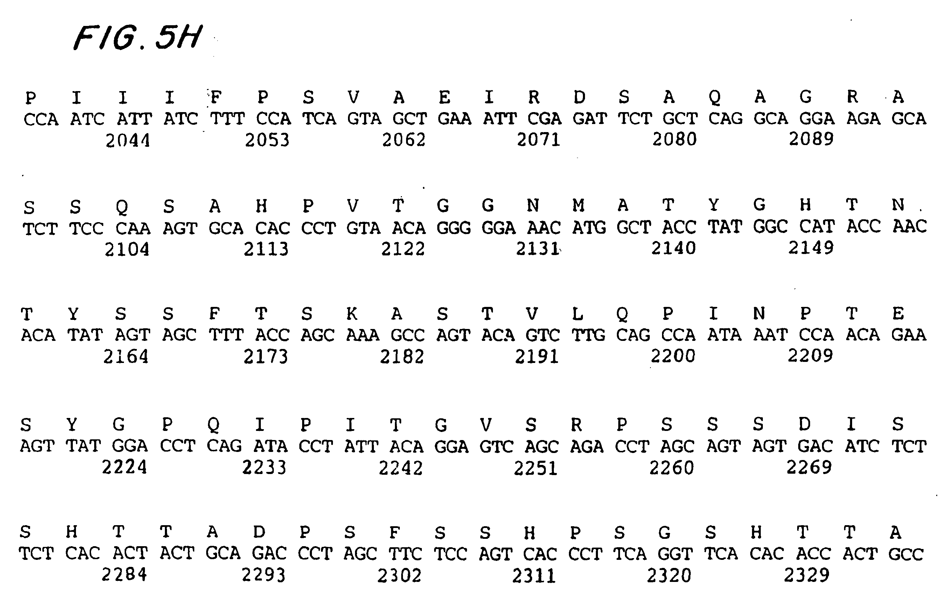

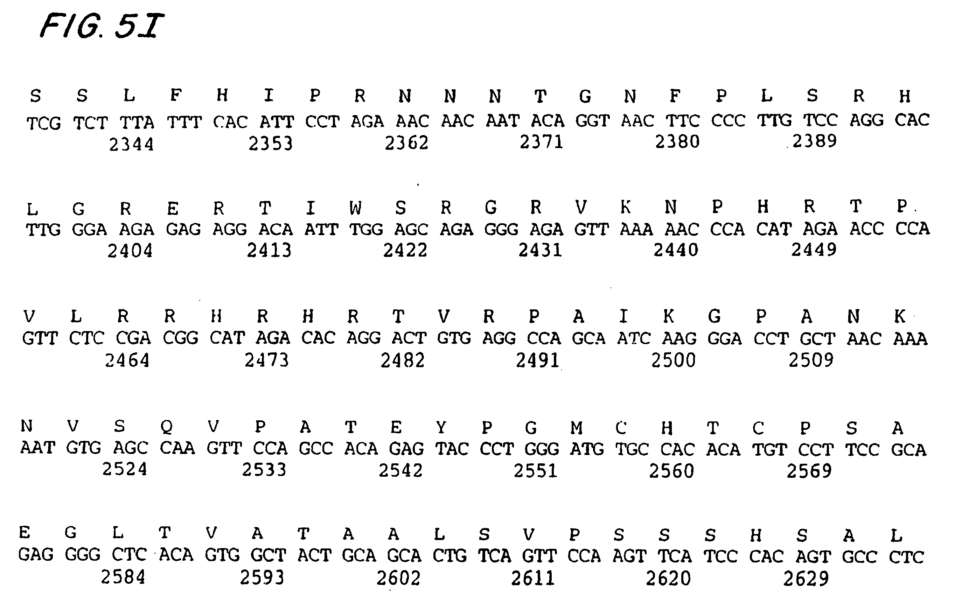

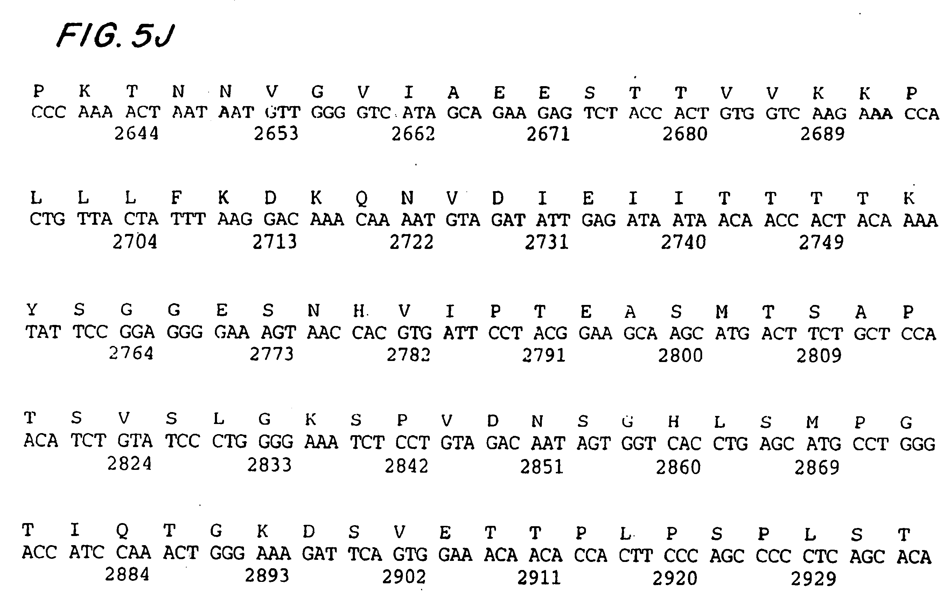

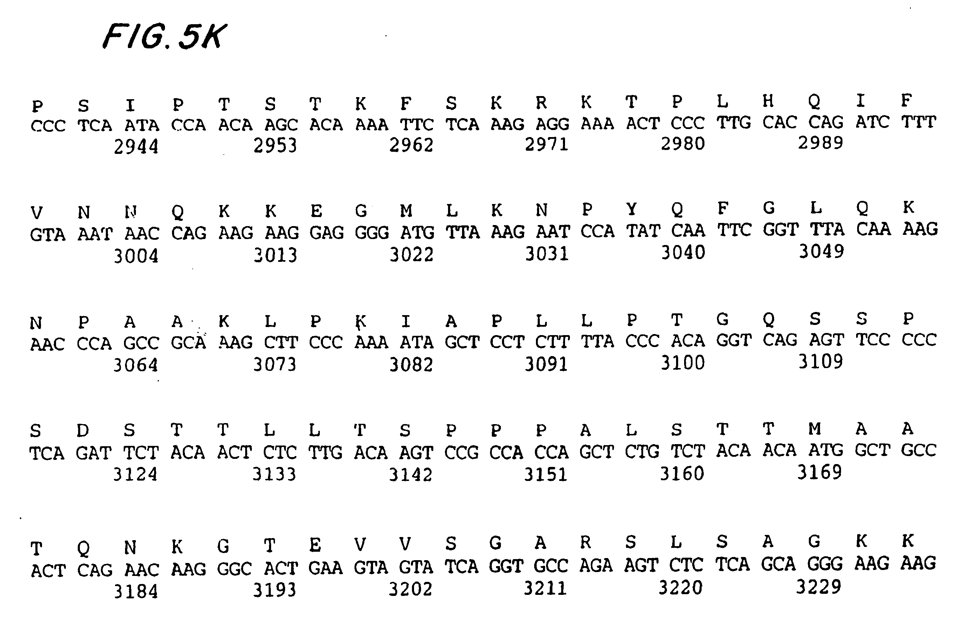

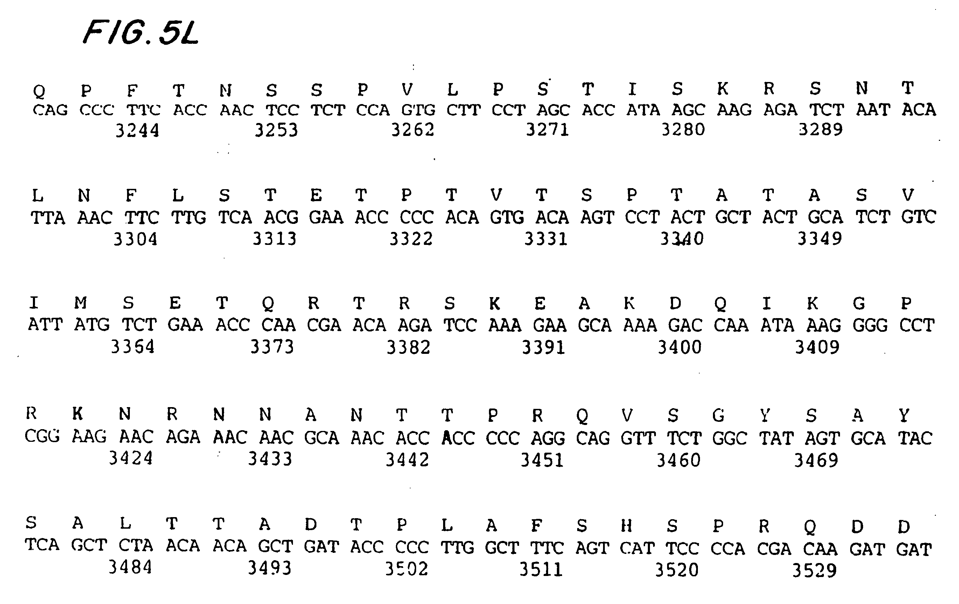

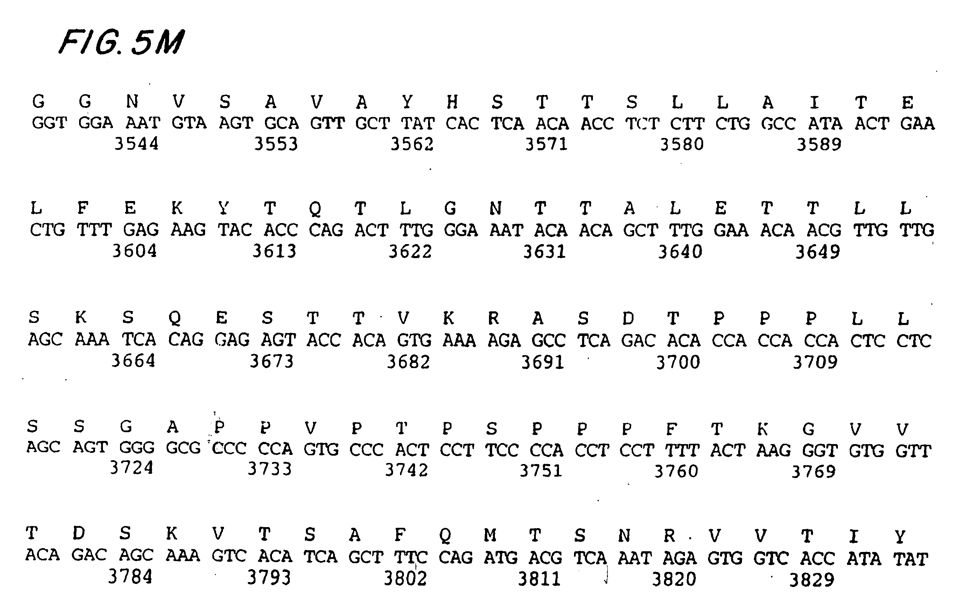

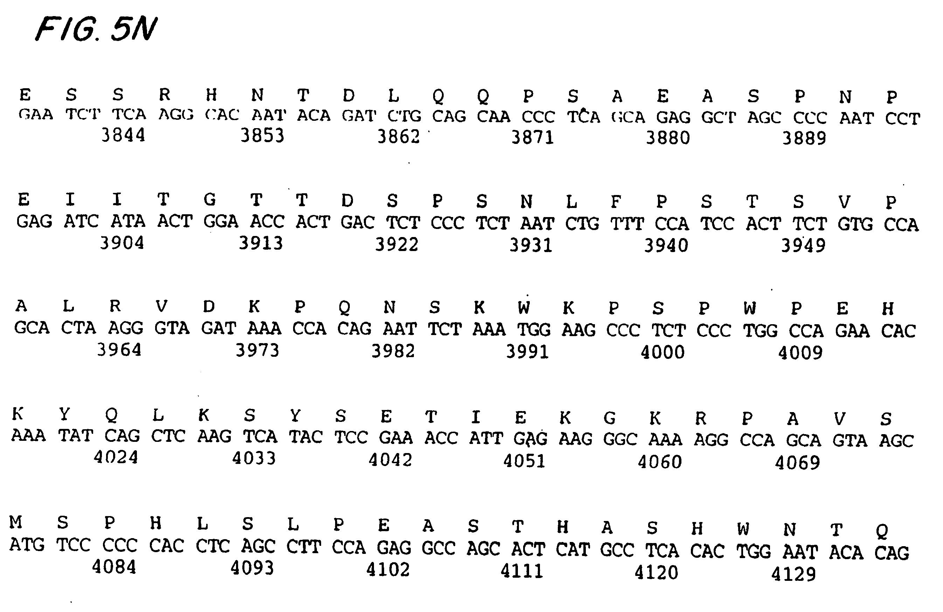

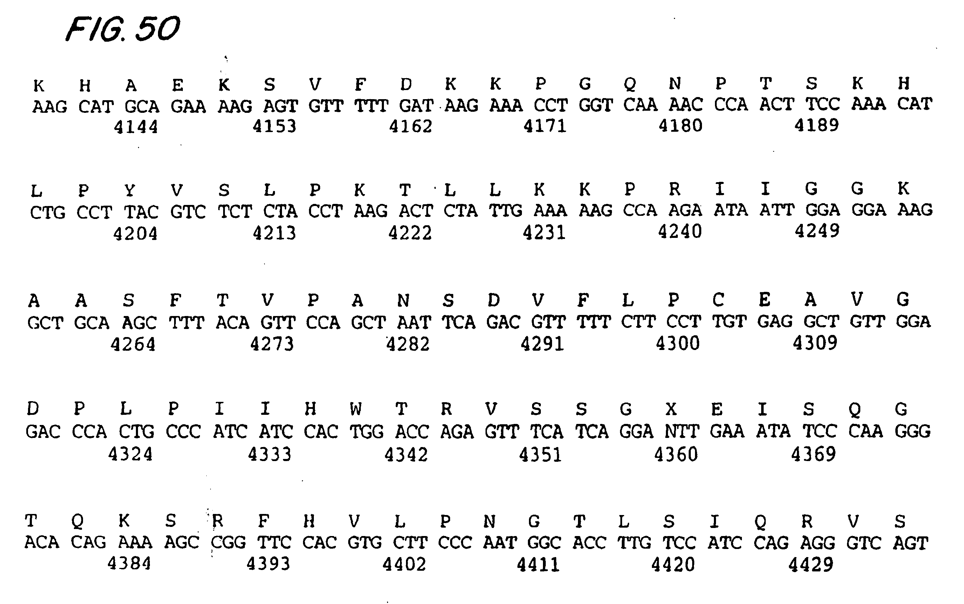

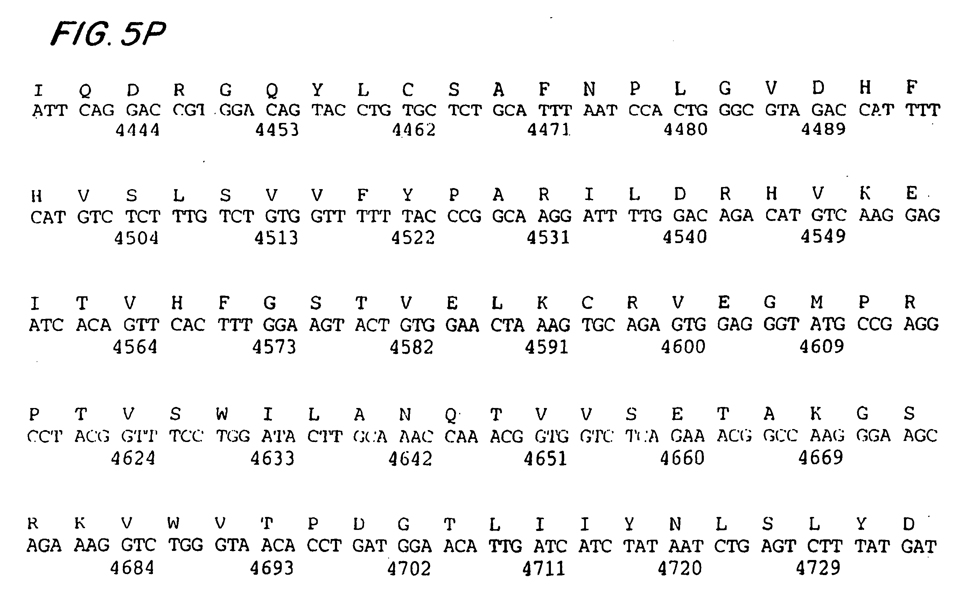

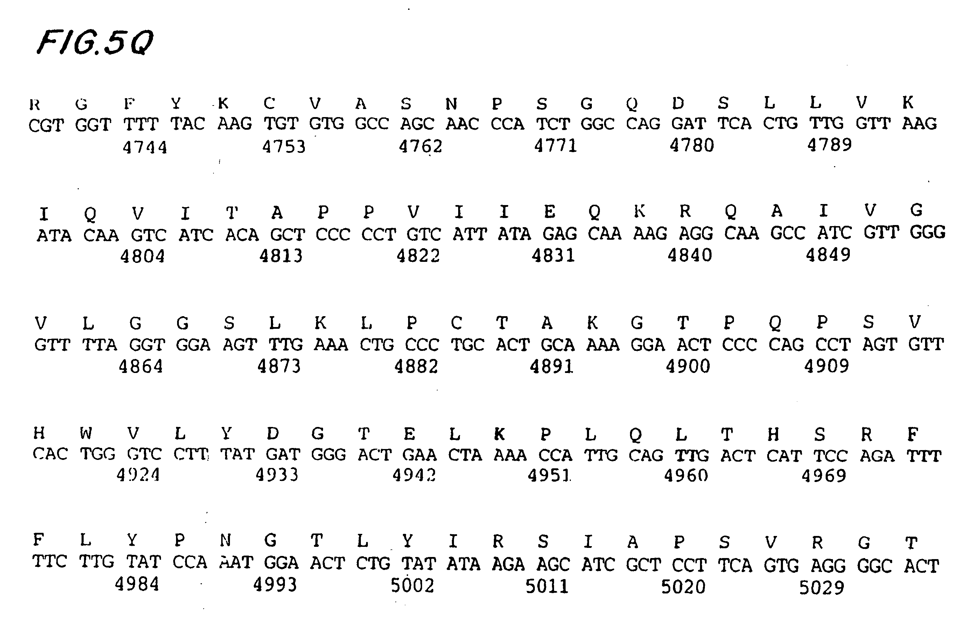

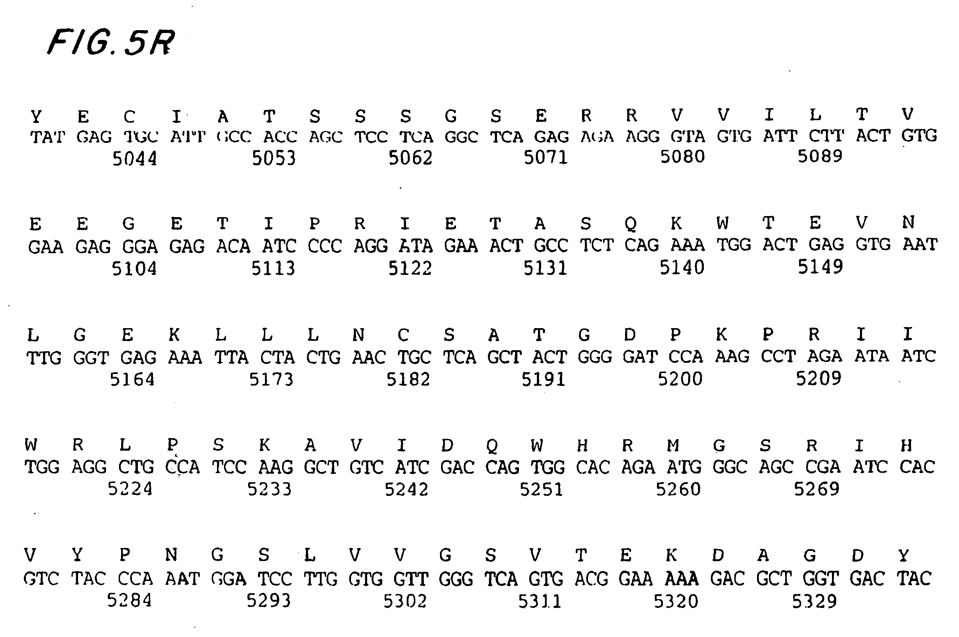

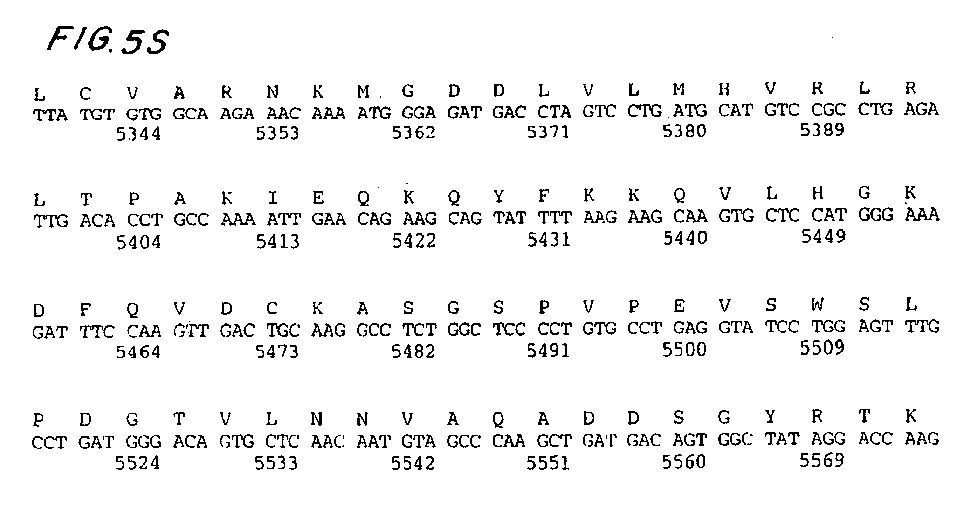

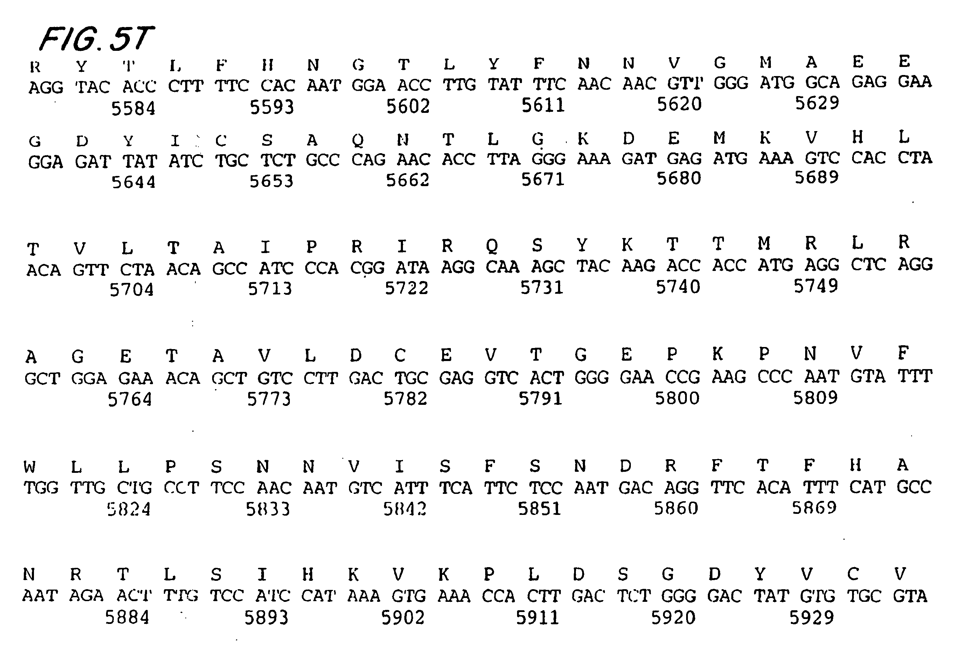

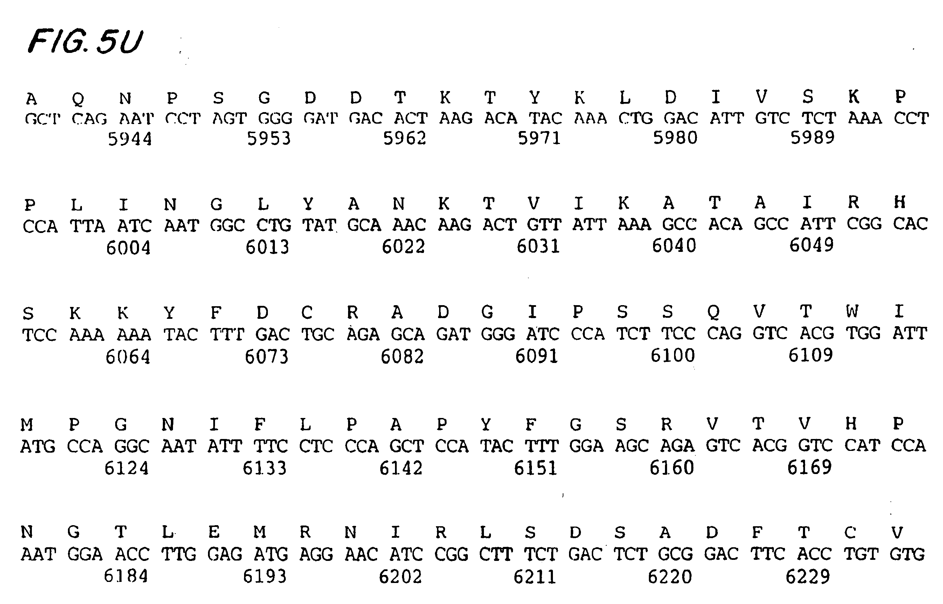

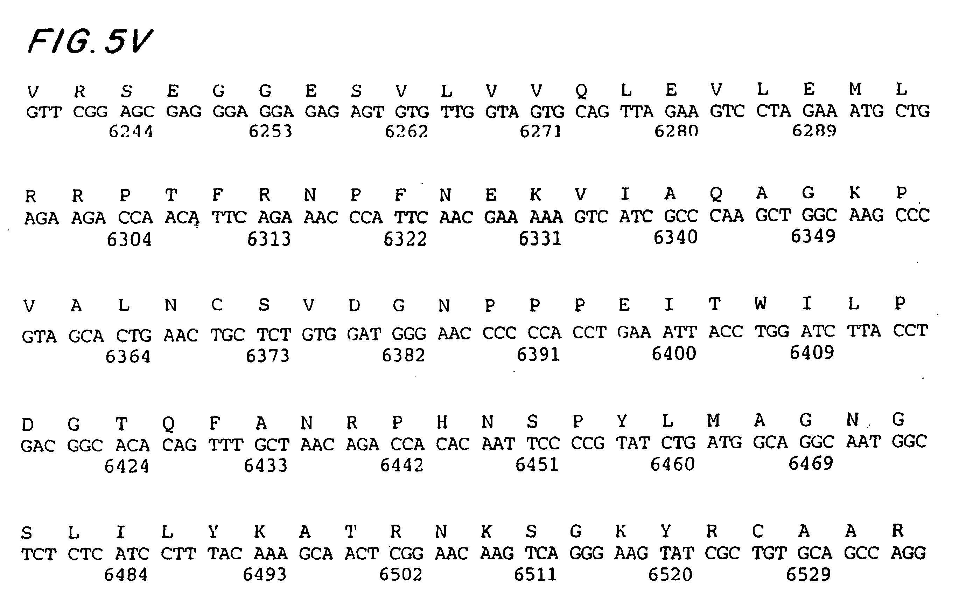

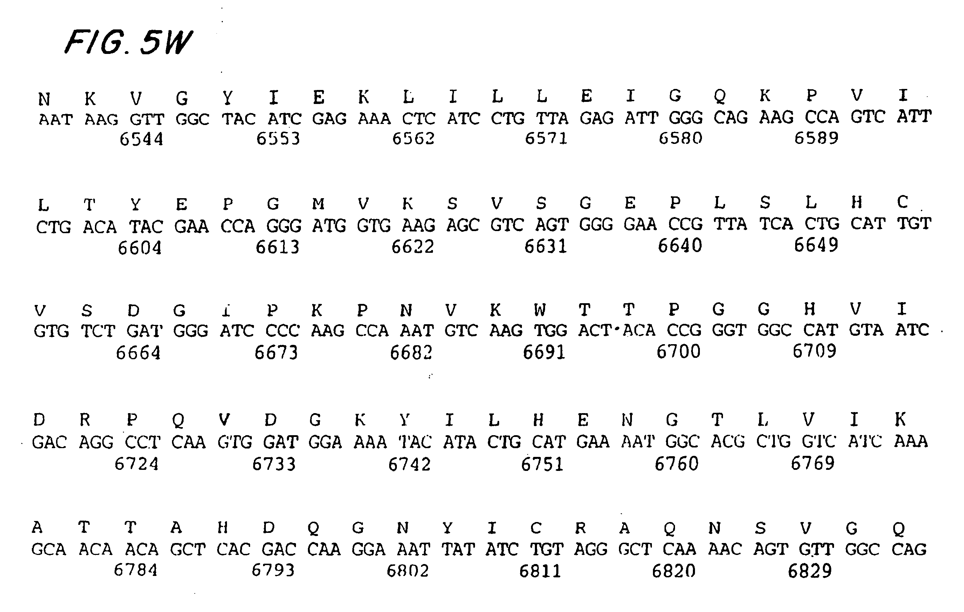

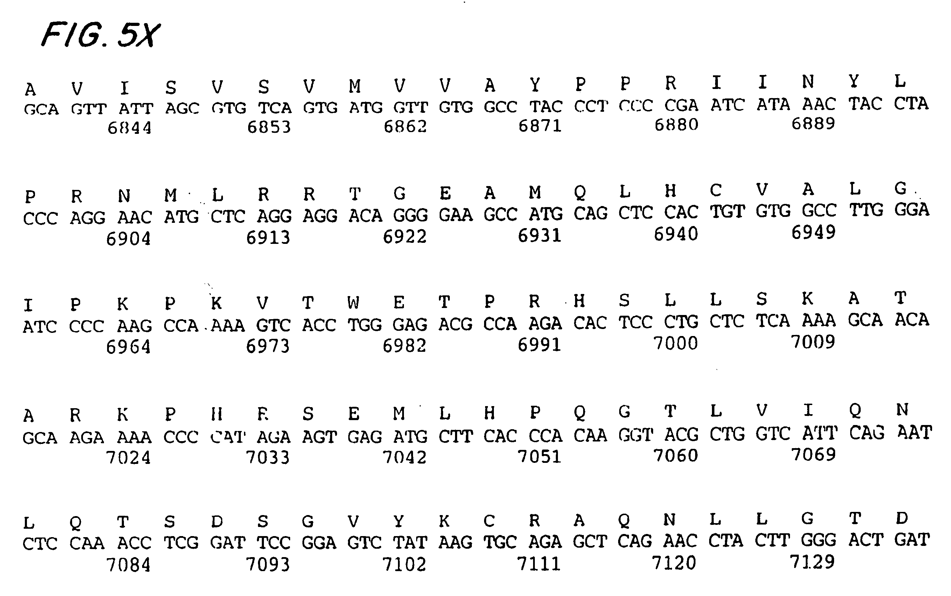

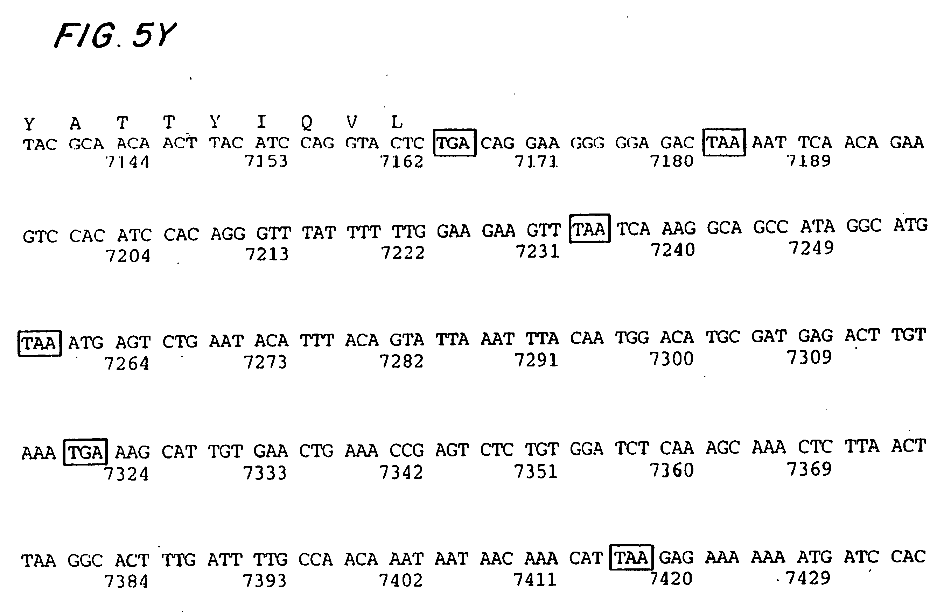

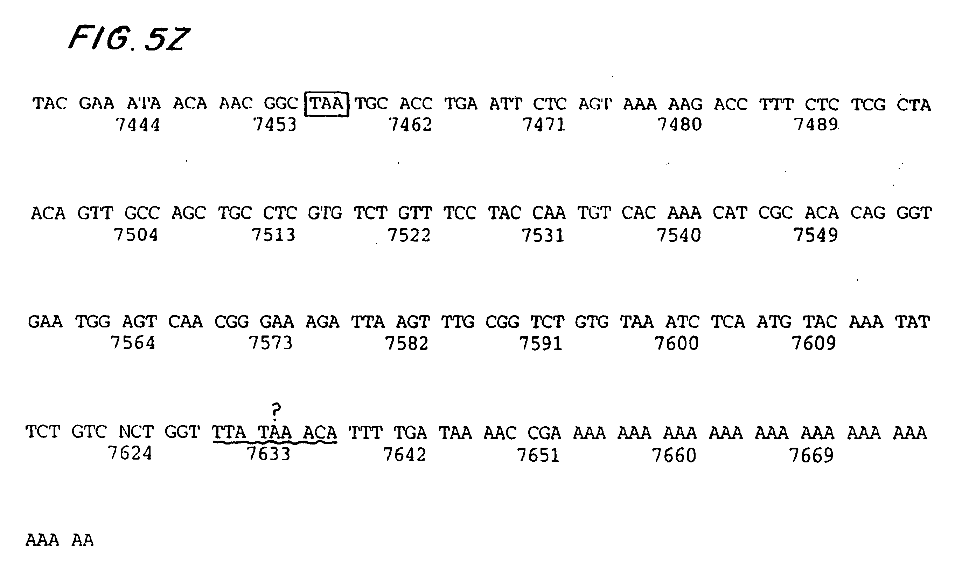

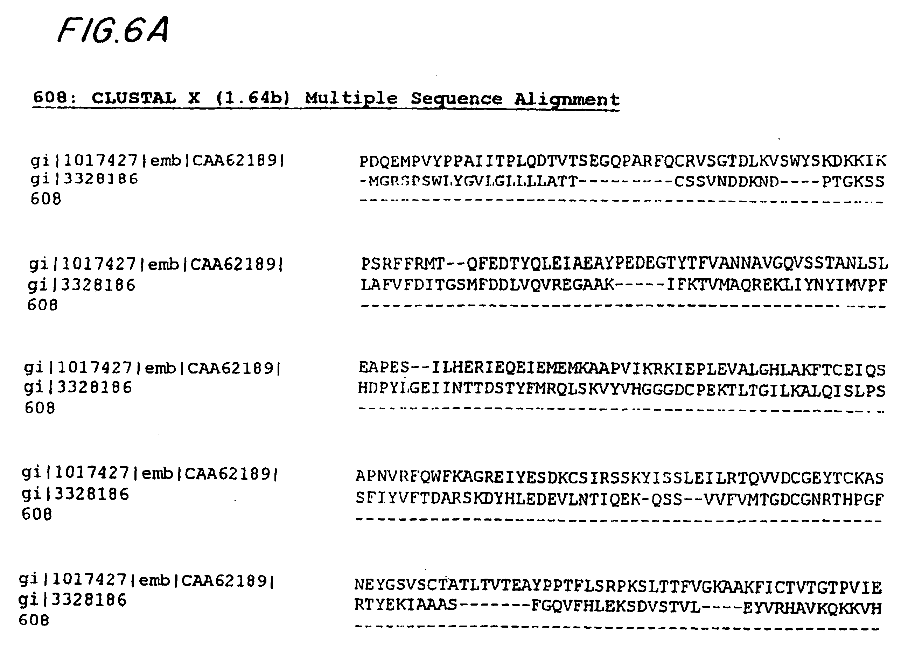

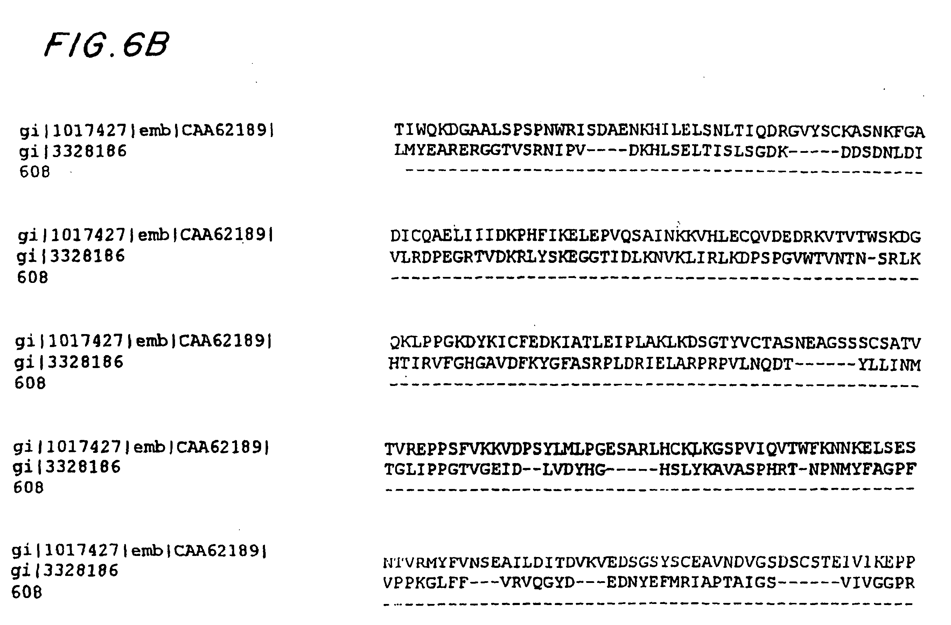

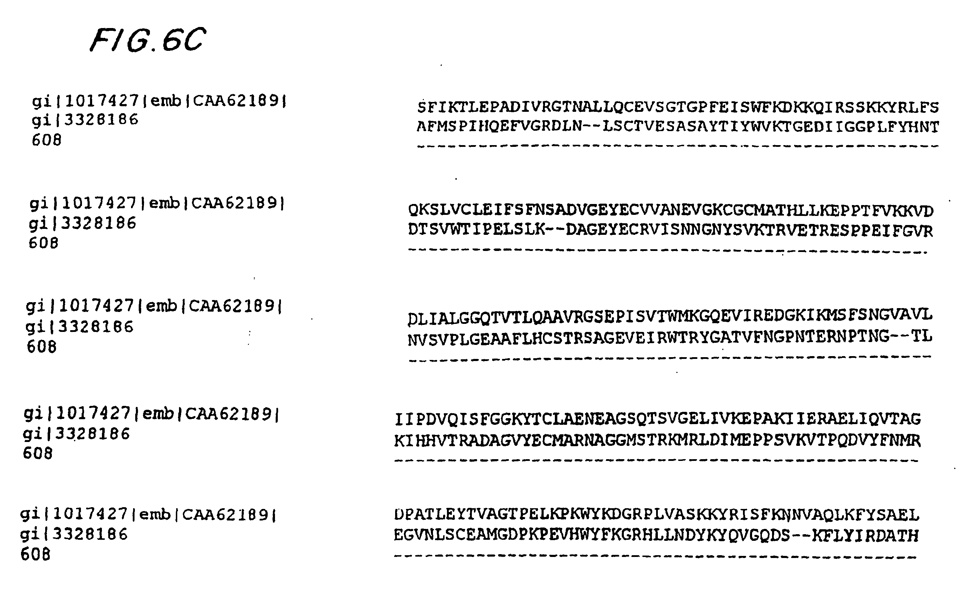

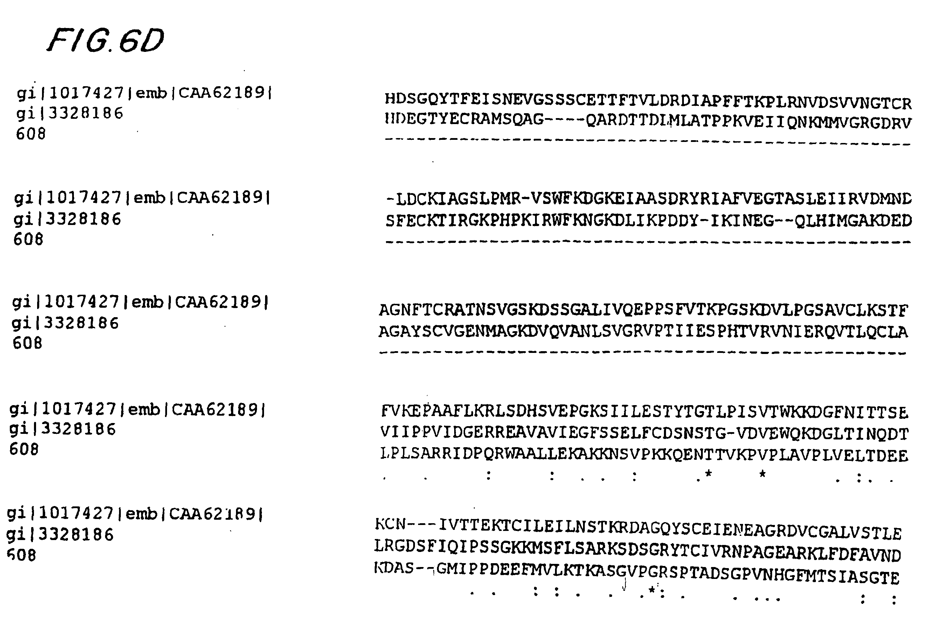

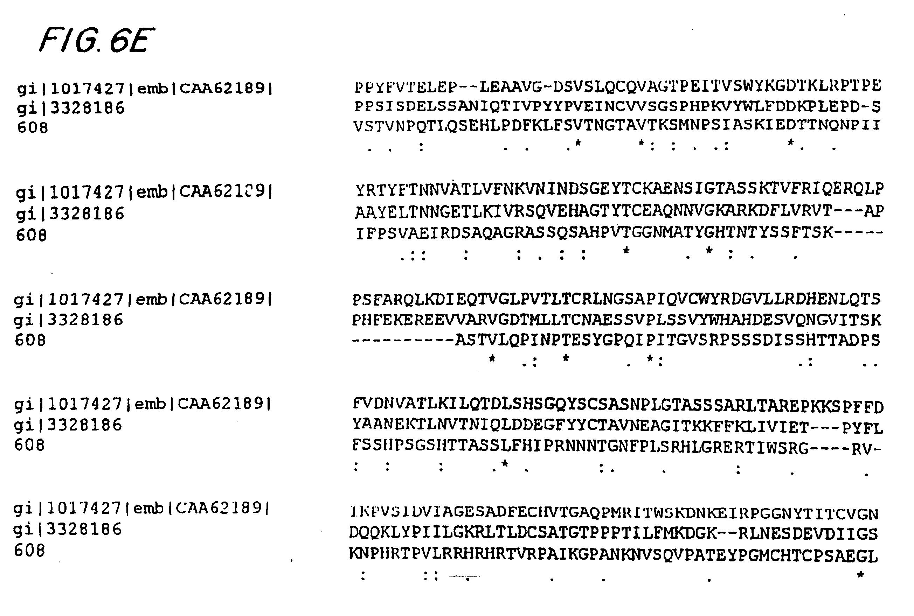

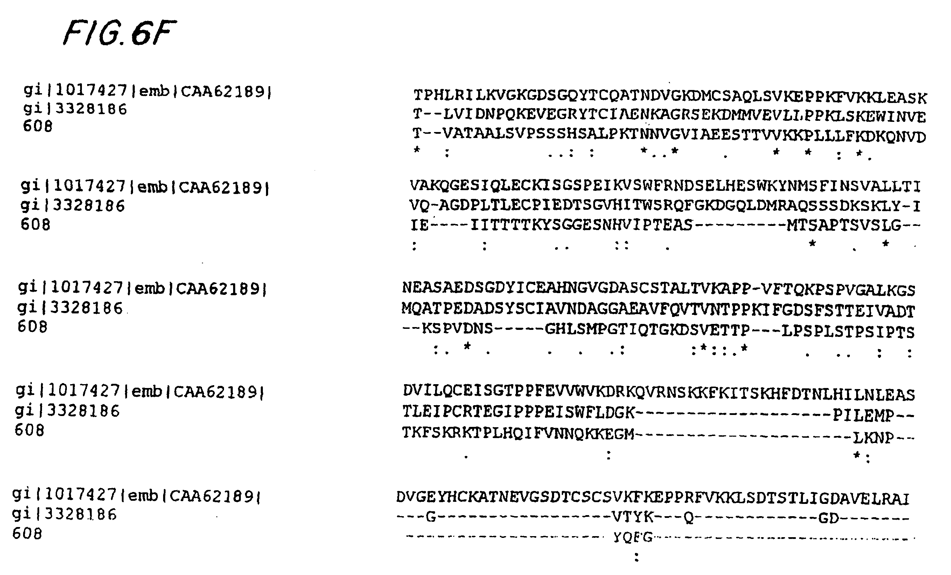

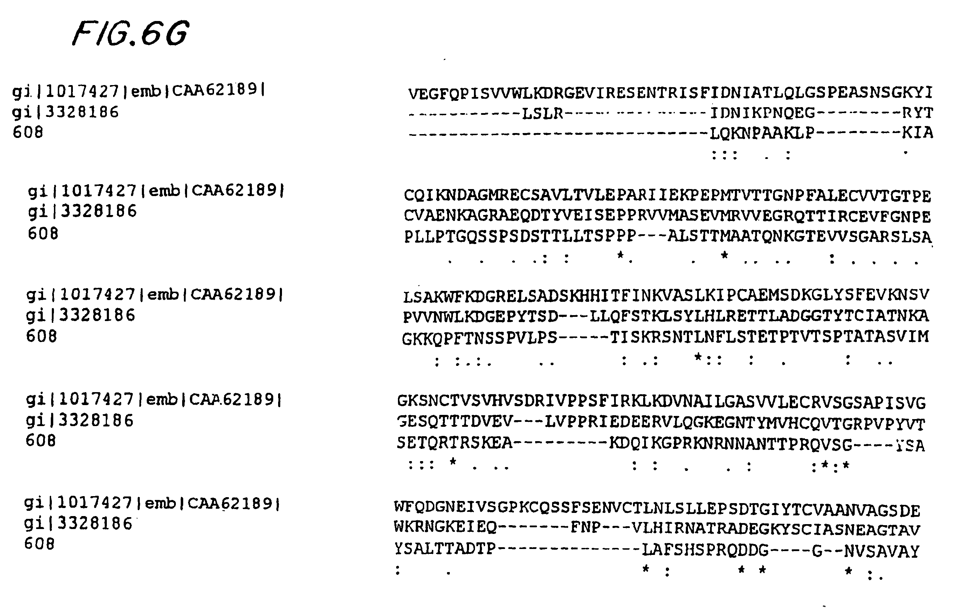

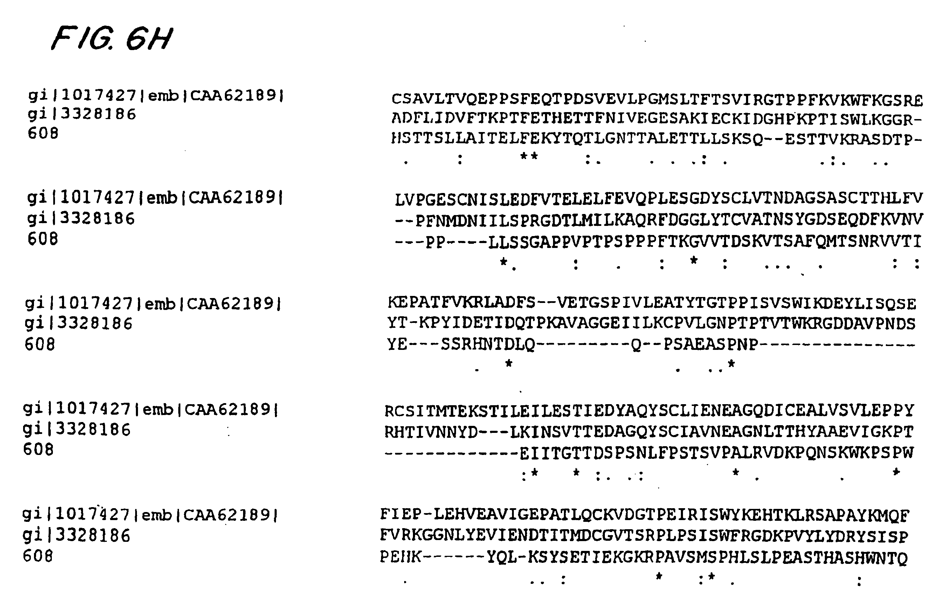

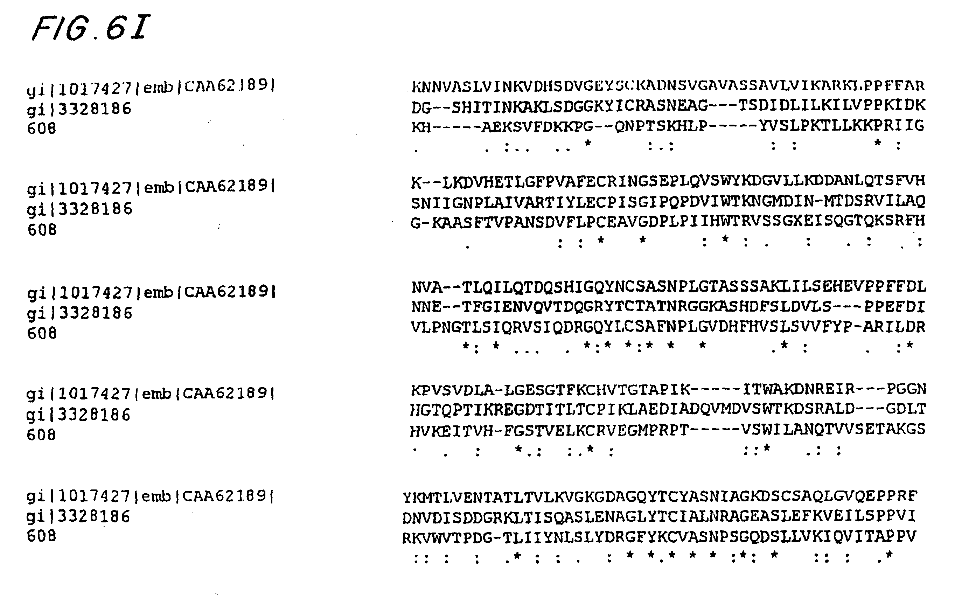

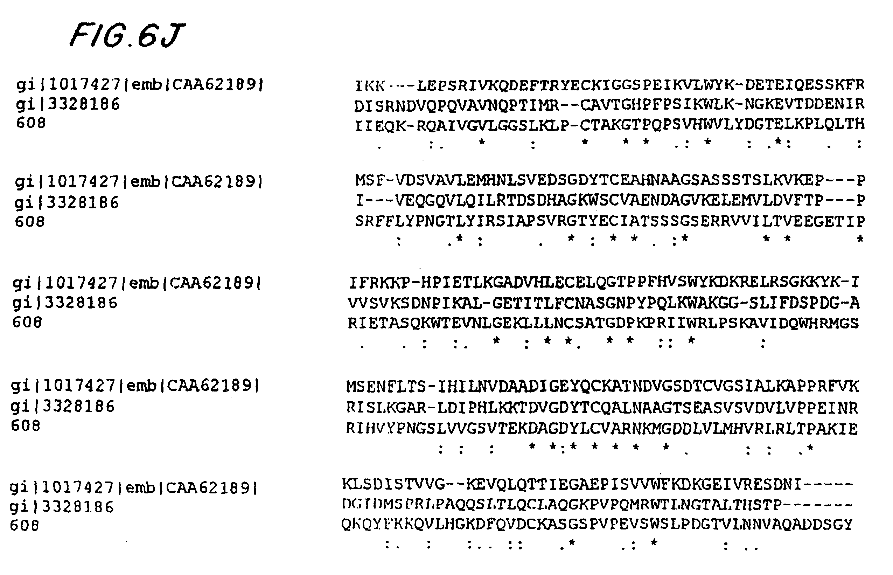

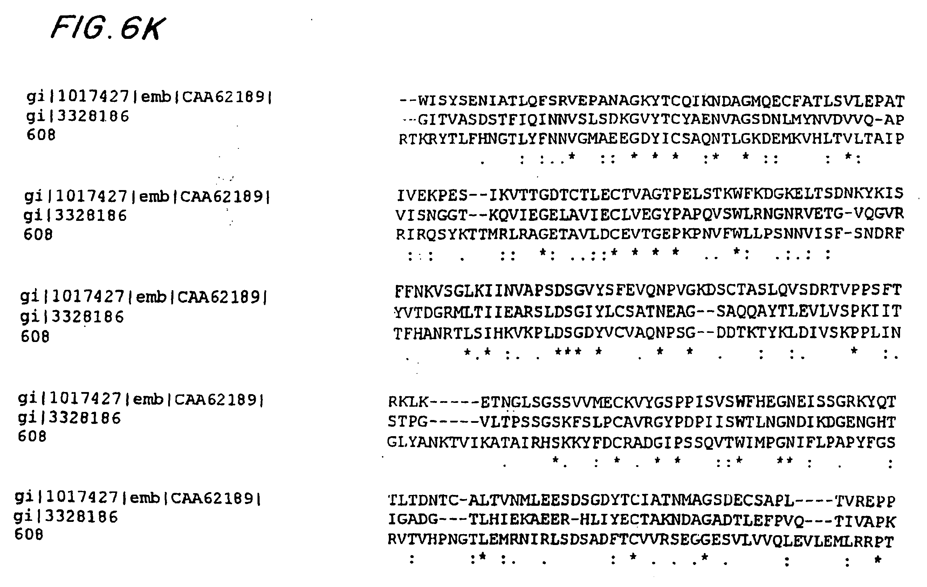

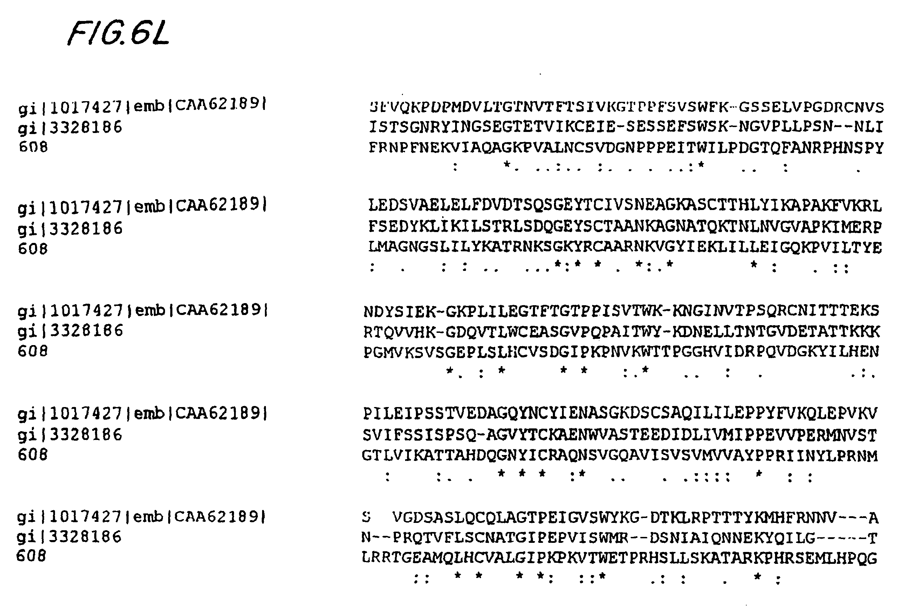

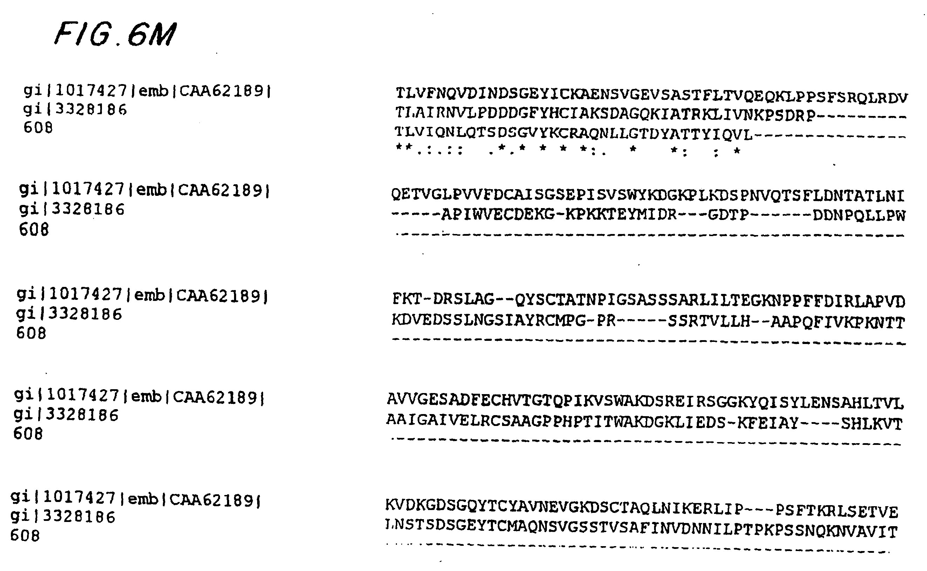

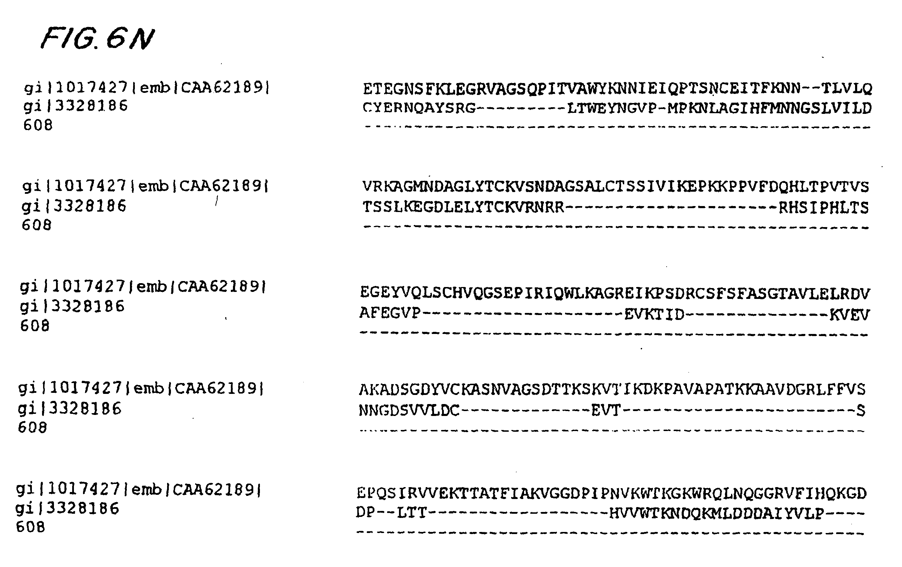

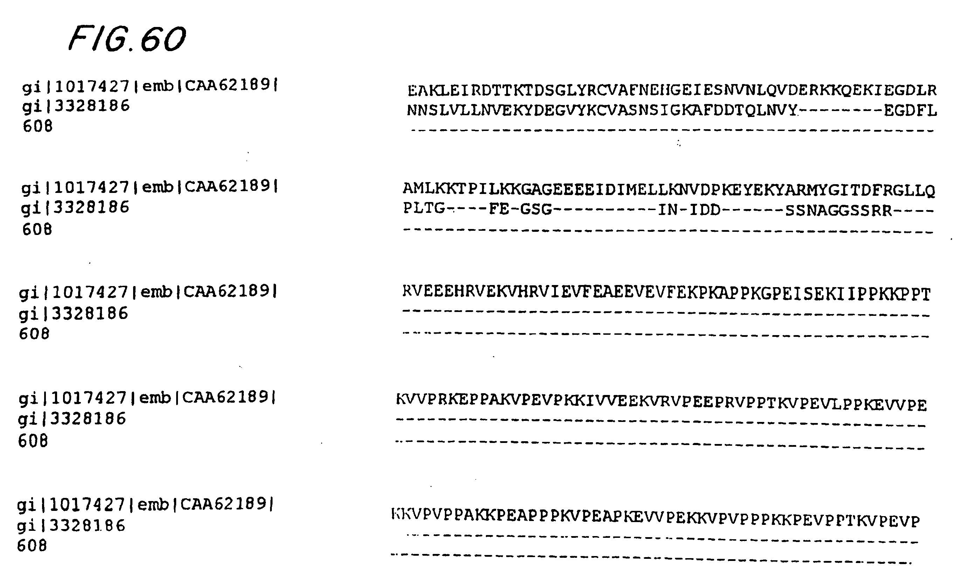

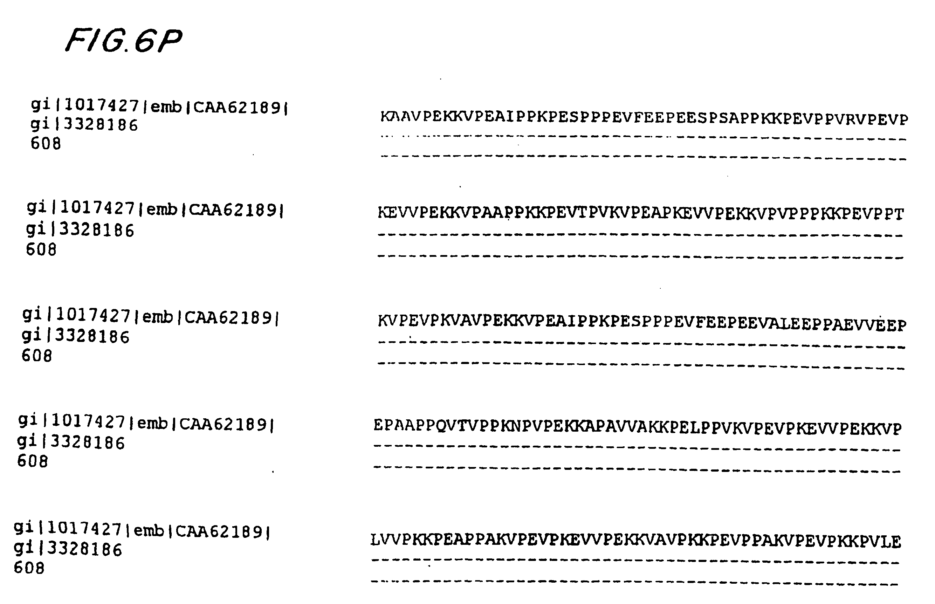

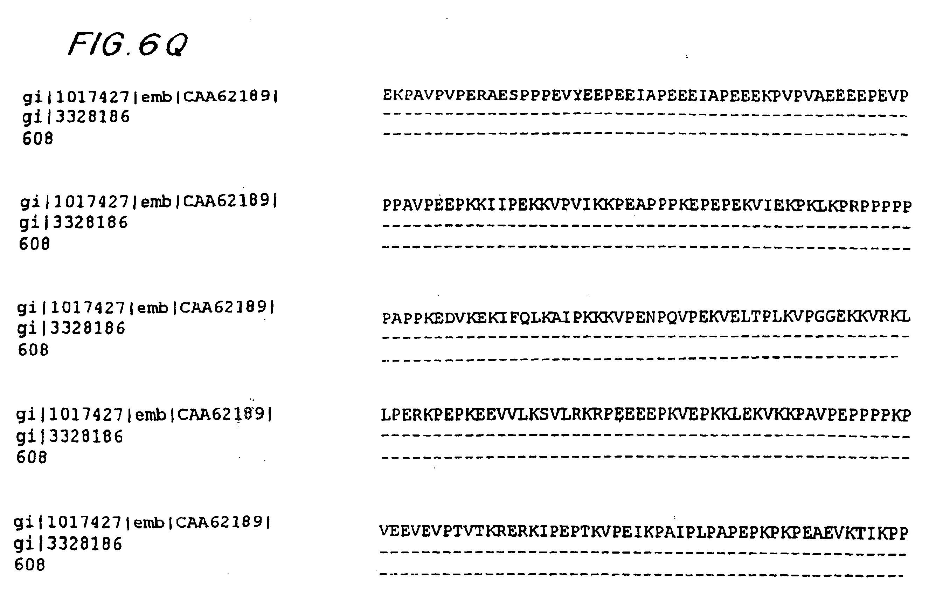

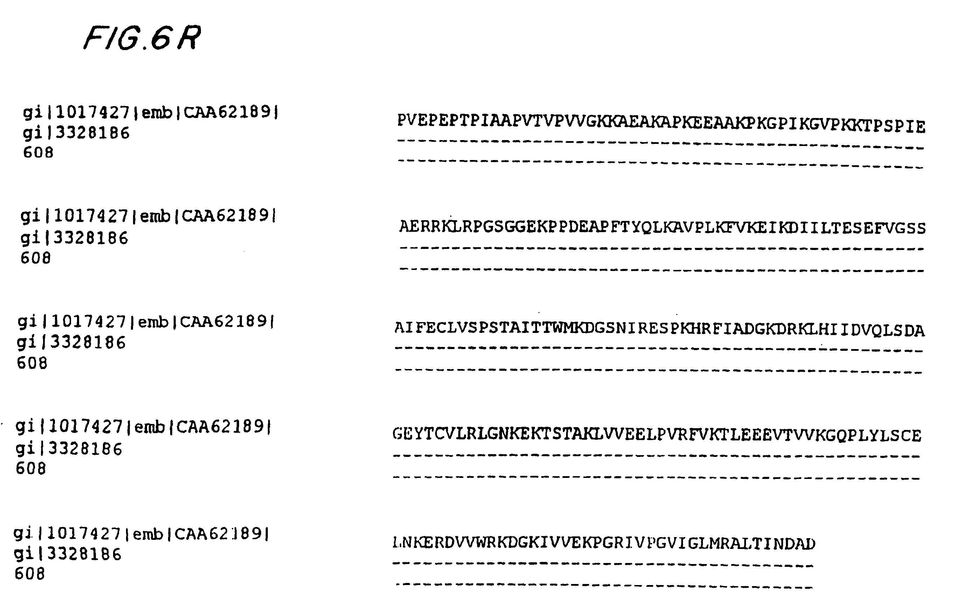

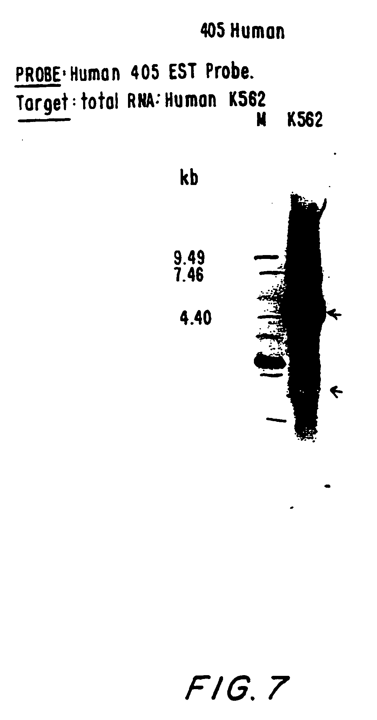

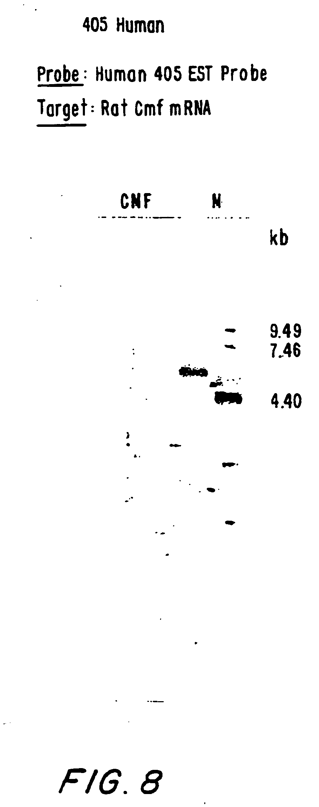

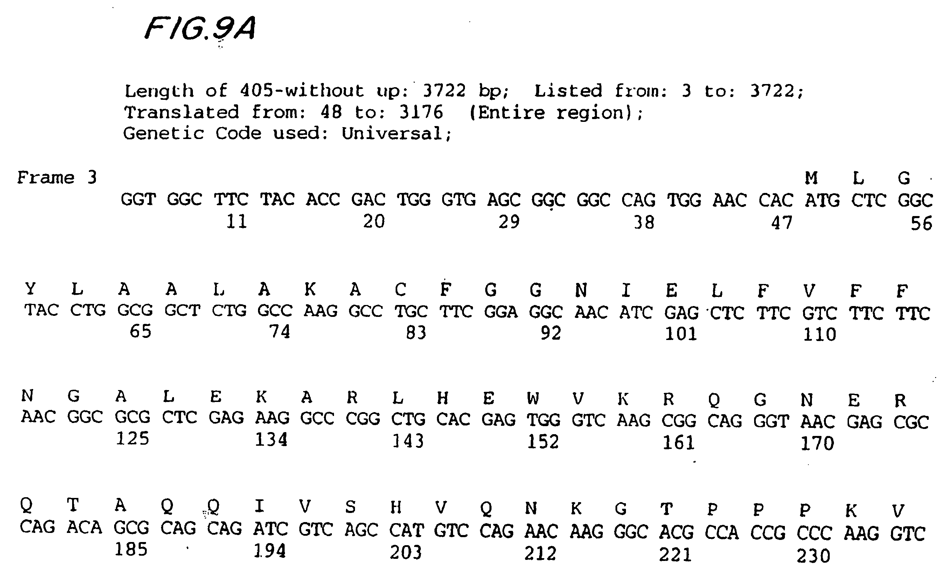

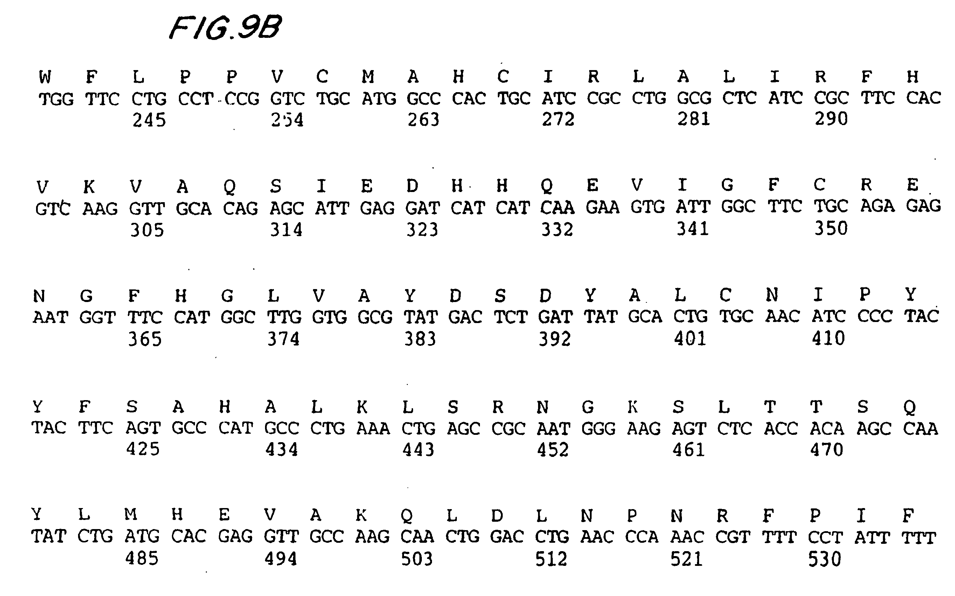

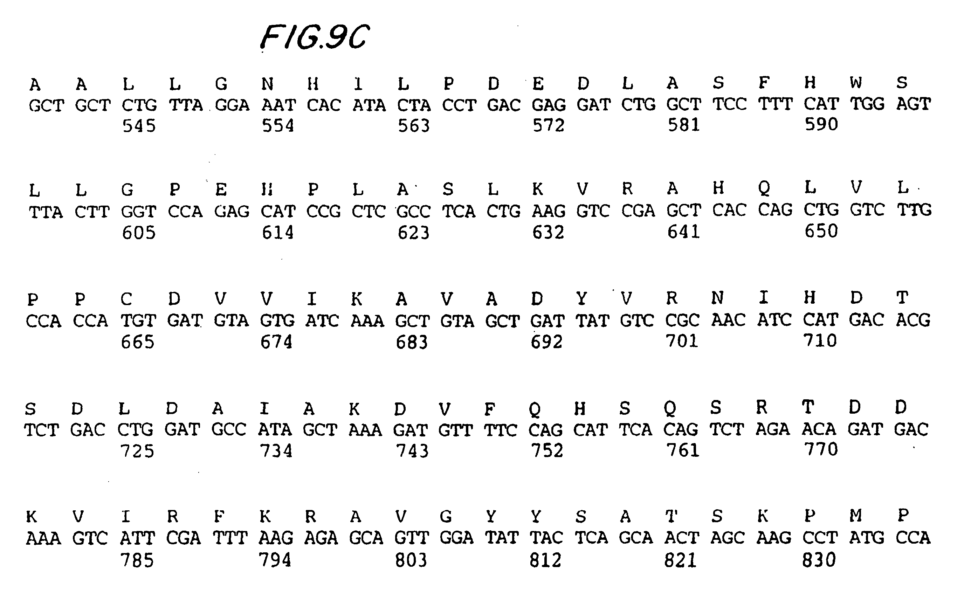

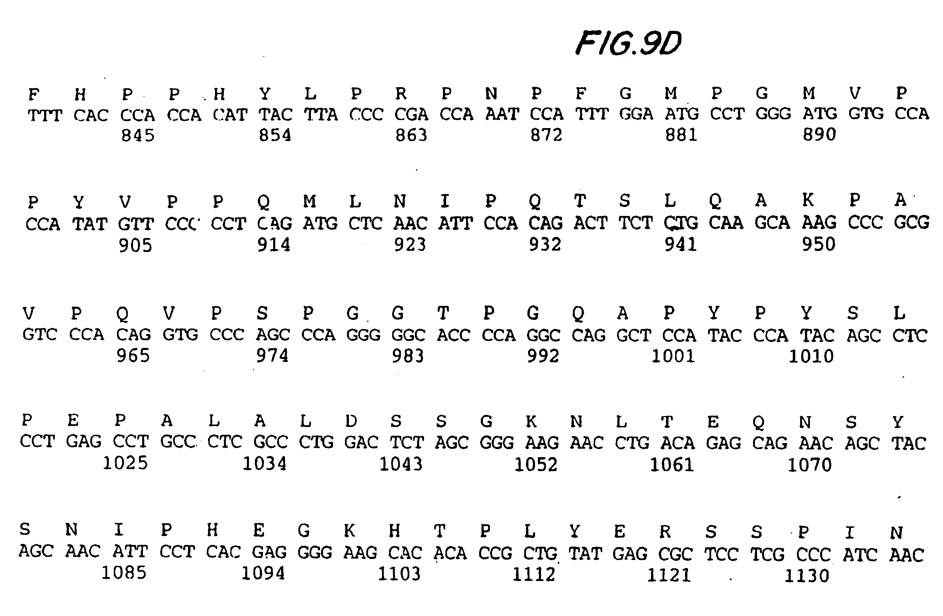

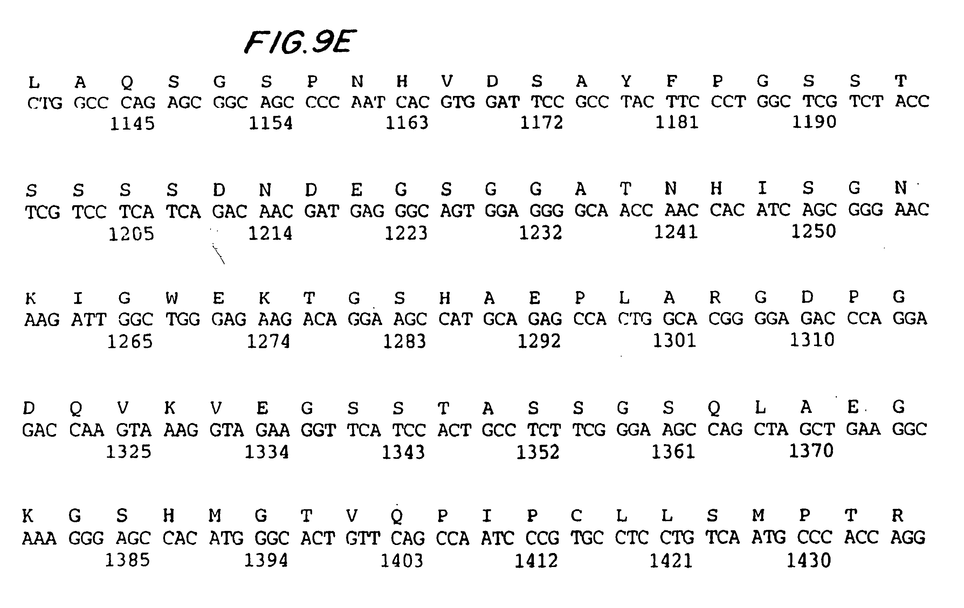

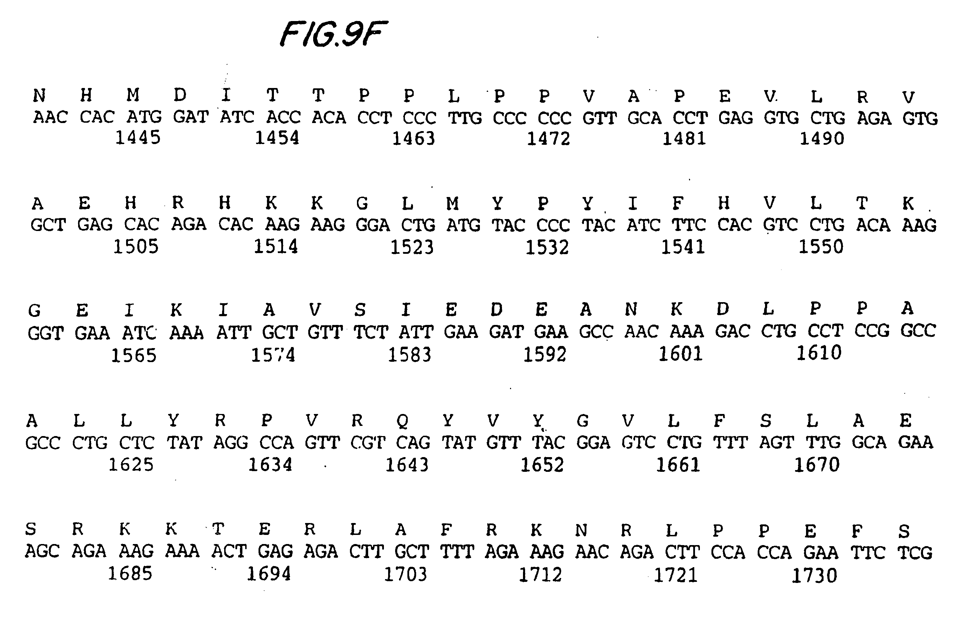

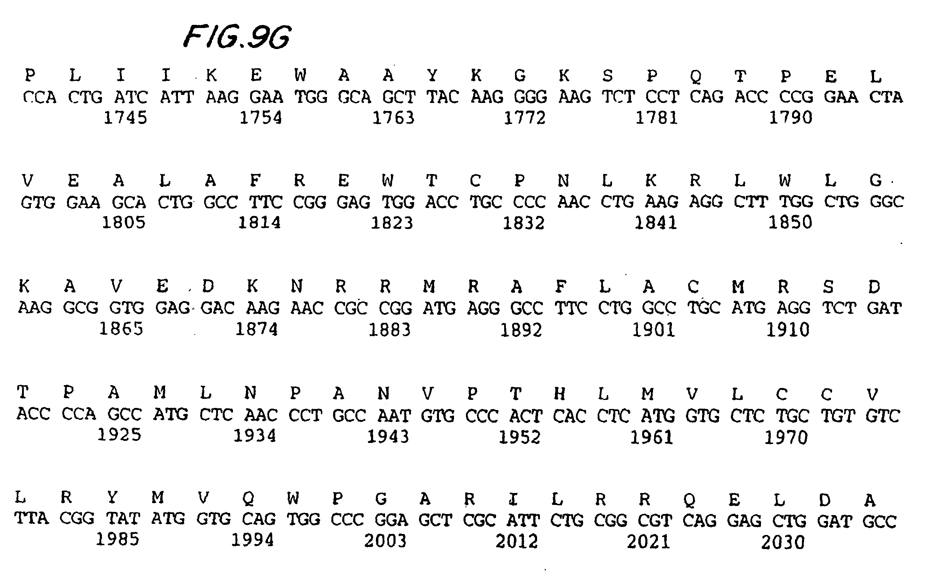

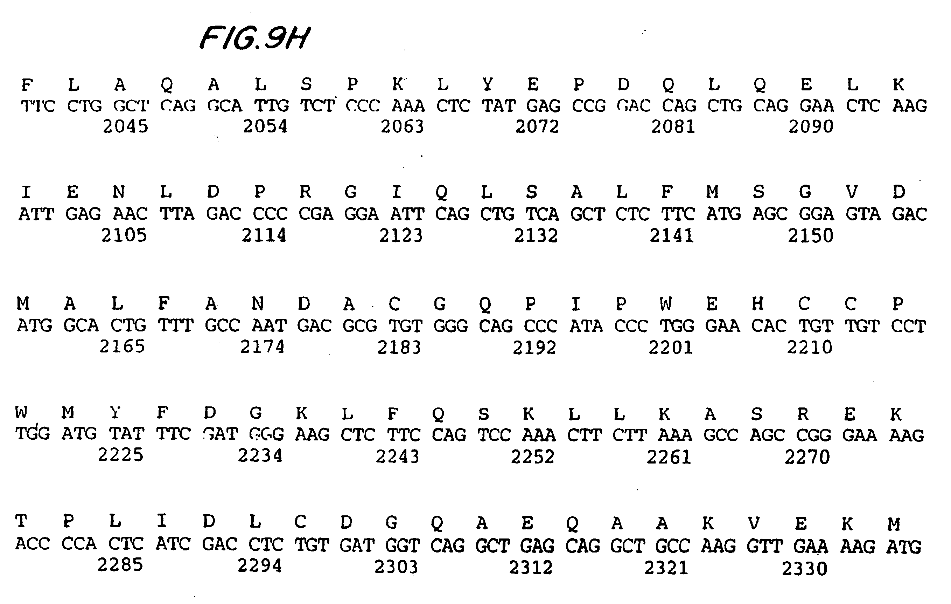

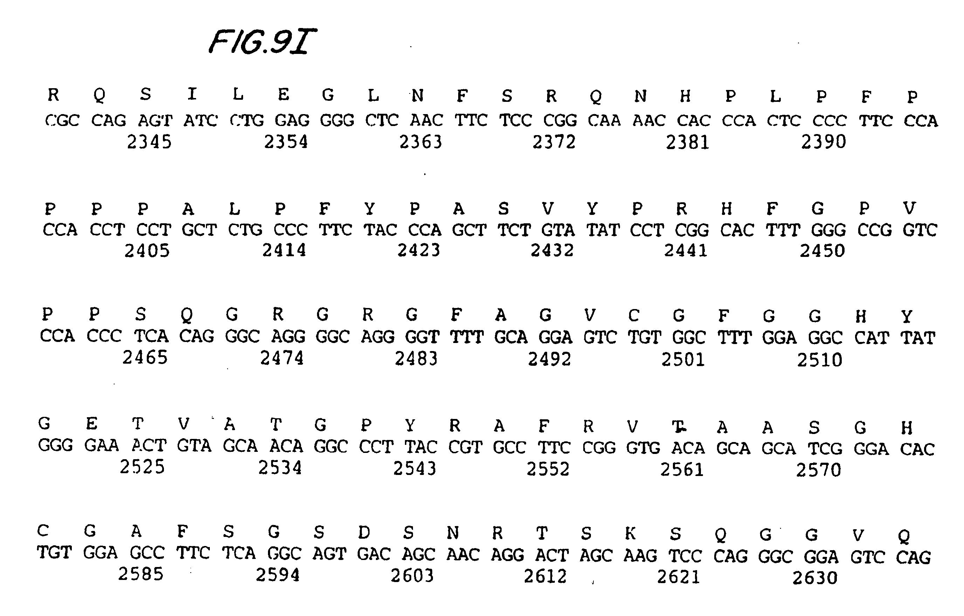

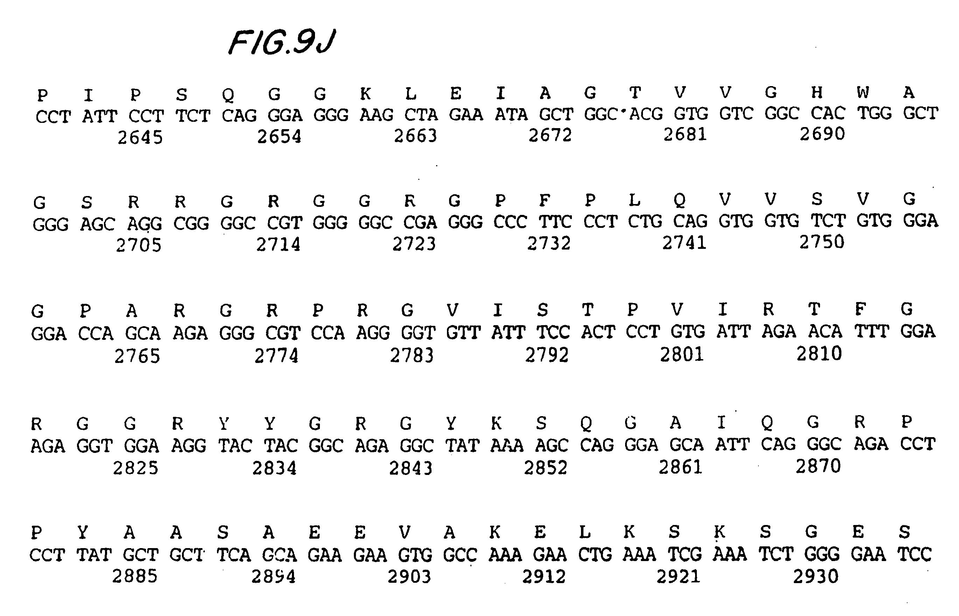

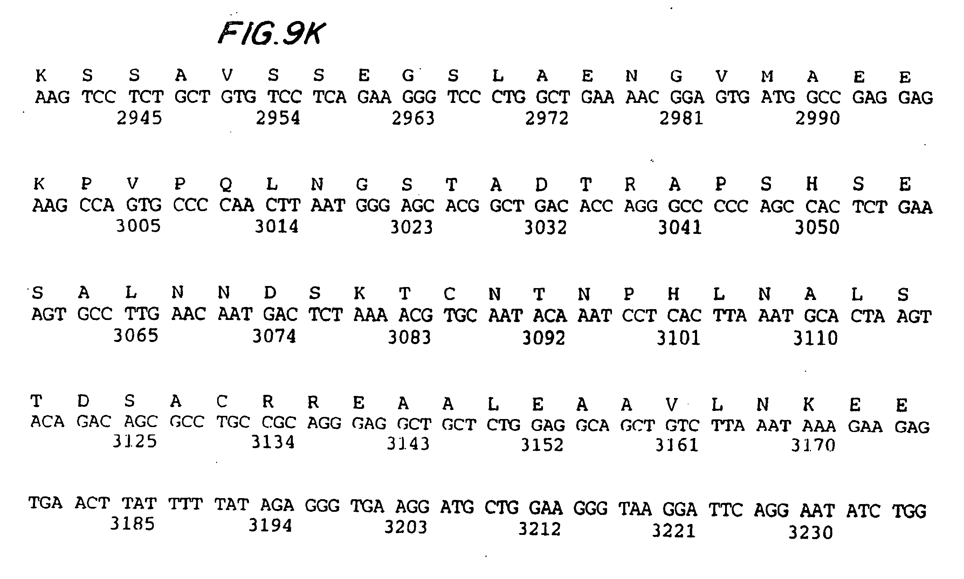

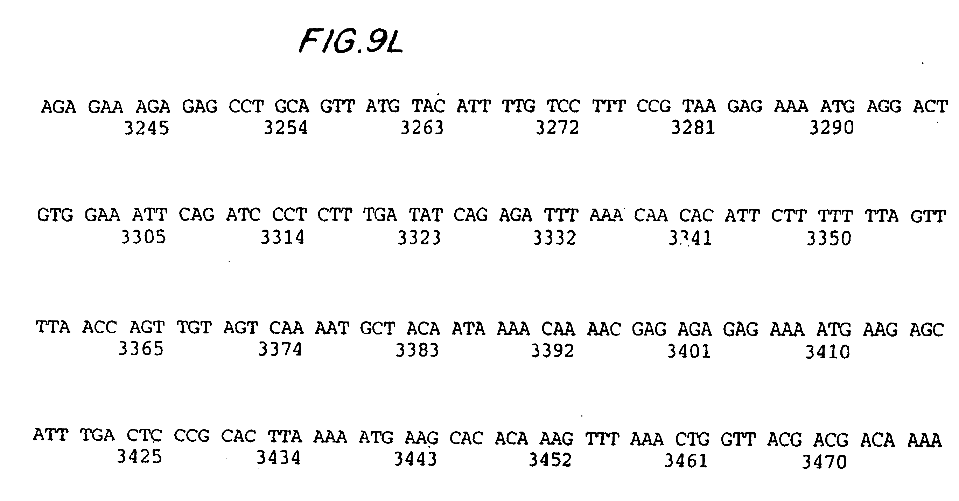

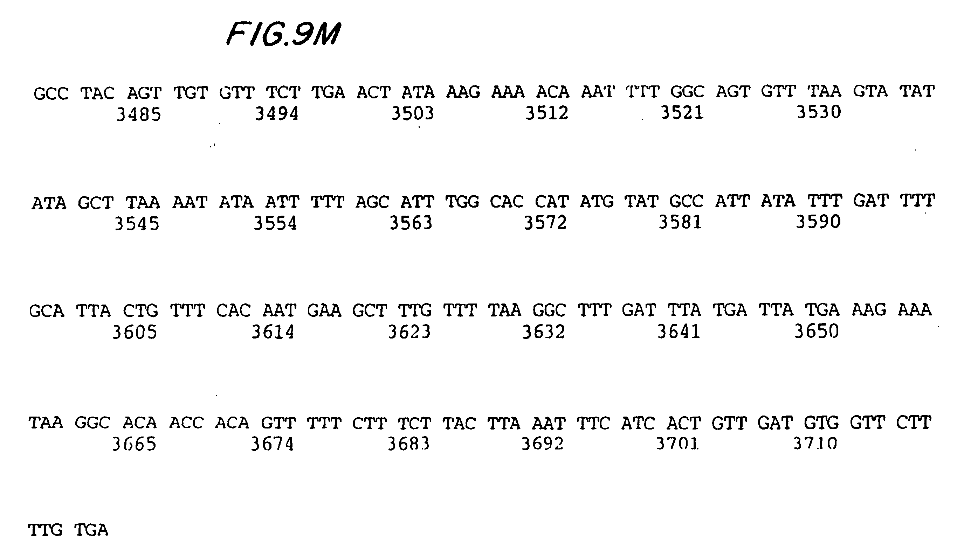

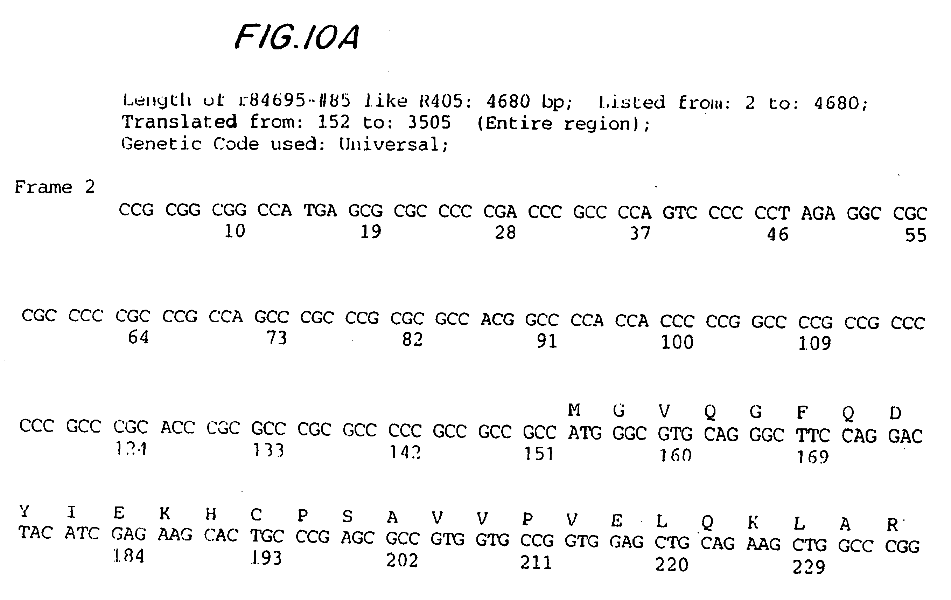

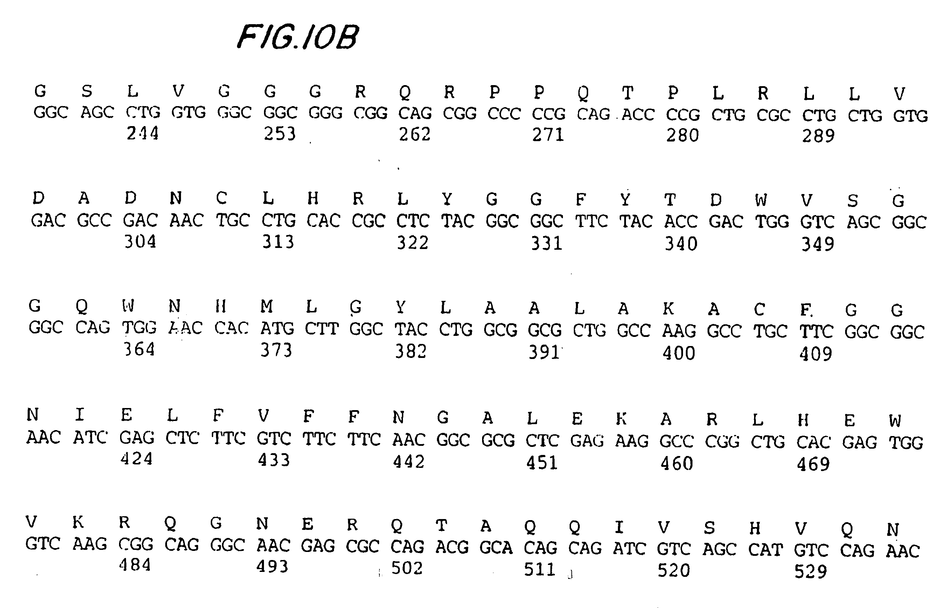

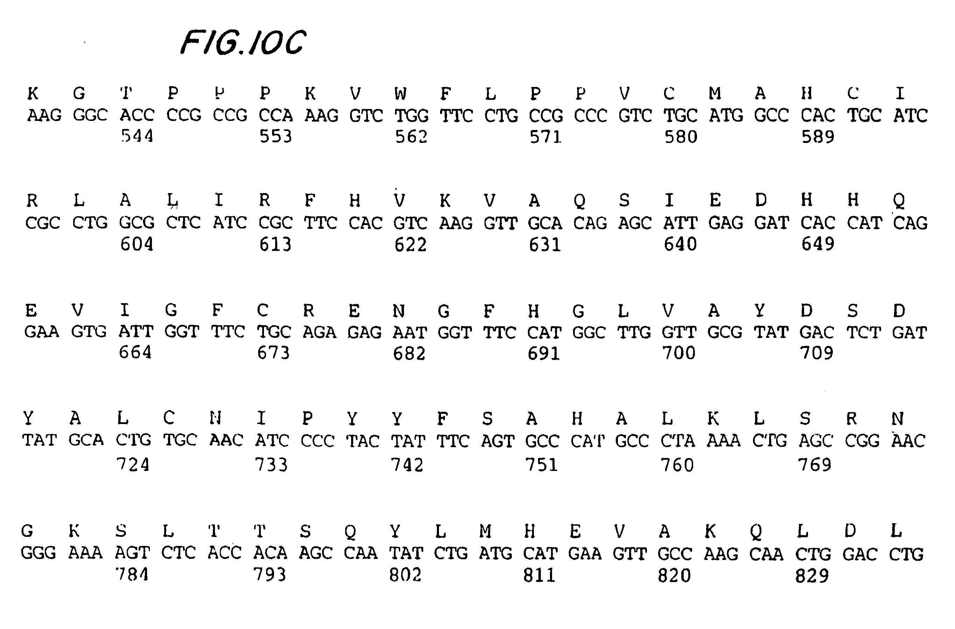

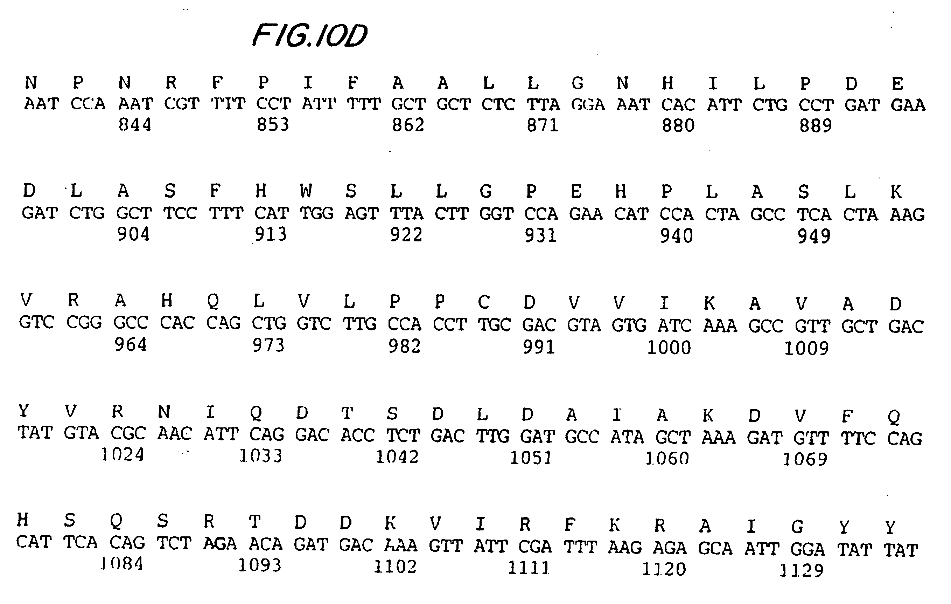

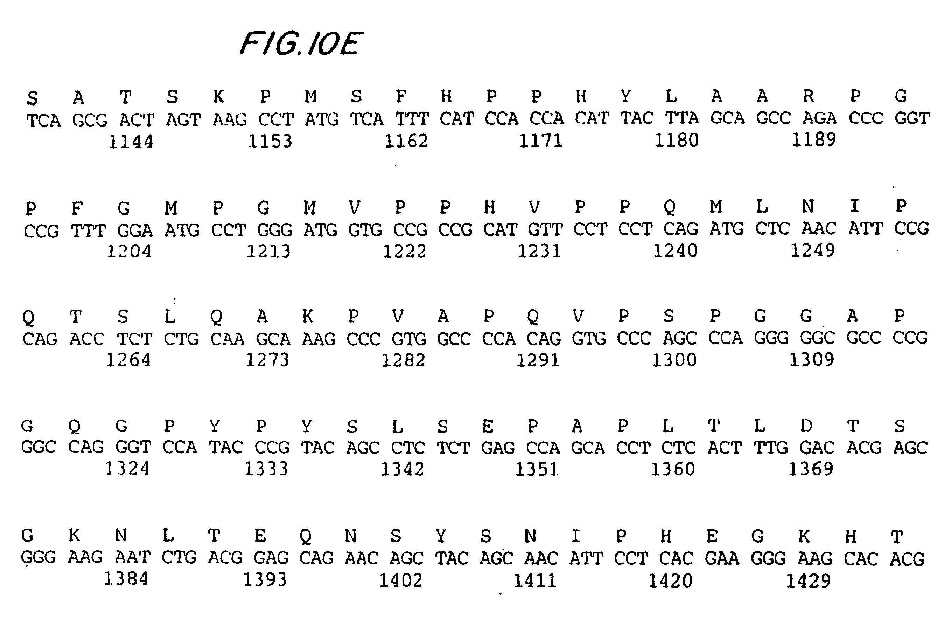

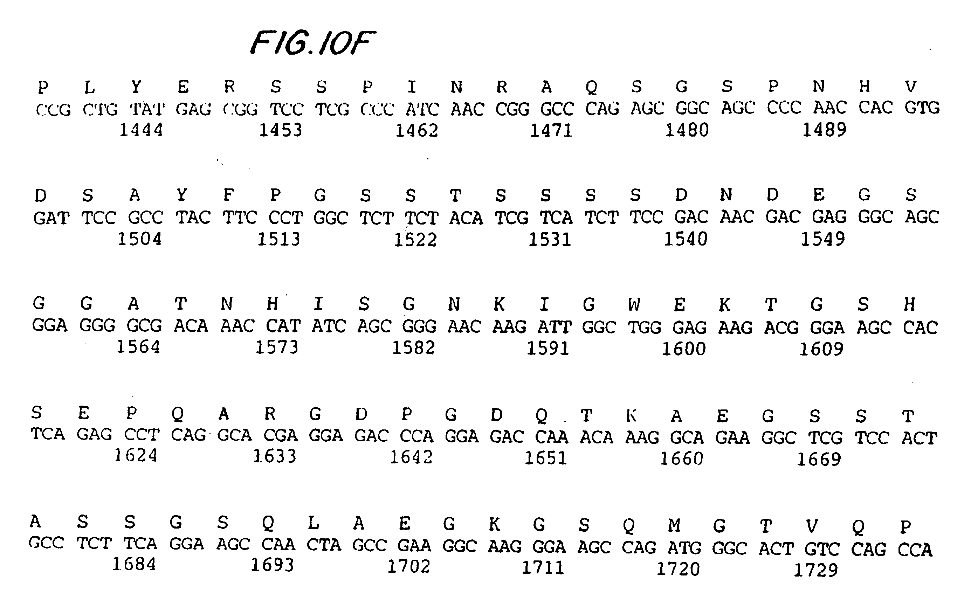

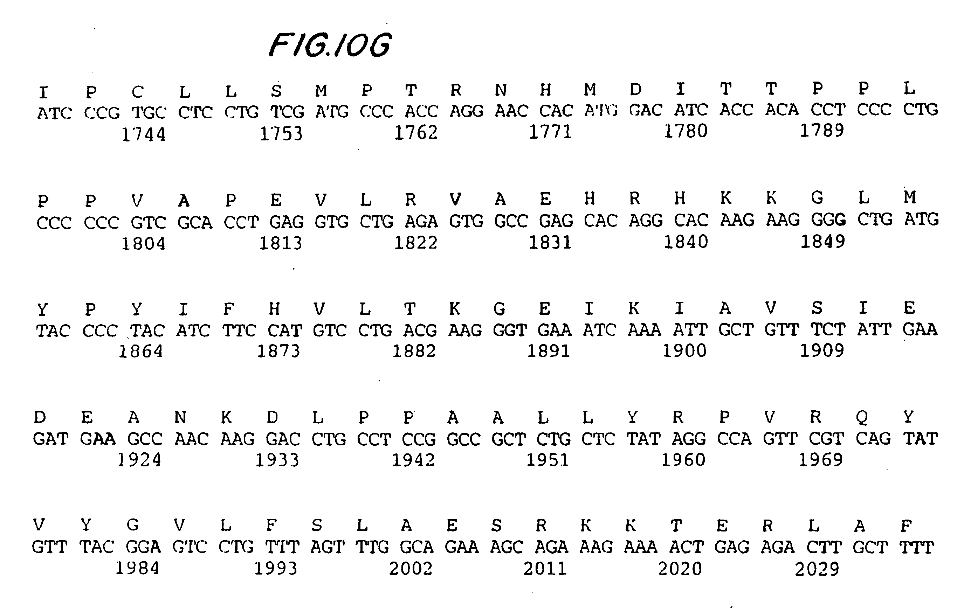

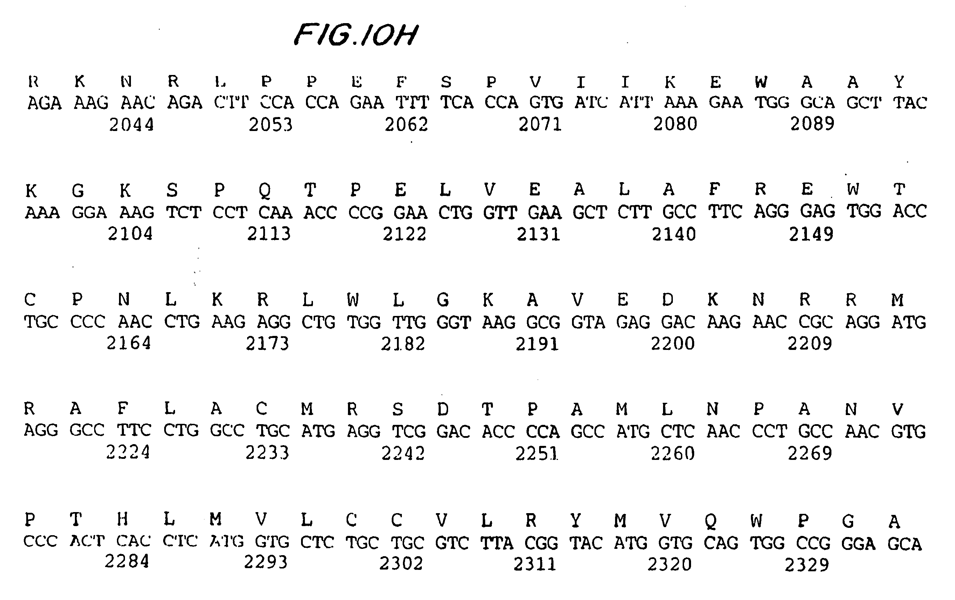

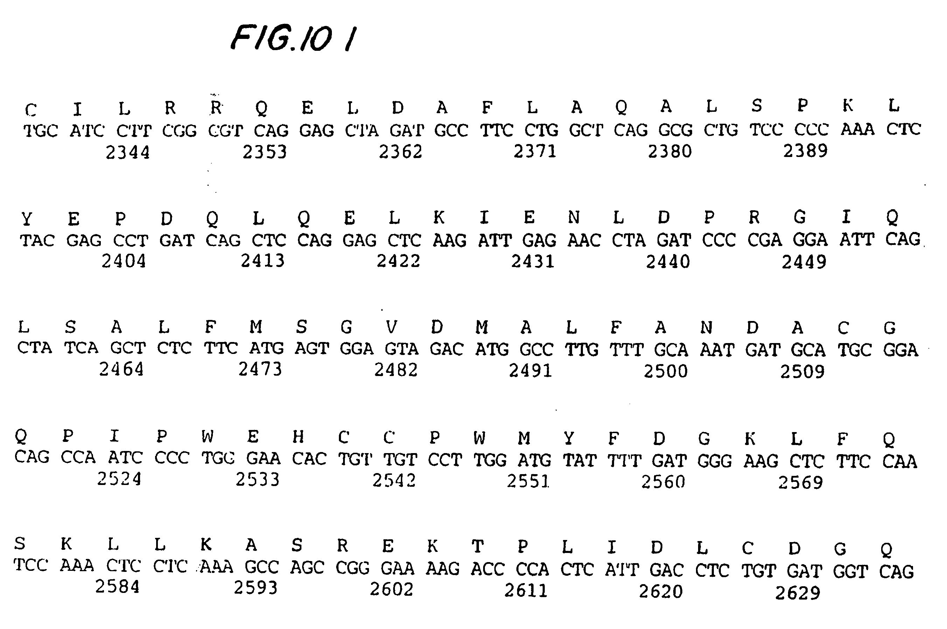

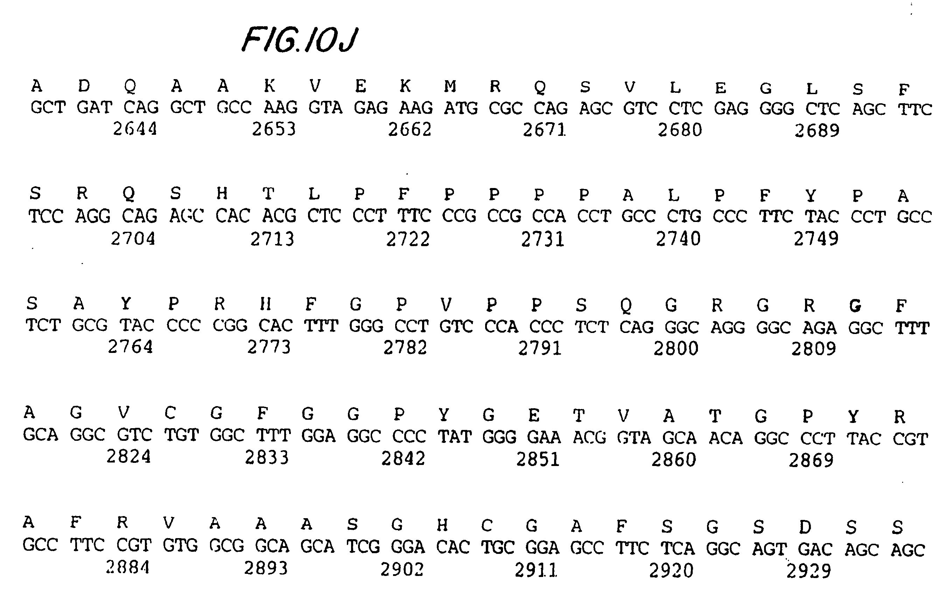

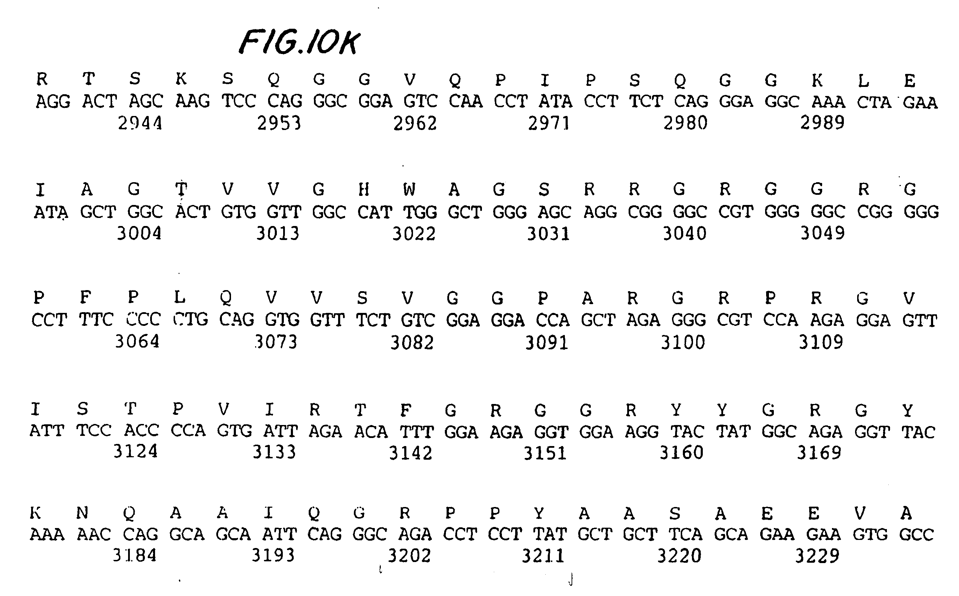

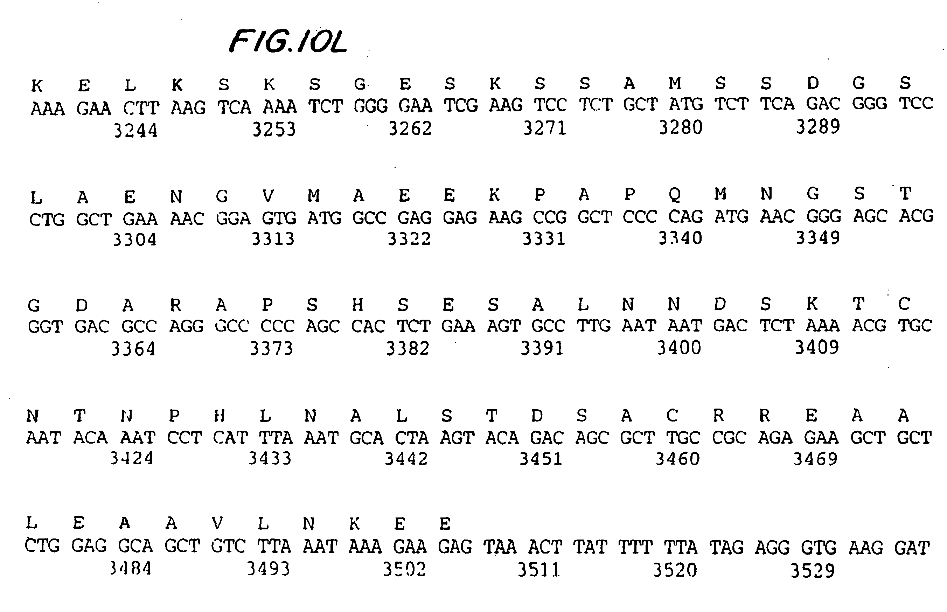

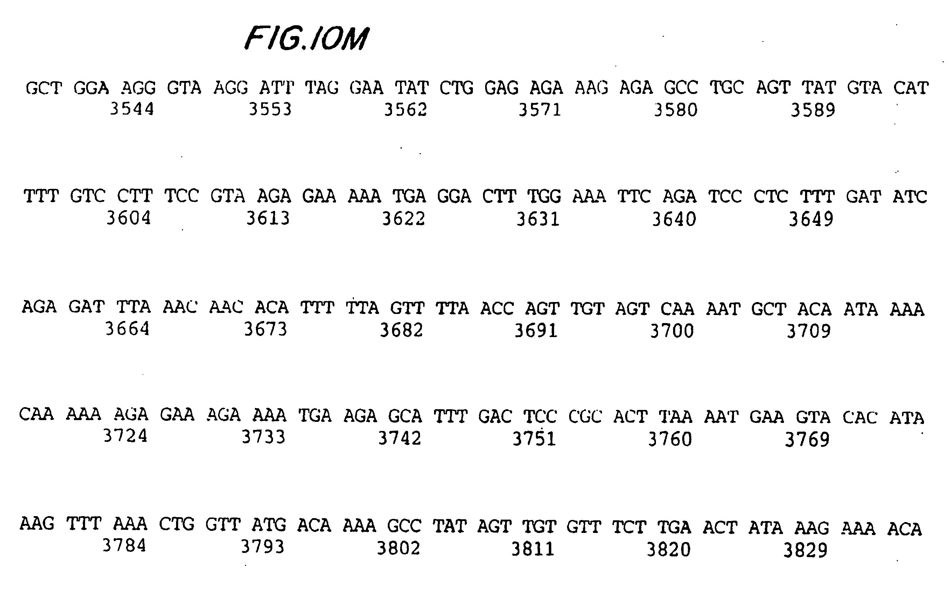

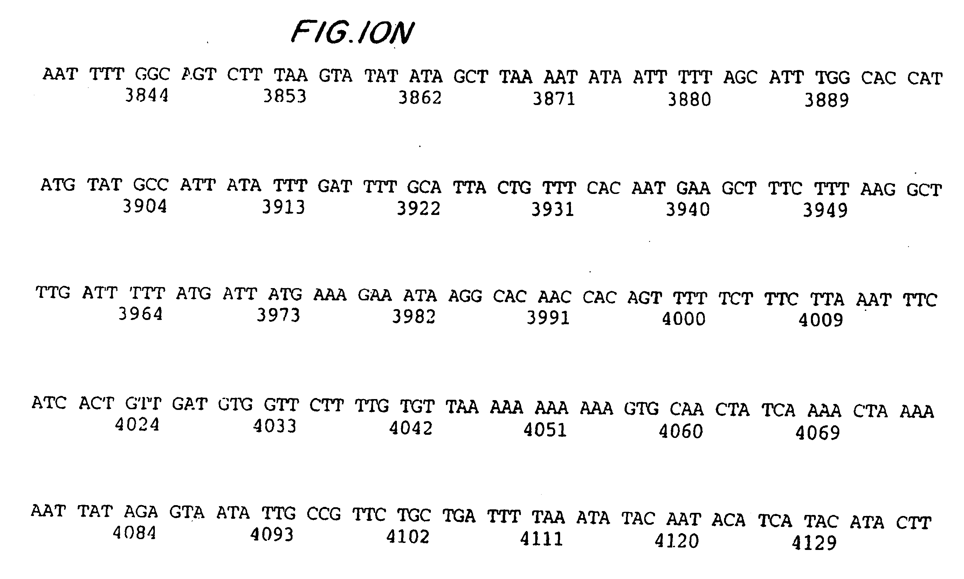

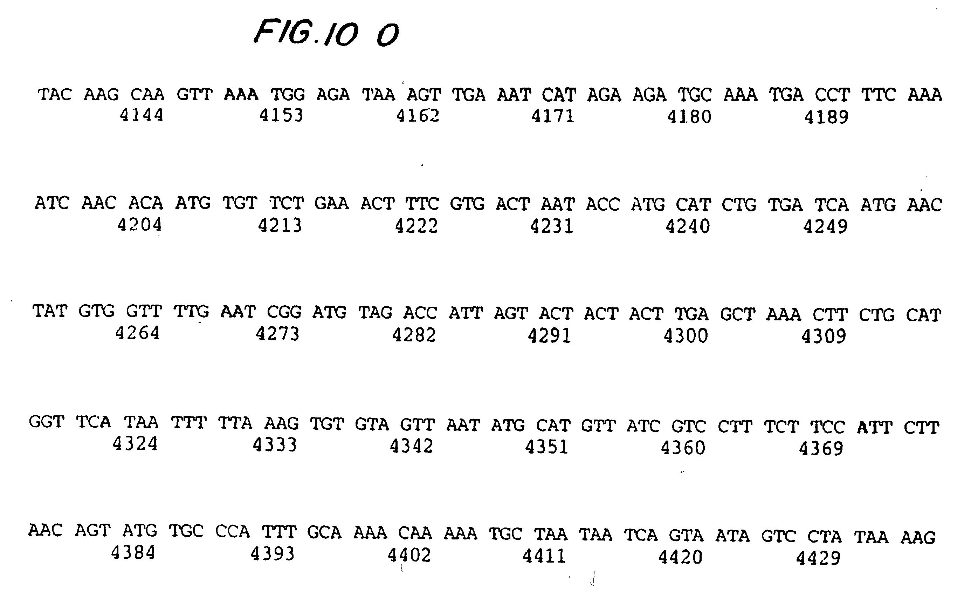

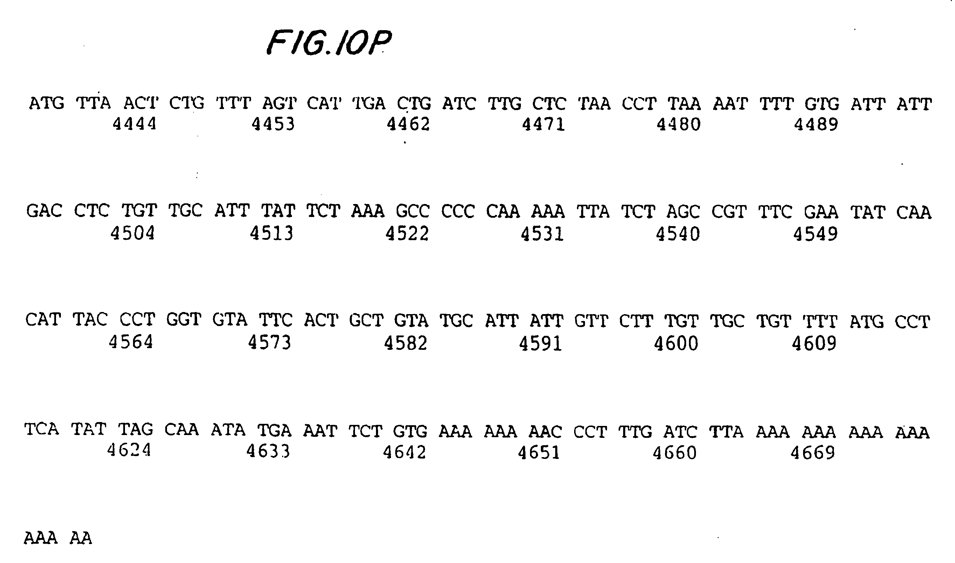

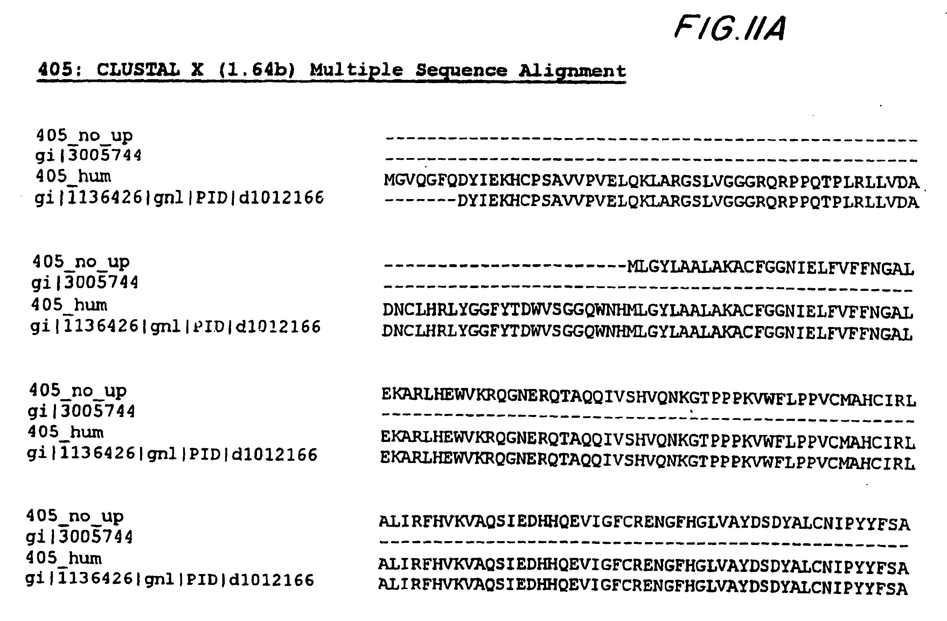

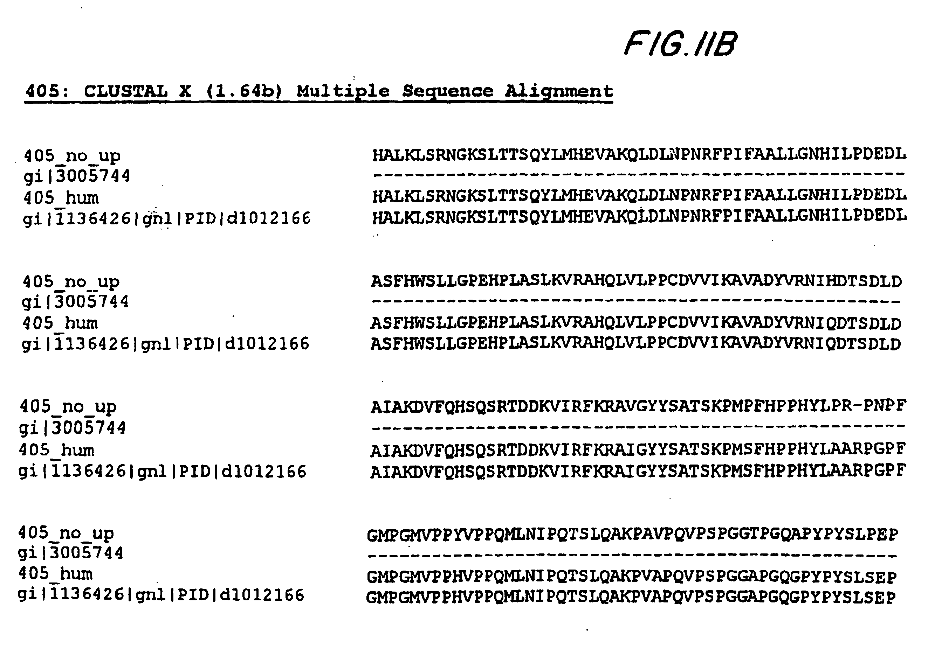

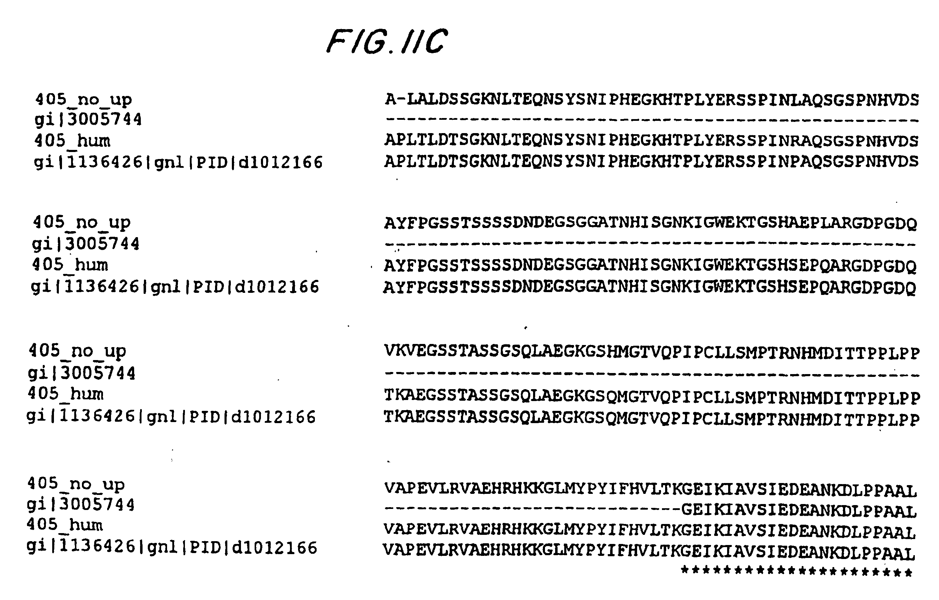

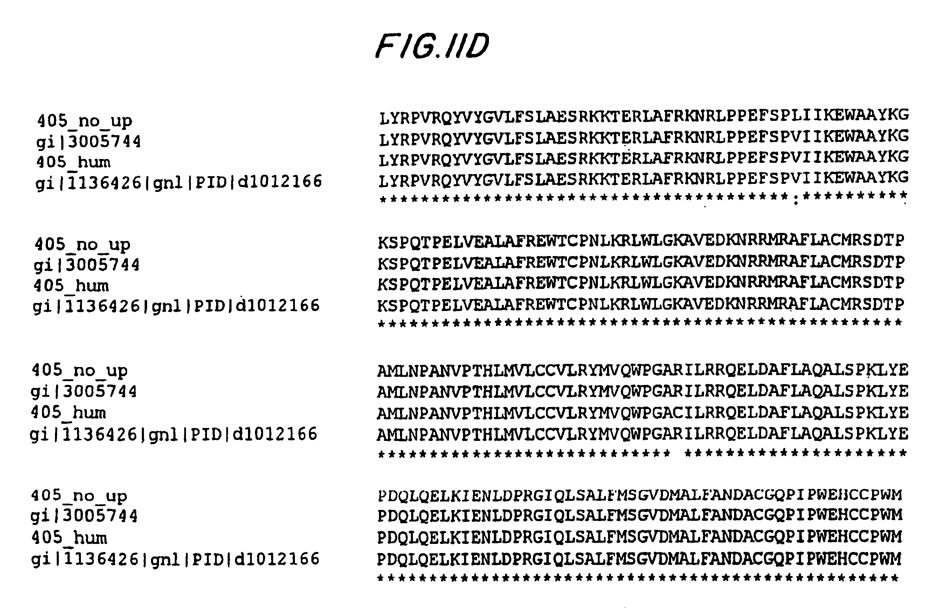

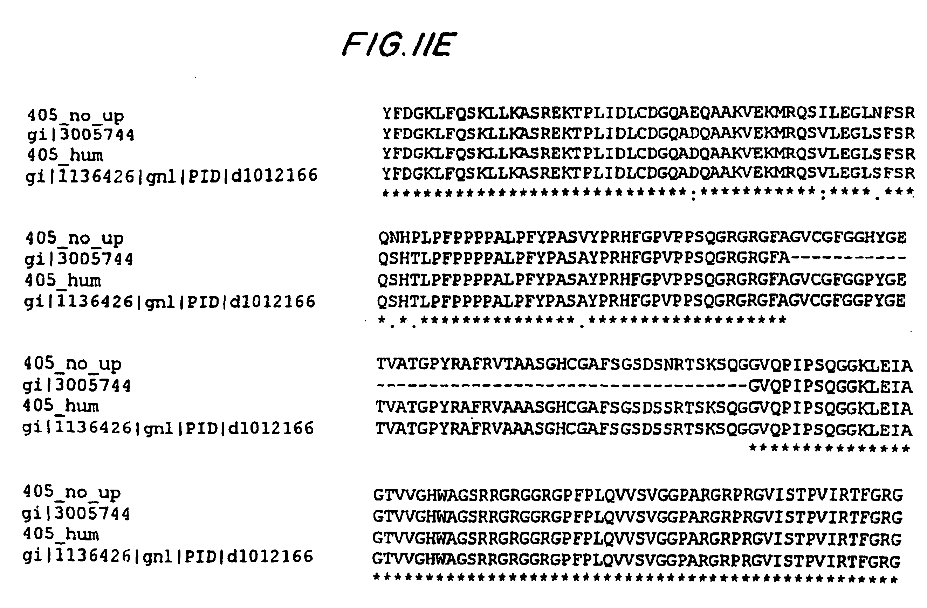

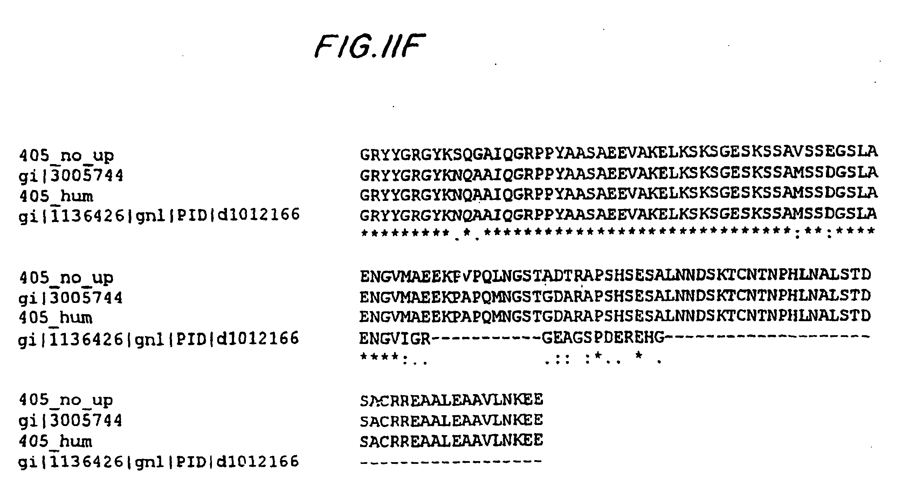

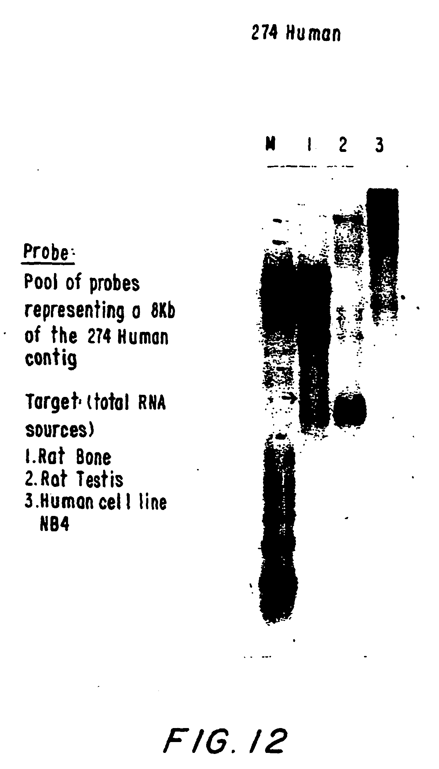

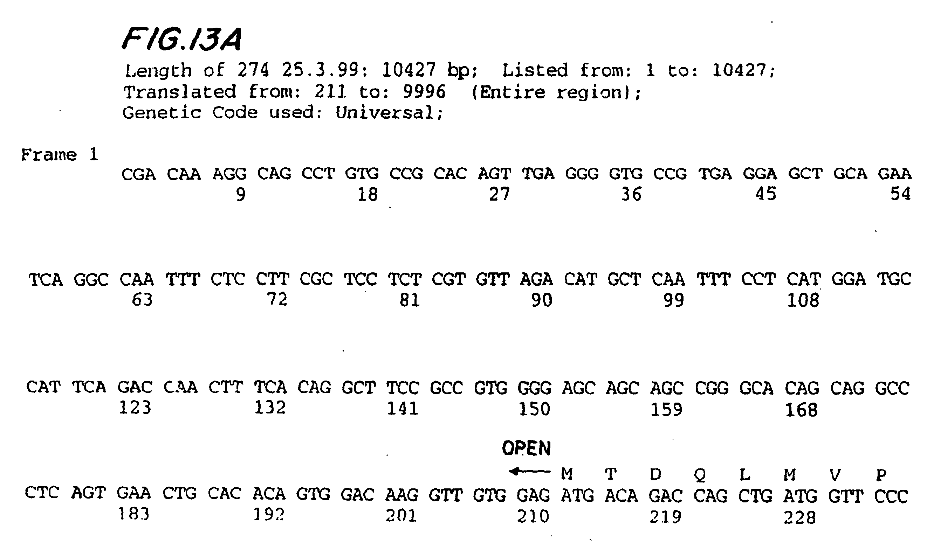

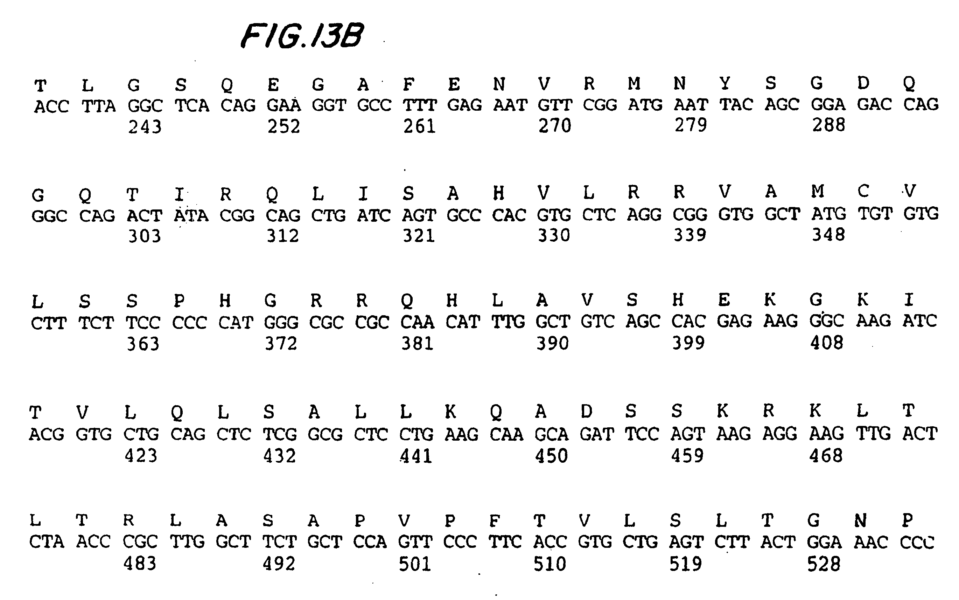

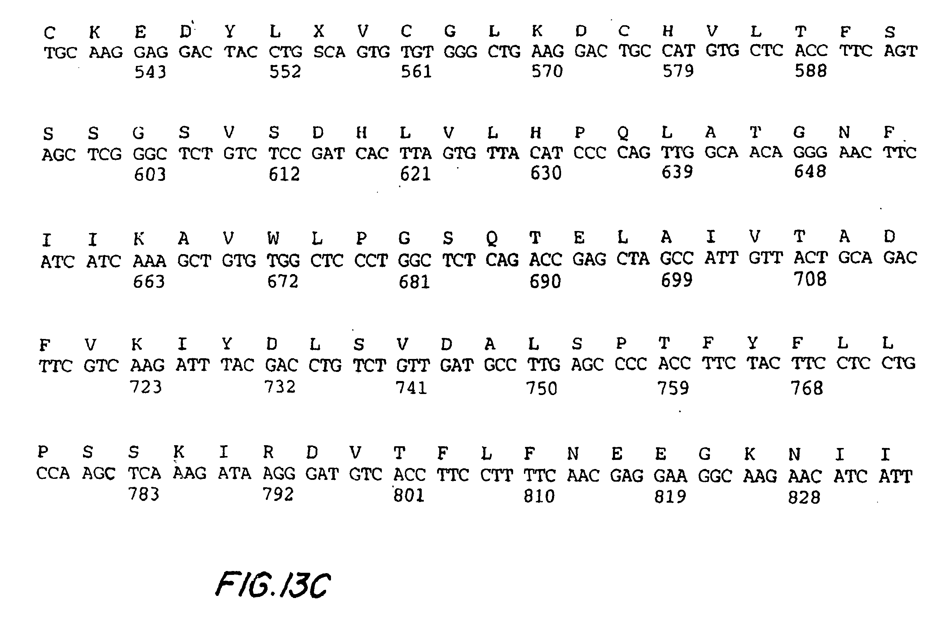

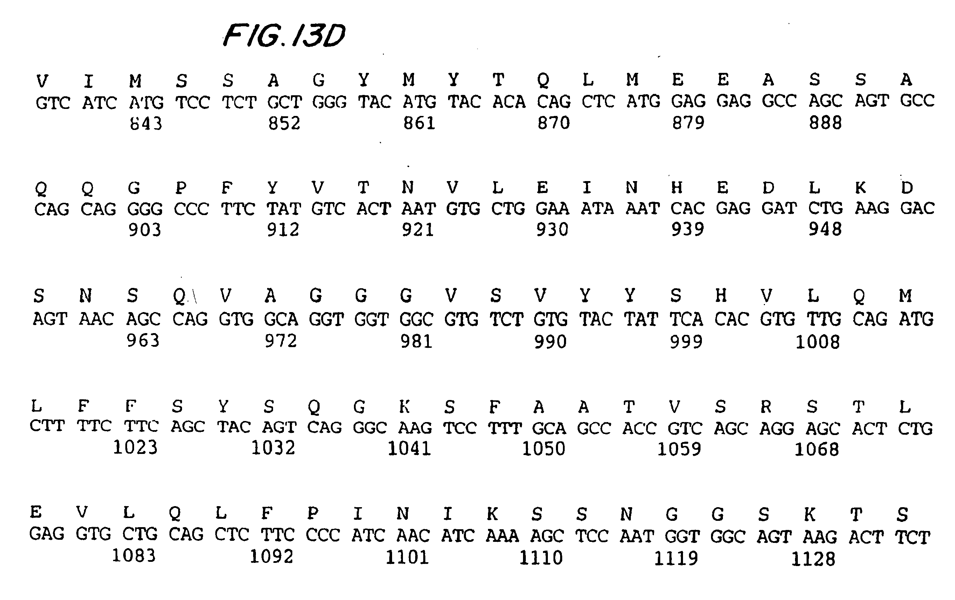

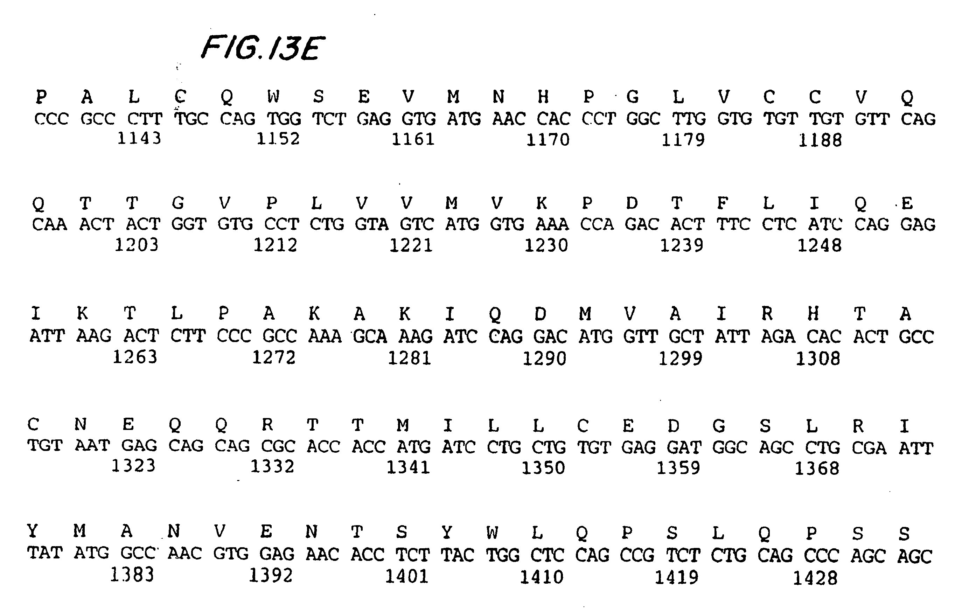

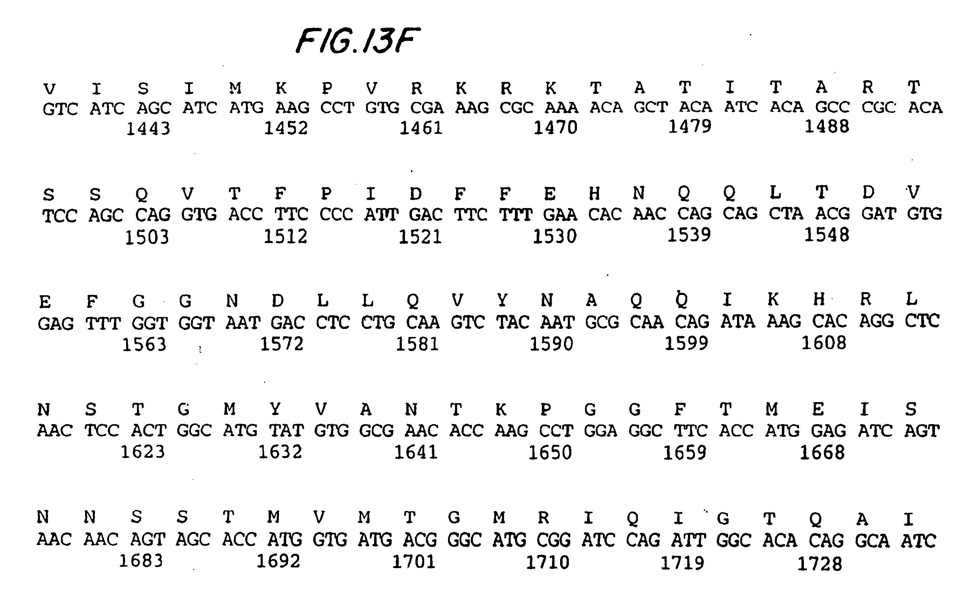

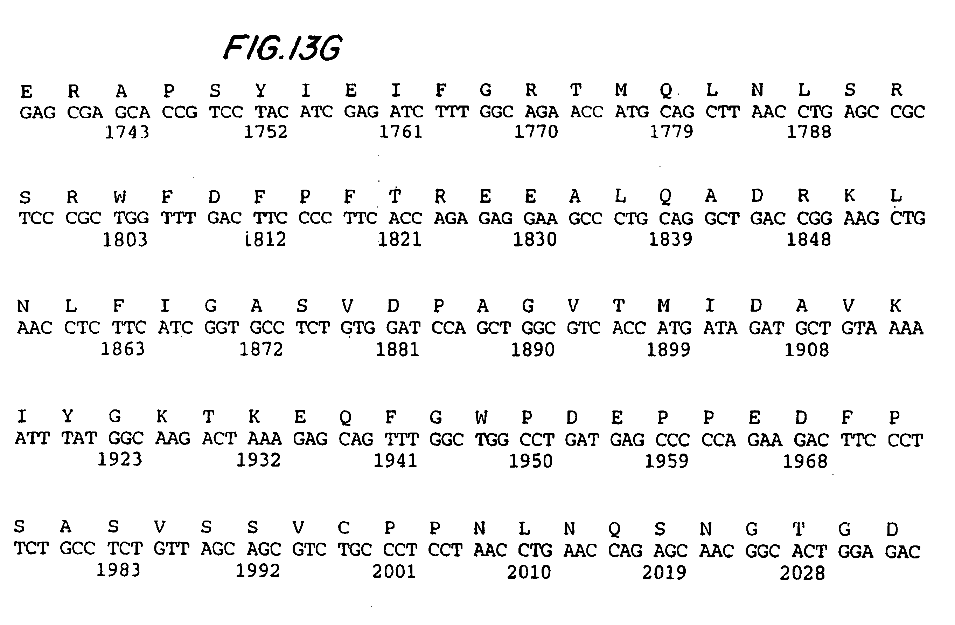

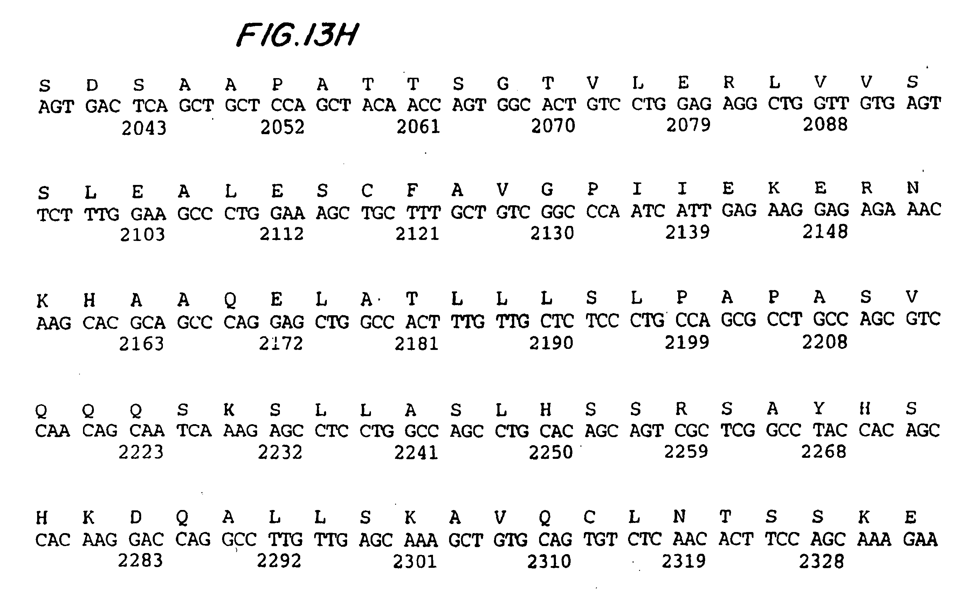

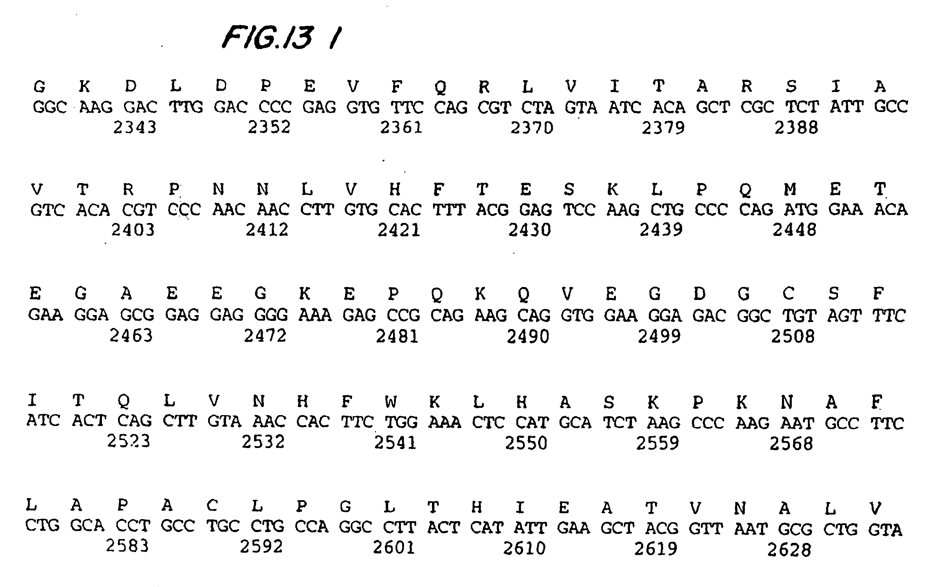

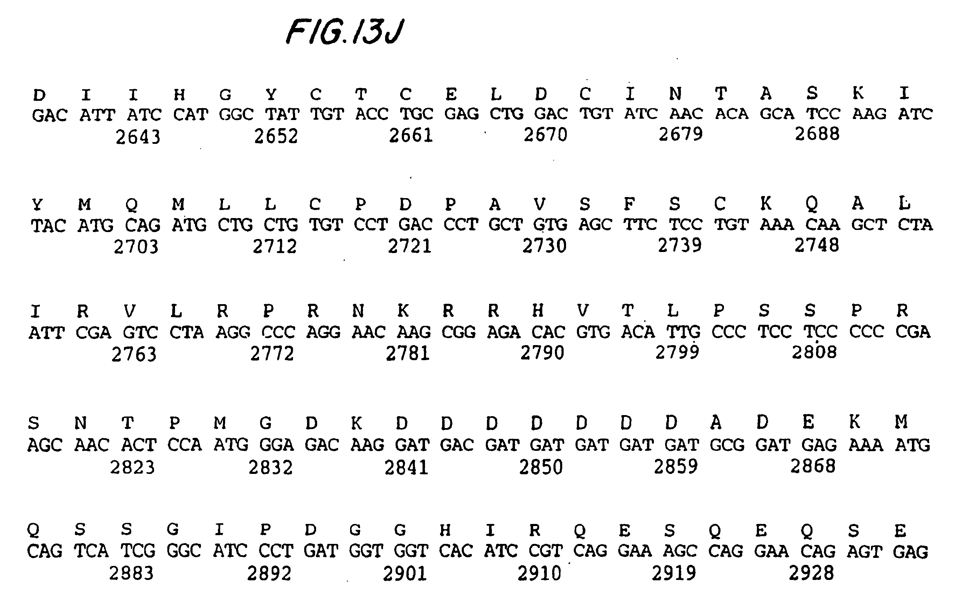

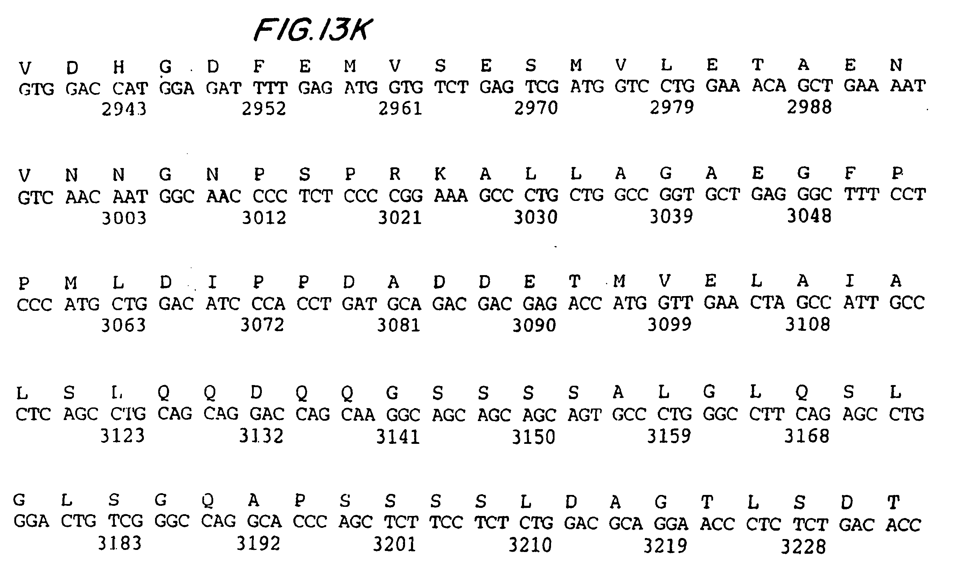

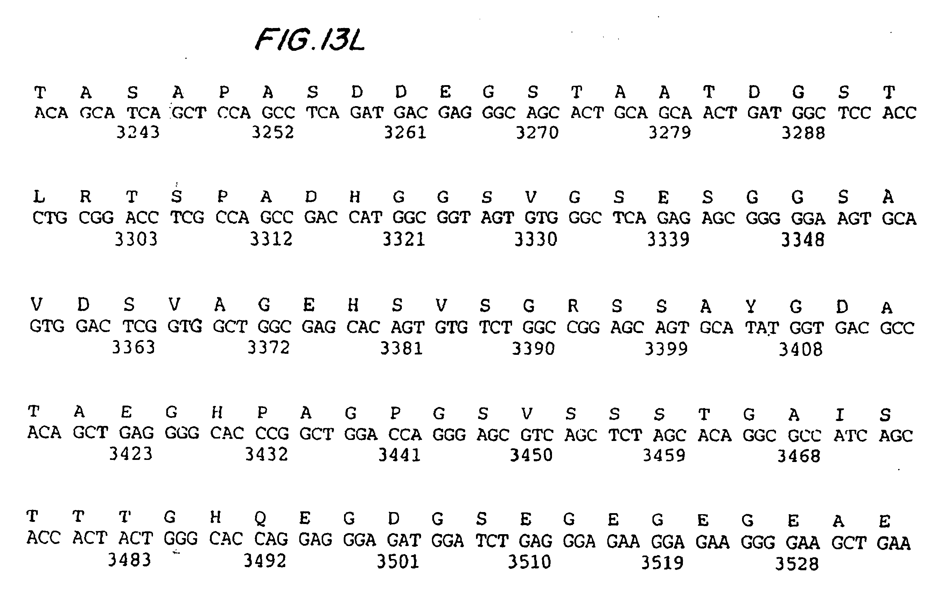

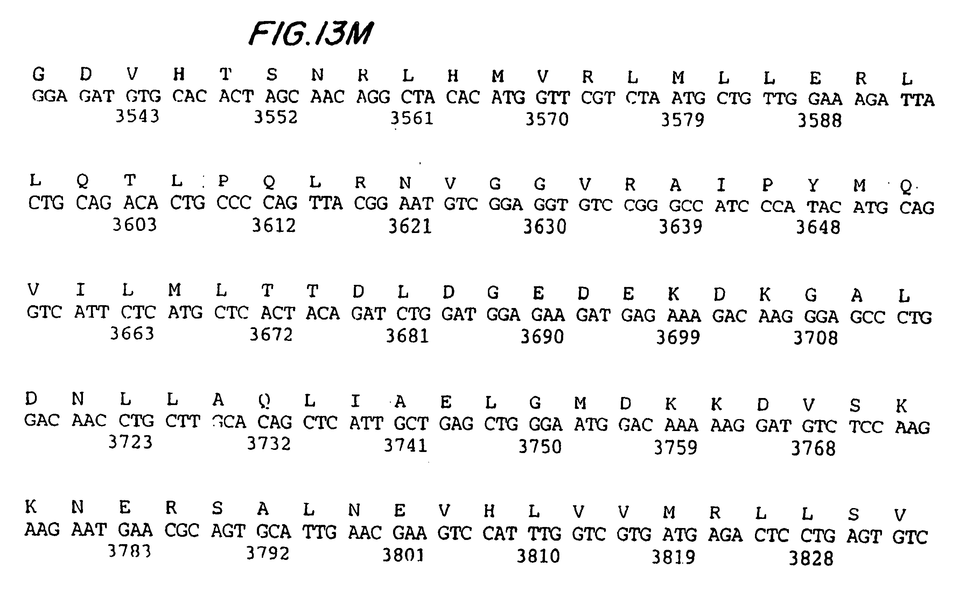

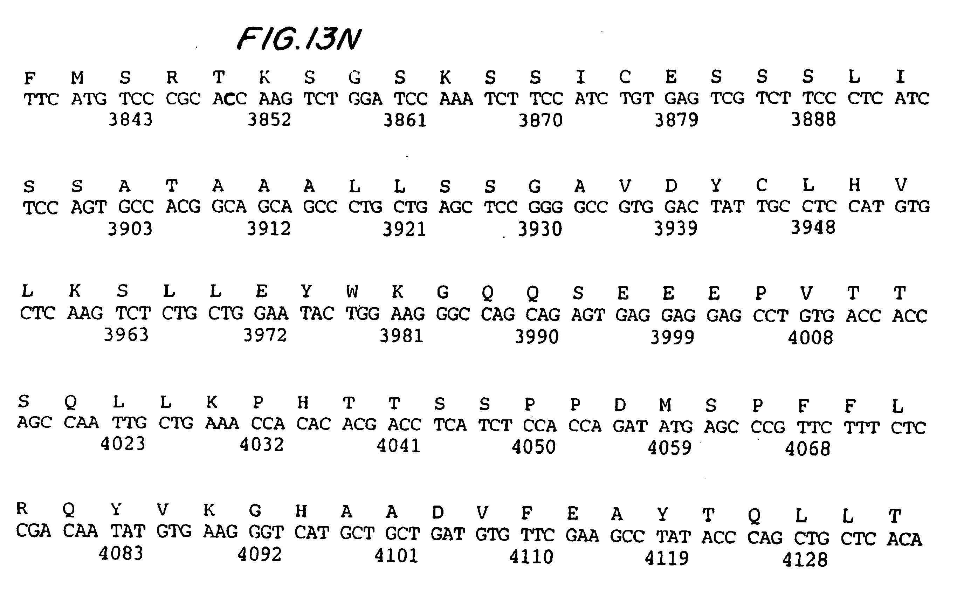

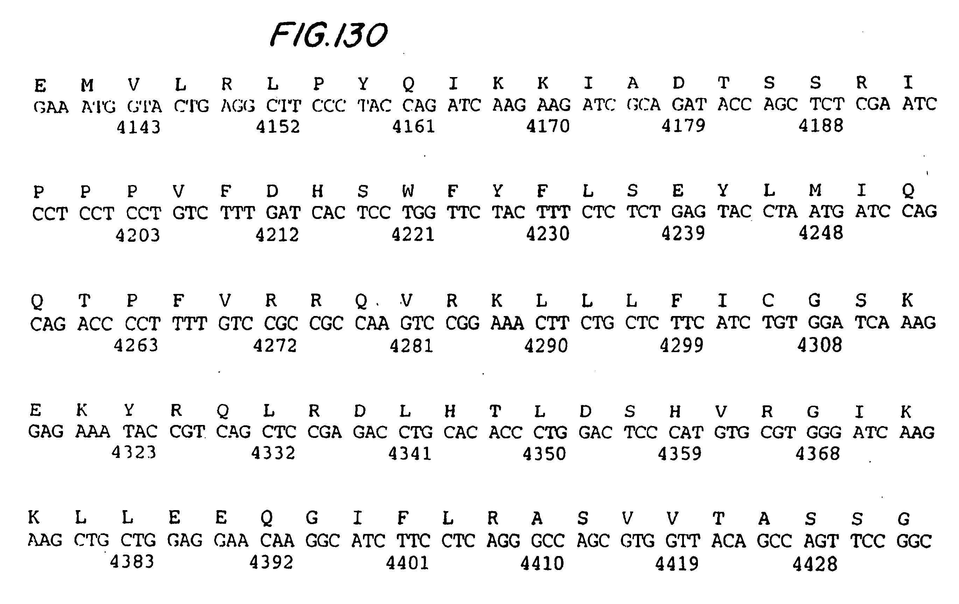

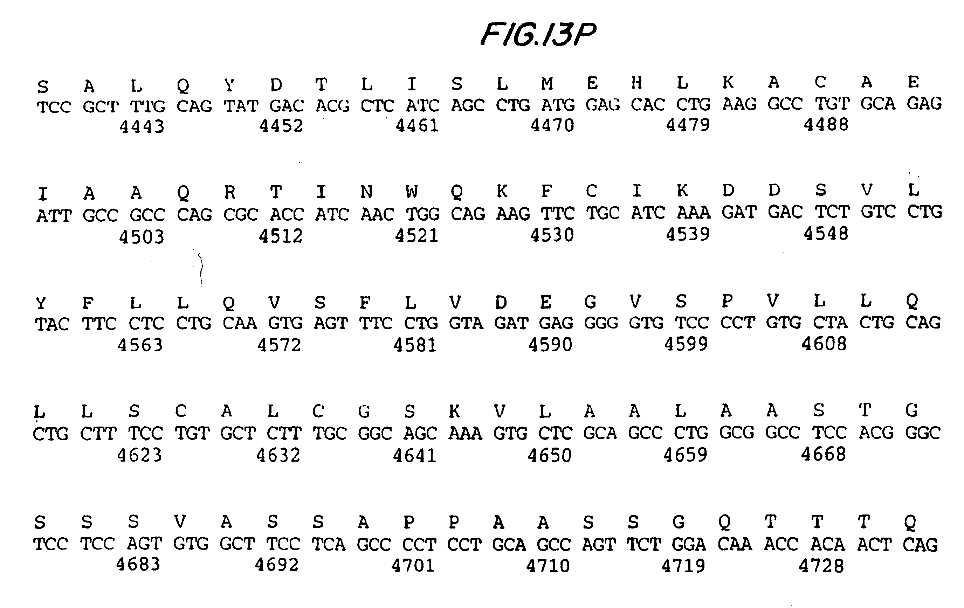

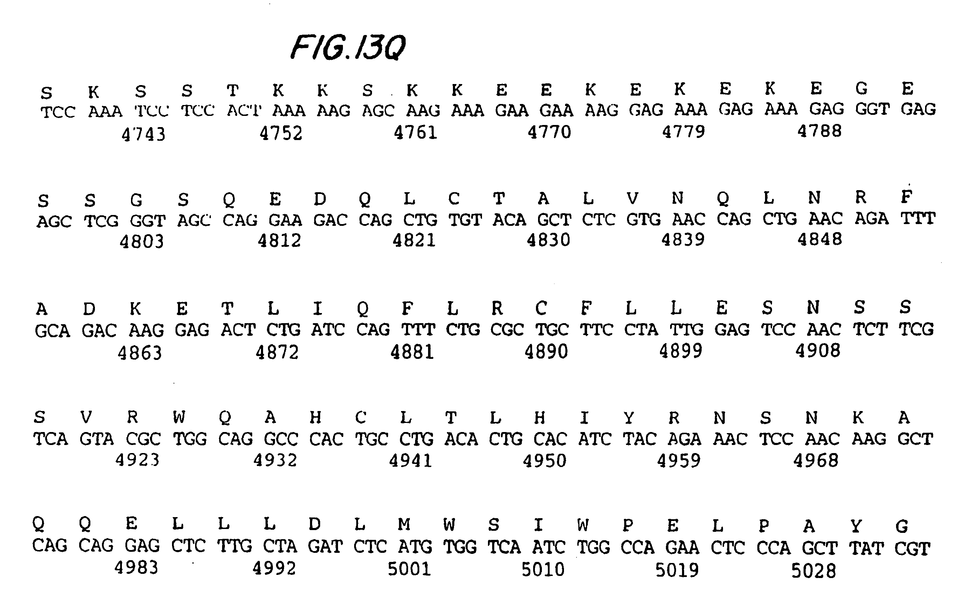

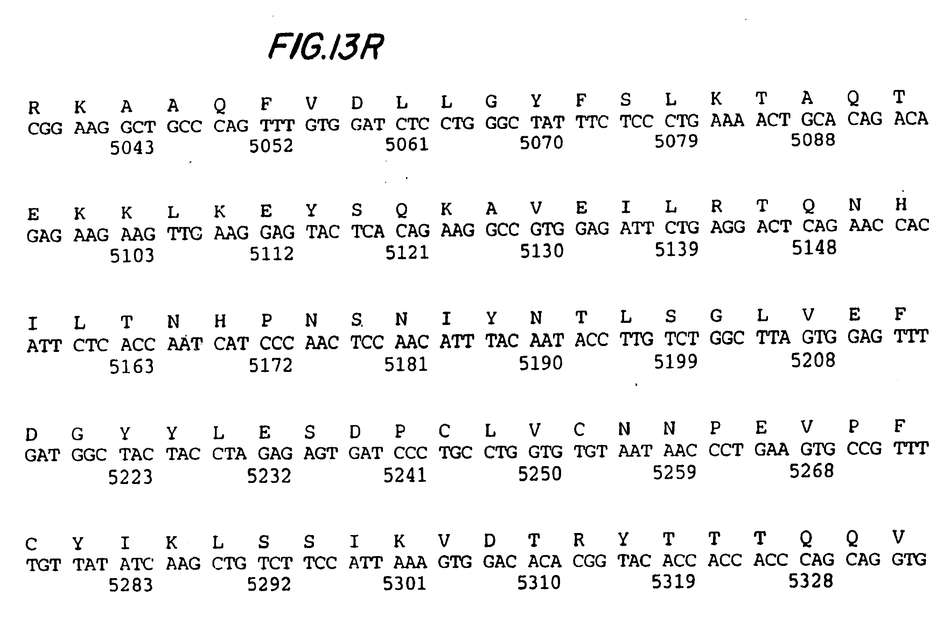

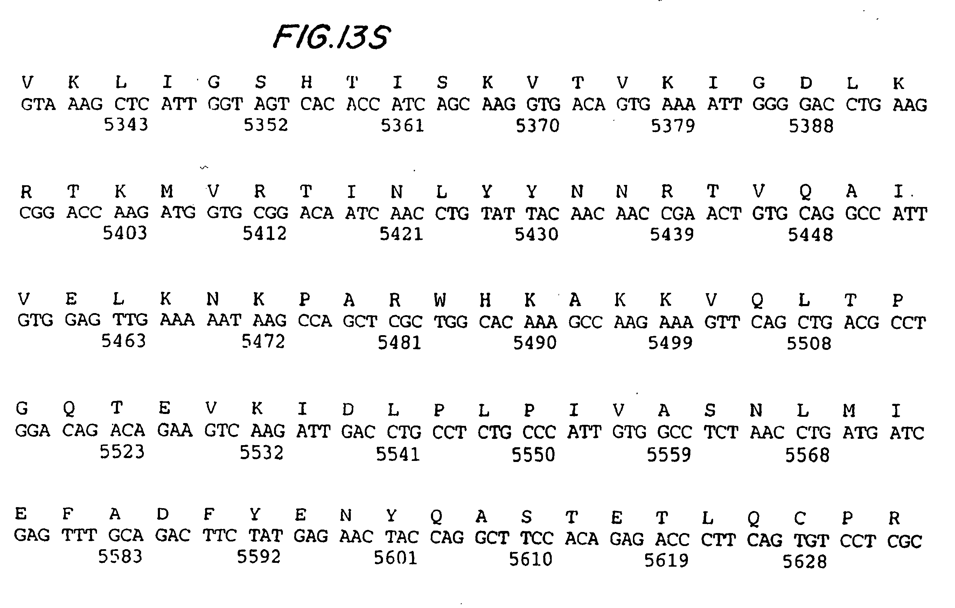

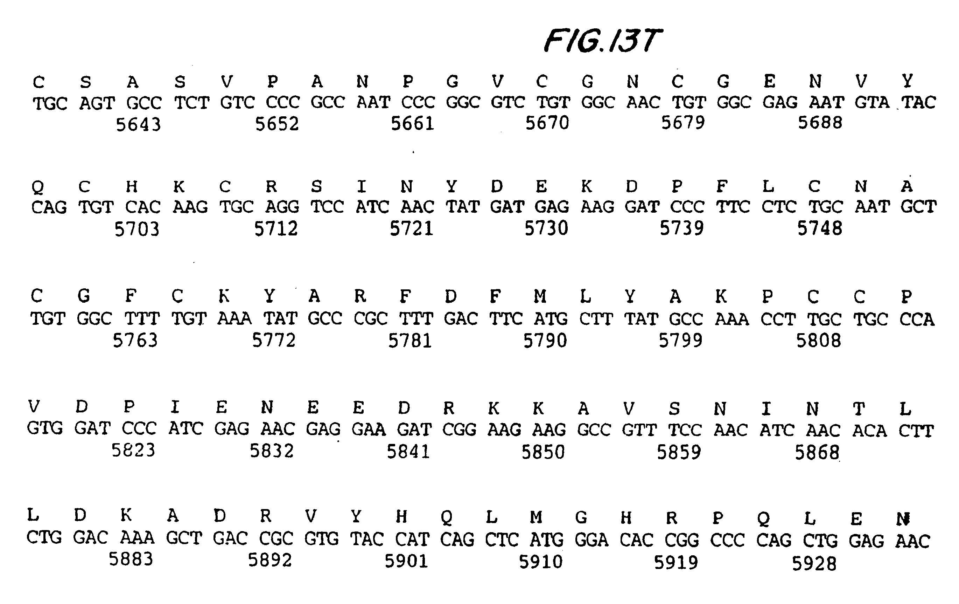

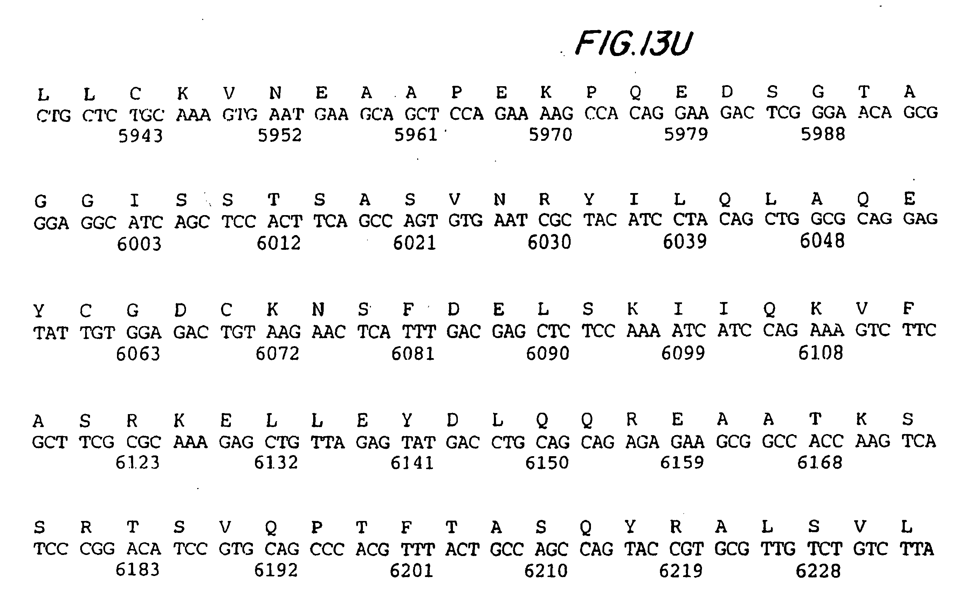

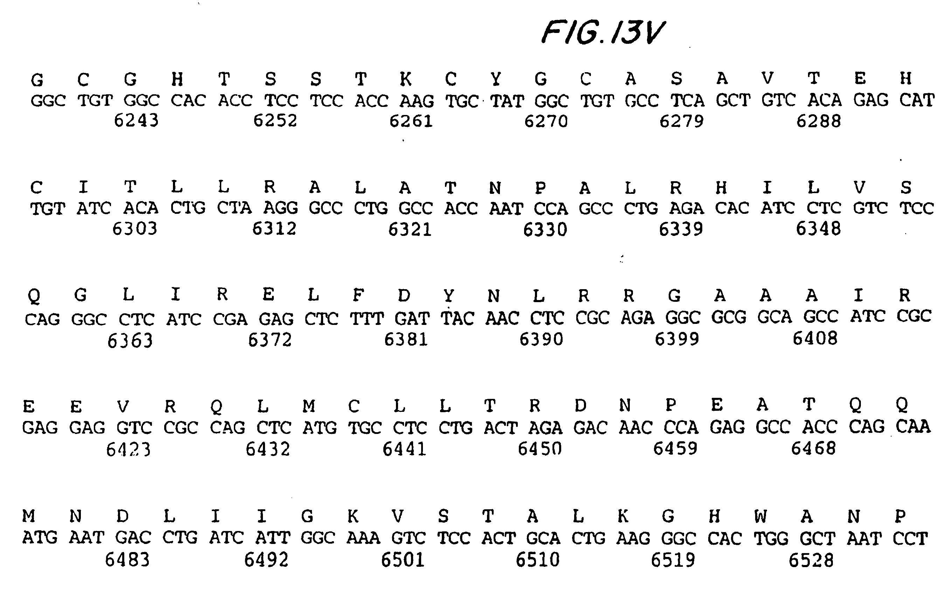

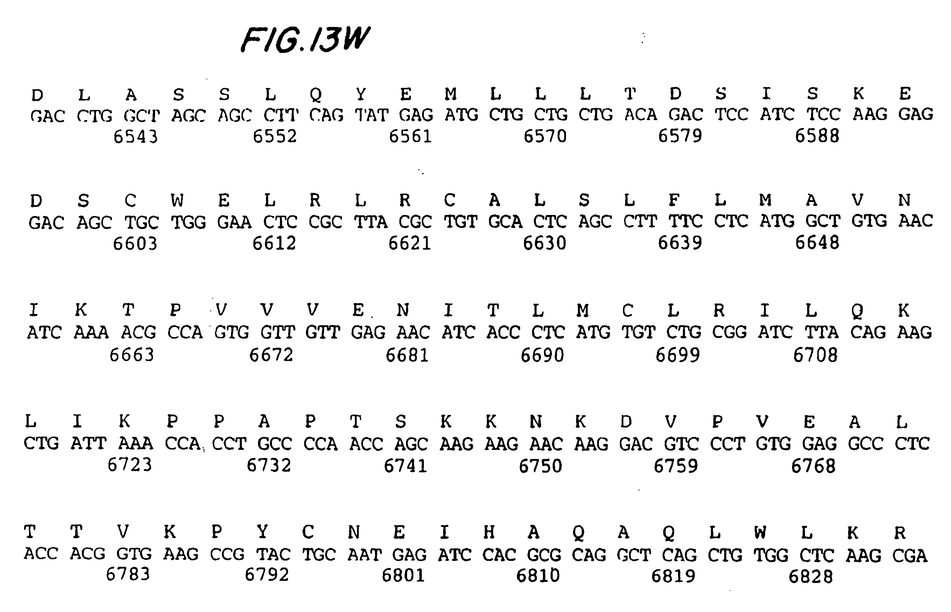

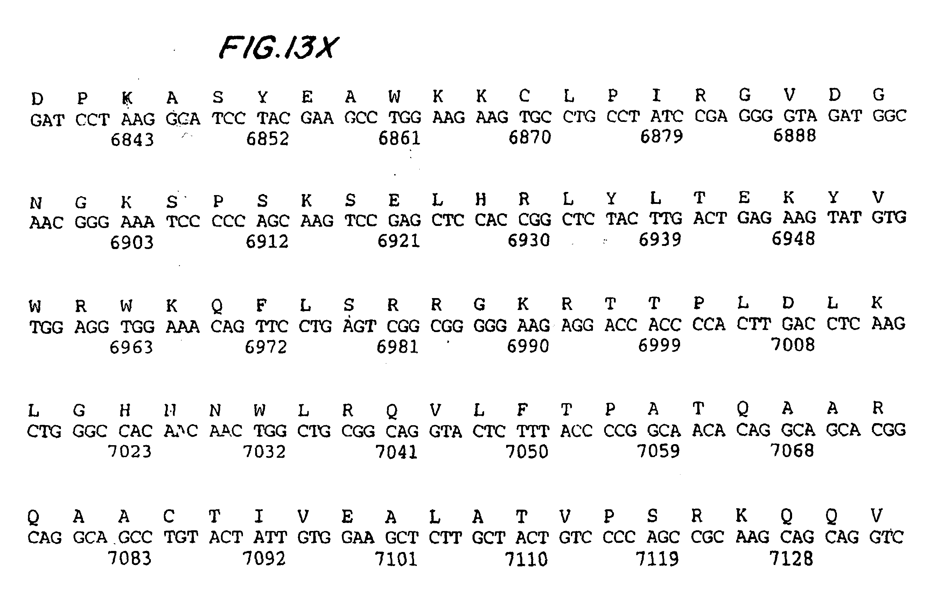

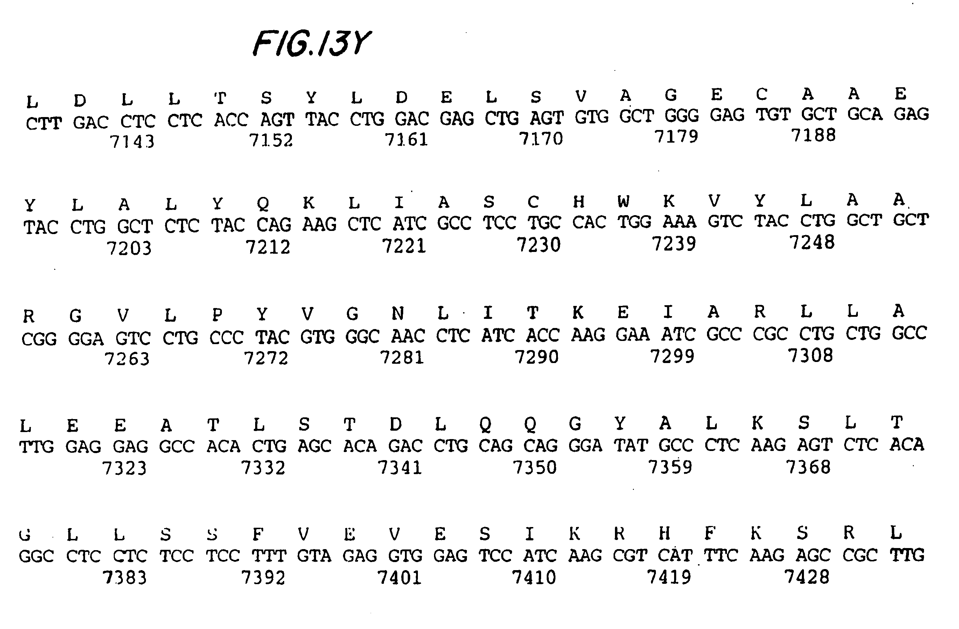

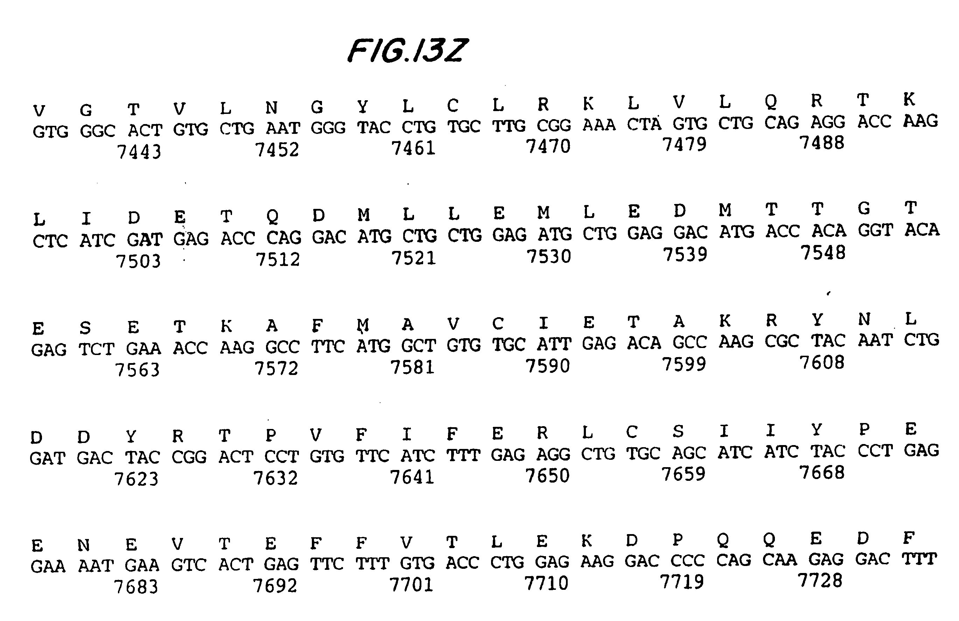

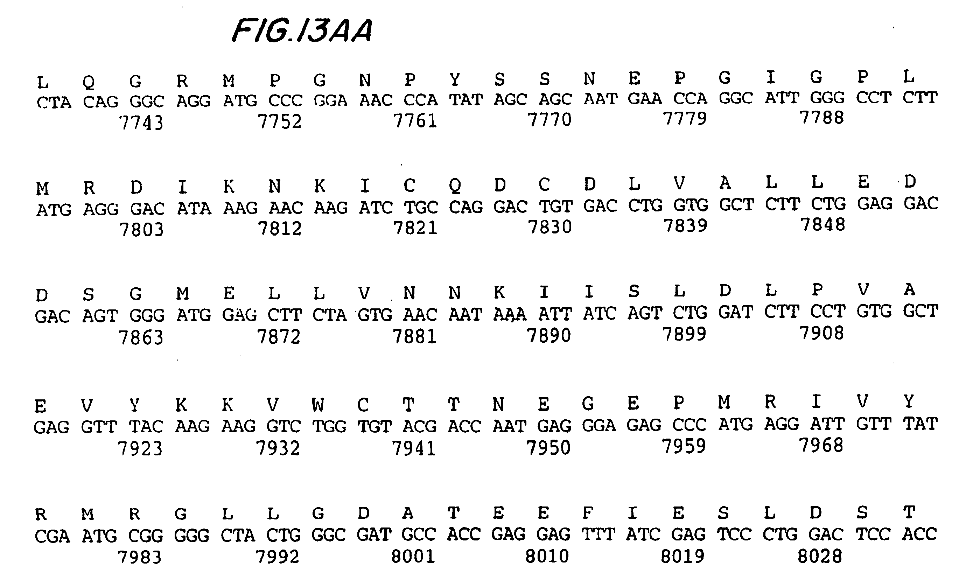

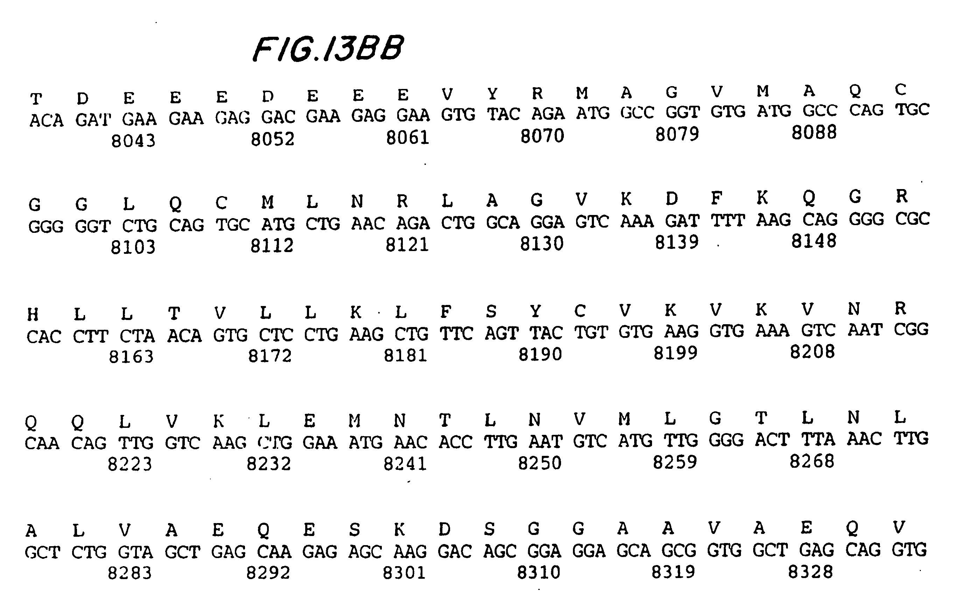

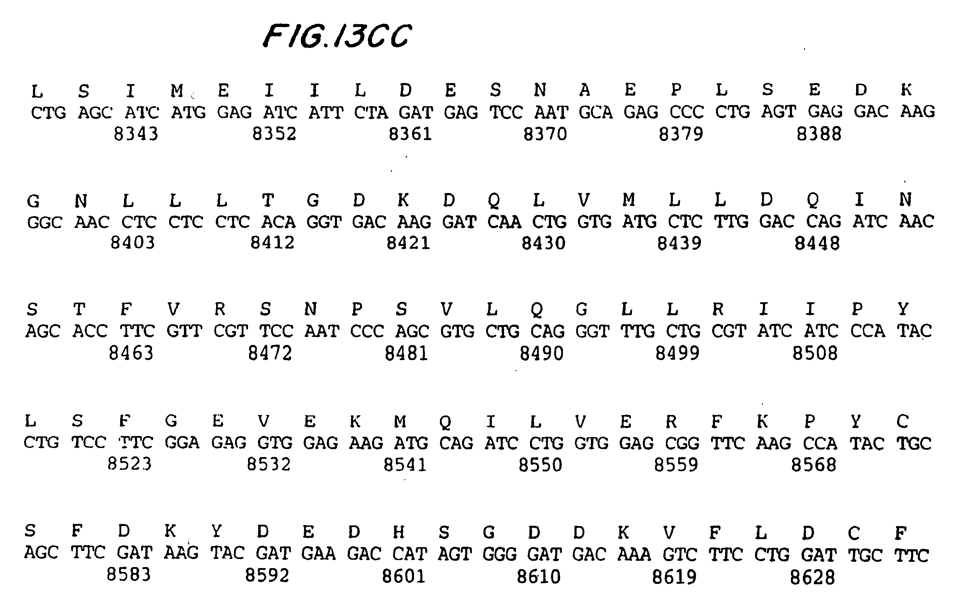

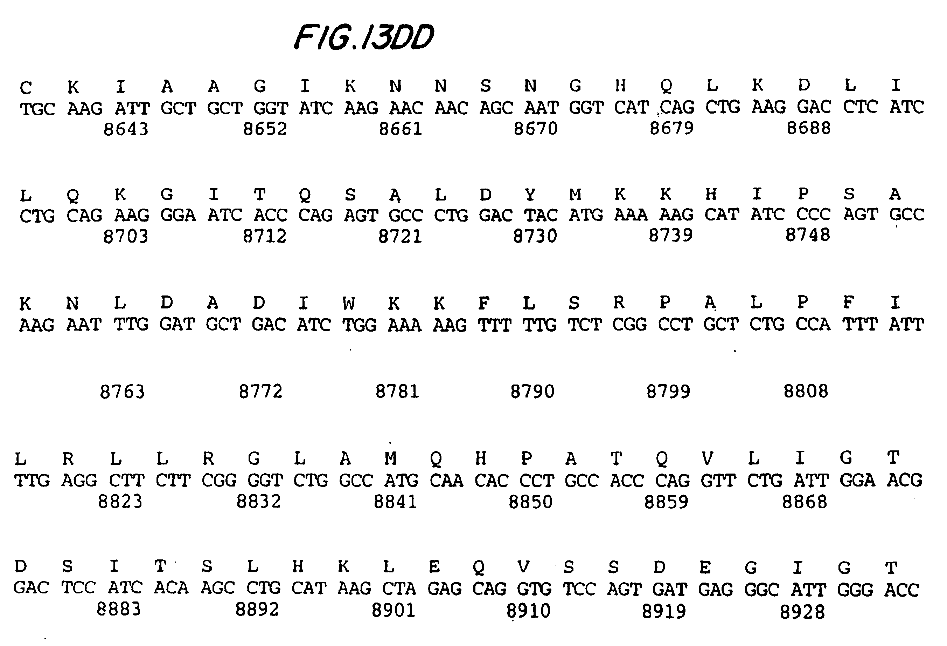

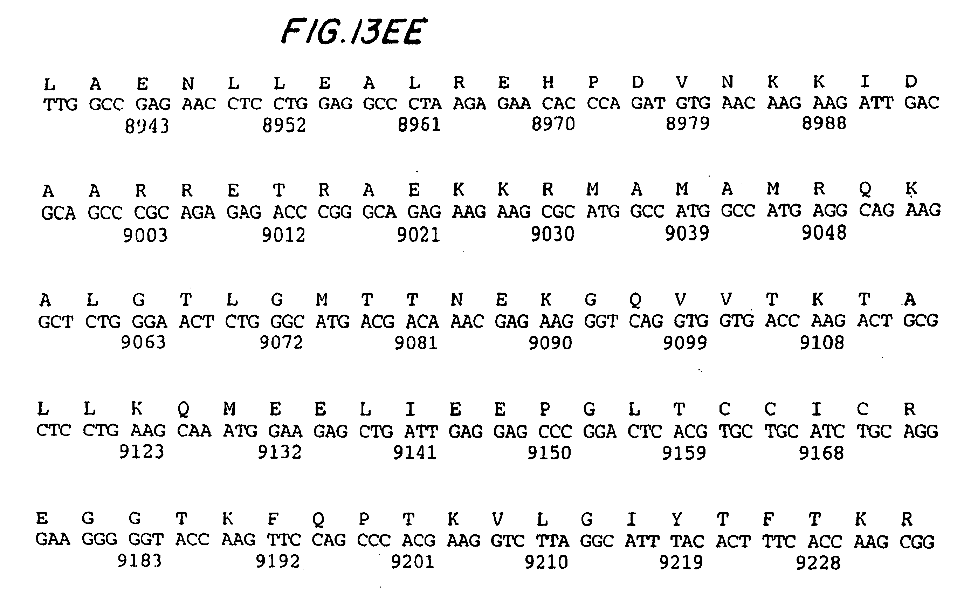

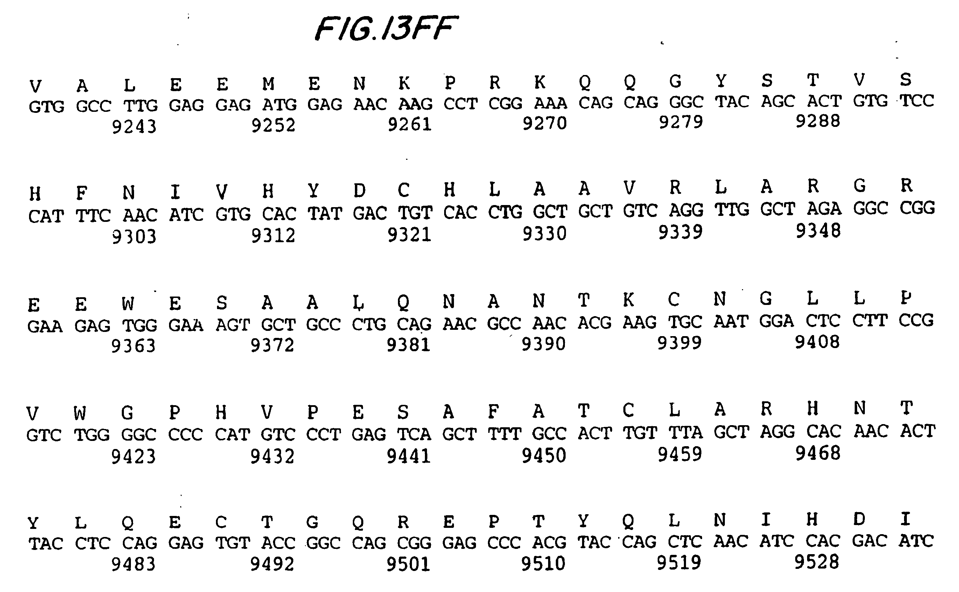

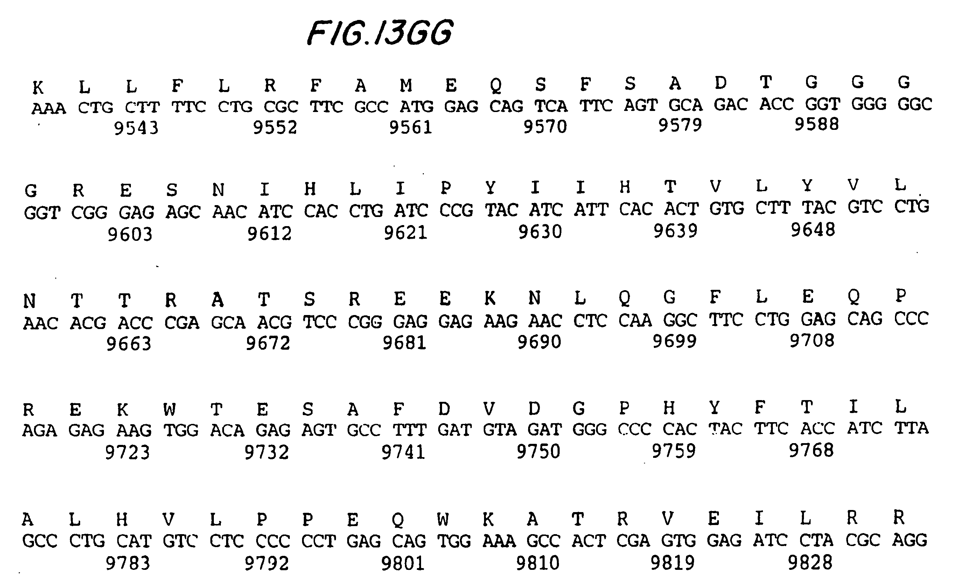

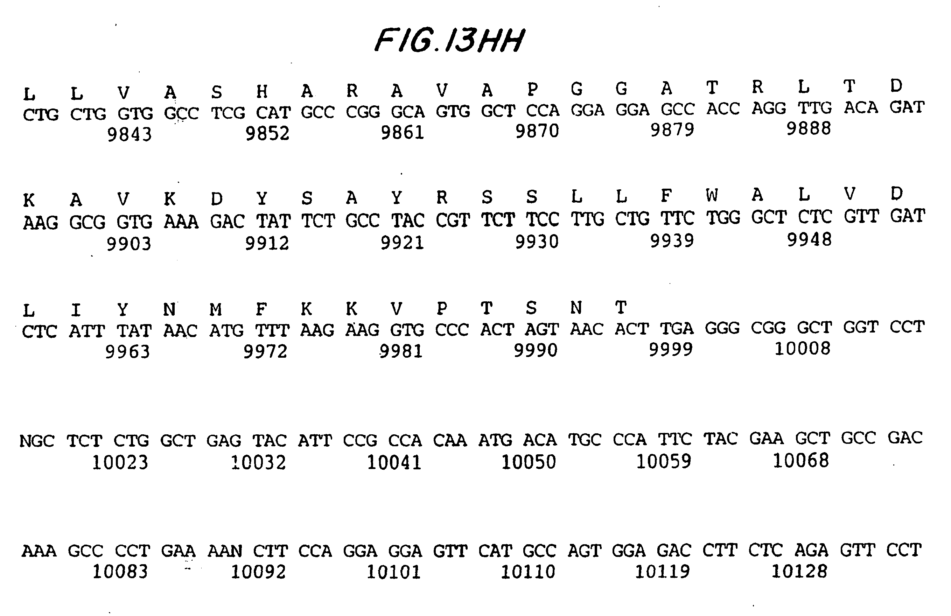

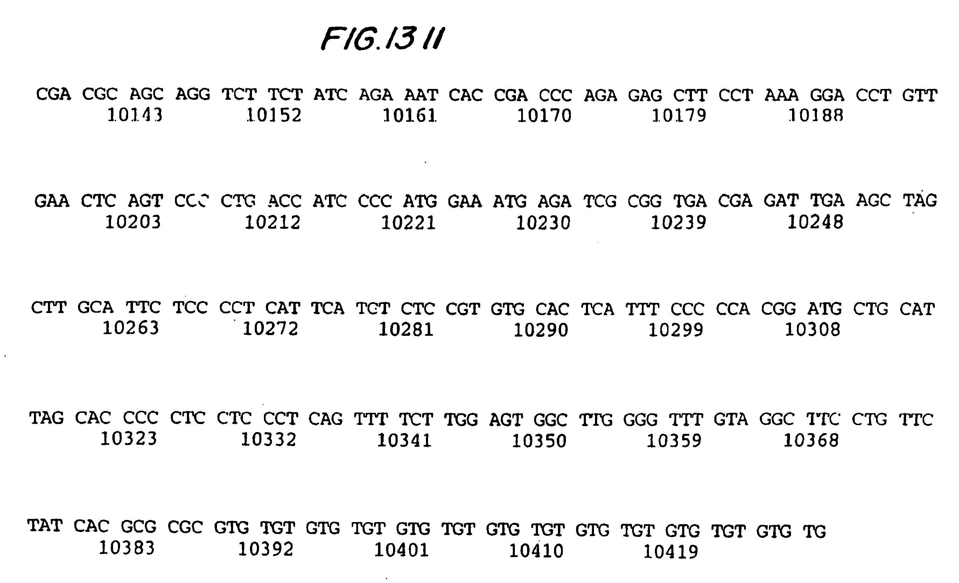

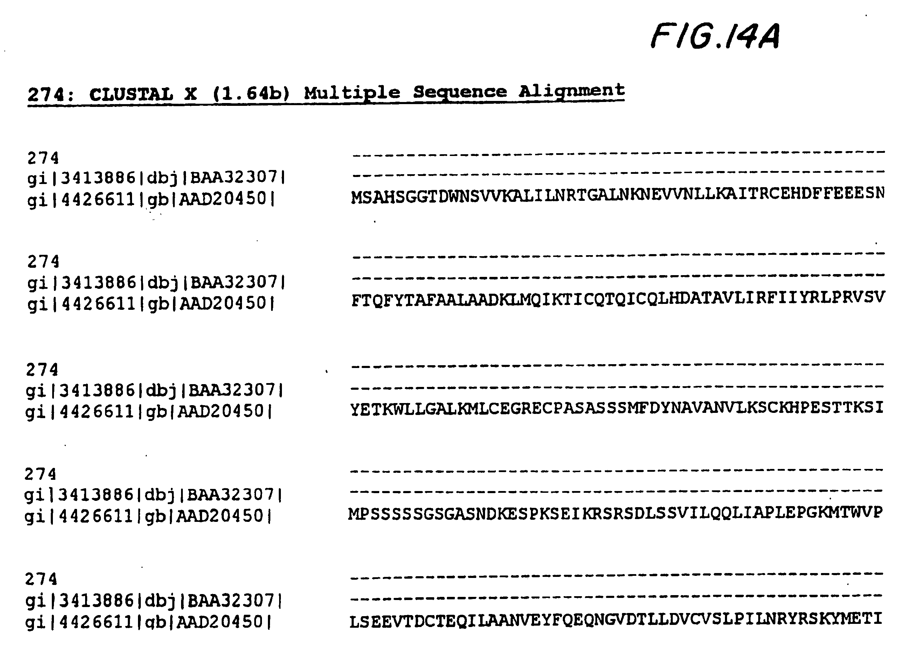

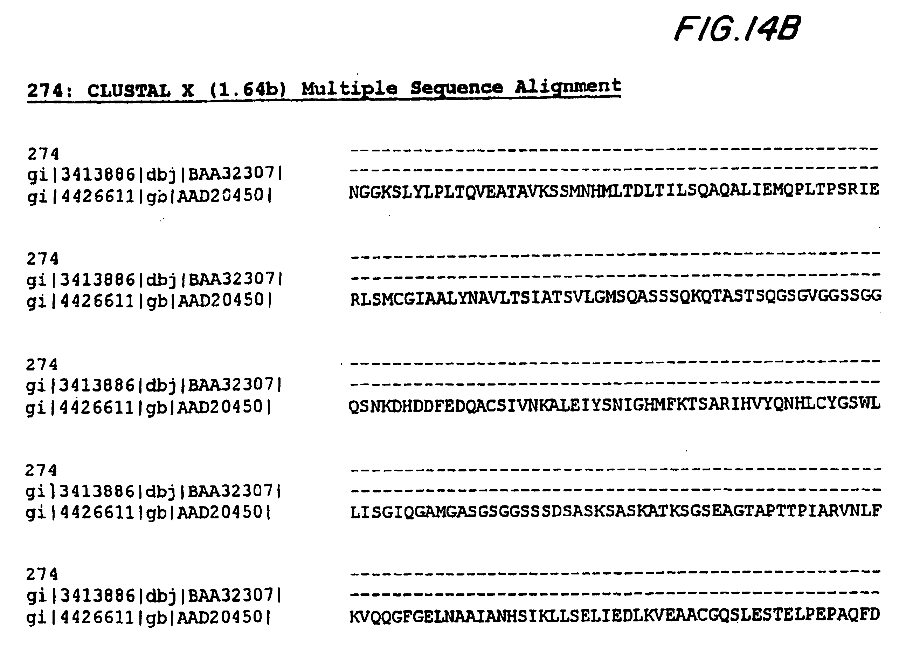

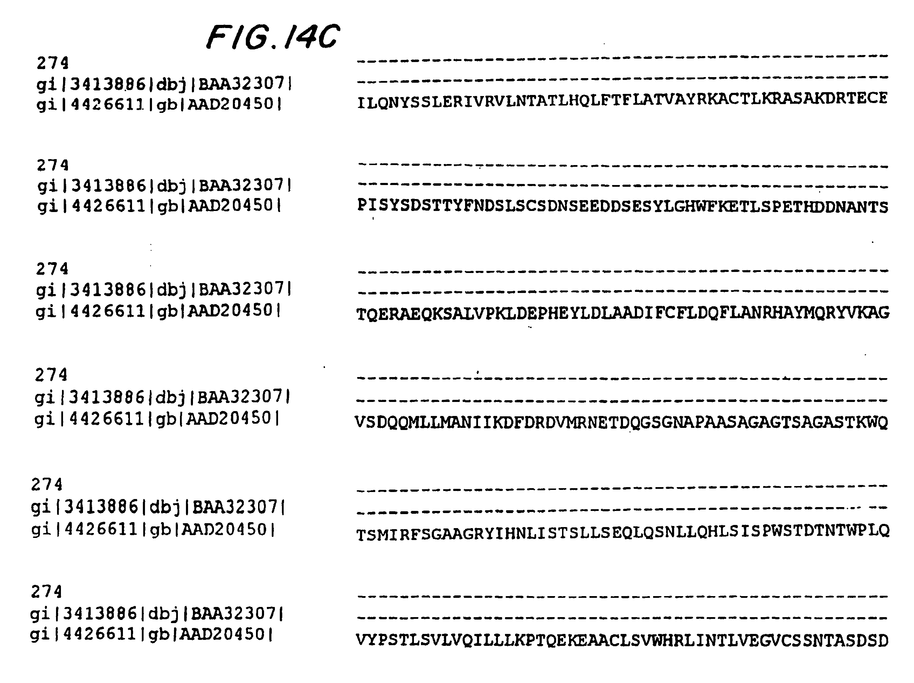

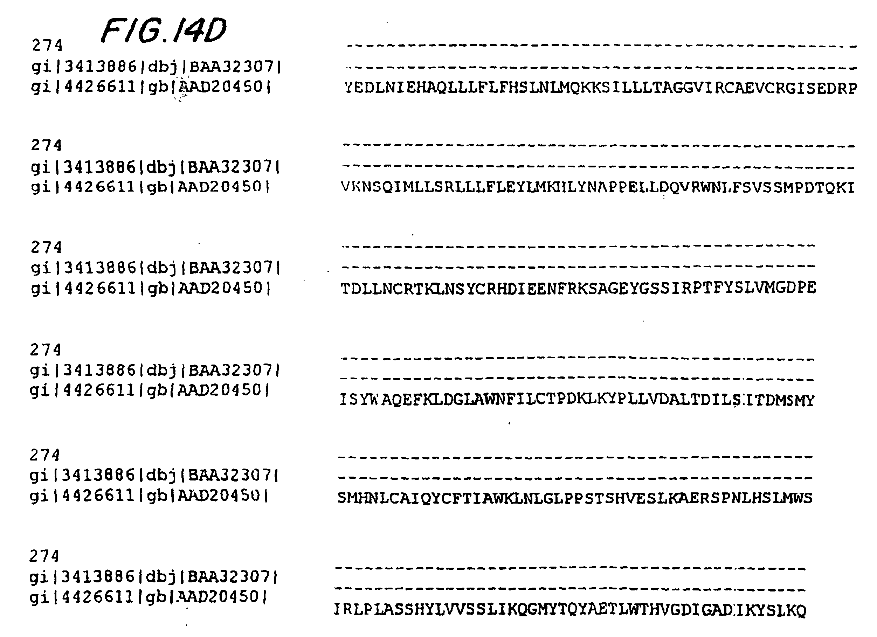

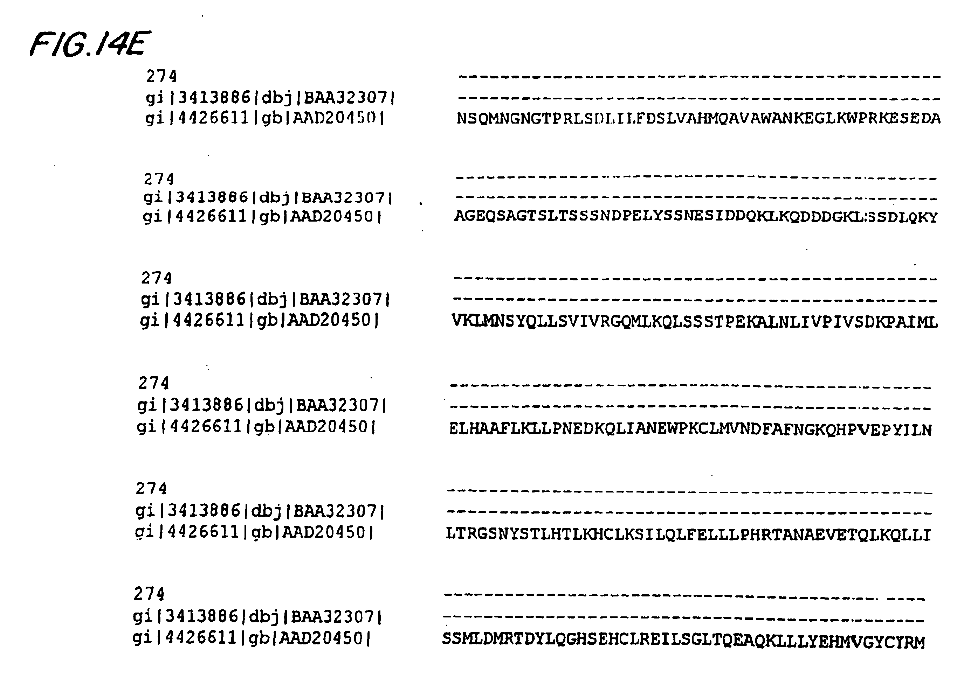

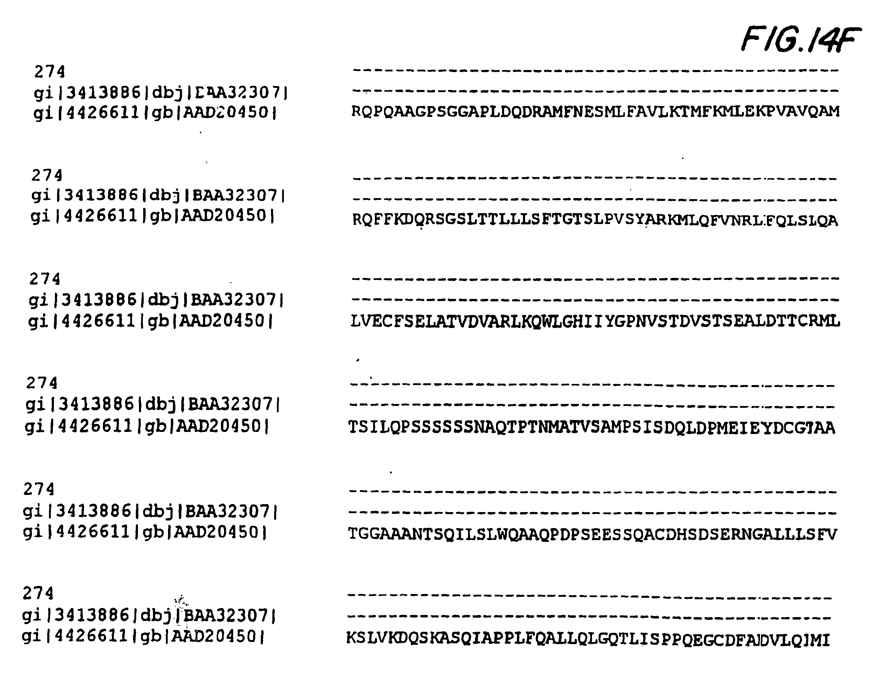

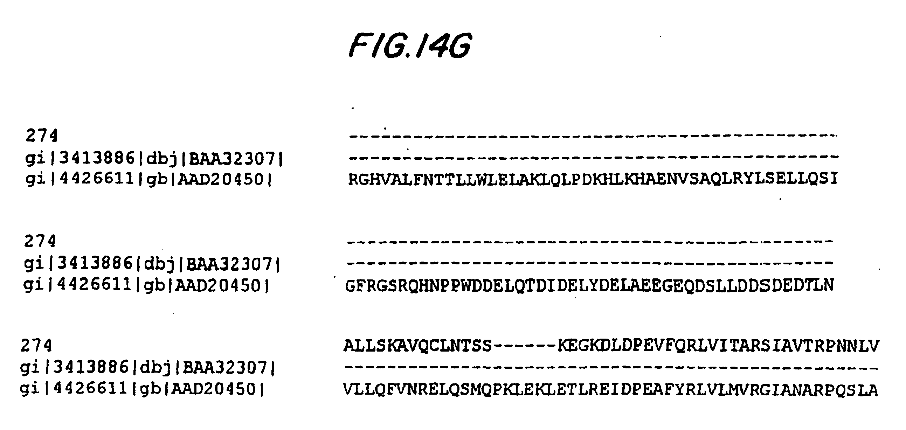

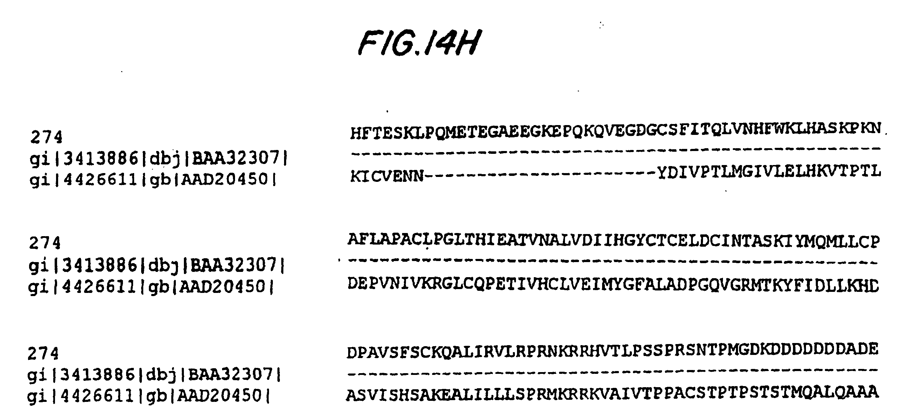

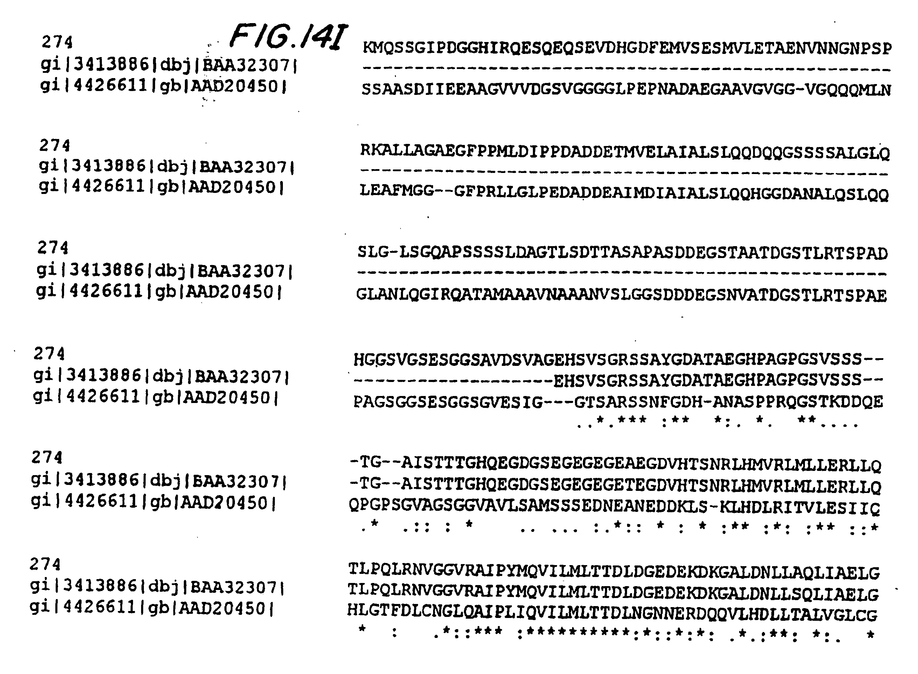

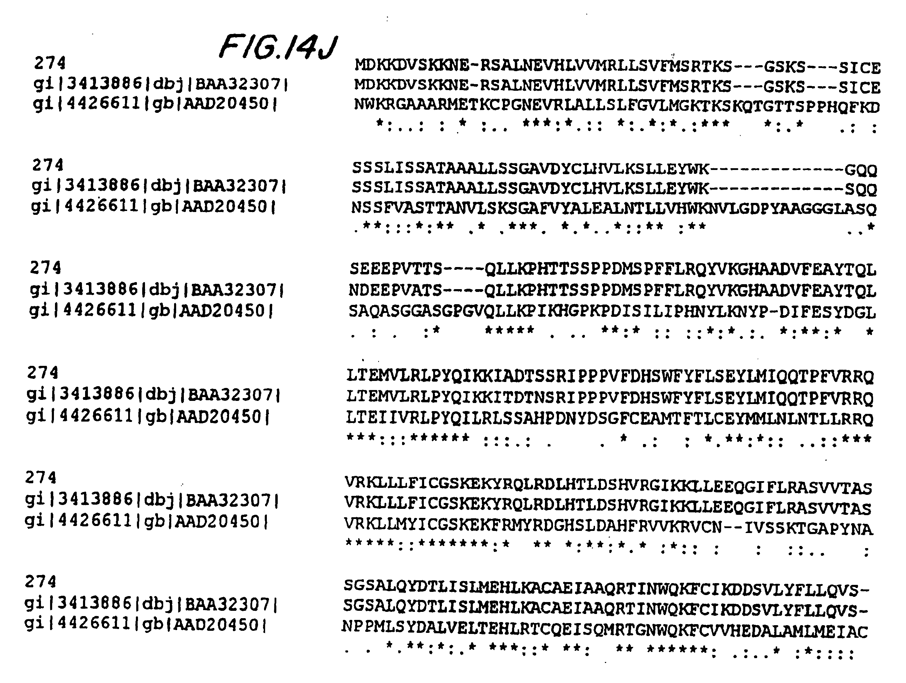

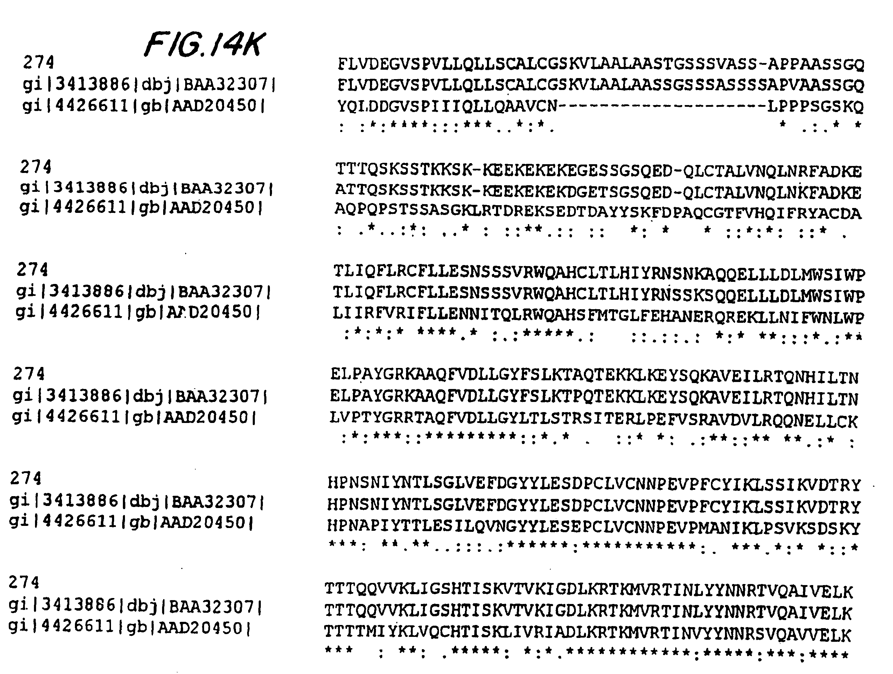

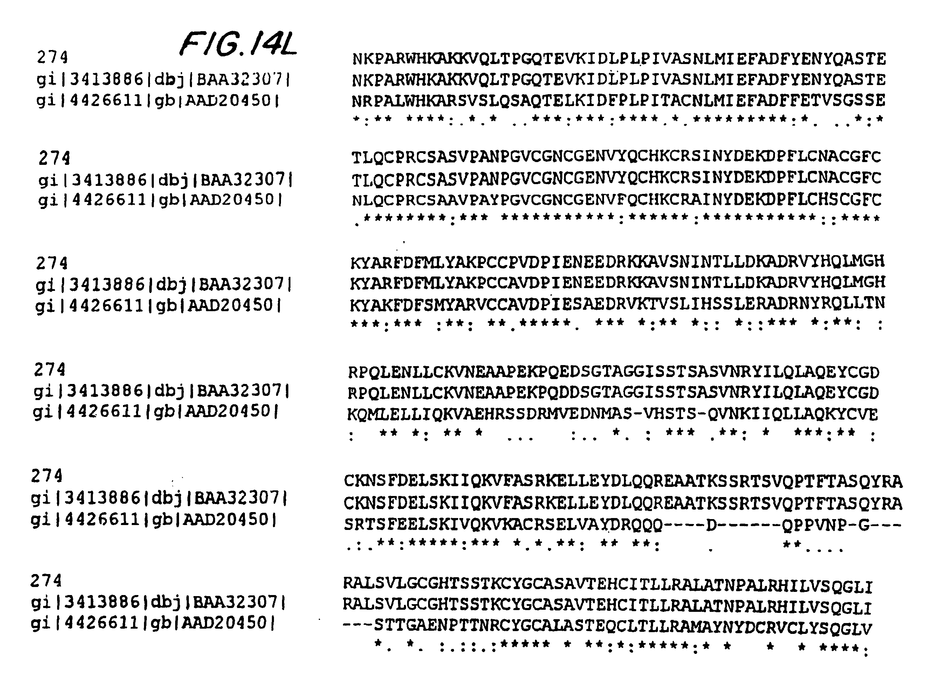

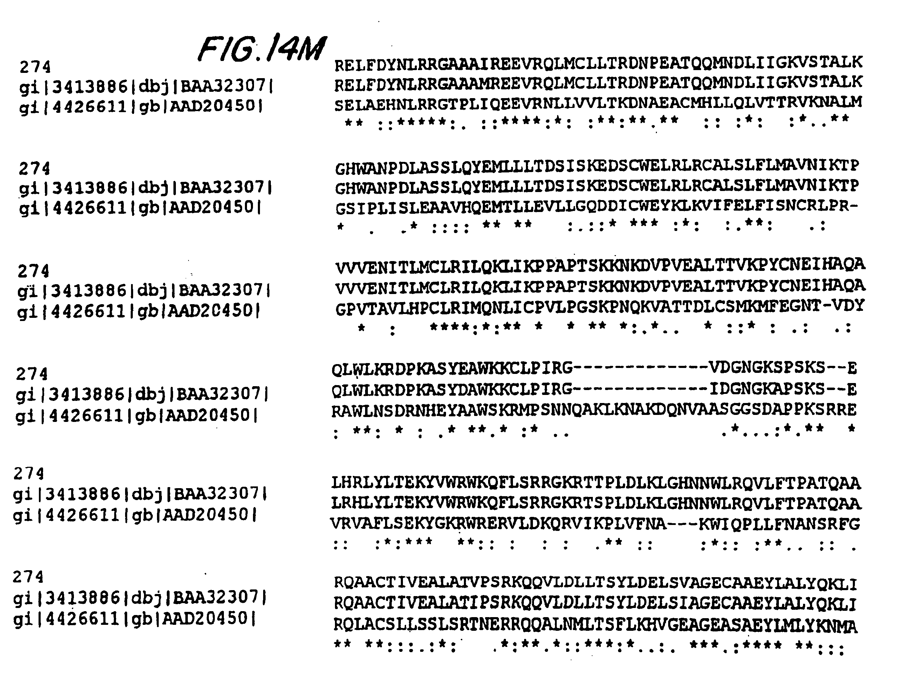

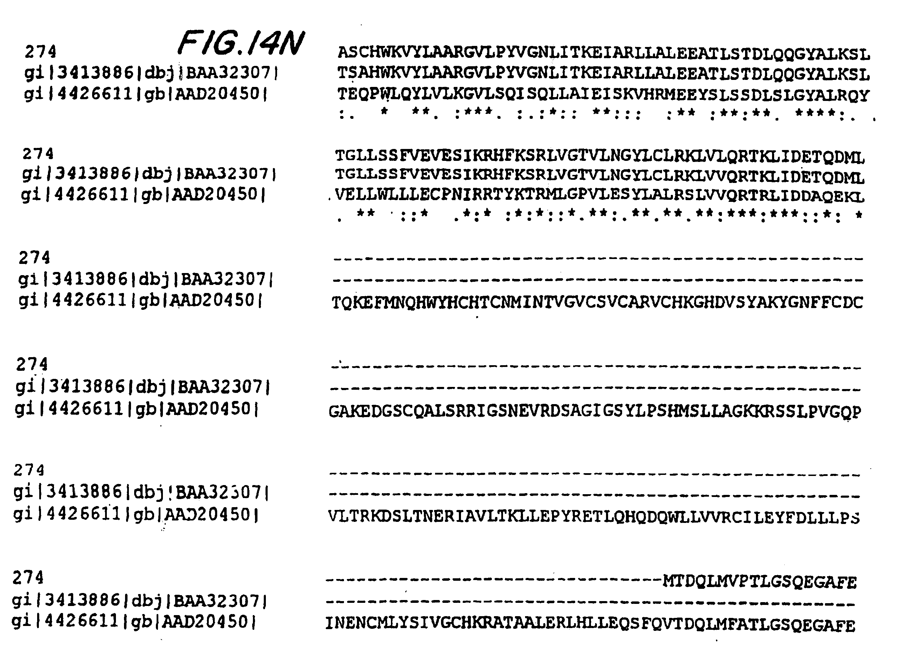

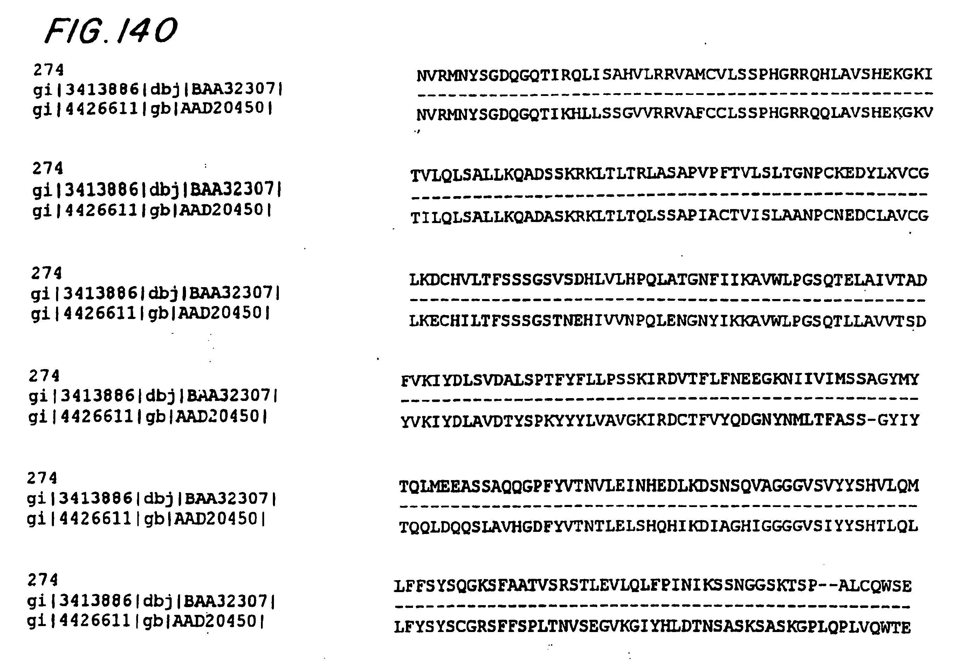

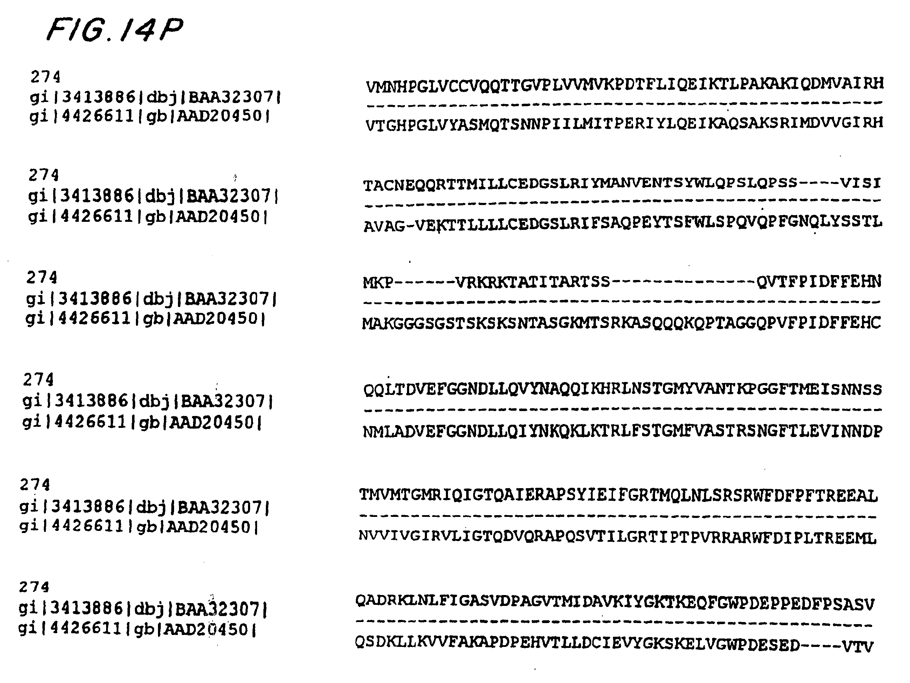

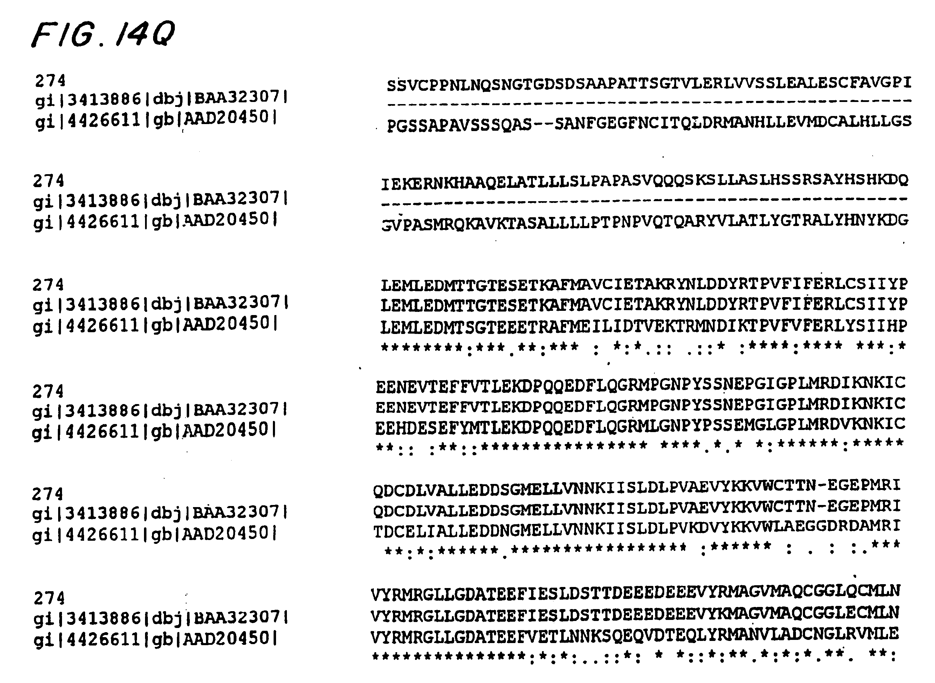

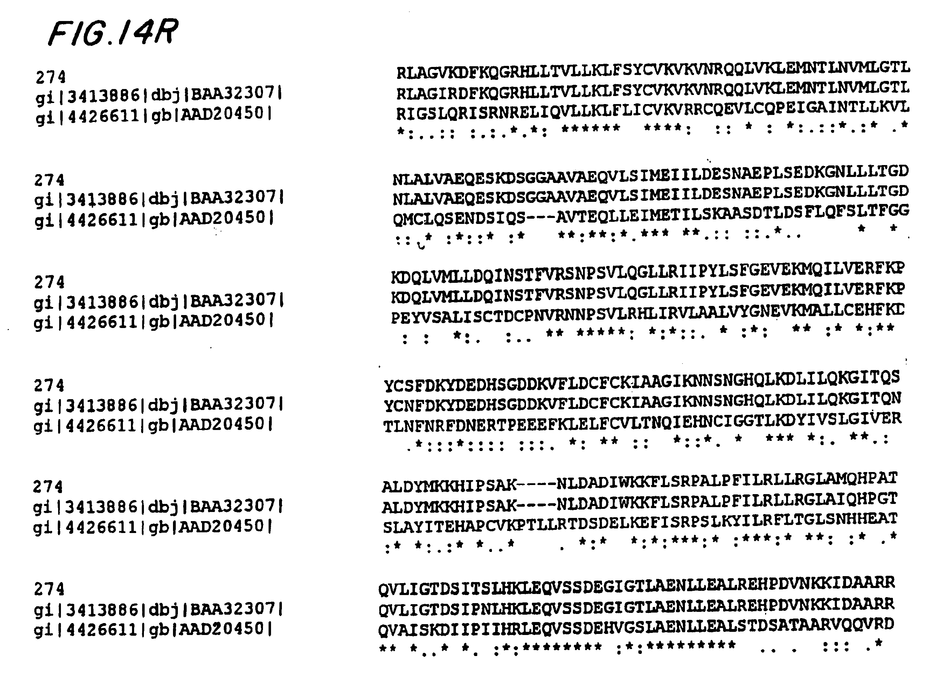

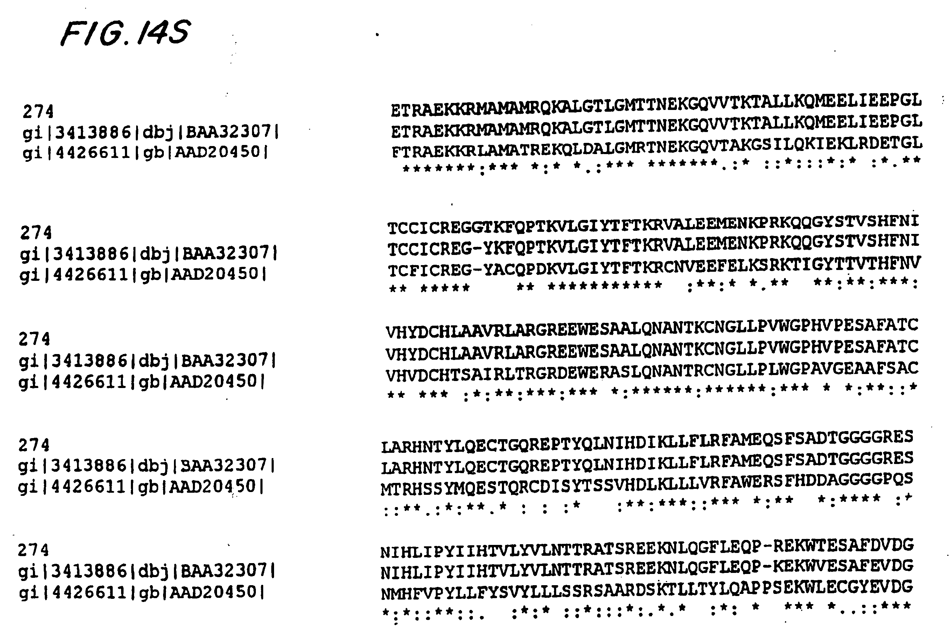

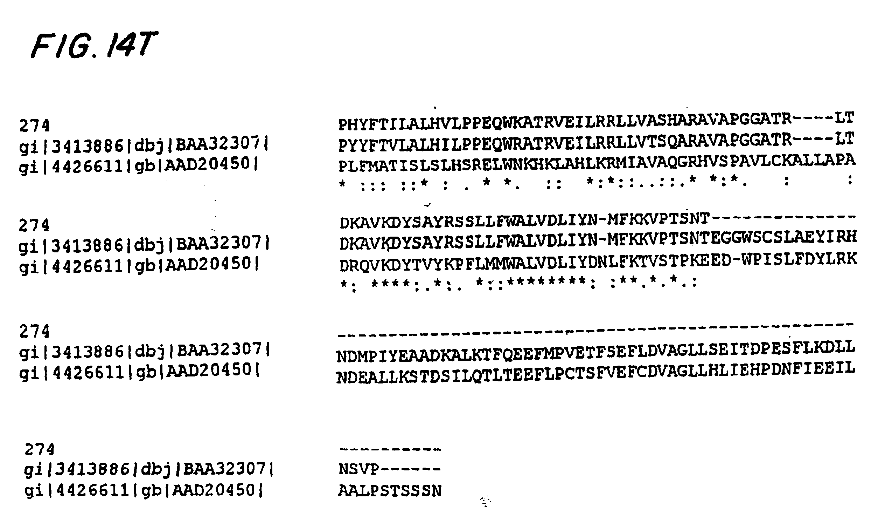

The results from this Example are shown in the Table and sequences of Figure 2A. Novel sequences CMF608, CMF405 and CMF274 were identified, *inter alia,* as discussed below (see also Figs 2A-14).

### AN OSTEOPOROSIS (or mechanical stress) MODEL (calvaria cell cultures).

### Differentially regulated/differentially expressed genes post-mechanical stimulation

### Extracellular matrix, transmembranal and secreted proteins:

tenascin
collagen XII
thrombospondin 1
ADAMTS-1.
C3 complement component
alpha-2-macroglobulin receptor
fibronectin
connective tissue growth factor
endothelin converting enzyme
alpha-2u microglobulin-related protein
RB 13-6

### Genes connected to regulation of apoptosis

SARP1
cytochrome oxidase subunit 1
glutamyl-cystein synthetase

### Genes connected to intracellular fatty acid methabolism

3-hydroxy-3-methylglutaryl coenzyme A reductase
yeast ERG3 homologue
and yeast ERG25 homologue
steamyl-CoA desaturase

### Genes connected to cytoskeleton regulation

AHNAK
filamin
syntrophin 1

### Genes connected to regulation of water channels

aquaporin 1

### Novel genes or known anonymous genes without function

highly charged amino acid sequence
DEST274 (CMF274; see Figs 2A-14)
DEST405 (CMF405; see Figs 2A-14)
DEST608 (CMF 608.; see Figs 2A-14)

### General overview of identified genes.

**Tenascin** is an extracellular matrix glycoprotein whose expression is up-regulated in normal bone development during condensation. It is also involved in genesis and function of articular chondrocytes. Tenascin is secreted by osteoblasts, but is absent from mineralized bone matrix. Expression of alkaline phosphatase activity and **collagen XII** (markers of osteoblast differentiation) are tenascin-dependent (down-regulated by anti-tenascin RNA). Expression of tenascin is markedly increased in response to mechanical stress, its promoter (in chicken) was shown to contain a cis-acting "strain-responsive" element.

Another protein whose expression is known to be modulated by mechanical stress (fluid shear or stretched stress of i.e. mesothelial cells) is endothelin (**endothelin converting enzyme** that generates active endothelin molecules from inactive intermediates is upregulated in the present screen). In bone, endothelin stimulates the osteoblastic IL-1-induced production of IL-6 - mediator of osteoclastic differentiation, function and probably survival. Receptors to endothelin were demonstrated in osteoblastic cells by ligand binding (autocrine loop). Major endothelin signal transduction pathways in bone cells is stimulation of phospholipid turnover, by activation of phospholipases A, C, and D, stimulation of Ca flux from intra- and extracellular stores and activation of tyrosine kinases. Endothelins also modulated calcium signaling elicited by other agents (i.e. potentiation of PH-stimulated Ca transient) in osteoblastic cells. Phenotypic responses to endothelin include stimulation of osteocalcin and osteopontin messages (see herein), inhibition of osteoclast motility and stimulation of prostaglandin-dependent resorption.

One protein exhibiting channel characteristics was found upregulated. It is **aquaporin1 -** a water channel protein expressed in many fluid secreting and absorbing tissues such as kidney, brain, heart, eye, inner ear. Its promoter contains glucocorticoid responsive elements and can be activated in response to dexamethasone treatment. Induction of aquaporin-1 expression was detected by subtracted cloning of genes upregulated following cardiopulmonary bypass and reperfusion. However, its induction is delayed compared to inflammatory mediators (i.e. ICAM-1, E-selectin, IL-8). The only bone link can be traced in localization of aquaporin molecules in the inner ear, but this localization can be easily explained by the critical dependence of inner ear function on fluid homeostasis. In ear, the protein was found in close association with bone - in most of the cells lining the bony labirynth, and in other non-bony locations.

**AHNAK** (other names: neuroblast differentiation factor, desmoykin) - a 700 kD protein that was originally identified as differentially repressed (lost) in neuroblastoma cells. Its body is constituted of 128 amino acid repeats. The protein was initially identified as a nuclear one. However, when it was rediscovered under the name "desmoykin", its subcellular localization was reported as membranal (at the sites of desmosomes). AHNAK-like repeats were found in another protein VAP-1 (vesicle associated protein) - a novel high molecular weight protein found in sea urchin eggs. It is located at peripheral membrane in association with microsomal membrane fraction. Within AHNAK-like repeats of VAP-1 RNA -binding sequences - of RNP 1 and of RNP2 types (the same is true for AHNAK). Therefore, it is tempting to speculate, that the general increase in expression of secreted proteins observed in bone tissue in response to mechanical stress might dictate the need in the upregulation ofRNA-binding protein localized to a microsomal fraction.

**Filamin** (non-muscle type), ABP-280, plays a critical role in stabilizing the membrane-cytoskeletal interactions. It is a dimeric actin crosslinking protein that provides the major mode for attaching the cortical F-actin network to membrane glycoproteins. One fillamin molecule is able to crosslink up to 1,000 actin molecules. This ability makes filamin the most potent actin crosslinking agent known today.

**Syntrophin 1** is a member of multigene family of intracellular extrinsic membrane proteins found in complex with dystrophin. This particular syntrophin was demonstrated also in complex with nitric oxide (NO) synthase (in muscle tissue). The interaction is likely to be mediated by PDZ domains found in both proteins, but formation of this complex is probably dystrophin-dependent. NO is known to be implicated in the metabolism of bone, especially as a mediator of cytokine effects on remodeling of bone tissue in response to diverse stimuli such as pro-inflammatory cytokines, mechanical stress and sex hormones. Both estrogen and mechanical stress increase NO production by activating constitutive nitric oxide synthase. High concentrations of NO inhibit bone resorption by inhibiting osteoclast formation and by inhibiting the resorptive function of mature osteoclasts, whereas lower NO concentrations potentiate bone resorption and may be essential for normal osteoclast function. On the other hand, growth and differentiation of osteoblasts are also inhibited by high NO concentration.

**Thrombospondin 1** - a 450 kD adhesive glycoprotein involved in cellular attachment, spreading, proliferation, and migration. It was originally isolated from plateletes and endothelial cells, but it is also localized in osteoid of undermineralized fetal subperiosteum and in mineralized bone matrix of neonetal/young (growing) bone. TSP-1 can specifically interact with osteonectin - a 30 kD protein of bones and plateletes. This complex formation is Ca-dependent. In osteogenesis imperfecta, levels of osteonectin are reduced, while production ofthrombospondin is increased. Expression of trombospondin is a marker of osteoblast differentiation (together with alkaline phosphatase and alpha-1-collagen). Dexamethasone treatment decreases the levels of thrombospondin expression in cultured osteoblastic cells (glucocorticoids induce osteoporosis). 17-beta estradiol, on the contrary, induces trombospondin expression. Thrombospondin-1 gene expression is modulated during pericytes differentiation in vitro (pericytes are cells that are embedded within basement membrane of microvessels, believed to participate in angiogenesis, but are able to differentiate into osteogenic phenotype). It is markedly increased during nodule formation and then decreased when mineralization of the nodules has taken place. TSP-1 is excluded from the inner mass of such mineralized nodules.

Several non-trombospondin genes were found to contain **thrombospondin** motifs (cell-binding domain of thrombospondin). One of them also belongs to a metalloproteinase-disintegrin family (identified as an up-regulated gene in the present screen) - **ADAMTS-1.** It was initially cloned as a gene that is selectively expressed in cachegenic (in vivo) colon 26 adenocartsinoma subline. It is a putative secreted protein without transmembranal domain. ADAMTS-1 contains six protein modules: pro-, metalloproteinase, disintegrin-like. TSP type 1 motif, spacer, C-terminal TSP motifs.

Another TSP-motifs containing protein is properdin - a plasma glycoprotein which stabilizes the C3nBb enzyme complex of the alternative pathway of the complemet system through TSP motifs binding. Interestingly these motifs are aslo found in terminal complement components C6 - C9.

**C3 complement component** is produced by osteoblastic and marrow-derived stromal cells in response to vitamin D and regulates differentiation of mononuclear phagocytes into osteoclasts. This effect is bone-specific, since C3 serum, unlike bone, concentrations were unaffecetd in vitD-deficient mice. In normal mice the C3 protein is located manely in periosteal regions of calvaria and on the surfaces of bone trabeculae in tibial metaphyses. It is suggested that C3 deposition on mineralized bone surfaces mediates recrutement of mononuclear osteoclasts (unlike multinuclear, express C3 receptor) to this site. In biological fluids, activated C3 in complex with **alpha-2-macroglobulin** (whose receptor was found to be upregulated in this screen - this **receptor** is known to be expressed by bone marrow macrophages, so, probably, osteoclast precursors can be alpha-2M-receptor positive as well) binds IL-1. It is worth noting, that IL-1 is considered as one of the stimulators of osteoclastogenesis and treatement of ovariectomized mice with its inhibitor significantly decreases the bone loss. Increased osteoclast development after estrogen loss is also mediated by IL-6. Both cytokines expression is upregulated in vivo and in vitro following estrogen deprivation.

It seems now proven that estrogen induces apoptosis of bone-resorbing osteoclasts being applied directly. On the other hand, estrogen induces TGF-beta 1 production by osteoblasts, and anti-TGF-beta antibodies, in turn, can inhibit the estrogen-induced apoptosis of osteoclasts. In this light, finding of **SARP1** upregulation is of special interest. SARPs are a family of secreted apoptosis-related proteins. SARP1 was initially identified as a component of conditioned medium collected from quiescent cells, responsible for apoptosis resistance. SARP2, on the contrary, induces apoptosis sensitization. Structurally SARPs possess a cystein-rich domain (CRD), homologous to CRD of frizzled proteins, but lack the transmembranal domain.

Upregulation of **cytochrom oxidase subunit 1** may be a consequence of mechanical stress or oxidative stress/apoptosis possibly mediated in the system by, for example, increased NO levels.

**DEST (ACC#AA177798),** after the contige construction turned out to belong to a cDNA coding for glutamyl-cystein synthetase - a rate limiting enzyme in glutathione (GSH) synthesis. Its upregulation may be related to the stressed conditions like in the previous case. On the other hand there is one clinical work that correlates GSH reduction (low activity of antioxidant systems) in patients with hypomineralized state of bones.

TGF-beta l is known as a principal inducer of **connective tissue growth factor** (CTFG, cef10, fisp12, cyr61, betaIG-M1, beta IG-M2, nov-protoncogene) expression. The latter contains four distinct structural modules, each of them being homologous to distinct domains in other extracellular proteins such as Von Willebrand factor, slit, **trombospondins**, fibrillar collagenes, IGF-binding proteins and mucins. CTGF expression is induced not only by TGF-betal, but also by BMP2 (bone morphogenic factor 2), and during wound repair. In embryogenesis, its expression is found in developing cartilaginous elements, including limbs, ribs, prevertebrae, chondrocranium and craniofascial elements (Meckel's cartilage). Thus, CTGF transcription correlates with differentiation of chondrocytes of both mesodermal and ectodermal origin. In culture, CTGF is expressed in chondrocytes but not in osteoblasts. Possible role in endochondral ossification is suspected because of responsiveness to BMP2. In fibroblasts, CTGF expression causes upregulation of alpha-1-**collagen,** alpha-5-integrin and **fibronectin.**

Several enzymes known to participate in steroid synthesis were found transcriptionally elevated in the present system in response to mechanical stress. They include **3-hydroxy-3-methylglutaryl coenzyme A reductase** (the first rate limiting enzyme in the chain of cholesterol synthesis from 3 acethyl-CoA molecules), **yeast ERG3 homolog -** sterol-C5-desaturase and **yeast ERG25 homolog -** methyl-sterol-oxidase (both may play a role in formation of cholesterol from lanosterol). It is worth noting that cholecterol is the basis for estrogen and vitamin D3 synthesis. One additional enzyme beloning to fatty acid metabolic pathways that was found upregulated is **stearoyl-CoA desaturase,** that converts the saturated substrate into the D9-deasaturated oleoyl-CoA. Both compounds particpate in the synthesis of phospholipids building the cell membrane. Interestingly, estrogens and androsterons are known enhancers of the desaturation reaction.

**Alpha-2u microglobulin-related protein** (neutrophil gelatinase-associated lipocalin precursor - NGAL) belongs to a lipocalin superfamily embracing small extracellular proteins that can bind small hydrofobic molecules (i.e. retinols) and serve ligands to specific extracellular receptors. Many of them were implicated in regulation of cell homeostasis. NGAL was identified as a protein secreted from specific neutrophils' granules upon cell activation and it is identical to a 24p3 protein upregulated in SV-40 induced mitotic reaction. Interestingly, NGAL expression is increased in neu- but not in ras-induced expreimental mammary tumors. NGAL can be upregulated by dexamethasone through a responsive promoter element in vitro. In vivo, induction of NGAL in epithelial cells was observed in inflammatory and neoplastic colorectal deseases, but not in normal colon. Among other lipocalins, NGAL is mostly similar to lipocalin-type prostoglandin-D-synthase, responsible for synthesis of prostaglandin D2 from prostaglandin H2. However, NGAL is not supposed to have the enzymatic activity because of the absence of a specific Cys residue (position 65) which is crucial for prostaglandin-D-synthase function. Structural similarity between two proteins most probably stems from clustered localization of both genes at the same chromosomal locus. Nothing is known about NGAL function in bones.

In addition, two known (RB 13-6 antigen and highly charged amino acid sequence ACC# X59131) but without any attributed function and three novel proteins designated 274, 405 and 608 were found to be upregulated in the present model.

**RB13-6** is a cell surface 130 kD glycoprotein selectively recognized by monoclonal antibody with the same name. RB 13-6 is as a surface antigen of a subset of glial cells highly susceptible to malignant convertion by treatment with a certain carcinogen. This protein is related to the human and murine plasma cell membrane protein PC-1, a nucleotide pyrophosphatase / alkaline phosphodieterase, and possesses a 5'-nucleotidase activity. However, unlike PC-1, RB13-6 contains an RGD-sequence. The latter is a signature of integrin-interacting proteins.

So called **highly charged amino acid sequence (ACC#X59131)** is a putative protein encoded by anonymous open reading frame of 315 amino acids. It has no significant homology to any protein in the database. Charged amino acids are found in clusters with either Ser and Thr or Ser and Pro residues. Two prominent alpha-helices - one basic and one acidic - are positioned near the C-terminus.

**274 (novel gene):** In rat calvaria primary cell cultures, expression of this gene was found upregulated approximately 3-fold by mechanical strain. This was detected both by microarray analysis and by Northern hybridization. In rat calvaria this gene is expressed as a single RNA species of approximately 9 Kb. However, expansion of Northern analysis to RNA samples from other rat tissue sources we have found that 274 may probably be alternatively spliced in a tissue specific manner. Alternatively, there is a family of genes closely related to 274 genes that are differently expressed in different tissues. Transcripts of varying length (in general, 9 Kb or a slightly shorter) were found in rat small intestine, skeletal muscle, lung, kidney, eye, brain, colon and testis. The highest expression levels were found in testis, eye and kidney. Complex expression pattern was discovered in bone: two strong transcripts of more than 9 Kb and 4 Kb and three faint transcripts of 1.8 Kb, 0.5 Kb and 0.3 Kb. Interestingly, when human derived lymphoid cell line NB4 was hybridized to the same probe, these three faint transcripts appeared strong, while bone-specific two strong transcripts were not evident. This, probably, indicates that the origin of the three short transcripts is in some lymphoid precursors present in small amount in the bone marrow.

cDNA library was prepared from RNA extracted from calvaria cells after mechanical stimulation. 5291 bp 274-specific RACE product was synthesized and sequenced (see Figs 2A-14). Comparison to public databases revealed that 274 is a rat homologue (98% identity on the level of amino acids) of anonymous human cDNA KIAA0462. This sequence is 7150 bp long and contains open reading frame of 6900 bp capable of coding for 2300 amino acid protein. The frame is still open from the 5', indicating the lack of the N-terminal sequences. The open reading frame extends to the 5' direction for additional 900 bp compared to the human KIAA0462 sequence, but still does not reach the beginning of the protein. On the basis of human sequence information the inventors were able to synthesize a 6.8 Kb long human specific RACE contige. The putative KIAA0462 protein has no direct analogs in the database. It distantly (26%) resembles the *C.elegans* hypothetical protein (AF003140) that was, in turn, defined as having weak similarity to the drosophila hyperplastic disc protein. No known protein functional domains were identified either. A stretch of 24 hydrophobic amino acids between positions 165 and 188 of the KIAA0462 putative protein hints on its potential transmembranal location.

In situ hybridization analysis (discussed in more detail in further Examples, *infra*): in normal rat bones and bones obtained from ovaryectomized (osteoporotic) rats gave preliminary results indicating that gene 274 is expressed in long bones of normal rats in lining cells covering the inner surface of compact bone and in bone marrow. In normal trabecular bone specific signal was detected in bone marrow. Osteoporotic bones were 274 - negative or displayed extremely reduced signal.

CMF274 appears to encodes a huge protein. This gene seems to be bone specific. In the bone expression is in many compartments. Expressed in osteoblasts but not in lining cells. This gene seems different from the other two in that it is expressed in more mature cells.

With respect to CMF 274, mention is made of Xu et al, "Retinal Targets for Calmodulin Include Proteins Implicated in Synaptic Transmission," J Biol Chem 273(47):31297-307 (1998). Xu relates to what may be a homolog for CMF274 in drosophila, named "calossin". Xu et al. may provide homology to mouse ESTs and that the protein is highly conserved during evolution. The mouse ESTs may be from the mouse homolog of CMF274. However, in Xu et al., the mouse gene was not characterized, and Xu et al. do not provide any relation to bones.

CMF274 contains some interesting domains including calmodulin binding domain and two zinc finger domains. The first implies capability to bind the important Calcium sensor calmodulin, and the second implies DNA binding capabilities. This combination support the proposition that CMF274 is involved in a central aspect of bone biology: sensing amount of calcium and translating it into nuclear signals that change the expression of downstream genes. It is important to note that one of the zinc-finger binding domains (CRD1) "is most similar to the zinc-finger family defined by Requiem, a protein required for apoptosis" (Xu et al).

**405 (novel gene):** In rat calvaria primary cell cultures, expression of this gene was found to be downregulated in response to mechanical stimulation as detected both by microarray analysis and by Northern blots. A single 9 Kb transcript was detected in this tissue. However, being hybridized to rat tissue blot, the same probe had detected a major 5 Kb transcript ubiquitously expressed in bone, brain, colon, small intestine, testis, ovary, uterus, heart, kidney, liver, stomach, thymus, spleen, bladder, adipose tissue and mammary gland.

Partial 405 rat cDNA clone was isolated from rat calvaria cDNA library by RACE technique and sequenced. It contains 3684 bp, 3000 of them constituting an open reading frame closed from the 3' end (see Figs 2A-14). Comparison to public databases revealed that gene 405 is a rat homologue of human anonymous cDNA sequences KIAA0183 and AF055017. Interestingly, the latter cDNA lacks a 294 bp (98 amino acids) fragment corresponding to positions 2384 - 2483 of KIAA0183 putative amino acid sequence. Rat 405 homologue contains this region.

Thus, there is an indication that gene 405 is subjected to alternative splicing. KIAA0183 cDNA clone was obtained from Genbank and used as a probe for hybridization with human tissue RNA blot. As in the case of rat tissues, a 5 Kb transcript was detected. However, in humans its expression was not as uniformly distributed among different tissues as in rats. The highest levels were detected in early embryo, and in testis, placenta, ovary, tongue, intestine in adults. The only RNA of human origin where a faint 9 Kb transcript (together with the major 5 Kb one) could be seen was RNA from K562 early myeloid precursor cell line.

KIAA0183 cDNA clone represents a 1062 amino acid open reading frame, lacking the N-terminus. The available sequence has no transmembrane domain. On the other hand, four structural subdomains can be easily identified: N-terminal, highly charged alpha-helical region, Ser-Pro rich spacer domain, C-terminal highly charged alpha-helical region, and a tail region, rich in Ser, Pro, Gly, and Arg - amino acids, known to be clustered in this composition in the RNA-binding protein regions. A middle spacer region contains an RGD motif known to serve as a receptor to integrins. No significant homology to any known protein was found. Among those ones that displayed distant fragment homology, are FUS/TLS RNA binding protein (nuclear export) and various types of collagens.

Thus, as CMF405 the full sequence of the human cDNA is also presented. The pattern of expression in bone suggests involvement in osteoblast and chondrocyte differentiation. The presence of an RGD motif in this protein suggests involvement in response to integrins. Expression in few other tissues may suggest a broader function.

**608 (novel gene):** In rat calvaria primary cell cultures, expression of this gene was found upregulated approximately 3-fold by mechanical strain. This was detected both by microarray analysis and by Northern hybridization. In rat calvaria this gene is expressed as a single RNA species of approximately 9 Kb. Hiybridization signal was not detected in any other rat RNA from different tissue sources, including testis, colon, intestine, kidney, stomach, thymus, lung, uterus, heart, brain, liver, eye, and lymphnode.

Partial 608 rat cDNA clone was isolated from rat calvaria cDNA library by RACE technique and sequenced. RACE contige is 4007 bp long and contains a 3356 bp open reading frame closed from the 3'. Comparison to public databases revealed no sequence homologues. There are several human EST clones, similar 608 cDNA. The primary structure of the putative protein enables to attribute it to Ig superfamily.

By *in situ* hybridization (discussed in more details in Examples infta), expression of gene 608 was found in bone marrow from normal trabecular bones, but not of osteoporotic ones.

As to CMF608, the inventors found that it encodes a big protein that is most probably a part of the extra-cellular matrix. The gene may be actively involved in supporting osteoblast differentiation. Another option is that it marks regions were remodeling takes place. Such an hypothesis is also compatible with a role in directing osteoclast action and thus it may be a target for inhibition by small molecules.

In normal bone formation, activation of osteoblasts leads to secretion of various factors that attract octeoclast precursors or mature osteoclasts to the sites of bone formation to initiate the process of bone resoption. In normal bone formation both functions are balanced. Imbalance to any side causes either osteopetrosis (osteoblast function overwhelms) or osteoporosis (osteoclast function overwhelms).

Among known osteoblast activators - mechanical force stimulation - is actually applied in the present model. As proof of principle, increased expression of several genes known to respond to mechanical stress by transcriptional upregulation were found. They include tenascin, endothelin and possibly trombospondin. Upregulation of water channel encoding message is likely related to this mechanism too.

Among genes whose expression is found upregulated are those known to be expressed by activated osteoblasts (i.e. complement C3) or those whose upregulation may be logically connected:
1) Mechanical stress activates constitutive NO-synthase (NO plays an important role in bine building). In muscle, this enzyme is found in complex with syntrophin 1. The latter is found upregulated in our screen. Therefore, it can participate in NO-synthase activation in bone, too.
2) Some proteins secreted by osteoblasts participate osteoclast attraction through the RGD-mediated binding to integrin receptor highly expressed by osteoclasts, e.g., osteopontin. While this specific protein was not identified in the present screen, this is of no moment as other RGD-containing proteins were identified in the present screen, including the DEST405 and RB 13-6.
3) Additional protein complex that attracts osteoclasts is the complex of C3 complement. Such complexes are known to be stabilized in plasma by a thrombospondin motifs containing glycoprotein - properdin. Another trombospondin motifs-containing protein without any known function - ADAMTS-1 is found upregulated. It can participate in stabilization of C3 complexes in bone.

Several lines of evidence indicate that mechanical stress causes generation of both apoptotic (NO, cytochrom oxidase subunit 1) and antiapoptotic signalling (SARP1, glutamyl-cystein synthetase). That may be important for keeping the balance between osteoblast and osteoclast proliferation, differentiation and death.

Interestingly, the inventors found that in response to mechanical force, several enzymes that regulate the chain of chemical reactions potentially leading steroid synthesis are upregulated. In bone, these steroids may have the estrogen-like function that is reflected by at least two observations: first - upregulation of stearoyl-CoA-desaturase (elevation of this enzyme activity is known as estrogen-dependent); and, second - upregulation of connective tissue growth factor that is mainly induced in cells by TGF-beta -1, that, in turn, is known to be induced in osteoblasts by estrogen. Such a link may explain a common anti-osteoporotic action of estrogens and mechanical forse. It is worth noting, that estrogen induces osteoclasts apoptosis both directly (applied to cultured osteoclasts) and in TGF-beta-1-dependent manner.

Genes that are implicated in osteoporosis, osteoporosis prevention, treatment, or control, or in study or investigation to advance knowledge of osteoporosis, its prevention, treatment or control and/or in bone growth/formation study or investigation and/or for addressing maladies, conditions, symptoms, and the like, associated with bone growth/formation, include three novel genes DEST's 274, 405, 608, as well as RG13-6 (as RGD-containing protein), metalloproteinase ADAMTS-1, and proteins of SARP family (secreted apoptosis related proteins) including as potential modifiers of programmed cell death in bone formation.

Furthermore, as mentioned herein, this Example can also be performed without imparting mechanical stress to the cells; for instance, in reduced or zero gravity conditions, to develop a model with the lack of mechanical stress; and, the invention comprehends such a model and genes thereby identified.

### Example/Result 3: CMF608 GENE EXPRESSION BY IN SITU HYBRIDIZATION

Pattern of expression of CMF608 gene was studied by in situ hybridization on sections of bones from ovariectomized and sham-operated rats. Female Wistar rats weighting 300-350 g were subjected to ovariectomy under general anesthesia. Control rats were operated in the same way but ovaries were not excised - sham operation.

Three weeks after operation rats were sacrificed and tibia were excised together with the knee joint. Bones were fixed for three days in 4% paraformaldehyde and then decalcified for four days in solution containing 5% formic acid and 10% formalin. Decalcified bones were postfixed in 10% formalin for three days and embedded into paraffin.

To study the pattern of expression of CMF608 gene in developing bone the model of ectopic bone formation was employed. Rat bone marrow cells were seeded into cylinders of demineralized bone matrix prepared from rat tibiae. Cylinders were implanted subcutaneously into adult rats. After three weeks rats were sacrificed and implants were decalcified and embedded into paraffin as described above for tibial bones.

The 6 *u*m sections were prepared and subjected to *in situ* hybridization procedure. After hybridization sections were dipped into nuclear track emulsion and exposed for three weeks at 4°C. Autoradiographs were developed, stained with hematoxylin-eosin and studied under microscope using brightfield and darkfield illumination.

For the further assessment of cell and tissue specificity of CMF608 gene expression in situ hybridization study was performed on sections of multitissue block containing multiple samples of adult rat tissues. Developmental pattern of CMF608 expression was studied on sagittal sections of mouse embryos of 12.5, 14.5 and 16.5 days postconception (dpc) stages.

Microscopic study of hybridized sections of long bones revealed a peculiar pattern of CMF608 probe hybridization. The hybridization signal can be seen mainly in fibroblast-like cells found in several locations throughout the sections. Prominent accumulations of these cells can be seen in the area of periosteal modeling in metaphysis, and also in regions of active remodelling of compact bone in diaphysis: at the boundary between bone marrow and endosteal osteoblasts and in periosteum, also in close contact with osteoblasts. Perivascular connective tissue filling Volkmann's canals in compact bone in diaphysis and epiphysis also contains expressing cells. No hybridization is found within cancellous bone and in bone marrow. This pattern of hybridization suggests that cells showing expression of CMF608 are associated with areas of remodelling of preexisting bone and are not involved in primary endochondral ossification.

At the level of growth plate expressing cells can be seen in perichondral fibrous ring of LaCroix. Some investigators regard this fibrous tissue as the aggregation of residual mesenchymal cells able to differentiate into both osteoblasts and chondrocytes. In this respect it is noteworthy that single cells expressing CMF608 can be seen in epiphyseal cartilage. These expressing cells are rounded cells within the lateral segment of epiphysis (sometimes in close vicinity to the ring of LaCroix) and flattened cells covering the articulate surface. Most of cells in articulate cartilage and all chondrocytes of growth plate do not show expression of CMF608. Ovariectomy did not result in change of the intensity and pattern of CMF608 expression in bone tissue.

In sections of ectopic bones hybridization signal for CMF608 can be seen in some fibroblast-like cell either scattered within unmineralized connective tissue matrix or concentrated at the boundary between this tissue and osteoblasts of immature bone.

Pattern of expression of CMF608 gene revealed by in situ hybridization in bone and cartilage allows to speculate that its expression marks some skeletal tissue elements able to differentiate into two skeletal cell types - osteoblasts and chondrocytes. The terminal differentiation of these cells appears to be accompanied by down-regulation of CMF608 expression. The latter suggestion is supported by peculiar temporal pattern of CMF608 expression in primary cultures of osteogenic cells isolated from calvaria bones of rat fetuses. In these cultures expression was revealed by in situ hybridization in vast majority of cells after one and two weeks of incubation in vitro. Three and four weeks old cultures showing signs of ossification contain no expressing cells. Significantly, no hybridization signal was found on sections of multitissue block hybridized to CMF608 probe suggesting high specificity of this gene expression for the skeletal tissue in adult organism.

In situ hybridization study of embryonic sections demonstrated that at 12.5 dpc weak hybridization signal can be discerned in some mesenchymal cells in several locations throughout the embryonic body. The most prominent signal is found in the head: in loose mesenchymal tissue surrounding the olfactory epithelium and underlying the surface epithelium of nose tip. Other mesenchymal cells in the head also show hybridization signal: in non-cartilagenous part of basisphenoid bone primordium and in mesenchyme surrounding the dental laminae (tooth primordia) in the mandible.

In the trunk expression can be detected in less developed vertebrae primordia in thoraco-lumbar region. Hybridization signal here marks the condensed portion of sclerotomes.Another area showing hybridization signal in the trunk is comprised by thin layer of mesenshynal cells in the anterior part of thoracic body wall.

At later stages of development -14.5 and 16.5 dpc probe CMF608 gave no hybridization signal. Thus, it appears that during embryonic development CMF608 gene is transiently expressed by at least some mesenchymal and skeleton-forming cells cells. This expressio is down-regulated at later stages of development. More detailed study of late embryonic and postnatal stages of development will reveal the timing of appearance of CMF608 expressing cells in bone tissue.

### Example/Result 4: CMF405 GENE EXPRESSION BY IN SITU HYBRIDIZATION

Pattern of expression of CMF405 gene was studied by in situ hybridization on sections of bones from ovariectomized and sham-operated rats. Female Wistar rats weighting 300-350 g were subjected to ovariectomy under general anesthesia. Control rats were operated in the same way but ovaries were not excised - sham operation.

Three weeks after operation rats were sacrificed and tibia were excised together with the knee joint. Bones were fixed for three days in 4% paraformaldehyde and then decalcified for four days in solution containing 5% formic acid and 10% formalin. Decalcified bones were postfixed in 10% formalin for three days and embedded into paraffin.

To study the pattern of expression of CMF405 gene in developing bone the model of ectopic bone formation was employed. Rat bone marrow cells were seeded into cylinders of demineralized bone matrix prepared from rat tibiae. Cylinders were implanted subcutaneously into adult rats. After three weeks rats were sacrificed and implants were decalcified and embedded into paraffin as described above for tibial bones.

The 6 *u*m sections were prepared and subjected to in situ hybridization procedure. After hybridization sections were dipped into nuclear track emulsion and exposed for three weeks at 4°C. Autoradiographs were developed, stained with hematoxylin-eosin and studied under microscope using brightfield and darkfield illumination.

For the further assessment of cell and tissue specificity of CMF405 gene expression in situ hybridization study was performed on sections of multitissue block containing multiple samples of adult rat tissues. Developmental pattern of CMF405 expression was studied on sagittal sections of mouse embryos of 12.5, 14.5 and 16.5 days postconception (dpc) stages.

Bones: Hybridization signal for CMF405 gene is widely spread throughout different cell types on sections of long bones from sham-operated animals: cartilage, bone marrow and bone.

In the growth plate hybridization signal is concentrated in the transition zone from proliferating to hypertrophic cartilage so that most advanced proliferating chondrocytes and youngest hypertrophic chondrocytes display expression. Both young proliferating chondrocytes and most of mature hypertrophic chondrocytes do not show hybridization signal. Chondrocytes of articulate cartilage show no hybridization signal.

Some (but not all) hematopoietic cells within the bone marrow show clear hybridization signal. Poor morphology of decalcified section stained with hematoxylin-eosin does not allow identification of expressing cell types.

Within the bone tissue hybridization signal can be seen in osteoblasts localized in primary spongiosa and secondary spongiosa in methaphysis (cancellous bone). Osteoblasts covering the surface of marrow cavity and Volkmann's canals in diaphyseal (compact) bone also display hybridization signal. Flat bone lining cells and osteocytes are not expressed in any part of the bone.

Ovariectomy did not result in change of the intensity and pattern of CMF405 expression. In ectopic bone hybridization signal concentrates mainly in osteoblasts of immature bone. This signal is weak or absent from osteoblasts embedded into bone matrix.

The pattern of hybridization of CMF405 gene in adult skeletal tissues suggests that its expression is characteristic for osteogenic and chondrogenic cells at intermediate stage of their differentiation preceding intensive matrix calcification.

Tissue expression: The CMF405 probe was hybridized to multitissue block sections. The hybridization signal can be seen mainly in epithelial cells in many organs and tissues suggesting the wide expression of this gene in adult tissues.

The hybridization signal of varying intensity can be seen in epithelial lining of the digestive system. Weak hybridization signal can be seen in basal cells of stratified squamous epithelium of esophagus. Weak signal is displayed by surface epithelium of fundic stomach. In pyloric stomach strong hybridization signal is displayed by cells lining mucosal pits and weaker signal - by surface epithelium. In thin intestine expressing cells are localized in crypts and glands while villous epithelium shows no hybridization signal. Similar pattern is observed in colonic epithelium: weak hybridization signal can be seen only in crypts and not in villi. This pattern of hybridization throughout different parts of the alimentary canal allows to suggest that expression of CMF405 in digestive system appears to be confined mainly to actively proliferating epithelial cells and transition of epithelial cells into non-proliferative compartment (like suprabasal layers of esophagus or villous epithelium of intestine) is accompanied by down-regulation of CMF405 expression.

In urogenital system weak and diffuse signal can be seen in medullar part of kidney. The weak hybridization signal is displayed by transitional epithelium of kidney calyx, ureter and bladder. Hybridization signal is seen also in basal cells within seminiferous tubules. Low resolution of microautoradiographs does not allow unequivocal identification of expressing cells as spermatogonia or Sertoli cells.

All epidermal layers of skin show hybridization signal. Strongly expressing cells are localized also in all layers of the hair follicle.

Strong and uniform hybridization signal can be seen on sections of lymphoid organs: thymus, spleen and lymph nodes.

Positive hybridization was obtained also on eye sections: strong signal is displayed by corneal epithelium. Retina shows weaker hybridization signal throughout all layers excluding ganglion cell layer.

No hybridization signal can be seen in brain.

*In situ* hybridization study of sections of 12.5, 14.5 and 16.5 dpc embryos revealed strong and practically uniform hybridization signal throughout bodies of 12.5 and 14.5 dpc embryos. This suggests that all cell types at these stages of development express CMF405 gene. By 16.5 dpc stage expression appears to decline in some cells so that pattern of expression approaches to that in adult tissue although some structures showing no expression in adults display hybridization signal.

### Example/Result 5: EXPRESSION OF CMF274 GENE BY IN SITU HYBRIDIZATION

Hybridization of CMF274 probe to the sections of knee joint demonstrated wide expression throughout the bone, cartilage and bone marrow tissues. Hybridization signal can be seen in hematopoietic bone marrow cells. Accumulation of signal in cells of eosinophilic lineage is clear. Poor morphology of decalcified sections does not allow identification of other expressing cell types although it is apparent that not all the myeloid elements are expressing.

Chondrocytes display weak hybridization signal throughout all zones of growth plate. No expression is detected in epiphyseal articulate cartilage.

Within the bone tissue hybridization signal can be seen in osteoblasts localized in primary spongiosa and secondary spongiosa in methaphysis (cancellous bone). Periosteal and endosteal osteoblasts and osteoblasts of Volkmann's canals in diaphyseal (compact) bone also display hybridization signal. Flat lining cells and osteocytes are not expressing in any part of the bone. Ovariectomy did not result in change of the intensity and pattern of CMF274 expression in bone tissue.

The CMF274 probe was hybridized to multitissue block sections and to sections of 12.5, 14.5 and 16.5 dpc mouse embryos. No hybridization signal was found on these sections.

### Example/Result 6: CMF2-45 (SARP) GENE EXPRESSION BY IN SITU HYBRIDIZATION

Hybridization signal for CMF2-45 (SARP) gene is found in different cell types on sections of long bones from sham-operated animals: cartilage, bone marrow and bone. In all expressing cell types the level of hybridization signal is rather low.

In growth plate hybridization signal marks proliferating chondrocytes while hypertrophic chondrocytes show little or no signal. Chondrocytes of articulate cartilage in epiphysis do not show hybridization signal.

Hybridization signal in bone tissue proper marks osteoblasts located in all compartments of cancellous and compact bone: primary and secondary spongiosa, periosteum, endosteum and Volmann's canals. Bone lining cells and osteocytes show no hybridization signal.

Some (but not all) hematopoietic cells within the bone marrow show clear hybridization signal. Poor morphology of decalcified sections stained with hematoxylin-eosin does not allow identification of expressing cell types.

In ectopic bone hybridization signal can be seen in osteoblasts of immature bone. Beside of osteoblasts single fibrobast-like cells scattered throughout the connective tissue also show hybridization signal. Some of these expressing fibroblast-like cells can be seen in close contact with osteoblasts.

### Example/Result 7: CMF2-224 (Rb13-6) EXPRESSION BY IN SITU HYBRIDIZATION

*In situ* hybridization study of RB13-6 gene (GenBank Accession No.: Z47987) revealed expression of this gene in osteoblasts located in different compartments of long bone: in primary and secondary spongiosa of cancellous bone in metaphysis and within endosteum and Volkmann's canals of compact bone in diaphysis. Weak hybridization signal can be seen also in myeloid cells of bone marrow. Osteoblasts of immature bone developing within ectopic bone implants aslo display hybridization signal. Significantly, no hybridization signal was revealed on sections of long bones of ovariectomised rats and also on sections of ectopic bones implanted into ovariectomised rats. This result suggests that expression of RB 13-6 in osteoblasts and bone marrow cells is estrogen-dependent. Further study will be needed to clarify the involvement of RB 13-6 gene product into regulation of osteoblast function and development of osteoporosis.

Additional *in situ* hybridization study on sections of multitissue block demonstrated expression of RB 13-6 gene in distinct epithelial cell types and in lymphoid tissue. Expressing cells can be seen in epithelial lining of bronchi, villous (i.e. mature and non-proliferating) epithelium of thin intestine, in luminal and glandular epithelia of uterus, acinar and ductal epithelia of salivary glands. Very weak hybridization signal suggesting low level of expression was found in liver and in kidney. Liver expression appears to be uniform throughout hepatocytes. In kidney single expressing cells can be seen in thick ascending part of Henle's loop.

Within lymphoid tissue strongly expressing lymphocytes are concentrated in medullar zone of lymph nodes. Few expressing cells can be seen in spleen: in the perifollicular zone (the bordering area between the red and white pulp) and in perivascular aggregations of lymphocytes. No expressing cells were found in thymus.

### References

Consensus Development Conference: Prophylaxis and Treatment of Osteoporosis: *Am J Med* 94:646-650, 1993
WHO Study Group: In *Assessment of Fracture Risk and Its Application to Screening for Postmenopausal Osteoporosis.* WHO Technical Report Series: 843, 1994
Department of Health *Health and Personal Social Services Statistics for England, 1991 Edition.* HMSO, London, 1991, pp 10-11
Welsh Office *Health and Personal Social Services for Wales* HMSO. London, 1991, pp 2-3
Kanis JA, McCloskey EV: Epidemiology of vertebral osteoporosis. *Bone* 13:S1-S10, 1992
Kanis JA, Pitt FA: Epidemiology of osteoporosis. *Bone* 13:S7-S15, 1992
Cummings SR et al: Epidemiology of osteoporosis and osteoporotic fractures. *Epidemiol Rev* 7:178-207, 1985
Department of Health and Social Security Office of Population Censuses and Surveys *CancerStatistics: Registrations.* Series MBT, 20, HMSO, London, 1987
Riggs BL, Melton LJ: Involutional osteoporosis. *N Engl J Med* 314:1676-1686, 1986
Melton LJ, Eddy DM, Johnston CC: Screening for osteoporosis. *Ann Intern Med* 112:516-528, 1990
Department of Health and Social Security Office of Population Censuses and Surveys *Hospital In-Patient Enquiry Summary Tables.* Series MB4, 26, HMSO, London, 1985
Nordin BEC, Need AG: How can we prevent osteoporosis? In *Osteoporosis 1987* (Christiansen C, Riis BJ, eds.). Osteopress ApS, Copenhagen, 1987, pp 1204-1210
United Nations *Interpolated National Populations by Age and Sex: 1950-2025 (1992 Revision).* United Nations Population Division, United Nations, New York, 1992
Cooper C, Campion G, Melton LJ: Hip fractures in the elderly: A world-wide projection. *Osteoporosis Int* 2:285-289, 1992
Parfitt AM: Quantum concept of bone remodeling and turnover: Implications for the pathogenesis of osteoporosis. *Calcif Tissue Int 28: 1-5, 1979*
Parfitt AM: Bone remodeling in the pathogenesis of osteoporosis. *Res Staff Physician, December:60-72,* 1981
Parfitt AM: Bone remodeling: Relationship to the amount and structure of bone, and the pathogenesis and prevention of fractures. In *Osteoporosis: Etiology, Diagnosis, and Management* (Riggs BL, Melton LJ, eds.). Raven Press, New York, 1988, pp 74-93
Kanis JA *Pathophysiology and Treatment of Paget's Disease of Bone.* Martin Dunitz, London, 1991, pp 12-40
Smith DM et al: Genetic factors in determining bone mass. *J Clin Invest* 52:2800-2808, 1973
Pocock NA et al: Genetic determinants of bone mass in adults: A twin study. *J Clin Invest* 80:706-710, 1987
Matkovic V et al: Factors that influence peak bone mass formation: A study of calcium balance and the inheritance of bone mass in adolescent females. *Am J Clin Nutr* 52:878-888. 1990
Cohn SH et al: Comparative skeletal mass and radial bone mineral content in black and white women. *Metabolism* 26:171-178, 1977
Selby PL: Endocrinology and osteoporosis. In *HRT and Osteoporosis* (Drife JO, Studd JWW, eds.). Springer Verlag, London, 1990, pp 105-114
Stevenson JC et al: Exercise and the skeleton. In *Osteoporosis 1990* (Smith R, ed.). Royal College of Physicians of London, London, 1990, pp 119-124
Griffin J: *Osteoporosis and the Risk of Fracture.* Office of Health Economics, London, 1990, p 48
Mundy GR *Calcium Homeostasis: Hypercalcemia and Hypocalcemia,* 2nd ed. Martin Dunitz, London, 1990, pp 1, 32, 51-52, 179, 200
Christiansen C, Riis B *The Silent Epidemic: Postmenopausal Osteoporosis.* Christiansen and Riis, Denmark, 1990, pp 23, 66-67, 70-73
Christiansen C, Rodbro P, Tjellesen L: Serum alkaline phosphatase during hormone treatment in early postmenopausal women: A model for establishing optimal prophylaxis and treatment in postmenopausal osteoporosis. *Acta Med Scand* 216:11-17,1984
Gallagher JC et al: Intestinal calcium absorption and serum vitamin D metabolites in normal subjects and osteoporotic patients: Effect of age and dietary calcium. *J Clin Invest* 64:729-736, 1979
Heaney RP: Nutritional factors in bone health. In *Osteoporosis: Etiology, Diagnosis, and Management* (Riggs BL, Melton LJ, eds.). Raven Press, New York, 1988, pp 359-372
Heaney RP: Effect of calcium on skeletal development, bone loss, and risk of fractures. *Am J Med 91 (suppl* 5B):5B-23S-5B-29S, 1991
Delmas PD et al: Increase in serum bone gamma-carboxyglutamic acid protein with aging in women: Implications for the mechanism of age-related bone loss. *J Clin Invest* 71:1316-1321, 1983
Drinkwater BL et al: Bone mineral content of amenorrheic and eumenorrheic athletes. *N Engl J Med 311:277-281,* 1984
Silverman SL: The clinical consequences of vertebral compression fracture. *Bone* 13:S27-S31, 1992
Adami S et al: The radiological assessment of vertebral osteoporosis. *Bone* 13:S33-S36, 1992
Riggs BL: Practical management of the patient with osteoporosis. In *Osteoporosis: Etiology, Diagnosis, and Management* (Riggs BL, Melton LJ, eds.). Raven Press, New York, 1988, pp 481-490
Kanis JA et al: Screening techniques in the evaluation of osteoporosis. In *HRT and Osteoporosis* (Drife JO, Studd JWW, eds.). Springer Verlag, London, 1990, pp 135-147
Genant HK, Faulkner KG, Gluer C-C: Measurement of bone mineral density: Current status. *Am J Med (suppl 5B)* 91:5B-49S-5B-53S, 1991
Sartoris DJ, Resnick D: Digital radiography may spark renewal of bone densitometry. *Diagnostic Imaging,* January:145-150, 1988
Fogelman I, Rodin A: The measurement of bone density. In *HRT and Osteoporosis (Drife* JO, Studd JWW, eds.). Springer Verlag, London, 1990, pp 119-133
Mazess RB et al: Dual-energy X-ray absorptiometry for total-body and regional bone-mineral and soft-tissue composition. *Am J Clin Nutr 51:1106*-1112, 1990
Johnston CC et al: Clinical indications for bone mass measurements. *J Bone Min Res 4 (suppl 2): 1-29,* 1989
Delmas PD: Clinical use of biochemical markers of bone remodeling in osteoporosis. In *Osteoporosis 1990* (Christiansen C, Overgaard K, eds.). Osteopress ApS, Copenhagen, 1990, pp 450-458
Uebelhart D et al: Urinary excretion ofpyridinium crosslinks: A new marker of bone resorption in metabolic bone disease. *Bone Mineral* 8:87-96, 1990
Consensus Development Conference: Prophylaxis and Treatment of Osteoporosis: *Am J Med* 90:107-110, 1991
Delmas PD: Biochemical markers for the assessment of bone turnover. In *Osteoporosis: Etiology, Diagnosis, and Management.* Rev. ed. (Riggs BL, Melton LJ, eds.). Raven Press, New York, 1995, pp 319-333
Uebelhart D et al: Effect of menopause and hormone replacement therapy on the urinary excretion of pyridinium cross-links. J *Clin Endocrinol Metab* 72:367-373, 1991
Christiansen C, Riis BJ, Rodbro P: Screening procedure for women at risk of developing postmenopausal osteoporosis. *Osteoporosis Int* 1:35-40, 1990
Sinaki M: Exercise and physical therapy. In *Osteoporosis: Etiology, Diagnosis, and Management* (Riggs BL, Melton LJ, eds.). Raven Press, New York, 1988, pp 457-479
Davee AM et al: Exercise patterns and trabecular bone density in college women. *J Bone Min Res* 5:245-250, 1990
Gleeson PB et al: Effects of weight lifting on bone mineral density in premenopausal women. *J Bone Min Res* 5:153-158, 1990
Riggs BL, Melton LJ: The prevention and treatment of osteoporosis. *N Engl J Med* 327:620-627, 1992
Riggs BL: Treatment of osteoporosis with sodium fluoride or parathyroid hormone. *Am J Med* (suppl 5B) 91:5B-37S-5B-41S, 1991
Kanis JA et al: Osteoporosis: Causes and therapeutic implications. In *Osteoporosis 1990* (Smith R, ed.). Royal College of Physicians of London, London, 1990, pp 45-56
Rogers HJ, Spector AG, Trounce JR: *In A Textbook of Clinical Pharmacology.* Hodder and Stoughton, London, 1981, p 583
Eriksen EF et al: Evidence of estrogen receptors in normal human osteoblast-like cells. *Science* 241 :84-86, 1988
Lindsay R, Cosman F: Estradiol in prevention of osteoporosis. *In Osteoporosis 1990* (Christiansen C, Overgaard K, eds.). Osteopress ApS, Copenhagen, 1990, pp 2070-2075
Lindsay R: Estrogens, bone mass, and osteoporotic fracture. *Am J Med 91* (suppl 5B):5B10S-5B-13S, 1991
Lindsay R, Tohme JF: Estrogen treatment of patients with established postmenopausal osteoporosis. *Obstet Gynecol* 76:290-295, 1990
Christiansen C, Riis BJ: 17β-estradiol and continuous norethisterone: A unique treatment for established osteoporosis in elderly women. *J Clin Endocrinol Metab* 71:836-841, 1990
Lufkin EG et al: Treatment of postmenopausal osteoporosis with transdermal estrogen. *Ann intern Med* 117:1-9, 1992
Segre G, Bruni G, Dal Pra P: Calcitonin pharmacokinetics. In *Calcitonin* (Pecile A, ed.). Elsevier, New York, 1985, pp 99-107
Nicholson GC et al: Abundant calcitonin receptors in isolated rat osteoclasts. *J Clin Invest* 78:355-360, 1986
Baron R, Vignery A: Behaviour of osteoclasts during a rapid change in their number induced by high doses of parathyroid hormone or calcitonin in intact rats. *Metab Bone Dis Rel Res* 2:339-343, 1981
Overgaard K et at: Effect of salcatonin given intranasally on early postmenopausal bone loss. *Br Med J* 299:477-479, 1989
Rasmussen H et al: Effect of combined therapy with phosphate and calcitonin on bone volume in osteoporosis. *Metab Bone Dis Rel Res* 2:107-111, 1980
Gruber HE et al: Long-term calcitonin therapy in postmenopausal osteoporosis. *Metabolism* 33:295-303, 1984
Christiansen C et al: Intranasal calcitonin in the treatment of postmenopausal osteoporosis. In *Osteoporosis 1987* (Christiansen C, Riis BJ, eds.). Osteopress ApS, Copenhagen, 1987, pp 853-856
Overgaard K et al: Nasal calcitonin for treatment of established osteoporosis. *Clin Endocrinol* 30: 435-442, 1989
Overgaard K et al: Discontinuous calcitonin treatment of established osteoporosis: Effects of withdrawal of treatment. *Am J Med* 89:1-6, 1990
Fleisch H: Bisphosphonates: Pharmacology and use in the treatment of tumour-induced hypercalcemic and metastatic bone disease. *Drugs* 42:919-944, 1991
Watts NB et al: Intermittent cyclical etidronate treatment of postmenopausal osteoporosis. *N Engl J Med* 323:73-79, 1990
Filipponi P et al: Cyclical clodronate is effective in preventing postmenopausal bone loss: A comparative study with transcutaneous hormone replacement therapy. *J Bone Min Res* 10:697-703,1995
Chesnut CH et al: Alendronate treatment of the postmenopausal osteoporotic woman: Effect of multiple dosages on bone mass and bone remodeling. *Am J Med* 99: 144-152, 1995
Garnero P et al: Comparison of new biochemical markers of bone turnover in late postmenopausal osteoporotic women in response to alendronate treatment. *J Clin Endocrinol Metab* 79(6):1693-1700, 1994
Francis RM: The calcium controversy. In *Osteoporosis 1990* (Smith R. ed.). Royal College of Physicians of London, London, 1990, pp 125-133
Johnston CC et al: Calcium supplementation and increases in bone mineral density in children. *N Engl J Med* 327:82-87, 1992
Baran D et al: Dietary modification with dairy products for preventing vertebral bone loss in premenopausal women: A three-year prospective study. *J Clin Endocrinol Metab* 70:264-270, 1989
Dawson-Hughes B et al: A controlled trial of the effect of calcium supplementation on bone density in postmenopausal women. *N Engl J Med* 323:878-883, 1990
Chapuy MC et al: Vitamin D3 and calcium to prevent hip fractures in elderly women. *N Engl J Med* 327:1637-1642, 1992
Hassager C et al: Changes in soft tissue body composition and plasma lipid metabolism during nandrolone decanoate therapy in postmenopausal osteoporotic women. *Metabolism* 38:238-242, 1989
Johansen JS et al: Treatment of postmenopausal osteoporosis: Is the anabolic steroid nandrolone decanoate a candidate? *Bone Mineral* 6:77-86, 1989
Riggs BL: Treatment of osteoporosis with sodium fluoride: An appraisal. In *Bone and Mineral Research, Annual 2* (Peck WA, ed.). Elsevier, New York, 1983, pp 366-393
Delmas PD: Treatment of vertebral osteoporosis with disodium monfluorophosphate: Comparison with sodium fluoride. *J Bone Mineral Res* 5(suppl 1):S143-S147, 1990
Riggs BL et al: Effect of fluoride treatment on the fracture rate in postmenopausal women with osteoporosis. *N Engl J Med* 322:802-809, 1990
Mamelle N et al: Risk-benefit ratio of sodium fluoride treatment in primary vertebral osteoporosis. Lancet ii:361-365, 1988
Tietz NW, ed. *Textbook of Clinical Chemistry.* WB Saunders, Philadelphia, 1986, pp 1322-1323
Russell RGG: Bone cell biology: The role of cytokines and other mediators. In *Osteoporosis 1990* (Smith R, ed.). Royal College of Physicians of London, London, 1990, pp 9-33
Tilyard MW et al: Treatment of postmenopausal osteoporosis with calcitriol or calcium. *N Engl J Med* 326:357-362, 1992
Meunier PJ et al: Can we stop bone loss and prevent hip fractures in the elderly? *Osteoporosis Int* 4(suppl 1):S71-S76, 1994
Chapuy MC et al: Effect of calcium and cholecalciferol treatment for three years on hip fractures in elderly women. *Br Med J* 308:1081-1089, 1994
Neer R et al: Treatment of postmenopausal osteoporosis with daily parathyroid hormone plus calcitriol. In *Osteoporosis 1990* (Christiansen C, Overgaard K, eds.). Osteopress ApS, Copenhagen, 1990, pp 1314-1317
Reid DM: Corticosteroid-induced osteoporosis. In *Osteoporosis 1990* (Smith R, ed.). Royal College of Physicians of London, London, 1990, pp 99-117
Lukert BP, Raisz LG: Glucocorticoid-induced osteoporosis: Pathogenesis and management. *Ann Intern Med* 112:352-364,1990
Reid IR et al: Two-year follow-up of bisphosphonate (APD) treatment in steroid-induced osteoporosis. *Lancet* ii: 1144, 1988
Rinse JD, Welzel D: Salmon calcitonin in the therapy of corticoid-induced osteoporosis. *Eur J Clin Pharmacol* 33:35-39, 1987
Rosen, CJ: Beyond one gene-one disease: Alternative strategies for deciphering genetic determinants of osteoporosis. *Calcif Tissue Int* 60(3):225-28, 1997.
Keen et al.: Genetic factors in osteoporosis. What are the implicatins for prevention and treatment? *Drugs Aging (New Zealand)* 11 (5):333-7, 1997.
Eisman: Vitamin D receptor gene variants: Implications for therapy. *Curr Opin Genet Dev* 6(3): 361-65, 1996.
Ralston: The genetics of osteporosis. *QJM - Monthly Journal of the Association of Physicians (United Kingdom)* 90/4, pp 247-51, 1997.
Cole et al.: Osteoporosis And the Interface Between Nutrition And Genetics. INABIS '98 - 5^{th} Internet World Congress on Biomedical Sciences at McMaster University, Canada, Dec 7-16. http://www.mcmaster.ca/inabis98/atkinson/cole0195/index.html
Tokita et al.: Vitamin D receptor alleles, bone mineral density and turnover in premenopausal Japanese women. *J Bone Miner Res* 11 (7): 1003-09, 1996.
Jouanny et al.: Environmental and genetic factors affecting bone mass. Similarity of bone density among members of healthy families. *Arthritis Rheum* 38(1):61-67, 1995.
Garnero et al.: Genetic influence on bone turnover in postmenopausal twins. *J Clin Endocrinol Metab* 81(1):140-46, 1996.
Johnston et al.: Pathogenesis of osteoporosis. *Bone* 17(2 Suppl):19S-22S, 1995.
Fujita: Vitamin D in the treatment of osteoporosis revisited. *Proc Soc Exp Biol Med* 212(2):110-5, 1996.
Gong et al.: Osteoporosis-pseudoglioma syndrome, a disorder affecting skeletal strength and vision, is assigned to chromosome region 11q12-13. *Am J Hum Genet* 59(1):146-51, 1996.
Houston et al.: Vitamin D receptor polymorphism, bone mineral density, and osteoporotic vertebral fracture: studies in a UK population. *Bone* 18(3):249-52, 1996.
Cummings: Treatable and untreatable risk factors for hip fracture. *Bone* 18(3 Suppl):165S-167S,1996.
Wasnich: Vertebral fracture epidemiology. *Bone* 18(3 Suppl):179S-183S, 1996.
Riggs et al.: The contribution of vitamin D receptor gene alleles to the determination of bone mineral density in normal and osteoporotic women. *J Bone Miner Res* 10(6):991-6, 1995.
Wark: Osteoporotic fractures: background and prevention strategies. *Maturitas (Ireland)* 23(2):193-207, 1996.
Fleet et al.: The Bsml vitamin D receptor restriction fragment length polymorphism (BB) predicts low bone density in premenopausal black and white women. *J Bone Miner Res* 10(6):985-90, 1995.
Krall et al.: Vitamin D receptor alleles and rates of bone loss: influences of years since menopause and calcium intake. *J Bone Miner Res* 10(6):978-84, 1995.
Livshits et al.: Population biology of human aging: segregation analysis of bone age characteristics. *Hum biol* 68(4):540-54, 1996.
Dalgleish: The human type I collagen mutation database. *Nucleic Acids Res* 25(1):181-7, 1997 (citing http://www.le.ac.uk/depts/ge/collagen/collagen.html)
Lonzer et al.: Effects of heredity, age, weight, puberty, activity and calcium intake on bone mineral density in children. *Clin Pediatr* 35(4):185-9, 1996.
Pereira et al.: Bone fragility in transgenic mice expressing a mutated gene for type I procollagen (COL1A1) parallels the age-dependent phenotype of human osteogenesis imperfecta. *J Bone Miner Res* 10(12):1837-43, 1995.
Sano et al.: Association of estrogen receptor dinucleotide repeat polymorphism with osteporposis. *Biochem Biopphys Res Commun* 217(1):378-83, 1995.
Burke and Olson, "Preparation of Clone Libraries in Yeast Artificial -Chromosome Vectors" in Methods in Enzymology, Vol. 194, "Guide to Yeast Genetics and Molecular Biology", eds. C. Guthrie and G. Fink, Academic Press, Inc., Chap. 17, pp. 251-270(1991).
Capecchi, "Altering the genome by homologous recombination" Science 244:1288-1292 (1989).
Davies et al., "Targeted alterations in yeast artificial chromosomes for inter-species gene transfer", Nucleic Acids Research, Vol. 20, No. 11, pp. 2693-2698 (1992).
Dickinson et al., "High frequency gene targeting using insertional vectors", Human Molecular Genetics, Vol. 2, No. 8, pp. 1299-1302 (1993).
Duff and Lincoln, "Insertion of a pathogenic mutation into a yeast artificial chromosome containing the human APP gene and expression in ES cells", Research Advances in Alzheimer's Disease and Related Disorders, 1995.
Huxley et al., "The human HPRT gene on a yeast artificial chromosome is functional when transferred to mouse cells by cell fusion", Genomics, 9:742-750 (1991).
Jakobovits et al., "Germ-line transmission and expression of a human-derived yeast artificial chromosome", Nature, Vol. 362, pp. 255-261 (1993).
Lamb et al., "Introduction and expression of the 400 kilobase precursor amyloid protein gene in transgenic mice", Nature Genetics, Vol. 5, pp. 22-29 (1993).
Pearson and Choi, Expression of the human 0-amyloid precursor protein gene from a heast artificial chromosome in transgenic mice. Proc. Natl. Scad. Sci. USA, 1993. 90:1057882.
Rothstein, "Targeting, disruption, replacement, and allele re-scue: integrative DNA transformation in yeast" in Methods in Enzymology, Vol. 194, "Guide to Yeast Genetics and Molecular Biology", eds. C. Guthrie and G. Fink, Academic Press, Inc., Chap. 19, pp. 281-301 (1991).
Schedl et al., "A yeast artificialchromosome covering the tyrosinase gene confers copy number- dependent expression in transgenic mice", Nature, Vol. 362, pp. 258-261 (1993).
Strauss et al., "Germ line transmission of a yeast artificial chromosome spanning the murine al (I) collagen locus", Science, Vol. 259, pp. 1904-1907 (1993).
Gilboa, E, Eglitis, MA, Kantoff, PW, Anderson, WF: Transfer and expression of cloned genes using retroviral vectors. BioTechniques 4(6):SO4-512, 198G.
Cregg JM, Vedvick TS, Raschke WC: Recent Advances in the Expression of Foreign Genes in Pichia pastoris, Bio/Technology 11:905-910, 1993
Culver, 1998. Site-Directed recombination for repair of mutations in the human ADA gene. (Abstract) Antisense DNA & RNA based therapeutics, February, 1998, Coronado, CA.
Huston et al, 1991 "Protein engineering of single-chain Fv analogs and fusion proteins" in Methods in Enzymology (ii Langone, ed.; Academic Press, New York, NY) 203:46-88.
Johnson and Bird, 1991 "Construction of single-chain Fvb derivatives of monoclonal antibodies and their production in Escherichia coli in Methods in Enzymology (JJ Langone, ed.; Academic Press, New York, NY) 203:88-99.
Mernaugh and Mernaugh, 1995 "An overview of phage-displayed recombinant antibodies" in Molecular Methods In Plant Pathology (RP Singh and US Singh, eds.; CRC Press Inc., Boca Raton, FL) pp. 359-365.
Aiello et al., "Identification of multiple genes in bovine retinal pericytes altered by exposure to elevated levels of glucose by using mRNA differential display" Proc. Natl. Acad. Sci. USA Vol. 9 1, pp. 623 1 6235 (1994).
Bauer et al., "Identification of differentially expressed mRNA species by an improved display technique (DDRT-PCR)" Nucleic Acids Research Vol. 21, No. 18 (1993).
Bhanicha and Ven Murthy, "Characterization of Polysomes and Polysomal mRNAs by Sucrose Density Gradient Centrifugation Followed by Immobilization in Polyacrylamide Gel Matrix" Methods in Enzymology Vol. 216, pp. 168-179 (1992).
Braun et al., "Identification of Target Genes for the Ewing's Sarcoma EWS/FLI Fusion Protein by Representational Difference Analysis" Molecular and Cellular Biology Vol. 15, No. 8, pp. 4623-4630 (1995).
Cold Springs Harbor Laboratory Press, Cold Spring Harbor, New York, 1989
Davis et al., " Expression of a single transfected cDNA converts fibroblasts to myoblasts." Cell 51:9871000, 1987.
Diatchenko et al., "Suppression subtractive hybridization: A method for generating differentially regulated or tissue-specific cDNA probes and libraries" Proc. Nad Acad. Sci., Vol. 93, pp. 6025-6030 (1996).
Ehrenfeld, "Initiation of Translation by Picornavirus RNAs", Translational Control Cold Spring Harbor Laboratory Press, pp. 549-573, 1996.
Hadman et al., "Modification to the differential display technique reduce background and increase sensitivity" Analytical Biochemistry 226:383-386 (1995).
Hanauske-Abel et al., "Detection of a sub-set of polysomal mRNAs associated with modulation of hypusine formation at the GI-S boundary. Proposal of a role for
EIF-5A in onset of DNA replication." FEBS Letters 386 pp. 92-98 (1995).
Hirama et al., "Direct Purification of Polyadenylated RNAs from Isolated Polysome Fractions" Analytical Biochemistry 155, pp. 385-390 (1986).
Hubank and Schatz, "Identifying differences in mRNA expression by representational difference analysis of cDNA" Nucleic Acids Research, Vol. 22, No. 25, p. 5640-5648 (1994). Jefferies et al., "Elongation Faction-la mRNA Is Selectively Translated following Mitogenic StimulatioW' The Journal of Biological Chemistry Vol. 269, No. 6, pp. 4367-4372 (1994).
Liang and Pardee, "Differential Display of Eukaryotic Messenger RNA by Means of the Polymerase Chain Reaction" ~S~cience, Vol. 257, pp. 967-971 (1992).
Liang et al., "Distribution and cloning of Eukaryotic mRNAs by means of differential display: refinements and optimization" Nucleic Acids Research Vol. 21, No. 14, pp. 3269-3275 (1993).
Liang and Pardee, "Recent advances in differential display" Current Opinion in Immunology, 7:274-280 (1995).
Linskens et al., Cataloging altered gene expression in young and senescent cells using enhanced differential display" Nuc. Ac. Res. 23: 3244-3251 (1995).
Lisitsvn and Wigler, "Cloning the Differences Between Two Complex Genomes" Science Vol. 259, pp. 946-951 (1993).
Mach et al., "Isolation of a cDNA Clone Encoding S-Adenosylrnethionine Decarboxylase" The Annual of Biological Chemistry Vol. 261, No. 25, pp. 11697-11703 (1986).
Macejak et al., "Internal inition of translation mediated by the 5' leader of a cellular mRNA" Nature, Vol. 353, pp. 990-94 (1991).
Mechler, "Isolation of messenger RNA from Membrane-Bound Polysomes" Methods in Enzymology Vol. 152, pp. 241-253 (1987).
Menaker et al., "A Method for the Isolation of Rat Submandibular Salivary Gland Polysomes on Linear Sucrose Density Gradients" Analytical Biochemistry 57, pp. 325-335 (1974).
Meyuhas et al., "Translational Control of Ribosomal Protein mRNAs in Eukaryotes" Translational Control" pp. 363-388 (1996).
Mountford et al., "Internal ribosome entry sites and dicistronic RNAs in mammalian transgenesis" TIG. Vol. 11. No. 5, pp. 179-184 (1995).
Ogishima, et al., "Fractionation of Mammalian Tissue mRNAs by High-Petformance Gel Filtration Chromatography" Analytical Biochemistry 138, pp. 309-313 (1984). Oh et al., "Gene regulation: translational initiation by internal ribosome binding" Current Opinion in Genetics and development pp. 295-300 (1993).
Pelletier et al., "Internal initiation of translation of eukaryotic mRNA directed by a sequence derived from poliovirus RNA" Nature Vol. 334, pp. 320-325 (1988).
Schena et al., "Quantitative Monitoring of Gene Expression Patterns with a Complementary DNA Microarray" Science Vol. 270, pp. 467-470 1995).
Shen ei al., "Identification of the Human Prostatic Carcinoma Oncogene PTI- I by Rapid Expression Cloning and Differential RNA Display" Proc. Natl. Acad. Sci. USA Vol. 92, pp. 6778-6782 (1995).
Vagner et al. "Alternative Translation of Human Fibroblast Growth Factor 2 mRNA Occurs by Internal Entry of Ribosomes" Molecular and Cellular Biology, Vol. 15, No. 1, pp. 35-44 (1995).
   Welsh et al., "Arbitrary primed PCR fingerprinting of RNA". Nuc. Ac. Res. 20:49654970 (1992).
Zhao et al., "New primer strategy improves precision of differential display" Biotechniques 18: 842-850 (1995).
Sperling et al., "Abundant Nuclear Ribonucleoprotein Form of CAD RNA," Mol Cell Biol. 1985 Mar;5(3):569-75.
Dimitrijevic & Wgner "A search for novel, naturally occurring antisense RNA systems"
Murphy, P.R. and Knee R.S. (1994) Identification and characterization of an antisense RNA transcript (gfg) prom the human basic fibroblast growth factor gene. Mol. Endo Vol. 8 no. 7 852-859.
Adelman, J.P. Bond, C.T., Douglass, J. and Herbert, E. (1987) Two mammalian genes transcribed from opposite strands of the same DNA locus. Science 235:1514:13I3.
Kimelman, Du Kirschner, M.W. (1989) An antisense m-RNA directs the covalent modification of the transcript encoding pibroblast growth factor in Xenopus oocytes Cell, 59:687-696.
Lazer, M.A. Hodin, R.A., Darling D.S., Chin, ww. (1989) A novel member of the thyoid/steroid hormone receptor family is encoded by the opposite strand of the rat c-erbA a transcriptional unit. Mol. Cell. Biol. 9:1128-1136.
Miyajima, N., Horiuchi, R., Shibuya, Yl, Fuhushige, S., Mutsubara, K., Toyoshimak, Yamamoto, T (1989) Tow erba homologs encoding proteins with different T3 binding capacities are transcribed from opposite DNA strands of the same genetic locus. Cell. 57:31-39.
Kindy, M., McCormack, J. Buckler, A., Levine, R., Sanenshein G. (1987) Independent regulation of the transcription of the two strands of the c-myc gene. Mol-Cell. Biol. 7:2857-2562.
Celano, P., Berchtold, C., Kizer, D., Weararatna, F., Nelkin, B., Baylin, S., Casero, R. (1992) Characterization of an edogenous RNA transcript with homology to the antisense strand of the human c-mye gene. J. Biol.. Chem. 267:15092-15096.
Armstrong, B.C., Krystal, G.W. (1992) Isolation and characterization of complementary DNA per N-cym, a gene encoded by the DNA strand opposite to N-myc Cell Growth Differ. 3:385-390.
Taylor, E.R., Seleiro, E.A.P. Brickell, P.M. (1991) Identification of antisense transcripts of the chicken insulin-like growth factor II gene. J. Mol. Endocrial 7:145
Mitchell, P.J., Carothers, M., Han. J.H., Harding, J.D., Kas, E. Vendia, L., Chasin, L.A. (1986) Multiple transcription start sites, Dnase I-hypersentive sites, and an opposite strand exon in the 5' region of the CHO dnfr gene. Mol. Cell. Biol. 6:425-A search for novel, naturally occuring antisense RNA systems, N. Dimitrijevic, E.G.H. Wagner, Dept. of Microbiology, SLU (Swedish University of Agricultural Sciences), Genetic Center, P.O. Box 7025, S-75007 Uppsala, Sweden

Antisense RNA control of gene expression has been demonstrated in many bacteria, whereas only few cases are known in eukaryotes. All antisense RNAs identified to date have been found fortuitously. Therefore, our goal is to develop a novel strategy for targeted identification of naturally occuring antisense systems. The approach is based on the complementarity between antisense and target RNA over a significantly long stretch of nucleotides. The method used is briefly described here. Total cellular RNA is extracted. Part of the RNA pool is immobilized on a membrane, another part converted into cDNA after ligation of oligodeoxynucleotides to the 3' ends. The use of biotinylated, complementary oligos for cDNA synthesis allows immobilization of a "minus" strand to streptavidin-coated magnetic beads. A second set of oligos is ligated to the cDNA at the previous 5' end of the RNA. Plus strands are eluted from the bound strands and hybridized to the membrane-bound RNA. Since the cDNA strand used has the same polarity of the RNAs, only cDNA sequences that can bind to complementary RNAs should be retained. PCR amplification and subsequent cloning of PCR-fragments is followed by sequence analysis. To test whether cloned sequences are correctly identified, probes are generated in sense and antisense direction. Positive clones will be structurally and functionally characterized. In order to work out this method, we started using a bacterial strain (Escherichia coli), containing plasmid R1 that regulates its copy number by antisense RNA. Previous work has identified both antisense (CopA) and target RNA (CopT) of R1 intracellularly. This procedure, if feasible, will then be used to screen for antisense RNA systems in other organisms.

## Claims

1. An isolated nucleic acid molecule comprising SEQ ID NO: 32 encoding protein 608 or a functional portion thereof, or a nucleic acid molecule which is at least substantially homologous or identical thereto encoding a polypeptide having the same function.

2. The isolated nucleic acid molecule according to Claim 1, encoding the human protein 608 or a functional portion thereof.

3. A vector comprising the isolated nucleic acid molecule according to Claim 1 or 2.

4. A composition comprising the vector of Claim 3.

5. A probe or primer capable of specifically hybridising to the isolated nucleic acid molecule according to Claim 1 or 2.

6. An expression product of the isolated nucleic acid molecule according to Claim 1 or 2.

7. An isolated protein 608 polypeptide comprising SEQ ID NO: 33 or a functional portion thereof, or a polypeptide which is at least substantially homologous or identical thereto having the same function.

8. The isolated polypeptide of Claim 7 which comprises the human protein 608 or a functional portion thereof.

9. An antibody elicited by a polypeptide according to any of Claim 7 or 8, or a functional portion thereof.

10. A composition comprising the isolated polypeptide according to Claim 7 or 8, or the antibody or functional portion thereof according to Claim 9.

## Patentansprüche

1. Isoliertes Nukleinsäuremolekül, welches SEQ ID NO:32 umfaßt und Protein 608 oder einen funktionellen Teil davon codiert, oder Nukleinsäuremolekül, welches wenigstens im wesentlichen homolog oder identisch dazu ist und ein Polypeptid mit derselben Funktion codiert.

2. Isoliertes Nukleinsäuremolekül nach Anspruch 1, welches das menschliche Protein 608 oder einen funktionellen Teil davon codiert.

3. Vektor, der das isolierte Nukleinsäuremolekül nach Anspruch 1 oder 2 umfaßt.

4. Zusammensetzung, die den Vektor nach Anspruch 3 umfaßt.

5. Sonde oder Primer, die bzw. der in der Lage ist, spezifisch an das isolierte Nukleinsäuremolekül nach Anspruch 1 oder 2 zu hybridisieren.

6. Expressionsprodukt des isolierten Nukleinsäuremoleküls nach Anspruch 1 oder 2.

7. Isoliertes Protein 608-Polypeptid, welches SEQ ID NO:33 umfaßt, oder ein funktioneller Teil davon, oder Polypeptid, welches wenigstens im wesentlichen homolog oder identisch dazu ist und dieselbe Funktion besitzt.

8. Isoliertes Polypeptid nach Anspruch 7, welches das menschliche Protein 608 oder einen funktionellen Teil davon umfaßt.

9. Antikörper, der durch ein Polypeptid nach Anspruch 7 oder 8 ausgelöst wird, oder ein funktioneller Teil davon.

10. Zusammensetzung, die das isolierte Polypeptid nach Anspruch 7 oder 8 oder den Antikörper oder den funktionellen Teil davon nach Anspruch 9 umfaßt.

## Revendications

1. Molécule d'acide nucléique isolée comprenant SEQ ID N° 32 codant pour la protéine 608 ou une portion fonctionnelle de celle-ci, ou molécule d'acide nucléique qui lui est au moins substantiellement homologue ou identique codant pour un polypeptide ayant la même fonction.

2. Molécule d'acide nucléique isolée selon la revendication 1, codant pour la protéine 608 humaine ou une portion fonctionnelle de celle-ci.

3. Vecteur comprenant la molécule d'acide nucléique isolée selon la revendication 1 ou 2.

4. Composition comprenant le vecteur de la revendication 3.

5. Sonde ou amorce capable de s'hybrider spécifiquement à la molécule d'acide nucléique isolée selon la revendication 1 ou 2.

6. Produit d'expression de la molécule d'acide nucléique isolée selon la revendication 1 ou 2.

7. Polypeptide isolé de protéine 608 comprenant SEQ ID N° 33 ou une portion fonctionnelle de celle-ci, ou polypeptide qui lui est au moins substantiellement homologue ou identique ayant la même fonction.

8. Polypeptide isolé selon la revendication 7, qui comprend la protéine 608 humaine ou une portion fonctionnelle de celle-ci.

9. Anticorps induit par un polypeptide selon l'une quelconque de la revendication 7 ou 8, ou une portion fonctionnelle de celui-ci.

10. Composition comprenant le polypeptide isolé selon la revendication 7 ou 8, ou l'anticorps ou une portion fonctionnelle de celui-ci selon la revendication 9.


### SEQUENCE LISTING

<110> Quark Biotech, Inc
<120> MECHANICAL STRESS INDUCED GENES, EXPRESSION PRODUCTS THEREFROM, AND USES THEREOF
<130> P10270EP
<140> 99923223.4
   <141> 1999-05-14
<150> 60/085,673
   <151> 1998-05-15
<160> 55
<170> PatentIn Ver. 2.1
<210> 1
   <211> 241
   <212> DNA
   <213> Rattus sp.
<220>
   <221> gene
   <222> (1)..(241)
   <223> alpha 2u globulin-related protein
<400> 1
   tactgtttgt tttcagaggt cttctggaaa aataccatgg caaactggtc gtagtcagtg 60
   tcggccactt gcacatcgta gctctgtatc tgagggtagc tgtgaatatt ccccagggtg 120
   aactggccag gcctggagct tggaacgaat gttctgatcc agtagcgaca gccctggccc 180
   ctgacgagga tggaagtgac gttgtagcta ttgtcttcct gtagctcata gatggtgctg 240
   t                                                                 241
<210> 2
   <211> 192
   <212> DNA
   <213> Rattus sp.
<220>
   <221> gene
   <222> (1)..(192)
   <223> gamma-actin, cytoplasmic
<400> 2
   acatggctgg ggtattgaag gtttcaaaca ttatctgcgt catcttctct ctgttagctt 60
   tggggttcag gggggcctcg gtcagaagca ccgggtgctc ctcaggggcc acacgcagct 120
   cgttgtagaa ggtgtggtgc cagatcttct ccatgtcgtc ccagttggtg acaatgccgt 180
   gctcaatagg gt                                                      192
<210> 3
   <211> 288
   <212> DNA
   <213> Rattus sp.
<220>
   <221> gene
   <222> (1)..(288)
   <223> alpha-actin, cardiac
<400> 3
   acaggtcttt gcggatatcg atgtcacact tcatgatgct gttataagta gtttcgtgaa 60
   tgccggcaga ctccatacca atgaaagagg gctggaagag agtctcagga cagcggaaac 120
   gctcgttgcc aatggtgatg acctggccat cgggaagttc gtagctcttc tccagagagg 180
   aggaagatgc agcggtggcc atctcattct caaaatccag ggcgacataa cacagttttt 240
   ctttgatgtc tcggacaatt tcacgttcag cggtggtgac aaaggagt               288
<210> 4
   <211> 388
   <212> DNA
   <213> Rattus sp.
<220>
   <221> gene
   <222> (1)..(388)
   <223> alkaline phosphodiesterase RB13-6 Ag
<220>
   <221> misc_feature
   <222> (4)..(4)
   <223> "n" can be a or g or c or t
<220>
   <221> misc_feature
   <222> (151)..(151)
   <223> "n" can be a or g or c or t
<220>
   <221> misc_feature
   <222> (250)..(251)
   <223> "n" can be a or g or c or t
<220>
   <221> misc_feature
   <222> (255).. (255)
   <223> "n" can be a or g or c or t
<220>
   <221> misc_feature
   <222> (277)..(277)
   <223> "n" can be a or g or c or t
<220>
   <221> misc_feature
   <222> (284)..(284)
   <223> "n" can be a or g or c or t
<220>
   <221> misc_feature
   <222> (309) .. (309)
   <223> "n" can be a or g or c or t
<220>
   <221> misc_feature
   <222> (317)..(317)
   <223> "n" can be a or g or c or t
<220>
   <221> misc_feature
   <222> (323)..(323)
   <223> "n" can be a or g or c or t
<220>
   <221> misc_feature
   <222> (330)..(330)
   <223> "n" can be a or g or c or t
<220>
   <221> misc_feature
   <222> (361)..(361)
   <223> "n" can be a or g or c or t
<220>
   <221> misc_feature
   <222> (389)..(384)
   <223> "n" can be a or g or c or t
<220>
   <221> misc_feature
   <222> (388)..(388)
   <223> "n" can be a or g or c or t
<400> 4
   accnaggacc tgcccctcga atcaggactc gtaatatacc tcaggacttc tttaccttta 60
   attccgaaga gattgtcaga gacctcagct gtcgaaaatc cgatcaacac ttcaaaccct 120
   acttgactcc ggatttgccg aaacgactgc nctatgccaa gaacgtcagg attgacaaag 180
   ttcatctaat ggtggatcgg ctatggctgg cttacaggaa ctaagggagt tccaactgcg 240
   aatgcggaan ncacngttac aactatgaat ttaaaanttt ggangcaatc tttttggcac 300
   atggacccng ctttaangag aanactgttn tccaaccatt tgaaaacatc gaagtctata 360
   ntctactgtg tgatcttctg cacntccn                                   388
<210> 5
   <211> 403
   <212> DNA
   <213> Rattus sp.
<220>
   <221> gene
   <222> (1)..(403)
   <223> am2 receptor/LDL receptor related/LPR/A-2-macroglobulin receptor
<400> 5
   acccttctgc ctgtccctgt tgaagacctt gatgtcttta agctgcacgc cnatgcccgt 60
   tctcagaggc acggagtctg tggcattgcc tttgcatcca cgcttgatgg agccgttggc 120
   gtgagttctg tcactccagt agatgaaatc ctcaaacaca gacacagaga acatgtccat 180
   gttgttactg gacaggacca cctcacggtt ctcacccgtc tccagatcga tacgttcgat 240
   cttgtccatc cgggcatcac accagtagag cttgccgccc tgatagtcta ctgagatgcc 300
   attgggccag ctgatgctga cattaaccag caccactctc tctgtgccgt caaggcgaga 360
   ccgctcaatc cgtgggtagt gaccccactc agtccagaac aag                  403
<210> 6
   <211> 520
   <212> DNA
   <213> Rattus sp.
<220>
   <221> gene
   <222> (1)..(520)
   <223> aquaporin (AQA1), Channel integral membrane protein
<400> 6
   accatggcac caactggctg tttccctcta gtctcccttg cagtgcagat gtgacgtgtg 60
   tgtttattaa agagcactgg gctattgcag cgtcatgtct gaggaaagaa gcagctagac 120
   atgcaacaga ccccagacag atgcccatgc cgggcacaca ggggtttgga tgcccctagt 180
   tcatcatgat caaggaggcc actcttgtcc tggcttatga gctgctggct ggatgaattt 240
   gaccagagcc tggacaatct gaaggggctc actatgtgac tccaggcaca gtctccttat 300
   tgcaaggacc tgatgctgtg gcttctgcta tagcccaaga acatctcaga gtgcattcag 360
   ctcagggctt gcatttagct ctctgggtta ttctattcaa tcccaccaag ccagagcagc 420
   tctaccactg tgccgttaac catgtcgtga accgagacca cattcttcag gtgcttagaa 480
   gcagcagaat aatcaggagg ccattgacca ctggcatagt                      520
<210> 7
   <211> 492
   <212> DNA
   <213> Rattus sp.
<220>
   <221> gene
   <222> (1)..(492)
   <223> collagen type XII a-1
<400> 7
   acttaaatgt gacagatctg aaaacttacc aggttggatg ggacaccttc tgtgtcaaat 60
   ggtcacctca ccgggcagcg acctcttaca ggctaaagct gagtcctgcc gatggaacca 120
   ggggacaaga aataacagtg agaggatcag agaccagtca ctgcttcact ggcctctccc 180
   cggaggctga gtatggcgtt actgtttttg tgcagacacc aaatctcgag gggccgggtg 240
   tccccatcaa agaacagacg actgtgaaac caacagaggc tcccacagaa ccgcccacgc 300
   cttcacctcc tcccactatc ccacctgccc gtgatgtatg caaaggggcc aaggcagata 360
   ttgtgttcct gacggatgcc tcttggagta ttggagatga caattttaac aaagttgtaa 420
   aatttatttt taatactgtg ggggcctttg atgaaatcag ccctgctggg attcaggttt 480
   cttttgtgca gt                                                    492
<210> 8
   <211> 756
   <212> DNA
   <213> Rattus sp.
<220>
   <221> gene
   <222> (1)..(756)
   <223> complement component C3, prepo complement C3, rat pcRC201 mRNA prepo complement C3 (X52477)
<220>
   <221> misc_feature
   <222> (276)..(276)
   <223> "n" can be a or g or c or t
<220>
   <221> misc_feature
   <222> (290)..(290)
   <223> "n" can be a or g or c or t
<220>
   <221> misc_feature
   <222> (389)..(384)
   <223> "n" can be a or g or c or t
<220>
   <221> misc_feature
   <222> (408)..(908)
   <223> "n" can be a or g or c or t
<220>
   <221> misc_feature
   <222> (492)..(492)
   <223> "n" can be a or g or c or t
<220>
   <221> misc_feature
   <222> (505)..(505)
   <223> "n" can be a or g or c or t
<220>
   <221> misc_feature
   <222> (509)..(509)
   <223> "n" can be a or g or c or t
<220>
   <221> misc_feature
   <222> (536)..(536)
   <223> "n" can be a or g or c or t
<220>
   <221> misc_feature
   <222> (569)..(569)
   <223> "n" can be a or g or c or t
<220>
   <221> misc_feature
   <222> (582)..(582)
   <223> "n" can be a or g or c or t
<220>
   <221> misc_feature
   <222> (602)..(602)
   <223> "n" can be a or g or c or t
<220>
   <221> misc_feature
   <222> (625)..(625)
   <223> "n" can be a or g or c or t
<220>
   <221> misc_feature
   <222> (628)..(628)
   <223> "n" can be a or g or c or t
<220>
   <221> misc_feature
   <222> (632)..(632)
   <223> "n" can be a or g or c or t
<220>
   <221> misc_feature
   <222> (648)..(648)
   <223> "n" can be a or g or c or t
<220>
   <221> misc_feature
   <222> (668)..(668)
   <223> "n" can be a or g or c or t
<220>
   <221> misc_feature
   <222> (692)..(692)
   <223> "n" can be a or g or c or t
<220>
   <221> misc_feature
   <222> (697)..(697)
   <223> "n" can be a or g or c or t
<220>
   <221> misc_feature
   <222> (702)..(702)
   <223> "n" can be a or g or c or t
<220>
   <221> misc_feature
   <222> (714)..(714)
   <223> "n" can be a or g or c or t
<220>
   <221> misc_feature
   <222> (729)..(729)
   <223> "n" can be a or g or c or t
<220>
   <221> misc_feature
   <222> (737)..(737)
   <223> "n" can be a or g or c or t
<220>
   <221> misc_feature
   <222> (742) .. (792)
   <223> "n" can be a or g or c or t
<400> 8
   acttgggaaa cttggatgct actatgtcca tcctggacat ctccatgatg actggcttta 60
   ttccagacac aaacgacctg gaactgctga gctctggagt agacagatac atttccaagt 120
   atgagatgga caaagccttc tccaacaaga acaccctcat catctaccta gaaaagatct 180
   cacactccga agaagactgc ctgtccttca aagtccacca gttctttaac gtgggactta 240
   tccagccggg gtcggtcaag gtctactcct actacnatct aaaggagtcn tgcacccggt 300
   tctatcatcc ggagaaggac gatggaatgc tgagcaagct gtgccacaat gaaatgtgcc 360
   gctgtgccga ggagaactgc ttcntgcatc agtcacagga tcaggtcngc ctgaatgaac 420
   gactacacaa ggcttgtgag cctggagtgg actacgtgta cctcggccgc gaccacgctg 480
   ctgagccttg anccggggaa acaanaacng cccaccccat ctggggaggc ctttcnaagt 540
   ggtgctggga caagggatcc cctgctctng tgctcccctc cngacttatt gatttctttc 600
   cnttaacctg tcctgggcac gcccnttncc tnagggggac tagttcantg tttctgggga 660
   aaaatcancc ccctcactgg gttttgaccc tntggcnaga anaacacccc cccncccttt 720
   atgcccccng gtgttgncct tncctcgtta tgggcc 756
<210> 9
   <211> 340
   <212> DNA
   <213> Rattus sp.
<220>
   <221> gene
   <222> (1)..(340)
   <223> cytochrome oxidase C
<400> 9
   acgatccctg ttaggccccc tactgtgaat aagaagataa accctaaggc tcataatatg 60
   gcgggggatc atttgatatt ccctccatgt agtgtagcga gtcagctgaa tacttttacg 120
   cctgtaggaa ttgcgataat tatagtggca gatgtaaagt aggctcgggt gtctacatct 180
   aggcctactg tgaatatgtg atgtgctcat acaataaacc ctaggaagcc aatagatatt 240
   atggctcata ccatacctat atatccgaag ggttcttttt ttccagagta ataggtaact 300
   acatgtgaaa taattccaaa ccctggaaga attaagatgt                       340
<210> 10
   <211> 201
   <212> DNA
   <213> Rattus sp.
<220>
   <221> gene
   <222> (1)..(201)
   <223> DEST (274)
<400> 10
   accgtgtgtc cactttaatg gaagacagct tgatataaca aaacggcact tcagggttat 60
   tacacaccag gcagggatca ctctctaggt agtagccatc aaactccact aagccagaca 120
   aggtattgta aatgttggag ttgggatgat tggtgagaat gtggttctga gtcctcagaa 180
   tctccacggc cttctgtgag t                                           201
<210> 11
   <211> 755
   <212> DNA
   <213> Rattus sp.
<220>
   <221> gene
   <222> (1)..(755)
   <223> DEST (2-144)
<220>
   <221> misc_feature
   <222> (520)..(520)
   <223> "n" can be a or g or c or t
<220>
   <221> misc_feature
   <222> (526)..(526)
   <223> "n" can be a or g or c or t
<220>
   <221> misc_feature
   <222> (601)..(601)
   <223> "n" can be a or g or c or t
<220>
   <221> misc_feature
   <222> (603)..(603)
   <223> "n" can be a or g or c or t
<220>
   <221> misc_feature
   <222> (665)..(665)
   <223> "n" can be a or g or c or t
<220>
   <221> misc_feature
   <222> (694) .. (694)
   <223> "n" can be a or g or c or t
<220>
   <221> misc_feature
   <222> (708) .. (709)
   <223> "n" can be a or g or c or t
<220>
   <221> misc feature
   <222> (720)..(720)
   <223> "n" can be a or g or c or t
<400> 11
   acacactgcc cactctcctc ttggcagcag ccctgccact ttctttacac tgccagccac 60
   atgtggacat gggctactca tgactgtgga attctcttgg tgccacctta ttcctcagtg 120
   gtgctggtat gcccaatgga tagcatcaag atatgccaat gtgtttttgt tgggccagtt 180
   ggttccttgt ggggatctga gagtggttga ctccagtcca gggtctttca gtttctattc 240
   actctgtctg agcaaacagc ttcaatgccc ggatgagtgg aaactatcca tgctcagtaa 300
   tcttctgcga catcagggtt tctctgtgtt tcatgaatgg aattggttcc ctttcctcca 360
   caaccacaaa ttcattcttt caaagaagac ttgctttggt cccctctgtc cagtggtgtc 420
   aagggagcct tagtcccaga gtccactctc caggttgagc caactcccag agggtctttc 480
   ccactgaatg ggtcttccag ctgctgggaa gtggctgtgn gaaccncgcc tcttaagaca 540
   cagtggcttc agttgccaat gttcagaggg aagacctctt ctggggagat nantggggaa 600
   gggaagggat aaaaatgacc tcctgttttt cttggtgtaa gtttcattga atttttaatg 660
   gcganggtct cacactggtt aaccacttaa aganaccaat cactttcnng gttggccatn 720
   aaatgttgaa tgtctttggg ctcagtttca tttca                            755
<210> 12
   <211> 837
   <212> DNA
   <213> Rattus sp.
<220>
   <221> gene
   <222> (1) .. (837)
   <223> DEST (608) homology NCAM-1
<220>
   <221> misc_feature
   <222> (626)..(626)
   <223> "n" can be a or g or c or t
<220>
   <221> misc_feature
   <222> (735)..(735)
   <223> "n" can be a or g or c or t
<220>
   <221> misc_feature
   <222> (741)..(741)
   <223> "n" can be a or g or c or t
<220>
   <221> misc_feature
   <222> (751)..(751)
   <223> "n" can be a or g or c or t
<220>
   <221> misc_feature
   <222> (774)..(774)
   <223> "n" can be a or g or c or t
<220>
   <221> misc_feature
   <222> (805)..(805)
   <223> "n" can be a or g or c or t
<220>
   <221> misc_feature
   <222> (817)..(817)
   <223> "n" can be a or g or c or t
<220>
   <221> misc_feature
   <222> (820)..(820)
   <223> "n" can be a or g or c or t
<400> 12
   acctcttggt cctatagcca ctgtcatcag cttgggctac attgttgagc actgtcccat 60
   caggcaaact ccaggatacc tcaggcacag gggagccaga ggccttgcag tcaacttgga 120
   aatctttccc atggagcact tgcttcttaa aatactgctt ctgttcaatt ttggcaggtg 180
   tcaatctcag gcggacatgc atcaggacta ggtcatctcc cattttgttt cttgccacac 240
   ataagtagtc accagcgtct ttttccgtca ctgacccaac caccaaggat ccatttgggt 300
   agacgtggat tcggctgccc attctgtgcc actggtcgat gacagccttg gatggcagcc 360
   tccagattat tctaggcttt ggatccccag tagctgagca gttcagtagt aatttctcac 420
   ccaaattcac ctcagtccat gtctgagagg cagtttctat cctgggggtt gtctctccct 480
   cttccacagt aagaacacta cccttctctc tgagcctgag gagctggtgg caatgcactc 540
   ataagtgccc ctcactgaag gacgatgctt cttatataca gagttccatt tggatacaag 600
   aaaaatctgg gaatgagtca actgcnatgg ttttagttca gtcccatcat aaaaggaccc 660
   agtgaacact agggtggggg aattcctttt gcagtgcagg gcatttcaaa cttccaccta 720
   aaaaccccaa cgatnggctt nctcttttgg ntcctataat gaacaggggg aacngttgaa 780
   ttaacttttt tctttaaccc aacanttgaa tcctggncca naatgggttg cctggcc    837
<210> 13
   <211> 277
   <212> DNA
   <213> Rattus sp.
<220>
   <221> gene
   <222> (1)..(277)
   <223> DEST (2-197) similarity to erg3 (Y10624)
<400> 13
   acctgttccc caccagcgcc gaagactgca aattcacagg caatttcttc tacaactaca 60
   tgatggggat tgagttcaac ccccgcatcg gaaagtggtt tgacttcaag ctgttcttca 120
   atgggcgccc ggggattgtg gcctggactc ttatcaacct gtcctttgct gccaagcagc 180
   aggagcttta tggccatgtg accaactcca tgattttggt caatgtctta caggccatct 240
   atgtgttaga cttcttctgg aacgaaacct ggtatca                          277
<210> 14
   <211> 589
   <212> DNA
   <213> Rattus sp.
<220>
   <221> gene
   <222> (1)..(589)
   <223> DEST KIAA0183
<400> 14
   acatcagtcc cttcttgtgt ctgtgctcag ccactctcag cacctcaggt gcaacggggg 60
   gcaagggagg tgtggtgata tccatgtggt tcctggtggg cattgacagg aggcacggga 120
   ttggctgaac agtgcccatg tggctccctt tgccttcagc tagctggctt cccgaagagg 180
   cagtggatga accttctacc tttacttggt ctcctgggtc tccccgtgcc agtggctctg 240
   catggcttcc tgtcttctcc cagccaatct tgttcccgct gatgtggttg gttgcccctc 300
   cactgccctc atcgttgtct gatgaggacg aggtagacga gccaggggaa gtaggcggaa 360
   tccacgtgat tggggctgcc gctctgggcc aggttgatgg gcgaagaaac gctcatacag 420
   cggtgtgtgc ttcccctcgt gaggaaatgt tgctgtagct gttctgctct gtcaggttct 480
   tcccgctaga gtccagggcg aaggcaggct cagggaagct gtatgggtat ggaacctggc 540
   ctggggtgcc cctgggctgg gcacctgttg gaccgcgggc tttgcttgc             589
<210> 15
   <211> 768
   <212> DNA
   <213> Rattus sp.
<220>
   <221> gene
   <222> (1)..(768)
   <223> endothelin converting enzyme
<220>
   <221> misc_feature
   <222> (501)..(501)
   <223> "n" can be a or g or c or t
<220>
   <221> misc_feature
   <222> (558)..(558)
   <223> "n" can be a or g or c or t
<220>
   <221> misc_feature
   <222> (576) .. (576)
   <223> "n" can be a or g or c or t
<220>
   <221> misc_feature
   <222> (588)..(588)
   <223> "n" can be a or g or c or t
<220>
   <221> misc_feature
   <222> (605)..(605)
   <223> "n" can be a or g or c or t
<220>
   <221> misc_feature
   <222> (610)..(610)
   <223> "n" can be a or g or c or t
<220>
   <221> misc_feature
   <222> (634)..(634)
   <223> "n" can be a or g or c or t
<220>
   <221> misc_feature
   <222> (653)..(653)
   <223> "n" can be a or g or c or t
<220>
   <221> misc_feature
   <222> (656)..(656)
   <223> "n" can be a or g or c or t
<220>
   <221> misc_feature
   <222> (659)..(659)
   <223> "n" can be a or g or c or t
<220>
   <221> misc_feature
   <222> (698)..(698)
   <223> "n" can be a or g or c or t
<220>
   <221> misc_feature
   <222> (703)..(703)
   <223> "n" can be a or g or c or t
<220>
   <221> misc_feature
   <222> (738)..(738)
   <223> "n" can be a or g or c or t
<400> 15
   acgggacaaa gaagacctgt cttccccgct ggaagttttg cgtgagtgac acagaaaaca 60
   acctgggctt tgccctgggc cccatgtttg tgaaagcaac ctttgcggag gacagcaaga 120
   acatagccag cgagatcatc ctggagatca agaaggcatt cgaggagagc ctgagcaccc 180
   tgaaatggat ggatgaagat actcggaggt cagccaagga gaaggcggac gccatctaca 240
   acatgatagg ctaccccaac ttcatcatgg accccaagga gctggacaaa gtgttcaatg 300
   actacacagc agttcccgat ctctactttg agaacgccat gcgatttttc aacttctcat 360
   ggagggtcac agccgaccag ctcaggaaag cccccaacag agatcagtgg agtatgaccc 420
   cgcccatggt gaacgcctac tactcgccca ccaagaacga gattgtgttt ccagctggaa 480
   tcctgcaggc gctattttat ncccgctctt cgcccaacgc cttgaacttt ggtggtatcc 540
   gggtctttgt tgggcacnaa ctgactcctg ctttcnacga tcagggcngg agtttgacaa 600
   ggatnggaan ctcccggcct ggttgggaaa aacncctcgg tggaagcttc canccncana 660
   accaatgctt ggtttcaacc ttttcaaaac ctccaacncc aanggggggg cccggttccc 720
   accttttgtt ccccttantt gaaggggtta atttccaacc ttggggtt 768
<210> 16
   <211> 341
   <212> DNA
   <213> Rattus sp.
<220>
   <221> gene
   <222> (1)..(341)
   <223> erg25 methyl sterol (U60205) filamin (or region between filamin & GPI)
<400> 16
   actgaggcgg gccagggtag atgtcagcat tggtatcaag tgtgcccctg gagtactggg 60
   ccccactgag gctgatattg actttgatat catccgtaat gacaatgaca ccttcactgt 120
   gaaatacaca ccctgtgggg ctggcagcta taccatcatg gttctttttg ctgaccaggc 180
   cacacccacc agccccatca cactcaaatt ggagccttct catgatgcca gtaaagtgaa 240
   tgctgagggt cctgtcctaa atcgcactgg tgttgagctt ggcaaaccca cccatttcac 300
   agtcaatgct aaaactgctg ggaaaggcaa gccggagatt c 341
<210> 17
   <211> 382
   <212> DNA
   <213> Rattus sp.
<220>
   <221> gene
   <222> (1)..(382)
   <223> highly charged amino acid sequence (×59131)
<220>
   <221> misc_feature
   <222> (301)..(301)
   <223> "n" can be a or g or c or t
<220>
   <221> misc_feature
   <222> (307)..(307)
   <223> "n" can be a or g or c or t
<220>
   <221> misc_feature
   <222> (363)..(363)
   <223> "n" can be a or g or c or t
<400> 17
   actaggatag agcggaggcc taggagagga gaagccaccc acaaggctcc ctgtccacag 60
   tgcacagtca gttgttgaga tttccactgt ctgcagggtc ccttgtctca aatacctgtt 120
   aataatattt acattgaaat aataggtccc cgaagccact tttgatattc taggagtccg 180
   ggagtatttc aaaagctagg gccatgcgga gagggccttt gttcaggatt ttagtcttag 240
   gtcatcaggc ctccaaactt atcagccttg agcaactcaa acaagactcc tatccaccca 300
   ncctggncct cccccctccc cccacctttc taccgtctca ggcatgggac actgtggttc 360
   ctnaacctgt gactgatgcc cc                                          382
<210> 18
   <211> 459
   <212> DNA
   <213> Rattus sp.
<220>
   <221> gene
   <222> (1)..(459)
   <223> kidney aminopeptidase M/kidney Zn-peptidase
<220>
   <221> misc_feature
   <222> (158)..(158)
   <223> "n" can be a or g or c or t
<220>
   <221> misc feature
   <222> (374)..(375)
   <223> "n" can be a or g or c or t
<220>
   <221> misc_feature
   <222> (378)..(378)
   <223> "n" can be a or g or c or t
<220>
   <221> misc_feature
   <222> (408)..(408)
   <223> "n" can be a or g or c or t
<220>
   <221> misc feature
   <222> (411)..(411)
   <223> "n" can be a or g or c or t
<220>
   <221> misc_feature
   <222> (447)..(447)
   <223> "n" can be a or g or c or t
<400> 18
   acacgtagtc cactccaggc tcacaagcct tgtctagtcg ttcattcagg ctgacctgat 60
   cctgtgactg atgcatgaag cagttctcct cggcacagcg gcacatttca ttgtggcaca 120
   gcttgctcag cattccatcg tccttctccg gatgatanaa ccgggtgcat gactcctcta 180
   gattgtagta ggagtagacc ttgaccgacc ccggctggat aagtcccacg ttaaagaact 240
   ggtggacttt gaaggacagg cagtcttctt cggagtgtga gatcttttct aggtagatga 300
   tgagggtgtt cttgttggag aaggctttgt ccatctcata cttggaaatg tatctgtcta 360
   ctccagagct cagnngtncc aggtcgtttg tgtctggaat aaagccantc ntcatggaga 420
   tgtccaggat ggacatagta gcatccncgt ctcccaagt                        459
<210> 19
   <211> 479
   <212> DNA
   <213> Rattus sp.
<220>
   <221> gene
   <222> (1)..(479)
   <223> myosin heavy chain A
<220>
   <221> misc_feature
   <222> (17)..(17)
   <223> "n" can be a or g or c or t
<220>
   <221> misc_feature
   <222> (58)..(58)
   <223> "n" can be a or g or c or t
<220>
   <221> misc feature
   <222> (62)..(62)
   <223> "n" can be a or g or c or t
<220>
   <221> misc_feature
   <222> (67)..(67)
   <223> "n" can be a or g or c or t
<220>
   <221> misc_feature
   <222> (70)..(70)
   <223> "n" can be a or g or c or t
<220>
   <221> misc_feature
   <222> (126).. (126)
   <223> "n" can be a or g or c or t
<220>
   <221> misc_feature
   <222> (131)..(131)
   <223> "n" can be a or g or c or t
<220>
   <221> misc_feature
   <222> (135)..(136)
   <223> "n" can be a or g or c or t
<220>
   <221> misc_feature
   <222> (142)..(142)
   <223> "n" can be a or g or c or t
<220>
   <221> misc_feature
   <222> (145)..(145)
   <223> "n" can be a or g or c or t
<220>
   <221> misc_feature
   <222> (166)..(167)
   <223> "n" can be a or g or c or t
<220>
   <221> misc_feature
   <222> (182)..(182)
   <223> "n" can be a or g or c or t
<220>
   <221> misc_feature
   <222> (209)..(209)
   <223> "n" can be a or g or c or t
<220>
   <221> misc_feature
   <222> (223)..(223)
   <223> "n" can be a or g or c or t
<220>
   <221> misc_feature
   <222> (225)..(225)
   <223> "n" can be a or g or c or t
<220>
   <221> misc_feature
   <222> (254)..(254)
   <223> "n" can be a or g or c or t
<220>
   <221> misc_feature
   <222> (269)..(269)
   <223> "n" can be a or g or c or t
<220>
   <221> misc_feature
   <222> (275)..(275)
   <223> "n" can be a or g or c or t
<220>
   <221> misc_feature
   <222> (279)..(279)
   <223> "n" can be a or g or c or t
<220>
   <221> misc_feature
   <222> (289)..(289)
   <223> "n" can be a or g or c or t
<220>
   <221> misc_feature
   <222> (291)..(291)
   <223> "n" can be a or g or c or t
<220>
   <221> misc_feature
   <222> (293)..(293)
   <223> "n" can be a or g or c or t
<220>
   <221> misc_feature
   <222> (295) .. (295)
   <223> "n" can be a or g or c or t
<220>
   <221> misc_feature
   <222> (300)..(300)
   <223> "n" can be a or g or c or t
<220>
   <221> misc_feature
   <222> (315) .. (315)
   <223> "n" can be a or g or c or t
<220>
   <221> misc_feature
   <222> (335)..(335)
   <223> "n" can be a or g or c or t
<220>
   <221> misc_feature
   <222> (337)..(337)
   <223> "n" can be a or g or c or t
<220>
   <221> misc_feature
   <222> (340)..(340)
   <223> "n" can be a or g or c or t
<220>
   <221> misc_feature
   <222> (345)..(345)
   <223> "n" can be a or g or c or t
<220>
   <221> misc_feature
   <222> (358)..(358)
   <223> "n" can be a or g or c or t
<220>
   <221> misc_feature
   <222> (364)..(364)
   <223> "n" can be a or g or c or t
<220>
   <221> misc_feature
   <222> (367)..(367)
   <223> "n" can be a or g or c or t
<220>
   <221> misc_feature
   <222> (407)..(407)
   <223> "n" can be a or g or c or t
<220>
   <221> misc_feature
   <222> (421)..(421)
   <223> "n" can be a or g or c or t
<220>
   <221> misc_feature
   <222> (423)..(423)
   <223> "n" can be a or g or c or t
<220>
   <221> misc_feature
   <222> (429)..(430)
   <223> "n" can be a or g or c or t
<220>
   <221> misc_feature
   <222> (437)..(437)
   <223> "n" can be a or g or c or t
<220>
   <221> misc_feature
   <222> (440)..(440)
   <223> "n" can be a or g or c or t
<220>
   <221> misc_feature
   <222> (452)..(452)
   <223> "n" can be a or g or c or t
<220>
   <221> misc_feature
   <222> (467)..(467)
   <223> "n" can be a or g or c or t
<400> 19
   actcctcctg ctccagnatg aacatggtgt ggttgaacag ctgctgcagc ttctcgtngg 60
   tntactngat ntcaaagttg ctcgaatgag ttcacatcaa agatctcgaa gccagcggat 120
   gtccangatc ncganntatg angcnccctg cctcttggtc ttgtcnntaa ctttgttatt 180
   gngaaccacc agccagggga atatccgcnc gtaggtaccc ttngncagag cctccatggt 240
   aaaatcagcc tgcnctttct ctgtggccnt ctgcncatnc tcgctgccnt ncntnatgcn 300
   cggggtcaag ggatncctct ggttgacctc ggtcncnttn catcncccac gcaagtgngg 360
   acanctnttg aactcctgtg ttatctggca tggacgactt gttcagntgc tcccctccct 420
   ntncttgann gacccangtn cccctctgtc tnaatgtacc ccttganatg atctctcag  479
<210> 20
   <211> 539
   <212> DNA
   <213> Rattus sp.
<220>
   <221> gene
   <222> (1)..(539)
   <223> NOVEL PROTEIN AHNAK NUCLEOPROTEIN (M80899)
<220>
   <221> misc_feature
   <222> (127)..(127)
   <223> "n" can be a or g or c or t
<400> 20
   acatttttcc aaacatggtt ttcttgactt tgggagattt tacttcagct ttgagcgcag 60
   catcaggccc tgcaacgctg acatccacat caggaacttt gaggctggca tcaatctcac 120
   ctccagnaac attcaaatca aatccctgtt tcttggtctt gactttaggg ccactcatgt 180
   gaattttagg cattttaaac ttgcttttct tgcccttgcc accaatgttt atctcaggag 240
   tctcaacact cagttctgct tcagggacag tcacaccaac tgtgggggtc ttaatgtcag 300
   ctttggggct ttttgcacca aatccaaact tgggtttttt aaacttcgga actttaacat 360
   ctggcccttc aatattaata tctggactct ccatgtggac atctaagctt ggagtttcta 420
   aatcaacttt aggccccctg aggtcaactt cacctcctcc taacttaggc ccagaaattc 480
   caacatttgg tgtcttgagg tgcacattag catcaggccc acttatttta ggaacaggt  539
<210> 21
   <211> 349
   <212> DNA
   <213> Rattus sp.
<220>
   <221> gene
   <222> (1)..(349)
   <223> secretory protein containing thrombospondin motifs
<400> 21
   accagacctt gtgcagacct tccttgccca cgttggcagg tgggggattg gtcaccatgt 60
   tctaaaactt gtgggaaggg ttataagaag agaaccttga aatgtctgtc ccatgatggc 120
   ggtgtgttat caaatgagag ctgtgatcct ttgaataaac caaagcatta cattgacttt 180
   tgcatactga cacagtgcag ttaacaggtt ttgaggacaa ggtagcgtgg gagggctgat 240
   acactgaaag caagagtgct ggagggatcc tgtgaatcaa accagtaaac agtgaggtgt 300
   ggcaatgagg tgtgtatagg ggatacatta gcaaaagagg tagatccgt             349
<210> 22
   <211> 323
   <212> DNA
   <213> Rattus sp.
<220>
   <221> gene
   <222> (1)..(323)
   <223> steraoyl CoA desaturase (protein kinase)
<400> 22
   actgtgcagc gggctggctg cagaaactta agtcataaca ctctttgtcg ctgaggtctg 60
   aagctctctg cacgttctca tccctgggaa cgtgacccca gcatccgacg ccaagatgcc 120
   ggctcacata ctgcaagaga tctctggctc ttactcggcc accaccacaa tcacagcgcc 180
   accttctggg ggacagcaga atggaggaga gaagtttgaa aggaatcctc accactgggg 240
   agcagatgtt cgccctgaaa ttaaagatga cctatacgac cccagctacc aggatgagga 300
   ggggcccccg cccaagctgg agt                                         323
<210> 23
   <211> 551
   <212> DNA
   <213> Rattus sp.
<220>
   <221> gene
   <222> (1)..(551)
   <223> tenascin
<220>
   <221> misc_feature
   <222> (151)..(151)
   <223> "n" can be a or g or c or t
<220>
   <221> misc_feature
   <222> (158)..(158)
   <223> "n" can be a or g or c or t
<220>
   <221> misc_feature
   <222> (186)..(186)
   <223> "n" can be a or g or c or t
<220>
   <221> misc_feature
   <222> (217)..(217)
   <223> "n" can be a or g or c or t
<220>
   <221> misc_feature
   <222> (235)..(235)
   <223> "n" can be a or g or c or t
<220>
   <221> misc_feature
   <222> (238)..(238)
   <223> "n" can be a or g or c or t
<220>
   <221> misc_feature
   <222> (241)..(241)
   <223> "n" can be a or g or c or t
<220>
   <221> misc_feature
   <222> (390)..(390)
   <223> "n" can be a or g or c or t
<220>
   <221> misc feature
   <222> (500)..(500)
   <223> "n" can be a or g or c or t
<220>
   <221> misc_feature
   <222> (535)..(535)
   <223> "n" can be a or g or c or t
<400> 23
   cattggagct ccaccgcggt ggcggccgct ctagaatagt ggatcccccg ggctgcagga 60
   attcgatact catgggcccc ttccagttgg aaaccattta aaaaccctga atggtgaata 120
   atgttcccat atctgcccat caaggttgaa nccatganag ttcttatacc agaaagctcc 180
   tttgtnggac agggcacagt tggtgatggc tgaatcngtg tccttgtcat aggtnganaa 240
   ngatctacca ttgtgatagt tcatggaatc acctaccgtt ccactgtatc cttctacctt 300
   cagcttgtag cgactcttgg catctccaac actgaacctg tcatacacag cataagctga 360
   ctccccatgg tcttgtaggt ccacccggan ctcatattgc ccttgggctg tgattttgct 420
   caggttatcc agtccaagcc agaattcttc tctgcggtct ccaaacccaa tggcattagc 480
   cttccagttt ccaaagaaan tcctcacgtc catttttgcg tctcaagaaa acgantcatc 540
   cacctccatc c                                                      551
<210> 24
   <211> 462
   <212> DNA
   <213> Rattus sp.
<220>
   <221> gene
   <222> (1)..(462)
   <223> thrombospondin 1
<400> 24
   accactgaag tccacggcat taaactcatc ataacctaca gcaagtccag ggtcacagtt 60
   tacagtctgg acaagttctt tgccctgatg gcggacaacc cagttagggt cattttggga 120
   ggttcctttg ggatctagag gaatcatctg gaatcggcgg aaatcggttt cactgatgtc 180
   aacattctca ggacagatgt catcaatgtc tggcacattg tcatggtcaa agtcgtcttt 240
   gcaggcatcg cctcggccat caccatcaga gtccttctgg tcaggattgg gcaccagcct 300
   gcagttgtct ctgtcatcag ggatgccgtc gttgtcatcg tcatggtcac aggcgtctcc 360
   tttaccatct ttatcgtggt cggcctggtt ggcattgggc acatagggac agttgtccac 420
   gttgttctga tggccatcct catcgatgtc ctgattgttg tc                    462
<210> 25
   <211> 283
   <212> DNA
   <213> Rattus sp.
<220>
   <221> gene
   <222> (1) .. (283)
   <223> DEST 700
<220>
   <221> misc feature
   <222> (171) .. (171)
   <223> "n" can be a or g or c or t
<400> 25
   actctcccca ggtgttgagg tgggaggaaa ggggcttctg caccttcgat gatggcctcg 60
   atacctgact gcgggttctg cagctctgtt tggtagcact gctggcaggg aaacttcctg 120
   cctcctggat gaaggaaggg tttactcatc atcactggca gctgcaggaa ntcgggggcc 180
   tgataaacaa tttcataacc cctttctgct agtccgtctg ccaaaaaact caaaggaccc 240
   aagcagggca gctgttccac aggcgagcca ctgtcagctg agt                   283
<210> 26
   <211> 344
   <212> DNA
   <213> Rattus sp.
<220>
   <221> gene
   <222> (1)..(344)
   <223> H.sapiens mRNA for ctGF (78974)
<220>
   <221> misc feature
   <222> (329)..(329)
   <223> "n" can be a or g or c or t
<400> 26
   actacaggaa gatgtatgga gacatggcgt aaagccaggg agtaagggac acgaactcat 60
   ttagactata acttgaactg agttacatct cattttcttc tgtaaaaaaa caaaaaggat 120
   tacagtagca cattaattta aatctgggtt cctaactgct gtgggagaaa acaccccacc 180
   gaagtgagaa ccgtgtgtca ttgtcatgca aatagcctgt caatctcaga cactggtttc 240
   gagacagttt agacttgaca gttgttcact agcgcacagt gacagaacgc acactaaggt 300
   gagcctcctg gcaagagtgg acatgccanc gagaaaagac aggt                  344
<210> 27
   <211> 239
   <212> DNA
   <213> Rattus sp.
<220>
   <221> gene
   <222> (1)..(239)
   <223> sarp 1 (AF017989)
<400> 27
   actacagcta ttcaaaacta cccttaaaat gagctgtttt aaaacttcat aaaaaacagt 60
   catgatttta ttagttgtga atatttctac aagatctgga tggacttttc gctttatgtg 120
   aaaatagcta tctgttcccg gggcctccta gggccttata actcaggaca cgttaggata 180
   cacacatgca aaaggcaggg caagctgctc caagctgaga ctgtagctct cccgacagt  239
<210> 28
   <211> 290
   <212> DNA
   <213> Rattus sp.
<220>
   <221> gene
   <222> (1)..(290)
   <223> Syntrophin 1 mus musculus (U00677)
<220>
   <221> misc_feature
   <222> (143)..(143)
   <223> "n" can be a or g or c or t
<400> 28
   acacagaagc cattccgagg ctgggacctg accctgtggg gatccacccc atctccagtc 60
   aacagcagag ctgccacacc tcctggaggc ccctaagcca ccctgagatc tctgcagatg 120
   gagtcttctg ggtcagtggc aanaggagga agaggagcct ttctgttcac tctcccttgg 180
   gccccgaggg ctgtctgggc tctctcctcc tctctctcct tcctcttgat aataaacagc 240
   ctgtgagcac atagacaaaa aaaaaaaaaa aaaaaaaaaa aaaagcttgt            290
<210> 29
   <211> 592
   <212> DNA
   <213> Rattus sp.
<220>
   <221> gene
   <222> (1)..(592)
   <223> erg25 methyl sterol (U60205) filamin (or region between filamin & GPI)
<400> 29
   actggacatc cgtcccaaag attctgtccc accacgtgaa ggtggaggcg taattcccaa 60
   tgaagttcat gtggtggaaa tcgtgatgcc gagccccggt ataaaaaggg atgtagttca 120
   acgggttgag agggatatca taaccactgt gcacatcgat ggtttctagc aaacgcatgg 180
   tcacccatgc ccacagaaga attacatgat cacacaaaag cacaattcca atgaaaaaat 240
   ccagtcccaa gaattagggt ttccaaggga tgtgcatatt ctgcttcgat tccaaatgga 300
   gcctggaaac tcgtgatgga ctttatgaat gtatttataa atcctcttgt ggtgaaggag 360
   tctgtgcagg aaatagtgcc aggtatcctc gatgactgca cagcccaaac accttgccaa 420
   agtgaaatac catcttggca ttctttccca atcataagga atattgaaaa actctgtaaa 480
   atagtatgtt ccacaaatca gaagcaactg gataaaaaaa tgattaaaca gaattccttt 540
   caaacacttc cactggcctt caaaggttct ggtttatcct tttgaatctt gt         592
<210> 30
   <211> 344
   <212> DNA
   <213> Rattus sp.
<220>
   <221> gene
   <222> (1)..(330)
   <223> DEST (AA240223)
<220>
   <221> misc_feature
   <222> (334)..(334)
   <223> "n" can be a or g or c or t
<400> 30
   accataagca gtttccatgg ggtcagagaa tggctggaga gttgtggaaa ccagcaggag 60
   gtattgtttt ggagacagga cctcactgtg tagccctggc ctcaaactca gagatccacc 120
   tgcctctgcc tcctgagtgc ttggattaaa ggcatgcacc accaccgccg cctgacaggg 180
   aggaagtttc tgatggagga gctattgaca ggtagcagag acagccgaaa gacgatcatg 240
   ggccatctgt ggggtcagtg ggggctgctg tatgtctttg ctgctttaca gggaaggtga 300
   tgtatgcatc gtagccaaac agcaaggtag ccantaagcc cagt                   344
<210> 31
   <211> 102
   <212> DNA
   <213> Rattus sp.
<220>
   <221> gene
   <222> (1)..(102)
   <223> OSTEONECTINE
<400> 31
   acttgtcatt gtctaggtca caggtctcaa agaagcgagt ggtgcaatgt tccatgggaa 60
   tgaggggagc gcgcagtggg gccagctccg tgtgggacag gt                     102
<210> 32
   <211> 8882
   <212> DNA
   <213> Rattus sp.
<220>
   <221> CDS
   <222> (574)..(8367)
   <223> Coding sequence for the inventive nucleic acid molecule 608.
<220>
   <221> misc_feature
   <222> (5560) .. (5560)
   <223> "n" can be a or g or c or t
<220>
   <221> misc_feature
   <222> (1663)..(1663)
   <223> "Xaa" can be any amino acid
<400> 32
   gagagacgac agaaggttac ggctgcgaga agacgacaga agggtccaga aaaaggaaag 60
   tgctggaggg gagtggggac aaaagcagcg accaagtgaa tgtcacttca gtgactgagg 120
   ccaggcaaaa cgcgcgggaa ggattttgtg tagcttggga ccctttcata gacactgatg 180
   acacgtttac gcaaaataga aatttgagga gaaacgcctg ggccttcgga aaggagtgat 240
   tgattagtac ttgcaagttt aggtgacttt aaggagaact aactaatgta tactattgag 300
   ggaggaggaa gagcattaca gagtttccag cagcagcagg aaagctttgg ttaatttgga 360
   aatggatgat agcattaaaa taacagaagc gcctccaggt ctctgaagct tcagtccccc 420
   agctgaaagc cagaaaagac taagcccact aagccttttg atccctttgg aagcaaagaa 480
   ctttccttcc ctggggtgaa gactctcctc agaagatttc ctgtctctgc ctatgttaca 540
   agaggaatca aaaccaagac agaagagctc agg atg cag gtg aga ggc agg gaa   594
                                        Met Gln Val Arg Gly Arg Glu
                                         1                   5
   gtc agc ggc ttg ttg atc tcc ctc act gct gtc tgc ctg gtg gtc acc    642
   Val Ser Gly Leu Leu Ile Ser Leu Thr Ala Val Cys Leu Val Val Thr
           10                  15                  20
   cct ggg agc agg gcc tgt cct cgc cgc tgt gcc tgc tat gtg ccc aca    690
   Pro Gly Ser Arg Ala Cys Pro Arg Arg Cys Ala Cys Tyr Val Pro Thr
       25                  30                  35
   gag gtg cac tgt aca ttt cgg tac ctg acc tcc atc cca gat ggc atc    738
   Glu Val His Cys Thr Phe Arg Tyr Leu Thr Ser Ile Pro Asp Gly Ile
    40                  45                  50                  55
   ccg gcc aat gtg gaa cga ata aat tta gga tat aac agc ctt act aga    786
   Pro Ala Asn Val Glu Arg Ile Asn Leu Gly Tyr Asn Ser Leu Thr Arg
                    60                  65                  70
   ttg aca gaa aac gac ttt gat ggc ctg agc aaa ctg gag tta ctc atg    834
   Leu Thr Glu Asn Asp Phe Asp Gly Leu Ser Lys Leu Glu Leu Leu Met
                75                  80                  85
   ctg cac agt aat ggc att cac aga gtc agt gac aag acc ttc tcg ggc    882
   Leu His Ser Asn Gly Ile His Arg Val Ser Asp Lys Thr Phe Ser Gly
            90                  95                 100
   ttg cag tcc ttg cag gtc tta aaa atg agc tat aac aaa gtc caa atc    930
   Leu Gln Ser Leu Gln Val Leu Lys Met Ser Tyr Asn Lys Val Gln Ile
       105                 110                 115
   att cgg aag gat act ttc tac gga ctc ggg agc ttg gtc cgg ttg cac    978
   Ile Arg Lys Asp Thr Phe Tyr Gly Leu Gly Ser Leu Val Arg Leu His
   120                 125                 130                 135
   ctg gat cac aac aac att gaa ttc atc aac cct gag gcc ttt tat gga   1026
   Leu Asp His Asn Asn Ile Glu Phe Ile Asn Pro Glu Ala Phe Tyr Gly
                   140                 145                 150
   ctt acc tcg ctc cgc ttg gta cat tta gaa gga aac cgg ctc aca aag   1074
   Leu Thr Ser Leu Arg Leu Val His Leu Glu Gly Asn Arg Leu Thr Lys
               155                 160                 165
   ctc cat cca gac aca ttt gtc tca tta agc tat ctc cag ata ttt aaa   1122
   Leu His Pro Asp Thr Phe Val Ser Leu Ser Tyr Leu Gln Ile Phe Lys
           170                 175                 180
   acc tct ttc att aag tac ctg ttc ttg tct gat aac ttc ctg acc tcc   1170
   Thr Ser Phe Ile Lys Tyr Leu Phe Leu Ser Asp Asn Phe Leu Thr Ser
       185                 190                 195
   ctc cca aaa gaa atg gtc tcc tac atg cca aac cta gaa agc ctg tat   1218
   Leu Pro Lys Glu Met Val Ser Tyr Met Pro Asn Leu Glu Ser Leu Tyr
   200                 205                 210                 215
   ttg cat gga aac cca tgg acc tgt gac tgc cat tta aag tgg ttg tct   1266
   Leu His Gly Asn Pro Trp Thr Cys Asp Cys His Leu Lys Trp Leu Ser
                   220                 225                 230
   gag tgg atg cag gga aac cca gat ata ata aaa tgc aag aaa gac aga   1314
   Glu Trp Met Gln Gly Asn Pro Asp Ile Ile Lys Cys Lys Lys Asp Arg
               235                 240                 245
   agc tct tcc agt cct cag caa tgt ccc ctt tgc atg aac ccc agg atc    1362
   Ser Ser Ser Ser Pro Gln Gln Cys Pro Leu Cys Met Asn Pro Arg Ile
           250                 255                 260
   tct aaa ggc aga ccc ttt gct atg gta cca tct gga gct ttc cta tgt    1410
   Ser Lys Gly Arg Pro Phe Ala Met Val Pro Ser Gly Ala Phe Leu Cys
       265                 270                 275
   aca aag cca acc att gat cca tca ctg aag tca aag agc ctg gtt act    1458
   Thr Lys Pro Thr Ile Asp Pro Ser Leu Lys Ser Lys Ser Leu Val Thr
   280                 285                 290                 295
   cag gag gac aat gga tct gcc tcc acc tca cct caa gat ttc ata gaa    1506
   Gln Glu Asp Asn Gly Ser Ala Ser Thr Ser Pro Gln Asp Phe Ile Glu
                   300                 305                 310
   ccc ttt ggc tcc ttg tct ttg aac atg aca kas mtk tct gga aat aag    1554
   Pro Phe Gly Ser Leu Ser Leu Asn Met Thr Xaa Xaa Ser Gly Asn Lys
               315                 320                 325
   gcc gac atg gtc tgt agt atc caa aag cca tca agg aca tca cca act    1602
   Ala Asp Met Val Cys Ser Ile Gln Lys Pro Ser Arg Thr Ser Pro Thr
           330                 335                 340
   gca ttc act gaa gaa aat gac tac atc atg cta aat gcg tca ttt tcc    1650
   Ala Phe Thr Glu Glu Asn Asp Tyr Ile Met Leu Asn Ala Ser Phe Ser
       345                 350                 355
   aca aat ctt gtg tgc agt gta gat tat aat cac atc cag cca gtg tgg    1698
   Thr Asn Leu Val Cys Ser Val Asp Tyr Asn His Ile Gln Pro Val Trp
   360                 365                 370                 375
   caa ctt ctg gct tta tac agt gac tct cct ctg ata cta gaa agg aag    1746
   Gln Leu Leu Ala Leu Tyr Ser Asp Ser Pro Leu Ile Leu Glu Arg Lys
                   380                 385                 390
   ccc cag ctt acc gag act cct tca ctg tct tct aga tat aaa cag gtg    1794
   Pro Gln Leu Thr Glu Thr Pro Ser Leu Ser Ser Arg Tyr Lys Gln Val
               395                 400                 405
   gct ctt agg cct gaa gac att ttt acc agc ata gag gct gat gtc aga    1842
   Ala Leu Arg Pro Glu Asp Ile Phe Thr Ser Ile Glu Ala Asp Val Arg
           410                 415                 420
   gca gac cct ttt tgg ttc caa caa gaa aaa att gtc ttg cag ctg aac    1890
   Ala Asp Pro Phe Trp Phe Gln Gln Glu Lys Ile Val Leu Gln Leu Asn
       425                 430                 435
   aga act gcc acc aca ctt agc aca tta cag atc cag ttt tcc act gat    1938
   Arg Thr Ala Thr Thr Leu Ser Thr Leu Gln Ile Gln Phe Ser Thr Asp
   440                 445                 450                 455
   gct caa atc gct tta cca agg gcg gag atg aga gcg gag aga ctc aaa    1986
   Ala Gln Ile Ala Leu Pro Arg Ala Glu Met Arg Ala Glu Arg Leu Lys
                   460                 465                 470
   tgg acc atg atc ctg atg atg aac aat ccc aaa ctg gaa cgc act gtc    2034
   Trp Thr Met Ile Leu Met Met Asn Asn Pro Lys Leu Glu Arg Thr Val
                    475                      480                       485
   ctg gtt ggc ggc act att gcc ctg agc tgt cca ggc aaa ggc gac cct     2082
   Leu Val Gly Gly Thr Ile Ala Leu Ser Cys Pro Gly Lys Gly Asp Pro
           490                     495                     500
   tca cct cac ttg gaa tgg ctt cta gct gat ggg agt aaa gtg aga gcc     2130
   Ser Pro His Leu Glu Trp Leu Leu Ala Asp Gly Ser Lys Val Arg Ala
       505                     510                     515
   cct tac gtt agc gag gat ggg cga atc cta ata gac aaa aat ggg aag     2178
   Pro Tyr Val Ser Glu Asp Gly Arg Ile Leu Ile Asp Lys Asn Gly Lys
   520                     525                     530                 535
   ttg gaa ctg cag atg gct gac agc ttt gat gca ggt ctt tac cac tgc     2226
   Leu Glu Leu Gln Met Ala Asp Ser Phe Asp Ala Gly Leu Tyr His Cys
                   540                     545                     550
   ata agc acc aat gat gca gat gcg gat gtt ctc aca tac agg ata act     2274
   Ile Ser Thr Asn Asp Ala Asp Ala Asp Val Leu Thr Tyr Arg Ile Thr
               555                     560                     565
   gtg gta gag ccc tat gga gaa agc aca cat gac agt gga gtc cag cac     2322
   Val Val Glu Pro Tyr Gly Glu Ser Thr His Asp Ser Gly Val Gln His
           570                     575                     580
   aca gtg gtt acg ggt gag acg ctc gac ctt cca tgc ctt tcc acg ggt     2370
   Thr Val Val Thr Gly Glu Thr Leu Asp Leu Pro Cys Leu Ser Thr Gly
       585                     590                     595
   gtt cca gat gct tct att agc tgg att ctt cca ggg aac act gtg ttc     2418
   Val Pro Asp Ala Ser Ile Ser Trp Ile Leu Pro Gly Asn Thr Val Phe
   600                     605                     610                 615
   tct cag cca tca aga gac agg caa att ctt aac aat ggg acc tta aga     2466
   Ser Gln Pro Ser Arg Asp Arg Gln Ile Leu Asn Asn Gly Thr Leu Arg
                   620                     625                     630
   ata tta cag gtt acg cca aaa gat caa ggt cat tac caa tgt gtg gct     2514
   Ile Leu Gln Val Thr Pro Lys Asp Gln Gly His Tyr Gln Cys Val Ala
               635                     640                     645
   gcc aac cca tca ggg gcc gac ttt tcc agt ttt aaa gtt tca gtt caa     2562
   Ala Asn Pro Ser Gly Ala Asp Phe Ser Ser Phe Lys Val Ser Val Gln
           650                     655                     660
   aag aaa ggc caa agg atg gtt gag cat gac agg gag gca ggt gga tct     2610
   Lys Lys Gly Gln Arg Met Val Glu His Asp Arg Glu Ala Gly Gly Ser
       665                     670                     675
   gga ctt gga gaa ccc aac tcc agt gtt tcc ctt aag cag cca gca tct     2658
   Gly Leu Gly Glu Pro Asn Ser Ser Val Ser Leu Lys Gln Pro Ala Ser
   680                     685                     690                 695
   ttg aaa ctc tct gca tca gct ttg aca ggg tca gag gct gga aaa caa     2706
   Leu Lys Leu Ser Ala Ser Ala Leu Thr Gly Ser Glu Ala Gly Lys Gln
                   700                     705                     710
   gtc tcc ggt gta cat agg aag aac aaa cat aga gac tta ata cat cgg     2754
   Val Ser Gly Val His Arg Lys Asn Lys His Arg Asp Leu Ile His Arg
               715                     720                     725
   cgg cgt ggg gat tcc acg ctc cgg cga ttc agg gag cat agg agg cag    2802
   Arg Arg Gly Asp Ser Thr Leu Arg Arg Phe Arg Glu His Arg Arg Gln
           730                 735                 740
   ctc cct ctc tct gct cgg aga att gac ccg caa cgc tgg gca gca ctt    2850
   Leu Pro Leu Ser Ala Arg Arg Ile Asp Pro Gln Arg Trp Ala Ala Leu
       745                 750                 755
   cta gaa aaa gcc aaa aag aat tct gtg cca aaa aag caa gaa aat acc    2898
   Leu Glu Lys Ala Lys Lys Asn Ser Val Pro Lys Lys Gln Glu Asn Thr
   760                 765                 770                 775
   aca gta aag cca gtg cca ctg gct gtt ccc ctc gtg gaa ctc act gac    2946
   Thr Val Lys Pro Val Pro Leu Ala Val Pro Leu Val Glu Leu Thr Asp
                   780                 785                 790
   gag gaa aag gat gcc tct ggc atg att cct cca gat gaa gaa ttc atg    2994
   Glu Glu Lys Asp Ala Ser Gly Met Ile Pro Pro Asp Glu Glu Phe Met
               795                 800                 805
   gtt ctg aaa act aag gct tct ggt gtc cca gga agg tca cca act gct    3042
   Val Leu Lys Thr Lys Ala Ser Gly Val Pro Gly Arg Ser Pro Thr Ala
           810                 815                 820
   gac tct gga cca gta aat cat ggt ttt atg acg agt ata gct tct ggc    3090
   Asp Ser Gly Pro Val Asn His Gly Phe Met Thr Ser Ile Ala Ser Gly
       825                 830                 835
   aca gaa gtc tca act gtg aat cca caa aca cta caa tct gag cac ctt    3138
   Thr Glu Val Ser Thr Val Asn Pro Gln Thr Leu Gln Ser Glu His Leu
   840                 845                 850                 855
   cct gat ttc aaa tta ttt agt gta aca aac ggt aca gct gtg aca aag    3186
   Pro Asp Phe Lys Leu Phe Ser Val Thr Asn Gly Thr Ala Val Thr Lys
                   860                 865                 870
   agt atg aac cca tcc ata gca agc aaa ata gaa gat aca acc aac caa    3234
   Ser Met Asn Pro Ser Ile Ala Ser Lys Ile Glu Asp Thr Thr Asn Gln
               875                 880                 885
   aac cca atc att atc ttt cca tca gta gct gaa att cga gat tct gct    3282
   Asn Pro Ile Ile Ile Phe Pro Ser Val Ala Glu Ile Arg Asp Ser Ala
           890                 895                 900
   cag gca gga aga gca tct tcc caa agt gca cac cct gta aca ggg gga    3330
   Gln Ala Gly Arg Ala Ser Ser Gln Ser Ala His Pro Val Thr Gly Gly
       905                 910                 915
   aac atg gct acc tat ggc cat acc aac aca tat agt agc ttt acc agc    3378
   Asn Met Ala Thr Tyr Gly His Thr Asn Thr Tyr Ser Ser Phe Thr Ser
   920                 925                 930                 935
   aaa gcc agt aca gtc ttg cag cca ata aat cca aca gaa agt tat gga    3426
   Lys Ala Ser Thr Val Leu Gln Pro Ile Asn Pro Thr Glu Ser Tyr Gly
                   940                 945                 950
   cct cag ata cct att aca gga gtc agc aga cct agc agt agt gac atc    3474
   Pro Gln Ile Pro Ile Thr Gly Val Ser Arg Pro Ser Ser Ser Asp Ile
               955                 960                 965
   tct tct cac act act gca gac cct agc ttc tcc agt cac cct tca ggt   3522
   Ser Ser His Thr Thr Ala Asp Pro Ser Phe Ser Ser His Pro Ser Gly
           970                 975                 980
   tca cac acc act gcc tcg tct tta ttt cac att cct aga aac aac aat   3570
   Ser His Thr Thr Ala Ser Ser Leu Phe His Ile Pro Arg Asn Asn Asn
       985                 990                 995
   aca ggt aac ttc ccc ttg tcc agg cac ttg gga aga gag agg aca att   3618
   Thr Gly Asn Phe Pro Leu Ser Arg His Leu Gly Arg Glu Arg Thr Ile
   1000                1005                1010                1015
   tgg agc aga ggg aga gtt aaa aac cca cat aga acc cca gtt ctc cga   3666
   Trp Ser Arg Gly Arg Val Lys Asn Pro His Arg Thr Pro Val Leu Arg
                   1020                1025                1030
   cgg cat aga cac agg act gtg agg cca gca atc aag gga cct gct aac   3714
   Arg His Arg His Arg Thr Val Arg Pro Ala Ile Lys Gly Pro Ala Asn
               1035                1040                1045
   aaa aat gtg agc caa gtt cca gcc aca gag tac cct ggg atg tgc cac   3762
   Lys Asn Val Ser Gln Val Pro Ala Thr Glu Tyr Pro Gly Met Cys His
           1050                1055                1060
   aca tgt cct tcc gca gag ggg ctc aca gtg gct act gca gca ctg tca   3810
   Thr Cys Pro Ser Ala Glu Gly Leu Thr Val Ala Thr Ala Ala Leu Ser
       1065                1070                1075
   gtt cca agt tca tcc cac agt gcc ctc ccc aaa act aat aat gtt ggg   3858
   Val Pro Ser Ser Ser His Ser Ala Leu Pro Lys Thr Asn Asn Val Gly
   1080                1085                1090                1095
   gtc ata gca gaa gag tct acc act gtg gtc aag aaa cca ctg tta cta   3906
   Val Ile Ala Glu Glu Ser Thr Thr Val Val Lys Lys Pro Leu Leu Leu
                   1100                1105                1110
   ttt aag gac aaa caa aat gta gat att gag ata ata aca acc act aca   3954
   Phe Lys Asp Lys Gln Asn Val Asp Ile Glu Ile Ile Thr Thr Thr Thr
               1115                1120                1125
   aaa tat tcc gga ggg gaa agt aac cac gtg att cct acg gaa gca agc   4002
   Lys Tyr Ser Gly Gly Glu Ser Asn His Val Ile Pro Thr Glu Ala Ser
           1130                1135                1140
   atg act tct gct cca aca tct gta tcc ctg ggg aaa tct cct gta gac   4050
   Met Thr Ser Ala Pro Thr Ser Val Ser Leu Gly Lys Ser Pro Val Asp
       1145                1150                1155
   aat agt ggt cac ctg agc atg cct ggg acc atc caa act ggg aaa gat   4098
   Asn Ser Gly His Leu Ser Met Pro Gly Thr Ile Gln Thr Gly Lys Asp
   1160                1165                1170                1175
   tca gtg gaa aca aca cca ctt ccc agc ccc ctc agc aca ccc tca ata   4146
   Ser Val Glu Thr Thr Pro Leu Pro Ser Pro Leu Ser Thr Pro Ser Ile
                   1180                1185                1190
   cca aca agc aca aaa ttc tca aag agg aaa act ccc ttg cac cag atc   4194
   Pro Thr Ser Thr Lys Phe Ser Lys Arg Lys Thr Pro Leu His Gln Ile
               1195                1200                1205
   ttt gta aat aac cag aag aag gag ggg atg tta aag aat cca tat caa   4242
   Phe Val Asn Asn Gln Lys Lys Glu Gly Met Leu Lys Asn Pro Tyr Gln
           1210                1215                1220
   ttc ggt tta caa aag aac cca gcc gca aag ctt ccc aaa ata gct cct    4290
   Phe Gly Leu Gln Lys Asn Pro Ala Ala Lys Leu Pro Lys Ile Ala Pro
       1225                1230                1235
   ctt tta ccc aca ggt cag agt tcc ccc tca gat tct aca act ctc ttg    4338
   Leu Leu Pro Thr Gly Gln Ser Ser Pro Ser Asp Ser Thr Thr Leu Leu
   1240                1245                1250                1255
   aca agt ccg cca cca gct ctg tct aca aca atg gct gcc act cag aac    4386
   Thr Ser Pro Pro Pro Ala Leu Ser Thr Thr Met Ala Ala Thr Gln Asn
                   1260                1265                1270
   aag ggc act gaa gta gta tca ggt gcc aga agt ctc tca gca ggg aag    4434
   Lys Gly Thr Glu Val Val Ser Gly Ala Arg Ser Leu Ser Ala Gly Lys
               1275                1280                1285
   aag cag ccc ttc acc aac tcc tct cca gtg ctt cct agc acc ata agc    4482
   Lys Gln Pro Phe Thr Asn Ser Ser Pro Val Leu Pro Ser Thr Ile Ser
           1290                1295                1300
   aag aga tct aat aca tta aac ttc ttg tca acg gaa acc ccc aca gtg    4530
   Lys Arg Ser Asn Thr Leu Asn Phe Leu Ser Thr Glu Thr Pro Thr Val
       1305                1310                1315
   aca agt cct act gct act gca tct gtc att atg tct gaa acc caa cga    4578
   Thr Ser Pro Thr Ala Thr Ala Ser Val Ile Met Ser Glu Thr Gln Arg
   1320                1325                1330                1335
   aca aga tcc aaa gaa gca aaa gac caa ata aag ggg cct cgg aag aac    4626
   Thr Arg Ser Lys Glu Ala Lys Asp Gln Ile Lys Gly Pro Arg Lys Asn
                   1340                1345                1350
   aga aac aac gca aac acc acc ccc agg cag gtt tct ggc tat agt gca    4674
   Arg Asn Asn Ala Asn Thr Thr Pro Arg Gln Val Ser Gly Tyr Ser Ala
               1355                1360                1365
   tac tca gct cta aca aca gct gat acc ccc ttg gct ttc agt cat tcc    4722
   Tyr Ser Ala Leu Thr Thr Ala Asp Thr Pro Leu Ala Phe Ser His Ser
           1370                1375                1380
   cca cga caa gat gat ggt gga aat gta agt gca gtt gct tat cac tca    4770
   Pro Arg Gln Asp Asp Gly Gly Asn Val Ser Ala Val Ala Tyr His Ser
       1385                1390                1395
   aca acc tct ctt ctg gcc ata act gaa ctg ttt gag aag tac acc cag    4818
   Thr Thr Ser Leu Leu Ala Ile Thr Glu Leu Phe Glu Lys Tyr Thr Gln
   1400                1405                1410                1415
   act ttg gga aat aca aca gct ttg gaa aca acg ttg ttg agc aaa tca    4866
   Thr Leu Gly Asn Thr Thr Ala Leu Glu Thr Thr Leu Leu Ser Lys Ser
                   1420                1425                1430
   cag gag agt acc aca gtg aaa aga gcc tca gac aca cca cca cca ctc    4914
   Gln Glu Ser Thr Thr Val Lys Arg Ala Ser Asp Thr Pro Pro Pro Leu
               1435                1440                1445
   ctc agc agt ggg gcg ccc cca gtg ccc act cct tcc cca cct cct ttt    4962
   Leu Ser Ser Gly Ala Pro Pro Val Pro Thr Pro Ser Pro Pro Pro Phe
           1450                1455                1460
   act aag ggt gtg gtt aca gac agc aaa gtc aca tca gct ttc cag atg    5010
   Thr Lys Gly Val Val Thr Asp Ser Lys Val Thr Ser Ala Phe Gln Met
       1465                1470                1475
   acg tca aat aga gtg gtc acc ata tat gaa tct tca agg cac aat aca    5058
   Thr Ser Asn Arg Val Val Thr Ile Tyr Glu Ser Ser Arg His Asn Thr
   1480                1485                1490                1495
   gat ctg cag caa ccc tca gca gag gct agc ccc aat cct gag atc ata    5106
   Asp Leu Gln Gln Pro Ser Ala Glu Ala Ser Pro Asn Pro Glu Ile Ile
                   1500                1505                1510
   act gga acc act gac tct ccc tct aat ctg ttt cca tcc act tct gtg    5154
   Thr Gly Thr Thr Asp Ser Pro Ser Asn Leu Phe Pro Ser Thr Ser Val
               1515                1520                1525
   cca gca cta agg gta gat aaa cca cag aat tct aaa tgg aag ccc tct    5202
   Pro Ala Leu Arg Val Asp Lys Pro Gln Asn Ser Lys Trp Lys Pro Ser
           1530                1535                1540
   ccc tgg cca gaa cac aaa tat cag ctc aag tca tac tcc gaa acc att    5250
   Pro Trp Pro Glu His Lys Tyr Gln Leu Lys Ser Tyr Ser Glu Thr Ile
       1545                1550                1555
   gag aag ggc aaa agg cca gca gta agc atg tcc ccc cac ctc agc ctt    5298
   Glu Lys Gly Lys Arg Pro Ala Val Ser Met Ser Pro His Leu Ser Leu
   1560                1565                1570                1575
   cca gag gcc agc act cat gcc tca cac tgg aat aca cag aag cat gca    5346
   Pro Glu Ala Ser Thr His Ala Ser His Trp Asn Thr Gln Lys His Ala
                   1580                1585                1590
   gaa aag agt gtt ttt gat aag aaa cct ggt caa aac cca act tcc aaa    5394
   Glu Lys Ser Val Phe Asp Lys Lys Pro Gly Gln Asn Pro Thr Ser Lys
               1595                1600                1605
   cat ctg cct tac gtc tct cta cct aag act cta ttg aaa aag cca aga    5442
   His Leu Pro Tyr Val Ser Leu Pro Lys Thr Leu Leu Lys Lys Pro Arg
           1610                1615                1620
   ata att gga gga aag gct gca agc ttt aca gtt cca gct aat tca gac    5490
   Ile Ile Gly Gly Lys Ala Ala Ser Phe Thr Val Pro Ala Asn Ser Asp
       1625                1630                1635
   gtt ttt ctt cct tgt gag gct gtt gga gac cca ctg ccc atc atc cac    5538
   Val Phe Leu Pro Cys Glu Ala Val Gly Asp Pro Leu Pro Ile Ile His
   1640                1645                1650                1655
   tgg acc aga gtt tca tca gga ntt gaa ata tcc caa ggg aca cag aaa    5586
   Trp Thr Arg Val Ser Ser Gly Xaa Glu Ile Ser Gln Gly Thr Gln Lys
                   1660                1665                1670
   agc cgg ttc cac gtg ctt ccc aat ggc acc ttg tcc atc cag agg gtc    5634
   Ser Arg Phe His Val Leu Pro Asn Gly Thr Leu Ser Ile Gln Arg Val
               1675                1680                1685
   agt att cag gac cgt gga cag tac ctg tgc tct gca ttt aat cca ctg    5682
   Ser Ile Gln Asp Arg Gly Gln Tyr Leu Cys Ser Ala Phe Asn Pro Leu
           1690                1695                1700
   ggc gta gac cat ttt cat gtc tct ttg tct gtg gtt ttt tac ccg gca    5730
   Gly Val Asp His Phe His Val Ser Leu Ser Val Val Phe Tyr Pro Ala
       1705                1710                1715
   agg att ttg gac aga cat gtc aag gag atc aca gtt cac ttt gga agt    5778
   Arg Ile Leu Asp Arg His Val Lys Glu Ile Thr Val His Phe Gly Ser
   1720                1725                1730                1735
   act gtg gaa cta aag tgc aga gtg gag ggt atg ccg agg cct acg gtt    5826
   Thr Val Glu Leu Lys Cys Arg Val Glu Gly Met Pro Arg Pro Thr Val
                   1740                1745                1750
   tcc tgg ata ctt gca aac caa acg gtg gtc tca gaa acg gcc aag gga    5874
   Ser Trp Ile Leu Ala Asn Gln Thr Val Val Ser Glu Thr Ala Lys Gly
               1755                1760                1765
   agc aga aag gtc tgg gta aca cct gat gga aca ttg atc atc tat aat    5922
   Ser Arg Lys Val Trp Val Thr Pro Asp Gly Thr Leu Ile Ile Tyr Asn
           1770                1775                1780
   ctg agt ctt tat gat cgt ggt ttt tac aag tgt gtg gcc agc aac cca    5970
   Leu Ser Leu Tyr Asp Arg Gly Phe Tyr Lys Cys Val Ala Ser Asn Pro
       1785                1790                1795
   tct ggc cag gat tca ctg ttg gtt aag ata caa gtc atc aca gct ccc    6018
   Ser Gly Gln Asp Ser Leu Leu Val Lys Ile Gln Val Ile Thr Ala Pro
   1800                1805                1810                1815
   cct gtc att ata gag caa aag agg caa gcc atc gtt ggg gtt tta ggt    6066
   Pro Val Ile Ile Glu Gln Lys Arg Gln Ala Ile Val Gly Val Leu Gly
                   1820                1825                1830
   gga agt ttg aaa ctg ccc tgc act gca aaa gga act ccc cag cct agt    6114
   Gly Ser Leu Lys Leu Pro Cys Thr Ala Lys Gly Thr Pro Gln Pro Ser
               1835                1840                1845
   gtt cac tgg gtc ctt tat gat ggg act gaa cta aaa cca ttg cag ttg    6162
   Val His Trp Val Leu Tyr Asp Gly Thr Glu Leu Lys Pro Leu Gln Leu
           1850                1855                1860
   act cat tcc aga ttt ttc ttg tat cca aat gga act ctg tat ata aga    6210
   Thr His Ser Arg Phe Phe Leu Tyr Pro Asn Gly Thr Leu Tyr Ile Arg
       1865                1870                1875
   agc atc gct cct tca gtg agg ggc act tat gag tgc att gcc acc agc    6258
   Ser Ile Ala Pro Ser Val Arg Gly Thr Tyr Glu Cys Ile Ala Thr Ser
   1880                1885                1890                1895
   tcc tca ggc tca gag aga agg gta gtg att ctt act gtg gaa gag gga    6306
   Ser Ser Gly Ser Glu Arg Arg Val Val Ile Leu Thr Val Glu Glu Gly
                   1900                1905                1910
   gag aca atc ccc agg ata gaa act gcc tct cag aaa tgg act gag gtg    6354
   Glu Thr Ile Pro Arg Ile Glu Thr Ala Ser Gln Lys Trp Thr Glu Val
               1915                1920                1925
   aat ttg ggt gag aaa tta cta ctg aac tgc tca gct act ggg gat cca    6402
   Asn Leu Gly Glu Lys Leu Leu Leu Asn Cys Ser Ala Thr Gly Asp Pro
           1930                1935                1940
   aag cct aga ata atc tgg agg ctg cca tcc aag gct gtc atc gac cag    6450
   Lys Pro Arg Ile Ile Trp Arg Leu Pro Ser Lys Ala Val Ile Asp Gln
       1945                1950                1955
   tgg cac aga atg ggc agc cga atc cac gtc tac cca aat gga tcc ttg    6498
   Trp His Arg Met Gly Ser Arg Ile His Val Tyr Pro Asn Gly Ser Leu
   1960                1965                1970                1975
   gtg gtt ggg tca gtg acg gaa aaa gac gct ggt gac tac tta tgt gtg    6546
   Val Val Gly Ser Val Thr Glu Lys Asp Ala Gly Asp Tyr Leu Cys Val
                   1980                1985                1990
   gca aga aac aaa atg gga gat gac cta gtc ctg atg cat gtc cgc ctg    6594
   Ala Arg Asn Lys Met Gly Asp Asp Leu Val Leu Met His Val Arg Leu
               1995                2000                2005
   aga ttg aca cct gcc aaa att gaa cag aag cag tat ttt aag aag caa    6642
   Arg Leu Thr Pro Ala Lys Ile Glu Gln Lys Gln Tyr Phe Lys Lys Gln
           2010                2015                2020
   gtg ctc cat ggg aaa gat ttc caa gtt gac tgc aag gcc tct ggc tcc    6690
   Val Leu His Gly Lys Asp Phe Gln Val Asp Cys Lys Ala Ser Gly Ser
       2025                2030                2035
   cct gtg cct gag gta tcc tgg agt ttg cct gat ggg aca gtg ctc aac    6738
   Pro Val Pro Glu Val Ser Trp Ser Leu Pro Asp Gly Thr Val Leu Asn
   2040                2045                2050                2055
   aat gta gcc caa gct gat gac agt ggc tat agg acc aag agg tac acc    6786
   Asn Val Ala Gln Ala Asp Asp Ser Gly Tyr Arg Thr Lys Arg Tyr Thr
                   2060                2065                2070
   ctt ttc cac aat gga acc ttg tat ttc aac aac gtt ggg atg gca gag    6834
   Leu Phe His Asn Gly Thr Leu Tyr Phe Asn Asn Val Gly Met Ala Glu
               2075                2080                2085
   gaa gga gat tat atc tgc tct gcc cag aac acc tta ggg aaa gat gag    6882
   Glu Gly Asp Tyr Ile Cys Ser Ala Gln Asn Thr Leu Gly Lys Asp Glu
           2090                2095                2100
   atg aaa gtc cac cta aca gtt cta aca gcc atc cca cgg ata agg caa    6930
   Met Lys Val His Leu Thr Val Leu Thr Ala Ile Pro Arg Ile Arg Gln
       2105                2110                2115
   agc tac aag acc acc atg agg ctc agg gct gga gaa aca gct gtc ctt    6978
   Ser Tyr Lys Thr Thr Met Arg Leu Arg Ala Gly Glu Thr Ala Val Leu
   2120                2125                2130                2135
   gac tgc gag gtc act ggg gaa ccg aag ccc aat gta ttt tgg ttg ctg    7026
   Asp Cys Glu Val Thr Gly Glu Pro Lys Pro Asn Val Phe Trp Leu Leu
                   2140                2145                2150
   cct tcc aac aat gtc att tca ttc tcc aat gac agg ttc aca ttt cat    7074
   Pro Ser Asn Asn Val Ile Ser Phe Ser Asn Asp Arg Phe Thr Phe His
               2155                2160                2165
   gcc aat aga act ttg tcc atc cat aaa gtg aaa cca ctt gac tct ggg    7122
   Ala Asn Arg Thr Leu Ser Ile His Lys Val Lys Pro Leu Asp Ser Gly
           2170                2175                2180
   gac tat gtg tgc gta gct cag aat cct agt ggg gat gac act aag aca    7170
   Asp Tyr Val Cys Val Ala Gln Asn Pro Ser Gly Asp Asp Thr Lys Thr
       2185                2190                2195
   tac aaa ctg gac att gtc tct aaa cct cca tta atc aat ggc ctg tat    7218
   Tyr Lys Leu Asp Ile Val Ser Lys Pro Pro Leu Ile Asn Gly Leu Tyr
   2200                2205                2210                2215
   gca aac aag act gtt att aaa gcc aca gcc att cgg cac tcc aaa aaa    7266
   Ala Asn Lys Thr Val Ile Lys Ala Thr Ala Ile Arg His Ser Lys Lys
                   2220                2225                2230
   tac ttt gac tgc aga gca gat ggg atc cca tct tcc cag gtc acg tgg    7314
   Tyr Phe Asp Cys Arg Ala Asp Gly Ile Pro Ser Ser Gln Val Thr Trp
               2235                2240                2245
   att atg cca ggc aat att ttc ctc cca gct cca tac ttt gga agc aga    7362
   Ile Met Pro Gly Asn Ile Phe Leu Pro Ala Pro Tyr Phe Gly Ser Arg
           2250                2255                2260
   gtc acg gtc cat cca aat gga acc ttg gag atg agg aac atc cgg ctt    7410
   Val Thr Val His Pro Asn Gly Thr Leu Glu Met Arg Asn Ile Arg Leu
       2265                2270                2275
   tct gac tct gcg gac ttc acc tgt gtg gtt cgg agc gag gga gga gag    7458
   Ser Asp Ser Ala Asp Phe Thr Cys Val Val Arg Ser Glu Gly Gly Glu
   2280                2285                2290                2295
   agt gtg ttg gta gtg cag tta gaa gtc cta gaa atg ctg aga aga cca    7506
   Ser Val Leu Val Val Gln Leu Glu Val Leu Glu Met Leu Arg Arg Pro
                   2300                2305                2310
   aca ttc aga aac cca ttc aac gaa aaa gtc atc gcc caa gct ggc aag    7554
   Thr Phe Arg Asn Pro Phe Asn Glu Lys Val Ile Ala Gln Ala Gly Lys
               2315                2320                2325
   ccc gta gca ctg aac tgc tct gtg gat ggg aac ccc cca cct gaa att    7602
   Pro Val Ala Leu Asn Cys Ser Val Asp Gly Asn Pro Pro Pro Glu Ile
           2330                2335                2340
   acc tgg atc tta cct gac ggc aca cag ttt gct aac aga cca cac aat    7650
   Thr Trp Ile Leu Pro Asp Gly Thr Gln Phe Ala Asn Arg Pro His Asn
       2345                2350                2355
   tcc ccg tat ctg atg gca ggc aat ggc tct ctc atc ctt tac aaa gca    7698
   Ser Pro Tyr Leu Met Ala Gly Asn Gly Ser Leu Ile Leu Tyr Lys Ala
   2360                2365                2370                2375
   act cgg aac aag tca ggg aag tat cgc tgt gca gcc agg aat aag gtt    7746
   Thr Arg Asn Lys Ser Gly Lys Tyr Arg Cys Ala Ala Arg Asn Lys Val
                   2380                2385                2390
   ggc tac atc gag aaa ctc atc ctg tta gag att ggg cag aag cca gtc    7794
   Gly Tyr Ile Glu Lys Leu Ile Leu Leu Glu Ile Gly Gln Lys Pro Val
               2395                2400                2405
   att ctg aca tac gaa cca ggg atg gtg aag agc gtc agt ggg gaa ccg    7842
   Ile Leu Thr Tyr Glu Pro Gly Met Val Lys Ser Val Ser Gly Glu Pro
           2410                2415                2420
   tta tca ctg cat tgt gtg tct gat ggg atc ccc aag cca aat gtc aag    7890
   Leu Ser Leu His Cys Val Ser Asp Gly Ile Pro Lys Pro Asn Val Lys
   2425 2430 2435
   tgg act aca ccg ggt ggc cat gta atc gac agg cct caa gtg gat gga 7938
   Trp Thr Thr Pro Gly Gly His Val Ile Asp Arg Pro Gln Val Asp Gly
   2440 2445 2450 2455
   aaa tac ata ctg cat gaa aat ggc acg ctg gtc atc aaa gca aca aca 7986
   Lys Tyr Ile Leu His Glu Asn Gly Thr Leu Val Ile Lys Ala Thr Thr
   2460 2465 2470
   gct cac gac caa gga aat tat atc tgt agg gct caa aac agt gtt ggc 8034
   Ala His Asp Gln Gly Asn Tyr Ile Cys Arg Ala Gln Asn Ser Val Gly
   2475 2480 2485
   cag gca gtt att agc gtg tca gtg atg gtt gtg gcc tac cct ccc cga 8082
   Gln Ala Val Ile Ser Val Ser Val Met Val Val Ala Tyr Pro Pro Arg
   2490 2495 2500
   atc ata aac tac cta ccc agg aac atg ctc agg agg aca ggg gaa gcc 8130
   Ile Ile Asn Tyr Leu Pro Arg Asn Met Leu Arg Arg Thr Gly Glu Ala
   2505 2510 2515
   atg cag ctc cac tgt gtg gcc ttg gga atc ccc aag cca aaa gtc acc 8178
   Met Gln Leu His Cys Val Ala Leu Gly Ile Pro Lys Pro Lys Val Thr
   2520 2525 2530 2535
   tgg gag acg cca aga cac tcc ctg ctg tca aaa gca aca gca aga aaa 8226
   Trp Glu Thr Pro Arg His Ser Leu Leu Ser Lys Ala Thr Ala Arg Lys
   2540 2545 2550
   ccc cat aga agt gag atg ctt cac cca caa ggt acg ctg gtc att cag 8274
   Pro His Arg Ser Glu Met Leu His Pro Gln Gly Thr Leu Val Ile Gln
   2555 2560 2565
   aat ctc caa acc tcg gat tcc gga gtc tat aag tgc aga gct cag aac 8322
   Asn Leu Gln Thr Ser Asp Ser Gly Val Tyr Lys Cys Arg Ala Gln Asn
   2570 2575 2580
   cta ctt ggg act gat tac gca aca act tac atc cag gta ctc tga 8367
   Leu Leu Gly Thr Asp Tyr Ala Thr Thr Tyr Ile Gln Val Leu
   2585 2590 2595
   caggaagggg gagactaaaa ttcaacagaa gtccacatcc acagggttta ttttttggaa 8427
   gaagtttaat caaaggcagc cataggcatg taaatgagtc tgaatacatt tacagtatta 8487
   aatttacaat ggacatgcga tgagacttgt aaatgaaagc attgtgaact gaaaccgagt 8547
   ctctgtggat ctcaaagcaa actcttaact taaggcactt tgattttgcc aacaaataat 8607
   aacaaacatt aagagaaaaa aatgatccac tacgaaataa caaacggcta atgcacctga 8667
   attctcagta aaaagacctt tctctcgcta acagttgcca gctgcctcgt gtctgtttcc 8727
   taccaatgtc acaaacatcg cacacagggt gaatggagtc aacgggaaag attaagtttg 8787
   cggtctgtgt aaatctcaat gtacaaatat tctgtcnctg gtttataaac attttgataa 8847
   aaccgaaaaa aaaaaaaaaa aaaaaaaaaa aaaaa 8882
<210> 33
   <211> 2597
   <212> PRT
   <213> Rattus sp.
<400> 33
   Met Gln Val Arg Gly Arg Glu Val Ser Gly Leu Leu Ile Ser Leu Thr
   1 5 10 15
   Ala Val Cys Leu Val Val Thr Pro Gly Ser Arg Ala Cys Pro Arg Arg
   20 25 30
   Cys Ala Cys Tyr Val Pro Thr Glu Val His Cys Thr Phe Arg Tyr Leu
   35 40 45
   Thr Ser Ile Pro Asp Gly Ile Pro Ala Asn Val Glu Arg Ile Asn Leu
   50 55 60
   Gly Tyr Asn Ser Leu Thr Arg Leu Thr Glu Asn Asp Phe Asp Gly Leu
   65 70 75 80
   Ser Lys Leu Glu Leu Leu Met Leu His Ser Asn Gly Ile His Arg Val
   85 90 95
   Ser Asp Lys Thr Phe Ser Gly Leu Gln Ser Leu Gln Val Leu Lys Met
   100 105 110
   Ser Tyr Asn Lys Val Gln Ile Ile Arg Lys Asp Thr Phe Tyr Gly Leu
   115 120 125
   Gly Ser Leu Val Arg Leu His Leu Asp His Asn Asn Ile Glu Phe Ile
   130 135 140
   Asn Pro Glu Ala Phe Tyr Gly Leu Thr Ser Leu Arg Leu Val His Leu
   145 150 155 160
   Glu Gly Asn Arg Leu Thr Lys Leu His Pro Asp Thr Phe Val Ser Leu
   165 170 175
   Ser Tyr Leu Gln Ile Phe Lys Thr Ser Phe Ile Lys Tyr Leu Phe Leu
   180 185 190
   Ser Asp Asn Phe Leu Thr Ser Leu Pro Lys Glu Met Val Ser Tyr Met
   195 200 205
   Pro Asn Leu Glu Ser Leu Tyr Leu His Gly Asn Pro Trp Thr Cys Asp
   210 215 220
   Cys His Leu Lys Trp Leu Ser Glu Trp Met Gln Gly Asn Pro Asp Ile
   225 230 235 240
   Ile Lys Cys Lys Lys Asp Arg Ser Ser Ser Ser Pro Gln Gln Cys Pro
   245 250 255
   Leu Cys Met Asn Pro Arg Ile Ser Lys Gly Arg Pro Phe Ala Met Val
   260 265 270
   Pro Ser Gly Ala Phe Leu Cys Thr Lys Pro Thr Ile Asp Pro Ser Leu
   275 280 285
   Lys Ser Lys Ser Leu Val Thr Gln Glu Asp Asn Gly Ser Ala Ser Thr
   290 295 300
   Ser Pro Gln Asp Phe Ile Glu Pro Phe Gly Ser Leu Ser Leu Asn Met
   305 310 315 320
   Thr Xaa Xaa Ser Gly Asn Lys Ala Asp Met Val Cys Ser Ile Gln Lys
   325 330 335
   Pro Ser Arg Thr Ser Pro Thr Ala Phe Thr Glu Glu Asn Asp Tyr Ile
   340 345 350
   Met Leu Asn Ala Ser Phe Ser Thr Asn Leu Val Cys Ser Val Asp Tyr
   355 360 365
   Asn His Ile Gln Pro Val Trp Gln Leu Leu Ala Leu Tyr Ser Asp Ser
   370 375 380
   Pro Leu Ile Leu Glu Arg Lys Pro Gln Leu Thr Glu Thr Pro Ser Leu
   385 390 395 400
   Ser Ser Arg Tyr Lys Gln Val Ala Leu Arg Pro Glu Asp Ile Phe Thr
   405 410 415
   Ser Ile Glu Ala Asp Val Arg Ala Asp Pro Phe Trp Phe Gln Gln Glu
   420 425 430
   Lys Ile Val Leu Gln Leu Asn Arg Thr Ala Thr Thr Leu Ser Thr Leu
           435                 440                 445
   Gln Ile Gln Phe Ser Thr Asp Ala Gln Ile Ala Leu Pro Arg Ala Glu
       450                 455                 460
   Met Arg Ala Glu Arg Leu Lys Trp Thr Met Ile Leu Met Met Asn Asn
   465                 470                 475                 480
   Pro Lys Leu Glu Arg Thr Val Leu Val Gly Gly Thr Ile Ala Leu Ser
                   485                 490                 495
   Cys Pro Gly Lys Gly Asp Pro Ser Pro His Leu Glu Trp Leu Leu Ala
               500                 505                 510
   Asp Gly Ser Lys Val Arg Ala Pro Tyr Val Ser Glu Asp Gly Arg Ile
           515                 520                 525
   Leu Ile Asp Lys Asn Gly Lys Leu Glu Leu Gln Met Ala Asp Ser Phe
       530                 535                 540
   Asp Ala Gly Leu Tyr His Cys Ile Ser Thr Asn Asp Ala Asp Ala Asp
   545                 550                 555                 560
   Val Leu Thr Tyr Arg Ile Thr Val Val Glu Pro Tyr Gly Glu Ser Thr
                   565                 570                 575
   His Asp Ser Gly Val Gln His Thr Val Val Thr Gly Glu Thr Leu Asp
               580                 585                 590
   Leu Pro Cys Leu Ser Thr Gly Val Pro Asp Ala Ser Ile Ser Trp Ile
           595                 600                 605
   Leu Pro Gly Asn Thr Val Phe Ser Gln Pro Ser Arg Asp Arg Gln Ile
       610                 615                 620
   Leu Asn Asn Gly Thr Leu Arg Ile Leu Gln Val Thr Pro Lys Asp Gln
   625                 630                 635                 640
   Gly His Tyr Gln Cys Val Ala Ala Asn Pro Ser Gly Ala Asp Phe Ser
                   645                 650                 655
   Ser Phe Lys Val Ser Val Gln Lys Lys Gly Gln Arg Met Val Glu His
               660                 665                 670
   Asp Arg Glu Ala Gly Gly Ser Gly Leu Gly Glu Pro Asn Ser Ser Val
           675                 680                 685
   Ser Leu Lys Gln Pro Ala Ser Leu Lys Leu Ser Ala Ser Ala Leu Thr
       690                 695                 700
   Gly Ser Glu Ala Gly Lys Gln Val Ser Gly Val His Arg Lys Asn Lys
   705                 710                 715                 720
   His Arg Asp Leu Ile His Arg Arg Arg Gly Asp Ser Thr Leu Arg Arg
                   725                 730                 735
   Phe Arg Glu His Arg Arg Gln Leu Pro Leu Ser Ala Arg Arg Ile Asp
               740                 745                 750
   Pro Gln Arg Trp Ala Ala Leu Leu Glu Lys Ala Lys Lys Asn Ser Val
           755                 760                 765
   Pro Lys Lys Gln Glu Asn Thr Thr Val Lys Pro Val Pro Leu Ala Val
       770                 775                 780
   Pro Leu Val Glu Leu Thr Asp Glu Glu Lys Asp Ala Ser Gly Met Ile
   785                 790                 795                 800
   Pro Pro Asp Glu Glu Phe Met Val Leu Lys Thr Lys Ala Ser Gly Val
                   805                 810                 815
   Pro Gly Arg Ser Pro Thr Ala Asp Ser Gly Pro Val Asn His Gly Phe
               820                 825                 830
   Met Thr Ser Ile Ala Ser Gly Thr Glu Val Ser Thr Val Asn Pro Gln
           835                 840                 845
   Thr Leu Gln Ser Glu His Leu Pro Asp Phe Lys Leu Phe Ser Val Thr
       850                 855                 860
   Asn Gly Thr Ala Val Thr Lys Ser Met Asn Pro Ser Ile Ala Ser Lys
   865                 870                 875                 880
   Ile Glu Asp Thr Thr Asn Gln Asn Pro Ile Ile Ile Phe Pro Ser Val
                   885                 890                 895
   Ala Glu Ile Arg Asp Ser Ala Gln Ala Gly Arg Ala Ser Ser Gln Ser
               900                 905                 910
   Ala His Pro Val Thr Gly Gly Asn Met Ala Thr Tyr Gly His Thr Asn
           915                 920                 925
   Thr Tyr Ser Ser Phe Thr Ser Lys Ala Ser Thr Val Leu Gln Pro Ile
       930                 935                 940
   Asn Pro Thr Glu Ser Tyr Gly Pro Gln Ile Pro Ile Thr Gly Val Ser
   945                 950                 955                 960
   Arg Pro Ser Ser Ser Asp Ile Ser Ser His Thr Thr Ala Asp Pro Ser
                   965                 970                 975
   Phe Ser Ser His Pro Ser Gly Ser His Thr Thr Ala Ser Ser Leu Phe
               980                 985                 990
   His Ile Pro Arg Asn Asn Asn Thr Gly Asn Phe Pro Leu Ser Arg His
           995                 1000                1005
   Leu Gly Arg Glu Arg Thr Ile Trp Ser Arg Gly Arg Val Lys Asn Pro
       1010                1015                1020
   His Arg Thr Pro Val Leu Arg Arg His Arg His Arg Thr Val Arg Pro
   1025                1030                1035                1040
   Ala Ile Lys Gly Pro Ala Asn Lys Asn Val Ser Gln Val Pro Ala Thr
                   1045                1050                1055
   Glu Tyr Pro Gly Met Cys His Thr Cys Pro Ser Ala Glu Gly Leu Thr
               1060                1065                1070
   Val Ala Thr Ala Ala Leu Ser Val Pro Ser Ser Ser His Ser Ala Leu
           1075                1080                1085
   Pro Lys Thr Asn Asn Val Gly Val Ile Ala Glu Glu Ser Thr Thr Val
       1090                1095                1100
   Val Lys Lys Pro Leu Leu Leu Phe Lys Asp Lys Gln Asn Val Asp Ile
   1105                1110                1115                1120
   Glu Ile Ile Thr Thr Thr Thr Lys Tyr Ser Gly Gly Glu Ser Asn His
                   1125                1130                1135
   Val Ile Pro Thr Glu Ala Ser Met Thr Ser Ala Pro Thr Ser Val Ser
               1140                1145                1150
   Leu Gly Lys Ser Pro Val Asp Asn Ser Gly His Leu Ser Met Pro Gly
           1155                1160                1165
   Thr Ile Gln Thr Gly Lys Asp Ser Val Glu Thr Thr Pro Leu Pro Ser
       1170                1175                1180
   Pro Leu Ser Thr Pro Ser Ile Pro Thr Ser Thr Lys Phe Ser Lys Arg
   1185                1190                1195                1200
   Lys Thr Pro Leu His Gln Ile Phe Val Asn Asn Gln Lys Lys Glu Gly
                   1205                1210                1215
   Met Leu Lys Asn Pro Tyr Gln Phe Gly Leu Gln Lys Asn Pro Ala Ala
               1220                1225                1230
   Lys Leu Pro Lys Ile Ala Pro Leu Leu Pro Thr Gly Gln Ser Ser Pro
           1235                1240                1245
   Ser Asp Ser Thr Thr Leu Leu Thr Ser Pro Pro Pro Ala Leu Ser Thr
       1250                1255                1260
   Thr Met Ala Ala Thr Gln Asn Lys Gly Thr Glu Val Val Ser Gly Ala
   1265                1270                1275                1280
   Arg Ser Leu Ser Ala Gly Lys Lys Gln Pro Phe Thr Asn Ser Ser Pro
                   1285                1290                1295
   Val Leu Pro Ser Thr Ile Ser Lys Arg Ser Asn Thr Leu Asn Phe Leu
               1300                1305                1310
   Ser Thr Glu Thr Pro Thr Val Thr Ser Pro Thr Ala Thr Ala Ser Val
           1315                1320                1325
   Ile Met Ser Glu Thr Gln Arg Thr Arg Ser Lys Glu Ala Lys Asp Gln
       1330                1335                1340
   Ile Lys Gly Pro Arg Lys Asn Arg Asn Asn Ala Asn Thr Thr Pro Arg
   1345                1350                1355                1360
   Gln Val Ser Gly Tyr Ser Ala Tyr Ser Ala Leu Thr Thr Ala Asp Thr
                   1365                1370                1375
   Pro Leu Ala Phe Ser His Ser Pro Arg Gln Asp Asp Gly Gly Asn Val
               1380                1385                1390
   Ser Ala Val Ala Tyr His Ser Thr Thr Ser Leu Leu Ala Ile Thr Glu
           1395                1400                1405
   Leu Phe Glu Lys Tyr Thr Gln Thr Leu Gly Asn Thr Thr Ala Leu Glu
   1410 1415 1420
   Thr Thr Leu Leu Ser Lys Ser Gln Glu Ser Thr Thr Val Lys Arg Ala
   1425 1430 1435 1440
   Ser Asp Thr Pro Pro Pro Leu Leu Ser Ser Gly Ala Pro Pro Val Pro
   1445 1450 1455
   Thr Pro Ser Pro Pro Pro Phe Thr Lys Gly Val Val Thr Asp Ser Lys
   1460 1465 1470
   Val Thr Ser Ala Phe Gln Met Thr Ser Asn Arg Val Val Thr Ile Tyr
   1475 1480 1485
   Glu Ser Ser Arg His Asn Thr Asp Leu Gln Gln Pro Ser Ala Glu Ala
   1490 1495 1500
   Ser Pro Asn Pro Glu Ile Ile Thr Gly Thr Thr Asp Ser Pro Ser Asn
   1505 1510 1515 1520
   Leu Phe Pro Ser Thr Ser Val Pro Ala Leu Arg Val Asp Lys Pro Gln
   1525 1530 1535
   Asn Ser Lys Trp Lys Pro Ser Pro Trp Pro Glu His Lys Tyr Gln Leu
   1540 1545 1550
   Lys Ser Tyr Ser Glu Thr Ile Glu Lys Gly Lys Arg Pro Ala Val Ser
   1555 1560 1565
   Met Ser Pro His Leu Ser Leu Pro Glu Ala Ser Thr His Ala Ser His
   1570 1575 1580
   Trp Asn Thr Gln Lys His Ala Glu Lys Ser Val Phe Asp Lys Lys Pro
   1585 1590 1595 1600
   Gly Gln Asn Pro Thr Ser Lys His Leu Pro Tyr Val Ser Leu Pro Lys
   1605 1610 1615
   Thr Leu Leu Lys Lys Pro Arg Ile Ile Gly Gly Lys Ala Ala Ser Phe
   1620 1625 1630
   Thr Val Pro Ala Asn Ser Asp Val Phe Leu Pro Cys Glu Ala Val Gly
   1635 1640 1645
   Asp Pro Leu Pro Ile Ile His Trp Thr Arg Val Ser Ser Gly Xaa Glu
   1650 1655 1660
   Ile Ser Gln Gly Thr Gln Lys Ser Arg Phe His Val Leu Pro Asn Gly
   1665 1670 1675 1680
   Thr Leu Ser Ile Gln Arg Val Ser Ile Gln Asp Arg Gly Gln Tyr Leu
   1685 1690 1695
   Cys Ser Ala Phe Asn Pro Leu Gly Val Asp His Phe His Val Ser Leu
   1700 1705 1710
   Ser Val Val Phe Tyr Pro Ala Arg Ile Leu Asp Arg His Val Lys Glu
   1715 1720 1725
   Ile Thr Val His Phe Gly Ser Thr Val Glu Leu Lys Cys Arg Val Glu
   1730 1735 1740
   Gly Met Pro Arg Pro Thr Val Ser Trp Ile Leu Ala Asn Gln Thr Val
   1745 1750 1755 1760
   Val Ser Glu Thr Ala Lys Gly Ser Arg Lys Val Trp Val Thr Pro Asp
   1765 1770 1775
   Gly Thr Leu Ile Ile Tyr Asn Leu Ser Leu Tyr Asp Arg Gly Phe Tyr
   1780 1785 1790
   Lys Cys Val Ala Ser Asn Pro Ser Gly Gln Asp Ser Leu Leu Val Lys
   1795 1800 1805
   Ile Gln Val Ile Thr Ala Pro Pro Val Ile Ile Glu Gln Lys Arg Gln
   1810 1815 1820
   Ala Ile Val Gly Val Leu Gly Gly Ser Leu Lys Leu Pro Cys Thr Ala
   1825 1830 1835 1840
   Lys Gly Thr Pro Gln Pro Ser Val His Trp Val Leu Tyr Asp Gly Thr
   1845 1850 1855
   Glu Leu Lys Pro Leu Gln Leu Thr His Ser Arg Phe Phe Leu Tyr Pro
   1860 1865 1870
   Asn Gly Thr Leu Tyr Ile Arg Ser Ile Ala Pro Ser Val Arg Gly Thr
   1875 1880 1885
   Tyr Glu Cys Ile Ala Thr Ser Ser Ser Gly Ser Glu Arg Arg Val Val
   1890 1895 1900
   Ile Leu Thr Val Glu Glu Gly Glu Thr Ile Pro Arg Ile Glu Thr Ala
   1905 1910 1915 1920
   Ser Gln Lys Trp Thr Glu Val Asn Leu Gly Glu Lys Leu Leu Leu Asn
   1925 1930 1935
   Cys Ser Ala Thr Gly Asp Pro Lys Pro Arg Ile Ile Trp Arg Leu Pro
   1940 1945 1950
   Ser Lys Ala Val Ile Asp Gln Trp His Arg Met Gly Ser Arg Ile His
   1955 1960 1965
   Val Tyr Pro Asn Gly Ser Leu Val Val Gly Ser Val Thr Glu Lys Asp
   1970 1975 1980
   Ala Gly Asp Tyr Leu Cys Val Ala Arg Asn Lys Met Gly Asp Asp Leu
   1985 1990 1995 2000
   Val Leu Met His Val Arg Leu Arg Leu Thr Pro Ala Lys Ile Glu Gln
   2005 2010 2015
   Lys Gln Tyr Phe Lys Lys Gln Val Leu His Gly Lys Asp Phe Gln Val
   2020 2025 2030
   Asp Cys Lys Ala Ser Gly Ser Pro Val Pro Glu Val Ser Trp Ser Leu
   2035 2040 2045
   Pro Asp Gly Thr Val Leu Asn Asn Val Ala Gln Ala Asp Asp Ser Gly
   2050 2055 2060
   Tyr Arg Thr Lys Arg Tyr Thr Leu Phe His Asn Gly Thr Leu Tyr Phe
   2065 2070 2075 2080
   Asn Asn Val Gly Met Ala Glu Glu Gly Asp Tyr Ile Cys Ser Ala Gln
   2085 2090 2095
   Asn Thr Leu Gly Lys Asp Glu Met Lys Val His Leu Thr Val Leu Thr
   2100 2105 2110
   Ala Ile Pro Arg Ile Arg Gln Ser Tyr Lys Thr Thr Met Arg Leu Arg
   2115 2120 2125
   Ala Gly Glu Thr Ala Val Leu Asp Cys Glu Val Thr Gly Glu Pro Lys
   2130 2135 2140
   Pro Asn Val Phe Trp Leu Leu Pro Ser Asn Asn Val Ile Ser Phe Ser
   2145 2150 2155 2160
   Asn Asp Arg Phe Thr Phe His Ala Asn Arg Thr Leu Ser Ile His Lys
   2165 2170 2175
   Val Lys Pro Leu Asp Ser Gly Asp Tyr Val Cys Val Ala Gln Asn Pro
   2180 2185 2190
   Ser Gly Asp Asp Thr Lys Thr Tyr Lys Leu Asp Ile Val Ser Lys Pro
   2195 2200 2205
   Pro Leu Ile Asn Gly Leu Tyr Ala Asn Lys Thr Val Ile Lys Ala Thr
   2210 2215 2220
   Ala Ile Arg His Ser Lys Lys Tyr Phe Asp Cys Arg Ala Asp Gly Ile
   2225 2230 2235 2240
   Pro Ser Ser Gln Val Thr Trp Ile Met Pro Gly Asn Ile Phe Leu Pro
   2245 2250 2255
   Ala Pro Tyr Phe Gly Ser Arg Val Thr Val His Pro Asn Gly Thr Leu
   2260 2265 2270
   Glu Met Arg Asn Ile Arg Leu Ser Asp Ser Ala Asp Phe Thr Cys Val
   2275 2280 2285
   Val Arg Ser Glu Gly Gly Glu Ser Val Leu Val Val Gln Leu Glu Val
   2290 2295 2300
   Leu Glu Met Leu Arg Arg Pro Thr Phe Arg Asn Pro Phe Asn Glu Lys
   2305 2310 2315 2320
   Val Ile Ala Gln Ala Gly Lys Pro Val Ala Leu Asn Cys Ser Val Asp
   2325 2330 2335
   Gly Asn Pro Pro Pro Glu Ile Thr Trp Ile Leu Pro Asp Gly Thr Gln
   2340 2345 2350
   Phe Ala Asn Arg Pro His Asn Ser Pro Tyr Leu Met Ala Gly Asn Gly
   2355 2360 2365
   Ser Leu Ile Leu Tyr Lys Ala Thr Arg Asn Lys Ser Gly Lys Tyr Arg
   2370 2375 2380
   Cys Ala Ala Arg Asn Lys Val Gly Tyr Ile Glu Lys Leu Ile Leu Leu
   2385                2390                2395                2400
   Glu Ile Gly Gln Lys Pro Val Ile Leu Thr Tyr Glu Pro Gly Met Val
                   2405                2410                2415
   Lys Ser Val Ser Gly Glu Pro Leu Ser Leu His Cys Val Ser Asp Gly
               2420                2425                2430
   Ile Pro Lys Pro Asn Val Lys Trp Thr Thr Pro Gly Gly His Val Ile
           2435                2440                2445
   Asp Arg Pro Gln Val Asp Gly Lys Tyr Ile Leu His Glu Asn Gly Thr
       2450                2455                2460
   Leu Val Ile Lys Ala Thr Thr Ala His Asp Gln Gly Asn Tyr Ile Cys
   2465                2470                2475                2480
   Arg Ala Gln Asn Ser Val Gly Gln Ala Val Ile Ser Val Ser Val Met
                   2485                2490                2495
   Val Val Ala Tyr Pro Pro Arg Ile Ile Asn Tyr Leu Pro Arg Asn Met
               2500                2505                2510
   Leu Arg Arg Thr Gly Glu Ala Met Gln Leu His Cys Val Ala Leu Gly
           2515                2520                2525
   Ile Pro Lys Pro Lys Val Thr Trp Glu Thr Pro Arg His Ser Leu Leu
       2530                2535                2540
   Ser Lys Ala Thr Ala Arg Lys Pro His Arg Ser Glu Met Leu His Pro
   2545                2550                2555                2560
   Gln Gly Thr Leu Val Ile Gln Asn Leu Gln Thr Ser Asp Ser Gly Val
                   2565                2570                2575
   Tyr Lys Cys Arg Ala Gln Asn Leu Leu Gly Thr Asp Tyr Ala Thr Thr
               2580                2585                2590
   Tyr Ile Gln Val Leu
           2595
<210> 34
   <211> 7679
   <212> DNA
   <213> Rattus sp.
<220>
   <221> CDS
   <222> (1)..(7161)
   <223> coding sequences for inventive nucleic acid molecule 608
<220>
   <221> misc_feature
   <222> (4357)..(4357)
   <223> "n" can be a or g or c or t
<220>
   <221> misc_feature
   <222> (1453) .. (1453)
   <223> "Xaa" can be any amino acid
<400> 34
   cta gaa agc ctg tat ttg cat gga aac cca tgg acc tgt gac tgc cat    48
   Leu Glu Ser Leu Tyr Leu His Gly Asn Pro Trp Thr Cys Asp Cys His
    1               5                  10                  15
   tta aag tgg ttg tct gag tgg atg cag gga aac cca gat ata ata aaa    96
   Leu Lys Trp Leu Ser Glu Trp Met Gln Gly Asn Pro Asp Ile Ile Lys
               20                  25                  30
   tgc aag aaa gac aga agc tct tcc agt cct cag caa tgt ccc ctt tgc    144
   Cys Lys Lys Asp Arg Ser Ser Ser Ser Pro Gln Gln Cys Pro Leu Cys
           35                  40                  45
   atg aac ccc agg atc tct aaa ggc aga ccc ttt gct atg gta cca tct      192
   Met Asn Pro Arg Ile Ser Lys Gly Arg Pro Phe Ala Met Val Pro Ser
       50                  55                  60
   gga gct ttc cta tgt aca aag cca acc att gat cca tca ctg aag tca      240
   Gly Ala Phe Leu Cys Thr Lys Pro Thr Ile Asp Pro Ser Leu Lys Ser
   65                  70                  75                  80
   aag agc ctg gtt act cag gag gac aat gga tct gcc tcc acc tca cct      288
   Lys Ser Leu Val Thr Gln Glu Asp Asn Gly Ser Ala Ser Thr Ser Pro
                   85                  90                  95
   caa gat ttc ata gaa ccc ttt ggc tcc ttg tct ttg aac atg aca gac      336
   Gln Asp Phe Ile Glu Pro Phe Gly Ser Leu Ser Leu Asn Met Thr Asp
               100                 105                 110
   ctg tct gga aat aag gcc gac atg gtc tgt agt atc caa aag cca tca      384
   Leu Ser Gly Asn Lys Ala Asp Met Val Cys Ser Ile Gln Lys Pro Ser
           115                 120                 125
   agg aca tca cca act gca ttc act gaa gaa aat gac tac atc atg cta      432
   Arg Thr Ser Pro Thr Ala Phe Thr Glu Glu Asn Asp Tyr Ile Met Leu
       130                 135                 140
   aat gcg tca ttt tcc aca aat ctt gtg tgc agt gta gat tat aat cac      480
   Asn Ala Ser Phe Ser Thr Asn Leu Val Cys Ser Val Asp Tyr Asn His
   145                 150                 155                 160
   atc cag cca gtg tgg caa ctt ctg gct tta tac agt gac tct cct ctg      528
   Ile Gln Pro Val Trp Gln Leu Leu Ala Leu Tyr Ser Asp Ser Pro Leu
                   165                 170                 175
   ata cta gaa agg aag ccc cag ctt acc gag act cct tca ctg tct tct      576
   Ile Leu Glu Arg Lys Pro Gln Leu Thr Glu Thr Pro Ser Leu Ser Ser
               180                 185                 190
   aga tat aaa cag gtg gct ctt agg cct gaa gac att ttt acc agc ata      624
   Arg Tyr Lys Gln Val Ala Leu Arg Pro Glu Asp Ile Phe Thr Ser Ile
           195                 200                 205
   gag gct gat gtc aga gca gac cct ttt tgg ttc caa caa gaa aaa att      672
   Glu Ala Asp Val Arg Ala Asp Pro Phe Trp Phe Gln Gln Glu Lys Ile
       210                 215                 220
   gtc ttg cag ctg aac aga act gcc acc aca ctt agc aca tta cag atc      720
   Val Leu Gln Leu Asn Arg Thr Ala Thr Thr Leu Ser Thr Leu Gln Ile
   225                 230                 235                 240
   cag ttt tcc act gat gct caa atc gct tta cca agg gcg gag atg aga      768
   Gln Phe Ser Thr Asp Ala Gln Ile Ala Leu Pro Arg Ala Glu Met Arg
                   245                 250                 255
   gcg gag aga ctc aaa tgg acc atg atc ctg atg atg aac aat ccc aaa      816
   Ala Glu Arg Leu Lys Trp Thr Met Ile Leu Met Met Asn Asn Pro Lys
               260                 265                 270
   ctg gaa cgc act gtc ctg gtt ggc ggc act att gcc ctg agc tgt cca      864
   Leu Glu Arg Thr Val Leu Val Gly Gly Thr Ile Ala Leu Ser Cys Pro
               275                     280                     285
   ggc aaa ggc gac cct tca cct cac ttg gaa tgg ctt cta gct gat ggg      912
   Gly Lys Gly Asp Pro Ser Pro His Leu Glu Trp Leu Leu Ala Asp Gly
       290                     295                     300
   agt aaa gtg aga gcc cct tac gtt agc gag gat ggg cga atc cta ata      960
   Ser Lys Val Arg Ala Pro Tyr Val Ser Glu Asp Gly Arg Ile Leu Ile
   305                     310                     315                 320
   gac aaa aat ggg aag ttg gaa ctg cag atg gct gac agc ttt gat gca     1008
   Asp Lys Asn Gly Lys Leu Glu Leu Gln Met Ala Asp Ser Phe Asp Ala
                   325                     330                     335
   ggt ctt tac cac tgc ata agc acc aat gat gca gat gcg gat gtt ctc     1056
   Gly Leu Tyr His Cys Ile Ser Thr Asn Asp Ala Asp Ala Asp Val Leu
               340                     345                     350
   aca tac agg ata act gtg gta gag ccc tat gga gaa agc aca cat gac     1104
   Thr Tyr Arg Ile Thr Val Val Glu Pro Tyr Gly Glu Ser Thr His Asp
           355                     360                     365
   agt gga gtc cag cac aca gtg gtt acg ggt gag acg ctc gac ctt cca     1152
   Ser Gly Val Gln His Thr Val Val Thr Gly Glu Thr Leu Asp Leu Pro
       370                     375                     380
   tgc ctt tcc acg ggt gtt cca gat gct tct att agc tgg att ctt cca     1200
   Cys Leu Ser Thr Gly Val Pro Asp Ala Ser Ile Ser Trp Ile Leu Pro
   385                     390                     395                 400
   ggg aac act gtg ttc tct cag cca tca aga gac agg caa att ctt aac     1248
   Gly Asn Thr Val Phe Ser Gln Pro Ser Arg Asp Arg Gln Ile Leu Asn
                   405                     410                     415
   aat ggg acc tta aga ata tta cag gtt acg cca aaa gat caa ggt cat     1296
   Asn Gly Thr Leu Arg Ile Leu Gln Val Thr Pro Lys Asp Gln Gly His
               420                     425                     430
   tac caa tgt gtg gct gcc aac cca tca ggg gcc gac ttt tcc agt ttt     1344
   Tyr Gln Cys Val Ala Ala Asn Pro Ser Gly Ala Asp Phe Ser Ser Phe
           435                     440                     445
   aaa gtt tca gtt caa aag aaa ggc caa agg atg gtt gag cat cac agg     1392
   Lys Val Ser Val Gln Lys Lys Gly Gln Arg Met Val Glu His His Arg
       450                     455                     460
   gag gca ggt gga tct gga ctt gga gaa ccc aac tcc agt gtt tcc ctt     1440
   Glu Ala Gly Gly Ser Gly Leu Gly Glu Pro Asn Ser Ser Val Ser Leu
   465                     470                     475                 480
   aag cag cca gca tct ttg aaa ctc tct gca tca gct ttg aca ggg tca     1488
   Lys Gln Pro Ala Ser Leu Lys Leu Ser Ala Ser Ala Leu Thr Gly Ser
                   485                     490                     495
   gag gct gga aaa caa gtc tcc ggt gta cat agg aag aac aaa cat aga     1536
   Glu Ala Gly Lys Gln Val Ser Gly Val His Arg Lys Asn Lys His Arg
               500                     505                     510
   gac tta ata cat cgg cgg cgt ggg gat tcc acg ctc cgg cga ttc agg     1584
   Asp Leu Ile His Arg Arg Arg Gly Asp Ser Thr Leu Arg Arg Phe Arg
           515                     520                     525
   gag cat agg agg cag ctc cct ctc tct gct cgg aga att gac ccg caa 1632
   Glu His Arg Arg Gln Leu Pro Leu Ser Ala Arg Arg Ile Asp Pro Gln
   530 535 540
   cgc tgg gca gca ctt cta gaa aaa gcc aaa aag aat tct gtg cca aaa 1680
   Arg Trp Ala Ala Leu Leu Glu Lys Ala Lys Lys Asn Ser Val Pro Lys
   545 550 555 560
   aag caa gaa aat acc aca gta aag cca gtg cca ctg gct gtt ccc ctc 1728
   Lys Gln Glu Asn Thr Thr Val Lys Pro Val Pro Leu Ala Val Pro Leu
   565 570 575
   gtg gaa ctc act gac gag gaa aag gat gcc tct ggc atg att cct cca 1776
   Val Glu Leu Thr Asp Glu Glu Lys Asp Ala Ser Gly Met Ile Pro Pro
   580 585 590
   gat gaa gaa ttc atg gtt ctg aaa act aag gct tct ggt gtc cca gga 1824
   Asp Glu Glu Phe Met Val Leu Lys Thr Lys Ala Ser Gly Val Pro Gly
   595 600 605
   agg tca cca act gct gac tct gga cca gta aat cat ggt ttt atg acg 1872
   Arg Ser Pro Thr Ala Asp Ser Gly Pro Val Asn His Gly Phe Met Thr
   610 615 620
   agt ata gct tct ggc aca gaa gtc tca act gtg aat cca caa aca cta 1920
   Ser Ile Ala Ser Gly Thr Glu Val Ser Thr Val Asn Pro Gln Thr Leu
   625 630 635 640
   caa tct gag cac ctt cct gat ttc aaa tta ttt agt gta aca aac ggt 1968
   Gln Ser Glu His Leu Pro Asp Phe Lys Leu Phe Ser Val Thr Asn Gly
   645 650 655
   aca gct gtg aca aag agt atg aac cca tcc ata gca agc aaa ata gaa 2016
   Thr Ala Val Thr Lys Ser Met Asn Pro Ser Ile Ala Ser Lys Ile Glu
   660 665 670
   gat aca acc aac caa aac cca atc att atc ttt cca tca gta gct gaa 2064
   Asp Thr Thr Asn Gln Asn Pro Ile Ile Ile Phe Pro Ser Val Ala Glu
   675 680 685
   att cga gat tct gct cag gca gga aga gca tct tcc caa agt gca cac 2112
   Ile Arg Asp Ser Ala Gln Ala Gly Arg Ala Ser Ser Gln Ser Ala His
   690 695 700
   cct gta aca ggg gga aac atg gct acc tat ggc cat acc aac aca tat 2160
   Pro Val Thr Gly Gly Asn Met Ala Thr Tyr Gly His Thr Asn Thr Tyr
   705 710 715 720
   agt agc ttt acc agc aaa gcc agt aca gtc ttg cag cca ata aat cca 2208
   Ser Ser Phe Thr Ser Lys Ala Ser Thr Val Leu Gln Pro Ile Asn Pro
   725 730 735
   aca gaa agt tat gga cct cag ata cct att aca gga gtc agc aga cct 2256
   Thr Glu Ser Tyr Gly Pro Gln Ile Pro Ile Thr Gly Val Ser Arg Pro
   740 745 750
   agc agt agt gac atc tct tct cac act act gca gac cct agc ttc tcc 2304
   Ser Ser Ser Asp Ile Ser Ser His Thr Thr Ala Asp Pro Ser Phe Ser
   755 760 765
   agt cac cct tca ggt tca cac acc act gcc tcg tct tta ttt cac att    2352
   Ser His Pro Ser Gly Ser His Thr Thr Ala Ser Ser Leu Phe His Ile
       770                 775                 780
   cct aga aac aac aat aca ggt aac ttc ccc ttg tcc agg cac ttg gga    2400
   Pro Arg Asn Asn Asn Thr Gly Asn Phe Pro Leu Ser Arg His Leu Gly
   785                 790                 795                 800
   aga gag agg aca att tgg agc aga ggg aga gtt aaa aac cca cat aga    2448
   Arg Glu Arg Thr Ile Trp Ser Arg Gly Arg Val Lys Asn Pro His Arg
                   805                 810                 815
   acc cca gtt ctc cga cgg cat aga cac agg act gtg agg cca gca atc    2496
   Thr Pro Val Leu Arg Arg His Arg His Arg Thr Val Arg Pro Ala Ile
               820                 825                 830
   aag gga cct gct aac aaa aat gtg agc caa gtt cca gcc aca gag tac    2544
   Lys Gly Pro Ala Asn Lys Asn Val Ser Gln Val Pro Ala Thr Glu Tyr
           835                 840                 845
   cct ggg atg tgc cac aca tgt cct tcc gca gag ggg ctc aca gtg gct    2592
   Pro Gly Met Cys His Thr Cys Pro Ser Ala Glu Gly Leu Thr Val Ala
       850                 855                 860
   act gca gca ctg tca gtt cca agt tca tcc cac agt gcc ctc ccc aaa    2640
   Thr Ala Ala Leu Ser Val Pro Ser Ser Ser His Ser Ala Leu Pro Lys
   865                 870                 875                 880
   act aat aat gtt ggg gtc ata gca gaa gag tct acc act gtg gtc aag    2688
   Thr Asn Asn Val Gly Val Ile Ala Glu Glu Ser Thr Thr Val Val Lys
                   885                 890                 895
   aaa cca ctg tta cta ttt aag gac aaa caa aat gta gat att gag ata    2736
   Lys Pro Leu Leu Leu Phe Lys Asp Lys Gln Asn Val Asp Ile Glu Ile
               900                 905                 910
   ata aca acc act aca aaa tat tcc gga ggg gaa agt aac cac gtg att    2784
   Ile Thr Thr Thr Thr Lys Tyr Ser Gly Gly Glu Ser Asn His Val Ile
           915                 920                 925
   cct acg gaa gca agc atg act tct gct cca aca tct gta tcc ctg ggg    2832
   Pro Thr Glu Ala Ser Met Thr Ser Ala Pro Thr Ser Val Ser Leu Gly
       930                 935                 940
   aaa tct cct gta gac aat agt ggt cac ctg agc atg cct ggg acc atc    2880
   Lys Ser Pro Val Asp Asn Ser Gly His Leu Ser Met Pro Gly Thr Ile
   945                 950                 955                 960
   caa act ggg aaa gat tca gtg gaa aca aca cca ctt ccc agc ccc ctc    2928
   Gln Thr Gly Lys Asp Ser Val Glu Thr Thr Pro Leu Pro Ser Pro Leu
                   965                 970                 975
   agc aca ccc tca ata cca aca agc aca aaa ttc tca aag agg aaa act    2976
   Ser Thr Pro Ser Ile Pro Thr Ser Thr Lys Phe Ser Lys Arg Lys Thr
               980                 985                 990
   ccc ttg cac cag atc ttt gta aat aac cag aag aag gag ggg atg tta    3024
   Pro Leu His Gln Ile Phe Val Asn Asn Gln Lys Lys Glu Gly Met Leu
           995                 1000                1005
   aag aat cca tat caa ttc ggt tta caa aag aac cca gcc gca aag ctt    3072
   Lys Asn Pro Tyr Gln Phe Gly Leu Gln Lys Asn Pro Ala Ala Lys Leu
       1010                1015                1020
   ccc aaa ata gct cct ctt tta ccc aca ggt cag agt tcc ccc tca gat     3120
   Pro Lys Ile Ala Pro Leu Leu Pro Thr Gly Gln Ser Ser Pro Ser Asp
   1025                1030                1035                1040
   tct aca act ctc ttg aca agt ccg cca cca gct ctg tct aca aca atg     3168
   Ser Thr Thr Leu Leu Thr Ser Pro Pro Pro Ala Leu Ser Thr Thr Met
                   1045                1050                1055
   gct gcc act cag aac aag ggc act gaa gta gta tca ggt gcc aga agt     3216
   Ala Ala Thr Gln Asn Lys Gly Thr Glu Val Val Ser Gly Ala Arg Ser
               1060                1065                1070
   ctc tca gca ggg aag aag cag ccc ttc acc aac tcc tct cca gtg ctt     3264
   Leu Ser Ala Gly Lys Lys Gln Pro Phe Thr Asn Ser Ser Pro Val Leu
           1075                1080                1085
   cct agc acc ata agc aag aga tct aat aca tta aac ttc ttg tca acg     3312
   Pro Ser Thr Ile Ser Lys Arg Ser Asn Thr Leu Asn Phe Leu Ser Thr
       1090                1095                1100
   gaa acc ccc aca gtg aca agt cct act gct act gca tct gtc att atg     3360
   Glu Thr Pro Thr Val Thr Ser Pro Thr Ala Thr Ala Ser Val Ile Met
   1105                1110                1115                1120
   tct gaa acc caa cga aca aga tcc aaa gaa gca aaa gac caa ata aag     3408
   Ser Glu Thr Gln Arg Thr Arg Ser Lys Glu Ala Lys Asp Gln Ile Lys
                   1125                1130                1135
   ggg cct cgg aag aac aga aac aac gca aac acc acc ccc agg cag gtt     3456
   Gly Pro Arg Lys Asn Arg Asn Asn Ala Asn Thr Thr Pro Arg Gln Val
               1140                1145                1150
   tct ggc tat agt gca tac tca gct cta aca aca gct gat acc ccc ttg     3504
   Ser Gly Tyr Ser Ala Tyr Ser Ala Leu Thr Thr Ala Asp Thr Pro Leu
           1155                1160                1165
   gct ttc agt cat tcc cca cga caa gat gat ggt gga aat gta agt gca     3552
   Ala Phe Ser His Ser Pro Arg Gln Asp Asp Gly Gly Asn Val Ser Ala
       1170                1175                1180
   gtt gct tat cac tca aca acc tct ctt ctg gcc ata act gaa ctg ttt     3600
   Val Ala Tyr His Ser Thr Thr Ser Leu Leu Ala Ile Thr Glu Leu Phe
   1185                1190                1195                1200
   gag aag tac acc cag act ttg gga aat aca aca gct ttg gaa aca acg     3648
   Glu Lys Tyr Thr Gln Thr Leu Gly Asn Thr Thr Ala Leu Glu Thr Thr
                   1205                1210                1215
   ttg ttg agc aaa tca cag gag agt acc aca gtg aaa aga gcc tca gac     3696
   Leu Leu Ser Lys Ser Gln Glu Ser Thr Thr Val Lys Arg Ala Ser Asp
               1220                1225                1230
   aca cca cca cca ctc ctc agc agt ggg gcg ccc cca gtg ccc act cct     3744
   Thr Pro Pro Pro Leu Leu Ser Ser Gly Ala Pro Pro Val Pro Thr Pro
           1235                1240                1245
   tcc cca cct cct ttt act aag ggt gtg gtt aca gac agc aaa gtc aca     3792
   Ser Pro Pro Pro Phe Thr Lys Gly Val Val Thr Asp Ser Lys Val Thr
   1250                1255                1260
   tca gct ttc cag atg acg tca aat aga gtg gtc acc ata tat gaa tct   3840
   Ser Ala Phe Gln Met Thr Ser Asn Arg Val Val Thr Ile Tyr Glu Ser
   1265                1270                1275                1280
   tca agg cac aat aca gat ctg cag caa ccc tca gca gag gct agc ccc   3888
   Ser Arg His Asn Thr Asp Leu Gln Gln Pro Ser Ala Glu Ala Ser Pro
                   1285                1290                1295
   aat cct gag atc ata act gga acc act gac tct ccc tct aat ctg ttt   3936
   Asn Pro Glu Ile Ile Thr Gly Thr Thr Asp Ser Pro Ser Asn Leu Phe
               1300                1305                1310
   cca tcc act tct gtg cca gca cta agg gta gat aaa cca cag aat tct   3984
   Pro Ser Thr Ser Val Pro Ala Leu Arg Val Asp Lys Pro Gln Asn Ser
           1315                1320                1325
   aaa tgg aag ccc tct ccc tgg cca gaa cac aaa tat cag ctc aag tca   4032
   Lys Trp Lys Pro Ser Pro Trp Pro Glu His Lys Tyr Gln Leu Lys Ser
       1330                1335                1340
   tac tcc gaa acc att gag aag ggc aaa agg cca gca gta agc atg tcc   4080
   Tyr Ser Glu Thr Ile Glu Lys Gly Lys Arg Pro Ala Val Ser Met Ser
   1345                1350                1355                1360
   ccc cac ctc agc ctt cca gag gcc agc act cat gcc tca cac tgg aat   4128
   Pro His Leu Ser Leu Pro Glu Ala Ser Thr His Ala Ser His Trp Asn
                   1365                1370                1375
   aca cag aag cat gca gaa aag agt gtt ttt gat aag aaa cct ggt caa   4176
   Thr Gln Lys His Ala Glu Lys Ser Val Phe Asp Lys Lys Pro Gly Gln
               1380                1385                1390
   aac cca act tcc aaa cat ctg cct tac gtc tct cta cct aag act cta   4224
   Asn Pro Thr Ser Lys His Leu Pro Tyr Val Ser Leu Pro Lys Thr Leu
           1395                1400                1405
   ttg aaa aag cca aga ata att gga gga aag gct gca agc ttt aca gtt   4272
   Leu Lys Lys Pro Arg Ile Ile Gly Gly Lys Ala Ala Ser Phe Thr Val
       1410                1415                1420
   cca gct aat tca gac gtt ttt ctt cct tgt gag gct gtt gga gac cca   4320
   Pro Ala Asn Ser Asp Val Phe Leu Pro Cys Glu Ala Val Gly Asp Pro
   1425                1430                1435                1440
   ctg ccc atc atc cac tgg acc aga gtt tca tca gga ntt gaa ata tcc   4368
   Leu Pro Ile Ile His Trp Thr Arg Val Ser Ser Gly Xaa Glu Ile Ser
                   1445                1450                1455
   caa ggg aca cag aaa agc cgg ttc cac gtg ctt ccc aat ggc acc ttg   4416
   Gln Gly Thr Gln Lys Ser Arg Phe His Val Leu Pro Asn Gly Thr Leu
               1460                1465                1470
   tcc atc cag agg gtc agt att cag gac cgt gga cag tac ctg tgc tct   4464
   Ser Ile Gln Arg Val Ser Ile Gln Asp Arg Gly Gln Tyr Leu Cys Ser
           1475                1480                1485
   gca ttt aat cca ctg ggc gta gac cat ttt cat gtc tct ttg tct gtg   4512
   Ala Phe Asn Pro Leu Gly Val Asp His Phe His Val Ser Leu Ser Val
       1490                1495                1500
   gtt ttt tac ccg gca agg att ttg gac aga cat gtc aag gag atc aca    4560
   Val Phe Tyr Pro Ala Arg Ile Leu Asp Arg His Val Lys Glu Ile Thr
   1505                1510                1515                1520
   gtt cac ttt gga agt act gtg gaa cta aag tgc aga gtg gag ggt atg    4608
   Val His Phe Gly Ser Thr Val Glu Leu Lys Cys Arg Val Glu Gly Met
                   1525                1530                1535
   ccg agg cct acg gtt tcc tgg ata ctt gca aac caa acg gtg gtc tca    4656
   Pro Arg Pro Thr Val Ser Trp Ile Leu Ala Asn Gln Thr Val Val Ser
               1540                1545                1550
   gaa acg gcc aag gga agc aga aag gtg tgg gta aca cct gat gga aca    4704
   Glu Thr Ala Lys Gly Ser Arg Lys Val Trp Val Thr Pro Asp Gly Thr
           1555                1560                1565
   ttg atc atc tat aat ctg agt ctt tat gat cgt ggt ttt tac aag tgt    4752
   Leu Ile Ile Tyr Asn Leu Ser Leu Tyr Asp Arg Gly Phe Tyr Lys Cys
       1570                1575                1580
   gtg gcc agc aac cca tct ggc cag gat tca ctg ttg gtt aag ata caa    4800
   Val Ala Ser Asn Pro Ser Gly Gln Asp Ser Leu Leu Val Lys Ile Gln
   1585                1590                1595                1600
   gtc atc aca gct ccc cct gtc att ata gag caa aag agg caa gcc atc    4848
   Val Ile Thr Ala Pro Pro Val Ile Ile Glu Gln Lys Arg Gln Ala Ile
                   1605                1610                1615
   gtt ggg gtt tta ggt gga agt ttg aaa ctg ccc tgc act gca aaa gga    4896
   Val Gly Val Leu Gly Gly Ser Leu Lys Leu Pro Cys Thr Ala Lys Gly
               1620                1625                1630
   act ccc cag cct agt gtt cac tgg gtc ctt tat gat ggg act gaa cta    4944
   Thr Pro Gln Pro Ser Val His Trp Val Leu Tyr Asp Gly Thr Glu Leu
           1635                1640                1645
   aaa cca ttg cag ttg act cat tcc aga ttt ttc ttg tat cca aat gga    4992
   Lys Pro Leu Gln Leu Thr His Ser Arg Phe Phe Leu Tyr Pro Asn Gly
       1650                1655                1660
   act ctg tat ata aga agc atc gct cct tca gtg agg ggc act tat gag    5040
   Thr Leu Tyr Ile Arg Ser Ile Ala Pro Ser Val Arg Gly Thr Tyr Glu
   1665                1670                1675                1680
   tgc att gcc acc agc tcc tca ggc tca gag aga agg gta gtg att ctt    5088
   Cys Ile Ala Thr Ser Ser Ser Gly Ser Glu Arg Arg Val Val Ile Leu
                   1685                1690                1695
   act gtg gaa gag gga gag aca atc ccc agg ata gaa act gcc tct cag    5136
   Thr Val Glu Glu Gly Glu Thr Ile Pro Arg Ile Glu Thr Ala Ser Gln
               1700                1705                1710
   aaa tgg act gag gtg aat ttg ggt gag aaa tta cta ctg aac tgc tca    5184
   Lys Trp Thr Glu Val Asn Leu Gly Glu Lys Leu Leu Leu Asn Cys Ser
           1715                1720                1725
   gct act ggg gat cca aag cct aga ata atc tgg agg ctg cca tcc aag    5232
   Ala Thr Gly Asp Pro Lys Pro Arg Ile Ile Trp Arg Leu Pro Ser Lys
       1730                1735                1740
   gct gtc atc gac cag tgg cac aga atg ggc agc cga atc cac gtc tac     5280
   Ala Val Ile Asp Gln Trp His Arg Met Gly Ser Arg Ile His Val Tyr
   1745                1750                1755                1760
   cca aat gga tcc ttg gtg gtt ggg tca gtg acg gaa aaa gac gct ggt     5328
   Pro Asn Gly Ser Leu Val Val Gly Ser Val Thr Glu Lys Asp Ala Gly
                   1765                1770                1775
   gac tac tta tgt gtg gca aga aac aaa atg gga gat gac cta gtc ctg     5376
   Asp Tyr Leu Cys Val Ala Arg Asn Lys Met Gly Asp Asp Leu Val Leu
               1780                1785                1790
   atg cat gtc cgc ctg aga ttg aca cct gcc aaa att gaa cag aag cag     5424
   Met His Val Arg Leu Arg Leu Thr Pro Ala Lys Ile Glu Gln Lys Gln
           1795                1800                1805
   tat ttt aag aag caa gtg ctc cat ggg aaa gat ttc caa gtt gac tgc     5472
   Tyr Phe Lys Lys Gln Val Leu His Gly Lys Asp Phe Gln Val Asp Cys
       1810                1815                1820
   aag gcc tct ggc tcc cct gtg cct gag gta tcc tgg agt ttg cct gat     5520
   Lys Ala Ser Gly Ser Pro Val Pro Glu Val Ser Trp Ser Leu Pro Asp
   1825                1830                1835                1840
   ggg aca gtg ctc aac aat gta gcc caa gct gat gac agt ggc tat agg     5568
   Gly Thr Val Leu Asn Asn Val Ala Gln Ala Asp Asp Ser Gly Tyr Arg
                   1845                1850                1855
   acc aag agg tac acc ctt ttc cac aat gga acc ttg tat ttc aac aac     5616
   Thr Lys Arg Tyr Thr Leu Phe His Asn Gly Thr Leu Tyr Phe Asn Asn
               1860                1865                1870
   gtt ggg atg gca gag gaa gga gat tat atc tgc tct gcc cag aac acc     5664
   Val Gly Met Ala Glu Glu Gly Asp Tyr Ile Cys Ser Ala Gln Asn Thr
           1875                1880                1885
   tta ggg aaa gat gag atg aaa gtc cac cta aca gtt cta aca gcc atc     5712
   Leu Gly Lys Asp Glu Met Lys Val His Leu Thr Val Leu Thr Ala Ile
       1890                1895                1900
   cca cgg ata agg caa agc tac aag acc acc atg agg ctc agg gct gga     5760
   Pro Arg Ile Arg Gln Ser Tyr Lys Thr Thr Met Arg Leu Arg Ala Gly
   1905                1910                1915                1920
   gaa aca gct gtc ctt gac tgc gag gtc act ggg gaa ccg aag ccc aat     5808
   Glu Thr Ala Val Leu Asp Cys Glu Val Thr Gly Glu Pro Lys Pro Asn
                   1925                1930                1935
   gta ttt tgg ttg ctg cct tcc aac aat gtc att tca ttc tcc aat gac     5856
   Val Phe Trp Leu Leu Pro Ser Asn Asn Val Ile Ser Phe Ser Asn Asp
               1940                1945                1950
   agg ttc aca ttt cat gcc aat aga act ttg tcc atc cat aaa gtg aaa     5904
   Arg Phe Thr Phe His Ala Asn Arg Thr Leu Ser Ile His Lys Val Lys
           1955                1960                1965
   cca ctt gac tct ggg gac tat gtg tgc gta gct cag aat cct agt ggg     5952
   Pro Leu Asp Ser Gly Asp Tyr Val Cys Val Ala Gln Asn Pro Ser Gly
       1970                1975                1980
   gat gac act aag aca tac aaa ctg gac att gtc tct aaa cct cca tta     6000
   Asp Asp Thr Lys Thr Tyr Lys Leu Asp Ile Val Ser Lys Pro Pro Leu
   1985                1990                1995                2000
   atc aat ggc ctg tat gca aac aag act gtt att aaa gcc aca gcc att     6048
   Ile Asn Gly Leu Tyr Ala Asn Lys Thr Val Ile Lys Ala Thr Ala Ile
               2005                2010                2015
   cgg cac tcc aaa aaa tac ttt gac tgc aga gca gat ggg atc cca tct     6096
   Arg His Ser Lys Lys Tyr Phe Asp Cys Arg Ala Asp Gly Ile Pro Ser
           2020                2025                2030
   tcc cag gtc acg tgg att atg cca ggc aat att ttc ctc cca gct cca     6144
   Ser Gln Val Thr Trp Ile Met Pro Gly Asn Ile Phe Leu Pro Ala Pro
       2035                2040                2045
   tac ttt gga agc aga gtc acg gtc cat cca aat gga acc ttg gag atg     6192
   Tyr Phe Gly Ser Arg Val Thr Val His Pro Asn Gly Thr Leu Glu Met
   2050                2055                2060
   agg aac atc cgg ctt tct gac tct gcg gac ttc acc tgt gtg gtt cgg     6240
   Arg Asn Ile Arg Leu Ser Asp Ser Ala Asp Phe Thr Cys Val Val Arg
   2065                2070                2075                2080
   agc gag gga gga gag agt gtg ttg gta gtg cag tta gaa gtc cta gaa     6288
   Ser Glu Gly Gly Glu Ser Val Leu Val Val Gln Leu Glu Val Leu Glu
                   2085                2090                2095
   atg ctg aga aga cca aca ttc aga aac cca ttc aac gaa aaa gtc atc     6336
   Met Leu Arg Arg Pro Thr Phe Arg Asn Pro Phe Asn Glu Lys Val Ile
               2100                2105                2110
   gcc caa gct ggc aag ccc gta gca ctg aac tgc tct gtg gat ggg aac     6384
   Ala Gln Ala Gly Lys Pro Val Ala Leu Asn Cys Ser Val Asp Gly Asn
           2115                2120                2125
   ccc cca cct gaa att acc tgg atc tta cct gac ggc aca cag ttt gct     6432
   Pro Pro Pro Glu Ile Thr Trp Ile Leu Pro Asp Gly Thr Gln Phe Ala
       2130                2135                2140
   aac aga cca cac aat tcc ccg tat ctg atg gca ggc aat ggc tct ctc     6480
   Asn Arg Pro His Asn Ser Pro Tyr Leu Met Ala Gly Asn Gly Ser Leu
   2145                2150                2155                2160
   atc ctt tac aaa gca act cgg aac aag tca ggg aag tat cgc tgt gca     6528
   Ile Leu Tyr Lys Ala Thr Arg Asn Lys Ser Gly Lys Tyr Arg Cys Ala
                   2165                2170                2175
   gcc agg aat aag gtt ggc tac atc gag aaa ctc atc ctg tta gag att     6576
   Ala Arg Asn Lys Val Gly Tyr Ile Glu Lys Leu Ile Leu Leu Glu Ile
               2180                2185                2190
   ggg cag aag cca gtc att ctg aca tac gaa cca ggg atg gtg aag agc     6624
   Gly Gln Lys Pro Val Ile Leu Thr Tyr Glu Pro Gly Met Val Lys Ser
           2195                2200                2205
   gtc agt ggg gaa ccg tta tca ctg cat tgt gtg tct gat ggg atc ccc     6672
   Val Ser Gly Glu Pro Leu Ser Leu His Cys Val Ser Asp Gly Ile Pro
       2210                2215                2220
   aag cca aat gtc aag tgg act aca ccg ggt ggc cat gta atc gac agg     6720
   Lys Pro Asn Val Lys Trp Thr Thr Pro Gly Gly His Val Ile Asp Arg
   2225                2230                2235                2240
   cct caa gtg gat gga aaa tac ata ctg cat gaa aat ggc acg ctg gtc    6768
   Pro Gln Val Asp Gly Lys Tyr Ile Leu His Glu Asn Gly Thr Leu Val
                   2245                2250                2255
   atc aaa gca aca aca gct cac gac caa gga aat tat atc tgt agg gct    6816
   Ile Lys Ala Thr Thr Ala His Asp Gln Gly Asn Tyr Ile Cys Arg Ala
               2260                2265                2270
   caa aac agt gtt ggc cag gca gtt att agc gtg tca gtg atg gtt gtg    6864
   Gln Asn Ser Val Gly Gln Ala Val Ile Ser Val Ser Val Met Val Val
           2275                2280                2285
   gcc tac cct ccc cga atc ata aac tac cta ccc agg aac atg ctc agg    6912
   Ala Tyr Pro Pro Arg Ile Ile Asn Tyr Leu Pro Arg Asn Met Leu Arg
       2290                2295                2300
   agg aca ggg gaa gcc atg cag ctc cac tgt gtg gcc ttg gga atc ccc    6960
   Arg Thr Gly Glu Ala Met Gln Leu His Cys Val Ala Leu Gly Ile Pro
   2305                2310                2315                2320
   aag cca aaa gtc acc tgg gag acg cca aga cac tcc ctg ctc tca aaa    7008
   Lys Pro Lys Val Thr Trp Glu Thr Pro Arg His Ser Leu Leu Ser Lys
                   2325                2330                2335
   gca aca gca aga aaa ccc cat aga agt gag atg ctt cac cca caa ggt    7056
   Ala Thr Ala Arg Lys Pro His Arg Ser Glu Met Leu His Pro Gln Gly
               2340                2345                2350
   acg ctg gtc att cag aat ctc caa acc tcg gat tcc gga gtc tat aag    7104
   Thr Leu Val Ile Gln Asn Leu Gln Thr Ser Asp Ser Gly Val Tyr Lys
           2355                2360                2365
   tgc aga gct cag aac cta ctt ggg act gat tac gca aca act tac atc    7152
   Cys Arg Ala Gln Asn Leu Leu Gly Thr Asp Tyr Ala Thr Thr Tyr Ile
       2370                2375                2380
   cag gta ctc tgacaggaag ggggagacta aaattcaaca gaagtccaca            7201
   Gln Val Leu
   2385
   tccacagggt ttattttttg gaagaagttt aatcaaaggc agccataggc atgtaaatga 7261
   gtctgaatac atttacagta ttaaatttac aatggacatg cgatgagact tgtaaatgaa 7321
   agcattgtga actgaaaccg agtctctgtg gatctcaaag caaactctta acttaaggca 7381
   ctttgatttt gccaacaaat aataacaaac attaagagaa aaaaatgatc cactacgaaa 7441
   taacaaacgg ctaatgcacc tgaattctca gtaaaaagac ctttctctcg ctaacagttg 7501
   ccagcagcct cgtgtctgtt tcctaccaat gtcacaaaca tcgcacacag ggtgaatgga 7561
   gtcaacggga aagattaagt ttgcggtctg tgtaaatctc aatgtacaaa tattctgtcn 7621
   ctggtttata aacattttga taaaaccgaa aaaaaaaaaa aaaaaaaaaa aaaaaaaa     7679
<210> 35
   <211> 2387
   <212> PRT
   <213> Rattus sp.
<220>
   <221> misc_feature
   <222> (1453)..(1453)
   <223> "Xaa" can be any amino acid
<400> 35
   Leu Glu Ser Leu Tyr Leu His Gly Asn Pro Trp Thr Cys Asp Cys His
    1               5                  10                  15
   Leu Lys Trp Leu Ser Glu Trp Met Gln Gly Asn Pro Asp Ile Ile Lys
               20                  25                  30
   Cys Lys Lys Asp Arg Ser Ser Ser Ser Pro Gln Gln Cys Pro Leu Cys
           35                  40                  45
   Met Asn Pro Arg Ile Ser Lys Gly Arg Pro Phe Ala Met Val Pro Ser
       50                  55                  60
   Gly Ala Phe Leu Cys Thr Lys Pro Thr Ile Asp Pro Ser Leu Lys Ser
   65                  70                  75                  80
   Lys Ser Leu Val Thr Gln Glu Asp Asn Gly Ser Ala Ser Thr Ser Pro
                   85                  90                  95
   Gln Asp Phe Ile Glu Pro Phe Gly Ser Leu Ser Leu Asn Met Thr Asp
               100                 105                 110
   Leu Ser Gly Asn Lys Ala Asp Met Val Cys Ser Ile Gln Lys Pro Ser
           115                 120                 125
   Arg Thr Ser Pro Thr Ala Phe Thr Glu Glu Asn Asp Tyr Ile Met Leu
       130                 135                 140
   Asn Ala Ser Phe Ser Thr Asn Leu Val Cys Ser Val Asp Tyr Asn His
   145                 150                 155                 160
   Ile Gln Pro Val Trp Gln Leu Leu Ala Leu Tyr Ser Asp Ser Pro Leu
                   165                 170                 175
   Ile Leu Glu Arg Lys Pro Gln Leu Thr Glu Thr Pro Ser Leu Ser Ser
               180                 185                 190
   Arg Tyr Lys Gln Val Ala Leu Arg Pro Glu Asp Ile Phe Thr Ser Ile
           195                 200                 205
   Glu Ala Asp Val Arg Ala Asp Pro Phe Trp Phe Gln Gln Glu Lys Ile
       210                 215                 220
   Val Leu Gln Leu Asn Arg Thr Ala Thr Thr Leu Ser Thr Leu Gln Ile
   225                 230                 235                 240
   Gln Phe Ser Thr Asp Ala Gln Ile Ala Leu Pro Arg Ala Glu Met Arg
                   245                 250                 255
   Ala Glu Arg Leu Lys Trp Thr Met Ile Leu Met Met Asn Asn Pro Lys
               260                 265                 270
   Leu Glu Arg Thr Val Leu Val Gly Gly Thr Ile Ala Leu Ser Cys Pro
   275 280 285
   Gly Lys Gly Asp Pro Ser Pro His Leu Glu Trp Leu Leu Ala Asp Gly
   290 295 300
   Ser Lys Val Arg Ala Pro Tyr Val Ser Glu Asp Gly Arg Ile Leu Ile
   305 310 315 320
   Asp Lys Asn Gly Lys Leu Glu Leu Gln Met Ala Asp Ser Phe Asp Ala
   325 330 335
   Gly Leu Tyr His Cys Ile Ser Thr Asn Asp Ala Asp Ala Asp Val Leu
   340 345 350
   Thr Tyr Arg Ile Thr Val Val Glu Pro Tyr Gly Glu Ser Thr His Asp
   355 360 365
   Ser Gly Val Gln His Thr Val Val Thr Gly Glu Thr Leu Asp Leu Pro
   370 375 380
   Cys Leu Ser Thr Gly Val Pro Asp Ala Ser Ile Ser Trp Ile Leu Pro
   385 390 395 400
   Gly Asn Thr Val Phe Ser Gln Pro Ser Arg Asp Arg Gln Ile Leu Asn
   405 410 415
   Asn Gly Thr Leu Arg Ile Leu Gln Val Thr Pro Lys Asp Gln Gly His
   420 425 430
   Tyr Gln Cys Val Ala Ala Asn Pro Ser Gly Ala Asp Phe Ser Ser Phe
   435 440 445
   Lys Val Ser Val Gln Lys Lys Gly Gln Arg Met Val Glu His His Arg
   450 455 460
   Glu Ala Gly Gly Ser Gly Leu Gly Glu Pro Asn Ser Ser Val Ser Leu
   465 470 475 480
   Lys Gln Pro Ala Ser Leu Lys Leu Ser Ala Ser Ala Leu Thr Gly Ser
   485 490 495
   Glu Ala Gly Lys Gln Val Ser Gly Val His Arg Lys Asn Lys His Arg
   500 505 510
   Asp Leu Ile His Arg Arg Arg Gly Asp Ser Thr Leu Arg Arg Phe Arg
   515 520 525
   Glu His Arg Arg Gln Leu Pro Leu Ser Ala Arg Arg Ile Asp Pro Gln
   530 535 540
   Arg Trp Ala Ala Leu Leu Glu Lys Ala Lys Lys Asn Ser Val Pro Lys
   545 550 555 560
   Lys Gln Glu Asn Thr Thr Val Lys Pro Val Pro Leu Ala Val Pro Leu
   565 570 575
   Val Glu Leu Thr Asp Glu Glu Lys Asp Ala Ser Gly Met Ile Pro Pro
   580 585 590
   Asp Glu Glu Phe Met Val Leu Lys Thr Lys Ala Ser Gly Val Pro Gly
   595 600 605
   Arg Ser Pro Thr Ala Asp Ser Gly Pro Val Asn His Gly Phe Met Thr
       610                 615                 620
   Ser Ile Ala Ser Gly Thr Glu Val Ser Thr Val Asn Pro Gln Thr Leu
   625                 630                 635                 640
   Gln Ser Glu His Leu Pro Asp Phe Lys Leu Phe Ser Val Thr Asn Gly
                   645                 650                 655
   Thr Ala Val Thr Lys Ser Met Asn Pro Ser Ile Ala Ser Lys Ile Glu
               660                 665                 670
   Asp Thr Thr Asn Gln Asn Pro Ile Ile Ile Phe Pro Ser Val Ala Glu
           675                 680                 685
   Ile Arg Asp Ser Ala Gln Ala Gly Arg Ala Ser Ser Gln Ser Ala His
       690                 695                 700
   Pro Val Thr Gly Gly Asn Met Ala Thr Tyr Gly His Thr Asn Thr Tyr
   705                 710                 715                 720
   Ser Ser Phe Thr Ser Lys Ala Ser Thr Val Leu Gln Pro Ile Asn Pro
                   725                 730                 735
   Thr Glu Ser Tyr Gly Pro Gln Ile Pro Ile Thr Gly Val Ser Arg Pro
               740                 745                 750
   Ser Ser Ser Asp Ile Ser Ser His Thr Thr Ala Asp Pro Ser Phe Ser
           755                 760                 765
   Ser His Pro Ser Gly Ser His Thr Thr Ala Ser Ser Leu Phe His Ile
       770                 775                 780
   Pro Arg Asn Asn Asn Thr Gly Asn Phe Pro Leu Ser Arg His Leu Gly
   785                 790                 795                 800
   Arg Glu Arg Thr Ile Trp Ser Arg Gly Arg Val Lys Asn Pro His Arg
                   805                 810                 815
   Thr Pro Val Leu Arg Arg His Arg His Arg Thr Val Arg Pro Ala Ile
               820                 825                 830
   Lys Gly Pro Ala Asn Lys Asn Val Ser Gln Val Pro Ala Thr Glu Tyr
           835                 840                 845
   Pro Gly Met Cys His Thr Cys Pro Ser Ala Glu Gly Leu Thr Val Ala
       850                 855                 860
   Thr Ala Ala Leu Ser Val Pro Ser Ser Ser His Ser Ala Leu Pro Lys
   865                 870                 875                 880
   Thr Asn Asn Val Gly Val Ile Ala Glu Glu Ser Thr Thr Val Val Lys
                   885                 890                 895
   Lys Pro Leu Leu Leu Phe Lys Asp Lys Gln Asn Val Asp Ile Glu Ile
               900                 905                 910
   Ile Thr Thr Thr Thr Lys Tyr Ser Gly Gly Glu Ser Asn His Val Ile
           915                 920                 925
   Pro Thr Glu Ala Ser Met Thr Ser Ala Pro Thr Ser Val Ser Leu Gly
   930 935 940
   Lys Ser Pro Val Asp Asn Ser Gly His Leu Ser Met Pro Gly Thr Ile
   945 950 955 960
   Gln Thr Gly Lys Asp Ser Val Glu Thr Thr Pro Leu Pro Ser Pro Leu
   965 970 975
   Ser Thr Pro Ser Ile Pro Thr Ser Thr Lys Phe Ser Lys Arg Lys Thr
   980 985 990
   Pro Leu His Gln Ile Phe Val Asn Asn Gln Lys Lys Glu Gly Met Leu
   995 1000 1005
   Lys Asn Pro Tyr Gln Phe Gly Leu Gln Lys Asn Pro Ala Ala Lys Leu
   1010 1015 1020
   Pro Lys Ile Ala Pro Leu Leu Pro Thr Gly Gln Ser Ser Pro Ser Asp
   1025 1030 1035 1040
   Ser Thr Thr Leu Leu Thr Ser Pro Pro Pro Ala Leu Ser Thr Thr Met
   1045 1050 1055
   Ala Ala Thr Gln Asn Lys Gly Thr Glu Val Val Ser Gly Ala Arg Ser
   1060 1065 1070
   Leu Ser Ala Gly Lys Lys Gln Pro Phe Thr Asn Ser Ser Pro Val Leu
   1075 1080 1085
   Pro Ser Thr Ile Ser Lys Arg Ser Asn Thr Leu Asn Phe Leu Ser Thr
   1090 1095 1100
   Glu Thr Pro Thr Val Thr Ser Pro Thr Ala Thr Ala Ser Val Ile Met
   1105 1110 1115 1120
   Ser Glu Thr Gln Arg Thr Arg Ser Lys Glu Ala Lys Asp Gln Ile Lys
   1125 1130 1135
   Gly Pro Arg Lys Asn Arg Asn Asn Ala Asn Thr Thr Pro Arg Gln Val
   1140 1145 1150
   Ser Gly Tyr Ser Ala Tyr Ser Ala Leu Thr Thr Ala Asp Thr Pro Leu
   1155 1160 1165
   Ala Phe Ser His Ser Pro Arg Gln Asp Asp Gly Gly Asn Val Ser Ala
   1170 1175 1180
   Val Ala Tyr His Ser Thr Thr Ser Leu Leu Ala Ile Thr Glu Leu Phe
   1185 1190 1195 1200
   Glu Lys Tyr Thr Gln Thr Leu Gly Asn Thr Thr Ala Leu Glu Thr Thr
   1205 1210 1215
   Leu Leu Ser Lys Ser Gln Glu Ser Thr Thr Val Lys Arg Ala Ser Asp
   1220 1225 1230
   Thr Pro Pro Pro Leu Leu Ser Ser Gly Ala Pro Pro Val Pro Thr Pro
   1235 1240 1245
   Ser Pro Pro Pro Phe Thr Lys Gly Val Val Thr Asp Ser Lys Val Thr
   1250 1255 1260
   Ser Ala Phe Gln Met Thr Ser Asn Arg Val Val Thr Ile Tyr Glu Ser
   1265 1270 1275 1280
   Ser Arg His Asn Thr Asp Leu Gln Gln Pro Ser Ala Glu Ala Ser Pro
   1285 1290 1295
   Asn Pro Glu Ile Ile Thr Gly Thr Thr Asp Ser Pro Ser Asn Leu Phe
   1300 1305 1310
   Pro Ser Thr Ser Val Pro Ala Leu Arg Val Asp Lys Pro Gln Asn Ser
   1315 1320 1325
   Lys Trp Lys Pro Ser Pro Trp Pro Glu His Lys Tyr Gln Leu Lys Ser
   1330 1335 1340
   Tyr Ser Glu Thr Ile Glu Lys Gly Lys Arg Pro Ala Val Ser Met Ser
   1345 1350 1355 1360
   Pro His Leu Ser Leu Pro Glu Ala Ser Thr His Ala Ser His Trp Asn
   1365 1370 1375
   Thr Gln Lys His Ala Glu Lys Ser Val Phe Asp Lys Lys Pro Gly Gln
   1380 1385 1390
   Asn Pro Thr Ser Lys His Leu Pro Tyr Val Ser Leu Pro Lys Thr Leu
   1395 1400 1405
   Leu Lys Lys Pro Arg Ile Ile Gly Gly Lys Ala Ala Ser Phe Thr Val
   1410 1415 1420
   Pro Ala Asn Ser Asp Val Phe Leu Pro Cys Glu Ala Val Gly Asp Pro
   1425 1430 1435 1440
   Leu Pro Ile Ile His Trp Thr Arg Val Ser Ser Gly Xaa Glu Ile Ser
   1445 1450 1455
   Gln Gly Thr Gln Lys Ser Arg Phe His Val Leu Pro Asn Gly Thr Leu
   1460 1465 1470
   Ser Ile Gln Arg Val Ser Ile Gln Asp Arg Gly Gln Tyr Leu Cys Ser
   1475 1480 1485
   Ala Phe Asn Pro Leu Gly Val Asp His Phe His Val Ser Leu Ser Val
   1490 1495 1500
   Val Phe Tyr Pro Ala Arg Ile Leu Asp Arg His Val Lys Glu Ile Thr
   1505 1510 1515 1520
   Val His Phe Gly Ser Thr Val Glu Leu Lys Cys Arg Val Glu Gly Met
   1525 1530 1535
   Pro Arg Pro Thr Val Ser Trp Ile Leu Ala Asn Gln Thr Val Val Ser
   1540 1545 1550
   Glu Thr Ala Lys Gly Ser Arg Lys Val Trp Val Thr Pro Asp Gly Thr
   1555 1560 1565
   Leu Ile Ile Tyr Asn Leu Ser Leu Tyr Asp Arg Gly Phe Tyr Lys Cys
   1570 1575 1580
   Val Ala Ser Asn Pro Ser Gly Gln Asp Ser Leu Leu Val Lys Ile Gln
   1585 1590 1595 1600
   Val Ile Thr Ala Pro Pro Val Ile Ile Glu Gln Lys Arg Gln Ala Ile
   1605 1610 1615
   Val Gly Val Leu Gly Gly Ser Leu Lys Leu Pro Cys Thr Ala Lys Gly
   1620 1625 1630
   Thr Pro Gln Pro Ser Val His Trp Val Leu Tyr Asp Gly Thr Glu Leu
   1635 1640 1645
   Lys Pro Leu Gln Leu Thr His Ser Arg Phe Phe Leu Tyr Pro Asn Gly
   1650 1655 1660
   Thr Leu Tyr Ile Arg Ser Ile Ala Pro Ser Val Arg Gly Thr Tyr Glu
   1665 1670 1675 1680
   Cys Ile Ala Thr Ser Ser Ser Gly Ser Glu Arg Arg Val Val Ile Leu
   1685 1690 1695
   Thr Val Glu Glu Gly Glu Thr Ile Pro Arg Ile Glu Thr Ala Ser Gln
   1700 1705 1710
   Lys Trp Thr Glu Val Asn Leu Gly Glu Lys Leu Leu Leu Asn Cys Ser
   1715 1720 1725
   Ala Thr Gly Asp Pro Lys Pro Arg Ile Ile Trp Arg Leu Pro Ser Lys
   1730 1735 1740
   Ala Val Ile Asp Gln Trp His Arg Met Gly Ser Arg Ile His Val Tyr
   1745 1750 1755 1760
   Pro Asn Gly Ser Leu Val Val Gly Ser Val Thr Glu Lys Asp Ala Gly
   1765 1770 1775
   Asp Tyr Leu Cys Val Ala Arg Asn Lys Met Gly Asp Asp Leu Val Leu
   1780 1785 1790
   Met His Val Arg Leu Arg Leu Thr Pro Ala Lys Ile Glu Gln Lys Gln
   1795 1800 1805
   Tyr Phe Lys Lys Gln Val Leu His Gly Lys Asp Phe Gln Val Asp Cys
   1810 1815 1820
   Lys Ala Ser Gly Ser Pro Val Pro Glu Val Ser Trp Ser Leu Pro Asp
   1825 1830 1835 1840
   Gly Thr Val Leu Asn Asn Val Ala Gln Ala Asp Asp Ser Gly Tyr Arg
   1845 1850 1855
   Thr Lys Arg Tyr Thr Leu Phe His Asn Gly Thr Leu Tyr Phe Asn Asn
   1860 1865 1870
   Val Gly Met Ala Glu Glu Gly Asp Tyr Ile Cys Ser Ala Gln Asn Thr
   1875 1880 1885
   Leu Gly Lys Asp Glu Met Lys Val His Leu Thr Val Leu Thr Ala Ile
   1890 1895 1900
   Pro Arg Ile Arg Gln Ser Tyr Lys Thr Thr Met Arg Leu Arg Ala Gly
   1905 1910 1915 1920
   Glu Thr Ala Val Leu Asp Cys Glu Val Thr Gly Glu Pro Lys Pro Asn
   1925 1930 1935
   Val Phe Trp Leu Leu Pro Ser Asn Asn Val Ile Ser Phe Ser Asn Asp
   1940 1945 1950
   Arg Phe Thr Phe His Ala Asn Arg Thr Leu Ser Ile His Lys Val Lys
   1955 1960 1965
   Pro Leu Asp Ser Gly Asp Tyr Val Cys Val Ala Gln Asn Pro Ser Gly
   1970 1975 1980
   Asp Asp Thr Lys Thr Tyr Lys Leu Asp Ile Val Ser Lys Pro Pro Leu
   1985 1990 1995 2000
   Ile Asn Gly Leu Tyr Ala Asn Lys Thr Val Ile Lys Ala Thr Ala Ile
   2005 2010 2015
   Arg His Ser Lys Lys Tyr Phe Asp Cys Arg Ala Asp Gly Ile Pro Ser
   2020 2025 2030
   Ser Gln Val Thr Trp Ile Met Pro Gly Asn Ile Phe Leu Pro Ala Pro
   2035 2040 2045
   Tyr Phe Gly Ser Arg Val Thr Val His Pro Asn Gly Thr Leu Glu Met
   2050 2055 2060
   Arg Asn Ile Arg Leu Ser Asp Ser Ala Asp Phe Thr Cys Val Val Arg
   2065 2070 2075 2080
   Ser Glu Gly Gly Glu Ser Val Leu Val Val Gln Leu Glu Val Leu Glu
   2085 2090 2095
   Met Leu Arg Arg Pro Thr Phe Arg Asn Pro Phe Asn Glu Lys Val Ile
   2100 2105 2110
   Ala Gln Ala Gly Lys Pro Val Ala Leu Asn Cys Ser Val Asp Gly Asn
   2115 2120 2125
   Pro Pro Pro Glu Ile Thr Trp Ile Leu Pro Asp Gly Thr Gln Phe Ala
   2130 2135 2140
   Asn Arg Pro His Asn Ser Pro Tyr Leu Met Ala Gly Asn Gly Ser Leu
   2145 2150 2155 2160
   Ile Leu Tyr Lys Ala Thr Arg Asn Lys Ser Gly Lys Tyr Arg Cys Ala
   2165 2170 2175
   Ala Arg Asn Lys Val Gly Tyr Ile Glu Lys Leu Ile Leu Leu Glu Ile
   2180 2185 2190
   Gly Gln Lys Pro Val Ile Leu Thr Tyr Glu Pro Gly Met Val Lys Ser
   2195 2200 2205
   Val Ser Gly Glu Pro Leu Ser Leu His Cys Val Ser Asp Gly Ile Pro
   2210 2215 2220
   Lys Pro Asn Val Lys Trp Thr Thr Pro Gly Gly His Val Ile Asp Arg
   2225 2230 2235 2240
   Pro Gln Val Asp Gly Lys Tyr Ile Leu His Glu Asn Gly Thr Leu Val
   2245 2250 2255
   Ile Lys Ala Thr Thr Ala His Asp Gln Gly Asn Tyr Ile Cys Arg Ala
   2260 2265 2270
   Gln Asn Ser Val Gly Gln Ala Val Ile Ser Val Ser Val Met Val Val
   2275 2280 2285
   Ala Tyr Pro Pro Arg Ile Ile Asn Tyr Leu Pro Arg Asn Met Leu Arg
   2290 2295 2300
   Arg Thr Gly Glu Ala Met Gln Leu His Cys Val Ala Leu Gly Ile Pro
   2305 2310 2315 2320
   Lys Pro Lys Val Thr Trp Glu Thr Pro Arg His Ser Leu Leu Ser Lys
   2325 2330 2335
   Ala Thr Ala Arg Lys Pro His Arg Ser Glu Met Leu His Pro Gln Gly
   2340 2345 2350
   Thr Leu Val Ile Gln Asn Leu Gln Thr Ser Asp Ser Gly Val Tyr Lys
   2355 2360 2365
   Cys Arg Ala Gln Asn Leu Leu Gly Thr Asp Tyr Ala Thr Thr Tyr Ile
   2370 2375 2380
   Gln Val Leu
   2385
<210> 36
   <211> 7962
   <212> PRT
   <213> homo sapiens
<220>
   <222> (1)..(7962)
   <223> elastic titin
<300>
   <301> Andersson, et al. Vogel, et al. Lbeit, et al. Ohara, O
   <302> Unknown protein [Homo sapiens].
   <308> GI 3005744
   <309> 1998-04-02
   <313> 1 TO 494
<400> 36
   Pro Asp Gln Glu Met Pro Val Tyr Pro Pro Ala Ile Ile Thr Pro Leu
   1 5 10 15
   Gln Asp Thr Val Thr Ser Glu Gly Gln Pro Ala Arg Phe Gln Cys Arg
   20 25 30
   Val Ser Gly Thr Asp Leu Lys Val Ser Trp Tyr Ser Lys Asp Lys Lys
   35 40 45
   Ile Lys Pro Ser Arg Phe Phe Arg Met Thr Gln Phe Glu Asp Thr Tyr
   50 55 60
   Gln Leu Glu Ile Ala Glu Ala Tyr Pro Glu Asp Glu Gly Thr Tyr Thr
   65 70 75 80
   Phe Val Ala Asn Asn Ala Val Gly Gln Val Ser Ser Thr Ala Asn Leu
   85 90 95
   Ser Leu Glu Ala Pro Glu Ser Ile Leu His Glu Arg Ile Glu Gln Glu
   100 105 110
   Ile Glu Met Glu Met Lys Ala Ala Pro Val Ile Lys Arg Lys Ile Glu
   115 120 125
   Pro Leu Glu Val Ala Leu Gly His Leu Ala Lys Phe Thr Cys Glu Ile
   130 135 140
   Gln Ser Ala Pro Asn Val Arg Phe Gln Trp Phe Lys Ala Gly Arg Glu
   145 150 155 160
   Ile Tyr Glu Ser Asp Lys Cys Ser Ile Arg Ser Ser Lys Tyr Ile Ser
   165 170 175
   Ser Leu Glu Ile Leu Arg Thr Gln Val Val Asp Cys Gly Glu Tyr Thr
   180 185 190
   Cys Lys Ala Ser Asn Glu Tyr Gly Ser Val Ser Cys Thr Ala Thr Leu
   195 200 205
   Thr Val Thr Glu Ala Tyr Pro Pro Thr Phe Leu Ser Arg Pro Lys Ser
   210 215 220
   Leu Thr Thr Phe Val Gly Lys Ala Ala Lys Phe Ile Cys Thr Val Thr
   225 230 235 240
   Gly Thr Pro Val Ile Glu Thr Ile Trp Gln Lys Asp Gly Ala Ala Leu
   245 250 255
   Ser Pro Ser Pro Asn Trp Arg Ile Ser Asp Ala Glu Asn Lys His Ile
   260 265 270
   Leu Glu Leu Ser Asn Leu Thr Ile Gln Asp Arg Gly Val Tyr Ser Cys
   275 280 285
   Lys Ala Ser Asn Lys Phe Gly Ala Asp Ile Cys Gln Ala Glu Leu Ile
   290 295 300
   Ile Ile Asp Lys Pro His Phe Ile Lys Glu Leu Glu Pro Val Gln Ser
   305 310 315 320
   Ala Ile Asn Lys Lys Val His Leu Glu Cys Gln Val Asp Glu Asp Arg
   325 330 335
   Lys Val Thr Val Thr Trp Ser Lys Asp Gly Gln Lys Leu Pro Pro Gly
   340 345 350
   Lys Asp Tyr Lys Ile Cys Phe Glu Asp Lys Ile Ala Thr Leu Glu Ile
   355 360 365
   Pro Leu Ala Lys Leu Lys Asp Ser Gly Thr Tyr Val Cys Thr Ala Ser
   370 375 380
   Asn Glu Ala Gly Ser Ser Ser Cys Ser Ala Thr Val Thr Val Arg Glu
   385 390 395 400
   Pro Pro Ser Phe Val Lys Lys Val Asp Pro Ser Tyr Leu Met Leu Pro
   405 410 415
   Gly Glu Ser Ala Arg Leu His Cys Lys Leu Lys Gly Ser Pro Val Ile
   420 425 430
   Gln Val Thr Trp Phe Lys Asn Asn Lys Glu Leu Ser Glu Ser Asn Thr
   435 440 445
   Val Arg Met Tyr Phe Val Asn Ser Glu Ala Ile Leu Asp Ile Thr Asp
   450 455 460
   Val Lys Val Glu Asp Ser Gly Ser Tyr Ser Cys Glu Ala Val Asn Asp
   465 470 475 480
   Val Gly Ser Asp Ser Cys Ser Thr Glu Ile Val Ile Lys Glu Pro Pro
   485 490 495
   Ser Phe Ile Lys Thr Leu Glu Pro Ala Asp Ile Val Arg Gly Thr Asn
   500 505 510
   Ala Leu Leu Gln Cys Glu Val Ser Gly Thr Gly Pro Phe Glu Ile Ser
   515 520 525
   Trp Phe Lys Asp Lys Lys Gln Ile Arg Ser Ser Lys Lys Tyr Arg Leu
   530 535 540
   Phe Ser Gln Lys Ser Leu Val Cys Leu Glu Ile Phe Ser Phe Asn Ser
   545 550 555 560
   Ala Asp Val Gly Glu Tyr Glu Cys Val Val Ala Asn Glu Val Gly Lys
   565 570 575
   Cys Gly Cys Met Ala Thr His Leu Leu Lys Glu Pro Pro Thr Phe Val
   580 585 590
   Lys Lys Val Asp Asp Leu Ile Ala Leu Gly Gly Gln Thr Val Thr Leu
   595 600 605
   Gln Ala Ala Val Arg Gly Ser Glu Pro Ile Ser Val Thr Trp Met Lys
   610 615 620
   Gly Gln Glu Val Ile Arg Glu Asp Gly Lys Ile Lys Met Ser Phe Ser
   625 630 635 640
   Asn Gly Val Ala Val Leu Ile Ile Pro Asp Val Gln Ile Ser Phe Gly
   645 650 655
   Gly Lys Tyr Thr Cys Leu Ala Glu Asn Glu Ala Gly Ser Gln Thr Ser
   660 665 670
   Val Gly Glu Leu Ile Val Lys Glu Pro Ala Lys Ile Ile Glu Arg Ala
   675 680 685
   Glu Leu Ile Gln Val Thr Ala Gly Asp Pro Ala Thr Leu Glu Tyr Thr
   690 695 700
   Val Ala Gly Thr Pro Glu Leu Lys Pro Lys Trp Tyr Lys Asp Gly Arg
   705 710 715 720
   Pro Leu Val Ala Ser Lys Lys Tyr Arg Ile Ser Phe Lys Asn Asn Val
   725 730 735
   Ala Gln Leu Lys Phe Tyr Ser Ala Glu Leu His Asp Ser Gly Gln Tyr
   740 745 750
   Thr Phe Glu Ile Ser Asn Glu Val Gly Ser Ser Ser Cys Glu Thr Thr
   755 760 765
   Phe Thr Val Leu Asp Arg Asp Ile Ala Pro Phe Phe Thr Lys Pro Leu
   770 775 780
   Arg Asn Val Asp Ser Val Val Asn Gly Thr Cys Arg Leu Asp Cys Lys
   785 790 795 800
   Ile Ala Gly Ser Leu Pro Met Arg Val Ser Trp Phe Lys Asp Gly Lys
   805 810 815
   Glu Ile Ala Ala Ser Asp Arg Tyr Arg Ile Ala Phe Val Glu Gly Thr
   820 825 830
   Ala Ser Leu Glu Ile Ile Arg Val Asp Met Asn Asp Ala Gly Asn Phe
   835 840 845
   Thr Cys Arg Ala Thr Asn Ser Val Gly Ser Lys Asp Ser Ser Gly Ala
   850 855 860
   Leu Ile Val Gln Glu Pro Pro Ser Phe Val Thr Lys Pro Gly Ser Lys
   865 870 875 880
   Asp Val Leu Pro Gly Ser Ala Val Cys Leu Lys Ser Thr Phe Gln Gly
   885 890 895
   Ser Thr Pro Leu Thr Ile Arg Trp Phe Lys Gly Asn Lys Glu Leu Val
   900 905 910
   Ser Gly Gly Ser Cys Tyr Ile Thr Lys Glu Ala Leu Glu Ser Ser Leu
   915 920 925
   Glu Leu Tyr Leu Val Lys Thr Ser Asp Ser Gly Thr Tyr Thr Cys Lys
   930 935 940
   Val Ser Asn Val Ala Gly Gly Val Glu Cys Ser Ala Asn Leu Phe Val
   945 950 955 960
   Lys Glu Pro Ala Thr Phe Val Glu Lys Leu Glu Pro Ser Gln Leu Leu
   965 970 975
   Lys Lys Gly Asp Ala Thr Gln Leu Ala Cys Lys Val Thr Gly Thr Pro
   980 985 990
   Pro Ile Lys Ile Thr Trp Phe Ala Asn Asp Arg Glu Ile Lys Glu Ser
   995 1000 1005
   Ser Lys His Arg Met Ser Phe Val Glu Ser Thr Ala Val Leu Arg Leu
   1010                1015                1020
   Thr Asp Val Gly Ile Glu Asp Ser Gly Glu Tyr Met Cys Glu Ala Gln
   1025                1030                1035                1040
   Asn Glu Ala Gly Ser Asp His Cys Ser Ser Ile Val Ile Val Lys Glu
                   1045                1050                1055
   Ser Pro Tyr Phe Thr Lys Glu Phe Lys Pro Ile Glu Val Leu Lys Glu
               1060                1065                1070
   Tyr Asp Val Met Leu Leu Ala Glu Val Ala Gly Thr Pro Pro Phe Glu
           1075                1080                1085
   Ile Thr Trp Phe Lys Asp Asn Thr Ile Leu Arg Ser Gly Arg Lys Tyr
       1090                1095                1100
   Lys Thr Phe Ile Gln Asp His Leu Val Ser Leu Gln Ile Leu Lys Phe
   1105                1110                1115                1120
   Val Ala Ala Asp Ala Gly Glu Tyr Gln Cys Arg Val Thr Asn Glu Val
                   1125                1130                1135
   Gly Ser Ser Ile Cys Ser Ala Arg Val Thr Leu Arg Glu Pro Pro Ser
               1140                1145                1150
   Phe Ile Lys Lys Ile Glu Ser Thr Ser Ser Leu Arg Gly Gly Thr Ala
           1155                1160                1165
   Ala Phe Gln Ala Thr Leu Lys Gly Ser Leu Pro Ile Thr Val Thr Trp
       1170                1175                1180
   Leu Lys Asp Ser Asp Glu Ile Thr Glu Asp Asp Asn Ile Arg Met Thr
   1185                1190                1195                1200
   Phe Glu Asn Asn Val Ala Ser Leu Tyr Leu Ser Gly Ile Glu Val Lys
                   1205                1210                1215
   His Asp Gly Lys Tyr Val Cys Gln Ala Lys Asn Asp Ala Gly Ile Gln
               1220                1225                1230
   Arg Cys Ser Ala Leu Leu Ser Val Lys Glu Pro Ala Thr Ile Thr Glu
           1235                1240                1245
   Glu Ala Val Ser Ile Asp Val Thr Gln Gly Asp Pro Ala Thr Leu Gln
       1250                1255                1260
   Val Lys Phe Ser Gly Thr Lys Glu Ile Thr Ala Lys Trp Phe Lys Asp
   1265                1270                1275                1280
   Gly Gln Glu Leu Thr Leu Gly Ser Lys Tyr Lys Ile Ser Val Thr Asp
                   1285                1290                1295
   Thr Val Ser Ile Leu Lys Ile Ile Ser Thr Glu Lys Lys Asp Ser Gly
               1300                1305                1310
   Glu Tyr Thr Phe Glu Val Gln Asn Asp Val Gly Arg Ser Ser Cys Lys
           1315                1320                1325
   Ala Arg Ile Asn Val Leu Asp Leu Ile Ile Pro Pro Ser Phe Thr Lys
       1330                1335                1340
   Lys Leu Lys Lys Met Asp Ser Ile Lys Gly Ser Phe Ile Asp Leu Glu
   1345 1350 1355 1360
   Cys Ile Val Ala Gly Ser His Pro Ile Ser Ile Gln Trp Phe Lys Asp
   1365 1370 1375
   Asp Gln Glu Ile Ser Ala Ser Glu Lys Tyr Lys Phe Ser Phe His Asp
   1380 1385 1390
   Asn Thr Ala Phe Leu Glu Ile Ser Gln Leu Glu Gly Thr Asp Ser Gly
   1395 1400 1405
   Thr Tyr Thr Cys Ser Ala Thr Asn Lys Ala Gly His Asn Gln Cys Ser
   1410 1415 1420
   Gly His Leu Thr Val Lys Glu Pro Pro Tyr Phe Val Glu Lys Pro Gln
   1425 1430 1435 1440
   Ser Gln Asp Val Asn Pro Asn Thr Arg Val Gln Leu Lys Ala Leu Val
   1445 1450 1455
   Gly Gly Thr Ala Pro Met Thr Ile Lys Trp Phe Lys Asp Asn Lys Glu
   1460 1465 1470
   Leu His Ser Gly Ala Ala Arg Ser Val Trp Lys Asp Asp Thr Ser Thr
   1475 1480 1485
   Ser Leu Glu Leu Phe Ala Ala Lys Ala Thr Asp Ser Gly Thr Tyr Ile
   1490 1495 1500
   Cys Gln Leu Ser Asn Asp Val Gly Thr Ala Thr Ser Lys Ala Thr Leu
   1505 1510 1515 1520
   Phe Val Lys Glu Pro Pro Gln Phe Ile Lys Lys Pro Ser Pro Val Leu
   1525 1530 1535
   Val Leu Arg Asn Gly Gln Ser Thr Thr Phe Glu Cys Gln Ile Thr Gly
   1540 1545 1550
   Thr Pro Lys Ile Arg Val Ser Trp Tyr Leu Asp Gly Asn Glu Ile Thr
   1555 1560 1565
   Ala Ile Gln Lys His Gly Ile Ser Phe Ile Asp Gly Leu Ala Thr Phe
   1570 1575 1580
   Gln Ile Ser Gly Ala Arg Val Glu Asn Ser Gly Thr Tyr Val Cys Glu
   1585 1590 1595 1600
   Ala Arg Asn Asp Ala Gly Thr Ala Ser Cys Ser Ile Glu Leu Lys Val
   1605 1610 1615
   Lys Glu Pro Pro Thr Phe Ile Arg Glu Leu Lys Pro Val Glu Val Val
   1620 1625 1630
   Lys Tyr Ser Asp Val Glu Leu Glu Cys Glu Val Thr Gly Thr Pro Pro
   1635 1640 1645
   Phe Glu Val Thr Trp Leu Lys Asn Asn Arg Glu Ile Arg Ser Ser Lys
   1650 1655 1660
   Lys Tyr Thr Leu Thr Asp Arg Val Ser Val Phe Asn Leu His Ile Thr
   1665 1670 1675 1680
   Lys Cys Asp Pro Ser Asp Thr Gly Glu Tyr Gln Cys Ile Val Ser Asn
   1685 1690 1695
   Glu Gly Gly Ser Cys Ser Cys Ser Thr Arg Val Ala Leu Lys Glu Pro
   1700 1705 1710
   Pro Ser Phe Ile Lys Lys Ile Glu Asn Thr Thr Thr Val Leu Lys Ser
   1715 1720 1725
   Ser Ala Thr Phe Gln Ser Thr Val Ala Gly Ser Pro Pro Ile Ser Ile
   1730 1735 1740
   Thr Trp Leu Lys Asp Asp Gln Ile Leu Asp Glu Asp Asp Asn Val Tyr
   1745 1750 1755 1760
   Ile Ser Phe Val Asp Ser Val Ala Thr Leu Gln Ile Arg Ser Val Asp
   1765 1770 1775
   Asn Gly His Ser Gly Arg Tyr Thr Cys Gln Ala Lys Asn Glu Ser Gly
   1780 1785 1790
   Val Glu Arg Cys Tyr Ala Phe Leu Leu Val Gln Glu Pro Ala Gln Ile
   1795 1800 1805
   Val Glu Lys Ala Lys Ser Val Asp Val Thr Glu Lys Asp Pro Met Thr
   1810 1815 1820
   Leu Glu Cys Val Val Ala Gly Thr Pro Glu Leu Lys Val Lys Trp Leu
   1825 1830 1835 1840
   Lys Asp Gly Lys Gln Ile Val Pro Ser Arg Tyr Phe Ser Met Ser Phe
   1845 1850 1855
   Glu Asn Asn Val Ala Ser Phe Arg Ile Gln Ser Val Met Lys Gln Asp
   1860 1865 1870
   Ser Gly Gln Tyr Thr Phe Lys Val Glu Asn Asp Phe Gly Ser Ser Ser
   1875 1880 1885
   Cys Asp Ala Tyr Leu Arg Val Leu Asp Gln Asn Ile Pro Pro Ser Phe
   1890 1895 1900
   Thr Lys Lys Leu Thr Lys Met Asp Lys Val Leu Gly Ser Ser Ile His
   1905 1910 1915 1920
   Met Glu Cys Lys Val Ser Gly Ser Leu Pro Ile Ser Ala Gln Trp Phe
   1925 1930 1935
   Lys Asp Gly Lys Glu Ile Ser Thr Ser Ala Lys Tyr Arg Leu Val Cys
   1940 1945 1950
   His Glu Arg Ser Val Ser Leu Glu Val Asn Asn Leu Glu Leu Glu Asp
   1955 1960 1965
   Thr Ala Asn Tyr Thr Cys Lys Val Ser Asn Val Ala Gly Asp Asp Ala
   1970 1975 1980
   Cys Ser Gly Ile Leu Thr Val Lys Glu Pro Pro Ser Phe Leu Val Lys
   1985 1990 1995 2000
   Pro Gly Arg Gln Gln Ala Ile Pro Asp Ser Thr Val Glu Phe Lys Ala
   2005 2010 2015
   Ile Leu Lys Gly Thr Pro Pro Phe Lys Ile Lys Trp Phe Lys Asp Asp
   2020 2025 2030
   Val Glu Leu Val Ser Gly Pro Lys Cys Phe Ile Gly Leu Glu Gly Ser
   2035 2040 2045
   Thr Ser Phe Leu Asn Leu Tyr Ser Val Asp Ala Ser Lys Thr Gly Gln
   2050 2055 2060
   Tyr Thr Cys His Val Thr Asn Asp Val Gly Ser Asp Ser Cys Thr Thr
   2065 2070 2075 2080
   Met Leu Leu Val Thr Glu Pro Pro Lys Phe Val Lys Lys Leu Glu Ala
   2085 2090 2095
   Ser Lys Ile Val Lys Ala Gly Asp Ser Ser Arg Leu Glu Cys Lys Ile
   2100 2105 2110
   Ala Gly Ser Pro Glu Ile Arg Val Val Trp Phe Arg Asn Glu His Glu
   2115 2120 2125
   Leu Pro Ala Ser Asp Lys Tyr Arg Met Thr Phe Ile Asp Ser Val Ala
   2130 2135 2140
   Val Ile Gln Met Asn Asn Leu Ser Thr Glu Asp Ser Gly Asp Phe Ile
   2145 2150 2155 2160
   Cys Glu Ala Gln Asn Pro Ala Gly Ser Thr Ser Cys Ser Thr Lys Val
   2165 2170 2175
   Ile Val Lys Glu Pro Pro Val Phe Ser Ser Phe Pro Pro Ile Val Glu
   2180 2185 2190
   Thr Leu Lys Asn Ala Glu Val Ser Leu Glu Cys Glu Leu Ser Gly Thr
   2195 2200 2205
   Pro Pro Phe Glu Val Val Trp Tyr Lys Asp Lys Arg Gln Leu Arg Ser
   2210 2215 2220
   Ser Lys Lys Tyr Lys Ile Ala Ser Lys Asn Phe His Thr Ser Ile His
   2225 2230 2235 2240
   Ile Leu Asn Val Asp Thr Ser Asp Ile Gly Glu Tyr His Cys Lys Ala
   2245 2250 2255
   Gln Asn Glu Val Gly Ser Asp Thr Cys Val Cys Thr Val Lys Leu Lys
   2260 2265 2270
   Glu Pro Pro Arg Phe Val Ser Lys Leu Asn Ser Leu Thr Val Val Ala
   2275 2280 2285
   Gly Glu Pro Ala Glu Leu Gln Ala Ser Ile Glu Gly Ala Gln Pro Ile
   2290 2295 2300
   Phe Val Gln Trp Leu Lys Glu Lys Glu Glu Val Ile Arg Glu Ser Glu
   2305 2310 2315 2320
   Asn Ile Arg Ile Thr Phe Val Glu Asn Val Ala Thr Leu Gln Phe Ala
   2325 2330 2335
   Lys Ala Glu Pro Ala Asn Ala Gly Lys Tyr Ile Cys Gln Ile Lys Asn
   2340 2345 2350
   Asp Gly Gly Met Arg Glu Asn Met Ala Thr Leu Met Val Leu Glu Pro
   2355 2360 2365
   Ala Val Ile Val Glu Lys Ala Gly Pro Met Thr Val Thr Val Gly Glu
   2370 2375 2380
   Thr Cys Thr Leu Glu Cys Lys Val Ala Gly Thr Pro Glu Leu Ser Val
   2385 2390 2395 2400
   Glu Trp Tyr Lys Asp Gly Lys Leu Leu Thr Ser Ser Gln Lys His Lys
   2405 2410 2415
   Phe Ser Phe Tyr Asn Lys Ile Ser Ser Leu Arg Ile Leu Ser Val Glu
   2420 2425 2430
   Arg Gln Asp Ala Gly Thr Tyr Thr Phe Gln Val Gln Asn Asn Val Gly
   2435 2440 2445
   Lys Ser Ser Cys Thr Ala Val Val Asp Val Ser Asp Arg Ala Val Pro
   2450 2455 2460
   Pro Ser Phe Thr Arg Arg Leu Lys Asn Thr Gly Gly Val Leu Gly Ala
   2465 2470 2475 2480
   Ser Cys Ile Leu Glu Cys Lys Val Ala Gly Ser Ser Pro Ile Ser Val
   2485 2490 2495
   Ala Trp Phe His Glu Lys Thr Lys Ile Val Ser Gly Ala Lys Tyr Gln
   2500 2505 2510
   Thr Thr Phe Ser Asp Asn Val Cys Thr Leu Gln Leu Asn Ser Leu Asp
   2515 2520 2525
   Ser Ser Asp Met Gly Asn Tyr Thr Cys Val Ala Ala Asn Val Ala Gly
   2530 2535 2540
   Ser Asp Glu Cys Arg Ala Val Leu Thr Val Gln Glu Pro Pro Ser Phe
   2545 2550 2555 2560
   Val Lys Glu Pro Glu Pro Leu Glu Val Leu Pro Gly Lys Asn Val Thr
   2565 2570 2575
   Phe Thr Ser Val Ile Arg Gly Thr Pro Pro Phe Lys Val Asn Trp Phe
   2580 2585 2590
   Arg Gly Ala Arg Glu Leu Val Lys Gly Asp Arg Cys Asn Ile Tyr Phe
   2595 2600 2605
   Glu Asp Thr Val Ala Glu Leu Glu Leu Phe Asn Ile Asp Ile Ser Gln
   2610 2615 2620
   Ser Gly Glu Tyr Thr Cys Val Val Ser Asn Asn Ala Gly Gln Ala Ser
   2625 2630 2635 2640
   Cys Thr Thr Arg Leu Phe Val Lys Glu Pro Ala Ala Phe Leu Lys Arg
   2645 2650 2655
   Leu Ser Asp His Ser Val Glu Pro Gly Lys Ser Ile Ile Leu Glu Ser
   2660 2665 2670
   Thr Tyr Thr Gly Thr Leu Pro Ile Ser Val Thr Trp Lys Lys Asp Gly
   2675 2680 2685
   Phe Asn Ile Thr Thr Ser Glu Lys Cys Asn Ile Val Thr Thr Glu Lys
   2690 2695 2700
   Thr Cys Ile Leu Glu Ile Leu Asn Ser Thr Lys Arg Asp Ala Gly Gln
   2705 2710 2715 2720
   Tyr Ser Cys Glu Ile Glu Asn Glu Ala Gly Arg Asp Val Cys Gly Ala
   2725 2730 2735
   Leu Val Ser Thr Leu Glu Pro Pro Tyr Phe Val Thr Glu Leu Glu Pro
   2740 2745 2750
   Leu Glu Ala Ala Val Gly Asp Ser Val Ser Leu Gln Cys Gln Val Ala
   2755 2760 2765
   Gly Thr Pro Glu Ile Thr Val Ser Trp Tyr Lys Gly Asp Thr Lys Leu
   2770 2775 2780
   Arg Pro Thr Pro Glu Tyr Arg Thr Tyr Phe Thr Asn Asn Val Ala Thr
   2785 2790 2795 2800
   Leu Val Phe Asn Lys Val Asn Ile Asn Asp Ser Gly Glu Tyr Thr Cys
   2805 2810 2815
   Lys Ala Glu Asn Ser Ile Gly Thr Ala Ser Ser Lys Thr Val Phe Arg
   2820 2825 2830
   Ile Gln Glu Arg Gln Leu Pro Pro Ser Phe Ala Arg Gln Leu Lys Asp
   2835 2840 2845
   Ile Glu Gln Thr Val Gly Leu Pro Val Thr Leu Thr Cys Arg Leu Asn
   2850 2855 2860
   Gly Ser Ala Pro Ile Gln Val Cys Trp Tyr Arg Asp Gly Val Leu Leu
   2865 2870 2875 2880
   Arg Asp His Glu Asn Leu Gln Thr Ser Phe Val Asp Asn Val Ala Thr
   2885 2890 2895
   Leu Lys Ile Leu Gln Thr Asp Leu Ser His Ser Gly Gln Tyr Ser Cys
   2900 2905 2910
   Ser Ala Ser Asn Pro Leu Gly Thr Ala Ser Ser Ser Ala Arg Leu Thr
   2915 2920 2925
   Ala Arg Glu Pro Lys Lys Ser Pro Phe Phe Asp Ile Lys Pro Val Ser
   2930 2935 2940
   Ile Asp Val Ile Ala Gly Glu Ser Ala Asp Phe Glu Cys His Val Thr
   2945 2950 2955 2960
   Gly Ala Gln Pro Met Arg Ile Thr Trp Ser Lys Asp Asn Lys Glu Ile
   2965 2970 2975
   Arg Pro Gly Gly Asn Tyr Thr Ile Thr Cys Val Gly Asn Thr Pro His
   2980 2985 2990
   Leu Arg Ile Leu Lys Val Gly Lys Gly Asp Ser Gly Gln Tyr Thr Cys
   2995 3000 3005
   Gln Ala Thr Asn Asp Val Gly Lys Asp Met Cys Ser Ala Gln Leu Ser
   3010 3015 3020
   Val Lys Glu Pro Pro Lys Phe Val Lys Lys Leu Glu Ala Ser Lys Val
   3025 3030 3035 3040
   Ala Lys Gln Gly Glu Ser Ile Gln Leu Glu Cys Lys Ile Ser Gly Ser
   3045 3050 3055
   Pro Glu Ile Lys Val Ser Trp Phe Arg Asn Asp Ser Glu Leu His Glu
   3060 3065 3070
   Ser Trp Lys Tyr Asn Met Ser Phe Ile Asn Ser Val Ala Leu Leu Thr
   3075 3080 3085
   Ile Asn Glu Ala Ser Ala Glu Asp Ser Gly Asp Tyr Ile Cys Glu Ala
   3090 3095 3100
   His Asn Gly Val Gly Asp Ala Ser Cys Ser Thr Ala Leu Thr Val Lys
   3105 3110 3115 3120
   Ala Pro Pro Val Phe Thr Gln Lys Pro Ser Pro Val Gly Ala Leu Lys
   3125 3130 3135
   Gly Ser Asp Val Ile Leu Gln Cys Glu Ile Ser Gly Thr Pro Pro Phe
   3140 3145 3150
   Glu Val Val Trp Val Lys Asp Arg Lys Gln Val Arg Asn Ser Lys Lys
   3155 3160 3165
   Phe Lys Ile Thr Ser Lys His Phe Asp Thr Asn Leu His Ile Leu Asn
   3170 3175 3180
   Leu Glu Ala Ser Asp Val Gly Glu Tyr His Cys Lys Ala Thr Asn Glu
   3185 3190 3195 3200
   Val Gly Ser Asp Thr Cys Ser Cys Ser Val Lys Phe Lys Glu Pro Pro
   3205 3210 3215
   Arg Phe Val Lys Lys Leu Ser Asp Thr Ser Thr Leu Ile Gly Asp Ala
   3220 3225 3230
   Val Glu Leu Arg Ala Ile Val Glu Gly Phe Gln Pro Ile Ser Val Val
   3235 3240 3245
   Trp Leu Lys Asp Arg Gly Glu Val Ile Arg Glu Ser Glu Asn Thr Arg
   3250 3255 3260
   Ile Ser Phe Ile Asp Asn Ile Ala Thr Leu Gln Leu Gly Ser Pro Glu
   3265 3270 3275 3280
   Ala Ser Asn Ser Gly Lys Tyr Ile Cys Gln Ile Lys Asn Asp Ala Gly
   3285 3290 3295
   Met Arg Glu Cys Ser Ala Val Leu Thr Val Leu Glu Pro Ala Arg Ile
   3300 3305 3310
   Ile Glu Lys Pro Glu Pro Met Thr Val Thr Thr Gly Asn Pro Phe Ala
   3315 3320 3325
   Leu Glu Cys Val Val Thr Gly Thr Pro Glu Leu Ser Ala Lys Trp Phe
   3330 3335 3340
   Lys Asp Gly Arg Glu Leu Ser Ala Asp Ser Lys His His Ile Thr Phe
   3345 3350 3355 3360
   Ile Asn Lys Val Ala Ser Leu Lys Ile Pro Cys Ala Glu Met Ser Asp
   3365 3370 3375
   Lys Gly Leu Tyr Ser Phe Glu Val Lys Asn Ser Val Gly Lys Ser Asn
   3380 3385 3390
   Cys Thr Val Ser Val His Val Ser Asp Arg Ile Val Pro Pro Ser Phe
   3395 3400 3405
   Ile Arg Lys Leu Lys Asp Val Asn Ala Ile Leu Gly Ala Ser Val Val
   3410 3415 3420
   Leu Glu Cys Arg Val Ser Gly Ser Ala Pro Ile Ser Val Gly Trp Phe
   3425 3430 3435 3440
   Gln Asp Gly Asn Glu Ile Val Ser Gly Pro Lys Cys Gln Ser Ser Phe
   3445 3450 3455
   Ser Glu Asn Val Cys Thr Leu Asn Leu Ser Leu Leu Glu Pro Ser Asp
   3460 3465 3470
   Thr Gly Ile Tyr Thr Cys Val Ala Ala Asn Val Ala Gly Ser Asp Glu
   3475 3480 3485
   Cys Ser Ala Val Leu Thr Val Gln Glu Pro Pro Ser Phe Glu Gln Thr
   3490 3495 3500
   Pro Asp Ser Val Glu Val Leu Pro Gly Met Ser Leu Thr Phe Thr Ser
   3505 3510 3515 3520
   Val Ile Arg Gly Thr Pro Pro Phe Lys Val Lys Trp Phe Lys Gly Ser
   3525 3530 3535
   Arg Glu Leu Val Pro Gly Glu Ser Cys Asn Ile Ser Leu Glu Asp Phe
   3540 3545 3550
   Val Thr Glu Leu Glu Leu Phe Glu Val Gln Pro Leu Glu Ser Gly Asp
   3555 3560 3565
   Tyr Ser Cys Leu Val Thr Asn Asp Ala Gly Ser Ala Ser Cys Thr Thr
   3570 3575 3580
   His Leu Phe Val Lys Glu Pro Ala Thr Phe Val Lys Arg Leu Ala Asp
   3585 3590 3595 3600
   Phe Ser Val Glu Thr Gly Ser Pro Ile Val Leu Glu Ala Thr Tyr Thr
   3605 3610 3615
   Gly Thr Pro Pro Ile Ser Val Ser Trp Ile Lys Asp Glu Tyr Leu Ile
   3620 3625 3630
   Ser Gln Ser Glu Arg Cys Ser Ile Thr Met Thr Glu Lys Ser Thr Ile
   3635 3640 3645
   Leu Glu Ile Leu Glu Ser Thr Ile Glu Asp Tyr Ala Gln Tyr Ser Cys
   3650 3655 3660
   Leu Ile Glu Asn Glu Ala Gly Gln Asp Ile Cys Glu Ala Leu Val Ser
   3665 3670 3675 3680
   Val Leu Glu Pro Pro Tyr Phe Ile Glu Pro Leu Glu His Val Glu Ala
   3685 3690 3695
   Val Ile Gly Glu Pro Ala Thr Leu Gln Cys Lys Val Asp Gly Thr Pro
   3700 3705 3710
   Glu Ile Arg Ile Ser Trp Tyr Lys Glu His Thr Lys Leu Arg Ser Ala
   3715 3720 3725
   Pro Ala Tyr Lys Met Gln Phe Lys Asn Asn Val Ala Ser Leu Val Ile
   3730 3735 3740
   Asn Lys Val Asp His Ser Asp Val Gly Glu Tyr Ser Cys Lys Ala Asp
   3745 3750 3755 3760
   Asn Ser Val Gly Ala Val Ala Ser Ser Ala Val Leu Val Ile Lys Ala
   3765 3770 3775
   Arg Lys Leu Pro Pro Phe Phe Ala Arg Lys Leu Lys Asp Val His Glu
   3780 3785 3790
   Thr Leu Gly Phe Pro Val Ala Phe Glu Cys Arg Ile Asn Gly Ser Glu
   3795 3800 3805
   Pro Leu Gln Val Ser Trp Tyr Lys Asp Gly Val Leu Leu Lys Asp Asp
   3810 3815 3820
   Ala Asn Leu Gln Thr Ser Phe Val His Asn Val Ala Thr Leu Gln Ile
   3825 3830 3835 3840
   Leu Gln Thr Asp Gln Ser His Ile Gly Gln Tyr Asn Cys Ser Ala Ser
   3845 3850 3855
   Asn Pro Leu Gly Thr Ala Ser Ser Ser Ala Lys Leu Ile Leu Ser Glu
   3860 3865 3870
   His Glu Val Pro Pro Phe Phe Asp Leu Lys Pro Val Ser Val Asp Leu
   3875 3880 3885
   Ala Leu Gly Glu Ser Gly Thr Phe Lys Cys His Val Thr Gly Thr Ala
   3890 3895 3900
   Pro Ile Lys Ile Thr Trp Ala Lys Asp Asn Arg Glu Ile Arg Pro Gly
   3905 3910 3915 3920
   Gly Asn Tyr Lys Met Thr Leu Val Glu Asn Thr Ala Thr Leu Thr Val
   3925 3930 3935
   Leu Lys Val Gly Lys Gly Asp Ala Gly Gln Tyr Thr Cys Tyr Ala Ser
   3940 3945 3950
   Asn Ile Ala Gly Lys Asp Ser Cys Ser Ala Gln Leu Gly Val Gln Glu
   3955 3960 3965
   Pro Pro Arg Phe Ile Lys Lys Leu Glu Pro Ser Arg Ile Val Lys Gln
   3970 3975 3980
   Asp Glu Phe Thr Arg Tyr Glu Cys Lys Ile Gly Gly Ser Pro Glu Ile
   3985 3990 3995 4000
   Lys Val Leu Trp Tyr Lys Asp Glu Thr Glu Ile Gln Glu Ser Ser Lys
   4005 4010 4015
   Phe Arg Met Ser Phe Val Asp Ser Val Ala Val Leu Glu Met His Asn
   4020 4025 4030
   Leu Ser Val Glu Asp Ser Gly Asp Tyr Thr Cys Glu Ala His Asn Ala
   4035 4040 4045
   Ala Gly Ser Ala Ser Ser Ser Thr Ser Leu Lys Val Lys Glu Pro Pro
   4050 4055 4060
   Ile Phe Arg Lys Lys Pro His Pro Ile Glu Thr Leu Lys Gly Ala Asp
   4065 4070 4075 4080
   Val His Leu Glu Cys Glu Leu Gln Gly Thr Pro Pro Phe His Val Ser
   4085 4090 4095
   Trp Tyr Lys Asp Lys Arg Glu Leu Arg Ser Gly Lys Lys Tyr Lys Ile
   4100 4105 4110
   Met Ser Glu Asn Phe Leu Thr Ser Ile His Ile Leu Asn Val Asp Ala
   4115 4120 4125
   Ala Asp Ile Gly Glu Tyr Gln Cys Lys Ala Thr Asn Asp Val Gly Ser
   4130 4135 4140
   Asp Thr Cys Val Gly Ser Ile Ala Leu Lys Ala Pro Pro Arg Phe Val
   4145 4150 4155 4160
   Lys Lys Leu Ser Asp Ile Ser Thr Val Val Gly Lys Glu Val Gln Leu
   4165 4170 4175
   Gln Thr Thr Ile Glu Gly Ala Glu Pro Ile Ser Val Val Trp Phe Lys
   4180 4185 4190
   Asp Lys Gly Glu Ile Val Arg Glu Ser Asp Asn Ile Trp Ile Ser Tyr
   4195 4200 4205
   Ser Glu Asn Ile Ala Thr Leu Gln Phe Ser Arg Val Glu Pro Ala Asn
   4210 4215 4220
   Ala Gly Lys Tyr Thr Cys Gln Ile Lys Asn Asp Ala Gly Met Gln Glu
   4225 4230 4235 4240
   Cys Phe Ala Thr Leu Ser Val Leu Glu Pro Ala Thr Ile Val Glu Lys
   4245 4250 4255
   Pro Glu Ser Ile Lys Val Thr Thr Gly Asp Thr Cys Thr Leu Glu Cys
   4260 4265 4270
   Thr Val Ala Gly Thr Pro Glu Leu Ser Thr Lys Trp Phe Lys Asp Gly
   4275 4280 4285
   Lys Glu Leu Thr Ser Asp Asn Lys Tyr Lys Ile Ser Phe Phe Asn Lys
   4290 4295 4300
   Val Ser Gly Leu Lys Ile Ile Asn Val Ala Pro Ser Asp Ser Gly Val
   4305 4310 4315 4320
   Tyr Ser Phe Glu Val Gln Asn Pro Val Gly Lys Asp Ser Cys Thr Ala
   4325 4330 4335
   Ser Leu Gln Val Ser Asp Arg Thr Val Pro Pro Ser Phe Thr Arg Lys
   4340 4345 4350
   Leu Lys Glu Thr Asn Gly Leu Ser Gly Ser Ser Val Val Met Glu Cys
   4355 4360 4365
   Lys Val Tyr Gly Ser Pro Pro Ile Ser Val Ser Trp Phe His Glu Gly
   4370 4375 4380
   Asn Glu Ile Ser Ser Gly Arg Lys Tyr Gln Thr Thr Leu Thr Asp Asn
   4385 4390 4395 4400
   Thr Cys Ala Leu Thr Val Asn Met Leu Glu Glu Ser Asp Ser Gly Asp
   4405 4410 4415
   Tyr Thr Cys Ile Ala Thr Asn Met Ala Gly Ser Asp Glu Cys Ser Ala
   4420 4425 4430
   Pro Leu Thr Val Arg Glu Pro Pro Ser Phe Val Gln Lys Pro Asp Pro
   4435 4440 4445
   Met Asp Val Leu Thr Gly Thr Asn Val Thr Phe Thr Ser Ile Val Lys
   4450 4455 4460
   Gly Thr Pro Pro Phe Ser Val Ser Trp Phe Lys Gly Ser Ser Glu Leu
   4465 4470 4475 4480
   Val Pro Gly Asp Arg Cys Asn Val Ser Leu Glu Asp Ser Val Ala Glu
   4485 4490 4495
   Leu Glu Leu Phe Asp Val Asp Thr Ser Gln Ser Gly Glu Tyr Thr Cys
   4500 4505 4510
   Ile Val Ser Asn Glu Ala Gly Lys Ala Ser Cys Thr Thr His Leu Tyr
   4515 4520 4525
   Ile Lys Ala Pro Ala Lys Phe Val Lys Arg Leu Asn Asp Tyr Ser Ile
   4530 4535 4540
   Glu Lys Gly Lys Pro Leu Ile Leu Glu Gly Thr Phe Thr Gly Thr Pro
   4545 4550 4555 4560
   Pro Ile Ser Val Thr Trp Lys Lys Asn Gly Ile Asn Val Thr Pro Ser
   4565 4570 4575
   Gln Arg Cys Asn Ile Thr Thr Thr Glu Lys Ser Pro Ile Leu Glu Ile
   4580 4585 4590
   Pro Ser Ser Thr Val Glu Asp Ala Gly Gln Tyr Asn Cys Tyr Ile Glu
   4595 4600 4605
   Asn Ala Ser Gly Lys Asp Ser Cys Ser Ala Gln Ile Leu Ile Leu Glu
   4610 4615 4620
   Pro Pro Tyr Phe Val Lys Gln Leu Glu Pro Val Lys Val Ser Val Gly
   4625 4630 4635 4640
   Asp Ser Ala Ser Leu Gln Cys Gln Leu Ala Gly Thr Pro Glu Ile Gly
   4645 4650 4655
   Val Ser Trp Tyr Lys Gly Asp Thr Lys Leu Arg Pro Thr Thr Thr Tyr
   4660 4665 4670
   Lys Met His Phe Arg Asn Asn Val Ala Thr Leu Val Phe Asn Gln Val
   4675 4680 4685
   Asp Ile Asn Asp Ser Gly Glu Tyr Ile Cys Lys Ala Glu Asn Ser Val
   4690 4695 4700
   Gly Glu Val Ser Ala Ser Thr Phe Leu Thr Val Gln Glu Gln Lys Leu
   4705 4710 4715 4720
   Pro Pro Ser Phe Ser Arg Gln Leu Arg Asp Val Gln Glu Thr Val Gly
   4725 4730 4735
   Leu Pro Val Val Phe Asp Cys Ala Ile Ser Gly Ser Glu Pro Ile Ser
   4740 4745 4750
   Val Ser Trp Tyr Lys Asp Gly Lys Pro Leu Lys Asp Ser Pro Asn Val
   4755 4760 4765
   Gln Thr Ser Phe Leu Asp Asn Thr Ala Thr Leu Asn Ile Phe Lys Thr
   4770 4775 4780
   Asp Arg Ser Leu Ala Gly Gln Tyr Ser Cys Thr Ala Thr Asn Pro Ile
   4785 4790 4795 4800
   Gly Ser Ala Ser Ser Ser Ala Arg Leu Ile Leu Thr Glu Gly Lys Asn
   4805 4810 4815
   Pro Pro Phe Phe Asp Ile Arg Leu Ala Pro Val Asp Ala Val Val Gly
   4820 4825 4830
   Glu Ser Ala Asp Phe Glu Cys His Val Thr Gly Thr Gln Pro Ile Lys
   4835 4840 4845
   Val Ser Trp Ala Lys Asp Ser Arg Glu Ile Arg Ser Gly Gly Lys Tyr
   4850 4855 4860
   Gln Ile Ser Tyr Leu Glu Asn Ser Ala His Leu Thr Val Leu Lys Val
   4865 4870 4875 4880
   Asp Lys Gly Asp Ser Gly Gln Tyr Thr Cys Tyr Ala Val Asn Glu Val
   4885 4890 4895
   Gly Lys Asp Ser Cys Thr Ala Gln Leu Asn Ile Lys Glu Arg Leu Ile
   4900 4905 4910
   Pro Pro Ser Phe Thr Lys Arg Leu Ser Glu Thr Val Glu Glu Thr Glu
   4915 4920 4925
   Gly Asn Ser Phe Lys Leu Glu Gly Arg Val Ala Gly Ser Gln Pro Ile
   4930 4935 4940
   Thr Val Ala Trp Tyr Lys Asn Asn Ile Glu Ile Gln Pro Thr Ser Asn
   4945 4950 4955 4960
   Cys Glu Ile Thr Phe Lys Asn Asn Thr Leu Val Leu Gln Val Arg Lys
   4965 4970 4975
   Ala Gly Met Asn Asp Ala Gly Leu Tyr Thr Cys Lys Val Ser Asn Asp
   4980 4985 4990
   Ala Gly Ser Ala Leu Cys Thr Ser Ser Ile Val Ile Lys Glu Pro Lys
   4995 5000 5005
   Lys Pro Pro Val Phe Asp Gln His Leu Thr Pro Val Thr Val Ser Glu
   5010 5015 5020
   Gly Glu Tyr Val Gln Leu Ser Cys His Val Gln Gly Ser Glu Pro Ile
   5025 5030 5035 5040
   Arg Ile Gln Trp Leu Lys Ala Gly Arg Glu Ile Lys Pro Ser Asp Arg
   5045 5050 5055
   Cys Ser Phe Ser Phe Ala Ser Gly Thr Ala Val Leu Glu Leu Arg Asp
   5060 5065 5070
   Val Ala Lys Ala Asp Ser Gly Asp Tyr Val Cys Lys Ala Ser Asn Val
   5075 5080 5085
   Ala Gly Ser Asp Thr Thr Lys Ser Lys Val Thr Ile Lys Asp Lys Pro
   5090 5095 5100
   Ala Val Ala Pro Ala Thr Lys Lys Ala Ala Val Asp Gly Arg Leu Phe
   5105 5110 5115 5120
   Phe Val Ser Glu Pro Gln Ser Ile Arg Val Val Glu Lys Thr Thr Ala
   5125 5130 5135
   Thr Phe Ile Ala Lys Val Gly Gly Asp Pro Ile Pro Asn Val Lys Trp
   5140 5145 5150
   Thr Lys Gly Lys Trp Arg Gln Leu Asn Gln Gly Gly Arg Val Phe Ile
   5155 5160 5165
   His Gln Lys Gly Asp Glu Ala Lys Leu Glu Ile Arg Asp Thr Thr Lys
   5170 5175 5180
   Thr Asp Ser Gly Leu Tyr Arg Cys Val Ala Phe Asn Glu His Gly Glu
   5185 5190 5195 5200
   Ile Glu Ser Asn Val Asn Leu Gln Val Asp Glu Arg Lys Lys Gln Glu
   5205 5210 5215
   Lys Ile Glu Gly Asp Leu Arg Ala Met Leu Lys Lys Thr Pro Ile Leu
   5220 5225 5230
   Lys Lys Gly Ala Gly Glu Glu Glu Glu Ile Asp Ile Met Glu Leu Leu
   5235 5240 5245
   Lys Asn Val Asp Pro Lys Glu Tyr Glu Lys Tyr Ala Arg Met Tyr Gly
   5250 5255 5260
   Ile Thr Asp Phe Arg Gly Leu Leu Gln Ala Phe Glu Leu Leu Lys Gln
   5265 5270 5275 5280
   Ser Gln Glu Glu Glu Thr His Arg Leu Glu Ile Glu Glu Ile Glu Arg
   5285 5290 5295
   Ser Glu Arg Asp Glu Lys Glu Phe Glu Glu Leu Val Ser Phe Ile Gln
   5300 5305 5310
   Gln Arg Leu Ser Gln Thr Glu Pro Val Thr Leu Ile Lys Asp Ile Glu
   5315 5320 5325
   Asn Gln Thr Val Leu Lys Asp Asn Asp Ala Val Phe Glu Ile Asp Ile
   5330 5335 5340
   Lys Ile Asn Tyr Pro Glu Ile Lys Leu Ser Trp Tyr Lys Gly Thr Glu
   5345 5350 5355 5360
   Lys Leu Glu Pro Ser Asp Lys Phe Glu Ile Ser Ile Asp Gly Asp Arg
   5365 5370 5375
   His Thr Leu Arg Val Lys Asn Cys Gln Leu Lys Asp Gln Gly Asn Tyr
   5380 5385 5390
   Arg Leu Val Cys Gly Pro His Ile Ala Ser Ala Lys Leu Thr Val Ile
   5395 5400 5405
   Glu Pro Ala Trp Glu Arg His Leu Gln Asp Val Thr Leu Lys Glu Gly
   5410 5415 5420
   Gln Thr Cys Thr Met Thr Val Gln Phe Ser Val Pro Asn Val Lys Ser
   5425 5430 5435 5440
   Glu Trp Phe Arg Asn Gly Arg Ile Leu Lys Pro Gln Gly Arg His Lys
   5445 5450 5455
   Thr Glu Val Glu His Lys Val His Lys Leu Thr Ile Ala Asp Val Arg
   5460 5465 5470
   Ala Glu Asp Gln Gly Gln Tyr Thr Cys Lys Tyr Glu Asp Leu Glu Thr
   5475 5480 5485
   Ser Ala Glu Leu Arg Ile Glu Ala Glu Pro Ile Gln Phe Thr Lys Arg
   5490 5495 5500
   Ile Gln Asn Ile Val Val Ser Glu His Gln Ser Ala Thr Phe Glu Cys
   5505 5510 5515 5520
   Glu Val Ser Phe Asp Asp Ala Ile Val Thr Trp Tyr Lys Gly Pro Thr
   5525 5530 5535
   Glu Leu Thr Glu Ser Gln Lys Tyr Asn Phe Arg Asn Asp Gly Arg Cys
   5540 5545 5550
   His Tyr Met Thr Ile His Asn Val Thr Pro Asp Asp Glu Gly Val Tyr
   5555 5560 5565
   Ser Val Ile Ala Arg Leu Glu Pro Arg Gly Glu Ala Arg Ser Thr Ala
   5570 5575 5580
   Glu Leu Tyr Leu Thr Thr Lys Glu Ile Lys Leu Glu Leu Lys Pro Pro
   5585 5590 5595 5600
   Asp Ile Pro Asp Ser Arg Val Pro Ile Pro Thr Met Pro Ile Arg Ala
   5605 5610 5615
   Val Pro Pro Glu Glu Ile Pro Pro Val Val Ala Pro Pro Val Pro Leu
   5620 5625 5630
   Leu Leu Pro Thr Pro Glu Glu Lys Lys Pro Pro Pro Lys Arg Ile Glu
   5635 5640 5645
   Val Thr Lys Lys Ala Val Lys Lys Asp Ala Lys Lys Val Val Ala Lys
   5650 5655 5660
   Pro Lys Glu Met Thr Pro Arg Glu Glu Ile Val Lys Lys Pro Pro Pro
   5665 5670 5675 5680
   Pro Thr Thr Leu Ile Pro Ala Lys Ala Pro Glu Ile Ile Asp Val Ser
   5685 5690 5695
   Ser Lys Ala Glu Glu Val Lys Ile Met Thr Ile Thr Arg Lys Lys Glu
   5700 5705 5710
   Val Gln Lys Glu Lys Glu Ala Val Tyr Glu Lys Lys Gln Ala Val His
   5715 5720 5725
   Lys Glu Lys Arg Val Phe Ile Glu Ser Phe Glu Glu Pro Tyr Asp Glu
   5730 5735 5740
   Leu Glu Val Glu Pro Tyr Thr Glu Pro Phe Glu Gln Pro Tyr Tyr Glu
   5745 5750 5755 5760
   Glu Pro Asp Glu Asp Tyr Glu Glu Ile Lys Val Glu Ala Lys Lys Glu
   5765 5770 5775
   Val His Glu Glu Trp Glu Glu Asp Phe Glu Glu Gly Gln Glu Tyr Tyr
   5780 5785 5790
   Glu Arg Glu Glu Gly Tyr Asp Glu Gly Glu Glu Glu Trp Glu Glu Ala
   5795 5800 5805
   Tyr Gln Glu Arg Glu Val Ile Gln Val Gln Lys Glu Val Tyr Glu Glu
   5810 5815 5820
   Ser His Glu Arg Lys Val Pro Ala Lys Val Pro Glu Lys Lys Ala Pro
   5825 5830 5835 5840
   Pro Pro Pro Lys Val Ile Lys Lys Pro Val Ile Glu Lys Ile Glu Lys
   5845 5850 5855
   Thr Ser Arg Arg Met Glu Glu Glu Lys Val Gln Val Thr Lys Val Pro
   5860 5865 5870
   Glu Val Ser Lys Lys Ile Val Pro Gln Lys Pro Ser Arg Thr Pro Val
   5875 5880 5885
   Gln Glu Glu Val Ile Glu Val Lys Val Pro Ala Val His Thr Lys Lys
   5890 5895 5900
   Met Val Ile Ser Glu Glu Lys Met Phe Phe Ala Ser His Thr Glu Glu
   5905 5910 5915 5920
   Glu Val Ser Val Thr Val Pro Glu Val Gln Lys Glu Ile Val Thr Glu
   5925 5930 5935
   Glu Lys Ile His Val Ala Val Ser Lys Arg Val Glu Pro Pro Pro Lys
   5940 5945 5950
   Val Pro Glu Leu Pro Glu Lys Pro Ala Pro Glu Glu Val Ala Pro Val
   5955 5960 5965
   Pro Ile Pro Lys Lys Val Glu Pro Pro Ala Pro Lys Val Pro Glu Val
   5970 5975 5980
   Pro Lys Lys Pro Val Pro Glu Glu Lys Lys Pro Val Pro Val Pro Lys
   5985 5990 5995 6000
   Lys Glu Pro Ala Ala Pro Pro Lys Val Pro Glu Val Pro Lys Lys Pro
   6005 6010 6015
   Val Pro Glu Glu Lys Ile Pro Val Pro Val Ala Lys Lys Lys Glu Ala
   6020 6025 6030
   Pro Pro Ala Lys Val Pro Glu Val Gln Lys Gly Val Val Thr Glu Glu
   6035 6040 6045
   Lys Ile Thr Ile Val Thr Gln Arg Glu Glu Ser Pro Pro Pro Ala Val
   6050 6055 6060
   Pro Glu Ile Pro Lys Lys Lys Val Pro Glu Glu Arg Lys Pro Val Pro
   6065 6070 6075 6080
   Arg Lys Glu Glu Glu Val Pro Pro Pro Pro Lys Val Pro Ala Leu Pro
   6085 6090 6095
   Lys Lys Pro Val Pro Glu Glu Lys Val Ala Val Pro Val Pro Val Ala
   6100 6105 6110
   Lys Lys Ala Pro Pro Pro Arg Ala Glu Val Ser Lys Lys Thr Val Val
   6115 6120 6125
   Glu Glu Lys Arg Phe Val Ala Glu Glu Lys Leu Ser Phe Ala Val Pro
   6130 6135 6140
   Gln Arg Val Glu Val Thr Arg His Glu Val Ser Ala Glu Glu Glu Trp
   6145 6150 6155 6160
   Ser Tyr Ser Glu Glu Glu Glu Gly Val Ser Ile Ser Val Tyr Arg Glu
   6165 6170 6175
   Glu Glu Arg Glu Glu Glu Glu Glu Ala Glu Val Thr Glu Tyr Glu Val
   6180 6185 6190
   Met Glu Glu Pro Glu Glu Tyr Val Val Glu Glu Lys Leu His Ile Ile
   6195 6200 6205
   Ser Lys Arg Val Glu Ala Glu Pro Ala Glu Val Thr Glu Arg Gln Glu
   6210 6215 6220
   Lys Lys Ile Val Leu Lys Pro Lys Ile Pro Ala Lys Ile Glu Glu Pro
   6225 6230 6235 6240
   Pro Pro Ala Lys Val Pro Glu Ala Pro Lys Lys Ile Val Pro Glu Lys
   6245 6250 6255
   Lys Val Pro Ala Pro Val Pro Lys Lys Glu Lys Val Pro Pro Pro Lys
   6260 6265 6270
   Val Pro Glu Glu Pro Lys Lys Pro Val Pro Glu Lys Lys Val Pro Pro
   6275 6280 6285
   Lys Val Ile Lys Met Glu Glu Pro Leu Pro Ala Lys Val Thr Glu Lys
   6290 6295 6300
   His Met Gln Ile Thr Gln Glu Glu Lys Val Leu Val Ala Val Thr Lys
   6305 6310 6315 6320
   Lys Glu Ala Pro Pro Lys Ala Arg Val Pro Glu Glu Pro Lys Arg Ala
   6325 6330 6335
   Val Pro Glu Glu Lys Val Leu Lys Leu Lys Pro Lys Arg Glu Glu Glu
   6340 6345 6350
   Pro Pro Ala Lys Val Thr Glu Phe Arg Lys Arg Val Val Lys Glu Glu
   6355 6360 6365
   Lys Val Ser Ile Glu Ala Pro Lys Arg Glu Pro Gln Pro Ile Lys Glu
   6370 6375 6380
   Val Thr Ile Met Glu Glu Lys Glu Arg Ala Tyr Thr Leu Glu Glu Glu
   6385 6390 6395 6400
   Ala Val Ser Val Gln Arg Glu Glu Glu Tyr Glu Glu Tyr Glu Glu Tyr
   6405 6410 6415
   Asp Tyr Lys Glu Phe Glu Glu Tyr Glu Pro Thr Glu Glu Tyr Asp Gln
   6420 6425 6430
   Tyr Glu Glu Tyr Glu Glu Arg Glu Tyr Glu Arg Tyr Glu Glu His Glu
   6435 6440 6445
   Glu Tyr Ile Thr Glu Pro Glu Lys Pro Ile Pro Val Lys Pro Val Pro
   6450 6455 6460
   Glu Glu Pro Val Pro Thr Lys Pro Lys Ala Pro Pro Ala Lys Val Leu
   6465 6470 6475 6480
   Lys Lys Ala Val Pro Glu Glu Lys Val Pro Val Pro Ile Pro Lys Lys
   6485 6490 6495
   Leu Lys Pro Pro Pro Pro Lys Val Pro Glu Glu Pro Lys Lys Val Phe
   6500 6505 6510
   Glu Glu Lys Ile His Ile Ser Ile Thr Lys Arg Glu Lys Glu Gln Val
   6515 6520 6525
   Thr Glu Pro Ala Ala Lys Val Pro Met Lys Pro Lys Arg Val Val Ala
   6530 6535 6540
   Glu Glu Lys Val Pro Val Pro Arg Lys Glu Val Ala Pro Pro Val Arg
   6545 6550 6555 6560
   Val Pro Glu Val Pro Lys Glu Leu Glu Pro Glu Glu Val Ala Phe Glu
   6565 6570 6575
   Glu Glu Val Val Thr His Val Glu Glu Tyr Leu Val Glu Glu Glu Glu
   6580 6585 6590
   Glu Tyr Ile His Glu Glu Glu Glu Phe Ile Thr Glu Glu Glu Val Val
   6595 6600 6605
   Pro Val Ile Pro Val Lys Val Pro Glu Val Pro Arg Lys Pro Val Pro
   6610 6615 6620
   Glu Glu Lys Lys Pro Val Pro Val Pro Lys Lys Lys Glu Ala Pro Pro
   6625 6630 6635 6640
   Ala Lys Val Pro Glu Val Pro Lys Lys Pro Glu Glu Lys Val Pro Val
   6645 6650 6655
   Leu Ile Pro Lys Lys Glu Lys Pro Pro Pro Ala Lys Val Pro Glu Val
   6660 6665 6670
   Pro Lys Lys Pro Val Pro Glu Glu Lys Val Pro Val Pro Val Pro Lys
   6675 6680 6685
   Lys Val Glu Ala Pro Pro Ala Lys Val Pro Glu Val Pro Lys Lys Pro
   6690 6695 6700
   Val Pro Glu Lys Lys Val Pro Val Pro Ala Pro Lys Lys Val Glu Ala
   6705 6710 6715 6720
   Pro Pro Ala Lys Val Pro Glu Val Pro Lys Lys Leu Ile Pro Glu Glu
   6725 6730 6735
   Lys Lys Pro Thr Pro Val Pro Lys Lys Val Glu Ala Pro Pro Pro Lys
   6740 6745 6750
   Val Pro Lys Lys Arg Glu Pro Val Pro Val Pro Val Ala Leu Pro Gln
   6755 6760 6765
   Glu Glu Glu Val Leu Phe Glu Glu Glu Ile Val Pro Glu Glu Glu Val
   6770 6775 6780
   Leu Pro Glu Glu Glu Glu Val Leu Pro Glu Glu Glu Glu Val Leu Pro
   6785 6790 6795 6800
   Glu Glu Glu Glu Val Leu Pro Glu Glu Glu Glu Ile Pro Pro Glu Glu
   6805 6810 6815
   Glu Glu Val Pro Pro Glu Glu Glu Tyr Val Pro Glu Glu Glu Glu Phe
   6820 6825 6830
   Val Pro Glu Glu Glu Val Leu Pro Glu Val Lys Pro Lys Val Pro Val
   6835 6840 6845
   Pro Ala Pro Val Pro Glu Ile Lys Lys Lys Val Thr Glu Lys Lys Val
   6850 6855 6860
   Val Ile Pro Lys Lys Glu Glu Ala Pro Pro Ala Lys Val Pro Glu Val
   6865 6870 6875 6880
   Pro Lys Lys Val Glu Glu Lys Arg Ile Ile Leu Pro Lys Glu Glu Glu
   6885 6890 6895
   Val Leu Pro Val Glu Val Thr Glu Glu Pro Glu Glu Glu Pro Ile Ser
   6900 6905 6910
   Glu Glu Glu Ile Pro Glu Glu Pro Pro Ser Ile Glu Glu Val Glu Glu
   6915 6920 6925
   Val Ala Pro Pro Arg Val Pro Glu Val Ile Lys Lys Ala Val Pro Glu
   6930 6935 6940
   Ala Pro Thr Pro Val Pro Lys Lys Val Glu Ala Pro Pro Ala Lys Val
   6945 6950 6955 6960
   Ser Lys Lys Ile Pro Glu Glu Lys Val Pro Val Pro Val Gln Lys Lys
   6965 6970 6975
   Glu Ala Pro Pro Ala Lys Val Pro Glu Val Pro Lys Lys Val Pro Glu
   6980 6985 6990
   Lys Lys Val Leu Val Pro Lys Lys Glu Ala Val Pro Pro Ala Lys Gly
   6995 7000 7005
   Arg Thr Val Leu Glu Glu Lys Val Ser Val Ala Phe Arg Gln Glu Val
   7010 7015 7020
   Val Val Lys Glu Arg Leu Glu Leu Glu Val Val Glu Ala Glu Val Glu
   7025 7030 7035 7040
   Glu Ile Pro Glu Glu Glu Glu Phe His Glu Val Glu Glu Tyr Phe Glu
   7045 7050 7055
   Glu Gly Glu Phe His Glu Val Glu Glu Phe Ile Lys Leu Glu Gln His
   7060 7065 7070
   Arg Val Glu Glu Glu His Arg Val Glu Lys Val His Arg Val Ile Glu
   7075 7080 7085
   Val Phe Glu Ala Glu Glu Val Glu Val Phe Glu Lys Pro Lys Ala Pro
   7090 7095 7100
   Pro Lys Gly Pro Glu Ile Ser Glu Lys Ile Ile Pro Pro Lys Lys Pro
   7105 7110 7115 7120
   Pro Thr Lys Val Val Pro Arg Lys Glu Pro Pro Ala Lys Val Pro Glu
   7125 7130 7135
   Val Pro Lys Lys Ile Val Val Glu Glu Lys Val Arg Val Pro Glu Glu
   7140 7145 7150
   Pro Arg Val Pro Pro Thr Lys Val Pro Glu Val Leu Pro Pro Lys Glu
   7155 7160 7165
   Val Val Pro Glu Lys Lys Val Pro Val Pro Pro Ala Lys Lys Pro Glu
   7170 7175 7180
   Ala Pro Pro Pro Lys Val Pro Glu Ala Pro Lys Glu Val Val Pro Glu
   7185 7190 7195 7200
   Lys Lys Val Pro Val Pro Pro Pro Lys Lys Pro Glu Val Pro Pro Thr
   7205 7210 7215
   Lys Val Pro Glu Val Pro Lys Ala Ala Val Pro Glu Lys Lys Val Pro
   7220 7225 7230
   Glu Ala Ile Pro Pro Lys Pro Glu Ser Pro Pro Pro Glu Val Phe Glu
   7235 7240 7245
   Glu Pro Glu Glu Ser Pro Ser Ala Pro Pro Lys Lys Pro Glu Val Pro
   7250 7255 7260
   Pro Val Arg Val Pro Glu Val Pro Lys Glu Val Val Pro Glu Lys Lys
   7265 7270 7275 7280
   Val Pro Ala Ala Pro Pro Lys Lys Pro Glu Val Thr Pro Val Lys Val
   7285 7290 7295
   Pro Glu Ala Pro Lys Glu Val Val Pro Glu Lys Lys Val Pro Val Pro
   7300 7305 7310
   Pro Pro Lys Lys Pro Glu Val Pro Pro Thr Lys Val Pro Glu Val Pro
   7315 7320 7325
   Lys Val Ala Val Pro Glu Lys Lys Val Pro Glu Ala Ile Pro Pro Lys
   7330 7335 7340
   Pro Glu Ser Pro Pro Pro Glu Val Phe Glu Glu Pro Glu Glu Val Ala
   7345 7350 7355 7360
   Leu Glu Glu Pro Pro Ala Glu Val Val Glu Glu Pro Glu Pro Ala Ala
   7365 7370 7375
   Pro Pro Gln Val Thr Val Pro Pro Lys Asn Pro Val Pro Glu Lys Lys
   7380 7385 7390
   Ala Pro Ala Val Val Ala Lys Lys Pro Glu Leu Pro Pro Val Lys Val
   7395 7400 7405
   Pro Glu Val Pro Lys Glu Val Val Pro Glu Lys Lys Val Pro Leu Val
   7410 7415 7420
   Val Pro Lys Lys Pro Glu Ala Pro Pro Ala Lys Val Pro Glu Val Pro
   7425 7430 7435 7440
   Lys Glu Val Val Pro Glu Lys Lys Val Ala Val Pro Lys Lys Pro Glu
   7445 7450 7455
   Val Pro Pro Ala Lys Val Pro Glu Val Pro Lys Lys Pro Val Leu Glu
   7460 7465 7470
   Glu Lys Pro Ala Val Pro Val Pro Glu Arg Ala Glu Ser Pro Pro Pro
   7475 7480 7485
   Glu Val Tyr Glu Glu Pro Glu Glu Ile Ala Pro Glu Glu Glu Ile Ala
   7490 7495 7500
   Pro Glu Glu Glu Lys Pro Val Pro Val Ala Glu Glu Glu Glu Pro Glu
   7505 7510 7515 7520
   Val Pro Pro Pro Ala Val Pro Glu Glu Pro Lys Lys Ile Ile Pro Glu
   7525 7530 7535
   Lys Lys Val Pro Val Ile Lys Lys Pro Glu Ala Pro Pro Pro Lys Glu
   7540 7545 7550
   Pro Glu Pro Glu Lys Val Ile Glu Lys Pro Lys Leu Lys Pro Arg Pro
   7555 7560 7565
   Pro Pro Pro Pro Pro Ala Pro Pro Lys Glu Asp Val Lys Glu Lys Ile
   7570 7575 7580
   Phe Gln Leu Lys Ala Ile Pro Lys Lys Lys Val Pro Glu Asn Pro Gln
   7585 7590 7595 7600
   Val Pro Glu Lys Val Glu Leu Thr Pro Leu Lys Val Pro Gly Gly Glu
   7605 7610 7615
   Lys Lys Val Arg Lys Leu Leu Pro Glu Arg Lys Pro Glu Pro Lys Glu
   7620 7625 7630
   Glu Val Val Leu Lys Ser Val Leu Arg Lys Arg Pro Glu Glu Glu Glu
   7635 7640 7645
   Pro Lys Val Glu Pro Lys Lys Leu Glu Lys Val Lys Lys Pro Ala Val
   7650 7655 7660
   Pro Glu Pro Pro Pro Pro Lys Pro Val Glu Glu Val Glu Val Pro Thr
   7665 7670 7675 7680
   Val Thr Lys Arg Glu Arg Lys Ile Pro Glu Pro Thr Lys Val Pro Glu
   7685 7690 7695
   Ile Lys Pro Ala Ile Pro Leu Pro Ala Pro Glu Pro Lys Pro Lys Pro
   7700 7705 7710
   Glu Ala Glu Val Lys Thr Ile Lys Pro Pro Pro Val Glu Pro Glu Pro
   7715 7720 7725
   Thr Pro Ile Ala Ala Pro Val Thr Val Pro Val Val Gly Lys Lys Ala
   7730 7735 7740
   Glu Ala Lys Ala Pro Lys Glu Glu Ala Ala Lys Pro Lys Gly Pro Ile
   7745 7750 7755 7760
   Lys Gly Val Pro Lys Lys Thr Pro Ser Pro Ile Glu Ala Glu Arg Arg
   7765 7770 7775
   Lys Leu Arg Pro Gly Ser Gly Gly Glu Lys Pro Pro Asp Glu Ala Pro
   7780 7785 7790
   Phe Thr Tyr Gln Leu Lys Ala Val Pro Leu Lys Phe Val Lys Glu Ile
   7795 7800 7805
   Lys Asp Ile Ile Leu Thr Glu Ser Glu Phe Val Gly Ser Ser Ala Ile
   7810 7815 7820
   Phe Glu Cys Leu Val Ser Pro Ser Thr Ala Ile Thr Thr Trp Met Lys
   7825 7830 7835 7840
   Asp Gly Ser Asn Ile Arg Glu Ser Pro Lys His Arg Phe Ile Ala Asp
   7845 7850 7855
   Gly Lys Asp Arg Lys Leu His Ile Ile Asp Val Gln Leu Ser Asp Ala
   7860 7865 7870
   Gly Glu Tyr Thr Cys Val Leu Arg Leu Gly Asn Lys Glu Lys Thr Ser
   7875 7880 7885
   Thr Ala Lys Leu Val Val Glu Glu Leu Pro Val Arg Phe Val Lys Thr
   7890 7895 7900
   Leu Glu Glu Glu Val Thr Val Val Lys Gly Gln Pro Leu Tyr Leu Ser
   7905 7910 7915 7920
   Cys Glu Leu Asn Lys Glu Arg Asp Val Val Trp Arg Lys Asp Gly Lys
   7925 7930 7935
   Ile Val Val Glu Lys Pro Gly Arg Ile Val Pro Gly Val Ile Gly Leu
   7940 7945 7950
   Met Arg Ala Leu Thr Ile Asn Asp Ala Asp
   7955 7960
<210> 37
   <211> 5198
   <212> PRT
   <213> Caenorhabditis elegans
<220>
   <222> (1)..(5198)
   <223> hemicentin precursor
<300>
   <301> Andersson, et al. Vogel, et al. Lbeit, et al. Ohara, O
   <302> Unknown protein [Homo sapiens].
   <308> GI 3005744
   <309> 1998-04-02
   <313> 1 TO 494
<400> 37
   Met Gly Arg Ser Pro Ser Trp Leu Tyr Gly Val Leu Gly Leu Leu Leu
   1 5 10 15
   Leu Ala Thr Thr Cys Ser Ser Val Asn Asp Asp Lys Asn Asp Pro Thr
   20 25 30
   Gly Lys Ser Ser Leu Ala Phe Val Phe Asp Ile Thr Gly Ser Met Phe
   35 40 45
   Asp Asp Leu Val Gln Val Arg Glu Gly Ala Ala Lys Ile Phe Lys Thr
   50 55 60
   Val Met Ala Gln Arg Glu Lys Leu Ile Tyr Asn Tyr Ile Met Val Pro
   65 70 75 80
   Phe His Asp Pro Tyr Leu Gly Glu Ile Ile Asn Thr Thr Asp Ser Thr
   85 90 95
   Tyr Phe Met Arg Gln Leu Ser Lys Val Tyr Val His Gly Gly Gly Asp
               100                 105                 110
   Cys Pro Glu Lys Thr Leu Thr Gly Ile Leu Lys Ala Leu Gln Ile Ser
           115                 120                 125
   Leu Pro Ser Ser Phe Ile Tyr Val Phe Thr Asp Ala Arg Ser Lys Asp
       130                 135                 140
   Tyr His Leu Glu Asp Glu Val Leu Asn Thr Ile Gln Glu Lys Gln Ser
   145                 150                 155                 160
   Ser Val Val Phe Val Met Thr Gly Asp Cys Gly Asn Arg Thr His Pro
                   165                 170                 175
   Gly Phe Arg Thr Tyr Glu Lys Ile Ala Ala Ala Ser Phe Gly Gln Val
               180                 185                 190
   Phe His Leu Glu Lys Ser Asp Val Ser Thr Val Leu Glu Tyr Val Arg
           195                 200                 205
   His Ala Val Lys Gln Lys Lys Val His Leu Met Tyr Glu Ala Arg Glu
       210                 215                 220
   Arg Gly Gly Thr Val Ser Arg Asn Ile Pro Val Asp Lys His Leu Ser
   225                 230                 235                 240
   Glu Leu Thr Ile Ser Leu Ser Gly Asp Lys Asp Asp Ser Asp Asn Leu
                   245                 250                 255
   Asp Ile Val Leu Arg Asp Pro Glu Gly Arg Thr Val Asp Lys Arg Leu
               260                 265                 270
   Tyr Ser Lys Glu Gly Gly Thr Ile Asp Leu Lys Asn Val Lys Leu Ile
           275                 280                 285
   Arg Leu Lys Asp Pro Ser Pro Gly Val Trp Thr Val Asn Thr Asn Ser
       290                 295                 300
   Arg Leu Lys His Thr Ile Arg Val Phe Gly His Gly Ala Val Asp Phe
   305                 310                 315                 320
   Lys Tyr Gly Phe Ala Ser Arg Pro Leu Asp Arg Ile Glu Leu Ala Arg
                   325                 330                 335
   Pro Arg Pro Val Leu Asn Gln Asp Thr Tyr Leu Leu Ile Asn Met Thr
               340                 345                 350
   Gly Leu Ile Pro Pro Gly Thr Val Gly Glu Ile Asp Leu Val Asp Tyr
           355                 360                 365
   His Gly His Ser Leu Tyr Lys Ala Val Ala Ser Pro His Arg Thr Asn
       370                 375                 380
   Pro Asn Met Tyr Phe Ala Gly Pro Phe Val Pro Pro Lys Gly Leu Phe
   385                 390                 395                 400
   Phe Val Arg Val Gln Gly Tyr Asp Glu Asp Asn Tyr Glu Phe Met Arg
                   405                 410                 415
   Ile Ala Pro Thr Ala Ile Gly Ser Val Ile Val Gly Gly Pro Arg Ala
   420 425 430
   Phe Met Ser Pro Ile His Gln Glu Phe Val Gly Arg Asp Leu Asn Leu
   435 440 445
   Ser Cys Thr Val Glu Ser Ala Ser Ala Tyr Thr Ile Tyr Trp Val Lys
   450 455 460
   Thr Gly Glu Asp Ile Ile Gly Gly Pro Leu Phe Tyr His Asn Thr Asp
   465 470 475 480
   Thr Ser Val Trp Thr Ile Pro Glu Leu Ser Leu Lys Asp Ala Gly Glu
   485 490 495
   Tyr Glu Cys Arg Val Ile Ser Asn Asn Gly Asn Tyr Ser Val Lys Thr
   500 505 510
   Arg Val Glu Thr Arg Glu Ser Pro Pro Glu Ile Phe Gly Val Arg Asn
   515 520 525
   Val Ser Val Pro Leu Gly Glu Ala Ala Phe Leu His Cys Ser Thr Arg
   530 535 540
   Ser Ala Gly Glu Val Glu Ile Arg Trp Thr Arg Tyr Gly Ala Thr Val
   545 550 555 560
   Phe Asn Gly Pro Asn Thr Glu Arg Asn Pro Thr Asn Gly Thr Leu Lys
   565 570 575
   Ile His His Val Thr Arg Ala Asp Ala Gly Val Tyr Glu Cys Met Ala
   580 585 590
   Arg Asn Ala Gly Gly Met Ser Thr Arg Lys Met Arg Leu Asp Ile Met
   595 600 605
   Glu Pro Pro Ser Val Lys Val Thr Pro Gln Asp Val Tyr Phe Asn Met
   610 615 620
   Arg Glu Gly Val Asn Leu Ser Cys Glu Ala Met Gly Asp Pro Lys Pro
   625 630 635 640
   Glu Val His Trp Tyr Phe Lys Gly Arg His Leu Leu Asn Asp Tyr Lys
   645 650 655
   Tyr Gln Val Gly Gln Asp Ser Lys Phe Leu Tyr Ile Arg Asp Ala Thr
   660 665 670
   His His Asp Glu Gly Thr Tyr Glu Cys Arg Ala Met Ser Gln Ala Gly
   675 680 685
   Gln Ala Arg Asp Thr Thr Asp Leu Met Leu Ala Thr Pro Pro Lys Val
   690 695 700
   Glu Ile Ile Gln Asn Lys Met Met Val Gly Arg Gly Asp Arg Val Ser
   705 710 715 720
   Phe Glu Cys Lys Thr Ile Arg Gly Lys Pro His Pro Lys Ile Arg Trp
   725 730 735
   Phe Lys Asn Gly Lys Asp Leu Ile Lys Pro Asp Asp Tyr Ile Lys Ile
   740 745 750
   Asn Glu Gly Gln Leu His Ile Met Gly Ala Lys Asp Glu Asp Ala Gly
   755 760 765
   Ala Tyr Ser Cys Val Gly Glu Asn Met Ala Gly Lys Asp Val Gln Val
   770 775 780
   Ala Asn Leu Ser Val Gly Arg Val Pro Thr Ile Ile Glu Ser Pro His
   785 790 795 800
   Thr Val Arg Val Asn Ile Glu Arg Gln Val Thr Leu Gln Cys Leu Ala
   805 810 815
   Val Gly Ile Pro Pro Pro Glu Ile Glu Trp Gln Lys Gly Asn Val Leu
   820 825 830
   Leu Ala Thr Leu Asn Asn Pro Arg Tyr Thr Gln Leu Ala Asp Gly Asn
   835 840 845
   Leu Leu Ile Thr Asp Ala Gln Ile Glu Asp Gln Gly Gln Phe Thr Cys
   850 855 860
   Ile Ala Arg Asn Thr Tyr Gly Gln Gln Ser Gln Ser Thr Thr Leu Met
   865 870 875 880
   Val Thr Gly Leu Val Ser Pro Val Leu Gly His Val Pro Pro Glu Glu
   885 890 895
   Gln Leu Ile Glu Gly Gln Asp Leu Thr Leu Ser Cys Val Val Val Leu
   900 905 910
   Gly Thr Pro Lys Pro Ser Ile Val Trp Ile Lys Asp Asp Lys Pro Val
   915 920 925
   Glu Glu Gly Pro Thr Ile Lys Ile Glu Gly Gly Gly Ser Leu Leu Arg
   930 935 940
   Leu Arg Gly Gly Asn Pro Lys Asp Glu Gly Lys Tyr Thr Cys Ile Ala
   945 950 955 960
   Val Ser Pro Ala Gly Asn Ser Thr Leu His Ile Asn Val Gln Leu Ile
   965 970 975
   Lys Lys Pro Glu Phe Val Tyr Lys Pro Glu Gly Gly Ile Val Phe Lys
   980 985 990
   Pro Thr Ile Ser Gly Met Asp Glu Lys His Val Ala Val Val Asn Ser
   995 1000 1005
   Thr His Asp Val Leu Asp Gly Glu Gly Phe Ala Ile Pro Cys Val Val
   1010 1015 1020
   Ser Gly Thr Pro Pro Pro Ile Ile Thr Trp Tyr Leu Asp Gly Arg Pro
   1025 1030 1035 1040
   Ile Thr Pro Asn Ser Arg Asp Phe Thr Val Thr Ala Asp Asn Thr Leu
   1045 1050 1055
   Ile Val Arg Lys Ala Asp Lys Ser Tyr Ser Gly Val Tyr Thr Cys Gln
   1060 1065 1070
   Ala Thr Asn Ser Ala Gly Asp Asn Glu Gln Lys Thr Thr Ile Arg Ile
   1075 1080 1085
   Met Asn Thr Pro Met Ile Ser Pro Gly Gln Ser Ser Phe Asn Met Val
   1090 1095 1100
   Val Asp Asp Leu Phe Thr Ile Pro Cys Asp Val Tyr Gly Asp Pro Lys
   1105 1110 1115 1120
   Pro Val Ile Thr Trp Leu Leu Asp Asp Lys Pro Phe Thr Glu Gly Val
   1125 1130 1135
   Val Asn Glu Asp Gly Ser Leu Thr Ile Pro Asn Val Asn Glu Ala His
   1140 1145 1150
   Arg Gly Thr Phe Thr Cys His Ala Gln Asn Ala Ala Gly Asn Asp Thr
   1155 1160 1165
   Arg Thr Val Thr Leu Thr Val His Thr Thr Pro Thr Ile Asn Ala Glu
   1170 1175 1180
   Asn Gln Glu Lys Ile Ala Leu Gln Asn Asp Asp Ile Val Leu Glu Cys
   1185 1190 1195 1200
   Pro Ala Lys Ala Leu Pro Pro Pro Val Arg Leu Trp Thr Tyr Glu Gly
   1205 1210 1215
   Glu Lys Ile Asp Ser Gln Leu Ile Pro His Thr Ile Arg Glu Asp Gly
   1220 1225 1230
   Ala Leu Val Leu Gln Asn Val Lys Leu Glu Asn Thr Gly Val Phe Val
   1235 1240 1245
   Cys Gln Val Ser Asn Leu Ala Gly Glu Asp Ser Leu Ser Tyr Thr Leu
   1250 1255 1260
   Thr Val His Glu Lys Pro Lys Ile Ile Ser Glu Val Pro Gly Val Val
   1265 1270 1275 1280
   Asp Val Val Lys Gly Phe Thr Ile Glu Ile Pro Cys Arg Ala Thr Gly
   1285 1290 1295
   Val Pro Glu Val Ile Arg Thr Trp Asn Lys Asn Gly Ile Asp Leu Lys
   1300 1305 1310
   Met Asp Glu Lys Lys Phe Ser Val Asp Asn Leu Gly Thr Leu Arg Ile
   1315 1320 1325
   Tyr Glu Ala Asp Lys Asn Asp Ile Gly Asn Tyr Asn Cys Val Val Thr
   1330 1335 1340
   Asn Glu Ala Gly Thr Ser Gln Met Thr Thr His Val Asp Val Gln Glu
   1345 1350 1355 1360
   Pro Pro Ile Ile Leu Pro Ser Thr Gln Thr Asn Asn Thr Ala Val Val
   1365 1370 1375
   Gly Asp Arg Val Glu Leu Lys Cys Tyr Val Glu Ala Ser Pro Pro Ala
   1380 1385 1390
   Ser Val Thr Trp Phe Arg Arg Gly Ile Ala Ile Gly Thr Asp Thr Lys
   1395 1400 1405
   Gly Tyr Val Val Glu Ser Asp Gly Thr Leu Val Ile Gln Ser Ala Ser
   1410 1415 1420
   Val Glu Asp Ala Thr Ile Tyr Thr Cys Lys Ala Ser Asn Pro Ala Gly
   1425 1430 1435 1440
   Lys Ala Glu Ala Asn Leu Gln Val Thr Val Ile Ala Ser Pro Asp Ile
   1445 1450 1455
   Lys Asp Pro Asp Val Val Thr Gln Glu Ser Ile Lys Glu Ser His Pro
   1460 1465 1470
   Phe Ser Leu Tyr Cys Pro Val Phe Ser Asn Pro Leu Pro Gln Ile Ser
   1475 1480 1485
   Trp Tyr Leu Asn Asp Lys Pro Leu Ile Asp Asp Lys Thr Ser Trp Lys
   1490 1495 1500
   Thr Ser Asp Asp Lys Arg Lys Leu His Val Phe Lys Ala Lys Ile Thr
   1505 1510 1515 1520
   Asp Ser Gly Val Tyr Lys Cys Val Ala Arg Asn Ala Ala Gly Glu Gly
   1525 1530 1535
   Ser Lys Ser Phe Gln Val Glu Val Ile Val Pro Leu Asn Leu Asp Glu
   1540 1545 1550
   Ser Lys Tyr Lys Lys Lys Val Phe Ala Lys Glu Gly Glu Glu Val Thr
   1555 1560 1565
   Leu Gly Cys Pro Val Ser Gly Phe Pro Val Pro Gln Ile Asn Trp Val
   1570 1575 1580
   Val Asp Gly Thr Val Val Glu Pro Gly Lys Lys Tyr Lys Gly Ala Thr
   1585 1590 1595 1600
   Leu Ser Asn Asp Gly Leu Thr Leu His Phe Asp Ser Val Ser Val Lys
   1605 1610 1615
   Gln Glu Gly Asn Tyr His Cys Val Ala Gln Ser Lys Gly Asn Ile Leu
   1620 1625 1630
   Asp Ile Asp Val Glu Leu Ser Val Leu Ala Val Pro Ile Val Gly Glu
   1635 1640 1645
   Asp Asp Asn Leu Glu Val Phe Leu Gly Lys Asp Ile Ser Leu Ser Cys
   1650 1655 1660
   Asp Leu Gln Thr Glu Ser Asp Asp Lys Thr Thr Phe Val Trp Ser Ile
   1665 1670 1675 1680
   Asn Gly Ser Glu Ser Asp Arg Pro Asp Asn Val Gln Ile Pro Ser Asp
   1685 1690 1695
   Gly His Arg Leu Tyr Ile Thr Asp Ala Lys Pro Glu Asn Asn Gly Lys
   1700 1705 1710
   Tyr Met Cys Arg Val Thr Asn Ser Ala Gly Lys Ala Glu Arg Thr Leu
   1715 1720 1725
   Thr Leu Asp Val Leu Glu Pro Pro Val Phe Val Glu Pro Val Phe Glu
   1730 1735 1740
   Ala Asn Gln Lys Leu Ile Gly Asn Asn Pro Ile Ile Leu Gln Cys Gln
   1745 1750 1755 1760
   Val Thr Gly Asn Pro Lys Pro Thr Val Ile Trp Lys Ile Asp Gly Asn
   1765 1770 1775
   Asp Val Asp Lys Ser Trp Leu Phe Asp Glu Ser Leu Ser Leu Leu Arg
   1780 1785 1790
   Ile Glu Lys Leu Thr Gly Lys Ser Ala Gln Ile Ser Cys Thr Ala Glu
   1795 1800 1805
   Asn Lys Ala Gly Thr Ala Ser Arg Asp Phe Phe Ile Gln Asn Ile Ala
   1810 1815 1820
   Ala Pro Thr Phe Lys Asn Glu Gly Asp Gln Glu Thr Ile Phe Arg Glu
   1825 1830 1835 1840
   Ser Glu Thr Ile Thr Leu Asp Cys Pro Val Ser Leu Gly Asp Phe Gln
   1845 1850 1855
   Ile Thr Trp Met Lys Gln Gly Leu Pro Leu Thr Glu Asn Asp Ala Ile
   1860 1865 1870
   Phe Thr Leu Asp Asn Thr Arg Leu Thr Ile Leu Asn Ala Asn Arg Asp
   1875 1880 1885
   His Glu Asp Ile Tyr Thr Cys Val Ala Asn Asn Thr Ala Gly Gln Val
   1890 1895 1900
   Ser Lys Asp Phe Asp Val Val Val Gln Val Leu Pro Lys Ile Lys Asn
   1905 1910 1915 1920
   Ala Val Val Thr Leu Glu Ile Asn Glu Gly Glu Glu Ile Ile Leu Thr
   1925 1930 1935
   Cys Asp Ala Glu Gly Asn Pro Thr Pro Thr Ala Lys Trp Asp Phe Asn
   1940 1945 1950
   Gln Gly Asp Leu Pro Lys Glu Ala Val Phe Val Asn Asn Asn His Thr
   1955 1960 1965
   Val Val Val Asn Asn Val Thr Lys Tyr His Thr Gly Val Tyr Lys Cys
   1970 1975 1980
   Tyr Ala Thr Asn Lys Val Gly Gln Ala Val Lys Thr Ile Asn Val His
   1985 1990 1995 2000
   Val Arg Thr Lys Pro Arg Phe Glu Ser Gly Leu Thr Glu Ser Glu Leu
   2005 2010 2015
   Thr Val Asn Leu Thr Arg Ser Ile Thr Leu Glu Cys Asp Val Asp Asp
   2020 2025 2030
   Ala Ile Gly Val Gly Ile Ser Trp Thr Val Asn Gly Lys Pro Phe Leu
   2035 2040 2045
   Ala Glu Thr Asp Gly Val Gln Thr Leu Ala Gly Gly Arg Phe Leu His
   2050 2055 2060
   Ile Val Ser Ala Lys Thr Asp Asp His Gly Ser Tyr Ala Cys Thr Val
   2065 2070 2075 2080
   Thr Asn Glu Ala Gly Val Ala Thr Lys Thr Phe Asn Leu Phe Val Gln
   2085 2090 2095
   Val Pro Pro Thr Ile Val Asn Glu Gly Gly Glu Tyr Thr Val Ile Glu
   2100 2105 2110
   Asn Asn Ser Leu Val Leu Pro Cys Glu Val Thr Gly Lys Pro Asn Pro
   2115 2120 2125
   Val Val Thr Trp Thr Lys Asp Gly Arg Pro Val Gly Asp Leu Lys Ser
   2130 2135 2140
   Val Gln Val Leu Ser Glu Gly Gln Gln Phe Lys Ile Val His Ala Glu
   2145 2150 2155 2160
   Ile Ala His Lys Gly Ser Tyr Ile Cys Met Ala Lys Asn Asp Val Gly
   2165 2170 2175
   Thr Ala Glu Ile Ser Phe Asp Val Asp Ile Ile Thr Arg Pro Met Ile
   2180 2185 2190
   Gln Lys Gly Ile Lys Asn Ile Val Thr Ala Ile Lys Gly Gly Ala Leu
   2195 2200 2205
   Pro Phe Lys Cys Pro Ile Asp Asp Asp Lys Asn Phe Lys Gly Gln Ile
   2210 2215 2220
   Ile Trp Leu Arg Asn Tyr Gln Pro Ile Asp Leu Glu Ala Glu Asp Ala
   2225 2230 2235 2240
   Arg Ile Thr Arg Leu Ser Asn Asp Arg Arg Leu Thr Ile Leu Asn Val
   2245 2250 2255
   Thr Glu Asn Asp Glu Gly Gln Tyr Ser Cys Arg Val Lys Asn Asp Ala
   2260 2265 2270
   Gly Glu Asn Ser Phe Asp Phe Lys Ala Thr Val Leu Val Pro Pro Thr
   2275 2280 2285
   Ile Ile Met Leu Asp Lys Asp Lys Asn Lys Thr Ala Val Glu His Ser
   2290 2295 2300
   Thr Val Thr Leu Ser Cys Pro Ala Thr Gly Lys Pro Glu Pro Asp Ile
   2305 2310 2315 2320
   Thr Trp Phe Lys Asp Gly Glu Ala Ile His Ile Glu Asn Ile Ala Asp
   2325 2330 2335
   Ile Ile Pro Asn Gly Glu Leu Asn Gly Asn Gln Leu Lys Ile Thr Arg
   2340 2345 2350
   Ile Lys Glu Gly Asp Ala Gly Lys Tyr Thr Cys Glu Ala Asp Asn Ser
   2355 2360 2365
   Ala Gly Ser Val Glu Gln Asp Val Asn Val Asn Val Ile Thr Ile Pro
   2370 2375 2380
   Lys Ile Glu Lys Asp Gly Ile Pro Ser Asp Tyr Glu Ser Gln Gln Asn
   2385 2390 2395 2400
   Glu Arg Val Val Ile Ser Cys Pro Val Tyr Ala Arg Pro Pro Ala Lys
   2405 2410 2415
   Ile Thr Trp Leu Lys Ala Gly Lys Pro Leu Gln Ser Asp Lys Phe Val
   2420 2425 2430
   Lys Thr Ser Ala Asn Gly Gln Lys Leu Tyr Leu Phe Lys Leu Arg Glu
   2435 2440 2445
   Thr Asp Ser Ser Lys Tyr Thr Cys Ile Ala Thr Asn Glu Ala Gly Thr
   2450 2455 2460
   Asp Lys Arg Asp Phe Lys Val Ser Met Leu Val Ala Pro Ser Phe Asp
   2465 2470 2475 2480
   Glu Pro Asn Ile Val Arg Arg Ile Thr Val Asn Ser Gly Asn Pro Ser
   2485 2490 2495
   Thr Leu His Cys Pro Ala Lys Gly Ser Pro Ser Pro Thr Ile Thr Trp
   2500 2505 2510
   Leu Lys Asp Gly Asn Ala Ile Glu Pro Asn Asp Arg Tyr Val Phe Phe
   2515 2520 2525
   Asp Ala Gly Arg Gln Leu Gln Ile Ser Lys Thr Glu Gly Ser Asp Gln
   2530 2535 2540
   Gly Arg Tyr Thr Cys Ile Ala Thr Asn Ser Val Gly Ser Asp Asp Leu
   2545 2550 2555 2560
   Glu Asn Thr Leu Glu Val Ile Ile Pro Pro Val Ile Asp Gly Glu Arg
   2565 2570 2575
   Arg Glu Ala Val Ala Val Ile Glu Gly Phe Ser Ser Glu Leu Phe Cys
   2580 2585 2590
   Asp Ser Asn Ser Thr Gly Val Asp Val Glu Trp Gln Lys Asp Gly Leu
   2595 2600 2605
   Thr Ile Asn Gln Asp Thr Leu Arg Gly Asp Ser Phe Ile Gln Ile Pro
   2610 2615 2620
   Ser Ser Gly Lys Lys Met Ser Phe Leu Ser Ala Arg Lys Ser Asp Ser
   2625 2630 2635 2640
   Gly Arg Tyr Thr Cys Ile Val Arg Asn Pro Ala Gly Glu Ala Arg Lys
   2645 2650 2655
   Leu Phe Asp Phe Ala Val Asn Asp Pro Pro Ser Ile Ser Asp Glu Leu
   2660 2665 2670
   Ser Ser Ala Asn Ile Gln Thr Ile Val Pro Tyr Tyr Pro Val Glu Ile
   2675 2680 2685
   Asn Cys Val Val Ser Gly Ser Pro His Pro Lys Val Tyr Trp Leu Phe
   2690 2695 2700
   Asp Asp Lys Pro Leu Glu Pro Asp Ser Ala Ala Tyr Glu Leu Thr Asn
   2705 2710 2715 2720
   Asn Gly Glu Thr Leu Lys Ile Val Arg Ser Gln Val Glu His Ala Gly
   2725 2730 2735
   Thr Tyr Thr Cys Glu Ala Gln Asn Asn Val Gly Lys Ala Arg Lys Asp
   2740 2745 2750
   Phe Leu Val Arg Val Thr Ala Pro Pro His Phe Glu Lys Glu Arg Glu
   2755 2760 2765
   Glu Val Val Ala Arg Val Gly Asp Thr Met Leu Leu Thr Cys Asn Ala
   2770 2775 2780
   Glu Ser Ser Val Pro Leu Ser Ser Val Tyr Trp His Ala His Asp Glu
   2785 2790 2795 2800
   Ser Val Gln Asn Gly Val Ile Thr Ser Lys Tyr Ala Ala Asn Glu Lys
   2805 2810 2815
   Thr Leu Asn Val Thr Asn Ile Gln Leu Asp Asp Glu Gly Phe Tyr Tyr
   2820 2825 2830
   Cys Thr Ala Val Asn Glu Ala Gly Ile Thr Lys Lys Phe Phe Lys Leu
   2835 2840 2845
   Ile Val Ile Glu Thr Pro Tyr Phe Leu Asp Gln Gln Lys Leu Tyr Pro
   2850 2855 2860
   Ile Ile Leu Gly Lys Arg Leu Thr Leu Asp Cys Ser Ala Thr Gly Thr
   2865 2870 2875 2880
   Pro Pro Pro Thr Ile Leu Phe Met Lys Asp Gly Lys Arg Leu Asn Glu
   2885 2890 2895
   Ser Asp Glu Val Asp Ile Ile Gly Ser Thr Leu Val Ile Asp Asn Pro
   2900 2905 2910
   Gln Lys Glu Val Glu Gly Arg Tyr Thr Cys Ile Ala Glu Asn Lys Ala
   2915 2920 2925
   Gly Arg Ser Glu Lys Asp Met Met Val Glu Val Leu Leu Pro Pro Lys
   2930 2935 2940
   Leu Ser Lys Glu Trp Ile Asn Val Glu Val Gln Ala Gly Asp Pro Leu
   2945 2950 2955 2960
   Thr Leu Glu Cys Pro Ile Glu Asp Thr Ser Gly Val His Ile Thr Trp
   2965 2970 2975
   Ser Arg Gln Phe Gly Lys Asp Gly Gln Leu Asp Met Arg Ala Gln Ser
   2980 2985 2990
   Ser Ser Asp Lys Ser Lys Leu Tyr Ile Met Gln Ala Thr Pro Glu Asp
   2995 3000 3005
   Ala Asp Ser Tyr Ser Cys Ile Ala Val Asn Asp Ala Gly Gly Ala Glu
   3010 3015 3020
   Ala Val Phe Gln Val Thr Val Asn Thr Pro Pro Lys Ile Phe Gly Asp
   3025 3030 3035 3040
   Ser Phe Ser Thr Thr Glu Ile Val Ala Asp Thr Thr Leu Glu Ile Pro
   3045 3050 3055
   Cys Arg Thr Glu Gly Ile Pro Pro Pro Glu Ile Ser Trp Phe Leu Asp
   3060 3065 3070
   Gly Lys Pro Ile Leu Glu Met Pro Gly Val Thr Tyr Lys Gln Gly Asp
   3075 3080 3085
   Leu Ser Leu Arg Ile Asp Asn Ile Lys Pro Asn Gln Glu Gly Arg Tyr
   3090 3095 3100
   Thr Cys Val Ala Glu Asn Lys Ala Gly Arg Ala Glu Gln Asp Thr Tyr
   3105 3110 3115 3120
   Val Glu Ile Ser Glu Pro Pro Arg Val Val Met Ala Ser Glu Val Met
   3125 3130 3135
   Arg Val Val Glu Gly Arg Gln Thr Thr Ile Arg Cys Glu Val Phe Gly
   3140 3145 3150
   Asn Pro Glu Pro Val Val Asn Trp Leu Lys Asp Gly Glu Pro Tyr Thr
   3155 3160 3165
   Ser Asp Leu Leu Gln Phe Ser Thr Lys Leu Ser Tyr Leu His Leu Arg
   3170 3175 3180
   Glu Thr Thr Leu Ala Asp Gly Gly Thr Tyr Thr Cys Ile Ala Thr Asn
   3185 3190 3195 3200
   Lys Ala Gly Glu Ser Gln Thr Thr Thr Asp Val Glu Val Leu Val Pro
   3205 3210 3215
   Pro Arg Ile Glu Asp Glu Glu Arg Val Leu Gln Gly Lys Glu Gly Asn
   3220 3225 3230
   Thr Tyr Met Val His Cys Gln Val Thr Gly Arg Pro Val Pro Tyr Val
   3235 3240 3245
   Thr Trp Lys Arg Asn Gly Lys Glu Ile Glu Gln Phe Asn Pro Val Leu
   3250 3255 3260
   His Ile Arg Asn Ala Thr Arg Ala Asp Glu Gly Lys Tyr Ser Cys Ile
   3265 3270 3275 3280
   Ala Ser Asn Glu Ala Gly Thr Ala Val Ala Asp Phe Leu Ile Asp Val
   3285 3290 3295
   Phe Thr Lys Pro Thr Phe Glu Thr His Glu Thr Thr Phe Asn Ile Val
   3300 3305 3310
   Glu Gly Glu Ser Ala Lys Ile Glu Cys Lys Ile Asp Gly His Pro Lys
   3315 3320 3325
   Pro Thr Ile Ser Trp Leu Lys Gly Gly Arg Pro Phe Asn Met Asp Asn
   3330 3335 3340
   Ile Ile Leu Ser Pro Arg Gly Asp Thr Leu Met Ile Leu Lys Ala Gln
   3345 3350 3355 3360
   Arg Phe Asp Gly Gly Leu Tyr Thr Cys Val Ala Thr Asn Ser Tyr Gly
   3365 3370 3375
   Asp Ser Glu Gln Asp Phe Lys Val Asn Val Tyr Thr Lys Pro Tyr Ile
   3380 3385 3390
   Asp Glu Thr Ile Asp Gln Thr Pro Lys Ala Val Ala Gly Gly Glu Ile
   3395 3400 3405
   Ile Leu Lys Cys Pro Val Leu Gly Asn Pro Thr Pro Thr Val Thr Trp
   3410 3415 3420
   Lys Arg Gly Asp Asp Ala Val Pro Asn Asp Ser Arg His Thr Ile Val
   3425 3430 3435 3440
   Asn Asn Tyr Asp Leu Lys Ile Asn Ser Val Thr Thr Glu Asp Ala Gly
   3445 3450 3455
   Gln Tyr Ser Cys Ile Ala Val Asn Glu Ala Gly Asn Leu Thr Thr His
   3460 3465 3470
   Tyr Ala Ala Glu Val Ile Gly Lys Pro Thr Phe Val Arg Lys Gly Gly
   3475 3480 3485
   Asn Leu Tyr Glu Val Ile Glu Asn Asp Thr Ile Thr Met Asp Cys Gly
   3490 3495 3500
   Val Thr Ser Arg Pro Leu Pro Ser Ile Ser Trp Phe Arg Gly Asp Lys
   3505 3510 3515 3520
   Pro Val Tyr Leu Tyr Asp Arg Tyr Ser Ile Ser Pro Asp Gly Ser His
   3525 3530 3535
   Ile Thr Ile Asn Lys Ala Lys Leu Ser Asp Gly Gly Lys Tyr Ile Cys
   3540 3545 3550
   Arg Ala Ser Asn Glu Ala Gly Thr Ser Asp Ile Asp Leu Ile Leu Lys
   3555 3560 3565
   Ile Leu Val Pro Pro Lys Ile Asp Lys Ser Asn Ile Ile Gly Asn Pro
   3570 3575 3580
   Leu Ala Ile Val Ala Arg Thr Ile Tyr Leu Glu Cys Pro Ile Ser Gly
   3585 3590 3595 3600
   Ile Pro Gln Pro Asp Val Ile Trp Thr Lys Asn Gly Met Asp Ile Asn
   3605 3610 3615
   Met Thr Asp Ser Arg Val Ile Leu Ala Gln Asn Asn Glu Thr Phe Gly
   3620 3625 3630
   Ile Glu Asn Val Gln Val Thr Asp Gln Gly Arg Tyr Thr Cys Thr Ala
   3635 3640 3645
   Thr Asn Arg Gly Gly Lys Ala Ser His Asp Phe Ser Leu Asp Val Leu
   3650 3655 3660
   Ser Pro Pro Glu Phe Asp Ile His Gly Thr Gln Pro Thr Ile Lys Arg
   3665 3670 3675 3680
   Glu Gly Asp Thr Ile Thr Leu Thr Cys Pro Ile Lys Leu Ala Glu Asp
   3685 3690 3695
   Ile Ala Asp Gln Val Met Asp Val Ser Trp Thr Lys Asp Ser Arg Ala
   3700 3705 3710
   Leu Asp Gly Asp Leu Thr Asp Asn Val Asp Ile Ser Asp Asp Gly Arg
   3715 3720 3725
   Lys Leu Thr Ile Ser Gln Ala Ser Leu Glu Asn Ala Gly Leu Tyr Thr
   3730 3735 3740
   Cys Ile Ala Leu Asn Arg Ala Gly Glu Ala Ser Leu Glu Phe Lys Val
   3745 3750 3755 3760
   Glu Ile Leu Ser Pro Pro Val Ile Asp Ile Ser Arg Asn Asp Val Gln
   3765 3770 3775
   Pro Gln Val Ala Val Asn Gln Pro Thr Ile Met Arg Cys Ala Val Thr
   3780 3785 3790
   Gly His Pro Phe Pro Ser Ile Lys Trp Leu Lys Asn Gly Lys Glu Val
   3795 3800 3805
   Thr Asp Asp Glu Asn Ile Arg Ile Val Glu Gln Gly Gln Val Leu Gln
   3810 3815 3820
   Ile Leu Arg Thr Asp Ser Asp His Ala Gly Lys Trp Ser Cys Val Ala
   3825 3830 3835 3840
   Glu Asn Asp Ala Gly Val Lys Glu Leu Glu Met Val Leu Asp Val Phe
   3845 3850 3855
   Thr Pro Pro Val Val Ser Val Lys Ser Asp Asn Pro Ile Lys Ala Leu
   3860 3865 3870
   Gly Glu Thr Ile Thr Leu Phe Cys Asn Ala Ser Gly Asn Pro Tyr Pro
   3875 3880 3885
   Gln Leu Lys Trp Ala Lys Gly Gly Ser Leu Ile Phe Asp Ser Pro Asp
   3890 3895 3900
   Gly Ala Arg Ile Ser Leu Lys Gly Ala Arg Leu Asp Ile Pro His Leu
   3905 3910 3915 3920
   Lys Lys Thr Asp Val Gly Asp Tyr Thr Cys Gln Ala Leu Asn Ala Ala
   3925 3930 3935
   Gly Thr Ser Glu Ala Ser Val Ser Val Asp Val Leu Val Pro Pro Glu
   3940 3945 3950
   Ile Asn Arg Asp Gly Ile Asp Met Ser Pro Arg Leu Pro Ala Gln Gln
   3955 3960 3965
   Ser Leu Thr Leu Gln Cys Leu Ala Gln Gly Lys Pro Val Pro Gln Met
   3970 3975 3980
   Arg Trp Thr Leu Asn Gly Thr Ala Leu Thr His Ser Thr Pro Gly Ile
   3985 3990 3995 4000
   Thr Val Ala Ser Asp Ser Thr Phe Ile Gln Ile Asn Asn Val Ser Leu
   4005 4010 4015
   Ser Asp Lys Gly Val Tyr Thr Cys Tyr Ala Glu Asn Val Ala Gly Ser
   4020 4025 4030
   Asp Asn Leu Met Tyr Asn Val Asp Val Val Gln Ala Pro Val Ile Ser
   4035 4040 4045
   Asn Gly Gly Thr Lys Gln Val Ile Glu Gly Glu Leu Ala Val Ile Glu
   4050 4055 4060
   Cys Leu Val Glu Gly Tyr Pro Ala Pro Gln Val Ser Trp Leu Arg Asn
   4065 4070 4075 4080
   Gly Asn Arg Val Glu Thr Gly Val Gln Gly Val Arg Tyr Val Thr Asp
   4085 4090 4095
   Gly Arg Met Leu Thr Ile Ile Glu Ala Arg Ser Leu Asp Ser Gly Ile
   4100 4105 4110
   Tyr Leu Cys Ser Ala Thr Asn Glu Ala Gly Ser Ala Gln Gln Ala Tyr
   4115 4120 4125
   Thr Leu Glu Val Leu Val Ser Pro Lys Ile Ile Thr Ser Thr Pro Gly
   4130 4135 4140
   Val Leu Thr Pro Ser Ser Gly Ser Lys Phe Ser Leu Pro Cys Ala Val
   4145 4150 4155 4160
   Arg Gly Tyr Pro Asp Pro Ile Ile Ser Trp Thr Leu Asn Gly Asn Asp
   4165 4170 4175
   Ile Lys Asp Gly Glu Asn Gly His Thr Ile Gly Ala Asp Gly Thr Leu
   4180 4185 4190
   His Ile Glu Lys Ala Glu Glu Arg His Leu Ile Tyr Glu Cys Thr Ala
   4195 4200 4205
   Lys Asn Asp Ala Gly Ala Asp Thr Leu Glu Phe Pro Val Gln Thr Ile
   4210 4215 4220
   Val Ala Pro Lys Ile Ser Thr Ser Gly Asn Arg Tyr Ile Asn Gly Ser
   4225 4230 4235 4240
   Glu Gly Thr Glu Thr Val Ile Lys Cys Glu Ile Glu Ser Glu Ser Ser
   4245 4250 4255
   Glu Phe Ser Trp Ser Lys Asn Gly Val Pro Leu Leu Pro Ser Asn Asn
   4260 4265 4270
   Leu Ile Phe Ser Glu Asp Tyr Lys Leu Ile Lys Ile Leu Ser Thr Arg
   4275 4280 4285
   Leu Ser Asp Gln Gly Glu Tyr Ser Cys Thr Ala Ala Asn Lys Ala Gly
   4290 4295 4300
   Asn Ala Thr Gln Lys Thr Asn Leu Asn Val Gly Val Ala Pro Lys Ile
   4305 4310 4315 4320
   Met Glu Arg Pro Arg Thr Gln Val Val His Lys Gly Asp Gln Val Thr
   4325 4330 4335
   Leu Trp Cys Glu Ala Ser Gly Val Pro Gln Pro Ala Ile Thr Trp Tyr
   4340 4345 4350
   Lys Asp Asn Glu Leu Leu Thr Asn Thr Gly Val Asp Glu Thr Ala Thr
   4355 4360 4365
   Thr Lys Lys Lys Ser Val Ile Phe Ser Ser Ile Ser Pro Ser Gln Ala
   4370 4375 4380
   Gly Val Tyr Thr Cys Lys Ala Glu Asn Trp Val Ala Ser Thr Glu Glu
   4385 4390 4395 4400
   Asp Ile Asp Leu Ile Val Met Ile Pro Pro Glu Val Val Pro Glu Arg
   4405 4410 4415
   Met Asn Val Ser Thr Asn Pro Arg Gln Thr Val Phe Leu Ser Cys Asn
   4420 4425 4430
   Ala Thr Gly Ile Pro Glu Pro Val Ile Ser Trp Met Arg Asp Ser Asn
   4435 4440 4445
   Ile Ala Ile Gln Asn Asn Glu Lys Tyr Gln Ile Leu Gly Thr Thr Leu
   4450 4455 4460
   Ala Ile Arg Asn Val Leu Pro Asp Asp Asp Gly Phe Tyr His Cys Ile
   4465 4470 4475 4480
   Ala Lys Ser Asp Ala Gly Gln Lys Ile Ala Thr Arg Lys Leu Ile Val
   4485 4490 4495
   Asn Lys Pro Ser Asp Arg Pro Ala Pro Ile Trp Val Glu Cys Asp Glu
   4500 4505 4510
   Lys Gly Lys Pro Lys Lys Thr Glu Tyr Met Ile Asp Arg Gly Asp Thr
   4515 4520 4525
   Pro Asp Asp Asn Pro Gln Leu Leu Pro Trp Lys Asp Val Glu Asp Ser
   4530 4535 4540
   Ser Leu Asn Gly Ser Ile Ala Tyr Arg Cys Met Pro Gly Pro Arg Ser
   4545 4550 4555 4560
   Ser Arg Thr Val Leu Leu His Ala Ala Pro Gln Phe Ile Val Lys Pro
   4565 4570 4575
   Lys Asn Thr Thr Ala Ala Ile Gly Ala Ile Val Glu Leu Arg Cys Ser
   4580 4585 4590
   Ala Ala Gly Pro Pro His Pro Thr Ile Thr Trp Ala Lys Asp Gly Lys
   4595 4600 4605
   Leu Ile Glu Asp Ser Lys Phe Glu Ile Ala Tyr Ser His Leu Lys Val
   4610 4615 4620
   Thr Leu Asn Ser Thr Ser Asp Ser Gly Glu Tyr Thr Cys Met Ala Gln
   4625 4630 4635 4640
   Asn Ser Val Gly Ser Ser Thr Val Ser Ala Phe Ile Asn Val Asp Asn
   4645 4650 4655
   Asn Ile Leu Pro Thr Pro Lys Pro Ser Ser Asn Gln Lys Asn Val Ala
   4660 4665 4670
   Val Ile Thr Cys Tyr Glu Arg Asn Gln Ala Tyr Ser Arg Gly Leu Thr
   4675 4680 4685
   Trp Glu Tyr Asn Gly Val Pro Met Pro Lys Asn Leu Ala Gly Ile His
   4690 4695 4700
   Phe Met Asn Asn Gly Ser Leu Val Ile Leu Asp Thr Ser Ser Leu Lys
   4705 4710 4715 4720
   Glu Gly Asp Leu Glu Leu Tyr Thr Cys Lys Val Arg Asn Arg Arg Arg
   4725 4730 4735
   His Ser Ile Pro His Leu Thr Ser Ala Phe Glu Gly Val Pro Glu Val
   4740 4745 4750
   Lys Thr Ile Asp Lys Val Glu Val Asn Asn Gly Asp Ser Val Val Leu
   4755 4760 4765
   Asp Cys Glu Val Thr Ser Asp Pro Leu Thr Thr His Val Val Trp Thr
   4770 4775 4780
   Lys Asn Asp Gln Lys Met Leu Asp Asp Asp Ala Ile Tyr Val Leu Pro
   4785 4790 4795 4800
   Asn Asn Ser Leu Val Leu Leu Asn Val Glu Lys Tyr Asp Glu Gly Val
   4805 4810 4815
   Tyr Lys Cys Val Ala Ser Asn Ser Ile Gly Lys Ala Phe Asp Asp Thr
   4820 4825 4830
   Gln Leu Asn Val Tyr Glu Gly Asp Phe Leu Pro Leu Thr Gly Phe Glu
   4835 4840 4845
   Gly Ser Gly Ile Asn Ile Asp Asp Ser Ser Asn Ala Gly Gly Ser Ser
   4850 4855 4860
   Arg Arg Glu Ala Tyr Lys Lys Glu Asn Glu Asp Ala Ser Thr Thr Thr
   4865 4870 4875 4880
   Ile Thr Thr Thr Ser Pro Thr Thr Thr Thr Thr Glu Thr Pro Leu Thr
   4885 4890 4895
   Thr Thr Ile Ile Pro Ala Leu Ile Thr Leu Pro Ala Lys Gln Tyr Pro
   4900 4905 4910
   Thr Asp Asp Tyr His Glu Gly Ser Ala Asn Asp Asp Gly Phe Gly Pro
   4915 4920 4925
   Thr Thr Gln Asp Ser Leu Phe Glu Phe Asn Pro Pro Leu His Pro Glu
   4930 4935 4940
   Ile Ser Val Val Asn Thr Asp Cys Ala Gly Thr Ile Asn Glu Asn Gly
   4945 4950 4955 4960
   Asp Cys Val Asp Lys Asp Gly Lys Thr His Asn Leu Lys Ile Leu Thr
   4965 4970 4975
   Gly Glu Asn His Cys Pro Glu Gly Phe Ala Met Asn Pro His Thr Arg
   4980 4985 4990
   Ile Cys Glu Asp Leu Asp Glu Cys Ala Phe Tyr Gln Pro Cys Asp Phe
   4995 5000 5005
   Glu Cys Ile Asn Tyr Asp Gly Gly Phe Gln Cys Asn Cys Pro Leu Gly
   5010 5015 5020
   Tyr Glu Leu Ala Glu Glu Gly Cys Arg Asp Val Asn Glu Cys Glu Ser
   5025 5030 5035 5040
   Val Arg Cys Glu Asp Gly Lys Ala Cys Phe Asn Gln Leu Gly Gly Tyr
   5045 5050 5055
   Glu Cys Ile Asp Asp Pro Cys Pro Ala Asn Tyr Ser Leu Val Asp Asp
   5060 5065 5070
   Arg Tyr Cys Glu Pro Glu Cys Glu Asn Cys Thr Ser Thr Pro Ile Gln
   5075 5080 5085
   Val His Met Leu Ala Ile Pro Ser Gly Leu Pro Ile Ser His Ile Ala
   5090 5095 5100
   Thr Leu Thr Ala Tyr Asp Lys Ser Gly Arg Val Leu Asn Asp Thr Thr
   5105 5110 5115 5120
   Tyr Ala Ile Ser Asp Thr Gly Ala Pro Leu Ala Arg Gly Arg Met Thr
   5125 5130 5135
   Ser Gly Pro Phe Thr Ile Lys Ala Val Lys Arg Gly His Ala Gln Val
   5140 5145 5150
   Trp Thr Asn Arg Val Leu Ala Ala Gly Asp His His Lys Val Arg Val
   5155 5160 5165
   Arg Ala His Ser Asp His Ala Thr Asn Glu Leu His Ala Pro Lys Glu
   5170 5175 5180
   Thr Asn Phe Leu Val Leu Ile Asn Val Gly Gln Tyr Pro Phe
   5185 5190 5195
<210> 38
   <211> 3720
   <212> DNA
   <213> Rattus sp.
<220>
   <221> CDS
   <222> (46)..(3174)
   <223> coding sequence for inventive nucleic acid product 405
<210> 39
   <211> 1043
   <212> PRT
   <213> Rattus sp.
<400> 39
   Met Leu Gly Tyr Leu Ala Ala Leu Ala Lys Ala Cys Phe Gly Gly Asn
   1 5 10 15
   Ile Glu Leu Phe Val Phe Phe Asn Gly Ala Leu Glu Lys Ala Arg Leu
   20 25 30
   His Glu Trp Val Lys Arg Gln Gly Asn Glu Arg Gln Thr Ala Gln Gln
   35 40 45
   Ile Val Ser His Val Gln Asn Lys Gly Thr Pro Pro Pro Lys Val Trp
   50 55 60
   Phe Leu Pro Pro Val Cys Met Ala His Cys Ile Arg Leu Ala Leu Ile
   65 70 75 80
   Arg Phe His Val Lys Val Ala Gln Ser Ile Glu Asp His His Gln Glu
   85 90 95
   Val Ile Gly Phe Cys Arg Glu Asn Gly Phe His Gly Leu Val Ala Tyr
                   100                 105                 110
   Asp Ser Asp Tyr Ala Leu Cys Asn Ile Pro Tyr Tyr Phe Ser Ala His
           115                 120                 125
   Ala Leu Lys Leu Ser Arg Asn Gly Lys Ser Leu Thr Thr Ser Gln Tyr
       130                 135                 140
   Leu Met His Glu Val Ala Lys Gln Leu Asp Leu Asn Pro Asn Arg Phe
   145                 150                 155                 160
   Pro Ile Phe Ala Ala Leu Leu Gly Asn His Ile Leu Pro Asp Glu Asp
                   165                 170                 175
   Leu Ala Ser Phe His Trp Ser Leu Leu Gly Pro Glu His Pro Leu Ala
               180                 185                 190
   Ser Leu Lys Val Arg Ala His Gln Leu Val Leu Pro Pro Cys Asp Val
           195                 200                 205
   Val Ile Lys Ala Val Ala Asp Tyr Val Arg Asn Ile His Asp Thr Ser
       210                 215                 220
   Asp Leu Asp Ala Ile Ala Lys Asp Val Phe Gln His Ser Gln Ser Arg
   225                 230                 235                 240
   Thr Asp Asp Lys Val Ile Arg Phe Lys Arg Ala Val Gly Tyr Tyr Ser
                   245                 250                 255
   Ala Thr Ser Lys Pro Met Pro Phe His Pro Pro His Tyr Leu Pro Arg
               260                 265                 270
   Pro Asn Pro Phe Gly Met Pro Gly Met Val Pro Pro Tyr Val Pro Pro
           275                 280                 285
   Gln Met Leu Asn Ile Pro Gln Thr Ser Leu Gln Ala Lys Pro Ala Val
       290                 295                 300
   Pro Gln Val Pro Ser Pro Gly Gly Thr Pro Gly Gln Ala Pro Tyr Pro
   305                 310                 315                 320
   Tyr Ser Leu Pro Glu Pro Ala Leu Ala Leu Asp Ser Ser Gly Lys Asn
                   325                 330                 335
   Leu Thr Glu Gln Asn Ser Tyr Ser Asn Ile Pro His Glu Gly Lys His
               340                 345                 350
   Thr Pro Leu Tyr Glu Arg Ser Ser Pro Ile Asn Leu Ala Gln Ser Gly
           355                 360                 365
   Ser Pro Asn His Val Asp Ser Ala Tyr Phe Pro Gly Ser Ser Thr Ser
       370                 375                 380
   Ser Ser Ser Asp Asn Asp Glu Gly Ser Gly Gly Ala Thr Asn His Ile
   385                 390                 395                 400
   Ser Gly Asn Lys Ile Gly Trp Glu Lys Thr Gly Ser His Ala Glu Pro
                   405                 410                 415
   Leu Ala Arg Gly Asp Pro Gly Asp Gln Val Lys Val Glu Gly Ser Ser
               420                 425                 430
   Thr Ala Ser Ser Gly Ser Gln Leu Ala Glu Gly Lys Gly Ser His Met
   435 440 445
   Gly Thr Val Gln Pro Ile Pro Cys Leu Leu Ser Met Pro Thr Arg Asn
   450 455 460
   His Met Asp Ile Thr Thr Pro Pro Leu Pro Pro Val Ala Pro Glu Val
   465 470 475 480
   Leu Arg Val Ala Glu His Arg His Lys Lys Gly Leu Met Tyr Pro Tyr
   485 490 495
   Ile Phe His Val Leu Thr Lys Gly Glu Ile Lys Ile Ala Val Ser Ile
   500 505 510
   Glu Asp Glu Ala Asn Lys Asp Leu Pro Pro Ala Ala Leu Leu Tyr Arg
   515 520 525
   Pro Val Arg Gln Tyr Val Tyr Gly Val Leu Phe Ser Leu Ala Glu Ser
   530 535 540
   Arg Lys Lys Thr Glu Arg Leu Ala Phe Arg Lys Asn Arg Leu Pro Pro
   545 550 555 560
   Glu Phe Ser Pro Leu Ile Ile Lys Glu Trp Ala Ala Tyr Lys Gly Lys
   565 570 575
   Ser Pro Gln Thr Pro Glu Leu Val Glu Ala Leu Ala Phe Arg Glu Trp
   580 585 590
   Thr Cys Pro Asn Leu Lys Arg Leu Trp Leu Gly Lys Ala Val Glu Asp
   595 600 605
   Lys Asn Arg Arg Met Arg Ala Phe Leu Ala Cys Met Arg Ser Asp Thr
   610 615 620
   Pro Ala Met Leu Asn Pro Ala Asn Val Pro Thr His Leu Met Val Leu
   625 630 635 640
   Cys Cys Val Leu Arg Tyr Met Val Gln Trp Pro Gly Ala Arg Ile Leu
   645 650 655
   Arg Arg Gln Glu Leu Asp Ala Phe Leu Ala Gln Ala Leu Ser Pro Lys
   660 665 670
   Leu Tyr Glu Pro Asp Gln Leu Gln Glu Leu Lys Ile Glu Asn Leu Asp
   675 680 685
   Pro Arg Gly Ile Gln Leu Ser Ala Leu Phe Met Ser Gly Val Asp Met
   690 695 700
   Ala Leu Phe Ala Asn Asp Ala Cys Gly Gln Pro Ile Pro Trp Glu His
   705 710 715 720
   Cys Cys Pro Trp Met Tyr Phe Asp Gly Lys Leu Phe Gln Ser Lys Leu
   725 730 735
   Leu Lys Ala Ser Arg Glu Lys Thr Pro Leu Ile Asp Leu Cys Asp Gly
   740 745 750
   Gln Ala Glu Gln Ala Ala Lys Val Glu Lys Met Arg Gln Ser Ile Leu
   755 760 765
   Glu Gly Leu Asn Phe Ser Arg Gln Asn His Pro Leu Pro Phe Pro Pro
   770 775 780
   Pro Pro Ala Leu Pro Phe Tyr Pro Ala Ser Val Tyr Pro Arg His Phe
   785 790 795 800
   Gly Pro Val Pro Pro Ser Gln Gly Arg Gly Arg Gly Phe Ala Gly Val
   805 810 815
   Cys Gly Phe Gly Gly His Tyr Gly Glu Thr Val Ala Thr Gly Pro Tyr
   820 825 830
   Arg Ala Phe Arg Val Thr Ala Ala Ser Gly His Cys Gly Ala Phe Ser
   835 840 845
   Gly Ser Asp Ser Asn Arg Thr Ser Lys Ser Gln Gly Gly Val Gln Pro
   850 855 860
   Ile Pro Ser Gln Gly Gly Lys Leu Glu Ile Ala Gly Thr Val Val Gly
   865 870 875 880
   His Trp Ala Gly Ser Arg Arg Gly Arg Gly Gly Arg Gly Pro Phe Pro
   885 890 895
   Leu Gln Val Val Ser Val Gly Gly Pro Ala Arg Gly Arg Pro Arg Gly
   900 905 910
   Val Ile Ser Thr Pro Val Ile Arg Thr Phe Gly Arg Gly Gly Arg Tyr
   915 920 925
   Tyr Gly Arg Gly Tyr Lys Ser Gln Gly Ala Ile Gln Gly Arg Pro Pro
   930 935 940
   Tyr Ala Ala Ser Ala Glu Glu Val Ala Lys Glu Leu Lys Ser Lys Ser
   945 950 955 960
   Gly Glu Ser Lys Ser Ser Ala Val Ser Ser Glu Gly Ser Leu Ala Glu
   965 970 975
   Asn Gly Val Met Ala Glu Glu Lys Pro Val Pro Gln Leu Asn Gly Ser
   980 985 990
   Thr Ala Asp Thr Arg Ala Pro Ser His Ser Glu Ser Ala Leu Asn Asn
   995 1000 1005
   Asp Ser Lys Thr Cys Asn Thr Asn Pro His Leu Asn Ala Leu Ser Thr
   1010 1015 1020
   Asp Ser Ala Cys Arg Arg Glu Ala Ala Leu Glu Ala Ala Val Leu Asn
   1025 1030 1035 1040
   Lys Glu Glu
<210> 40
   <211> 4679
   <212> DNA
   <213> homo sapiens
<220>
   <221> CDS
   <222> (151)..(3504)
<400> 40
   ccgcggcggc catgagcgcg cccccgaccc gccccagtcc cccctagagg ccgccgcccc 60
   cgcccgccag cccgcccgcg cgccacggcc ccaccacccc cggccccgcc gccccccgcc 120
   cgcacccgcg cccgcgcccc cgccgccgcc atg ggc gtg cag ggc ttc cag gac  174
                                    Met Gly Val Gln Gly Phe Gln Asp
                                    1               5
   tac atc gag aag cac tgc ccg agc gcc gtg gtg ccg gtg gag ctg cag   222
   Tyr Ile Glu Lys His Cys Pro Ser Ala Val Val Pro Val Glu Leu Gln
       10                  15                  20
   aag ctg gcc cgg ggc agc ctg gtg ggc ggc ggg cgg cag cgg ccc ccg   270
   Lys Leu Ala Arg Gly Ser Leu Val Gly Gly Gly Arg Gln Arg Pro Pro
   25                  30                  35                  40
   cag acc ccg ctg cgc ctg ctg gtg gac gcc gac aac tgc ctg cac cgc   318
   Gln Thr Pro Leu Arg Leu Leu Val Asp Ala Asp Asn Cys Leu His Arg
                   45                  50                  55
   ctc tac ggc ggc ttc tac acc gac tgg gtc agc ggc ggc cag tgg aac   366
   Leu Tyr Gly Gly Phe Tyr Thr Asp Trp Val Ser Gly Gly Gln Trp Asn
               60                  65                  70
   cac atg ctt ggc tac ctg gcg gcg ctg gcc aag gcc tgc ttc ggc ggc   414
   His Met Leu Gly Tyr Leu Ala Ala Leu Ala Lys Ala Cys Phe Gly Gly
           75                  80                  85
   aac atc gag ctc ttc gtc ttc ttc aac ggc gcg ctc gag aag gcc cgg   462
   Asn Ile Glu Leu Phe Val Phe Phe Asn Gly Ala Leu Glu Lys Ala Arg
       90                  95                  100
   ctg cac gag tgg gtc aag cgg cag ggc aac gag cgc cag acg gca cag   510
   Leu His Glu Trp Val Lys Arg Gln Gly Asn Glu Arg Gln Thr Ala Gln
   105                 110                 115                 120
   cag atc gtc agc cat gtc cag aac aag ggc acc ccg ccg cca aag gtc   558
   Gln Ile Val Ser His Val Gln Asn Lys Gly Thr Pro Pro Pro Lys Val
                   125                 130                 135
   tgg ttc ctg ccg ccc gtc tgc atg gcc cac tgc atc cgc ctg gcg ctc   606
   Trp Phe Leu Pro Pro Val Cys Met Ala His Cys Ile Arg Leu Ala Leu
               140                 145                 150
   atc cgc ttc cac gtc aag gtt gca cag agc att gag gat cac cat cag   654
   Ile Arg Phe His Val Lys Val Ala Gln Ser Ile Glu Asp His His Gln
           155                 160                 165
   gaa gtg att ggt ttc tgc aga gag aat ggt ttc cat ggc ttg gtt gcg   702
   Glu Val Ile Gly Phe Cys Arg Glu Asn Gly Phe His Gly Leu Val Ala
       170                 175                 180
   tat gac tct gat tat gca ctg tgc aac atc ccc tac tat ttc agt gcc   750
   Tyr Asp Ser Asp Tyr Ala Leu Cys Asn Ile Pro Tyr Tyr Phe Ser Ala
   185 190 195 200
   cat gcc cta aaa ctg agc cgg aac ggg aaa agt ctc acc aca agc caa 798
   His Ala Leu Lys Leu Ser Arg Asn Gly Lys Ser Leu Thr Thr Ser Gln
   205 210 215
   tat ctg atg cat gaa gtt gcc aag caa ctg gac ctg aat cca aat cgt 846
   Tyr Leu Met His Glu Val Ala Lys Gln Leu Asp Leu Asn Pro Asn Arg
   220 225 230
   ttt cct att ttt gct gct ctc tta gga aat cac att ctg cct gat gaa 894
   Phe Pro Ile Phe Ala Ala Leu Leu Gly Asn His Ile Leu Pro Asp Glu
   235 240 245
   gat ctg gct tcc ttt cat tgg agt tta ctt ggt cca gaa cat cca cta 942
   Asp Leu Ala Ser Phe His Trp Ser Leu Leu Gly Pro Glu His Pro Leu
   250 255 260
   gcc tca cta aag gtc cgg gcc cac cag ctg gtc ttg cca cct tgc gac 990
   Ala Ser Leu Lys Val Arg Ala His Gln Leu Val Leu Pro Pro Cys Asp
   265 270 275 280
   gta gtg atc aaa gcc gtt gct gac tat gta cgc aac att cag gac acc 1038
   Val Val Ile Lys Ala Val Ala Asp Tyr Val Arg Asn Ile Gln Asp Thr
   285 290 295
   tct gac ttg gat gcc ata gct aaa gat gtt ttc cag cat tca cag tct 1086
   Ser Asp Leu Asp Ala Ile Ala Lys Asp Val Phe Gln His Ser Gln Ser
   300 305 310
   aga aca gat gac aaa gtt att cga ttt aag aga gca att gga tat tat 1134
   Arg Thr Asp Asp Lys Val Ile Arg Phe Lys Arg Ala Ile Gly Tyr Tyr
   315 320 325
   tca gcg act agt aag cct atg tca ttt cat cca cca cat tac tta gca 1182
   Ser Ala Thr Ser Lys Pro Met Ser Phe His Pro Pro His Tyr Leu Ala
   330 335 340
   gcc aga ccc ggt ccg ttt gga atg cct ggg atg gtg ccg ccg cat gtt 1230
   Ala Arg Pro Gly Pro Phe Gly Met Pro Gly Met Val Pro Pro His Val
   345 350 355 360
   cct cct cag atg ctc aac att ccg cag acc tct ctg caa gca aag ccc 1278
   Pro Pro Gln Met Leu Asn Ile Pro Gln Thr Ser Leu Gln Ala Lys Pro
   365 370 375
   gtg gcc cca cag gtg ccc agc cca ggg ggc gcc ccg ggc cag ggt cca 1326
   Val Ala Pro Gln Val Pro Ser Pro Gly Gly Ala Pro Gly Gln Gly Pro
   380 385 390
   tac ccg tac agc ctc tct gag cca gca cct ctc act ttg gac acg agc 1374
   Tyr Pro Tyr Ser Leu Ser Glu Pro Ala Pro Leu Thr Leu Asp Thr Ser
   395 400 405
   ggg aag aat ctg acg gag cag aac agc tac agc aac att cct cac gaa 1422
   Gly Lys Asn Leu Thr Glu Gln Asn Ser Tyr Ser Asn Ile Pro His Glu
   410 415 420
   ggg aag cac acg ccg ctg tat gag cgg tcc tcg ccc atc aac cgg gcc 1470
   Gly Lys His Thr Pro Leu Tyr Glu Arg Ser Ser Pro Ile Asn Arg Ala
   425                 430                 435                 440
   cag agc ggc agc ccc aac cac gtg gat tcc gcc tac ttc cct ggc tct    1518
   Gln Ser Gly Ser Pro Asn His Val Asp Ser Ala Tyr Phe Pro Gly Ser
                   445                 450                 455
   tct aca tcg tca tct tcc gac aac gac gag ggc agc gga ggg gcg aca    1566
   Ser Thr Ser Ser Ser Ser Asp Asn Asp Glu Gly Ser Gly Gly Ala Thr
               460                 465                 470
   aac cat atc agc ggg aac aag att ggc tgg gag aag acg gga agc cac    1614
   Asn His Ile Ser Gly Asn Lys Ile Gly Trp Glu Lys Thr Gly Ser His
           475                 480                 485
   tca gag cct cag gca cga gga gac cca gga gac caa aca aag gca gaa    1662
   Ser Glu Pro Gln Ala Arg Gly Asp Pro Gly Asp Gln Thr Lys Ala Glu
       490                 495                 500
   ggc tcg tcc act gcc tct tca gga agc caa cta gcc gaa ggc aag gga    1710
   Gly Ser Ser Thr Ala Ser Ser Gly Ser Gln Leu Ala Glu Gly Lys Gly
   505                 510                 515                 520
   agc cag atg ggc act gtc cag cca atc ccg tgc ctc ctg tcg atg ccc    1758
   Ser Gln Met Gly Thr Val Gln Pro Ile Pro Cys Leu Leu Ser Met Pro
                   525                 530                 535
   acc agg aac cac atg gac atc acc aca cct ccc ctg ccc ccc gtc gca    1806
   Thr Arg Asn His Met Asp Ile Thr Thr Pro Pro Leu Pro Pro Val Ala
               540                 545                 550
   cct gag gtg ctg aga gtg gcc gag cac agg cac aag aag ggg ctg atg    1854
   Pro Glu Val Leu Arg Val Ala Glu His Arg His Lys Lys Gly Leu Met
           555                 560                 565
   tac ccc tac atc ttc cat gtc ctg acg aag ggt gaa atc aaa att gct    1902
   Tyr Pro Tyr Ile Phe His Val Leu Thr Lys Gly Glu Ile Lys Ile Ala
       570                 575                 580
   gtt tct att gaa gat gaa gcc aac aag gac ctg cct ccg gcc gct ctg    1950
   Val Ser Ile Glu Asp Glu Ala Asn Lys Asp Leu Pro Pro Ala Ala Leu
   585                 590                 595                 600
   ctc tat agg cca gtt cgt cag tat gtt tac gga gtc ctg ttt agt ttg    1998
   Leu Tyr Arg Pro Val Arg Gln Tyr Val Tyr Gly Val Leu Phe Ser Leu
                   605                 610                 615
   gca gaa agc aga aag aaa act gag aga ctt gct ttt aga aag aac aga    2046
   Ala Glu Ser Arg Lys Lys Thr Glu Arg Leu Ala Phe Arg Lys Asn Arg
               620                 625                 630
   ctt cca cca gaa ttt tca cca gtg atc att aaa gaa tgg gca gct tac    2094
   Leu Pro Pro Glu Phe Ser Pro Val Ile Ile Lys Glu Trp Ala Ala Tyr
           635                 640                 645
   aaa gga aag tct cct caa acc ccg gaa ctg gtt gaa gct ctt gcc ttc    2142
   Lys Gly Lys Ser Pro Gln Thr Pro Glu Leu Val Glu Ala Leu Ala Phe
       650                 655                 660
   agg gag tgg acc tgc ccc aac ctg aag agg ctg tgg ttg ggt aag gcg    2190
   Arg Glu Trp Thr Cys Pro Asn Leu Lys Arg Leu Trp Leu Gly Lys Ala
   665                 670                 675                 680
   gta gag gac aag aac cgc agg atg agg gcc ttc ctg gcc tgc atg agg    2238
   Val Glu Asp Lys Asn Arg Arg Met Arg Ala Phe Leu Ala Cys Met Arg
                   685                 690                 695
   tcg gac acc cca gcc atg ctc aac cct gcc aac gtg ccc act cac ctc    2286
   Ser Asp Thr Pro Ala Met Leu Asn Pro Ala Asn Val Pro Thr His Leu
               700                 705                 710
   atg gtg ctc tgc tgc gtc tta cgg tac atg gtg cag tgg ccg gga gca    2334
   Met Val Leu Cys Cys Val Leu Arg Tyr Met Val Gln Trp Pro Gly Ala
           715                 720                 725
   tgc atc ctt cgg cgt cag gag cta gat gcc ttc ctg gct cag gcg ctg    2382
   Cys Ile Leu Arg Arg Gln Glu Leu Asp Ala Phe Leu Ala Gln Ala Leu
       730                 735                 740
   tcc ccc aaa ctc tac gag cct gat cag ctc cag gag ctc aag att gag    2430
   Ser Pro Lys Leu Tyr Glu Pro Asp Gln Leu Gln Glu Leu Lys Ile Glu
   745                 750                 755                 760
   aac cta gat ccc cga gga att cag cta tca gct ctc ttc atg agt gga    2478
   Asn Leu Asp Pro Arg Gly Ile Gln Leu Ser Ala Leu Phe Met Ser Gly
                   765                 770                 775
   gta gac atg gcc ttg ttt gca aat gat gca tgc gga cag cca atc ccc    2526
   Val Asp Met Ala Leu Phe Ala Asn Asp Ala Cys Gly Gln Pro Ile Pro
               780                 785                 790
   tgg gaa cac tgt tgt cct tgg atg tat ttt gat ggg aag ctc ttc caa    2574
   Trp Glu His Cys Cys Pro Trp Met Tyr Phe Asp Gly Lys Leu Phe Gln
           795                 800                 805
   tcc aaa ctc ctc aaa gcc agc cgg gaa aag acc cca ctc att gac ctc    2622
   Ser Lys Leu Leu Lys Ala Ser Arg Glu Lys Thr Pro Leu Ile Asp Leu
       810                 815                 820
   tgt gat ggt cag gct gat cag gct gcc aag gta gag aag atg cgc cag    2670
   Cys Asp Gly Gln Ala Asp Gln Ala Ala Lys Val Glu Lys Met Arg Gln
   825                 830                 835                 840
   agc gtc ctc gag ggg ctc agc ttc tcc agg cag agc cac acg ctc cct    2718
   Ser Val Leu Glu Gly Leu Ser Phe Ser Arg Gln Ser His Thr Leu Pro
                   845                 850                 855
   ttc ccg ccg cca cct gcc ctg ccc ttc tac cct gcc tct gcg tac ccc    2766
   Phe Pro Pro Pro Pro Ala Leu Pro Phe Tyr Pro Ala Ser Ala Tyr Pro
               860                 865                 870
   cgg cac ttt ggg cct gtc cca ccc tct cag ggc agg ggc aga ggc ttt    2814
   Arg His Phe Gly Pro Val Pro Pro Ser Gln Gly Arg Gly Arg Gly Phe
           875                 880                 885
   gca ggc gtc tgt ggc ttt gga ggc ccc tat ggg gaa acg gta gca aca    2862
   Ala Gly Val Cys Gly Phe Gly Gly Pro Tyr Gly Glu Thr Val Ala Thr
       890                 895                 900
   ggc cct tac cgt gcc ttc cgt gtg gcg gca gca tcg gga cac tgc gga    2910
   Gly Pro Tyr Arg Ala Phe Arg Val Ala Ala Ala Ser Gly His Cys Gly
   905                 910                 915                 920
   gcc ttc tca ggc agt gac agc agc agg act agc aag tcc cag ggc gga    2958
   Ala Phe Ser Gly Ser Asp Ser Ser Arg Thr Ser Lys Ser Gln Gly Gly
                   925                 930                 935
   gtc caa cct ata cct tct cag gga ggc aaa cta gaa ata gct ggc act    3006
   Val Gln Pro Ile Pro Ser Gln Gly Gly Lys Leu Glu Ile Ala Gly Thr
               940                 945                 950
   gtg gtt ggc cat tgg gct ggg agc agg cgg ggc cgt ggg ggc cgg ggg    3054
   Val Val Gly His Trp Ala Gly Ser Arg Arg Gly Arg Gly Gly Arg Gly
           955                 960                 965
   cct ttc ccc ctg cag gtg gtt tct gtc gga gga cca gct aga ggg cgt    3102
   Pro Phe Pro Leu Gln Val Val Ser Val Gly Gly Pro Ala Arg Gly Arg
       970                 975                 980
   cca aga gga gtt att tcc acc cca gtg att aga aca ttt gga aga ggt    3150
   Pro Arg Gly Val Ile Ser Thr Pro Val Ile Arg Thr Phe Gly Arg Gly
   985                 990                 995                 1000
   gga agg tac tat ggc aga ggt tac aaa aac cag gca gca att cag ggc    3198
   Gly Arg Tyr Tyr Gly Arg Gly Tyr Lys Asn Gln Ala Ala Ile Gln Gly
                   1005                1010                1015
   aga cct cct tat gct gct tca gca gaa gaa gtg gcc aaa gaa ctt aag    3246
   Arg Pro Pro Tyr Ala Ala Ser Ala Glu Glu Val Ala Lys Glu Leu Lys
               1020                1025                1030
   tca aaa tct ggg gaa tcg aag tcc tct gct atg tct tca gac ggg tcc    3294
   Ser Lys Ser Gly Glu Ser Lys Ser Ser Ala Met Ser Ser Asp Gly Ser
           1035                1040                1045
   ctg gct gaa aac gga gtg atg gcc gag gag aag ccg gct ccc cag atg    3342
   Leu Ala Glu Asn Gly Val Met Ala Glu Glu Lys Pro Ala Pro Gln Met
       1050                1055                1060
   aac ggg agc acg ggt gac gcc agg gcc ccc agc cac tct gaa agt gcc    3390
   Asn Gly Ser Thr Gly Asp Ala Arg Ala Pro Ser His Ser Glu Ser Ala
   1065                1070                1075                1080
   ttg aat aat gac tct aaa acg tgc aat aca aat cct cat tta aat gca    3438
   Leu Asn Asn Asp Ser Lys Thr Cys Asn Thr Asn Pro His Leu Asn Ala
                   1085                1090                1095
   cta agt aca gac agc gct tgc cgc aga gaa gct gct ctg gag gca gct    3486
   Leu Ser Thr Asp Ser Ala Cys Arg Arg Glu Ala Ala Leu Glu Ala Ala
               1100                1105                1110
   gtc tta aat aaa gaa gag taaacttatt ttttatagag ggtgaaggat           3534
   Val Leu Asn Lys Glu Glu
           1115
   gctggaaggg taaggattta ggaatatctg gagagaaaga gagcctgcag ttatgtacat  3594
   tttgtccttt ccgtaagaga aaaatgagga ctttggaaat tcagatccct ctttgatatc  3654
   agagatttaa acaacacatt tttagtttta accagttgta gtcaaaatgc tacaataaaa  3714
   caaaaaagag aaagaaaatg aagagcattt gactcccgca cttaaaatga agtacacata  3774
   aagtttaaac tggttatgac aaaagcctat agttgtgttt cttgaactat aaagaaaaca  3834
   aattttggca gtctttaagt atatatagct taaaatataa tttttagcat ttggcaccat 3894
   atgtatgcca ttatatttga ttttgcatta ctgtttcaca atgaagcttt ctttaaggct 3954
   ttgattttta tgattatgaa agaaataagg cacaaccaca gtttttcttt cttaaatttc 4014
   atcactgttg atgtggttct tttgtgttaa aaaaaaaaag tgcaactatc aaaactaaaa 4074
   aattatagag taatattgcc gttctgctga ttttaaatat acaatacatc atacatactt 4134
   tacaagcaag ttaaatggag ataaagttga aatcatagaa gatgcaaatg acctttcaaa 4194
   atcaacacaa tgtgttctga aactttcgtg actaatacca tgcatctgtg atcaatgaac 4254
   tatgtggttt tgaatcggat gtagaccatt agtactacta cttgagctaa acttctgcat 4314
   ggttcataat ttttaaagtg tgtagttaat atgcatgtta tcgtcctttc ttccattctt 4374
   aacagtatgt gcccatttgc aaaacaaaaa tgctaataat cagtaatagt cctataaaag 4434
   atgttaactc tgtttagtca ttgactgatc ttgctctaac cttaaaattt tgtgattatt 4494
   gacctctgtt gcatttattc taaagccccc caaaaattat ctagccgttt cgaatatcaa 4554
   cattaccctg gtgtattcac tgctgtatgc attattgttc tttgttgctg ttttatgcct 4614
   tcatattagc aaatatgaaa ttctgtgaaa aaaaaccctt tgatcttaaa aaaaaaaaaa 4674
   aaaaa 4679
<210> 41
   <211> 1118
   <212> PRT
   <213> homo sapiens
<400> 41
   Met Gly Val Gln Gly Phe Gln Asp Tyr Ile Glu Lys His Cys Pro Ser
   1 5 10 15
   Ala Val Val Pro Val Glu Leu Gln Lys Leu Ala Arg Gly Ser Leu Val
   20 25 30
   Gly Gly Gly Arg Gln Arg Pro Pro Gln Thr Pro Leu Arg Leu Leu Val
   35 40 45
   Asp Ala Asp Asn Cys Leu His Arg Leu Tyr Gly Gly Phe Tyr Thr Asp
   50 55 60
   Trp Val Ser Gly Gly Gln Trp Asn His Met Leu Gly Tyr Leu Ala Ala
   65 70 75 80
   Leu Ala Lys Ala Cys Phe Gly Gly Asn Ile Glu Leu Phe Val Phe Phe
   85 90 95
   Asn Gly Ala Leu Glu Lys Ala Arg Leu His Glu Trp Val Lys Arg Gln
   100 105 110
   Gly Asn Glu Arg Gln Thr Ala Gln Gln Ile Val Ser His Val Gln Asn
   115 120 125
   Lys Gly Thr Pro Pro Pro Lys Val Trp Phe Leu Pro Pro Val Cys Met
       130                 135                 140
   Ala His Cys Ile Arg Leu Ala Leu Ile Arg Phe His Val Lys Val Ala
   145                 150                 155                 160
   Gln Ser Ile Glu Asp His His Gln Glu Val Ile Gly Phe Cys Arg Glu
                   165                 170                 175
   Asn Gly Phe His Gly Leu Val Ala Tyr Asp Ser Asp Tyr Ala Leu Cys
               180                 185                 190
   Asn Ile Pro Tyr Tyr Phe Ser Ala His Ala Leu Lys Leu Ser Arg Asn
           195                 200                 205
   Gly Lys Ser Leu Thr Thr Ser Gln Tyr Leu Met His Glu Val Ala Lys
       210                 215                 220
   Gln Leu Asp Leu Asn Pro Asn Arg Phe Pro Ile Phe Ala Ala Leu Leu
   225                 230                 235                 240
   Gly Asn His Ile Leu Pro Asp Glu Asp Leu Ala Ser Phe His Trp Ser
                   245                 250                 255
   Leu Leu Gly Pro Glu His Pro Leu Ala Ser Leu Lys Val Arg Ala His
               260                 265                 270
   Gln Leu Val Leu Pro Pro Cys Asp Val Val Ile Lys Ala Val Ala Asp
           275                 280                 285
   Tyr Val Arg Asn Ile Gln Asp Thr Ser Asp Leu Asp Ala Ile Ala Lys
       290                 295                 300
   Asp Val Phe Gln His Ser Gln Ser Arg Thr Asp Asp Lys Val Ile Arg
   305                 310                 315                 320
   Phe Lys Arg Ala Ile Gly Tyr Tyr Ser Ala Thr Ser Lys Pro Met Ser
                   325                 330                 335
   Phe His Pro Pro His Tyr Leu Ala Ala Arg Pro Gly Pro Phe Gly Met
               340                 345                 350
   Pro Gly Met Val Pro Pro His Val Pro Pro Gln Met Leu Asn Ile Pro
           355                 360                 365
   Gln Thr Ser Leu Gln Ala Lys Pro Val Ala Pro Gln Val Pro Ser Pro
       370                 375                 380
   Gly Gly Ala Pro Gly Gln Gly Pro Tyr Pro Tyr Ser Leu Ser Glu Pro
   385                 390                 395                 400
   Ala Pro Leu Thr Leu Asp Thr Ser Gly Lys Asn Leu Thr Glu Gln Asn
                   405                 410                 415
   Ser Tyr Ser Asn Ile Pro His Glu Gly Lys His Thr Pro Leu Tyr Glu
               420                 425                 430
   Arg Ser Ser Pro Ile Asn Arg Ala Gln Ser Gly Ser Pro Asn His Val
           435                 440                 445
   Asp Ser Ala Tyr Phe Pro Gly Ser Ser Thr Ser Ser Ser Ser Asp Asn
   450 455 460
   Asp Glu Gly Ser Gly Gly Ala Thr Asn His Ile Ser Gly Asn Lys Ile
   465 470 475 480
   Gly Trp Glu Lys Thr Gly Ser His Ser Glu Pro Gln Ala Arg Gly Asp
   485 490 495
   Pro Gly Asp Gln Thr Lys Ala Glu Gly Ser Ser Thr Ala Ser Ser Gly
   500 505 510
   Ser Gln Leu Ala Glu Gly Lys Gly Ser Gln Met Gly Thr Val Gln Pro
   515 520 525
   Ile Pro Cys Leu Leu Ser Met Pro Thr Arg Asn His Met Asp Ile Thr
   530 535 540
   Thr Pro Pro Leu Pro Pro Val Ala Pro Glu Val Leu Arg Val Ala Glu
   545 550 555 560
   His Arg His Lys Lys Gly Leu Met Tyr Pro Tyr Ile Phe His Val Leu
   565 570 575
   Thr Lys Gly Glu Ile Lys Ile Ala Val Ser Ile Glu Asp Glu Ala Asn
   580 585 590
   Lys Asp Leu Pro Pro Ala Ala Leu Leu Tyr Arg Pro Val Arg Gln Tyr
   595 600 605
   Val Tyr Gly Val Leu Phe Ser Leu Ala Glu Ser Arg Lys Lys Thr Glu
   610 615 620
   Arg Leu Ala Phe Arg Lys Asn Arg Leu Pro Pro Glu Phe Ser Pro Val
   625 630 635 640
   Ile Ile Lys Glu Trp Ala Ala Tyr Lys Gly Lys Ser Pro Gln Thr Pro
   645 650 655
   Glu Leu Val Glu Ala Leu Ala Phe Arg Glu Trp Thr Cys Pro Asn Leu
   660 665 670
   Lys Arg Leu Trp Leu Gly Lys Ala Val Glu Asp Lys Asn Arg Arg Met
   675 680 685
   Arg Ala Phe Leu Ala Cys Met Arg Ser Asp Thr Pro Ala Met Leu Asn
   690 695 700
   Pro Ala Asn Val Pro Thr His Leu Met Val Leu Cys Cys Val Leu Arg
   705 710 715 720
   Tyr Met Val Gln Trp Pro Gly Ala Cys Ile Leu Arg Arg Gln Glu Leu
   725 730 735
   Asp Ala Phe Leu Ala Gln Ala Leu Ser Pro Lys Leu Tyr Glu Pro Asp
   740 745 750
   Gln Leu Gln Glu Leu Lys Ile Glu Asn Leu Asp Pro Arg Gly Ile Gln
   755 760 765
   Leu Ser Ala Leu Phe Met Ser Gly Val Asp Met Ala Leu Phe Ala Asn
   770 775 780
   Asp Ala Cys Gly Gln Pro Ile Pro Trp Glu His Cys Cys Pro Trp Met
   785 790 795 800
   Tyr Phe Asp Gly Lys Leu Phe Gln Ser Lys Leu Leu Lys Ala Ser Arg
   805 810 815
   Glu Lys Thr Pro Leu Ile Asp Leu Cys Asp Gly Gln Ala Asp Gln Ala
   820 825 830
   Ala Lys Val Glu Lys Met Arg Gln Ser Val Leu Glu Gly Leu Ser Phe
   835 840 845
   Ser Arg Gln Ser His Thr Leu Pro Phe Pro Pro Pro Pro Ala Leu Pro
   850 855 860
   Phe Tyr Pro Ala Ser Ala Tyr Pro Arg His Phe Gly Pro Val Pro Pro
   865 870 875 880
   Ser Gln Gly Arg Gly Arg Gly Phe Ala Gly Val Cys Gly Phe Gly Gly
   885 890 895
   Pro Tyr Gly Glu Thr Val Ala Thr Gly Pro Tyr Arg Ala Phe Arg Val
   900 905 910
   Ala Ala Ala Ser Gly His Cys Gly Ala Phe Ser Gly Ser Asp Ser Ser
   915 920 925
   Arg Thr Ser Lys Ser Gln Gly Gly Val Gln Pro Ile Pro Ser Gln Gly
   930 935 940
   Gly Lys Leu Glu Ile Ala Gly Thr Val Val Gly His Trp Ala Gly Ser
   945 950 955 960
   Arg Arg Gly Arg Gly Gly Arg Gly Pro Phe Pro Leu Gln Val Val Ser
   965 970 975
   Val Gly Gly Pro Ala Arg Gly Arg Pro Arg Gly Val Ile Ser Thr Pro
   980 985 990
   Val Ile Arg Thr Phe Gly Arg Gly Gly Arg Tyr Tyr Gly Arg Gly Tyr
   995 1000 1005
   Lys Asn Gln Ala Ala Ile Gln Gly Arg Pro Pro Tyr Ala Ala Ser Ala
   1010 1015 1020
   Glu Glu Val Ala Lys Glu Leu Lys Ser Lys Ser Gly Glu Ser Lys Ser
   1025 1030 1035 1040
   Ser Ala Met Ser Ser Asp Gly Ser Leu Ala Glu Asn Gly Val Met Ala
   1045 1050 1055
   Glu Glu Lys Pro Ala Pro Gln Met Asn Gly Ser Thr Gly Asp Ala Arg
   1060 1065 1070
   Ala Pro Ser His Ser Glu Ser Ala Leu Asn Asn Asp Ser Lys Thr Cys
   1075 1080 1085
   Asn Thr Asn Pro His Leu Asn Ala Leu Ser Thr Asp Ser Ala Cys Arg
   1090 1095 1100
   Arg Glu Ala Ala Leu Glu Ala Ala Val Leu Asn Lys Glu Glu
   1105 1110 1115
<210> 42
   <211> 494
   <212> PRT
   <213> homo sapiens
<220>
   <222> (1)..(494)
   <223> unknown [Homo sapiens].
<300>
   <301> Andersson, et al. Vogel, et al. Lbeit, et al. Ohara, O
   <302> Unknown protein [Homo sapiens].
   <308> GI 3005744
   <309> 1998-04-02
   <313> 1 TO 494
<400> 42
   Gly Glu Ile Lys Ile Ala Val Ser Ile Glu Asp Glu Ala Asn Lys Asp
   1 5 10 15
   Leu Pro Pro Ala Ala Leu Leu Tyr Arg Pro Val Arg Gln Tyr Val Tyr
   20 25 30
   Gly Val Leu Phe Ser Leu Ala Glu Ser Arg Lys Lys Thr Glu Arg Leu
   35 40 45
   Ala Phe Arg Lys Asn Arg Leu Pro Pro Glu Phe Ser Pro Val Ile Ile
   50 55 60
   Lys Glu Trp Ala Ala Tyr Lys Gly Lys Ser Pro Gln Thr Pro Glu Leu
   65 70 75 80
   Val Glu Ala Leu Ala Phe Arg Glu Trp Thr Cys Pro Asn Leu Lys Arg
   85 90 95
   Leu Trp Leu Gly Lys Ala Val Glu Asp Lys Asn Arg Arg Met Arg Ala
   100 105 110
   Phe Leu Ala Cys Met Arg Ser Asp Thr Pro Ala Met Leu Asn Pro Ala
   115 120 125
   Asn Val Pro Thr His Leu Met Val Leu Cys Cys Val Leu Arg Tyr Met
   130 135 140
   Val Gln Trp Pro Gly Ala Arg Ile Leu Arg Arg Gln Glu Leu Asp Ala
   145 150 155 160
   Phe Leu Ala Gln Ala Leu Ser Pro Lys Leu Tyr Glu Pro Asp Gln Leu
   165 170 175
   Gln Glu Leu Lys Ile Glu Asn Leu Asp Pro Arg Gly Ile Gln Leu Ser
   180 185 190
   Ala Leu Phe Met Ser Gly Val Asp Met Ala Leu Phe Ala Asn Asp Ala
           195                 200                 205
   Cys Gly Gln Pro Ile Pro Trp Glu His Cys Cys Pro Trp Met Tyr Phe
       210                 215                 220
   Asp Gly Lys Leu Phe Gln Ser Lys Leu Leu Lys Ala Ser Arg Glu Lys
   225                 230                 235                 240
   Thr Pro Leu Ile Asp Leu Cys Asp Gly Gln Ala Asp Gln Ala Ala Lys
                   245                 250                 255
   Val Glu Lys Met Arg Gln Ser Val Leu Glu Gly Leu Ser Phe Ser Arg
               260                 265                 270
   Gln Ser His Thr Leu Pro Phe Pro Pro Pro Pro Ala Leu Pro Phe Tyr
           275                 280                 285
   Pro Ala Ser Ala Tyr Pro Arg His Phe Gly Pro Val Pro Pro Ser Gln
       290                 295                 300
   Gly Arg Gly Arg Gly Phe Ala Gly Val Gln Pro Ile Pro Ser Gln Gly
   305                 310                 315                 320
   Gly Lys Leu Glu Ile Ala Gly Thr Val Val Gly His Trp Ala Gly Ser
                   325                 330                 335
   Arg Arg Gly Arg Gly Gly Arg Gly Pro Phe Pro Leu Gln Val Val Ser
               340                 345                 350
   Val Gly Gly Pro Ala Arg Gly Arg Pro Arg Gly Val Ile Ser Thr Pro
           355                 360                 365
   Val Ile Arg Thr Phe Gly Arg Gly Gly Arg Tyr Tyr Gly Arg Gly Tyr
       370                 375                 380
   Lys Asn Gln Ala Ala Ile Gln Gly Arg Pro Pro Tyr Ala Ala Ser Ala
   385                 390                 395                 400
   Glu Glu Val Ala Lys Glu Leu Lys Ser Lys Ser Gly Glu Ser Lys Ser
                   405                 410                 415
   Ser Ala Met Ser Ser Asp Gly Ser Leu Ala Glu Asn Gly Val Met Ala
               420                 425                 430
   Glu Glu Lys Pro Ala Pro Gln Met Asn Gly Ser Thr Gly Asp Ala Arg
           435                 440                 445
   Ala Pro Ser His Ser Glu Ser Ala Leu Asn Asn Asp Ser Lys Thr Cys
       450                 455                 460
   Asn Thr Asn Pro His Leu Asn Ala Leu Ser Thr Asp Ser Ala Cys Arg
   465                 470                 475                 480
   Arg Glu Ala Ala Leu Glu Ala Ala Val Leu Asn Lys Glu Glu
                   485                 490
<210> 43
   <211> 1063
   <212> PRT
   <213> Rattus sp.
<400> 43
   Asp Tyr Ile Glu Lys His Cys Pro Ser Ala Val Val Pro Val Glu Leu
   1               5                   10                  15
   Gln Lys Leu Ala Arg Gly Ser Leu Val Gly Gly Gly Arg Gln Arg Pro
               20                  25                  30
   Pro Gln Thr Pro Leu Arg Leu Leu Val Asp Ala Asp Asn Cys Leu His
           35                  40                  45
   Arg Leu Tyr Gly Gly Phe Tyr Thr Asp Trp Val Ser Gly Gly Gln Trp
       50                  55                  60
   Asn His Met Leu Gly Tyr Leu Ala Ala Leu Ala Lys Ala Cys Phe Gly
   65                  70                  75                  80
   Gly Asn Ile Glu Leu Phe Val Phe Phe Asn Gly Ala Leu Glu Lys Ala
                   85                  90                  95
   Arg Leu His Glu Trp Val Lys Arg Gln Gly Asn Glu Arg Gln Thr Ala
               100                 105                 110
   Gln Gln Ile Val Ser His Val Gln Asn Lys Gly Thr Pro Pro Pro Lys
           115                 120                 125
   Val Trp Phe Leu Pro Pro Val Cys Met Ala His Cys Ile Arg Leu Ala
       130                 135                 140
   Leu Ile Arg Phe His Val Lys Val Ala Gln Ser Ile Glu Asp His His
   145                 150                 155                 160
   Gln Glu Val Ile Gly Phe Cys Arg Glu Asn Gly Phe His Gly Leu Val
                   165                 170                 175
   Ala Tyr Asp Ser Asp Tyr Ala Leu Cys Asn Ile Pro Tyr Tyr Phe Ser
               180                 185                 190
   Ala His Ala Leu Lys Leu Ser Arg Asn Gly Lys Ser Leu Thr Thr Ser
           195                 200                 205
   Gln Tyr Leu Met His Glu Val Ala Lys Gln Leu Asp Leu Asn Pro Asn
       210                 215                 220
   Arg Phe Pro Ile Phe Ala Ala Leu Leu Gly Asn His Ile Leu Pro Asp
   225                 230                 235                 240
   Glu Asp Leu Ala Ser Phe His Trp Ser Leu Leu Gly Pro Glu His Pro
                   245                 250                 255
   Leu Ala Ser Leu Lys Val Arg Ala His Gln Leu Val Leu Pro Pro Cys
               260                 265                 270
   Asp Val Val Ile Lys Ala Val Ala Asp Tyr Val Arg Asn Ile Gln Asp
           275                 280                 285
   Thr Ser Asp Leu Asp Ala Ile Ala Lys Asp Val Phe Gln His Ser Gln
       290                 295                 300
   Ser Arg Thr Asp Asp Lys Val Ile Arg Phe Lys Arg Ala Ile Gly Tyr
   305 310 315 320
   Tyr Ser Ala Thr Ser Lys Pro Met Ser Phe His Pro Pro His Tyr Leu
   325 330 335
   Ala Ala Arg Pro Gly Pro Phe Gly Met Pro Gly Met Val Pro Pro His
   340 345 350
   Val Pro Pro Gln Met Leu Asn Ile Pro Gln Thr Ser Leu Gln Ala Lys
   355 360 365
   Pro Val Ala Pro Gln Val Pro Ser Pro Gly Gly Ala Pro Gly Gln Gly
   370 375 380
   Pro Tyr Pro Tyr Ser Leu Ser Glu Pro Ala Pro Leu Thr Leu Asp Thr
   385 390 395 400
   Ser Gly Lys Asn Leu Thr Glu Gln Asn Ser Tyr Ser Asn Ile Pro His
   405 410 415
   Glu Gly Lys His Thr Pro Leu Tyr Glu Arg Ser Ser Pro Ile Asn Pro
   420 425 430
   Ala Gln Ser Gly Ser Pro Asn His Val Asp Ser Ala Tyr Phe Pro Gly
   435 440 445
   Ser Ser Thr Ser Ser Ser Ser Asp Asn Asp Glu Gly Ser Gly Gly Ala
   450 455 460
   Thr Asn His Ile Ser Gly Asn Lys Ile Gly Trp Glu Lys Thr Gly Ser
   465 470 475 480
   His Ser Glu Pro Gln Ala Arg Gly Asp Pro Gly Asp Gln Thr Lys Ala
   485 490 495
   Glu Gly Ser Ser Thr Ala Ser Ser Gly Ser Gln Leu Ala Glu Gly Lys
   500 505 510
   Gly Ser Gln Met Gly Thr Val Gln Pro Ile Pro Cys Leu Leu Ser Met
   515 520 525
   Pro Thr Arg Asn His Met Asp Ile Thr Thr Pro Pro Leu Pro Pro Val
   530 535 540
   Ala Pro Glu Val Leu Arg Val Ala Glu His Arg His Lys Lys Gly Leu
   545 550 555 560
   Met Tyr Pro Tyr Ile Phe His Val Leu Thr Lys Gly Glu Ile Lys Ile
   565 570 575
   Ala Val Ser Ile Glu Asp Glu Ala Asn Lys Asp Leu Pro Pro Ala Ala
   580 585 590
   Leu Leu Tyr Arg Pro Val Arg Gln Tyr Val Tyr Gly Val Leu Phe Ser
   595 600 605
   Leu Ala Glu Ser Arg Lys Lys Thr Glu Arg Leu Ala Phe Arg Lys Asn
   610 615 620
   Arg Leu Pro Pro Glu Phe Ser Pro Val Ile Ile Lys Glu Trp Ala Ala
   625 630 635 640
   Tyr Lys Gly Lys Ser Pro Gln Thr Pro Glu Leu Val Glu Ala Leu Ala
   645 650 655
   Phe Arg Glu Trp Thr Cys Pro Asn Leu Lys Arg Leu Trp Leu Gly Lys
   660 665 670
   Ala Val Glu Asp Lys Asn Arg Arg Met Arg Ala Phe Leu Ala Cys Met
   675 680 685
   Arg Ser Asp Thr Pro Ala Met Leu Asn Pro Ala Asn Val Pro Thr His
   690 695 700
   Leu Met Val Leu Cys Cys Val Leu Arg Tyr Met Val Gln Trp Pro Gly
   705 710 715 720
   Ala Arg Ile Leu Arg Arg Gln Glu Leu Asp Ala Phe Leu Ala Gln Ala
   725 730 735
   Leu Ser Pro Lys Leu Tyr Glu Pro Asp Gln Leu Gln Glu Leu Lys Ile
   740 745 750
   Glu Asn Leu Asp Pro Arg Gly Ile Gln Leu Ser Ala Leu Phe Met Ser
   755 760 765
   Gly Val Asp Met Ala Leu Phe Ala Asn Asp Ala Cys Gly Gln Pro Ile
   770 775 780
   Pro Trp Glu His Cys Cys Pro Trp Met Tyr Phe Asp Gly Lys Leu Phe
   785 790 795 800
   Gln Ser Lys Leu Leu Lys Ala Ser Arg Glu Lys Thr Pro Leu Ile Asp
   805 810 815
   Leu Cys Asp Gly Gln Ala Asp Gln Ala Ala Lys Val Glu Lys Met Arg
   820 825 830
   Gln Ser Val Leu Glu Gly Leu Ser Phe Ser Arg Gln Ser His Thr Leu
   835 840 845
   Pro Phe Pro Pro Pro Pro Pro Ala Leu Pro Phe Tyr Pro Ala Ser Ala
   850 855 860
   Tyr Pro Arg His Phe Gly Pro Val Pro Pro Ser Gln Gly Arg Gly Arg
   865 870 875 880
   Gly Phe Ala Gly Val Cys Gly Phe Gly Gly Pro Tyr Gly Glu Thr Val
   885 890 895
   Ala Thr Gly Pro Tyr Arg Ala Phe Arg Val Ala Ala Ala Ser Gly His
   900 905 910
   Cys Gly Ala Phe Ser Gly Ser Asp Ser Ser Arg Thr Ser Lys Ser Gln
   915 920 925
   Gly Gly Val Gln Pro Ile Pro Ser Gln Gly Gly Lys Leu Glu Ile Ala
   930 935 940
   Gly Thr Val Val Gly His Trp Ala Gly Ser Arg Arg Gly Arg Gly Gly
   945 950 955 960
   Arg Gly Pro Phe Pro Leu Gln Val Val Ser Val Gly Gly Pro Ala Arg
   965 970 975
   Gly Arg Pro Arg Gly Val Ile Ser Thr Pro Val Ile Arg Thr Phe Gly
   980 985 990
   Arg Gly Gly Arg Tyr Tyr Gly Arg Gly Tyr Lys Asn Gln Ala Ala Ile
   995 1000 1005
   Gln Gly Arg Pro Pro Tyr Ala Ala Ser Ala Glu Glu Val Ala Lys Glu
   1010 1015 1020
   Leu Lys Ser Lys Ser Gly Glu Ser Lys Ser Ser Ala Met Ser Ser Asp
   1025 1030 1035 1040
   Gly Ser Leu Ala Glu Asn Gly Val Ile Gly Arg Gly Glu Ala Gly Ser
   1045 1050 1055
   Pro Asp Glu Arg Glu His Gly
   1060
<210> 44
   <211> 10427
   <212> DNA
   <213> Rattus sp.
<220>
   <221> CDS
   <222> (211)..(9996)
<220>
   <221> misc_feature
   <222> (115)..(115)
   <223> "Xaa" can be any amino acid
<400> 44
   cgacaaaggc agcctgtgcc gcacagttga ggggtgccgt gaggagctgc agaatcaggc 60
   caatttctcc ttcgctcctc tcgtgttaga catgctcaat ttcctcatgg atgccattca 120
   gaccaacttt cacaggcttc cgccgtgggg agcagcagcc gggcacagca ggccctcagt 180
   gaactgcaca cagtggacaa ggttgtggag atg aca gac cag ctg atg gtt ccc  234
                                    Met Thr Asp Gln Leu Met Val Pro
                                      1               5
   acc tta ggc tca cag gaa ggt gcc ttt gag aat gtt cgg atg aat tac  282
   Thr Leu Gly Ser Gln Glu Gly Ala Phe Glu Asn Val Arg Met Asn Tyr
        10                  15                  20
   agc gga gac cag ggc cag act ata cgg cag ctg atc agt gcc cac gtg  330
   Ser Gly Asp Gln Gly Gln Thr Ile Arg Gln Leu Ile Ser Ala His Val
    25                  30                  35                  40
   ctc agg cgg gtg gct atg tgt gtg ctt tct tcc ccc cat ggg cgc cgc  378
   Leu Arg Arg Val Ala Met Cys Val Leu Ser Ser Pro His Gly Arg Arg
                    45                  50                  55
   caa cat ttg gct gtc agc cac gag aag ggc aag atc acg gtg ctg cag  426
   Gln His Leu Ala Val Ser His Glu Lys Gly Lys Ile Thr Val Leu Gln
   60 65 70
   ctc tcg gcg ctc ctg aag caa gca gat tcc agt aag agg aag ttg act 474
   Leu Ser Ala Leu Leu Lys Gln Ala Asp Ser Ser Lys Arg Lys Leu Thr
   75 80 85
   cta acc cgc ttg gct tct gct cca gtt ccc ttc acc gtg ctg agt ctt 522
   Leu Thr Arg Leu Ala Ser Ala Pro Val Pro Phe Thr Val Leu Ser Leu
   90 95 100
   act gga aac ccc tgc aag gag gac tac ctg sca gtg tgt ggg ctg aag 570
   Thr Gly Asn Pro Cys Lys Glu Asp Tyr Leu Xaa Val Cys Gly Leu Lys
   105 110 115 120
   gac tgc cat gtg ctc acc ttc agt agc tcg ggc tct gtc tcc gat cac 618
   Asp Cys His Val Leu Thr Phe Ser Ser Ser Gly Ser Val Ser Asp His
   125 130 135
   tta gtg tta cat ccc cag ttg gca aca ggg aac ttc atc atc aaa gct 666
   Leu Val Leu His Pro Gln Leu Ala Thr Gly Asn Phe Ile Ile Lys Ala
   140 145 150
   gtg tgg ctc cct ggc tct cag acc gag cta gcc att gtt act gca gac 714
   Val Trp Leu Pro Gly Ser Gln Thr Glu Leu Ala Ile Val Thr Ala Asp
   155 160 165
   ttc gtc aag att tac gac ctg tct gtt gat gcc ttg agc ccc acc ttc 762
   Phe Val Lys Ile Tyr Asp Leu Ser Val Asp Ala Leu Ser Pro Thr Phe
   170 175 180
   tac ttc ctc ctg cca agc tca aag ata agg gat gtc acc ttc ctt ttc 810
   Tyr Phe Leu Leu Pro Ser Ser Lys Ile Arg Asp Val Thr Phe Leu Phe
   185 190 195 200
   aac gag gaa ggc aag aac atc att gtc atc atg tcc tct gct ggg tac 858
   Asn Glu Glu Gly Lys Asn Ile Ile Val Ile Met Ser Ser Ala Gly Tyr
   205 210 215
   atg tac aca cag ctc atg gag gag gcc agc agt gcc cag cag ggg ccc 906
   Met Tyr Thr Gln Leu Met Glu Glu Ala Ser Ser Ala Gln Gln Gly Pro
   220 225 230
   ttc tat gtc act aat gtg ctg gaa ata aat cac gag gat ctg aag gac 954
   Phe Tyr Val Thr Asn Val Leu Glu Ile Asn His Glu Asp Leu Lys Asp
   235 240 245
   agt aac agc cag gtg gca ggt ggt ggc gtg tct gtg tac tat tca cac 1002
   Ser Asn Ser Gln Val Ala Gly Gly Gly Val Ser Val Tyr Tyr Ser His
   250 255 260
   gtg ttg cag atg ctt ttc ttc agc tac agt cag ggc aag tcc ttt gca 1050
   Val Leu Gln Met Leu Phe Phe Ser Tyr Ser Gln Gly Lys Ser Phe Ala
   265 270 275 280
   gcc acc gtc agc agg agc act ctg gag gtg ctg cag ctc ttc ccc atc 1098
   Ala Thr Val Ser Arg Ser Thr Leu Glu Val Leu Gln Leu Phe Pro Ile
   285 290 295
   aac atc aaa agc tcc aat ggt ggc agt aag act tct ccc gcc ctt tgc 1146
   Asn Ile Lys Ser Ser Asn Gly Gly Ser Lys Thr Ser Pro Ala Leu Cys
                   300                     305                      310
   cag tgg tct gag gtg atg aac cac cct ggc ttg gtg tgt tgt gtt cag     1194
   Gln Trp Ser Glu Val Met Asn His Pro Gly Leu Val Cys Cys Val Gln
           315                 320                 325
   caa act act ggt gtg cct ctg gta gtc atg gtg aaa cca gac act ttc     1242
   Gln Thr Thr Gly Val Pro Leu Val Val Met Val Lys Pro Asp Thr Phe
       330                 335                 340
   ctc atc cag gag att aag act ctt ccc gcc aaa gca aag atc cag gac     1290
   Leu Ile Gln Glu Ile Lys Thr Leu Pro Ala Lys Ala Lys Ile Gln Asp
   345                 350                 355                 360
   atg gtt gct att aga cac act gcc tgt aat gag cag cag cgc acc acc     1338
   Met Val Ala Ile Arg His Thr Ala Cys Asn Glu Gln Gln Arg Thr Thr
                   365                 370                 375
   atg atc ctg ctg tgt gag gat ggc agc ctg cga att tat atg gcc aac     1386
   Met Ile Leu Leu Cys Glu Asp Gly Ser Leu Arg Ile Tyr Met Ala Asn
               380                 385                 390
   gtg gag aac acc tct tac tgg ctc cag ccg tct ctg cag ccc agc agc     1434
   Val Glu Asn Thr Ser Tyr Trp Leu Gln Pro Ser Leu Gln Pro Ser Ser
           395                 400                 405
   gtc atc agc atc atg aag cct gtg cga aag cgc aaa aca gct aca atc     1482
   Val Ile Ser Ile Met Lys Pro Val Arg Lys Arg Lys Thr Ala Thr Ile
       410                 415                 420
   aca gcc cgc aca tcc agc cag gtg acc ttc ccc att gac ttc ttt gaa     1530
   Thr Ala Arg Thr Ser Ser Gln Val Thr Phe Pro Ile Asp Phe Phe Glu
   425                 430                 435                 440
   cac aac cag cag cta acg gat gtg gag ttt ggt ggt aat gac ctc ctg     1578
   His Asn Gln Gln Leu Thr Asp Val Glu Phe Gly Gly Asn Asp Leu Leu
                   445                 450                 455
   caa gtc tac aat gcg caa cag ata aag cac agg ctc aac tcc act ggc     1626
   Gln Val Tyr Asn Ala Gln Gln Ile Lys His Arg Leu Asn Ser Thr Gly
               460                 465                 470
   atg tat gtg gcg aac acc aag cct gga ggc ttc acc atg gag atc agt     1674
   Met Tyr Val Ala Asn Thr Lys Pro Gly Gly Phe Thr Met Glu Ile Ser
           475                 480                 485
   aac aac agt agc acc atg gtg atg acg ggc atg cgg atc cag att ggc     1722
   Asn Asn Ser Ser Thr Met Val Met Thr Gly Met Arg Ile Gln Ile Gly
       490                 495                 500
   aca cag gca atc gag cga gca ccg tcc tac atc gag atc ttt ggc aga     1770
   Thr Gln Ala Ile Glu Arg Ala Pro Ser Tyr Ile Glu Ile Phe Gly Arg
   505                 510                 515                 520
   acc atg cag ctt aac ctg agc cgc tcc cgc tgg ttt gac ttc ccc ttc     1818
   Thr Met Gln Leu Asn Leu Ser Arg Ser Arg Trp Phe Asp Phe Pro Phe
                   525                 530                 535
   acc aga gag gaa gcc ctg cag gct gac cgg aag ctg aac ctc ttc atc     1866
   Thr Arg Glu Glu Ala Leu Gln Ala Asp Arg Lys Leu Asn Leu Phe Ile
               540                 545                 550
   ggt gcc tct gtg gat cca gct ggc gtc acc atg ata gat gct gta aaa    1914
   Gly Ala Ser Val Asp Pro Ala Gly Val Thr Met Ile Asp Ala Val Lys
           555                 560                 565
   att tat ggc aag act aaa gag cag ttt ggc tgg cct gat gag ccc cca    1962
   Ile Tyr Gly Lys Thr Lys Glu Gln Phe Gly Trp Pro Asp Glu Pro Pro
       570                 575                 580
   gaa gac ttc cct tct gcc tct gtt agc agc gtc tgc cct cct aac ctg    2010
   Glu Asp Phe Pro Ser Ala Ser Val Ser Ser Val Cys Pro Pro Asn Leu
   585                 590                 595                 600
   aac cag agc aac ggc act gga gac agt gac tca gct gct cca gct aca    2058
   Asn Gln Ser Asn Gly Thr Gly Asp Ser Asp Ser Ala Ala Pro Ala Thr
                   605                 610                 615
   acc agt ggc act gtc ctg gag agg ctg gtt gtg agt tct ttg gaa gcc    2106
   Thr Ser Gly Thr Val Leu Glu Arg Leu Val Val Ser Ser Leu Glu Ala
               620                 625                 630
   ctg gaa agc tgc ttt gct gtc ggc cca atc att gag aag gag aga aac    2154
   Leu Glu Ser Cys Phe Ala Val Gly Pro Ile Ile Glu Lys Glu Arg Asn
           635                 640                 645
   aag cac gca gcc cag gag ctg gcc act ttg ttg ctc tcc ctg cca gcg    2202
   Lys His Ala Ala Gln Glu Leu Ala Thr Leu Leu Leu Ser Leu Pro Ala
       650                 655                 660
   cct gcc agc gtc caa cag caa tca aag agc ctc ctg gcc agc ctg cac    2250
   Pro Ala Ser Val Gln Gln Gln Ser Lys Ser Leu Leu Ala Ser Leu His
   665                 670                 675                 680
   agc agt cgc tcg gcc tac cac agc cac aag gac cag gcc ttg ttg agc    2298
   Ser Ser Arg Ser Ala Tyr His Ser His Lys Asp Gln Ala Leu Leu Ser
                   685                 690                 695
   aaa gct gtg cag tgt ctc aac act tcc agc aaa gaa ggc aag gac ttg    2346
   Lys Ala Val Gln Cys Leu Asn Thr Ser Ser Lys Glu Gly Lys Asp Leu
               700                 705                 710
   gac ccc gag gtg ttc cag cgt cta gta atc aca gct cgc tct att gcc    2394
   Asp Pro Glu Val Phe Gln Arg Leu Val Ile Thr Ala Arg Ser Ile Ala
           715                 720                 725
   gtc aca cgt ccc aac aac ctt gtg cac ttt acg gag tcc aag ctg ccc    2442
   Val Thr Arg Pro Asn Asn Leu Val His Phe Thr Glu Ser Lys Leu Pro
       730                 735                 740
   cag atg gaa aca gaa gga gcg gag gag ggg aaa gag ccg cag aag cag    2490
   Gln Met Glu Thr Glu Gly Ala Glu Glu Gly Lys Glu Pro Gln Lys Gln
   745                 750                 755                 760
   gtg gaa gga gac ggc tgt agt ttc atc act cag ctt gta aac cac ttc    2538
   Val Glu Gly Asp Gly Cys Ser Phe Ile Thr Gln Leu Val Asn His Phe
                   765                 770                 775
   tgg aaa ctc cat gca tct aag ccc aag aat gcc ttc ctg gca cct gcc    2586
   Trp Lys Leu His Ala Ser Lys Pro Lys Asn Ala Phe Leu Ala Pro Ala
               780                 785                 790
   tgc ctg cca ggc ctt act cat att gaa gct acg gtt aat gcg ctg gta    2634
   Cys Leu Pro Gly Leu Thr His Ile Glu Ala Thr Val Asn Ala Leu Val
           795                 800                 805
   gac att atc cat ggc tat tgt acc tgc gag ctg gac tgt atc aac aca    2682
   Asp Ile Ile His Gly Tyr Cys Thr Cys Glu Leu Asp Cys Ile Asn Thr
       810                 815                 820
   gca tcc aag atc tac atg cag atg ctg ctg tgt cct gac cct gct gtg    2730
   Ala Ser Lys Ile Tyr Met Gln Met Leu Leu Cys Pro Asp Pro Ala Val
   825                 830                 835                 840
   agc ttc tcc tgt aaa caa gct cta att cga gtc cta agg ccc agg aac    2778
   Ser Phe Ser Cys Lys Gln Ala Leu Ile Arg Val Leu Arg Pro Arg Asn
                   845                 850                 855
   aag cgg aga cac gtg aca ttg ccc tcc tcc ccc cga agc aac act cca    2826
   Lys Arg Arg His Val Thr Leu Pro Ser Ser Pro Arg Ser Asn Thr Pro
               860                 865                 870
   atg gga gac aag gat gac gat gat gat gat gat gcg gat gag aaa atg    2874
   Met Gly Asp Lys Asp Asp Asp Asp Asp Asp Asp Ala Asp Glu Lys Met
           875                 880                 885
   cag tca tcg ggc atc cct gat ggt ggt cac atc cgt cag gaa agc cag    2922
   Gln Ser Ser Gly Ile Pro Asp Gly Gly His Ile Arg Gln Glu Ser Gln
       890                 895                 900
   gaa cag agt gag gtg gac cat gga gat ttt gag atg gtg tct gag tcg    2970
   Glu Gln Ser Glu Val Asp His Gly Asp Phe Glu Met Val Ser Glu Ser
   905                 910                 915                 920
   atg gtc ctg gaa aca gct gaa aat gtc aac aat ggc aac ccc tct ccc    3018
   Met Val Leu Glu Thr Ala Glu Asn Val Asn Asn Gly Asn Pro Ser Pro
                   925                 930                 935
   cgg aaa gcc ctg ctg gcc ggt gct gag ggc ttt cct ccc atg ctg gac    3066
   Arg Lys Ala Leu Leu Ala Gly Ala Glu Gly Phe Pro Pro Met Leu Asp
               940                 945                 950
   atc cca cct gat gca gac gac gag acc atg gtt gaa cta gcc att gcc    3114
   Ile Pro Pro Asp Ala Asp Asp Glu Thr Met Val Glu Leu Ala Ile Ala
           955                 960                 965
   ctc agc ctg cag cag gac cag caa ggc agc agc agc agt gcc ctg ggc    3162
   Leu Ser Leu Gln Gln Asp Gln Gln Gly Ser Ser Ser Ser Ala Leu Gly
       970                 975                 980
   ctt cag agc ctg gga ctg tcg ggc cag gca ccc agc tct tcc tct ctg    3210
   Leu Gln Ser Leu Gly Leu Ser Gly Gln Ala Pro Ser Ser Ser Ser Leu
   985                 990                 995                 1000
   gac gca gga acc ctc tct gac acc aca gca tca gct cca gcc tca gat    3258
   Asp Ala Gly Thr Leu Ser Asp Thr Thr Ala Ser Ala Pro Ala Ser Asp
                   1005                1010                1015
   gac gag ggc agc act gca gca act gat ggc tcc acc ctg cgg acc tcg    3306
   Asp Glu Gly Ser Thr Ala Ala Thr Asp Gly Ser Thr Leu Arg Thr Ser
               1020                1025                1030
   cca gcc gac cat ggc ggt agt gtg ggc tca gag agc ggg gga agt gca    3354
   Pro Ala Asp His Gly Gly Ser Val Gly Ser Glu Ser Gly Gly Ser Ala
           1035                1040                1045
   gtg gac tcg gtg gct ggc gag cac agt gtg tct ggc cgg agc agt gca    3402
   Val Asp Ser Val Ala Gly Glu His Ser Val Ser Gly Arg Ser Ser Ala
       1050                1055                1060
   tat ggt gac gcc aca gct gag ggg cac ccg gct gga cca ggg agc gtc    3450
   Tyr Gly Asp Ala Thr Ala Glu Gly His Pro Ala Gly Pro Gly Ser Val
   1065                1070                1075                1080
   agc tct agc aca ggc gcc atc agc acc act act ggg cac cag gag gga    3498
   Ser Ser Ser Thr Gly Ala Ile Ser Thr Thr Thr Gly His Gln Glu Gly
                   1085                1090                1095
   gat gga tct gag gga gaa gga gaa ggg gaa gct gaa gga gat gtg cac    3546
   Asp Gly Ser Glu Gly Glu Gly Glu Gly Glu Ala Glu Gly Asp Val His
               1100                1105                1110
   act agc aac agg cta cac atg gtt cgt cta atg ctg ttg gaa aga tta    3594
   Thr Ser Asn Arg Leu His Met Val Arg Leu Met Leu Leu Glu Arg Leu
           1115                1120                1125
   ctg cag aca ctg ccc cag tta cgg aat gtc gga ggt gtc cgg gcc atc    3642
   Leu Gln Thr Leu Pro Gln Leu Arg Asn Val Gly Gly Val Arg Ala Ile
       1130                1135                1140
   cca tac atg cag gtc att ctc atg ctc act aca gat ctg gat gga gaa    3690
   Pro Tyr Met Gln Val Ile Leu Met Leu Thr Thr Asp Leu Asp Gly Glu
   1145                1150                1155                1160
   gat gag aaa gac aag gga gcc ctg gac aac ctg ctt gca cag ctc att    3738
   Asp Glu Lys Asp Lys Gly Ala Leu Asp Asn Leu Leu Ala Gln Leu Ile
                   1165                1170                1175
   gct gag ctg gga atg gac aaa aag gat gtc tcc aag aag aat gaa cgc    3786
   Ala Glu Leu Gly Met Asp Lys Lys Asp Val Ser Lys Lys Asn Glu Arg
               1180                1185                1190
   agt gca ttg aac gaa gtc cat ttg gtc gtg atg aga ctc ctg agt gtc    3834
   Ser Ala Leu Asn Glu Val His Leu Val Val Met Arg Leu Leu Ser Val
           1195                1200                1205
   ttc atg tcc cgc acc aag tct gga tcc aaa tct tcc atc tgt gag tcg    3882
   Phe Met Ser Arg Thr Lys Ser Gly Ser Lys Ser Ser Ile Cys Glu Ser
       1210                1215                1220
   tct tcc ctc atc tcc agt gcc acg gca gca gcc ctg ctg agc tcc ggg    3930
   Ser Ser Leu Ile Ser Ser Ala Thr Ala Ala Ala Leu Leu Ser Ser Gly
   1225                1230                1235                1240
   gcc gtg gac tat tgc ctc cat gtg ctc aag tct ctg ctg gaa tac tgg    3978
   Ala Val Asp Tyr Cys Leu His Val Leu Lys Ser Leu Leu Glu Tyr Trp
                   1245                1250                1255
   aag ggc cag cag agt gag gag gag cct gtg acc acc agc caa ttg ctg    4026
   Lys Gly Gln Gln Ser Glu Glu Glu Pro Val Thr Thr Ser Gln Leu Leu
               1260                1265                1270
   aaa cca cac acg acc tca tct cca cca gat atg agc ccg ttc ttt ctc    4074
   Lys Pro His Thr Thr Ser Ser Pro Pro Asp Met Ser Pro Phe Phe Leu
               1275                1280                1285
   cga caa tat gtg aag ggt cat gct gct gat gtg ttc gaa gcc tat acc    4122
   Arg Gln Tyr Val Lys Gly His Ala Ala Asp Val Phe Glu Ala Tyr Thr
      1290                1295                1300
   cag ctg ctc aca gaa atg gta ctg agg ctt ccc tac cag atc aag aag    4170
   Gln Leu Leu Thr Glu Met Val Leu Arg Leu Pro Tyr Gln Ile Lys Lys
   1305                1310                1315                1320
   atc gca gat acc agc tct cga atc cct cct cct gtc ttt gat cac tcc    4218
   Ile Ala Asp Thr Ser Ser Arg Ile Pro Pro Pro Val Phe Asp His Ser
                   1325                1330                1335
   tgg ttc tac ttt ctc tct gag tac cta atg atc cag cag acc cct ttt    4266
   Trp Phe Tyr Phe Leu Ser Glu Tyr Leu Met Ile Gln Gln Thr Pro Phe
               1340                1345                1350
   gtc cgc cgc caa gtc cgg aaa ctt ctg ctc ttc atc tgt gga tca aag    4314
   Val Arg Arg Gln Val Arg Lys Leu Leu Leu Phe Ile Cys Gly Ser Lys
           1355                1360                1365
   gag aaa tac cgt cag ctc cga gac ctg cac acc ctg gac tcc cat gtg    4362
   Glu Lys Tyr Arg Gln Leu Arg Asp Leu His Thr Leu Asp Ser His Val
       1370                1375                1380
   cgt ggg atc aag aag ctg ctg gag gaa caa ggc atc ttc ctc agg gcc    4410
   Arg Gly Ile Lys Lys Leu Leu Glu Glu Gln Gly Ile Phe Leu Arg Ala
   1385                1390                1395                1400
   agc gtg gtt aca gcc agt tcc ggc tcc gct ttg cag tat gac acg ctc    4458
   Ser Val Val Thr Ala Ser Ser Gly Ser Ala Leu Gln Tyr Asp Thr Leu
                   1405                1410                1415
   atc agc ctg atg gag cac ctg aag gcc tgt gca gag att gcc gcc cag    4506
   Ile Ser Leu Met Glu His Leu Lys Ala Cys Ala Glu Ile Ala Ala Gln
               1420                1425                1430
   cgc acc atc aac tgg cag aag ttc tgc atc aaa gat gac tct gtc ctg    4554
   Arg Thr Ile Asn Trp Gln Lys Phe Cys Ile Lys Asp Asp Ser Val Leu
           1435                1440                1445
   tac ttc ctc ctg caa gtg agt ttc ctg gta gat gag ggg gtg tcc cct    4602
   Tyr Phe Leu Leu Gln Val Ser Phe Leu Val Asp Glu Gly Val Ser Pro
       1450                1455                1460
   gtg cta ctg cag ctg ctt tcc tgt gct ctt tgc ggc agc aaa gtg ctc    4650
   Val Leu Leu Gln Leu Leu Ser Cys Ala Leu Cys Gly Ser Lys Val Leu
   1465                1470                1475                1480
   gca gcc ctg gcg gcc tcc acg ggc tcc tcc agt gtg gct tcc tca gcc    4698
   Ala Ala Leu Ala Ala Ser Thr Gly Ser Ser Ser Val Ala Ser Ser Ala
                   1485                1490                1495
   cct cct gca gcc agt tct gga caa acc aca act cag tcc aaa tcc tcc    4746
   Pro Pro Ala Ala Ser Ser Gly Gln Thr Thr Thr Gln Ser Lys Ser Ser
               1500                1505                1510
   act aaa aag agc aag aaa gaa gaa aag gag aaa gag aaa gag ggt gag    4794
   Thr Lys Lys Ser Lys Lys Glu Glu Lys Glu Lys Glu Lys Glu Gly Glu
           1515                1520                1525
   agc tcg ggt agc cag gaa gac cag ctg tgt aca gct ctc gtg aac cag    4842
   Ser Ser Gly Ser Gln Glu Asp Gln Leu Cys Thr Ala Leu Val Asn Gln
       1530                1535                1540
   ctg aac aga ttt gca gac aag gag act ctg atc cag ttt ctg cgc tgc    4890
   Leu Asn Arg Phe Ala Asp Lys Glu Thr Leu Ile Gln Phe Leu Arg Cys
   1545                1550                1555                1560
   ttc cta ttg gag tcc aac tct tcg tca gta cgc tgg cag gcc cac tgc    4938
   Phe Leu Leu Glu Ser Asn Ser Ser Ser Val Arg Trp Gln Ala His Cys
                   1565                1570                1575
   ctg aca ctg cac atc tac aga aac tcc aac aag gct cag cag gag ctc    4986
   Leu Thr Leu His Ile Tyr Arg Asn Ser Asn Lys Ala Gln Gln Glu Leu
               1580                1585                1590
   ttg cta gat ctc atg tgg tca atc tgg cca gaa ctc cca gct tat ggt    5034
   Leu Leu Asp Leu Met Trp Ser Ile Trp Pro Glu Leu Pro Ala Tyr Gly
           1595                1600                1605
   cgg aag gct gcc cag ttt gtg gat ctc ctg ggc tat ttc tcc ctg aaa    5082
   Arg Lys Ala Ala Gln Phe Val Asp Leu Leu Gly Tyr Phe Ser Leu Lys
       1610                1615                1620
   act gca cag aca gag aag aag ttg aag gag tac tca cag aag gcc gtg    5130
   Thr Ala Gln Thr Glu Lys Lys Leu Lys Glu Tyr Ser Gln Lys Ala Val
   1625                1630                1635                1640
   gag att ctg agg act cag aac cac att ctc acc aat cat ccc aac tcc    5178
   Glu Ile Leu Arg Thr Gln Asn His Ile Leu Thr Asn His Pro Asn Ser
                   1645                1650                1655
   aac att tac aat acc ttg tct ggc tta gtg gag ttt gat ggc tac tac    5226
   Asn Ile Tyr Asn Thr Leu Ser Gly Leu Val Glu Phe Asp Gly Tyr Tyr
               1660                1665                1670
   cta gag agt gat ccc tgc ctg gtg tgt aat aac cct gaa gtg ccg ttt    5274
   Leu Glu Ser Asp Pro Cys Leu Val Cys Asn Asn Pro Glu Val Pro Phe
           1675                1680                1685
   tgt tat atc aag ctg tct tcc att aaa gtg gac aca cgg tac acc acc    5322
   Cys Tyr Ile Lys Leu Ser Ser Ile Lys Val Asp Thr Arg Tyr Thr Thr
       1690                1695                1700
   acc cag cag gtg gta aag ctc att ggt agt cac acc atc agc aag gtg    5370
   Thr Gln Gln Val Val Lys Leu Ile Gly Ser His Thr Ile Ser Lys Val
   1705                1710                1715                1720
   aca gtg aaa att ggg gac ctg aag cgg acc aag atg gtg cgg aca atc    5418
   Thr Val Lys Ile Gly Asp Leu Lys Arg Thr Lys Met Val Arg Thr Ile
                   1725                1730                1735
   aac ctg tat tac aac aac cga act gtg cag gcc att gtg gag ttg aaa    5466
   Asn Leu Tyr Tyr Asn Asn Arg Thr Val Gln Ala Ile Val Glu Leu Lys
               1740                1745                1750
   aat aag cca gct cgc tgg cac aaa gcc aag aaa gtt cag ctg acg cct    5514
   Asn Lys Pro Ala Arg Trp His Lys Ala Lys Lys Val Gln Leu Thr Pro
           1755                1760                1765
   gga cag aca gaa gtc aag att gac ctg cct ctg ccc att gtg gcc tct     5562
   Gly Gln Thr Glu Val Lys Ile Asp Leu Pro Leu Pro Ile Val Ala Ser
      1770                1775                1780
   aac ctg atg atc gag ttt gca gac ttc tat gag aac tac cag gct tcc     5610
   Asn Leu Met Ile Glu Phe Ala Asp Phe Tyr Glu Asn Tyr Gln Ala Ser
   1785                1790                1795                1800
   aca gag acc ctt cag tgt cct cgc tgc agt gcc tct gtc ccc gcc aat     5658
   Thr Glu Thr Leu Gln Cys Pro Arg Cys Ser Ala Ser Val Pro Ala Asn
                   1805                1810                1815
   ccc ggc gtc tgt ggc aac tgt ggc gag aat gta tac cag tgt cac aag     5706
   Pro Gly Val Cys Gly Asn Cys Gly Glu Asn Val Tyr Gln Cys His Lys
               1820                1825                1830
   tgc agg tcc atc aac tat gat gag aag gat ccc ttc ctc tgc aat gct     5754
   Cys Arg Ser Ile Asn Tyr Asp Glu Lys Asp Pro Phe Leu Cys Asn Ala
           1835                1840                1845
   tgt ggc ttt tgt aaa tat gcc cgc ttt gac ttc atg ctt tat gcc aaa     5802
   Cys Gly Phe Cys Lys Tyr Ala Arg Phe Asp Phe Met Leu Tyr Ala Lys
       1850                1855                1860
   cct tgc tgc cca gtg gat ccc atc gag aac gag gaa gat cgg aag aag     5850
   Pro Cys Cys Pro Val Asp Pro Ile Glu Asn Glu Glu Asp Arg Lys Lys
   1865                1870                1875                1880
   gcc gtt tcc aac atc aac aca ctt ctg gac aaa gct gac cgc gtg tac     5898
   Ala Val Ser Asn Ile Asn Thr Leu Leu Asp Lys Ala Asp Arg Val Tyr
                   1885                1890                1895
   cat cag ctc atg gga cac cgg ccc cag ctg gag aac ctg ctc tgc aaa     5946
   His Gln Leu Met Gly His Arg Pro Gln Leu Glu Asn Leu Leu Cys Lys
               1900                1905                1910
   gtg aat gaa gca gct cca gaa aag cca cag gaa gac tcg gga aca gcg     5994
   Val Asn Glu Ala Ala Pro Glu Lys Pro Gln Glu Asp Ser Gly Thr Ala
           1915                1920                1925
   gga ggc atc agc tcc act tca gcc agt gtg aat cgc tac atc cta cag     6042
   Gly Gly Ile Ser Ser Thr Ser Ala Ser Val Asn Arg Tyr Ile Leu Gln
       1930                1935                1940
   ctg gcg cag gag tat tgt gga gac tgt aag aac tca ttt gac gag ctc     6090
   Leu Ala Gln Glu Tyr Cys Gly Asp Cys Lys Asn Ser Phe Asp Glu Leu
   1945                1950                1955                1960
   tcc aaa atc atc cag aaa gtc ttc gct tcg cgc aaa gag ctg tta gag     6138
   Ser Lys Ile Ile Gln Lys Val Phe Ala Ser Arg Lys Glu Leu Leu Glu
                   1965                1970                1975
   tat gac ctg cag cag aga gaa gcg gcc acc aag tca tcc cgg aca tcc     6186
   Tyr Asp Leu Gln Gln Arg Glu Ala Ala Thr Lys Ser Ser Arg Thr Ser
               1980                1985                1990
   gtg cag ccc acg ttt act gcc agc cag tac cgt gcg ttg tct gtc tta     6234
   Val Gln Pro Thr Phe Thr Ala Ser Gln Tyr Arg Ala Leu Ser Val Leu
           1995                2000                2005
   ggc tgt ggc cac acc tcc tcc acc aag tgc tat ggc tgt gcc tca gct     6282
   Gly Cys Gly His Thr Ser Ser Thr Lys Cys Tyr Gly Cys Ala Ser Ala
       2010                2015                2020
   gtc aca gag cat tgt atc aca ctg cta agg gcc ctg gcc acc aat cca    6330
   Val Thr Glu His Cys Ile Thr Leu Leu Arg Ala Leu Ala Thr Asn Pro
   2025                2030                2035                2040
   gcc ctg aga cac atc ctc gtc tcc cag ggc ctc atc cga gag ctc ttt    6378
   Ala Leu Arg His Ile Leu Val Ser Gln Gly Leu Ile Arg Glu Leu Phe
                   2045                2050                2055
   gat tac aac ctc cgc aga ggc gcg gca gcc atc cgc gag gag gtc cgc    6426
   Asp Tyr Asn Leu Arg Arg Gly Ala Ala Ala Ile Arg Glu Glu Val Arg
               2060                2065                2070
   cag ctc atg tgc ctc ctg act aga gac aac cca gag gcc acc cag caa    6474
   Gln Leu Met Cys Leu Leu Thr Arg Asp Asn Pro Glu Ala Thr Gln Gln
           2075                2080                2085
   atg aat gac ctg atc att ggc aaa gtc tcc act gca ctg aag ggc cac    6522
   Met Asn Asp Leu Ile Ile Gly Lys Val Ser Thr Ala Leu Lys Gly His
       2090                2095                2100
   tgg gct aat cct gac ctg gct agc agc ctt cag tat gag atg ctg ctg    6570
   Trp Ala Asn Pro Asp Leu Ala Ser Ser Leu Gln Tyr Glu Met Leu Leu
   2105                2110                2115                2120
   ctg aca gac tcc atc tcc aag gag gac agc tgc tgg gaa ctc cgc tta    6618
   Leu Thr Asp Ser Ile Ser Lys Glu Asp Ser Cys Trp Glu Leu Arg Leu
                   2125                2130                2135
   cgc tgt gca ctc agc ctt ttc ctc atg gct gtg aac atc aaa acg cca    6666
   Arg Cys Ala Leu Ser Leu Phe Leu Met Ala Val Asn Ile Lys Thr Pro
               2140                2145                2150
   gtg gtt gtt gag aac atc acc ctc atg tgt ctg cgg atc tta cag aag    6714
   Val Val Val Glu Asn Ile Thr Leu Met Cys Leu Arg Ile Leu Gln Lys
           2155                2160                2165
   ctg att aaa cca cct gcc cca acc agc aag aag aac aag gac gtc cct    6762
   Leu Ile Lys Pro Pro Ala Pro Thr Ser Lys Lys Asn Lys Asp Val Pro
       2170                2175                2180
   gtg gag gcc ctc acc acg gtg aag ccg tac tgc aat gag atc cac gcg    6810
   Val Glu Ala Leu Thr Thr Val Lys Pro Tyr Cys Asn Glu Ile His Ala
   2185                2190                2195                2200
   cag gct cag ctg tgg ctc aag cga gat cct aag gca tcc tac gaa gcc    6858
   Gln Ala Gln Leu Trp Leu Lys Arg Asp Pro Lys Ala Ser Tyr Glu Ala
                   2205                2210                2215
   tgg aag aag tgc ctg cct atc cga ggg gta gat ggc aac ggg aaa tcc    6906
   Trp Lys Lys Cys Leu Pro Ile Arg Gly Val Asp Gly Asn Gly Lys Ser
               2220                2225                2230
   ccc agc aag tcc gag ctc cac cgg ctc tac ttg act gag aag tat gtg    6954
   Pro Ser Lys Ser Glu Leu His Arg Leu Tyr Leu Thr Glu Lys Tyr Val
           2235                2240                2245
   tgg agg tgg aaa cag ttc ctg agt cgg cgg ggg aag agg acc acc cca    7002
   Trp Arg Trp Lys Gln Phe Leu Ser Arg Arg Gly Lys Arg Thr Thr Pro
   2250 2255 2260
   ctt gac ctc aag ctg ggc cac aac aac tgg ctg cgg cag gta ctc ttt 7050
   Leu Asp Leu Lys Leu Gly His Asn Asn Trp Leu Arg Gln Val Leu Phe
   2265 2270 2275 2280
   acc ccg gca aca cag gca gca cgg cag gca gcc tgt act att gtg gaa 7098
   Thr Pro Ala Thr Gln Ala Ala Arg Gln Ala Ala Cys Thr Ile Val Glu
   2285 2290 2295
   gct ctt gct act gtc ccc agc cgc aag cag cag gtc ctt gac ctc ctc 7146
   Ala Leu Ala Thr Val Pro Ser Arg Lys Gln Gln Val Leu Asp Leu Leu
   2300 2305 2310
   acc agt tac ctg gac gag ctg agt gtg gct ggg gag tgt gct gca gag 7194
   Thr Ser Tyr Leu Asp Glu Leu Ser Val Ala Gly Glu Cys Ala Ala Glu
   2315 2320 2325
   tac ctg gct ctc tac cag aag ctc atc gcc tcc tgc cac tgg aaa gtc 7242
   Tyr Leu Ala Leu Tyr Gln Lys Leu Ile Ala Ser Cys His Trp Lys Val
   2330 2335 2340
   tac ctg gct gct cgg gga gtc ctg ccc tac gtg ggc aac ctc atc acc 7290
   Tyr Leu Ala Ala Arg Gly Val Leu Pro Tyr Val Gly Asn Leu Ile Thr
   2345 2350 2355 2360
   aag gaa atc gcc cgc ctg ctg gcc ttg gag gag gcc aca ctg agc aca 7338
   Lys Glu Ile Ala Arg Leu Leu Ala Leu Glu Glu Ala Thr Leu Ser Thr
   2365 2370 2375
   gac ctg cag cag gga tat gcc ctc aag agt ctc aca ggc ctc ctc tcc 7386
   Asp Leu Gln Gln Gly Tyr Ala Leu Lys Ser Leu Thr Gly Leu Leu Ser
   2380 2385 2390
   tcc ttt gta gag gtg gag tcc atc aag cgt cat ttc aag agc cgc ttg 7434
   Ser Phe Val Glu Val Glu Ser Ile Lys Arg His Phe Lys Ser Arg Leu
   2395 2400 2405
   gtg ggc act gtg ctg aat ggg tac ctg tgc ttg cgg aaa cta gtg ctg 7482
   Val Gly Thr Val Leu Asn Gly Tyr Leu Cys Leu Arg Lys Leu Val Leu
   2410 2415 2420
   cag agg acc aag ctc atc gat gag acc cag gac atg ctg ctg gag atg 7530
   Gln Arg Thr Lys Leu Ile Asp Glu Thr Gln Asp Met Leu Leu Glu Met
   2425 2430 2435 2440
   ctg gag gac atg acc aca ggt aca gag tct gaa acc aag gcc ttc atg 7578
   Leu Glu Asp Met Thr Thr Gly Thr Glu Ser Glu Thr Lys Ala Phe Met
   2445 2450 2455
   gct gtg tgc att gag aca gcc aag cgc tac aat ctg gat gac tac cgg 7626
   Ala Val Cys Ile Glu Thr Ala Lys Arg Tyr Asn Leu Asp Asp Tyr Arg
   2460 2465 2470
   act cct gtg ttc atc ttt gag agg ctg tgc agc atc atc tac cct gag 7674
   Thr Pro Val Phe Ile Phe Glu Arg Leu Cys Ser Ile Ile Tyr Pro Glu
   2475 2480 2485
   gaa aat gaa gtc act gag ttc ttt gtg acc ctg gag aag gac ccc cag 7722
   Glu Asn Glu Val Thr Glu Phe Phe Val Thr Leu Glu Lys Asp Pro Gln
   2490 2495 2500
   caa gag gac ttt cta cag ggc agg atg ccc gga aac cca tat agc agc     7770
   Gln Glu Asp Phe Leu Gln Gly Arg Met Pro Gly Asn Pro Tyr Ser Ser
   2505                2510                2515                2520
   aat gaa cca ggc att ggg cct ctt atg agg gac ata aag aac aag atc     7818
   Asn Glu Pro Gly Ile Gly Pro Leu Met Arg Asp Ile Lys Asn Lys Ile
                   2525                2530                2535
   tgc cag gac tgt gac ctg gtg gct ctt ctg gag gac gac agt ggg atg     7866
   Cys Gln Asp Cys Asp Leu Val Ala Leu Leu Glu Asp Asp Ser Gly Met
               2540                2545                2550
   gag ctt cta gtg aac aat aaa att atc agt ctg gat ctt cct gtg gct     7914
   Glu Leu Leu Val Asn Asn Lys Ile Ile Ser Leu Asp Leu Pro Val Ala
           2555                2560                2565
   gag gtt tac aag aag gtc tgg tgt acg acc aat gag gga gag ccc atg     7962
   Glu Val Tyr Lys Lys Val Trp Cys Thr Thr Asn Glu Gly Glu Pro Met
       2570                2575                2580
   agg att gtt tat cga atg cgg ggg cta ctg ggc gat gcc acc gag gag     8010
   Arg Ile Val Tyr Arg Met Arg Gly Leu Leu Gly Asp Ala Thr Glu Glu
   2585                2590                2595                2600
   ttt atc gag tcc ctg gac tcc acc aca gat gaa gaa gag gac gaa gag     8058
   Phe Ile Glu Ser Leu Asp Ser Thr Thr Asp Glu Glu Glu Asp Glu Glu
                   2605                2610                2615
   gaa gtg tac aga atg gcc ggt gtg atg gcc cag tgc ggg ggt ctg cag     8106
   Glu Val Tyr Arg Met Ala Gly Val Met Ala Gln Cys Gly Gly Leu Gln
               2620                2625                2630
   tgc atg ctg aac aga ctg gca gga gtc aaa gat ttt aag cag ggg cgc     8154
   Cys Met Leu Asn Arg Leu Ala Gly Val Lys Asp Phe Lys Gln Gly Arg
           2635                2640                2645
   cac ctt cta aca gtg ctc ctg aag ctg ttc agt tac tgt gtg aag gtg     8202
   His Leu Leu Thr Val Leu Leu Lys Leu Phe Ser Tyr Cys Val Lys Val
       2650                2655                2660
   aaa gtc aat cgg caa cag ttg gtc aag ctg gaa atg aac acc ttg aat     8250
   Lys Val Asn Arg Gln Gln Leu Val Lys Leu Glu Met Asn Thr Leu Asn
   2665                2670                2675                2680
   gtc atg ttg ggg act tta aac ttg gct ctg gta gct gag caa gag agc     8298
   Val Met Leu Gly Thr Leu Asn Leu Ala Leu Val Ala Glu Gln Glu Ser
                   2685                2690                2695
   aag gac agc gga gga gca gcg gtg gct gag cag gtg ctg agc atc atg     8346
   Lys Asp Ser Gly Gly Ala Ala Val Ala Glu Gln Val Leu Ser Ile Met
               2700                2705                2710
   gag atc att cta gat gag tcc aat gca gag ccc ctg agt gag gac aag     8394
   Glu Ile Ile Leu Asp Glu Ser Asn Ala Glu Pro Leu Ser Glu Asp Lys
           2715                2720                2725
   ggc aac ctc ctc ctc aca ggt gac aag gat caa ctg gtg atg ctc ttg     8442
   Gly Asn Leu Leu Leu Thr Gly Asp Lys Asp Gln Leu Val Met Leu Leu
       2730                2735                2740
   gac cag atc aac agc acc ttc gtt cgt tcc aat ccc agc gtg ctg cag    8490
   Asp Gln Ile Asn Ser Thr Phe Val Arg Ser Asn Pro Ser Val Leu Gln
   2745                2750                2755                2760
   ggt ttg ctg cgt atc atc cca tac ctg tcc ttc gga gag gtg gag aag    8538
   Gly Leu Leu Arg Ile Ile Pro Tyr Leu Ser Phe Gly Glu Val Glu Lys
                   2765                2770                2775
   atg cag atc ctg gtg gag cgg ttc aag cca tac tgc agc ttc gat aag    8586
   Met Gln Ile Leu Val Glu Arg Phe Lys Pro Tyr Cys Ser Phe Asp Lys
               2780                2785                2790
   tac gat gaa gac cat agt ggg gat gac aaa gtc ttc ctg gat tgc ttc    8634
   Tyr Asp Glu Asp His Ser Gly Asp Asp Lys Val Phe Leu Asp Cys Phe
           2795                2800                2805
   tgc aag att gct gct ggt atc aag aac aac agc aat ggt cat cag ctg    8682
   Cys Lys Ile Ala Ala Gly Ile Lys Asn Asn Ser Asn Gly His Gln Leu
       2810                2815                2820
   aag gac ctc atc ctg cag aag gga atc acc cag agt gcc ctg gac tac    8730
   Lys Asp Leu Ile Leu Gln Lys Gly Ile Thr Gln Ser Ala Leu Asp Tyr
   2825                2830                2835                2840
   atg aaa aag cat atc ccc agt gcc aag aat ttg gat gct gac atc tgg    8778
   Met Lys Lys His Ile Pro Ser Ala Lys Asn Leu Asp Ala Asp Ile Trp
                   2845                2850                2855
   aaa aag ttt ttg tct cgg cct gct ctg cca ttt att ttg agg ctt ctt    8826
   Lys Lys Phe Leu Ser Arg Pro Ala Leu Pro Phe Ile Leu Arg Leu Leu
               2860                2865                2870
   cgg ggt ctg gcc atg caa cac cct gcc acc cag gtt ctg att gga acg    8874
   Arg Gly Leu Ala Met Gln His Pro Ala Thr Gln Val Leu Ile Gly Thr
           2875                2880                2885
   gac tcc atc aca agc ctg cat aag cta gag cag gtg tcc agt gat gag    8922
   Asp Ser Ile Thr Ser Leu His Lys Leu Glu Gln Val Ser Ser Asp Glu
       2890                2895                2900
   ggc att ggg acc ttg gcc gag aac ctc ctg gag gcc cta aga gaa cac    8970
   Gly Ile Gly Thr Leu Ala Glu Asn Leu Leu Glu Ala Leu Arg Glu His
   2905                2910                2915                2920
   cca gat gtg aac aag aag att gac gca gcc cgc aga gag acc cgg gca    9018
   Pro Asp Val Asn Lys Lys Ile Asp Ala Ala Arg Arg Glu Thr Arg Ala
                   2925                2930                2935
   gag aag aag cgc atg gcc atg gcc atg agg cag aag gct ctg gga act    9066
   Glu Lys Lys Arg Met Ala Met Ala Met Arg Gln Lys Ala Leu Gly Thr
               2940                2945                2950
   ctg ggc atg acg aca aac gag aag ggt cag gtg gtg acc aag act gcg    9114
   Leu Gly Met Thr Thr Asn Glu Lys Gly Gln Val Val Thr Lys Thr Ala
           2955                2960                2965
   ctc ctg aag caa atg gaa gag ctg att gag gag ccc gga ctc acg tgc    9162
   Leu Leu Lys Gln Met Glu Glu Leu Ile Glu Glu Pro Gly Leu Thr Cys
       2970                2975                2980
   tgc atc tgc agg gaa ggg ggt acc aag ttc cag ccc acg aag gtc tta    9210
   Cys Ile Cys Arg Glu Gly Gly Thr Lys Phe Gln Pro Thr Lys Val Leu
   2985 2990 2995 3000
   ggc att tac act ttc acc aag cgg gtg gcc ttg gag gag atg gag aac 9258
   Gly Ile Tyr Thr Phe Thr Lys Arg Val Ala Leu Glu Glu Met Glu Asn
   3005 3010 3015
   aag cct cgg aaa cag cag ggc tac agc act gtg tcc cat ttc aac atc 9306
   Lys Pro Arg Lys Gln Gln Gly Tyr Ser Thr Val Ser His Phe Asn Ile
   3020 3025 3030
   gtg cac tat gac tgt cac ctg gct gct gtc agg ttg gct aga ggc cgg 9354
   Val His Tyr Asp Cys His Leu Ala Ala Val Arg Leu Ala Arg Gly Arg
   3035 3040 3045
   gaa gag tgg gaa agt gct gcc ctg cag aac gcc aac acg aag tgc aat 9402
   Glu Glu Trp Glu Ser Ala Ala Leu Gln Asn Ala Asn Thr Lys Cys Asn
   3050 3055 3060
   gga ctc ctt ccg gtc tgg ggc ccc cat gtc cct gag tca gct ttt gcc 9450
   Gly Leu Leu Pro Val Trp Gly Pro His Val Pro Glu Ser Ala Phe Ala
   3065 3070 3075 3080
   act tgt tta gct agg cac aac act tac ctc cag gag tgt acc ggc cag 9498
   Thr Cys Leu Ala Arg His Asn Thr Tyr Leu Gln Glu Cys Thr Gly Gln
   3085 3090 3095
   cgg gag ccc acg tac cag ctc aac atc cac gac atc aaa ctg ctt ttc 9546
   Arg Glu Pro Thr Tyr Gln Leu Asn Ile His Asp Ile Lys Leu Leu Phe
   3100 3105 3110
   ctg cgc ttc gcc atg gag cag tca ttc agt gca gac acc ggt ggg ggc 9594
   Leu Arg Phe Ala Met Glu Gln Ser Phe Ser Ala Asp Thr Gly Gly Gly
   3115 3120 3125
   ggt cgg gag agc aac atc cac ctg atc ccg tac atc att cac act gtg 9642
   Gly Arg Glu Ser Asn Ile His Leu Ile Pro Tyr Ile Ile His Thr Val
   3130 3135 3140
   ctt tac gtc ctg aac acg acc cga gca acg tcc cgg gag gag aag aac 9690
   Leu Tyr Val Leu Asn Thr Thr Arg Ala Thr Ser Arg Glu Glu Lys Asn
   3145 3150 3155 3160
   ctc caa ggc ttc ctg gag cag ccc aga gag aag tgg aca gag agt gcc 9738
   Leu Gln Gly Phe Leu Glu Gln Pro Arg Glu Lys Trp Thr Glu Ser Ala
   3165 3170 3175
   ttt gat gta gat ggg ccc cac tac ttc acc atc tta gcc ctg cat gtc 9786
   Phe Asp Val Asp Gly Pro His Tyr Phe Thr Ile Leu Ala Leu His Val
   3180 3185 3190
   ctc ccc cct gag cag tgg aaa gcc act cga gtg gag atc cta cgc agg 9834
   Leu Pro Pro Glu Gln Trp Lys Ala Thr Arg Val Glu Ile Leu Arg Arg
   3195 3200 3205
   ctg ctg gtg gcc tcg cat gcc cgg gca gtg gct cca gga gga gcc acc 9882
   Leu Leu Val Ala Ser His Ala Arg Ala Val Ala Pro Gly Gly Ala Thr
   3210 3215 3220
   agg ttg aca gat aag gcg gtg aaa gac tat tct gcc tac cgt tct tcc 9930
   Arg Leu Thr Asp Lys Ala Val Lys Asp Tyr Ser Ala Tyr Arg Ser Ser
   3225                3230                3235                3240
   ttg ctg ttc tgg gct ctc gtt gat ctc att tat aac atg ttt aag aag   9978
   Leu Leu Phe Trp Ala Leu Val Asp Leu Ile Tyr Asn Met Phe Lys Lys
                   3245                3250                3255
   gtg ccc act agt aac act tgagggcggg ctggtcctng ctctctggct         10026
   Val Pro Thr Ser Asn Thr
               3260
   gagtacattc cgccacaaat gacatgccca ttctacgaag ctgccgacaa agcccctgaa 10086
   aancttccag gaggagttca tgccagtgga gaccttctca gagttcctcg acgcagcagg 10146
   tcttctatca gaaatcaccg acccagagag cttcctaaag gacctgttga actcagtccc 10206
   ctgaccatcc ccatggaaat gagatcgcgg tgacgagatt gaagctagct tgcattctcc 10266
   cctcattcat ctctccgtgt gcactcattt cccccacgga tgctgcatta gcaccccctc 10326
   ctccctcagt tttcttggag tggcttgggg tttgtaggct tcctgttcta tcacgcgcgc 10386
   gtgtgtgtgt gtgtgtgtgt gtgtgtgtgt gtgtgtgtgt g                    10427
<210> 45
   <211> 3262
   <212> PRT
   <213> Rattus sp.
<400> 45
   Met Thr Asp Gln Leu Met Val Pro Thr Leu Gly Ser Gln Glu Gly Ala
   1 5 10 15
   Phe Glu Asn Val Arg Met Asn Tyr Ser Gly Asp Gln Gly Gln Thr Ile
   20 25 30
   Arg Gln Leu Ile Ser Ala His Val Leu Arg Arg Val Ala Met Cys Val
   35 40 45
   Leu Ser Ser Pro His Gly Arg Arg Gln His Leu Ala Val Ser His Glu
   50 55 60
   Lys Gly Lys Ile Thr Val Leu Gln Leu Ser Ala Leu Leu Lys Gln Ala
   65 70 75 80
   Asp Ser Ser Lys Arg Lys Leu Thr Leu Thr Arg Leu Ala Ser Ala Pro
   85 90 95
   Val Pro Phe Thr Val Leu Ser Leu Thr Gly Asn Pro Cys Lys Glu Asp
   100 105 110
   Tyr Leu Xaa Val Cys Gly Leu Lys Asp Cys His Val Leu Thr Phe Ser
   115 120 125
   Ser Ser Gly Ser Val Ser Asp His Leu Val Leu His Pro Gln Leu Ala
   130 135 140
   Thr Gly Asn Phe Ile Ile Lys Ala Val Trp Leu Pro Gly Ser Gln Thr
   145 150 155 160
   Glu Leu Ala Ile Val Thr Ala Asp Phe Val Lys Ile Tyr Asp Leu Ser
   165 170 175
   Val Asp Ala Leu Ser Pro Thr Phe Tyr Phe Leu Leu Pro Ser Ser Lys
   180 185 190
   Ile Arg Asp Val Thr Phe Leu Phe Asn Glu Glu Gly Lys Asn Ile Ile
   195 200 205
   Val Ile Met Ser Ser Ala Gly Tyr Met Tyr Thr Gln Leu Met Glu Glu
   210 215 220
   Ala Ser Ser Ala Gln Gln Gly Pro Phe Tyr Val Thr Asn Val Leu Glu
   225 230 235 240
   Ile Asn His Glu Asp Leu Lys Asp Ser Asn Ser Gln Val Ala Gly Gly
   245 250 255
   Gly Val Ser Val Tyr Tyr Ser His Val Leu Gln Met Leu Phe Phe Ser
   260 265 270
   Tyr Ser Gln Gly Lys Ser Phe Ala Ala Thr Val Ser Arg Ser Thr Leu
   275 280 285
   Glu Val Leu Gln Leu Phe Pro Ile Asn Ile Lys Ser Ser Asn Gly Gly
   290 295 300
   Ser Lys Thr Ser Pro Ala Leu Cys Gln Trp Ser Glu Val Met Asn His
   305 310 315 320
   Pro Gly Leu Val Cys Cys Val Gln Gln Thr Thr Gly Val Pro Leu Val
   325 330 335
   Val Met Val Lys Pro Asp Thr Phe Leu Ile Gln Glu Ile Lys Thr Leu
   340 345 350
   Pro Ala Lys Ala Lys Ile Gln Asp Met Val Ala Ile Arg His Thr Ala
   355 360 365
   Cys Asn Glu Gln Gln Arg Thr Thr Met Ile Leu Leu Cys Glu Asp Gly
   370 375 380
   Ser Leu Arg Ile Tyr Met Ala Asn Val Glu Asn Thr Ser Tyr Trp Leu
   385 390 395 400
   Gln Pro Ser Leu Gln Pro Ser Ser Val Ile Ser Ile Met Lys Pro Val
   405 410 415
   Arg Lys Arg Lys Thr Ala Thr Ile Thr Ala Arg Thr Ser Ser Gln Val
   420 425 430
   Thr Phe Pro Ile Asp Phe Phe Glu His Asn Gln Gln Leu Thr Asp Val
   435 440 445
   Glu Phe Gly Gly Asn Asp Leu Leu Gln Val Tyr Asn Ala Gln Gln Ile
   450 455 460
   Lys His Arg Leu Asn Ser Thr Gly Met Tyr Val Ala Asn Thr Lys Pro
   465 470 475 480
   Gly Gly Phe Thr Met Glu Ile Ser Asn Asn Ser Ser Thr Met Val Met
   485 490 495
   Thr Gly Met Arg Ile Gln Ile Gly Thr Gln Ala Ile Glu Arg Ala Pro
   500 505 510
   Ser Tyr Ile Glu Ile Phe Gly Arg Thr Met Gln Leu Asn Leu Ser Arg
   515 520 525
   Ser Arg Trp Phe Asp Phe Pro Phe Thr Arg Glu Glu Ala Leu Gln Ala
   530 535 540
   Asp Arg Lys Leu Asn Leu Phe Ile Gly Ala Ser Val Asp Pro Ala Gly
   545 550 555 560
   Val Thr Met Ile Asp Ala Val Lys Ile Tyr Gly Lys Thr Lys Glu Gln
   565 570 575
   Phe Gly Trp Pro Asp Glu Pro Pro Glu Asp Phe Pro Ser Ala Ser Val
   580 585 590
   Ser Ser Val Cys Pro Pro Asn Leu Asn Gln Ser Asn Gly Thr Gly Asp
   595 600 605
   Ser Asp Ser Ala Ala Pro Ala Thr Thr Ser Gly Thr Val Leu Glu Arg
   610 615 620
   Leu Val Val Ser Ser Leu Glu Ala Leu Glu Ser Cys Phe Ala Val Gly
   625 630 635 640
   Pro Ile Ile Glu Lys Glu Arg Asn Lys His Ala Ala Gln Glu Leu Ala
   645 650 655
   Thr Leu Leu Leu Ser Leu Pro Ala Pro Ala Ser Val Gln Gln Gln Ser
   660 665 670
   Lys Ser Leu Leu Ala Ser Leu His Ser Ser Arg Ser Ala Tyr His Ser
   675 680 685
   His Lys Asp Gln Ala Leu Leu Ser Lys Ala Val Gln Cys Leu Asn Thr
   690 695 700
   Ser Ser Lys Glu Gly Lys Asp Leu Asp Pro Glu Val Phe Gln Arg Leu
   705 710 715 720
   Val Ile Thr Ala Arg Ser Ile Ala Val Thr Arg Pro Asn Asn Leu Val
   725 730 735
   His Phe Thr Glu Ser Lys Leu Pro Gln Met Glu Thr Glu Gly Ala Glu
   740 745 750
   Glu Gly Lys Glu Pro Gln Lys Gln Val Glu Gly Asp Gly Cys Ser Phe
   755 760 765
   Ile Thr Gln Leu Val Asn His Phe Trp Lys Leu His Ala Ser Lys Pro
   770 775 780
   Lys Asn Ala Phe Leu Ala Pro Ala Cys Leu Pro Gly Leu Thr His Ile
   785 790 795 800
   Glu Ala Thr Val Asn Ala Leu Val Asp Ile Ile His Gly Tyr Cys Thr
   805 810 815
   Cys Glu Leu Asp Cys Ile Asn Thr Ala Ser Lys Ile Tyr Met Gln Met
   820 825 830
   Leu Leu Cys Pro Asp Pro Ala Val Ser Phe Ser Cys Lys Gln Ala Leu
   835 840 845
   Ile Arg Val Leu Arg Pro Arg Asn Lys Arg Arg His Val Thr Leu Pro
   850 855 860
   Ser Ser Pro Arg Ser Asn Thr Pro Met Gly Asp Lys Asp Asp Asp Asp
   865 870 875 880
   Asp Asp Asp Ala Asp Glu Lys Met Gln Ser Ser Gly Ile Pro Asp Gly
   885 890 895
   Gly His Ile Arg Gln Glu Ser Gln Glu Gln Ser Glu Val Asp His Gly
   900 905 910
   Asp Phe Glu Met Val Ser Glu Ser Met Val Leu Glu Thr Ala Glu Asn
   915 920 925
   Val Asn Asn Gly Asn Pro Ser Pro Arg Lys Ala Leu Leu Ala Gly Ala
   930 935 940
   Glu Gly Phe Pro Pro Met Leu Asp Ile Pro Pro Asp Ala Asp Asp Glu
   945 950 955 960
   Thr Met Val Glu Leu Ala Ile Ala Leu Ser Leu Gln Gln Asp Gln Gln
   965 970 975
   Gly Ser Ser Ser Ser Ala Leu Gly Leu Gln Ser Leu Gly Leu Ser Gly
   980 985 990
   Gln Ala Pro Ser Ser Ser Ser Leu Asp Ala Gly Thr Leu Ser Asp Thr
   995 1000 1005
   Thr Ala Ser Ala Pro Ala Ser Asp Asp Glu Gly Ser Thr Ala Ala Thr
   1010 1015 1020
   Asp Gly Ser Thr Leu Arg Thr Ser Pro Ala Asp His Gly Gly Ser Val
   1025 1030 1035 1040
   Gly Ser Glu Ser Gly Gly Ser Ala Val Asp Ser Val Ala Gly Glu His
   1045 1050 1055
   Ser Val Ser Gly Arg Ser Ser Ala Tyr Gly Asp Ala Thr Ala Glu Gly
   1060 1065 1070
   His Pro Ala Gly Pro Gly Ser Val Ser Ser Ser Thr Gly Ala Ile Ser
   1075 1080 1085
   Thr Thr Thr Gly His Gln Glu Gly Asp Gly Ser Glu Gly Glu Gly Glu
   1090 1095 1100
   Gly Glu Ala Glu Gly Asp Val His Thr Ser Asn Arg Leu His Met Val
   1105 1110 1115 1120
   Arg Leu Met Leu Leu Glu Arg Leu Leu Gln Thr Leu Pro Gln Leu Arg
   1125 1130 1135
   Asn Val Gly Gly Val Arg Ala Ile Pro Tyr Met Gln Val Ile Leu Met
   1140 1145 1150
   Leu Thr Thr Asp Leu Asp Gly Glu Asp Glu Lys Asp Lys Gly Ala Leu
   1155 1160 1165
   Asp Asn Leu Leu Ala Gln Leu Ile Ala Glu Leu Gly Met Asp Lys Lys
   1170 1175 1180
   Asp Val Ser Lys Lys Asn Glu Arg Ser Ala Leu Asn Glu Val His Leu
   1185 1190 1195 1200
   Val Val Met Arg Leu Leu Ser Val Phe Met Ser Arg Thr Lys Ser Gly
   1205 1210 1215
   Ser Lys Ser Ser Ile Cys Glu Ser Ser Ser Leu Ile Ser Ser Ala Thr
   1220 1225 1230
   Ala Ala Ala Leu Leu Ser Ser Gly Ala Val Asp Tyr Cys Leu His Val
   1235 1240 1245
   Leu Lys Ser Leu Leu Glu Tyr Trp Lys Gly Gln Gln Ser Glu Glu Glu
   1250 1255 1260
   Pro Val Thr Thr Ser Gln Leu Leu Lys Pro His Thr Thr Ser Ser Pro
   1265 1270 1275 1280
   Pro Asp Met Ser Pro Phe Phe Leu Arg Gln Tyr Val Lys Gly His Ala
   1285 1290 1295
   Ala Asp Val Phe Glu Ala Tyr Thr Gln Leu Leu Thr Glu Met Val Leu
   1300 1305 1310
   Arg Leu Pro Tyr Gln Ile Lys Lys Ile Ala Asp Thr Ser Ser Arg Ile
   1315 1320 1325
   Pro Pro Pro Val Phe Asp His Ser Trp Phe Tyr Phe Leu Ser Glu Tyr
   1330 1335 1340
   Leu Met Ile Gln Gln Thr Pro Phe Val Arg Arg Gln Val Arg Lys Leu
   1345 1350 1355 1360
   Leu Leu Phe Ile Cys Gly Ser Lys Glu Lys Tyr Arg Gln Leu Arg Asp
   1365 1370 1375
   Leu His Thr Leu Asp Ser His Val Arg Gly Ile Lys Lys Leu Leu Glu
   1380 1385 1390
   Glu Gln Gly Ile Phe Leu Arg Ala Ser Val Val Thr Ala Ser Ser Gly
   1395 1400 1405
   Ser Ala Leu Gln Tyr Asp Thr Leu Ile Ser Leu Met Glu His Leu Lys
   1410 1415 1420
   Ala Cys Ala Glu Ile Ala Ala Gln Arg Thr Ile Asn Trp Gln Lys Phe
   1425 1430 1435 1440
   Cys Ile Lys Asp Asp Ser Val Leu Tyr Phe Leu Leu Gln Val Ser Phe
   1445 1450 1455
   Leu Val Asp Glu Gly Val Ser Pro Val Leu Leu Gln Leu Leu Ser Cys
   1460 1465 1470
   Ala Leu Cys Gly Ser Lys Val Leu Ala Ala Leu Ala Ala Ser Thr Gly
   1475 1480 1485
   Ser Ser Ser Val Ala Ser Ser Ala Pro Pro Ala Ala Ser Ser Gly Gln
   1490 1495 1500
   Thr Thr Thr Gln Ser Lys Ser Ser Thr Lys Lys Ser Lys Lys Glu Glu
   1505 1510 1515 1520
   Lys Glu Lys Glu Lys Glu Gly Glu Ser Ser Gly Ser Gln Glu Asp Gln
   1525 1530 1535
   Leu Cys Thr Ala Leu Val Asn Gln Leu Asn Arg Phe Ala Asp Lys Glu
   1540 1545 1550
   Thr Leu Ile Gln Phe Leu Arg Cys Phe Leu Leu Glu Ser Asn Ser Ser
   1555 1560 1565
   Ser Val Arg Trp Gln Ala His Cys Leu Thr Leu His Ile Tyr Arg Asn
   1570 1575 1580
   Ser Asn Lys Ala Gln Gln Glu Leu Leu Leu Asp Leu Met Trp Ser Ile
   1585 1590 1595 1600
   Trp Pro Glu Leu Pro Ala Tyr Gly Arg Lys Ala Ala Gln Phe Val Asp
   1605 1610 1615
   Leu Leu Gly Tyr Phe Ser Leu Lys Thr Ala Gln Thr Glu Lys Lys Leu
   1620 1625 1630
   Lys Glu Tyr Ser Gln Lys Ala Val Glu Ile Leu Arg Thr Gln Asn His
   1635 1640 1645
   Ile Leu Thr Asn His Pro Asn Ser Asn Ile Tyr Asn Thr Leu Ser Gly
   1650 1655 1660
   Leu Val Glu Phe Asp Gly Tyr Tyr Leu Glu Ser Asp Pro Cys Leu Val
   1665 1670 1675 1680
   Cys Asn Asn Pro Glu Val Pro Phe Cys Tyr Ile Lys Leu Ser Ser Ile
   1685 1690 1695
   Lys Val Asp Thr Arg Tyr Thr Thr Thr Gln Gln Val Val Lys Leu Ile
   1700 1705 1710
   Gly Ser His Thr Ile Ser Lys Val Thr Val Lys Ile Gly Asp Leu Lys
   1715 1720 1725
   Arg Thr Lys Met Val Arg Thr Ile Asn Leu Tyr Tyr Asn Asn Arg Thr
   1730 1735 1740
   Val Gln Ala Ile Val Glu Leu Lys Asn Lys Pro Ala Arg Trp His Lys
   1745 1750 1755 1760
   Ala Lys Lys Val Gln Leu Thr Pro Gly Gln Thr Glu Val Lys Ile Asp
   1765 1770 1775
   Leu Pro Leu Pro Ile Val Ala Ser Asn Leu Met Ile Glu Phe Ala Asp
   1780 1785 1790
   Phe Tyr Glu Asn Tyr Gln Ala Ser Thr Glu Thr Leu Gln Cys Pro Arg
   1795 1800 1805
   Cys Ser Ala Ser Val Pro Ala Asn Pro Gly Val Cys Gly Asn Cys Gly
   1810 1815 1820
   Glu Asn Val Tyr Gln Cys His Lys Cys Arg Ser Ile Asn Tyr Asp Glu
   1825 1830 1835 1840
   Lys Asp Pro Phe Leu Cys Asn Ala Cys Gly Phe Cys Lys Tyr Ala Arg
   1845 1850 1855
   Phe Asp Phe Met Leu Tyr Ala Lys Pro Cys Cys Pro Val Asp Pro Ile
   1860 1865 1870
   Glu Asn Glu Glu Asp Arg Lys Lys Ala Val Ser Asn Ile Asn Thr Leu
   1875 1880 1885
   Leu Asp Lys Ala Asp Arg Val Tyr His Gln Leu Met Gly His Arg Pro
   1890 1895 1900
   Gln Leu Glu Asn Leu Leu Cys Lys Val Asn Glu Ala Ala Pro Glu Lys
   1905 1910 1915 1920
   Pro Gln Glu Asp Ser Gly Thr Ala Gly Gly Ile Ser Ser Thr Ser Ala
   1925 1930 1935
   Ser Val Asn Arg Tyr Ile Leu Gln Leu Ala Gln Glu Tyr Cys Gly Asp
   1940 1945 1950
   Cys Lys Asn Ser Phe Asp Glu Leu Ser Lys Ile Ile Gln Lys Val Phe
   1955 1960 1965
   Ala Ser Arg Lys Glu Leu Leu Glu Tyr Asp Leu Gln Gln Arg Glu Ala
   1970 1975 1980
   Ala Thr Lys Ser Ser Arg Thr Ser Val Gln Pro Thr Phe Thr Ala Ser
   1985 1990 1995 2000
   Gln Tyr Arg Ala Leu Ser Val Leu Gly Cys Gly His Thr Ser Ser Thr
   2005 2010 2015
   Lys Cys Tyr Gly Cys Ala Ser Ala Val Thr Glu His Cys Ile Thr Leu
   2020 2025 2030
   Leu Arg Ala Leu Ala Thr Asn Pro Ala Leu Arg His Ile Leu Val Ser
   2035 2040 2045
   Gln Gly Leu Ile Arg Glu Leu Phe Asp Tyr Asn Leu Arg Arg Gly Ala
   2050 2055 2060
   Ala Ala Ile Arg Glu Glu Val Arg Gln Leu Met Cys Leu Leu Thr Arg
   2065 2070 2075 2080
   Asp Asn Pro Glu Ala Thr Gln Gln Met Asn Asp Leu Ile Ile Gly Lys
   2085 2090 2095
   Val Ser Thr Ala Leu Lys Gly His Trp Ala Asn Pro Asp Leu Ala Ser
   2100 2105 2110
   Ser Leu Gln Tyr Glu Met Leu Leu Leu Thr Asp Ser Ile Ser Lys Glu
   2115 2120 2125
   Asp Ser Cys Trp Glu Leu Arg Leu Arg Cys Ala Leu Ser Leu Phe Leu
   2130 2135 2140
   Met Ala Val Asn Ile Lys Thr Pro Val Val Val Glu Asn Ile Thr Leu
   2145 2150 2155 2160
   Met Cys Leu Arg Ile Leu Gln Lys Leu Ile Lys Pro Pro Ala Pro Thr
   2165 2170 2175
   Ser Lys Lys Asn Lys Asp Val Pro Val Glu Ala Leu Thr Thr Val Lys
   2180 2185 2190
   Pro Tyr Cys Asn Glu Ile His Ala Gln Ala Gln Leu Trp Leu Lys Arg
   2195 2200 2205
   Asp Pro Lys Ala Ser Tyr Glu Ala Trp Lys Lys Cys Leu Pro Ile Arg
   2210 2215 2220
   Gly Val Asp Gly Asn Gly Lys Ser Pro Ser Lys Ser Glu Leu His Arg
   2225 2230 2235 2240
   Leu Tyr Leu Thr Glu Lys Tyr Val Trp Arg Trp Lys Gln Phe Leu Ser
   2245 2250 2255
   Arg Arg Gly Lys Arg Thr Thr Pro Leu Asp Leu Lys Leu Gly His Asn
   2260 2265 2270
   Asn Trp Leu Arg Gln Val Leu Phe Thr Pro Ala Thr Gln Ala Ala Arg
   2275 2280 2285
   Gln Ala Ala Cys Thr Ile Val Glu Ala Leu Ala Thr Val Pro Ser Arg
   2290 2295 2300
   Lys Gln Gln Val Leu Asp Leu Leu Thr Ser Tyr Leu Asp Glu Leu Ser
   2305 2310 2315 2320
   Val Ala Gly Glu Cys Ala Ala Glu Tyr Leu Ala Leu Tyr Gln Lys Leu
   2325 2330 2335
   Ile Ala Ser Cys His Trp Lys Val Tyr Leu Ala Ala Arg Gly Val Leu
   2340 2345 2350
   Pro Tyr Val Gly Asn Leu Ile Thr Lys Glu Ile Ala Arg Leu Leu Ala
   2355 2360 2365
   Leu Glu Glu Ala Thr Leu Ser Thr Asp Leu Gln Gln Gly Tyr Ala Leu
   2370 2375 2380
   Lys Ser Leu Thr Gly Leu Leu Ser Ser Phe Val Glu Val Glu Ser Ile
   2385 2390 2395 2400
   Lys Arg His Phe Lys Ser Arg Leu Val Gly Thr Val Leu Asn Gly Tyr
   2405 2410 2415
   Leu Cys Leu Arg Lys Leu Val Leu Gln Arg Thr Lys Leu Ile Asp Glu
   2420 2425 2430
   Thr Gln Asp Met Leu Leu Glu Met Leu Glu Asp Met Thr Thr Gly Thr
   2435 2440 2445
   Glu Ser Glu Thr Lys Ala Phe Met Ala Val Cys Ile Glu Thr Ala Lys
   2450 2455 2460
   Arg Tyr Asn Leu Asp Asp Tyr Arg Thr Pro Val Phe Ile Phe Glu Arg
   2465 2470 2475 2480
   Leu Cys Ser Ile Ile Tyr Pro Glu Glu Asn Glu Val Thr Glu Phe Phe
   2485 2490 2495
   Val Thr Leu Glu Lys Asp Pro Gln Gln Glu Asp Phe Leu Gln Gly Arg
   2500 2505 2510
   Met Pro Gly Asn Pro Tyr Ser Ser Asn Glu Pro Gly Ile Gly Pro Leu
   2515 2520 2525
   Met Arg Asp Ile Lys Asn Lys Ile Cys Gln Asp Cys Asp Leu Val Ala
   2530 2535 2540
   Leu Leu Glu Asp Asp Ser Gly Met Glu Leu Leu Val Asn Asn Lys Ile
   2545 2550 2555 2560
   Ile Ser Leu Asp Leu Pro Val Ala Glu Val Tyr Lys Lys Val Trp Cys
   2565 2570 2575
   Thr Thr Asn Glu Gly Glu Pro Met Arg Ile Val Tyr Arg Met Arg Gly
   2580 2585 2590
   Leu Leu Gly Asp Ala Thr Glu Glu Phe Ile Glu Ser Leu Asp Ser Thr
   2595 2600 2605
   Thr Asp Glu Glu Glu Asp Glu Glu Glu Val Tyr Arg Met Ala Gly Val
   2610 2615 2620
   Met Ala Gln Cys Gly Gly Leu Gln Cys Met Leu Asn Arg Leu Ala Gly
   2625 2630 2635 2640
   Val Lys Asp Phe Lys Gln Gly Arg His Leu Leu Thr Val Leu Leu Lys
   2645 2650 2655
   Leu Phe Ser Tyr Cys Val Lys Val Lys Val Asn Arg Gln Gln Leu Val
   2660 2665 2670
   Lys Leu Glu Met Asn Thr Leu Asn Val Met Leu Gly Thr Leu Asn Leu
   2675 2680 2685
   Ala Leu Val Ala Glu Gln Glu Ser Lys Asp Ser Gly Gly Ala Ala Val
   2690 2695 2700
   Ala Glu Gln Val Leu Ser Ile Met Glu Ile Ile Leu Asp Glu Ser Asn
   2705 2710 2715 2720
   Ala Glu Pro Leu Ser Glu Asp Lys Gly Asn Leu Leu Leu Thr Gly Asp
   2725 2730 2735
   Lys Asp Gln Leu Val Met Leu Leu Asp Gln Ile Asn Ser Thr Phe Val
   2740 2745 2750
   Arg Ser Asn Pro Ser Val Leu Gln Gly Leu Leu Arg Ile Ile Pro Tyr
   2755 2760 2765
   Leu Ser Phe Gly Glu Val Glu Lys Met Gln Ile Leu Val Glu Arg Phe
   2770 2775 2780
   Lys Pro Tyr Cys Ser Phe Asp Lys Tyr Asp Glu Asp His Ser Gly Asp
   2785 2790 2795 2800
   Asp Lys Val Phe Leu Asp Cys Phe Cys Lys Ile Ala Ala Gly Ile Lys
   2805 2810 2815
   Asn Asn Ser Asn Gly His Gln Leu Lys Asp Leu Ile Leu Gln Lys Gly
   2820 2825 2830
   Ile Thr Gln Ser Ala Leu Asp Tyr Met Lys Lys His Ile Pro Ser Ala
   2835 2840 2845
   Lys Asn Leu Asp Ala Asp Ile Trp Lys Lys Phe Leu Ser Arg Pro Ala
   2850 2855 2860
   Leu Pro Phe Ile Leu Arg Leu Leu Arg Gly Leu Ala Met Gln His Pro
   2865 2870 2875 2880
   Ala Thr Gln Val Leu Ile Gly Thr Asp Ser Ile Thr Ser Leu His Lys
   2885 2890 2895
   Leu Glu Gln Val Ser Ser Asp Glu Gly Ile Gly Thr Leu Ala Glu Asn
   2900 2905 2910
   Leu Leu Glu Ala Leu Arg Glu His Pro Asp Val Asn Lys Lys Ile Asp
   2915 2920 2925
   Ala Ala Arg Arg Glu Thr Arg Ala Glu Lys Lys Arg Met Ala Met Ala
   2930 2935 2940
   Met Arg Gln Lys Ala Leu Gly Thr Leu Gly Met Thr Thr Asn Glu Lys
   2945 2950 2955 2960
   Gly Gln Val Val Thr Lys Thr Ala Leu Leu Lys Gln Met Glu Glu Leu
   2965 2970 2975
   Ile Glu Glu Pro Gly Leu Thr Cys Cys Ile Cys Arg Glu Gly Gly Thr
   2980 2985 2990
   Lys Phe Gln Pro Thr Lys Val Leu Gly Ile Tyr Thr Phe Thr Lys Arg
   2995 3000 3005
   Val Ala Leu Glu Glu Met Glu Asn Lys Pro Arg Lys Gln Gln Gly Tyr
   3010 3015 3020
   Ser Thr Val Ser His Phe Asn Ile Val His Tyr Asp Cys His Leu Ala
   3025 3030 3035 3040
   Ala Val Arg Leu Ala Arg Gly Arg Glu Glu Trp Glu Ser Ala Ala Leu
   3045 3050 3055
   Gln Asn Ala Asn Thr Lys Cys Asn Gly Leu Leu Pro Val Trp Gly Pro
   3060 3065 3070
   His Val Pro Glu Ser Ala Phe Ala Thr Cys Leu Ala Arg His Asn Thr
   3075 3080 3085
   Tyr Leu Gln Glu Cys Thr Gly Gln Arg Glu Pro Thr Tyr Gln Leu Asn
   3090 3095 3100
   Ile His Asp Ile Lys Leu Leu Phe Leu Arg Phe Ala Met Glu Gln Ser
   3105 3110 3115 3120
   Phe Ser Ala Asp Thr Gly Gly Gly Gly Arg Glu Ser Asn Ile His Leu
   3125 3130 3135
   Ile Pro Tyr Ile Ile His Thr Val Leu Tyr Val Leu Asn Thr Thr Arg
   3140 3145 3150
   Ala Thr Ser Arg Glu Glu Lys Asn Leu Gln Gly Phe Leu Glu Gln Pro
   3155 3160 3165
   Arg Glu Lys Trp Thr Glu Ser Ala Phe Asp Val Asp Gly Pro His Tyr
   3170 3175 3180
   Phe Thr Ile Leu Ala Leu His Val Leu Pro Pro Glu Gln Trp Lys Ala
   3185 3190 3195 3200
   Thr Arg Val Glu Ile Leu Arg Arg Leu Leu Val Ala Ser His Ala Arg
   3205 3210 3215
   Ala Val Ala Pro Gly Gly Ala Thr Arg Leu Thr Asp Lys Ala Val Lys
   3220 3225 3230
   Asp Tyr Ser Ala Tyr Arg Ser Ser Leu Leu Phe Trp Ala Leu Val Asp
   3235 3240 3245
   Leu Ile Tyr Asn Met Phe Lys Lys Val Pro Thr Ser Asn Thr
   3250 3255 3260
<210> 46
   <211> 2276
   <212> PRT
   <213> homo sapiens
<220>
   <222> (1)..(2276)
   <223> KIAA0462 protein
<300>
   <301> Andersson, et al. Vogel, et al. Lbeit, et al. Ohara, 0
   <302> Unknown protein [Homo sapiens].
   <308> GI 3005744
   <309> 1998-04-02.
   <313> 1 TO 494
<400> 46
   Glu His Ser Val Ser Gly Arg Ser Ser Ala Tyr Gly Asp Ala Thr Ala
   1 5 10 15
   Glu Gly His Pro Ala Gly Pro Gly Ser Val Ser Ser Ser Thr Gly Ala
   20 25 30
   Ile Ser Thr Thr Thr Gly His Gln Glu Gly Asp Gly Ser Glu Gly Glu
   35 40 45
   Gly Glu Gly Glu Thr Glu Gly Asp Val His Thr Ser Asn Arg Leu His
   50 55 60
   Met Val Arg Leu Met Leu Leu Glu Arg Leu Leu Gln Thr Leu Pro Gln
   65 70 75 80
   Leu Arg Asn Val Gly Gly Val Arg Ala Ile Pro Tyr Met Gln Val Ile
   85 90 95
   Leu Met Leu Thr Thr Asp Leu Asp Gly Glu Asp Glu Lys Asp Lys Gly
   100 105 110
   Ala Leu Asp Asn Leu Leu Ser Gln Leu Ile Ala Glu Leu Gly Met Asp
   115 120 125
   Lys Lys Asp Val Ser Lys Lys Asn Glu Arg Ser Ala Leu Asn Glu Val
   130 135 140
   His Leu Val Val Met Arg Leu Leu Ser Val Phe Met Ser Arg Thr Lys
   145 150 155 160
   Ser Gly Ser Lys Ser Ser Ile Cys Glu Ser Ser Ser Leu Ile Ser Ser
   165 170 175
   Ala Thr Ala Ala Ala Leu Leu Ser Ser Gly Ala Val Asp Tyr Cys Leu
   180 185 190
   His Val Leu Lys Ser Leu Leu Glu Tyr Trp Lys Ser Gln Gln Asn Asp
   195 200 205
   Glu Glu Pro Val Ala Thr Ser Gln Leu Leu Lys Pro His Thr Thr Ser
   210 215 220
   Ser Pro Pro Asp Met Ser Pro Phe Phe Leu Arg Gln Tyr Val Lys Gly
   225 230 235 240
   His Ala Ala Asp Val Phe Glu Ala Tyr Thr Gln Leu Leu Thr Glu Met
   245 250 255
   Val Leu Arg Leu Pro Tyr Gln Ile Lys Lys Ile Thr Asp Thr Asn Ser
   260 265 270
   Arg Ile Pro Pro Pro Val Phe Asp His Ser Trp Phe Tyr Phe Leu Ser
   275 280 285
   Glu Tyr Leu Met Ile Gln Gln Thr Pro Phe Val Arg Arg Gln Val Arg
   290 295 300
   Lys Leu Leu Leu Phe Ile Cys Gly Ser Lys Glu Lys Tyr Arg Gln Leu
   305 310 315 320
   Arg Asp Leu His Thr Leu Asp Ser His Val Arg Gly Ile Lys Lys Leu
   325 330 335
   Leu Glu Glu Gln Gly Ile Phe Leu Arg Ala Ser Val Val Thr Ala Ser
   340 345 350
   Ser Gly Ser Ala Leu Gln Tyr Asp Thr Leu Ile Ser Leu Met Glu His
   355 360 365
   Leu Lys Ala Cys Ala Glu Ile Ala Ala Gln Arg Thr Ile Asn Trp Gln
   370 375 380
   Lys Phe Cys Ile Lys Asp Asp Ser Val Leu Tyr Phe Leu Leu Gln Val
   385 390 395 400
   Ser Phe Leu Val Asp Glu Gly Val Ser Pro Val Leu Leu Gln Leu Leu
   405 410 415
   Ser Cys Ala Leu Cys Gly Ser Lys Val Leu Ala Ala Leu Ala Ala Ser
   420 425 430
   Ser Gly Ser Ser Ser Ala Ser Ser Ser Ser Ala Pro Val Ala Ala Ser
   435 440 445
   Ser Gly Gln Ala Thr Thr Gln Ser Lys Ser Ser Thr Lys Lys Ser Lys
   450 455 460
   Lys Glu Glu Lys Glu Lys Glu Lys Asp Gly Glu Thr Ser Gly Ser Gln
   465 470 475 480
   Glu Asp Gln Leu Cys Thr Ala Leu Val Asn Gln Leu Asn Lys Phe Ala
   485 490 495
   Asp Lys Glu Thr Leu Ile Gln Phe Leu Arg Cys Phe Leu Leu Glu Ser
   500 505 510
   Asn Ser Ser Ser Val Arg Trp Gln Ala His Cys Leu Thr Leu His Ile
   515 520 525
   Tyr Arg Asn Ser Ser Lys Ser Gln Gln Glu Leu Leu Leu Asp Leu Met
   530 535 540
   Trp Ser Ile Trp Pro Glu Leu Pro Ala Tyr Gly Arg Lys Ala Ala Gln
   545 550 555 560
   Phe Val Asp Leu Leu Gly Tyr Phe Ser Leu Lys Thr Pro Gln Thr Glu
   565 570 575
   Lys Lys Leu Lys Glu Tyr Ser Gln Lys Ala Val Glu Ile Leu Arg Thr
   580 585 590
   Gln Asn His Ile Leu Thr Asn His Pro Asn Ser Asn Ile Tyr Asn Thr
   595 600 605
   Leu Ser Gly Leu Val Glu Phe Asp Gly Tyr Tyr Leu Glu Ser Asp Pro
   610 615 620
   Cys Leu Val Cys Asn Asn Pro Glu Val Pro Phe Cys Tyr Ile Lys Leu
   625 630 635 640
   Ser Ser Ile Lys Val Asp Thr Arg Tyr Thr Thr Thr Gln Gln Val Val
   645 650 655
   Lys Leu Ile Gly Ser His Thr Ile Ser Lys Val Thr Val Lys Ile Gly
   660 665 670
   Asp Leu Lys Arg Thr Lys Met Val Arg Thr Ile Asn Leu Tyr Tyr Asn
   675 680 685
   Asn Arg Thr Val Gln Ala Ile Val Glu Leu Lys Asn Lys Pro Ala Arg
   690 695 700
   Trp His Lys Ala Lys Lys Val Gln Leu Thr Pro Gly Gln Thr Glu Val
   705 710 715 720
   Lys Ile Asp Leu Pro Leu Pro Ile Val Ala Ser Asn Leu Met Ile Glu
   725 730 735
   Phe Ala Asp Phe Tyr Glu Asn Tyr Gln Ala Ser Thr Glu Thr Leu Gln
   740 745 750
   Cys Pro Arg Cys Ser Ala Ser Val Pro Ala Asn Pro Gly Val Cys Gly
   755 760 765
   Asn Cys Gly Glu Asn Val Tyr Gln Cys His Lys Cys Arg Ser Ile Asn
   770 775 780
   Tyr Asp Glu Lys Asp Pro Phe Leu Cys Asn Ala Cys Gly Phe Cys Lys
   785 790 795 800
   Tyr Ala Arg Phe Asp Phe Met Leu Tyr Ala Lys Pro Cys Cys Ala Val
   805 810 815
   Asp Pro Ile Glu Asn Glu Glu Asp Arg Lys Lys Ala Val Ser Asn Ile
   820 825 830
   Asn Thr Leu Leu Asp Lys Ala Asp Arg Val Tyr His Gln Leu Met Gly
   835 840 845
   His Arg Pro Gln Leu Glu Asn Leu Leu Cys Lys Val Asn Glu Ala Ala
   850 855 860
   Pro Glu Lys Pro Gln Asp Asp Ser Gly Thr Ala Gly Gly Ile Ser Ser
   865 870 875 880
   Thr Ser Ala Ser Val Asn Arg Tyr Ile Leu Gln Leu Ala Gln Glu Tyr
   885 890 895
   Cys Gly Asp Cys Lys Asn Ser Phe Asp Glu Leu Ser Lys Ile Ile Gln
   900 905 910
   Lys Val Phe Ala Ser Arg Lys Glu Leu Leu Glu Tyr Asp Leu Gln Gln
   915 920 925
   Arg Glu Ala Ala Thr Lys Ser Ser Arg Thr Ser Val Gln Pro Thr Phe
   930 935 940
   Thr Ala Ser Gln Tyr Arg Ala Leu Ser Val Leu Gly Cys Gly His Thr
   945 950 955 960
   Ser Ser Thr Lys Cys Tyr Gly Cys Ala Ser Ala Val Thr Glu His Cys
   965 970 975
   Ile Thr Leu Leu Arg Ala Leu Ala Thr Asn Pro Ala Leu Arg His Ile
   980 985 990
   Leu Val Ser Gln Gly Leu Ile Arg Glu Leu Phe Asp Tyr Asn Leu Arg
   995 1000 1005
   Arg Gly Ala Ala Ala Met Arg Glu Glu Val Arg Gln Leu Met Cys Leu
   1010 1015 1020
   Leu Thr Arg Asp Asn Pro Glu Ala Thr Gln Gln Met Asn Asp Leu Ile
   1025 1030 1035 1040
   Ile Gly Lys Val Ser Thr Ala Leu Lys Gly His Trp Ala Asn Pro Asp
   1045 1050 1055
   Leu Ala Ser Ser Leu Gln Tyr Glu Met Leu Leu Leu Thr Asp Ser Ile
   1060 1065 1070
   Ser Lys Glu Asp Ser Cys Trp Glu Leu Arg Leu Arg Cys Ala Leu Ser
   1075 1080 1085
   Leu Phe Leu Met Ala Val Asn Ile Lys Thr Pro Val Val Val Glu Asn
   1090 1095 1100
   Ile Thr Leu Met Cys Leu Arg Ile Leu Gln Lys Leu Ile Lys Pro Pro
   1105 1110 1115 1120
   Ala Pro Thr Ser Lys Lys Asn Lys Asp Val Pro Val Glu Ala Leu Thr
   1125 1130 1135
   Thr Val Lys Pro Tyr Cys Asn Glu Ile His Ala Gln Ala Gln Leu Trp
   1140 1145 1150
   Leu Lys Arg Asp Pro Lys Ala Ser Tyr Asp Ala Trp Lys Lys Cys Leu
   1155 1160 1165
   Pro Ile Arg Gly Ile Asp Gly Asn Gly Lys Ala Pro Ser Lys Ser Glu
   1170 1175 1180
   Leu Arg His Leu Tyr Leu Thr Glu Lys Tyr Val Trp Arg Trp Lys Gln
   1185 1190 1195 1200
   Phe Leu Ser Arg Arg Gly Lys Arg Thr Ser Pro Leu Asp Leu Lys Leu
   1205 1210 1215
   Gly His Asn Asn Trp Leu Arg Gln Val Leu Phe Thr Pro Ala Thr Gln
   1220 1225 1230
   Ala Ala Arg Gln Ala Ala Cys Thr Ile Val Glu Ala Leu Ala Thr Ile
   1235 1240 1245
   Pro Ser Arg Lys Gln Gln Val Leu Asp Leu Leu Thr Ser Tyr Leu Asp
   1250 1255 1260
   Glu Leu Ser Ile Ala Gly Glu Cys Ala Ala Glu Tyr Leu Ala Leu Tyr
   1265 1270 1275 1280
   Gln Lys Leu Ile Thr Ser Ala His Trp Lys Val Tyr Leu Ala Ala Arg
   1285 1290 1295
   Gly Val Leu Pro Tyr Val Gly Asn Leu Ile Thr Lys Glu Ile Ala Arg
   1300 1305 1310
   Leu Leu Ala Leu Glu Glu Ala Thr Leu Ser Thr Asp Leu Gln Gln Gly
   1315 1320 1325
   Tyr Ala Leu Lys Ser Leu Thr Gly Leu Leu Ser Ser Phe Val Glu Val
   1330 1335 1340
   Glu Ser Ile Lys Arg His Phe Lys Ser Arg Leu Val Gly Thr Val Leu
   1345 1350 1355 1360
   Asn Gly Tyr Leu Cys Leu Arg Lys Leu Val Val Gln Arg Thr Lys Leu
   1365 1370 1375
   Ile Asp Glu Thr Gln Asp Met Leu Leu Glu Met Leu Glu Asp Met Thr
   1380 1385 1390
   Thr Gly Thr Glu Ser Glu Thr Lys Ala Phe Met Ala Val Cys Ile Glu
   1395 1400 1405
   Thr Ala Lys Arg Tyr Asn Leu Asp Asp Tyr Arg Thr Pro Val Phe Ile
   1410 1415 1420
   Phe Glu Arg Leu Cys Ser Ile Ile Tyr Pro Glu Glu Asn Glu Val Thr
   1425 1430 1435 1440
   Glu Phe Phe Val Thr Leu Glu Lys Asp Pro Gln Gln Glu Asp Phe Leu
   1445 1450 1455
   Gln Gly Arg Met Pro Gly Asn Pro Tyr Ser Ser Asn Glu Pro Gly Ile
   1460 1465 1470
   Gly Pro Leu Met Arg Asp Ile Lys Asn Lys Ile Cys Gln Asp Cys Asp
   1475 1480 1485
   Leu Val Ala Leu Leu Glu Asp Asp Ser Gly Met Glu Leu Leu Val Asn
   1490 1495 1500
   Asn Lys Ile Ile Ser Leu Asp Leu Pro Val Ala Glu Val Tyr Lys Lys
   1505 1510 1515 1520
   Val Trp Cys Thr Thr Asn Glu Gly Glu Pro Met Arg Ile Val Tyr Arg
   1525 1530 1535
   Met Arg Gly Leu Leu Gly Asp Ala Thr Glu Glu Phe Ile Glu Ser Leu
   1540 1545 1550
   Asp Ser Thr Thr Asp Glu Glu Glu Asp Glu Glu Glu Val Tyr Lys Met
   1555 1560 1565
   Ala Gly Val Met Ala Gln Cys Gly Gly Leu Glu Cys Met Leu Asn Arg
   1570 1575 1580
   Leu Ala Gly Ile Arg Asp Phe Lys Gln Gly Arg His Leu Leu Thr Val
   1585 1590 1595 1600
   Leu Leu Lys Leu Phe Ser Tyr Cys Val Lys Val Lys Val Asn Arg Gln
   1605 1610 1615
   Gln Leu Val Lys Leu Glu Met Asn Thr Leu Asn Val Met Leu Gly Thr
   1620 1625 1630
   Leu Asn Leu Ala Leu Val Ala Glu Gln Glu Ser Lys Asp Ser Gly Gly
   1635 1640 1645
   Ala Ala Val Ala Glu Gln Val Leu Ser Ile Met Glu Ile Ile Leu Asp
   1650 1655 1660
   Glu Ser Asn Ala Glu Pro Leu Ser Glu Asp Lys Gly Asn Leu Leu Leu
   1665 1670 1675 1680
   Thr Gly Asp Lys Asp Gln Leu Val Met Leu Leu Asp Gln Ile Asn Ser
   1685 1690 1695
   Thr Phe Val Arg Ser Asn Pro Ser Val Leu Gln Gly Leu Leu Arg Ile
   1700 1705 1710
   Ile Pro Tyr Leu Ser Phe Gly Glu Val Glu Lys Met Gln Ile Leu Val
   1715 1720 1725
   Glu Arg Phe Lys Pro Tyr Cys Asn Phe Asp Lys Tyr Asp Glu Asp His
   1730 1735 1740
   Ser Gly Asp Asp Lys Val Phe Leu Asp Cys Phe Cys Lys Ile Ala Ala
   1745 1750 1755 1760
   Gly Ile Lys Asn Asn Ser Asn Gly His Gln Leu Lys Asp Leu Ile Leu
   1765 1770 1775
   Gln Lys Gly Ile Thr Gln Asn Ala Leu Asp Tyr Met Lys Lys His Ile
   1780 1785 1790
   Pro Ser Ala Lys Asn Leu Asp Ala Asp Ile Trp Lys Lys Phe Leu Ser
   1795 1800 1805
   Arg Pro Ala Leu Pro Phe Ile Leu Arg Leu Leu Arg Gly Leu Ala Ile
   1810 1815 1820
   Gln His Pro Gly Thr Gln Val Leu Ile Gly Thr Asp Ser Ile Pro Asn
   1825 1830 1835 1840
   Leu His Lys Leu Glu Gln Val Ser Ser Asp Glu Gly Ile Gly Thr Leu
   1845 1850 1855
   Ala Glu Asn Leu Leu Glu Ala Leu Arg Glu His Pro Asp Val Asn Lys
   1860 1865 1870
   Lys Ile Asp Ala Ala Arg Arg Glu Thr Arg Ala Glu Lys Lys Arg Met
   1875 1880 1885
   Ala Met Ala Met Arg Gln Lys Ala Leu Gly Thr Leu Gly Met Thr Thr
   1890 1895 1900
   Asn Glu Lys Gly Gln Val Val Thr Lys Thr Ala Leu Leu Lys Gln Met
   1905 1910 1915 1920
   Glu Glu Leu Ile Glu Glu Pro Gly Leu Thr Cys Cys Ile Cys Arg Glu
   1925 1930 1935
   Gly Tyr Lys Phe Gln Pro Thr Lys Val Leu Gly Ile Tyr Thr Phe Thr
   1940 1945 1950
   Lys Arg Val Ala Leu Glu Glu Met Glu Asn Lys Pro Arg Lys Gln Gln
   1955 1960 1965
   Gly Tyr Ser Thr Val Ser His Phe Asn Ile Val His Tyr Asp Cys His
   1970 1975 1980
   Leu Ala Ala Val Arg Leu Ala Arg Gly Arg Glu Glu Trp Glu Ser Ala
   1985 1990 1995 2000
   Ala Leu Gln Asn Ala Asn Thr Lys Cys Asn Gly Leu Leu Pro Val Trp
   2005 2010 2015
   Gly Pro His Val Pro Glu Ser Ala Phe Ala Thr Cys Leu Ala Arg His
   2020 2025 2030
   Asn Thr Tyr Leu Gln Glu Cys Thr Gly Gln Arg Glu Pro Thr Tyr Gln
   2035 2040 2045
   Leu Asn Ile His Asp Ile Lys Leu Leu Phe Leu Arg Phe Ala Met Glu
   2050 2055 2060
   Gln Ser Phe Ser Ala Asp Thr Gly Gly Gly Gly Arg Glu Ser Asn Ile
   2065 2070 2075 2080
   His Leu Ile Pro Tyr Ile Ile His Thr Val Leu Tyr Val Leu Asn Thr
   2085 2090 2095
   Thr Arg Ala Thr Ser Arg Glu Glu Lys Asn Leu Gln Gly Phe Leu Glu
   2100 2105 2110
   Gln Pro Lys Glu Lys Trp Val Glu Ser Ala Phe Glu Val Asp Gly Pro
   2115 2120 2125
   Tyr Tyr Phe Thr Val Leu Ala Leu His Ile Leu Pro Pro Glu Gln Trp
   2130 2135 2140
   Arg Ala Thr Arg Val Glu Ile Leu Arg Arg Leu Leu Val Thr Ser Gln
   2145 2150 2155 2160
   Ala Arg Ala Val Ala Pro Gly Gly Ala Thr Arg Leu Thr Asp Lys Ala
   2165 2170 2175
   Val Lys Asp Tyr Ser Ala Tyr Arg Ser Ser Leu Leu Phe Trp Ala Leu
   2180 2185 2190
   Val Asp Leu Ile Tyr Asn Met Phe Lys Lys Val Pro Thr Ser Asn Thr
   2195 2200 2205
   Glu Gly Gly Trp Ser Cys Ser Leu Ala Glu Tyr Ile Arg His Asn Asp
   2210 2215 2220
   Met Pro Ile Tyr Glu Ala Ala Asp Lys Ala Leu Lys Thr Phe Gln Glu
   2225 2230 2235 2240
   Glu Phe Met Pro Val Glu Thr Phe Ser Glu Phe Leu Asp Val Ala Gly
   2245 2250 2255
   Leu Leu Ser Glu Ile Thr Asp Pro Glu Ser Phe Leu Lys Asp Leu Leu
   2260 2265 2270
   Asn Ser Val Pro
   2275
<210> 47
   <211> 5322
   <212> PRT
   <213> Drosophila melanogastor
<400> 47
   Met Ser Ala His Ser Gly Gly Thr Asp Trp Asn Ser Val Val Lys Ala
   1 5 10 15
   Leu Ile Leu Asn Arg Thr Gly Ala Leu Asn Lys Asn Glu Val Val Asn
   20 25 30
   Leu Leu Lys Ala Ile Thr Arg Cys Glu His Asp Phe Phe Glu Glu Glu
   35 40 45
   Ser Asn Phe Thr Gln Phe Tyr Thr Ala Phe Ala Ala Leu Ala Ala Asp
   50 55 60
   Lys Leu Met Gln Ile Lys Thr Ile Cys Gln Thr Gln Ile Cys Gln Leu
   65 70 75 80
   His Asp Ala Thr Ala Val Leu Ile Arg Phe Ile Ile Tyr Arg Leu Pro
   85 90 95
   Arg Val Ser Val Tyr Glu Thr Lys Trp Leu Leu Gly Ala Leu Lys Met
   100 105 110
   Leu Cys Glu Gly Arg Glu Cys Pro Ala Ser Ala Ser Ser Ser Met Phe
   115 120 125
   Asp Tyr Asn Ala Val Ala Asn Val Leu Lys Ser Cys Lys His Pro Glu
   130 135 140
   Ser Thr Thr Lys Ser Ile Met Pro Ser Ser Ser Ser Ser Gly Ser Gly
   145 150 155 160
   Ala Ser Asn Asp Lys Glu Ser Pro Lys Ser Glu Ile Lys Arg Ser Arg
   165 170 175
   Ser Asp Leu Ser Ser Val Ile Leu Gln Gln Leu Ile Ala Pro Leu Glu
   180 185 190
   Pro Gly Lys Met Thr Trp Val Pro Leu Ser Glu Glu Val Thr Asp Cys
   195 200 205
   Thr Glu Gln Ile Leu Ala Ala Asn Val Glu Tyr Phe Gln Glu Gln Asn
   210 215 220
   Gly Val Asp Thr Leu Leu Asp Val Cys Val Ser Leu Pro Ile Leu Asn
   225 230 235 240
   Arg Tyr Arg Ser Lys Tyr Met Glu Thr Ile Asn Gly Gly Lys Ser Leu
   245 250 255
   Tyr Leu Pro Leu Thr Gln Val Glu Ala Thr Ala Val Lys Ser Ser Met
   260 265 270
   Asn His Met Leu Thr Asp Leu Thr Ile Leu Ser Gln Ala Gln Ala Leu
   275 280 285
   Ile Glu Met Gln Pro Leu Thr Pro Ser Arg Ile Glu Arg Leu Ser Met
   290 295 300
   Cys Gly Ile Ala Ala Leu Tyr Asn Ala Val Leu Thr Ser Ile Ala Thr
   305 310 315 320
   Ser Val Leu Gly Met Ser Gln Ala Ser Ser Ser Gln Lys Gln Thr Ala
   325 330 335
   Ser Thr Ser Gln Gly Ser Gly Val Gly Gly Ser Ser Gly Gly Gln Ser
   340 345 350
   Asn Lys Asp His Asp Asp Phe Glu Asp Gln Ala Cys Ser Ile Val Asn
   355 360 365
   Lys Ala Leu Glu Ile Tyr Ser Asn Ile Gly His Met Phe Lys Thr Ser
   370 375 380
   Ala Arg Ile His Val Tyr Gln Asn His Leu Cys Tyr Gly Ser Trp Leu
   385 390 395 400
   Leu Ile Ser Gly Ile Gln Gly Ala Met Gly Ala Ser Gly Ser Gly Gly
   405 410 415
   Ser Ser Ser Asp Ser Ala Ser Lys Ser Ala Ser Lys Ala Thr Lys Ser
   420 425 430
   Gly Ser Glu Ala Gly Thr Ala Pro Thr Thr Pro Ile Ala Arg Val Asn
   435 440 445
   Leu Phe Lys Val Gln Gln Gly Phe Gly Glu Leu Asn Ala Ala Ile Ala
   450 455 460
   Asn His Ser Ile Lys Leu Leu Ser Glu Leu Ile Glu Asp Leu Lys Val
   465 470 475 480
   Glu Ala Ala Cys Gly Gln Ser Leu Glu Ser Thr Glu Leu Pro Glu Pro
   485 490 495
   Ala Gln Phe Asp Ile Leu Gln Asn Tyr Ser Ser Leu Glu Arg Ile Val
   500 505 510
   Arg Val Leu Asn Thr Ala Thr Leu His Gln Leu Phe Thr Phe Leu Ala
   515 520 525
   Thr Val Ala Tyr Arg Lys Ala Cys Thr Leu Lys Arg Ala Ser Ala Lys
   530 535 540
   Asp Arg Thr Glu Cys Glu Pro Ile Ser Tyr Ser Asp Ser Thr Thr Tyr
   545 550 555 560
   Phe Asn Asp Ser Leu Ser Cys Ser Asp Asn Ser Glu Glu Asp Asp Ser
   565 570 575
   Glu Ser Tyr Leu Gly His Trp Phe Lys Glu Thr Leu Ser Pro Glu Thr
   580 585 590
   His Asp Asp Asn Ala Asn Thr Ser Thr Gln Glu Arg Ala Glu Gln Lys
   595 600 605
   Ser Ala Leu Val Pro Lys Leu Asp Glu Pro His Glu Tyr Leu Asp Leu
   610 615 620
   Ala Ala Asp Ile Phe Cys Phe Leu Asp Gln Phe Leu Ala Asn Arg His
   625 630 635 640
   Ala Tyr Met Gln Arg Tyr Val Lys Ala Gly Val Ser Asp Gln Gln Met
   645 650 655
   Leu Leu Met Ala Asn Ile Ile Lys Asp Phe Asp Arg Asp Val Met Arg
   660 665 670
   Asn Glu Thr Asp Gln Gly Ser Gly Asn Ala Pro Ala Ala Ser Ala Gly
   675 680 685
   Ala Gly Thr Ser Ala Gly Ala Ser Thr Lys Trp Gln Thr Ser Met Ile
   690 695 700
   Arg Phe Ser Gly Ala Ala Gly Arg Tyr Ile His Asn Leu Ile Ser Thr
   705 710 715 720
   Ser Leu Leu Ser Glu Gln Leu Gln Ser Asn Leu Leu Gln His Leu Ser
   725 730 735
   Ile Ser Pro Trp Ser Thr Asp Thr Asn Thr Trp Pro Leu Gln Val Tyr
   740 745 750
   Pro Ser Thr Leu Ser Val Leu Val Gln Ile Leu Leu Leu Lys Pro Thr
   755 760 765
   Gln Glu Lys Glu Ala Ala Cys Leu Ser Val Trp His Arg Leu Ile Asn
   770 775 780
   Thr Leu Val Glu Gly Val Cys Ser Ser Asn Thr Ala Ser Asp Ser Asp
   785 790 795 800
   Tyr Glu Asp Leu Asn Ile Glu His Ala Gln Leu Leu Leu Phe Leu Phe
   805 810 815
   His Ser Leu Asn Leu Met Gln Lys Lys Ser Ile Leu Leu Leu Thr Ala
   820 825 830
   Gly Gly Val Ile Arg Cys Ala Glu Val Cys Arg Gly Ile Ser Glu Asp
   835 840 845
   Arg Pro Val Lys Asn Ser Gln Ile Met Leu Leu Ser Arg Leu Leu Leu
   850 855 860
   Phe Leu Glu Tyr Leu Met Lys His Leu Tyr Asn Ala Pro Pro Glu Leu
   865 870 875 880
   Leu Asp Gln Val Arg Trp Asn Leu Phe Ser Val Ser Ser Met Pro Asp
   885 890 895
   Thr Gln Lys Ile Thr Asp Leu Leu Asn Cys Arg Thr Lys Leu Asn Ser
   900 905 910
   Tyr Cys Arg His Asp Ile Glu Glu Asn Phe Arg Lys Ser Ala Gly Glu
   915 920 925
   Tyr Gly Ser Ser Ile Arg Pro Thr Phe Tyr Ser Leu Val Met Gly Asp
   930 935 940
   Pro Glu Ile Ser Tyr Trp Ala Gln Glu Phe Lys Leu Asp Gly Leu Ala
   945 950 955 960
   Trp Asn Phe Ile Leu Cys Thr Pro Asp Lys Leu Lys Tyr Pro Leu Leu
                   965                 970                 975
   Val Asp Ala Leu Thr Asp Ile Leu Ser Ile Thr Asp Met Ser Met Tyr
               980                 985                 990
   Ser Lys Glu Lys Asp Lys Glu Ala Ser Met His Asn Leu Cys Ala Ile
           995                 1000                1005
   Gln Tyr Cys Phe Thr Ile Ala Trp Lys Leu Asn Leu Gly Leu Pro Pro
       1010                1015                1020
   Ser Thr Ser His Val Glu Ser Leu Lys Ala Glu Arg Ser Pro Asn Leu
   1025                1030                1035                1040
   His Ser Leu Met Trp Ser Ile Arg Leu Pro Leu Ala Ser Ser His Tyr
                   1045                1050                1055
   Leu Val Val Ser Ser Leu Ile Lys Gln Gly Met Tyr Thr Gln Tyr Ala
               1060                1065                1070
   Glu Thr Leu Trp Thr His Val Gly Asp Ile Gly Ala Asp Ile Lys Tyr
           1075                1080                1085
   Ser Leu Lys Gln Thr Ile Leu Gly Val Glu Ala Phe Asn Ser Gln Met
       1090                1095                1100
   Asn Gly Asn Gly Thr Pro Arg Leu Ser Asp Leu Ile Leu Phe Asp Ser
   1105                1110                1115                1120
   Leu Val Ala His Met Gln Ala Val Ala Trp Ala Asn Lys Glu Gly Leu
                   1125                1130                1135
   Lys Trp Pro Arg Lys Glu Ser Glu Asp Ala Ala Gly Glu Gln Ser Ala
               1140                1145                1150
   Gly Thr Ser Leu Thr Ser Ser Ser Asn Asp Pro Glu Leu Tyr Ser Ser
           1155                1160                1165
   Asn Glu Ser Ile Asp Asp Gln Lys Leu Lys Gln Asp Asp Asp Gly Lys
       1170                1175                1180
   Leu Ser Ser Asp Leu Gln Lys Tyr Asn Gln Leu Val Asn Glu Leu Met
   1185                1190                1195                1200
   Val Lys Leu Met Asn Ser Tyr Gln Leu Leu Ser Val Ile Val Arg Gly
                   1205                1210                1215
   Gln Met Leu Lys Gln Leu Ser Ser Ser Thr Pro Glu Lys Ala Leu Asn
               1220                1225                1230
   Leu Ile Val Pro Ile Val Ser Asp Lys Pro Ala Ile Met Leu Glu Leu
           1235                1240                1245
   His Ala Ala Phe Leu Lys Leu Leu Pro Asn Glu Asp Lys Gln Leu Ile
       1250                1255                1260
   Ala Asn Glu Trp Pro Lys Cys Leu Met Val Asn Asp Phe Ala Phe Asn
   1265                1270                1275                1280
   Gly Lys Gln His Pro Val Glu Pro Tyr Ile Leu Asn Val Ile Asp Ala
   1285 1290 1295
   His Ile Ile Glu Leu Thr Arg Gly Ser Asn Tyr Ser Thr Leu His Thr
   1300 1305 1310
   Leu Lys His Cys Leu Lys Ser Ile Leu Gln Leu Phe Glu Leu Leu Leu
   1315 1320 1325
   Pro His Arg Thr Ala Asn Ala Glu Val Glu Thr Gln Leu Lys Gln Leu
   1330 1335 1340
   Leu Ile Ser Ser Met Leu Asp Met Arg Thr Asp Tyr Leu Gln Gly His
   1345 1350 1355 1360
   Ser Glu His Cys Leu Arg Glu Ile Leu Ser Gly Leu Thr Gln Glu Ala
   1365 1370 1375
   Gln Lys Leu Leu Leu Tyr Glu His Met Val Gly Tyr Cys Tyr Arg Met
   1380 1385 1390
   Leu Lys Glu Phe Ala Ala Asp Leu Arg Gln Pro Gln Ala Ala Gly Pro
   1395 1400 1405
   Ser Gly Gly Ala Pro Leu Asp Gln Asp Arg Ala Met Phe Asn Glu Ser
   1410 1415 1420
   Met Leu Phe Ala Val Leu Lys Thr Met Phe Lys Met Leu Glu Lys Pro
   1425 1430 1435 1440
   Val Ala Val Gln Ala Met Arg Gln Phe Phe Lys Asp Gln Arg Ser Gly
   1445 1450 1455
   Ser Leu Thr Thr Leu Leu Leu Ser Phe Thr Gly Thr Ser Leu Pro Val
   1460 1465 1470
   Ser Tyr Ala Arg Lys Met Leu Gln Phe Val Asn Arg Leu Phe Gln Leu
   1475 1480 1485
   Ser Leu Gln Ala Asp Ser Gln Phe Gln His Glu Asp Leu Val Glu Cys
   1490 1495 1500
   Phe Ser Glu Leu Ala Thr Val Asp Val Ala Arg Leu Lys Gln Trp Leu
   1505 1510 1515 1520
   Gly His Ile Ile Tyr Gly Pro Asn Val Ser Thr Asp Val Ser Thr Ser
   1525 1530 1535
   Glu Ala Leu Asp Thr Thr Cys Arg Met Leu Thr Ser Ile Leu Gln Pro
   1540 1545 1550
   Ser Ser Ser Ser Ser Ser Asn Ala Gln Thr Pro Thr Asn Met Ala Thr
   1555 1560 1565
   Val Ser Ala Met Pro Ser Ile Ser Asp Gln Leu Asp Pro Met Glu Ile
   1570 1575 1580
   Glu Tyr Asp Cys Gly Thr Ala Ala Gly Gly Gly Glu Ser Asn Pro Gly
   1585 1590 1595 1600
   Thr Gly Gly Ala Ala Ala Asn Thr Ser Gln Ile Leu Ser Leu Trp Gln
   1605 1610 1615
   Ala Ala Gln Pro Asp Pro Ser Glu Glu Ser Ser Gln Ala Cys Asp His
   1620 1625 1630
   Ser Asp Ser Glu Arg Asn Gly Ala Leu Leu Leu Ser Phe Val Lys Ser
   1635 1640 1645
   Leu Val Lys Asp Gln Ser Lys Ala Ser Gln Ile Ala Pro Pro Leu Phe
   1650 1655 1660
   Gln Ala Leu Leu Gln Leu Gly Gln Thr Leu Ile Ser Pro Pro Gln Glu
   1665 1670 1675 1680
   Gly Cys Asp Phe Ala Asp Val Leu Gln Ile Met Ile Thr Leu Ala Asp
   1685 1690 1695
   Ala Ser Pro Ala Arg Gly His Val Ala Leu Phe Asn Thr Thr Leu Leu
   1700 1705 1710
   Trp Leu Glu Leu Ala Lys Leu Gln Leu Pro Asp Lys His Leu Lys His
   1715 1720 1725
   Ala Glu Asn Val Ser Ala Gln Leu Arg Tyr Leu Ser Glu Leu Leu Gln
   1730 1735 1740
   Ser Ile Gly Phe Arg Gly Ser Arg Gln His Asn Pro Pro Trp Asp Asp
   1745 1750 1755 1760
   Glu Leu Gln Thr Asp Ile Asp Glu Leu Tyr Asp Glu Leu Ala Glu Glu
   1765 1770 1775
   Gly Glu Gln Asp Ser Leu Leu Asp Asp Ser Asp Glu Asp Thr Leu Asn
   1780 1785 1790
   Asn Lys Leu Cys Thr Phe Ser Gln Thr Gln Lys Glu Phe Met Asn Gln
   1795 1800 1805
   His Trp Tyr His Cys His Thr Cys Asn Met Ile Asn Thr Val Gly Val
   1810 1815 1820
   Cys Ser Val Cys Ala Arg Val Cys His Lys Gly His Asp Val Ser Tyr
   1825 1830 1835 1840
   Ala Lys Tyr Gly Asn Phe Phe Cys Asp Cys Gly Ala Lys Glu Asp Gly
   1845 1850 1855
   Ser Cys Gln Ala Leu Ser Arg Arg Ile Gly Ser Asn Glu Val Arg Asp
   1860 1865 1870
   Ser Ala Gly Ile Gly Ser Tyr Leu Pro Ser His Met Ser Leu Leu Ala
   1875 1880 1885
   Gly Lys Lys Arg Ser Ser Leu Pro Val Gly Gln Pro Val Leu Thr Arg
   1890 1895 1900
   Lys Asp Ser Leu Thr Asn Glu Arg Ile Ala Val Leu Thr Lys Leu Leu
   1905 1910 1915 1920
   Glu Pro Tyr Arg Glu Thr Leu Gln His Gln Asp Gln Trp Leu Leu Val
   1925 1930 1935
   Val Arg Cys Ile Leu Glu Tyr Phe Asp Leu Leu Leu Pro Ser Ile Asn
   1940 1945 1950
   Glu Asn Cys Met Leu Tyr Ser Ile Val Gly Cys His Lys Arg Ala Thr
   1955 1960 1965
   Ala Ala Leu Glu Arg Leu His Leu Leu Glu Gln Ser Phe Gln Val Thr
   1970 1975 1980
   Asp Gln Leu Met Phe Ala Thr Leu Gly Ser Gln Glu Gly Ala Phe Glu
   1985 1990 1995 2000
   Asn Val Arg Met Asn Tyr Ser Gly Asp Gln Gly Gln Thr Ile Lys His
   2005 2010 2015
   Leu Leu Ser Ser Gly Val Val Arg Arg Val Ala Phe Cys Cys Leu Ser
   2020 2025 2030
   Ser Pro His Gly Arg Arg Gln Gln Leu Ala Val Ser His Glu Lys Gly
   2035 2040 2045
   Lys Val Thr Ile Leu Gln Leu Ser Ala Leu Leu Lys Gln Ala Asp Ala
   2050 2055 2060
   Ser Lys Arg Lys Leu Thr Leu Thr Gln Leu Ser Ser Ala Pro Ile Ala
   2065 2070 2075 2080
   Cys Thr Val Ile Ser Leu Ala Ala Asn Pro Cys Asn Glu Asp Cys Leu
   2085 2090 2095
   Ala Val Cys Gly Leu Lys Glu Cys His Ile Leu Thr Phe Ser Ser Ser
   2100 2105 2110
   Gly Ser Thr Asn Glu His Ile Val Val Asn Pro Gln Leu Glu Asn Gly
   2115 2120 2125
   Asn Tyr Ile Lys Lys Ala Val Trp Leu Pro Gly Ser Gln Thr Leu Leu
   2130 2135 2140
   Ala Val Val Thr Ser Asp Tyr Val Lys Ile Tyr Asp Leu Ala Val Asp
   2145 2150 2155 2160
   Thr Tyr Ser Pro Lys Tyr Tyr Tyr Leu Val Ala Val Gly Lys Ile Arg
   2165 2170 2175
   Asp Cys Thr Phe Val Tyr Gln Asp Gly Asn Tyr Asn Met Leu Thr Phe
   2180 2185 2190
   Ala Ser Ser Gly Tyr Ile Tyr Thr Gln Gln Leu Asp Gln Gln Ser Leu
   2195 2200 2205
   Ala Val His Gly Asp Phe Tyr Val Thr Asn Thr Leu Glu Leu Ser His
   2210 2215 2220
   Gln His Ile Lys Asp Ile Ala Gly His Ile Gly Gly Gly Gly Val Ser
   2225 2230 2235 2240
   Ile Tyr Tyr Ser His Thr Leu Gln Leu Leu Phe Tyr Ser Tyr Ser Cys
   2245 2250 2255
   Gly Arg Ser Phe Phe Ser Pro Leu Thr Asn Val Ser Glu Gly Val Lys
   2260 2265 2270
   Gly Ile Tyr His Leu Asp Thr Asn Ser Ala Ser Lys Ser Ala Ser Lys
   2275 2280 2285
   Gly Pro Leu Gln Pro Leu Val Gln Trp Thr Glu Val Thr Gly His Pro
   2290 2295 2300
   Gly Leu Val Tyr Ala Ser Met Gln Thr Ser Asn Asn Pro Ile Ile Leu
   2305 2310 2315 2320
   Met Ile Thr Pro Glu Arg Ile Tyr Leu Gln Glu Ile Lys Ala Gln Ser
   2325 2330 2335
   Ala Lys Ser Arg Ile Met Asp Val Val Gly Ile Arg His Ala Val Ala
   2340 2345 2350
   Gly Val Glu Lys Thr Thr Leu Leu Leu Leu Cys Glu Asp Gly Ser Leu
   2355 2360 2365
   Arg Ile Phe Ser Ala Gln Pro Glu Tyr Thr Ser Phe Trp Leu Ser Pro
   2370 2375 2380
   Gln Val Gln Pro Phe Gly Asn Gln Leu Tyr Ser Ser Thr Leu Met Ala
   2385 2390 2395 2400
   Lys Gly Gly Gly Ser Gly Ser Thr Ser Lys Ser Lys Ser Asn Thr Ala
   2405 2410 2415
   Ser Gly Lys Met Thr Ser Arg Lys Ala Ser Gln Gln Gln Lys Gln Pro
   2420 2425 2430
   Thr Ala Gly Gly Gln Pro Val Phe Pro Ile Asp Phe Phe Glu His Cys
   2435 2440 2445
   Asn Met Leu Ala Asp Val Glu Phe Gly Gly Asn Asp Leu Leu Gln Ile
   2450 2455 2460
   Tyr Asn Lys Gln Lys Leu Lys Thr Arg Leu Phe Ser Thr Gly Met Phe
   2465 2470 2475 2480
   Val Ala Ser Thr Arg Ser Asn Gly Phe Thr Leu Glu Val Ile Asn Asn
   2485 2490 2495
   Asp Pro Asn Val Val Ile Val Gly Ile Arg Val Leu Ile Gly Thr Gln
   2500 2505 2510
   Asp Val Gln Arg Ala Pro Gln Ser Val Thr Ile Leu Gly Arg Thr Ile
   2515 2520 2525
   Pro Thr Pro Val Arg Arg Ala Arg Trp Phe Asp Ile Pro Leu Thr Arg
   2530 2535 2540
   Glu Glu Met Leu Gln Ser Asp Lys Leu Leu Lys Val Val Phe Ala Lys
   2545 2550 2555 2560
   Ala Pro Asp Pro Glu His Val Thr Leu Leu Asp Cys Ile Glu Val Tyr
   2565 2570 2575
   Gly Lys Ser Lys Glu Leu Val Gly Trp Pro Asp Glu Ser Glu Asp Val
   2580 2585 2590
   Thr Val Pro Gly Ser Ser Ala Pro Ala Val Ser Ser Ser Gln Ala Ser
   2595 2600 2605
   Ser Ala Asn Phe Gly Glu Gly Phe Asn Cys Ile Thr Gln Leu Asp Arg
   2610 2615 2620
   Met Ala Asn His Leu Leu Glu Val Met Asp Cys Ala Leu His Leu Leu
   2625 2630 2635 2640
   Gly Ser Gly Val Pro Ala Ser Met Arg Gln Lys Ala Val Lys Thr Ala
   2645 2650 2655
   Ser Ala Leu Leu Leu Leu Pro Thr Pro Asn Pro Val Gln Thr Gln Ala
   2660 2665 2670
   Arg Tyr Val Leu Ala Thr Leu Tyr Gly Thr Arg Ala Leu Tyr His Asn
   2675 2680 2685
   Tyr Lys Asp Gly Val Leu Leu Gln Phe Val Asn Arg Glu Leu Gln Ser
   2690 2695 2700
   Met Gln Pro Lys Leu Glu Lys Leu Glu Thr Leu Arg Glu Ile Asp Pro
   2705 2710 2715 2720
   Glu Ala Phe Tyr Arg Leu Val Leu Met Val Arg Gly Ile Ala Asn Ala
   2725 2730 2735
   Arg Pro Gln Ser Leu Ala Lys Ile Cys Val Glu Asn Asn Tyr Asp Ile
   2740 2745 2750
   Val Pro Thr Leu Met Gly Ile Val Leu Glu Leu His Lys Val Thr Pro
   2755 2760 2765
   Thr Leu Asp Glu Pro Val Asn Ile Val Lys Arg Gly Leu Cys Gln Pro
   2770 2775 2780
   Glu Thr Ile Val His Cys Leu Val Glu Ile Met Tyr Gly Phe Ala Leu
   2785 2790 2795 2800
   Ala Asp Pro Gly Gln Val Gly Arg Met Thr Lys Tyr Phe Ile Asp Leu
   2805 2810 2815
   Leu Lys His Asp Ala Ser Val Ile Ser His Ser Ala Lys Glu Ala Leu
   2820 2825 2830
   Ile Leu Leu Leu Ser Pro Arg Met Lys Arg Arg Lys Val Ala Ile Val
   2835 2840 2845
   Thr Pro Pro Ala Cys Ser Thr Pro Thr Pro Ser Thr Ser Thr Met Gln
   2850 2855 2860
   Ala Leu Gln Ala Ala Ala Ser Ser Ala Ala Ser Asp Ile Ile Glu Glu
   2865 2870 2875 2880
   Ala Ala Gly Val Val Val Asp Gly Ser Val Gly Gly Gly Gly Leu Pro
   2885 2890 2895
   Glu Pro Asn Ala Asp Ala Glu Gly Ala Ala Val Gly Val Gly Gly Val
   2900 2905 2910
   Gly Gln Gln Gln Met Leu Asn Leu Glu Ala Phe Met Gly Gly Gly Phe
   2915 2920 2925
   Pro Arg Leu Leu Gly Leu Pro Glu Asp Ala Asp Asp Glu Ala Ile Met
   2930 2935 2940
   Asp Ile Ala Ile Ala Leu Ser Leu Gln Gln His Gly Gly Asp Ala Asn
   2945 2950 2955 2960
   Ala Leu Gln Ser Leu Gln Gln Gly Leu Ala Asn Leu Gln Gly Ile Arg
   2965 2970 2975
   Gln Ala Thr Ala Met Ala Ala Ala Val Asn Ala Ala Ala Asn Val Ser
   2980 2985 2990
   Leu Gly Gly Ser Asp Asp Asp Glu Gly Ser Asn Val Ala Thr Asp Gly
   2995 3000 3005
   Ser Thr Leu Arg Thr Ser Pro Ala Glu Pro Ala Gly Ser Gly Gly Ser
   3010 3015 3020
   Glu Ser Gly Gly Ser Gly Val Glu Ser Ile Gly Gly Thr Ser Ala Arg
   3025 3030 3035 3040
   Ser Ser Asn Phe Gly Asp His Ala Asn Ala Ser Pro Pro Arg Gln Gly
   3045 3050 3055
   Ser Thr Lys Asp Asp Gln Glu Gln Pro Gly Pro Ser Gly Val Ala Gly
   3060 3065 3070
   Ser Gly Gly Val Ala Val Leu Ser Ala Met Ser Ser Ser Glu Asp Asn
   3075 3080 3085
   Glu Ala Asn Glu Asp Asp Lys Leu Ser Lys Leu His Asp Leu Arg Ile
   3090 3095 3100
   Thr Val Leu Glu Ser Ile Ile Gln His Leu Gly Thr Phe Asp Leu Cys
   3105 3110 3115 3120
   Asn Gly Leu Gln Ala Ile Pro Leu Ile Gln Val Ile Leu Met Leu Thr
   3125 3130 3135
   Thr Asp Leu Asn Gly Asn Asn Glu Arg Asp Gln Gln Val Leu His Asp
   3140 3145 3150
   Leu Leu Thr Ala Leu Val Gly Leu Cys Gly Asn Trp Lys Arg Gly Ala
   3155 3160 3165
   Ala Ala Arg Met Glu Thr Lys Cys Pro Gly Asn Glu Val Arg Leu Ala
   3170 3175 3180
   Leu Leu Ser Leu Phe Gly Val Leu Met Gly Lys Thr Lys Ser Lys Gln
   3185 3190 3195 3200
   Thr Gly Thr Thr Ser Pro Pro His Gln Phe Lys Asp Asn Ser Ser Phe
   3205 3210 3215
   Val Ala Ser Thr Thr Ala Asn Val Leu Ser Lys Ser Gly Ala Phe Val
   3220 3225 3230
   Tyr Ala Leu Glu Ala Leu Asn Thr Leu Leu Val His Trp Lys Asn Val
   3235 3240 3245
   Leu Gly Asp Pro Tyr Ala Ala Gly Gly Gly Leu Ala Ser Gln Ser Ala
   3250 3255 3260
   Gln Ala Ser Gly Gly Ala Ser Gly Pro Gly Val Gln Leu Leu Lys Pro
   3265 3270 3275 3280
   Ile Lys His Gly Pro Lys Pro Asp Ile Ser Ile Leu Ile Pro His Asn
   3285 3290 3295
   Tyr Leu Lys Asn Tyr Pro Asp Ile Phe Glu Ser Tyr Asp Gly Leu Leu
   3300 3305 3310
   Thr Glu Ile Ile Val Arg Leu Pro Tyr Gln Ile Leu Arg Leu Ser Ser
   3315 3320 3325
   Ala His Pro Asp Asn Tyr Asp Ser Gly Phe Cys Glu Ala Met Thr Phe
   3330 3335 3340
   Thr Leu Cys Glu Tyr Met Met Leu Asn Leu Asn Thr Leu Leu Arg Arg
   3345 3350 3355 3360
   Gln Val Arg Lys Leu Leu Met Tyr Ile Cys Gly Ser Lys Glu Lys Phe
   3365 3370 3375
   Arg Met Tyr Arg Asp Gly His Ser Leu Asp Ala His Phe Arg Val Val
   3380 3385 3390
   Lys Arg Val Cys Asn Ile Val Ser Ser Lys Thr Gly Ala Pro Tyr Asn
   3395 3400 3405
   Ala Asn Pro Pro Met Leu Ser Tyr Asp Ala Leu Val Glu Leu Thr Glu
   3410 3415 3420
   His Leu Arg Thr Cys Gln Glu Ile Ser Gln Met Arg Thr Gly Asn Trp
   3425 3430 3435 3440
   Gln Lys Phe Cys Val Val His Glu Asp Ala Leu Ala Met Leu Met Glu
   3445 3450 3455
   Ile Ala Cys Tyr Gln Leu Asp Asp Gly Val Ser Pro Ile Ile Ile Gln
   3460 3465 3470
   Leu Leu Gln Ala Ala Val Cys Asn Leu Pro Pro Pro Ser Gly Ser Lys
   3475 3480 3485
   Gln Ala Gln Pro Gln Pro Ser Thr Ser Ser Ala Ser Gly Lys Leu Arg
   3490 3495 3500
   Thr Asp Arg Glu Lys Ser Glu Asp Thr Asp Ala Tyr Tyr Ser Lys Phe
   3505 3510 3515 3520
   Asp Pro Ala Gln Cys Gly Thr Phe Val His Gln Ile Phe Arg Tyr Ala
   3525 3530 3535
   Cys Asp Ala Leu Ile Ile Arg Phe Val Arg Ile Phe Leu Leu Glu Asn
   3540 3545 3550
   Asn Ile Thr Gln Leu Arg Trp Gln Ala His Ser Phe Met Thr Gly Leu
   3555 3560 3565
   Phe Glu His Ala Asn Glu Arg Gln Arg Glu Lys Leu Leu Asn Ile Phe
   3570 3575 3580
   Trp Asn Leu Trp Pro Leu Val Pro Thr Tyr Gly Arg Arg Thr Ala Gln
   3585 3590 3595 3600
   Phe Val Asp Leu Leu Gly Tyr Leu Thr Leu Ser Thr Arg Ser Ile Thr
   3605 3610 3615
   Glu Arg Leu Pro Glu Phe Val Ser Arg Ala Val Asp Val Leu Arg Gln
   3620 3625 3630
   Gln Asn Glu Leu Leu Cys Lys His Pro Asn Ala Pro Ile Tyr Thr Thr
   3635 3640 3645
   Leu Glu Ser Ile Leu Gln Val Asn Gly Tyr Tyr Leu Glu Ser Glu Pro
   3650 3655 3660
   Cys Leu Val Cys Asn Asn Pro Glu Val Pro Met Ala Asn Ile Lys Leu
   3665 3670 3675 3680
   Pro Ser Val Lys Ser Asp Ser Lys Tyr Thr Thr Thr Thr Met Ile Tyr
   3685 3690 3695
   Lys Leu Val Gln Cys His Thr Ile Ser Lys Leu Ile Val Arg Ile Ala
   3700 3705 3710
   Asp Leu Lys Arg Thr Lys Met Val Arg Thr Ile Asn Val Tyr Tyr Asn
   3715 3720 3725
   Asn Arg Ser Val Gln Ala Val Val Glu Leu Lys Asn Arg Pro Ala Leu
   3730 3735 3740
   Trp His Lys Ala Arg Ser Val Ser Leu Gln Ser Ala Gln Thr Glu Leu
   3745 3750 3755 3760
   Lys Ile Asp Phe Pro Leu Pro Ile Thr Ala Cys Asn Leu Met Ile Glu
   3765 3770 3775
   Phe Ala Asp Phe Phe Glu Thr Val Ser Gly Ser Ser Glu Asn Leu Gln
   3780 3785 3790
   Cys Pro Arg Cys Ser Ala Ala Val Pro Ala Tyr Pro Gly Val Cys Gly
   3795 3800 3805
   Asn Cys Gly Glu Asn Val Phe Gln Cys His Lys Cys Arg Ala Ile Asn
   3810 3815 3820
   Tyr Asp Glu Lys Asp Pro Phe Leu Cys His Ser Cys Gly Phe Cys Lys
   3825 3830 3835 3840
   Tyr Ala Lys Phe Asp Phe Ser Met Tyr Ala Arg Val Cys Cys Ala Val
   3845 3850 3855
   Asp Pro Ile Glu Ser Ala Glu Asp Arg Val Lys Thr Val Ser Leu Ile
   3860 3865 3870
   His Ser Ser Leu Glu Arg Ala Asp Arg Asn Tyr Arg Gln Leu Leu Thr
   3875 3880 3885
   Asn Lys Gln Met Leu Glu Leu Leu Ile Gln Lys Val Ala Glu His Arg
   3890 3895 3900
   Ser Ser Asp Arg Met Val Glu Asp Asn Met Ala Ser Val His Ser Thr
   3905 3910 3915 3920
   Ser Gln Val Asn Lys Ile Ile Gln Leu Leu Ala Gln Lys Tyr Cys Val
   3925 3930 3935
   Glu Ser Arg Thr Ser Phe Glu Glu Leu Ser Lys Ile Val Gln Lys Val
   3940 3945 3950
   Lys Ala Cys Arg Ser Glu Leu Val Ala Tyr Asp Arg Gln Gln Gln Asp
   3955 3960 3965
   Gln Pro Pro Val Asn Pro Gly Ser Thr Thr Gly Ala Glu Asn Pro Thr
   3970 3975 3980
   Thr Asn Arg Cys Tyr Gly Cys Ala Leu Ala Ser Thr Glu Gln Cys Leu
   3985 3990 3995 4000
   Thr Leu Leu Arg Ala Met Ala Tyr Asn Tyr Asp Cys Arg Val Cys Leu
   4005 4010 4015
   Tyr Ser Gln Gly Leu Val Ser Glu Leu Ala Glu His Asn Leu Arg Arg
   4020 4025 4030
   Gly Thr Pro Leu Ile Gln Glu Glu Val Arg Asn Leu Leu Val Val Leu
   4035 4040 4045
   Thr Lys Asp Asn Ala Glu Ala Cys Met His Leu Leu Gln Leu Val Thr
   4050 4055 4060
   Thr Arg Val Lys Asn Ala Leu Met Gly Ser Ile Pro Leu Ile Ser Leu
   4065 4070 4075 4080
   Glu Ala Ala Val His Gln Glu Met Thr Leu Leu Glu Val Leu Leu Gly
   4085 4090 4095
   Gln Asp Asp Ile Cys Trp Glu Tyr Lys Leu Lys Val Ile Phe Glu Leu
   4100 4105 4110
   Phe Ile Ser Asn Cys Arg Leu Pro Arg Gly Pro Val Thr Ala Val Leu
   4115 4120 4125
   His Pro Cys Leu Arg Ile Met Gln Asn Leu Ile Cys Pro Val Leu Pro
   4130 4135 4140
   Gly Ser Lys Pro Asn Gln Lys Val Ala Thr Thr Asp Leu Cys Ser Met
   4145 4150 4155 4160
   Lys Met Phe Glu Gly Asn Thr Val Asp Tyr Arg Ala Trp Leu Asn Ser
   4165 4170 4175
   Asp Arg Asn His Glu Tyr Ala Ala Trp Ser Lys Arg Met Pro Ser Asn
   4180 4185 4190
   Asn Gln Ala Lys Leu Lys Asn Ala Lys Asp Gln Asn Val Ala Ala Ser
   4195 4200 4205
   Gly Gly Ser Asp Ala Pro Pro Lys Ser Arg Arg Glu Val Arg Val Ala
   4210 4215 4220
   Phe Leu Ser Glu Lys Tyr Gly Lys Arg Trp Arg Glu Arg Val Leu Asp
   4225 4230 4235 4240
   Lys Gln Arg Val Ile Lys Pro Leu Val Phe Asn Ala Lys Trp Ile Gln
   4245 4250 4255
   Pro Leu Leu Phe Asn Ala Asn Ser Arg Phe Gly Arg Gln Leu Ala Cys
   4260 4265 4270
   Ser Leu Leu Ser Ser Leu Ser Arg Thr Asn Glu Arg Arg Gln Gln Ala
   4275 4280 4285
   Leu Asn Met Leu Thr Ser Phe Leu Lys His Val Gly Glu Ala Gly Glu
   4290 4295 4300
   Ala Ser Ala Glu Tyr Leu Met Leu Tyr Lys Asn Met Ala Thr Glu Gln
   4305 4310 4315 4320
   Pro Trp Leu Gln Tyr Leu Val Leu Lys Gly Val Leu Ser Gln Ile Ser
   4325 4330 4335
   Gln Leu Leu Ala Ile Glu Ile Ser Lys Val His Arg Met Glu Glu Tyr
   4340 4345 4350
   Ser Leu Ser Ser Asp Leu Ser Leu Gly Tyr Ala Leu Arg Gln Tyr Val
   4355 4360 4365
   Glu Leu Leu Trp Leu Leu Leu Glu Cys Pro Asn Ile Arg Arg Thr Tyr
   4370 4375 4380
   Lys Thr Arg Met Leu Gly Pro Val Leu Glu Ser Tyr Leu Ala Leu Arg
   4385 4390 4395 4400
   Ser Leu Val Val Gln Arg Thr Arg Leu Ile Asp Asp Ala Gln Glu Lys
   4405 4410 4415
   Leu Leu Glu Met Leu Glu Asp Met Thr Ser Gly Thr Glu Glu Glu Thr
   4420 4425 4430
   Arg Ala Phe Met Glu Ile Leu Ile Asp Thr Val Glu Lys Thr Arg Met
   4435 4440 4445
   Asn Asp Ile Lys Thr Pro Val Phe Val Phe Glu Arg Leu Tyr Ser Ile
   4450 4455 4460
   Ile His Pro Glu Glu His Asp Glu Ser Glu Phe Tyr Met Thr Leu Glu
   4465 4470 4475 4480
   Lys Asp Pro Gln Gln Glu Asp Phe Leu Gln Gly Arg Met Leu Gly Asn
   4485 4490 4495
   Pro Tyr Pro Ser Ser Glu Met Gly Leu Gly Pro Leu Met Arg Asp Val
   4500 4505 4510
   Lys Asn Lys Ile Cys Thr Asp Cys Glu Leu Ile Ala Leu Leu Glu Asp
   4515 4520 4525
   Asp Asn Gly Met Glu Leu Leu Val Asn Asn Lys Ile Ile Ser Leu Asp
   4530 4535 4540
   Leu Pro Val Lys Asp Val Tyr Lys Lys Val Trp Leu Ala Glu Gly Gly
   4545 4550 4555 4560
   Asp Arg Asp Ala Met Arg Ile Val Tyr Arg Met Arg Gly Leu Leu Gly
   4565 4570 4575
   Asp Ala Thr Glu Glu Phe Val Glu Thr Leu Asn Asn Lys Ser Gln Glu
   4580 4585 4590
   Gln Val Asp Thr Glu Gln Leu Tyr Arg Met Ala Asn Val Leu Ala Asp
   4595 4600 4605
   Cys Asn Gly Leu Arg Val Met Leu Glu Arg Ile Gly Ser Leu Gln Arg
   4610 4615 4620
   Ile Ser Arg Asn Arg Glu Leu Ile Gln Val Leu Leu Lys Leu Phe Leu
   4625 4630 4635 4640
   Ile Cys Val Lys Val Arg Arg Cys Gln Glu Val Leu Cys Gln Pro Glu
   4645 4650 4655
   Ile Gly Ala Ile Asn Thr Leu Leu Lys Val Leu Gln Met Cys Leu Gln
   4660 4665 4670
   Ser Glu Asn Asp Ser Ile Gln Ser Ala Val Thr Glu Gln Leu Leu Glu
   4675 4680 4685
   Ile Met Glu Thr Ile Leu Ser Lys Ala Ala Ser Asp Thr Leu Asp Ser
   4690 4695 4700
   Phe Leu Gln Phe Ser Leu Thr Phe Gly Gly Pro Glu Tyr Val Ser Ala
   4705 4710 4715 4720
   Leu Ile Ser Cys Thr Asp Cys Pro Asn Val Arg Asn Asn Pro Ser Val
   4725 4730 4735
   Leu Arg His Leu Ile Arg Val Leu Ala Ala Leu Val Tyr Gly Asn Glu
   4740 4745 4750
   Val Lys Met Ala Leu Leu Cys Glu His Phe Lys Asp Thr Leu Asn Phe
   4755 4760 4765
   Asn Arg Phe Asp Asn Glu Arg Thr Pro Glu Glu Glu Phe Lys Leu Glu
   4770 4775 4780
   Leu Phe Cys Val Leu Thr Asn Gln Ile Glu His Asn Cys Ile Gly Gly
   4785 4790 4795 4800
   Thr Leu Lys Asp Tyr Ile Val Ser Leu Gly Ile Val Glu Arg Ser Leu
   4805 4810 4815
   Ala Tyr Ile Thr Glu His Ala Pro Cys Val Lys Pro Thr Leu Leu Arg
   4820 4825 4830
   Thr Asp Ser Asp Glu Leu Lys Glu Phe Ile Ser Arg Pro Ser Leu Lys
   4835 4840 4845
   Tyr Ile Leu Arg Phe Leu Thr Gly Leu Ser Asn His His Glu Ala Thr
   4850 4855 4860
   Gln Val Ala Ile Ser Lys Asp Ile Ile Pro Ile Ile His Arg Leu Glu
   4865 4870 4875 4880
   Gln Val Ser Ser Asp Glu His Val Gly Ser Leu Ala Glu Asn Leu Leu
   4885 4890 4895
   Glu Ala Leu Ser Thr Asp Ser Ala Thr Ala Ala Arg Val Gln Gln Val
   4900 4905 4910
   Arg Asp Phe Thr Arg Ala Glu Lys Lys Arg Leu Ala Met Ala Thr Arg
   4915 4920 4925
   Glu Lys Gln Leu Asp Ala Leu Gly Met Arg Thr Asn Glu Lys Gly Gln
   4930 4935 4940
   Val Thr Ala Lys Gly Ser Ile Leu Gln Lys Ile Glu Lys Leu Arg Asp
   4945 4950 4955 4960
   Glu Thr Gly Leu Thr Cys Phe Ile Cys Arg Glu Gly Tyr Ala Cys Gln
   4965 4970 4975
   Pro Asp Lys Val Leu Gly Ile Tyr Thr Phe Thr Lys Arg Cys Asn Val
   4980 4985 4990
   Glu Glu Phe Glu Leu Lys Ser Arg Lys Thr Ile Gly Tyr Thr Thr Val
   4995 5000 5005
   Thr His Phe Asn Val Val His Val Asp Cys His Thr Ser Ala Ile Arg
   5010 5015 5020
   Leu Thr Arg Gly Arg Asp Glu Trp Glu Arg Ala Ser Leu Gln Asn Ala
   5025 5030 5035 5040
   Asn Thr Arg Cys Asn Gly Leu Leu Pro Leu Trp Gly Pro Ala Val Gly
   5045 5050 5055
   Glu Ala Ala Phe Ser Ala Cys Met Thr Arg His Ser Ser Tyr Met Gln
   5060 5065 5070
   Glu Ser Thr Gln Arg Cys Asp Ile Ser Tyr Thr Ser Ser Val His Asp
   5075 5080 5085
   Leu Lys Leu Leu Leu Val Arg Phe Ala Trp Glu Arg Ser Phe His Asp
   5090 5095 5100
   Asp Ala Gly Gly Gly Gly Pro Gln Ser Asn Met His Phe Val Pro Tyr
   5105 5110 5115 5120
   Leu Leu Phe Tyr Ser Val Tyr Leu Leu Leu Ser Ser Arg Ser Ala Ala
   5125 5130 5135
   Arg Asp Ser Lys Thr Leu Leu Thr Tyr Leu Gln Ala Pro Pro Ser Glu
   5140 5145 5150
   Lys Trp Leu Glu Cys Gly Tyr Glu Val Asp Gly Pro Leu Phe Met Ala
   5155 5160 5165
   Thr Ile Ser Leu Ser Leu His Ser Arg Glu Leu Trp Asn Lys His Lys
   5170 5175 5180
   Leu Ala His Leu Lys Arg Met Ile Ala Val Ala Gln Gly Arg His Val
   5185 5190 5195 5200
   Ser Pro Ala Val Leu Cys Lys Ala Leu Leu Ala Pro Ala Asp Arg Gln
   5205 5210 5215
   Val Lys Asp Tyr Thr Val Tyr Lys Pro Phe Leu Met Met Trp Ala Leu
   5220 5225 5230
   Val Asp Leu Ile Tyr Asp Asn Leu Phe Lys Thr Val Ser Thr Pro Lys
   5235 5240 5245
   Glu Glu Asp Trp Pro Ile Ser Leu Phe Asp Tyr Leu Arg Lys Asn Asp
   5250 5255 5260
   Glu Ala Leu Leu Lys Ser Thr Asp Ser Ile Leu Gln Thr Leu Thr Glu
   5265 5270 5275 5280
   Glu Phe Leu Pro Cys Thr Ser Phe Val Glu Phe Cys Asp Val Ala Gly
   5285 5290 5295
   Leu Leu His Leu Ile Glu His Pro Asp Asn Phe Ile Glu Glu Ile Leu
   5300 5305 5310
   Ala Ala Leu Pro Ser Thr Ser Ser Ser Asn
   5315 5320
<210> 48
   <211> 29
   <212> DNA
   <213> Rattus sp.
<400> 48
   cattatgctg agtgatatct ttttttttvv 30
<210> 49
   <211> 25
   <212> DNA
   <213> Rattus sp.
<400> 49
   attaaccctc actaaatgct gggga 25
<210> 50
   <211> 12
   <212> DNA
   <213> Rattus sp.
<400> 50
   gatctgcggt ga 12
<210> 51
   <211> 24
   <212> DNA
   <213> Rattus sp.
<400> 51
   agcactctcc agcctctcac cgca 24
<210> 52
   <211> 12
   <212> DNA
   <213> Rattus sp.
<400> 52
   gatctgttca tg 12
<210> 53
   <211> 24
   <212> DNA
   <213> Rattus sp.
<400> 53
   accgacgtcg actatccatg aaca 24
<210> 54
   <211> 12
   <212> DNA
   <213> Rattus sp.
<400> 54
   gatcttccct cg 12
<210> 55
   <211> 24
   <212> DNA
   <213> Rattus sp.
<400> 55
   aggcaactgt gctatccgag ggaa 24
   1
   1

### SEQUENCE LISTING

<110> Quark Biotech, Inc
<120> MECHANICAL STRESS INDUCED GENES, EXPRESSION PRODUCTS THEREFROM, AND USES THEREOF
<130> P10270EP
<140> 99923223.4
   <141> 1999-05-14
   <150> 60/085,673
   <151> 1998-05-15
<160> 55
<170> PatentIn Ver. 2.1
<210> 1
   <211> 241
   <212> DNA
   <213> Rattus sp.
<220>
   <221> gene
   <222> (1)..(241)
   <223> alpha 2u globulin-related protein
<400> 1
   tactgtttgt tttcagaggt cttctggaaa aataccatgg caaactggtc gtagtcagtg 60
   tcggccactt gcacatcgta gctctgtatc tgagggtagc tgtgaatatt ccccagggtg 120
   aactggccag gcctggagct tggaacgaat gttctgatcc agtagcgaca gccctggccc 180
   ctgacgagga tggaagtgac gttgtagcta ttgtcttcct gtagctcata gatggtgctg 240
   t                                                                 241
<210> 2
   <211> 192
   <212> DNA
   <213> Rattus sp.
<220>
   <221> gene
   <222> (1)..(192)
   <223> gamma-actin, cytoplasmic
<400> 2
   acatggctgg ggtattgaag gtttcaaaca ttatctgcgt catcttctct ctgttagctt 60
   tggggttcag gggggcctcg gtcagaagca ccgggtgctc ctcaggggcc acacgcagct 120
   cgttgtagaa ggtgtggtgc cagatcttct ccatgtcgtc ccagttggtg acaatgccgt 180
   gctcaatagg gt                                                     192
<210> 3
   <211> 288
   <212> DNA
   <213> Rattus sp.
<220>
   <221> gene
   <222> (1)..(288)
   <223> alpha-actin, cardiac
<400> 3
   acaggtcttt gcggatatcg atgtcacact tcatgatgct gttataagta gtttcgtgaa 60
   tgccggcaga ctccatacca atgaaagagg gctggaagag agtctcagga cagcggaaac 120
   gctcgttgcc aatggtgatg acctggccat cgggaagttc gtagctcttc tccagagagg 180
   aggaagatgc agcggtggcc atctcattct caaaatccag ggcgacataa cacagttttt 240
   ctttgatgtc tcggacaatt tcacgttcag cggtggtgac aaaggagt              288
<210> 4
   <211> 388
   <212> DNA
   <213> Rattus sp.
<220>
   <221> gene
   <222> (1)..(388)
   <223> alkaline phosphodiesterase RB13-6 Ag
<220>
   <221> misc_feature
   <222> (4)..(4)
   <223> "n" can be a or g or c or t
<220>
   <221> misc_feature
   <222> (151)..(151)
   <223> "n" can be a or g or c or t
<220>
   <221> misc_feature
   <222> (250)..(251)
   <223> "n" can be a or g or c or t
<220>
   <221> misc_feature
   <222> (255)..(255)
   <223> "n" can be a or g or c or t
<220>
   <221> misc_feature
   <222> (277)..(277)
   <223> "n" can be a or g or c or t
<220>
   <221> misc_feature
   <222> (284)..(284)
   <223> "n" can be a or g or c or t
<220>
   <221> misc_feature
   <222> (309)..(309)
   <223> "n" can be a or g or c or t
<220>
   <221> misc_feature
   <222> (317)..(317)
   <223> "n" can be a or g or c or t
<220>
   <221> misc_feature
   <222> (323)..(323)
   <223> "n" can be a or g or c or t
<220>
   <221> misc_feature
   <222> (330)..(330)
   <223> "n" can be a or g or c or t
<220>
   <221> misc_feature
   <222> (361)..(361)
   <223> "n" can be a or g or c or t
<220>
   <221> misc_feature
   <222> (384)..(384)
   <223> "n" can be a or g or c or t
<220>
   <221> misc_feature
   <222> (388)..(388)
   <223> "n" can be a or g or c or t
<400> 4
   accnaggacc tgcccctcga atcaggactc gtaatatacc tcaggacttc tttaccttta 60
   attccgaaga gattgtcaga gacctcagct gtcgaaaatc cgatcaacac ttcaaaccct 120
   acttgactcc ggatttgccg aaacgactgc nctatgccaa gaacgtcagg attgacaaag 180
   ttcatctaat ggtggatcgg ctatggctgg cttacaggaa ctaagggagt tccaactgcg 240
   aatgcggaan ncacngttac aactatgaat ttaaaanttt ggangcaatc tttttggcac 300
   atggacccng ctttaangag aanactgttn tccaaccatt tgaaaacatc gaagtctata 360
   ntctactgtg tgatcttctg cacntccn                                   388
<210> 5
   <211> 403
   <212> DNA
   <213> Rattus sp.
<220>
   <221> gene
   <222> (1)..(903)
   <223> am2 receptor/LDL receptor related/LPR/A-2-macroglobulin receptor
<400> 5
   acccttctgc ctgtccctgt tgaagacctt gatgtcttta agctgcacgc cnatgcccgt 60
   tctcagaggc acggagtctg tggcattgcc tttgcatcca cgcttgatgg agccgttggc 120
   gtgagttctg tcactccagt agatgaaatc ctcaaacaca gacacagaga acatgtccat 180
   gttgttactg gacaggacca cctcacggtt ctcacccgtc tccagatcga tacgttcgat 240
   cttgtccatc cgggcatcac accagtagag cttgccgccc tgatagtcta ctgagatgcc 300
   attgggccag ctgatgctga cattaaccag caccactctc tctgtgccgt caaggcgaga 360
   ccgctcaatc cgtgggtagt gaccccactc agtccagaac aag                  403
<210> 6
   <211> 520
   <212> DNA
   <213> Rattus sp.
<220>
   <221> gene
   <222> (1)..(520)
   <223> aquaporin (AQA1), Channel integral membrane protein
<400> 6
   accatggcac caactggctg tttccctcta gtctcccttg cagtgcagat gtgacgtgtg 60
   tgtttattaa agagcactgg gctattgcag cgtcatgtct gaggaaagaa gcagctagac 120
   atgcaacaga ccccagacag atgcccatgc cgggcacaca ggggtttgga tgcccctagt 180
   tcatcatgat caaggaggcc actcttgtcc tggcttatga gctgctggct ggatgaattt 240
   gaccagagcc tggacaatct gaaggggctc actatgtgac tccaggcaca gtctccttat 300
   tgcaaggacc tgatgctgtg gcttctgcta tagcccaaga acatctcaga gtgcattcag 360
   ctcagggctt gcatttagct ctctgggtta ttctattcaa tcccaccaag ccagagcagc 420
   tctaccactg tgccgttaac catgtcgtga accgagacca cattcttcag gtgcttagaa 480
   gcagcagaat aatcaggagg ccattgacca ctggcatagt                       520
<210> 7
   <211> 492
   <212> DNA
   <213> Rattus sp.
<220>
   <221> gene
   <222> (1)..(492)
   <223> collagen type XII a-1
<400> 7
   acttaaatgt gacagatctg aaaacttacc aggttggatg ggacaccttc tgtgtcaaat 60
   ggtcacctca ccgggcagcg acctcttaca ggctaaagct gagtcctgcc gatggaacca 120
   ggggacaaga aataacagtg agaggatcag agaccagtca ctgcttcact ggcctctccc 180
   cggaggctga gtatggcgtt actgtttttg tgcagacacc aaatctcgag gggccgggtg 240
   tccccatcaa agaacagacg actgtgaaac caacagaggc tcccacagaa ccgcccacgc 300
   cttcacctcc tcccactatc ccacctgccc gtgatgtatg caaaggggcc aaggcagata 360
   ttgtgttcct gacggatgcc tcttggagta ttggagatga caattttaac aaagttgtaa 420
   aatttatttt taatactgtg ggggcctttg atgaaatcag ccctgctggg attcaggttt 480
   cttttgtgca gt                                                     492
<210> 8
   <211> 756
   <212> DNA
   <213> Rattus sp.
<220>
   <221> gene
   <222> (1)..(756)
   <223> complement component C3, prepo complement C3, rat pcRC201 mRNA prepo complement C3 (X52477)
<220>
   <221> misc_feature
   <222> (276)..(276)
   <223> "n" can be a or g or c or t
<220>
   <221> misc_feature
   <222> (290)..(290)
   <223> "n" can be a or g or c or t
<220>
   <221> misc_feature
   <222> (389)..(384)
   <223> "n" can be a or g or c or t
<220>
   <221> misc_feature
   <222> (408)..(408)
   <223> "n" can be a or g or c or t
<220>
   <221> misc_feature
   <222> (492)..(492)
   <223> "n" can be a or g or c or t
<220>
   <221> misc_feature
   <222> (505)..(505)
   <223> "n" can be a or g or c or t
<220>
   <221> misc_feature
   <222> (509)..(509)
   <223> "n" can be a or g or c or t
<220>
   <221> misc_feature
   <222> (536)..(536)
   <223> "n" can be a or g or c or t
<220>
   <221> misc_feature
   <222> (569)..(569)
   <223> "n" can be a or g or c or t
<220>
   <221> misc_feature
   <222> (582)..(582)
   <223> "n" can be a or g or c or t
<220>
   <221> misc_feature
   <222> (602)..(602)
   <223> "n" can be a or g or c or t
<220>
   <221> misc_feature
   <222> (625)..(625)
   <223> "n" can be a or g or c or t
<220>
   <221> misc_feature
   <222> (628)..(628)
   <223> "n" can be a or g or c or t
<220>
   <221> misc_feature
   <222> (632)..(632)
   <223> "n" can be a or g or c or t
<220>
   <221> misc_feature
   <222> (648)..(648)
   <223> "n" can be a or g or c or t
<220>
   <221> misc_feature
   <222> (668)..(668)
   <223> "n" can be a or g or c or t
<220>
   <221> misc_feature
   <222> (692)..(692)
   <223> "n" can be a or g or c or t
<220>
   <221> misc_feature
   <222> (697)..(697)
   <223> "n" can be a or g or c or t
<220>
   <221> misc_feature
   <222> (702)..(702)
   <223> "n" can be a or g or c or t
<220>
   <221> misc_feature
   <222> (714)..(714)
   <223> "n" can be a or g or c or t
<220>
   <221> misc_feature
   <222> (729)..(729)
   <223> "n" can be a or g or c or t
<220>
   <221> misc_feature
   <222> (737)..(737)
   <223> "n" can be a or g or c or t
<220>
   <221> misc_feature
   <222> (742)..(742)
   <223> "n" can be a or g or c or t
<400> 8
   acttgggaaa cttggatgct actatgtcca tcctggacat ctccatgatg actggcttta 60
   ttccagacac aaacgacctg gaactgctga gctctggagt agacagatac atttccaagt 120
   atgagatgga caaagccttc tccaacaaga acaccctcat catctaccta gaaaagatct 180
   cacactccga agaagactgc ctgtccttca aagtccacca gttctttaac gtgggactta 240
   tccagccggg gtcggtcaag gtctactcct actacnatct aaaggagtcn tgcacccggt 300
   tctatcatcc ggagaaggac gatggaatgc tgagcaagct gtgccacaat gaaatgtgcc 360
   gctgtgccga ggagaactgc ttcntgcatc agtcacagga tcaggtcngc ctgaatgaac 420
   gactacacaa ggcttgtgag cctggagtgg actacgtgta cctcggccgc gaccacgctg 480
   ctgagccttg anccggggaa acaanaacng cccaccccat ctggggaggc ctttcnaagt 540
   ggtgctggga caagggatcc cctgctctng tgctcccctc cngacttatt gatttctttc 600
   cnttaacctg tcctgggcac gcccnttncc tnagggggac tagttcantg tttctgggga 660
   aaaatcancc ccctcactgg gttttgaccc tntggcnaga anaacacccc cccncccttt 720
   atgcccccng gtgttgncct tncctcgtta tgggcc 756
<210> 9
   <211> 340
   <212> DNA
   <213> Rattus sp.
<220>
   <221> gene
   <222> (1)..(340)
   <223> cytochrome oxidase C
<400> 9
   acgatccctg ttaggccccc tactgtgaat aagaagataa accctaaggc tcataatatg 60
   gcgggggatc atttgatatt ccctccatgt agtgtagcga gtcagctgaa tacttttacg 120
   cctgtaggaa ttgcgataat tatagtggca gatgtaaagt aggctcgggt gtctacatct 180
   aggcctactg tgaatatgtg atgtgctcat acaataaacc ctaggaagcc aatagatatt 240
   atggctcata ccatacctat atatccgaag ggttcttttt ttccagagta ataggtaact 300
   acatgtgaaa taattccaaa ccctggaaga attaagatgt                       340
<210> 10
   <211> 201
   <212> DNA
   <213> Rattus sp.
<220>
   <221> gene
   <222> (1)..(201)
   <223> DEST (274)
<400> 10
   accgtgtgtc cactttaatg gaagacagct tgatataaca aaacggcact tcagggttat 60
   tacacaccag gcagggatca ctctctaggt agtagccatc aaactccact aagccagaca 120
   aggtattgta aatgttggag ttgggatgat tggtgagaat gtggttctga gtcctcagaa 180
   tctccacggc cttctgtgag t                                           201
<210> 11
   <211> 755
   <212> DNA
   <213> Rattus sp.
<220>
   <221> gene
   <222> (1)..(755)
   <223> DEST (2-144)
<220>
   <221> misc_feature
   <222> (520)..(520)
   <223> "n" can be a or g or c or t
<220>
   <221> misc_feature
   <222> (526)..(526)
   <223> "n" can be a or g or c or t
<220>
   <221> misc_feature
   <222> (601)..(601)
   <223> "n" can be a or g or c or t
<220>
   <221> misc_feature
   <222> (603)..(603)
   <223> "n" can be a or g or c or t
<220>
   <221> misc_feature
   <222> (665)..(665)
   <223> "n" can be a or g or c or t
<220>
   <221> misc_feature
   <222> (694)..(694)
   <223> "n" can be a or g or c or t
<220>
   <221> misc_feature
   <222> (708)..(709)
   <223> "n" can be a or g or c or t
<220>
   <221> misc_feature
   <222> (720)..(720)
   <223> "n" can be a or g or c or t
<400> 11
   acacactgcc cactctcctc ttggcagcag ccctgccact ttctttacac tgccagccac 60
   atgtggacat gggctactca tgactgtgga attctcttgg tgccacctta ttcctcagtg 120
   gtgctggtat gcccaatgga tagcatcaag atatgccaat gtgtttttgt tgggccagtt 180
   ggttccttgt ggggatctga gagtggttga ctccagtcca gggtctttca gtttctattc 240
   actctgtctg agcaaacagc ttcaatgccc ggatgagtgg aaactatcca tgctcagtaa 300
   tcttctgcga catcagggtt tctctgtgtt tcatgaatgg aattggttcc ctttcctcca 360
   caaccacaaa ttcattcttt caaagaagac ttgctttggt cccctctgtc cagtggtgtc 420
   aagggagcct tagtcccaga gtccactctc caggttgagc caactcccag agggtctttc 480
   ccactgaatg ggtcttccag ctgctgggaa gtggctgtgn gaaccncgcc tcttaagaca 540
   cagtggcttc agttgccaat gttcagaggg aagacctctt ctggggagat nantggggaa 600
   gggaagggat aaaaatgacc tcctgttttt cttggtgtaa gtttcattga atttttaatg 660
   gcganggtct cacactggtt aaccacttaa aganaccaat cactttcnng gttggccatn 720
   aaatgttgaa tgtctttggg ctcagtttca tttca                            755
<210> 12
   <211> 837
   <212> DNA
   <213> Rattus sp.
<220>
   <221> gene
   <222> (1)..(837)
   <223> DEST (608) homology NCAM-1
<220>
   <221> misc_feature
   <222> (626)..(626)
   <223> "n" can be a or g or c or t
<220>
   <221> misc_feature
   <222> (735)..(735)
   <223> "n" can be a or g or c or t
<220>
   <221> misc_feature
   <222> (741)..(741)
   <223> "n" can be a or g or c or t
<220>
   <221> misc_feature
   <222> (751)..(751)
   <223> "n" can be a or g or c or t
<220>
   <221> misc_feature
   <222> (774)..(774)
   <223> "n" can be a or g or c or t
<220>
   <221> misc_feature
   <222> (805)..(805)
   <223> "n" can be a or g or c or t
<220>
   <221> misc_feature
   <222> (817)..(817)
   <223> "n" can be a or g or c or t
<220>
   <221> misc_feature
   <222> (820)..(820)
   <223> "n" can be a or g or c or t
<400> 12
   acctcttggt cctatagcca ctgtcatcag cttgggctac attgttgagc actgtcccat 60
   caggcaaact ccaggatacc tcaggcacag gggagccaga ggccttgcag tcaacttgga 120
   aatctttccc atggagcact tgcttcttaa aatactgctt ctgttcaatt ttggcaggtg 180
   tcaatctcag gcggacatgc atcaggacta ggtcatctcc cattttgttt cttgccacac 240
   ataagtagtc accagcgtct ttttccgtca ctgacccaac caccaaggat ccatttgggt 300
   agacgtggat tcggctgccc attctgtgcc actggtcgat gacagccttg gatggcagcc 360
   tccagattat tctaggcttt ggatccccag tagctgagca gttcagtagt aatttctcac 420
   ccaaattcac ctcagtccat gtctgagagg cagtttctat cctgggggtt gtctctccct 480
   cttccacagt aagaacacta cccttctctc tgagcctgag gagctggtgg caatgcactc 540
   ataagtgccc ctcactgaag gacgatgctt cttatataca gagttccatt tggatacaag 600
   aaaaatctgg gaatgagtca actgcnatgg ttttagttca gtcccatcat aaaaggaccc 660
   agtgaacact agggtggggg aattcctttt gcagtgcagg gcatttcaaa cttccaccta 720
   aaaaccccaa cgatnggctt nctcttttgg ntcctataat gaacaggggg aacngttgaa 780
   ttaacttttt tctttaaccc aacanttgaa tcctggncca naatgggttg cctggcc    837
<210> 13
   <211> 277
   <212> DNA
   <213> Rattus sp.
<220>
   <221> gene
   <222> (1)..(277)
   <223> DEST (2-197) similarity to erg3 (Y10624)
<400> 13
   acctgttccc caccagcgcc gaagactgca aattcacagg caatttcttc tacaactaca 60
   tgatggggat tgagttcaac ccccgcatcg gaaagtggtt tgacttcaag ctgttcttca 120
   atgggcgccc ggggattgtg gcctggactc ttatcaacct gtcctttgct gccaagcagc 180
   aggagcttta tggccatgtg accaactcca tgattttggt caatgtctta caggccatct 240
   atgtgttaga cttcttctgg aacgaaacct ggtatca                          277
<210> 14
   <211> 589
   <212> DNA
   <213> Rattus sp.
<220>
   <221> gene
   <222> (1)..(589)
   <223> DEST KIAA0183
<400> 14
   acatcagtcc cttcttgtgt ctgtgctcag ccactctcag cacctcaggt gcaacggggg 60
   gcaagggagg tgtggtgata tccatgtggt tcctggtggg cattgacagg aggcacggga 120
   ttggctgaac agtgcccatg tggctccctt tgccttcagc tagctggctt cccgaagagg 180
   cagtggatga accttctacc tttacttggt ctcctgggtc tccccgtgcc agtggctctg 240
   catggcttcc tgtcttctcc cagccaatct tgttcccgct gatgtggttg gttgcccctc 300
   cactgccctc atcgttgtct gatgaggacg aggtagacga gccaggggaa gtaggcggaa 360
   tccacgtgat tggggctgcc gctctgggcc aggttgatgg gcgaagaaac gctcatacag 420
   cggtgtgtgc ttcccctcgt gaggaaatgt tgctgtagct gttctgctct gtcaggttct 480
   tcccgctaga gtccagggcg aaggcaggct cagggaagct gtatgggtat ggaacctggc 540
   ctggggtgcc cctgggctgg gcacctgttg gaccgcgggc tttgcttgc             589
<210> 15
   <211> 768
   <212> DNA
   <213> Rattus sp.
<220>
   <221> gene
   <222> (1)..(768)
   <223> endothelin converting enzyme
<220>
   <221> misc_feature
   <222> (501)..(501)
   <223> "n" can be a or g or c or t
<220>
   <221> misc_feature
   <222> (558)..(558)
   <223> "n" can be a or g or c or t
<220>
   <221> misc_feature
   <222> (576)..(576)
   <223> "n" can be a or g or c or t
<220>
   <221> misc_feature
   <222> (588)..(588)
   <223> "n" can be a or g or c or t
<220>
   <221> misc_feature
   <222> (605)..(605)
   <223> "n" can be a or g or c or t
<220>
   <221> misc_feature
   <222> (610)..(610)
   <223> "n" can be a or g or c or t
<220>
   <221> misc_feature
   <222> (634)..(634)
   <223> "n" can be a or g or c or t
<220>
   <221> misc_feature
   <222> (653)..(653)
   <223> "n" can be a or g or c or t
<220>
   <221> misc_feature
   <222> (656)..(656)
   <223> "n" can be a or g or c or t
<220>
   <221> misc_feature
   <222> (659)..(659)
   <223> "n" can be a or g or c or t
<220>
   <221> misc_feature
   <222> (698)..(698)
   <223> "n" can be a or g or c or t
<220>
   <221> misc_feature
   <222> (703)..(703)
   <223> "n" can be a or g or c or t
<220>
   <221> misc_feature
   <222> (738)..(738)
   <223> "n" can be a or g or c or t
<400> 15
   acgggacaaa gaagacctgt cttccccgct ggaagttttg cgtgagtgac acagaaaaca 60
   acctgggctt tgccctgggc cccatgtttg tgaaagcaac ctttgcggag gacagcaaga 120
   acatagccag cgagatcatc ctggagatca agaaggcatt cgaggagagc ctgagcaccc 180
   tgaaatggat ggatgaagat actcggaggt cagccaagga gaaggcggac gccatctaca 240
   acatgatagg ctaccccaac ttcatcatgg accccaagga gctggacaaa gtgttcaatg 300
   actacacagc agttcccgat ctctactttg agaacgccat gcgatttttc aacttctcat 360
   ggagggtcac agccgaccag ctcaggaaag cccccaacag agatcagtgg agtatgaccc 420
   cgcccatggt gaacgcctac tactcgccca ccaagaacga gattgtgttt ccagctggaa 480
   tcctgcaggc gctattttat ncccgctctt cgcccaacgc cttgaacttt ggtggtatcc 540
   gggtctttgt tgggcacnaa ctgactcctg ctttcnacga tcagggcngg agtttgacaa 600
   ggatnggaan ctcccggcct ggttgggaaa aacncctcgg tggaagcttc canccncana 660
   accaatgctt ggtttcaacc ttttcaaaac ctccaacncc aanggggggg cccggttccc 720
   accttttgtt ccccttantt gaaggggtta atttccaacc ttggggtt           768
<210> 16
   <211> 341
   <212> DNA
   <213> Rattus sp.
<220>
   <221> gene
   <222> (1)..(341)
   <223> erg25 methyl sterol (U60205) filamin (or region between filamin & GPI)
<400> 16
   actgaggcgg gccagggtag atgtcagcat tggtatcaag tgtgcccctg gagtactggg 60
   ccccactgag gctgatattg actttgatat catccgtaat gacaatgaca ccttcactgt 120
   gaaatacaca ccctgtgggg ctggcagcta taccatcatg gttctttttg ctgaccaggc 180
   cacacccacc agccccatca cactcaaatt ggagccttct catgatgcca gtaaagtgaa 240
   tgctgagggt cctgtcctaa atcgcactgg tgttgagctt ggcaaaccca cccatttcac 300
   agtcaatgct aaaactgctg ggaaaggcaa gccggagatt c                    341
<210> 17
   <211> 382
   <212> DNA
   <213> Rattus sp.
<220>
   <221> gene
   <222> (1)..(382)
   <223> highly charged amino acid sequence (x59131)
<220>
   <221> misc_feature
   <222> (301)..(301)
   <223> "n" can be a or g or c or t
<220>
   <221> misc_feature
   <222> (307)..(307)
   <223> "n" can be a or g or c or t
<220>
   <221> misc_feature
   <222> (363)..(363)
   <223> "n" can be a or g or c or t
<400> 17
   actaggatag agcggaggcc taggagagga gaagccaccc acaaggctcc ctgtccacag 60
   tgcacagtca gttgttgaga tttccactgt ctgcagggtc ccttgtctca aatacctgtt 120
   aataatattt acattgaaat aataggtccc cgaagccact tttgatattc taggagtccg 180
   ggagtatttc aaaagctagg gccatgcgga gagggccttt gttcaggatt ttagtcttag 240
   gtcatcaggc ctccaaactt atcagccttg agcaactcaa acaagactcc tatccaccca 300
   ncctggncct cccccctccc cccacctttc taccgtctca ggcatgggac actgtggttc 360
   ctnaacctgt gactgatgcc cc                                         382
<210> 18
   <211> 459
   <212> DNA
   <213> Rattus sp.
<220>
   <221> gene
   <222> (1)..(459)
   <223> kidney aminopeptidase M/kidney Zn-peptidase
<220>
   <221> misc_feature
   <222> (158)..(158)
   <223> "n" can be a or g or c or t
<220>
   <221> misc_feature
   <222> (374)..(375)
   <223> "n" can be a or g or c or t
<220>
   <221> misc_feature
   <222> (378)..(378)
   <223> "n" can be a or g or c or t
<220>
   <221> misc_feature
   <222> (408)..(408)
   <223> "n" can be a or g or c or t
<220>
   <221> misc_feature
   <222> (411)..(411)
   <223> "n" can be a or g or c or t
<220>
   <221> misc_feature
   <222> (447)..(447)
   <223> "n" can be a or g or c or t
<400> 18
   acacgtagtc cactccaggc tcacaagcct tgtctagtcg ttcattcagg ctgacctgat 60
   cctgtgactg atgcatgaag cagttctcct cggcacagcg gcacatttca ttgtggcaca 120
   gcttgctcag cattccatcg tccttctccg gatgatanaa ccgggtgcat gactcctcta 180
   gattgtagta ggagtagacc ttgaccgacc ccggctggat aagtcccacg ttaaagaact 240
   ggtggacttt gaaggacagg cagtcttctt cggagtgtga gatcttttct aggtagatga 300
   tgagggtgtt cttgttggag aaggctttgt ccatctcata cttggaaatg tatctgtcta 360
   ctccagagct cagnngtncc aggtcgtttg tgtctggaat aaagccantc ntcatggaga 420
   tgtccaggat ggacatagta gcatccncgt ctcccaagt                        459
<210> 19
   <211> 479
   <212> DNA
   <213> Rattus sp.
<220>
   <221> gene
   <222> (1)..(479)
   <223> myosin heavy chain A
<220>
   <221> misc_feature
   <222> (17)..(17)
   <223> "n" can be a or g or c or t
<220>
   <221> misc_feature
   <222> (58)..(58)
   <223> "n" can be a or g or c or t
<220>
   <221> misc_feature
   <222> (62)..(62)
   <223> "n" can be a or g or c or t
<220>
   <221> misc_feature
   <222> (67)..(67)
   <223> "n" can be a or g or c or t
<220>
   <221> misc_feature
   <222> (70)..(70)
   <223> "n" can be a or g or c or t
<220>
   <221> misc_feature
   <222> (126)..(126)
   <223> "n" can be a or g or c or t
<220>
   <221> misc_feature
   <222> (131)..(131)
   <223> "n" can be a or g or c or t
<220>
   <221> misc_feature
   <222> (135)..(136)
   <223> "n" can be a or g or c or t
<220>
   <221> misc_feature
   <222> (142)..(142)
   <223> "n" can be a or g or c or t
<220>
   <221> misc_feature
   <222> (145)..(145)
   <223> "n" can be a or g or c or t
<220>
   <221> misc_feature
   <222> (166)..(167)
   <223> "n" can be a or g or c or t
<220>
   <221> misc_feature
   <222> (182)..(182)
   <223> "n" can be a or g or c or t
<220>
   <221> misc_feature
   <222> (209) .. (209)
   <223> "n" can be a or g or c or t
<220>
   <221> misc_feature
   <222> (223)..(223)
   <223> "n" can be a or g or c or t
<220>
   <221> misc_feature
   <222> (225)..(225)
   <223> "n" can be a or g or c or t
<220>
   <221> misc_feature
   <222> (254)..(254)
   <223> "n" can be a or g or c or t
<220>
   <221> misc_feature
   <222> (269)..(269)
   <223> "n" can be a or g or c or t
<220>
   <221> misc_feature
   <222> (275)..(275)
   <223> "n" can be a or g or c or t
<220>
   <221> misc_feature
   <222> (279)..(279)
   <223> "n" can be a or g or c or t
<220>
   <221> misc_feature
   <222> (289)..(289)
   <223> "n" can be a or g or c or t
<220>
   <221> misc_feature
   <222> (291)..(291)
   <223> "n" can be a or g or c or t
<220>
   <221> misc_feature
   <222> (293)..(293)
   <223> "n" can be a or g or c or t
<220>
   <221> misc_feature
   <222> (295)..(295)
   <223> "n" can be a or g or c or t
<220>
   <221> misc_feature
   <222> (300)..(300)
   <223> "n" can be a or g or c or t
<220>
   <221> misc_feature
   <222> (315)..(315)
   <223> "n" can be a or g or c or t
<220>
   <221> misc_feature
   <222> (335)..(335)
   <223> "n" can be a or g or c or t
<220>
   <221> misc_feature
   <222> (337)..(337)
   <223> "n" can be a or g or c or t
<220>
   <221> misc_feature
   <222> (340)..(340)
   <223> "n" can be a or g or c or t
<220>
   <221> misc_feature
   <222> (345)..(345)
   <223> "n" can be a or g or c or t
<220>
   <221> misc_feature
   <222> (358)..(358)
   <223> "n" can be a or g or c or t
<220>
   <221> misc_feature
   <222> (364)..(364)
   <223> "n" can be a or g or c or t
<220>
   <221> misc_feature
   <222> (367)..(367)
   <223> "n" can be a or g or c or t
<220>
   <221> misc_feature
   <222> (407)..(407)
   <223> "n" can be a or g or c or t
<220>
   <221> misc_feature
   <222> (421)..(421)
   <223> "n" can be a or g or c or t
<220>
   <221> misc_feature
   <222> (423)..(423)
   <223> "n" can be a or g or c or t
<220>
   <221> misc_feature
   <222> (429) .. (430)
   <223> "n" can be a or g or c or t
<220>
   <221> misc_feature
   <222> (437)..(437)
   <223> "n" can be a or g or c or t
<220>
   <221> misc_feature
   <222> (440)..(440)
   <223> "n" can be a or g or c or t
<220>
   <221> misc_feature
   <222> (452)..(452)
   <223> "n" can be a or g or c or t
<220>
   <221> misc_feature
   <222> (467)..(467)
   <223> "n" can be a or g or c or t
<400> 19
   actcctcctg ctccagnatg aacatggtgt ggttgaacag ctgctgcagc ttctcgtngg 60
   tntactngat ntcaaagttg ctcgaatgag ttcacatcaa agatctcgaa gccagcggat 120
   gtccangatc ncganntatg angcnccctg cctcttggtc ttgtcnntaa ctttgttatt 180
   gngaaccacc agccagggga atatccgcnc gtaggtaccc ttngncagag cctccatggt 240
   aaaatcagcc tgcnctttct ctgtggccnt ctgcncatnc tcgctgccnt ncntnatgcn 300
   cggggtcaag ggatncctct ggttgacctc ggtcncnttn catcncccac gcaagtgngg 360
   acanctnttg aactcctgtg ttatctggca tggacgactt gttcagntgc tcccctccct 420
   ntncttgann gacccangtn cccctctgtc tnaatgtacc ccttganatg atctctcag 479
<210> 20
   <211> 539
   <212> DNA
   <213> Rattus sp.
<220>
   <221> gene
   <222> (1)..(539)
   <223> NOVEL PROTEIN AHNAK NUCLEOPROTEIN (M80899)
<220>
   <221> misc_feature
   <222> (127)..(127)
   <223> "n" can be a or g or c or t
<400> 20
   acatttttcc aaacatggtt ttcttgactt tgggagattt tacttcagct ttgagcgcag 60
   catcaggccc tgcaacgctg acatccacat caggaacttt gaggctggca tcaatctcac 120
   ctccagnaac attcaaatca aatccctgtt tcttggtctt gactttaggg ccactcatgt 180
   gaattttagg cattttaaac ttgcttttct tgcccttgcc accaatgttt atctcaggag 240
   tctcaacact cagttctgct tcagggacag tcacaccaac tgtgggggtc ttaatgtcag 300
   ctttggggct ttttgcacca aatccaaact tgggtttttt aaacttcgga actttaacat 360
   ctggcccttc aatattaata tctggactct ccatgtggac atctaagctt ggagtttcta 420
   aatcaacttt aggccccctg aggtcaactt cacctcctcc taacttaggc ccagaaattc 480
   caacatttgg tgtcttgagg tgcacattag catcaggccc acttatttta ggaacaggt 539
<210> 21
   <211> 349
   <212> DNA
   <213> Rattus sp.
<220>
   <221> gene
   <222> (1)..(349)
   <223> secretory protein containing thrombospondin motifs
<400> 21
   accagacctt gtgcagacct tccttgccca cgttggcagg tgggggattg gtcaccatgt 60
   tctaaaactt gtgggaaggg ttataagaag agaaccttga aatgtctgtc ccatgatggc 120
   ggtgtgttat caaatgagag ctgtgatcct ttgaataaac caaagcatta cattgacttt 180
   tgcatactga cacagtgcag ttaacaggtt ttgaggacaa ggtagcgtgg gagggctgat 240
   acactgaaag caagagtgct ggagggatcc tgtgaatcaa accagtaaac agtgaggtgt 300
   ggcaatgagg tgtgtatagg ggatacatta gcaaaagagg tagatccgt 349
<210> 22
   <211> 323
   <212> DNA
   <213> Rattus sp.
<220>
   <221> gene
   <222> (1)..(323)
   <223> steraoyl CoA desaturase (protein kinase)
<400> 22
   actgtgcagc gggctggctg cagaaactta agtcataaca ctctttgtcg ctgaggtctg 60
   aagctctctg cacgttctca tccctgggaa cgtgacccca gcatccgacg ccaagatgcc 120
   ggctcacata ctgcaagaga tctctggctc ttactcggcc accaccacaa tcacagcgcc 180
   accttctggg ggacagcaga atggaggaga gaagtttgaa aggaatcctc accactgggg 240
   agcagatgtt cgccctgaaa ttaaagatga cctatacgac cccagctacc aggatgagga 300
   ggggcccccg cccaagctgg agt                                         323
<210> 23
   <211> 551
   <212> DNA
   <213> Rattus sp.
<220>
   <221> gene
   <222> (1)..(551)
   <223> tenascin
<220>
   <221> misc_feature
   <222> (151)..(151)
   <223> "n" can be a or g or c or t
<220>
   <221> misc_feature
   <222> (158)..(158)
   <223> "n" can be a or g or c or t
<220>
   <221> misc_feature
   <222> (186)..(186)
   <223> "n" can be a or g or c or t
<220>
   <221> misc_feature
   <222> (217)..(217)
   <223> "n" can be a or g or c or t
<220>
   <221> misc_feature
   <222> (235)..(235)
   <223> "n" can be a or g or c or t
<220>
   <221> misc_feature
   <222> (238)..(238)
   <223> "n" can be a or g or c or t
<220>
   <221> misc_feature
   <222> (241)..(241)
   <223> "n" can be a or g or c or t
<220>
   <221> misc_feature
   <222> (390)..(390)
   <223> "n" can be a or g or c or t
<220>
   <221> misc_feature
   <222> (500)..(500)
   <223> "n" can be a or g or c or t
<220>
   <221> misc_feature
   <222> (535)..(535)
   <223> "n" can be a or g or c or t
<400> 23
   cattggagct ccaccgcggt ggcggccgct ctagaatagt ggatcccccg ggctgcagga 60
   attcgatact catgggcccc ttccagttgg aaaccattta aaaaccctga atggtgaata 120
   atgttcccat atctgcccat caaggttgaa nccatganag ttcttatacc agaaagctcc 180
   tttgtnggac agggcacagt tggtgatggc tgaatcngtg tccttgtcat aggtnganaa 240
   ngatctacca ttgtgatagt tcatggaatc acctaccgtt ccactgtatc cttctacctt 300
   cagcttgtag cgactcttgg catctccaac actgaacctg tcatacacag cataagctga 360
   ctccccatgg tcttgtaggt ccacccggan ctcatattgc ccttgggctg tgattttgct 420
   caggttatcc agtccaagcc agaattcttc tctgcggtct ccaaacccaa tggcattagc 480
   cttccagttt ccaaagaaan tcctcacgtc catttttgcg tctcaagaaa acgantcatc 540
   cacctccatc c                                                      551
<210> 24
   <211> 462
   <212> DNA
   <213> Rattus sp.
<220>
   <221> gene
   <222> (1)..(462)
   <223> thrombospondin 1
<400> 24
   accactgaag tccacggcat taaactcatc ataacctaca gcaagtccag ggtcacagtt 60
   tacagtctgg acaagttctt tgccctgatg gcggacaacc cagttagggt cattttggga 120
   ggttcctttg ggatctagag gaatcatctg gaatcggcgg aaatcggttt cactgatgtc 180
   aacattctca ggacagatgt catcaatgtc tggcacattg tcatggtcaa agtcgtcttt 240
   gcaggcatcg cctcggccat caccatcaga gtccttctgg tcaggattgg gcaccagcct 300
   gcagttgtct ctgtcatcag ggatgccgtc gttgtcatcg tcatggtcac aggcgtctcc 360
   tttaccatct ttatcgtggt cggcctggtt ggcattgggc acatagggac agttgtccac 420
   gttgttctga tggccatcct catcgatgtc ctgattgttg tc                    462
<210> 25
   <211> 283
   <212> DNA
   <213> Rattus sp.
<220>
   <221> gene
   <222> (1)..(283)
   <223> DEST 700
<220>
   <221> misc_feature
   <222> (171)..(171)
   <223> "n" can be a or g or c or t
<400> 25
   actctcccca ggtgttgagg tgggaggaaa ggggcttctg caccttcgat gatggcctcg 60
   atacctgact gcgggttctg cagctctgtt tggtagcact gctggcaggg aaacttcctg 120
   cctcctggat gaaggaaggg tttactcatc atcactggca gctgcaggaa ntcgggggcc 180
   tgataaacaa tttcataacc cctttctgct agtccgtctg ccaaaaaact caaaggaccc 240
   aagcagggca gctgttccac aggcgagcca ctgtcagctg agt                   283
<210> 26
   <211> 344
   <212> DNA
   <213> Rattus sp.
<220>
   <221> gene
   <222> (1)..(344)
   <223> H.sapiens mRNA for ctGF (78974)
<220>
   <221> misc_feature
   <222> (329)..(329)
   <223> "n" can be a or g or c or t
<400> 26
   actacaggaa gatgtatgga gacatggcgt aaagccaggg agtaagggac acgaactcat 60
   ttagactata acttgaactg agttacatct cattttcttc tgtaaaaaaa caaaaaggat 120
   tacagtagca cattaattta aatctgggtt cctaactgct gtgggagaaa acaccccacc 180
   gaagtgagaa ccgtgtgtca ttgtcatgca aatagcctgt caatctcaga cactggtttc 240
   gagacagttt agacttgaca gttgttcact agcgcacagt gacagaacgc acactaaggt 300
   gagcctcctg gcaagagtgg acatgccanc gagaaaagac aggt                  344
<210> 27
   <211> 239
   <212> DNA
   <213> Rattus sp.
<220>
   <221> gene
   <222> (1)..(239)
   <223> sarp 1 (AF017989)
<400> 27
   actacagcta ttcaaaacta cccttaaaat gagctgtttt aaaacttcat aaaaaacagt 60
   catgatttta ttagttgtga atatttctac aagatctgga tggacttttc gctttatgtg 120
   aaaatagcta tctgttcccg gggcctccta gggccttata actcaggaca cgttaggata 180
   cacacatgca aaaggcaggg caagctgctc caagctgaga ctgtagctct cccgacagt  239
<210> 28
   <211> 290
   <212> DNA
   <213> Rattus sp.
<220>
   <221> gene
   <222> (1)..(290)
   <223> Syntrophin 1 mus musculus (U00677)
<220>
   <221> misc_feature
   <222> (143)..(143)
   <223> "n" can be a or g or c or t
<400> 28
   acacagaagc cattccgagg ctgggacctg accctgtggg gatccacccc atctccagtc 60
   aacagcagag ctgccacacc tcctggaggc ccctaagcca ccctgagatc tctgcagatg 120
   gagtcttctg ggtcagtggc aanaggagga agaggagcct ttctgttcac tctcccttgg 180
   gccccgaggg ctgtctgggc tctctcctcc tctctctcct tcctcttgat aataaacagc 240
   ctgtgagcac atagacaaaa aaaaaaaaaa aaaaaaaaaa aaaagcttgt            290
<210> 29
   <211> 592
   <212> DNA
   <213> Rattus sp.
<220>
   <221> gene
   <222> (1)..(592)
   <223> erg25 methyl sterol (U60205) filamin (or region between filamin & GPI)
<400> 29
   actggacatc cgtcccaaag attctgtccc accacgtgaa ggtggaggcg taattcccaa 60
   tgaagttcat gtggtggaaa tcgtgatgcc gagccccggt ataaaaaggg atgtagttca 120
   acgggttgag agggatatca taaccactgt gcacatcgat ggtttctagc aaacgcatgg 180
   tcacccatgc ccacagaaga attacatgat cacacaaaag cacaattcca atgaaaaaat 240
   ccagtcccaa gaattagggt ttccaaggga tgtgcatatt ctgcttcgat tccaaatgga 300
   gcctggaaac tcgtgatgga ctttatgaat gtatttataa atcctcttgt ggtgaaggag 360
   tctgtgcagg aaatagtgcc aggtatcctc gatgactgca cagcccaaac accttgccaa 420
   agtgaaatac catcttggca ttctttccca atcataagga atattgaaaa actctgtaaa 480
   atagtatgtt ccacaaatca gaagcaactg gataaaaaaa tgattaaaca gaattccttt 540
   caaacacttc cactggcctt caaaggttct ggtttatcct tttgaatctt gt         592
<210> 30
   <211> 344
   <212> DNA
   <213> Rattus sp.
<220>
   <221> gene
   <222> (1)..(330)
   <223> DEST (AA240223)
<220>
   <221> misc_feature
   <222> (334)..(334)
   <223> "n" can be a or g or c or t
<400> 30
   accataagca gtttccatgg ggtcagagaa tggctggaga gttgtggaaa ccagcaggag 60
   gtattgtttt ggagacagga cctcactgtg tagccctggc ctcaaactca gagatccacc 120
   tgcctctgcc tcctgagtgc ttggattaaa ggcatgcacc accaccgccg cctgacaggg 180
   aggaagtttc tgatggagga gctattgaca ggtagcagag acagccgaaa gacgatcatg 240
   ggccatctgt ggggtcagtg ggggctgctg tatgtctttg ctgctttaca gggaaggtga 300
   tgtatgcatc gtagccaaac agcaaggtag ccantaagcc cagt                  344
<210> 31
   <211> 102
   <212> DNA
   <213> Rattus sp.
<220>
   <221> gene
   <222> (1)..(102)
   <223> OSTEONECTINE
<400> 31
   acttgtcatt gtctaggtca caggtctcaa agaagcgagt ggtgcaatgt tccatgggaa 60
   tgaggggagc gcgcagtggg gccagctccg tgtgggacag gt                    102
<210> 32
   <211> 8882
   <212> DNA
   <213> Rattus sp.
<220>
   <221> CDS
   <222> (574)..(8367)
   <223> Coding sequence for the inventive nucleic acid molecule 608.
<220>
   <221> misc_feature
   <222> (5560)..(5560)
   <223> "n" can be a or g or c or t
<220>
   <221> misc_feature
   <222> (1663)..(1663)
   <223> "Xaa" can be any amino acid
<400> 32
   gagagacgac agaaggttac ggctgcgaga agacgacaga agggtccaga aaaaggaaag 60
   tgctggaggg gagtggggac aaaagcagcg accaagtgaa tgtcacttca gtgactgagg 120
   ccaggcaaaa cgcgcgggaa ggattttgtg tagcttggga ccctttcata gacactgatg 180
   acacgtttac gcaaaataga aatttgagga gaaacgcctg ggccttcgga aaggagtgat 240
   tgattagtac ttgcaagttt aggtgacttt aaggagaact aactaatgta tactattgag 300
   ggaggaggaa gagcattaca gagtttccag cagcagcagg aaagctttgg ttaatttgga 360
   aatggatgat agcattaaaa taacagaagc gcctccaggt ctctgaagct tcagtccccc 420
   agctgaaagc cagaaaagac taagcccact aagccttttg atccctttgg aagcaaagaa 480
   ctttccttcc ctggggtgaa gactctcctc agaagatttc ctgtctctgc ctatgttaca 540
   agaggaatca aaaccaagac agaagagctc agg atg cag gtg aga ggc agg gaa  594
                                        Met Gln Val Arg Gly Arg Glu
                                          1               5
   gtc agc ggc ttg ttg atc tcc ctc act gct gtc tgc ctg gtg gtc acc   642
   Val Ser Gly Leu Leu Ile Ser Leu Thr Ala Val Cys Leu Val Val Thr
           10                  15                  20
   cct ggg agc agg gcc tgt cct cgc cgc tgt gcc tgc tat gtg ccc aca   690
   Pro Gly Ser Arg Ala Cys Pro Arg Arg Cys Ala Cys Tyr Val Pro Thr
       25                  30                  35
   gag gtg cac tgt aca ttt cgg tac ctg acc tcc atc cca gat ggc atc   738
   Glu Val His Cys Thr Phe Arg Tyr Leu Thr Ser Ile Pro Asp Gly Ile
    40                  45                  50                  55
   ccg gcc aat gtg gaa cga ata aat tta gga tat aac agc ctt act aga   786
   Pro Ala Asn Val Glu Arg Ile Asn Leu Gly Tyr Asn Ser Leu Thr Arg
                   60                  65                  70
   ttg aca gaa aac gac ttt gat ggc ctg agc aaa ctg gag tta ctc atg   834
   Leu Thr Glu Asn Asp Phe Asp Gly Leu Ser Lys Leu Glu Leu Leu Met
               75                  80                  85
   ctg cac agt aat ggc att cac aga gtc agt gac aag acc ttc tcg ggc   882
   Leu His Ser Asn Gly Ile His Arg Val Ser Asp Lys Thr Phe Ser Gly
           90                  95                 100
   ttg cag tcc ttg cag gtc tta aaa atg agc tat aac aaa gtc caa atc   930
   Leu Gln Ser Leu Gln Val Leu Lys Met Ser Tyr Asn Lys Val Gln Ile
       105                 110                 115
   att cgg aag gat act ttc tac gga ctc ggg agc ttg gtc cgg ttg cac   978
   Ile Arg Lys Asp Thr Phe Tyr Gly Leu Gly Ser Leu Val Arg Leu His
   120                 125                 130                 135
   ctg gat cac aac aac att gaa ttc atc aac cct gag gcc ttt tat gga  1026
   Leu Asp His Asn Asn Ile Glu Phe Ile Asn Pro Glu Ala Phe Tyr Gly
                   140                 145                 150
   ctt acc tcg ctc cgc ttg gta cat tta gaa gga aac cgg ctc aca aag  1074
   Leu Thr Ser Leu Arg Leu Val His Leu Glu Gly Asn Arg Leu Thr Lys
               155                 160                 165
   ctc cat cca gac aca ttt gtc tca tta agc tat ctc cag ata ttt aaa  1122
   Leu His Pro Asp Thr Phe Val Ser Leu Ser Tyr Leu Gln Ile Phe Lys
           170                 175                 180
   acc tct ttc att aag tac ctg ttc ttg tct gat aac ttc ctg acc tcc  1170
   Thr Ser Phe Ile Lys Tyr Leu Phe Leu Ser Asp Asn Phe Leu Thr Ser
       185                 190                 195
   ctc cca aaa gaa atg gtc tcc tac atg cca aac cta gaa agc ctg tat  1218
   Leu Pro Lys Glu Met Val Ser Tyr Met Pro Asn Leu Glu Ser Leu Tyr
   200                 205                 210                 215
   ttg cat gga aac cca tgg acc tgt gac tgc cat tta aag tgg ttg tct  1266
   Leu His Gly Asn Pro Trp Thr Cys Asp Cys His Leu Lys Trp Leu Ser
                   220                 225                 230
   gag tgg atg cag gga aac cca gat ata ata aaa tgc aag aaa gac aga  1314
   Glu Trp Met Gln Gly Asn Pro Asp Ile Ile Lys Cys Lys Lys Asp Arg
               235                 240                 245
   agc tct tcc agt cct cag caa tgt ccc ctt tgc atg aac ccc agg atc    1362
   Ser Ser Ser Ser Pro Gln Gln Cys Pro Leu Cys Met Asn Pro Arg Ile
           250                 255                 260
   tct aaa ggc aga ccc ttt gct atg gta cca tct gga gct ttc cta tgt    1410
   Ser Lys Gly Arg Pro Phe Ala Met Val Pro Ser Gly Ala Phe Leu Cys
       265                 270                 275
   aca aag cca acc att gat cca tca ctg aag tca aag agc ctg gtt act    1458
   Thr Lys Pro Thr Ile Asp Pro Ser Leu Lys Ser Lys Ser Leu Val Thr
   280                 285                 290                 295
   cag gag gac aat gga tct gcc tcc acc tca cct caa gat ttc ata gaa    1506
   Gln Glu Asp Asn Gly Ser Ala Ser Thr Ser Pro Gln Asp Phe Ile Glu
                   300                 305                 310
   ccc ttt ggc tcc ttg tct ttg aac atg aca kas mtk tct gga aat aag    1554
   Pro Phe Gly Ser Leu Ser Leu Asn Met Thr Xaa Xaa Ser Gly Asn Lys
               315                 320                 325
   gcc gac atg gtc tgt agt atc caa aag cca tca agg aca tca cca act    1602
   Ala Asp Met Val Cys Ser Ile Gln Lys Pro Ser Arg Thr Ser Pro Thr
           330                 335                 340
   gca ttc act gaa gaa aat gac tac atc atg cta aat gcg tca ttt tcc    1650
   Ala Phe Thr Glu Glu Asn Asp Tyr Ile Met Leu Asn Ala Ser Phe Ser
       345                 350                 355
   aca aat ctt gtg tgc agt gta gat tat aat cac atc cag cca gtg tgg    1698
   Thr Asn Leu Val Cys Ser Val Asp Tyr Asn His Ile Gln Pro Val Trp
   360                 365                 370                 375
   caa ctt ctg gct tta tac agt gac tct cct ctg ata cta gaa agg aag    1746
   Gln Leu Leu Ala Leu Tyr Ser Asp Ser Pro Leu Ile Leu Glu Arg Lys
                   380                 385                 390
   ccc cag ctt acc gag act cct tca ctg tct tct aga tat aaa cag gtg    1794
   Pro Gln Leu Thr Glu Thr Pro Ser Leu Ser Ser Arg Tyr Lys Gln Val
               395                 400                 405
   gct ctt agg cct gaa gac att ttt acc agc ata gag gct gat gtc aga    1842
   Ala Leu Arg Pro Glu Asp Ile Phe Thr Ser Ile Glu Ala Asp Val Arg
           410                 415                 420
   gca gac cct ttt tgg ttc caa caa gaa aaa att gtc ttg cag ctg aac    1890
   Ala Asp Pro Phe Trp Phe Gln Gln Glu Lys Ile Val Leu Gln Leu Asn
       425                 430                 435
   aga act gcc acc aca ctt agc aca tta cag atc cag ttt tcc act gat    1938
   Arg Thr Ala Thr Thr Leu Ser Thr Leu Gln Ile Gln Phe Ser Thr Asp
   440                 445                 450                 455
   gct caa atc gct tta cca agg gcg gag atg aga gcg gag aga ctc aaa    1986
   Ala Gln Ile Ala Leu Pro Arg Ala Glu Met Arg Ala Glu Arg Leu Lys
                   460                 465                 470
   tgg acc atg atc ctg atg atg aac aat ccc aaa ctg gaa cgc act gtc    2034
   Trp Thr Met Ile Leu Met Met Asn Asn Pro Lys Leu Glu Arg Thr Val
               475                 480                 485
   ctg gtt ggc ggc act att gcc ctg agc tgt cca ggc aaa ggc gac cct    2082
   Leu Val Gly Gly Thr Ile Ala Leu Ser Cys Pro Gly Lys Gly Asp Pro
           490                 495                 500
   tca cct cac ttg gaa tgg ctt cta gct gat ggg agt aaa gtg aga gcc    2130
   Ser Pro His Leu Glu Trp Leu Leu Ala Asp Gly Ser Lys Val Arg Ala
       505                 510                 515
   cct tac gtt agc gag gat ggg cga atc cta ata gac aaa aat ggg aag    2178
   Pro Tyr Val Ser Glu Asp Gly Arg Ile Leu Ile Asp Lys Asn Gly Lys
   520                 525                 530                 535
   ttg gaa ctg cag atg gct gac agc ttt gat gca ggt ctt tac cac tgc    2226
   Leu Glu Leu Gln Met Ala Asp Ser Phe Asp Ala Gly Leu Tyr His Cys
                   540                 545                 550
   ata agc acc aat gat gca gat gcg gat gtt ctc aca tac agg ata act    2274
   Ile Ser Thr Asn Asp Ala Asp Ala Asp Val Leu Thr Tyr Arg Ile Thr
               555                 560                 565
   gtg gta gag ccc tat gga gaa agc aca cat gac agt gga gtc cag cac    2322
   Val Val Glu Pro Tyr Gly Glu Ser Thr His Asp Ser Gly Val Gln His
           570                 575                 580
   aca gtg gtt acg ggt gag acg ctc gac ctt cca tgc ctt tcc acg ggt    2370
   Thr Val Val Thr Gly Glu Thr Leu Asp Leu Pro Cys Leu Ser Thr Gly
       585                 590                 595
   gtt cca gat gct tct att agc tgg att ctt cca ggg aac act gtg ttc    2418
   Val Pro Asp Ala Ser Ile Ser Trp Ile Leu Pro Gly Asn Thr Val Phe
   600                 605                 610                 615
   tct cag cca tca aga gac agg caa att ctt aac aat ggg acc tta aga    2466
   Ser Gln Pro Ser Arg Asp Arg Gln Ile Leu Asn Asn Gly Thr Leu Arg
                   620                 625                 630
   ata tta cag gtt acg cca aaa gat caa ggt cat tac caa tgt gtg gct    2514
   Ile Leu Gln Val Thr Pro Lys Asp Gln Gly His Tyr Gln Cys Val Ala
               635                 640                 645
   gcc aac cca tca ggg gcc gac ttt tcc agt ttt aaa gtt tca gtt caa    2562
   Ala Asn Pro Ser Gly Ala Asp Phe Ser Ser Phe Lys Val Ser Val Gln
           650                 655                 660
   aag aaa ggc caa agg atg gtt gag cat gac agg gag gca ggt gga tct    2610
   Lys Lys Gly Gln Arg Met Val Glu His Asp Arg Glu Ala Gly Gly Ser
       665                 670                 675
   gga ctt gga gaa ccc aac tcc agt gtt tcc ctt aag cag cca gca tct    2658
   Gly Leu Gly Glu Pro Asn Ser Ser Val Ser Leu Lys Gln Pro Ala Ser
   680                 685                 690                 695
   ttg aaa ctc tct gca tca gct ttg aca ggg tca gag gct gga aaa caa    2706
   Leu Lys Leu Ser Ala Ser Ala Leu Thr Gly Ser Glu Ala Gly Lys Gln
                   700                 705                 710
   gtc tcc ggt gta cat agg aag aac aaa cat aga gac tta ata cat cgg    2754
   Val Ser Gly Val His Arg Lys Asn Lys His Arg Asp Leu Ile His Arg
               715                 720                 725
   cgg cgt ggg gat tcc acg ctc cgg cga ttc agg gag cat agg agg cag    2802
   Arg Arg Gly Asp Ser Thr Leu Arg Arg Phe Arg Glu His Arg Arg Gln
           730                 735                 740
   ctc cct ctc tct gct cgg aga att gac ccg caa cgc tgg gca gca ctt    2850
   Leu Pro Leu Ser Ala Arg Arg Ile Asp Pro Gln Arg Trp Ala Ala Leu
       745                 750                 755
   cta gaa aaa gcc aaa aag aat tct gtg cca aaa aag caa gaa aat acc    2898
   Leu Glu Lys Ala Lys Lys Asn Ser Val Pro Lys Lys Gln Glu Asn Thr
   760                 765                 770                 775
   aca gta aag cca gtg cca ctg gct gtt ccc ctc gtg gaa ctc act gac    2946
   Thr Val Lys Pro Val Pro Leu Ala Val Pro Leu Val Glu Leu Thr Asp
                   780                 785                 790
   gag gaa aag gat gcc tct ggc atg att cct cca gat gaa gaa ttc atg    2994
   Glu Glu Lys Asp Ala Ser Gly Met Ile Pro Pro Asp Glu Glu Phe Met
               795                 800                 805
   gtt ctg aaa act aag gct tct ggt gtc cca gga agg tca cca act gct    3042
   Val Leu Lys Thr Lys Ala Ser Gly Val Pro Gly Arg Ser Pro Thr Ala
           810                 815                 820
   gac tct gga cca gta aat cat ggt ttt atg acg agt ata gct tct ggc    3090
   Asp Ser Gly Pro Val Asn His Gly Phe Met Thr Ser Ile Ala Ser Gly
       825                 830                 835
   aca gaa gtc tca act gtg aat cca caa aca cta caa tct gag cac ctt    3138
   Thr Glu Val Ser Thr Val Asn Pro Gln Thr Leu Gln Ser Glu His Leu
   840                 845                 850                 855
   cct gat ttc aaa tta ttt agt gta aca aac ggt aca gct gtg aca aag    3186
   Pro Asp Phe Lys Leu Phe Ser Val Thr Asn Gly Thr Ala Val Thr Lys
                   860                 865                 870
   agt atg aac cca tcc ata gca agc aaa ata gaa gat aca acc aac caa    3234
   Ser Met Asn Pro Ser Ile Ala Ser Lys Ile Glu Asp Thr Thr Asn Gln
               875                 880                 885
   aac cca atc att atc ttt cca tca gta gct gaa att cga gat tct gct    3282
   Asn Pro Ile Ile Ile Phe Pro Ser Val Ala Glu Ile Arg Asp Ser Ala
           890                 895                 900
   cag gca gga aga gca tct tcc caa agt gca cac cct gta aca ggg gga    3330
   Gln Ala Gly Arg Ala Ser Ser Gln Ser Ala His Pro Val Thr Gly Gly
       905                 910                 915
   aac atg gct acc tat ggc cat acc aac aca tat agt agc ttt acc agc    3378
   Asn Met Ala Thr Tyr Gly His Thr Asn Thr Tyr Ser Ser Phe Thr Ser
   920                 925                 930                 935
   aaa gcc agt aca gtc ttg cag cca ata aat cca aca gaa agt tat gga    3426
   Lys Ala Ser Thr Val Leu Gln Pro Ile Asn Pro Thr Glu Ser Tyr Gly
                   940                 945                 950
   cct cag ata cct att aca gga gtc agc aga cct agc agt agt gac atc    3474
   Pro Gln Ile Pro Ile Thr Gly Val Ser Arg Pro Ser Ser Ser Asp Ile
               955                 960                 965
   tct tct cac act act gca gac cct agc ttc tcc agt cac cct tca ggt    3522
   Ser Ser His Thr Thr Ala Asp Pro Ser Phe Ser Ser His Pro Ser Gly
           970                 975                 980
   tca cac acc act gcc tcg tct tta ttt cac att cct aga aac aac aat    3570
   Ser His Thr Thr Ala Ser Ser Leu Phe His Ile Pro Arg Asn Asn Asn
       985                 990                 995
   aca ggt aac ttc ccc ttg tcc agg cac ttg gga aga gag agg aca att    3618
   Thr Gly Asn Phe Pro Leu Ser Arg His Leu Gly Arg Glu Arg Thr Ile
   1000                1005                1010                1015
   tgg agc aga ggg aga gtt aaa aac cca cat aga acc cca gtt ctc cga    3666
   Trp Ser Arg Gly Arg Val Lys Asn Pro His Arg Thr Pro Val Leu Arg
                   1020                1025                1030
   cgg cat aga cac agg act gtg agg cca gca atc aag gga cct gct aac    3714
   Arg His Arg His Arg Thr Val Arg Pro Ala Ile Lys Gly Pro Ala Asn
               1035                1040                1045
   aaa aat gtg agc caa gtt cca gcc aca gag tac cct ggg atg tgc cac    3762
   Lys Asn Val Ser Gln Val Pro Ala Thr Glu Tyr Pro Gly Met Cys His
           1050                1055                1060
   aca tgt cct tcc gca gag ggg ctc aca gtg gct act gca gca ctg tca    3810
   Thr Cys Pro Ser Ala Glu Gly Leu Thr Val Ala Thr Ala Ala Leu Ser
       1065                1070                1075
   gtt cca agt tca tcc cac agt gcc ctc ccc aaa act aat aat gtt ggg    3858
   Val Pro Ser Ser Ser His Ser Ala Leu Pro Lys Thr Asn Asn Val Gly
   1080                1085                1090                1095
   gtc ata gca gaa gag tct acc act gtg gtc aag aaa cca ctg tta cta    3906
   Val Ile Ala Glu Glu Ser Thr Thr Val Val Lys Lys Pro Leu Leu Leu
                   1100                1105                1110
   ttt aag gac aaa caa aat gta gat att gag ata ata aca acc act aca    3954
   Phe Lys Asp Lys Gln Asn Val Asp Ile Glu Ile Ile Thr Thr Thr Thr
               1115                1120                1125
   aaa tat tcc gga ggg gaa agt aac cac gtg att cct acg gaa gca agc    4002
   Lys Tyr Ser Gly Gly Glu Ser Asn His Val Ile Pro Thr Glu Ala Ser
           1130                1135                1140
   atg act tct gct cca aca tct gta tcc ctg ggg aaa tct cct gta gac    4050
   Met Thr Ser Ala Pro Thr Ser Val Ser Leu Gly Lys Ser Pro Val Asp
       1145                1150                1155
   aat agt ggt cac ctg agc atg cct ggg acc atc caa act ggg aaa gat    4098
   Asn Ser Gly His Leu Ser Met Pro Gly Thr Ile Gln Thr Gly Lys Asp
   1160                1165                1170                1175
   tca gtg gaa aca aca cca ctt ccc agc ccc ctc agc aca ccc tca ata    4146
   Ser Val Glu Thr Thr Pro Leu Pro Ser Pro Leu Ser Thr Pro Ser Ile
                   1180                1185                1190
   cca aca agc aca aaa ttc tca aag agg aaa act ccc ttg cac cag atc    4194
   Pro Thr Ser Thr Lys Phe Ser Lys Arg Lys Thr Pro Leu His Gln Ile
               1195                1200                1205
   ttt gta aat aac cag aag aag gag ggg atg tta aag aat cca tat caa    4242
   Phe Val Asn Asn Gln Lys Lys Glu Gly Met Leu Lys Asn Pro Tyr Gln
         1210                1215                1220
   ttc ggt tta caa aag aac cca gcc gca aag ctt ccc aaa ata gct cct     4290
   Phe Gly Leu Gln Lys Asn Pro Ala Ala Lys Leu Pro Lys Ile Ala Pro
      1225                1230                1235
   ctt tta ccc aca ggt cag agt tcc ccc tca gat tct aca act ctc ttg     4338
   Leu Leu Pro Thr Gly Gln Ser Ser Pro Ser Asp Ser Thr Thr Leu Leu
   1240                1245                1250                1255
   aca agt ccg cca cca gct ctg tct aca aca atg gct gcc act cag aac     4386
   Thr Ser Pro Pro Pro Ala Leu Ser Thr Thr Met Ala Ala Thr Gln Asn
               1260                1265                1270
   aag ggc act gaa gta gta tca ggt gcc aga agt ctc tca gca ggg aag     4434
   Lys Gly Thr Glu Val Val Ser Gly Ala Arg Ser Leu Ser Ala Gly Lys
           1275                1280                1285
   aag cag ccc ttc acc aac tcc tct cca gtg ctt cct agc acc ata agc     4482
   Lys Gln Pro Phe Thr Asn Ser Ser Pro Val Leu Pro Ser Thr Ile Ser
         1290                1295                1300
   aag aga tct aat aca tta aac ttc ttg tca acg gaa acc ccc aca gtg     4530
   Lys Arg Ser Asn Thr Leu Asn Phe Leu Ser Thr Glu Thr Pro Thr Val
      1305                1310                1315
   aca agt cct act gct act gca tct gtc att atg tct gaa acc caa cga     4578
   Thr Ser Pro Thr Ala Thr Ala Ser Val Ile Met Ser Glu Thr Gln Arg
   1320                1325                1330                1335
   aca aga tcc aaa gaa gca aaa gac caa ata aag ggg cct cgg aag aac     4626
   Thr Arg Ser Lys Glu Ala Lys Asp Gln Ile Lys Gly Pro Arg Lys Asn
               1340                1345                1350
   aga aac aac gca aac acc acc ccc agg cag gtt tct ggc tat agt gca     4674
   Arg Asn Asn Ala Asn Thr Thr Pro Arg Gln Val Ser Gly Tyr Ser Ala
           1355                1360                1365
   tac tca gct cta aca aca gct gat acc ccc ttg gct ttc agt cat tcc     4722
   Tyr Ser Ala Leu Thr Thr Ala Asp Thr Pro Leu Ala Phe Ser His Ser
         1370                1375                1380
   cca cga caa gat gat ggt gga aat gta agt gca gtt gct tat cac tca     4770
   Pro Arg Gln Asp Asp Gly Gly Asn Val Ser Ala Val Ala Tyr His Ser
      1385                1390                1395
   aca acc tct ctt ctg gcc ata act gaa ctg ttt gag aag tac acc cag     4818
   Thr Thr Ser Leu Leu Ala Ile Thr Glu Leu Phe Glu Lys Tyr Thr Gln
   1400                1405                1410                1415
   act ttg gga aat aca aca gct ttg gaa aca acg ttg ttg agc aaa tca     4866
   Thr Leu Gly Asn Thr Thr Ala Leu Glu Thr Thr Leu Leu Ser Lys Ser
               1420                1425                1430
   cag gag agt acc aca gtg aaa aga gcc tca gac aca cca cca cca ctc     4914
   Gln Glu Ser Thr Thr Val Lys Arg Ala Ser Asp Thr Pro Pro Pro Leu
           1435                1440                1445
   ctc agc agt ggg gcg ccc cca gtg ccc act cct tcc cca cct cct ttt     4962
   Leu Ser Ser Gly Ala Pro Pro Val Pro Thr Pro Ser Pro Pro Pro Phe
           1450                1455                1460
   act aag ggt gtg gtt aca gac agc aaa gtc aca tca gct ttc cag atg    5010
   Thr Lys Gly Val Val Thr Asp Ser Lys Val Thr Ser Ala Phe Gln Met
       1465                1470                1475
   acg tca aat aga gtg gtc acc ata tat gaa tct tca agg cac aat aca    5058
   Thr Ser Asn Arg Val Val Thr Ile Tyr Glu Ser Ser Arg His Asn Thr
   1480                1485                1490                1495
   gat ctg cag caa ccc tca gca gag gct agc ccc aat cct gag atc ata    5106
   Asp Leu Gln Gln Pro Ser Ala Glu Ala Ser Pro Asn Pro Glu Ile Ile
                   1500                1505                1510
   act gga acc act gac tct ccc tct aat ctg ttt cca tcc act tct gtg    5154
   Thr Gly Thr Thr Asp Ser Pro Ser Asn Leu Phe Pro Ser Thr Ser Val
               1515                1520                1525
   cca gca cta agg gta gat aaa cca cag aat tct aaa tgg aag ccc tct    5202
   Pro Ala Leu Arg Val Asp Lys Pro Gln Asn Ser Lys Trp Lys Pro Ser
           1530                1535                1540
   ccc tgg cca gaa cac aaa tat cag ctc aag tca tac tcc gaa acc att    5250
   Pro Trp Pro Glu His Lys Tyr Gln Leu Lys Ser Tyr Ser Glu Thr Ile
       1545                1550                1555
   gag aag ggc aaa agg cca gca gta agc atg tcc ccc cac ctc agc ctt    5298
   Glu Lys Gly Lys Arg Pro Ala Val Ser Met Ser Pro His Leu Ser Leu
   1560                1565                1570                1575
   cca gag gcc agc act cat gcc tca cac tgg aat aca cag aag cat gca    5346
   Pro Glu Ala Ser Thr His Ala Ser His Trp Asn Thr Gln Lys His Ala
                   1580                1585                1590
   gaa aag agt gtt ttt gat aag aaa cct ggt caa aac cca act tcc aaa    5394
   Glu Lys Ser Val Phe Asp Lys Lys Pro Gly Gln Asn Pro Thr Ser Lys
               1595                1600                1605
   cat ctg cct tac gtc tct cta cct aag act cta ttg aaa aag cca aga    5442
   His Leu Pro Tyr Val Ser Leu Pro Lys Thr Leu Leu Lys Lys Pro Arg
           1610                1615                1620
   ata att gga gga aag gct gca agc ttt aca gtt cca gct aat tca gac    5490
   Ile Ile Gly Gly Lys Ala Ala Ser Phe Thr Val Pro Ala Asn Ser Asp
       1625                1630                1635
   gtt ttt ctt cct tgt gag gct gtt gga gac cca ctg ccc atc atc cac    5538
   Val Phe Leu Pro Cys Glu Ala Val Gly Asp Pro Leu Pro Ile Ile His
   1640                1645                1650                1655
   tgg acc aga gtt tca tca gga ntt gaa ata tcc caa ggg aca cag aaa    5586
   Trp Thr Arg Val Ser Ser Gly Xaa Glu Ile Ser Gln Gly Thr Gln Lys
                   1660                1665                1670
   agc cgg ttc cac gtg ctt ccc aat ggc acc ttg tcc atc cag agg gtc    5634
   Ser Arg Phe His Val Leu Pro Asn Gly Thr Leu Ser Ile Gln Arg Val
               1675                1680                1685
   agt att cag gac cgt gga cag tac ctg tgc tct gca ttt aat cca ctg    5682
   Ser Ile Gln Asp Arg Gly Gln Tyr Leu Cys Ser Ala Phe Asn Pro Leu
           1690                1695                1700
   ggc gta gac cat ttt cat gtc tct ttg tct gtg gtt ttt tac ccg gca    5730
   Gly Val Asp His Phe His Val Ser Leu Ser Val Val Phe Tyr Pro Ala
      1705                1710                1715
   agg att ttg gac aga cat gtc aag gag atc aca gtt cac ttt gga agt    5778
   Arg Ile Leu Asp Arg His Val Lys Glu Ile Thr Val His Phe Gly Ser
   1720                1725                1730                1735
   act gtg gaa cta aag tgc aga gtg gag ggt atg ccg agg cct acg gtt    5826
   Thr Val Glu Leu Lys Cys Arg Val Glu Gly Met Pro Arg Pro Thr Val
                   1740                1745                1750
   tcc tgg ata ctt gca aac caa acg gtg gtc tca gaa acg gcc aag gga    5874
   Ser Trp Ile Leu Ala Asn Gln Thr Val Val Ser Glu Thr Ala Lys Gly
               1755                1760                1765
   agc aga aag gtc tgg gta aca cct gat gga aca ttg atc atc tat aat    5922
   Ser Arg Lys Val Trp Val Thr Pro Asp Gly Thr Leu Ile Ile Tyr Asn
           1770                1775                1780
   ctg agt ctt tat gat cgt ggt ttt tac aag tgt gtg gcc agc aac cca    5970
   Leu Ser Leu Tyr Asp Arg Gly Phe Tyr Lys Cys Val Ala Ser Asn Pro
      1785                1790                1795
   tct ggc cag gat tca ctg ttg gtt aag ata caa gtc atc aca gct ccc    6018
   Ser Gly Gln Asp Ser Leu Leu Val Lys Ile Gln Val Ile Thr Ala Pro
   1800                1805                1810                1815
   cct gtc att ata gag caa aag agg caa gcc atc gtt ggg gtt tta ggt    6066
   Pro Val Ile Ile Glu Gln Lys Arg Gln Ala Ile Val Gly Val Leu Gly
                   1820                1825                1830
   gga agt ttg aaa ctg ccc tgc act gca aaa gga act ccc cag cct agt    6114
   Gly Ser Leu Lys Leu Pro Cys Thr Ala Lys Gly Thr Pro Gln Pro Ser
               1835                1840                1845
   gtt cac tgg gtc ctt tat gat ggg act gaa cta aaa cca ttg cag ttg    6162
   Val His Trp Val Leu Tyr Asp Gly Thr Glu Leu Lys Pro Leu Gln Leu
           1850                1855                1860
   act cat tcc aga ttt ttc ttg tat cca aat gga act ctg tat ata aga    6210
   Thr His Ser Arg Phe Phe Leu Tyr Pro Asn Gly Thr Leu Tyr Ile Arg
      1865                1870                1875
   agc atc gct cct tca gtg agg ggc act tat gag tgc att gcc acc agc    6258
   Ser Ile Ala Pro Ser Val Arg Gly Thr Tyr Glu Cys Ile Ala Thr Ser
   1880                1885                1890                1895
   tcc tca ggc tca gag aga agg gta gtg att ctt act gtg gaa gag gga    6306
   Ser Ser Gly Ser Glu Arg Arg Val Val Ile Leu Thr Val Glu Glu Gly
                   1900                1905                1910
   gag aca atc ccc agg ata gaa act gcc tct cag aaa tgg act gag gtg    6354
   Glu Thr Ile Pro Arg Ile Glu Thr Ala Ser Gln Lys Trp Thr Glu Val
               1915                1920                1925
   aat ttg ggt gag aaa tta cta ctg aac tgc tca gct act ggg gat cca    6402
   Asn Leu Gly Glu Lys Leu Leu Leu Asn Cys Ser Ala Thr Gly Asp Pro
           1930                1935                1940
   aag cct aga ata atc tgg agg ctg cca tcc aag gct gtc atc gac cag    6450
   Lys Pro Arg Ile Ile Trp Arg Leu Pro Ser Lys Ala Val Ile Asp Gln
      1945                1950                1955
   tgg cac aga atg ggc agc cga atc cac gtc tac cca aat gga tcc ttg    6498
   Trp His Arg Met Gly Ser Arg Ile His Val Tyr Pro Asn Gly Ser Leu
   1960                1965                1970                1975
   gtg gtt ggg tca gtg acg gaa aaa gac gct ggt gac tac tta tgt gtg    6546
   Val Val Gly Ser Val Thr Glu Lys Asp Ala Gly Asp Tyr Leu Cys Val
                   1980                1985                1990
   gca aga aac aaa atg gga gat gac cta gtc ctg atg cat gtc cgc ctg    6594
   Ala Arg Asn Lys Met Gly Asp Asp Leu Val Leu Met His Val Arg Leu
               1995                2000                2005
   aga ttg aca cct gcc aaa att gaa cag aag cag tat ttt aag aag caa    6642
   Arg Leu Thr Pro Ala Lys Ile Glu Gln Lys Gln Tyr Phe Lys Lys Gln
           2010                2015                2020
   gtg ctc cat ggg aaa gat ttc caa gtt gac tgc aag gcc tct ggc tcc    6690
   Val Leu His Gly Lys Asp Phe Gln Val Asp Cys Lys Ala Ser Gly Ser
       2025                2030                2035
   cct gtg cct gag gta tcc tgg agt ttg cct gat ggg aca gtg ctc aac    6738
   Pro Val Pro Glu Val Ser Trp Ser Leu Pro Asp Gly Thr Val Leu Asn
   2040                2045                2050                2055
   aat gta gcc caa gct gat gac agt ggc tat agg acc aag agg tac acc    6786
   Asn Val Ala Gln Ala Asp Asp Ser Gly Tyr Arg Thr Lys Arg Tyr Thr
                   2060                2065                2070
   ctt ttc cac aat gga acc ttg tat ttc aac aac gtt ggg atg gca gag    6834
   Leu Phe His Asn Gly Thr Leu Tyr Phe Asn Asn Val Gly Met Ala Glu
               2075                2080                2085
   gaa gga gat tat atc tgc tct gcc cag aac acc tta ggg aaa gat gag    6882
   Glu Gly Asp Tyr Ile Cys Ser Ala Gln Asn Thr Leu Gly Lys Asp Glu
           2090                2095                2100
   atg aaa gtc cac cta aca gtt cta aca gcc atc cca cgg ata agg caa    6930
   Met Lys Val His Leu Thr Val Leu Thr Ala Ile Pro Arg Ile Arg Gln
       2105                2110                2115
   agc tac aag acc acc atg agg ctc agg gct gga gaa aca gct gtc ctt    6978
   Ser Tyr Lys Thr Thr Met Arg Leu Arg Ala Gly Glu Thr Ala Val Leu
   2120                2125                2130                2135
   gac tgc gag gtc act ggg gaa ccg aag ccc aat gta ttt tgg ttg ctg    7026
   Asp Cys Glu Val Thr Gly Glu Pro Lys Pro Asn Val Phe Trp Leu Leu
                   2140                2145                2150
   cct tcc aac aat gtc att tca ttc tcc aat gac agg ttc aca ttt cat    7074
   Pro Ser Asn Asn Val Ile Ser Phe Ser Asn Asp Arg Phe Thr Phe His
               2155                2160                2165
   gcc aat aga act ttg tcc atc cat aaa gtg aaa cca ctt gac tct ggg    7122
   Ala Asn Arg Thr Leu Ser Ile His Lys Val Lys Pro Leu Asp Ser Gly
           2170                2175                2180
   gac tat gtg tgc gta gct cag aat cct agt ggg gat gac act aag aca    7170
   Asp Tyr Val Cys Val Ala Gln Asn Pro Ser Gly Asp Asp Thr Lys Thr
      2185                2190                2195
   tac aaa ctg gac att gtc tct aaa cct cca tta atc aat ggc ctg tat    7218
   Tyr Lys Leu Asp Ile Val Ser Lys Pro Pro Leu Ile Asn Gly Leu Tyr
   2200                2205                2210                2215
   gca aac aag act gtt att aaa gcc aca gcc att cgg cac tcc aaa aaa    7266
   Ala Asn Lys Thr Val Ile Lys Ala Thr Ala Ile Arg His Ser Lys Lys
                   2220                2225                2230
   tac ttt gac tgc aga gca gat ggg atc cca tct tcc cag gtc acg tgg    7314
   Tyr Phe Asp Cys Arg Ala Asp Gly Ile Pro Ser Ser Gln Val Thr Trp
               2235                2240                2245
   att atg cca ggc aat att ttc ctc cca gct cca tac ttt gga agc aga    7362
   Ile Met Pro Gly Asn Ile Phe Leu Pro Ala Pro Tyr Phe Gly Ser Arg
           2250                2255                2260
   gtc acg gtc cat cca aat gga acc ttg gag atg agg aac atc cgg ctt    7410
   Val Thr Val His Pro Asn Gly Thr Leu Glu Met Arg Asn Ile Arg Leu
      2265                2270                2275
   tct gac tct gcg gac ttc acc tgt gtg gtt cgg agc gag gga gga gag    7458
   Ser Asp Ser Ala Asp Phe Thr Cys Val Val Arg Ser Glu Gly Gly Glu
   2280                2285                2290                2295
   agt gtg ttg gta gtg cag tta gaa gtc cta gaa atg ctg aga aga cca    7506
   Ser Val Leu Val Val Gln Leu Glu Val Leu Glu Met Leu Arg Arg Pro
                   2300                2305                2310
   aca ttc aga aac cca ttc aac gaa aaa gtc atc gcc caa gct ggc aag    7554
   Thr Phe Arg Asn Pro Phe Asn Glu Lys Val Ile Ala Gln Ala Gly Lys
               2315                2320                2325
   ccc gta gca ctg aac tgc tct gtg gat ggg aac ccc cca cct gaa att    7602
   Pro Val Ala Leu Asn Cys Ser Val Asp Gly Asn Pro Pro Pro Glu Ile
           2330                2335                2340
   acc tgg atc tta cct gac ggc aca cag ttt gct aac aga cca cac aat    7650
   Thr Trp Ile Leu Pro Asp Gly Thr Gln Phe Ala Asn Arg Pro His Asn
      2345                2350                2355
   tcc ccg tat ctg atg gca ggc aat ggc tct ctc atc ctt tac aaa gca    7698
   Ser Pro Tyr Leu Met Ala Gly Asn Gly Ser Leu Ile Leu Tyr Lys Ala
   2360                2365                2370                2375
   act cgg aac aag tca ggg aag tat cgc tgt gca gcc agg aat aag gtt    7746
   Thr Arg Asn Lys Ser Gly Lys Tyr Arg Cys Ala Ala Arg Asn Lys Val
                   2380                2385                2390
   ggc tac atc gag aaa ctc atc ctg tta gag att ggg cag aag cca gtc    7794
   Gly Tyr Ile Glu Lys Leu Ile Leu Leu Glu Ile Gly Gln Lys Pro Val
               2395                2400                2405
   att ctg aca tac gaa cca ggg atg gtg aag agc gtc agt ggg gaa ccg    7842
   Ile Leu Thr Tyr Glu Pro Gly Met Val Lys Ser Val Ser Gly Glu Pro
           2410                2415                2420
   tta tca ctg cat tgt gtg tct gat ggg atc ccc aag cca aat gtc aag    7890
   Leu Ser Leu His Cys Val Ser Asp Gly Ile Pro Lys Pro Asn Val Lys
   2425 2430 2435
   tgg act aca ccg ggt ggc cat gta atc gac agg cct caa gtg gat gga 7938
   Trp Thr Thr Pro Gly Gly His Val Ile Asp Arg Pro Gln Val Asp Gly
   2440 2445 2450 2455
   aaa tac ata ctg cat gaa aat ggc acg ctg gtc atc aaa gca aca aca 7986
   Lys Tyr Ile Leu His Glu Asn Gly Thr Leu Val Ile Lys Ala Thr Thr
   2460 2465 2470
   gct cac gac caa gga aat tat atc tgt agg gct caa aac agt gtt ggc 8034
   Ala His Asp Gln Gly Asn Tyr Ile Cys Arg Ala Gln Asn Ser Val Gly
   2475 2480 2485
   cag gca gtt att agc gtg tca gtg atg gtt gtg gcc tac cct ccc cga 8082
   Gln Ala Val Ile Ser Val Ser Val Met Val Val Ala Tyr Pro Pro Arg
   2490 2495 2500
   atc ata aac tac cta ccc agg aac atg ctc agg agg aca ggg gaa gcc 8130
   Ile Ile Asn Tyr Leu Pro Arg Asn Met Leu Arg Arg Thr Gly Glu Ala
   2505 2510 2515
   atg cag ctc cac tgt gtg gcc ttg gga atc ccc aag cca aaa gtc acc 8178
   Met Gln Leu His Cys Val Ala Leu Gly Ile Pro Lys Pro Lys Val Thr
   2520 2525 2530 2535
   tgg gag acg cca aga cac tcc ctg ctg tca aaa gca aca gca aga aaa 8226
   Trp Glu Thr Pro Arg His Ser Leu Leu Ser Lys Ala Thr Ala Arg Lys
   2540 2545 2550
   ccc cat aga agt gag atg ctt cac cca caa ggt acg ctg gtc att cag 8274
   Pro His Arg Ser Glu Met Leu His Pro Gln Gly Thr Leu Val Ile Gln
   2555 2560 2565
   aat ctc caa acc tcg gat tcc gga gtc tat aag tgc aga gct cag aac 8322
   Asn Leu Gln Thr Ser Asp Ser Gly Val Tyr Lys Cys Arg Ala Gln Asn
   2570 2575 2580
   cta ctt ggg act gat tac gca aca act tac atc cag gta ctc tga 8367
   Leu Leu Gly Thr Asp Tyr Ala Thr Thr Tyr Ile Gln Val Leu
   2585 2590 2595
   caggaagggg gagactaaaa ttcaacagaa gtccacatcc acagggttta ttttttggaa 8427
   gaagtttaat caaaggcagc cataggcatg taaatgagtc tgaatacatt tacagtatta 8487
   aatttacaat ggacatgcga tgagacttgt aaatgaaagc attgtgaact gaaaccgagt 8547
   ctctgtggat ctcaaagcaa actcttaact taaggcactt tgattttgcc aacaaataat 8607
   aacaaacatt aagagaaaaa aatgatccac tacgaaataa caaacggcta atgcacctga 8667
   attctcagta aaaagacctt tctctcgcta acagttgcca gctgcctcgt gtctgtttcc 8727
   taccaatgtc acaaacatcg cacacagggt gaatggagtc aacgggaaag attaagtttg 8787
   cggtctgtgt aaatctcaat gtacaaatat tctgtcnctg gtttataaac attttgataa 8847
   aaccgaaaaa aaaaaaaaaa aaaaaaaaaa aaaaa 8882
<210> 33
   <211> 2597
   <212> PRT
   <213> Rattus sp.
<400> 33
   Met Gln Val Arg Gly Arg Glu Val Ser Gly Leu Leu Ile Ser Leu Thr
    1               5                  10                  15
   Ala Val Cys Leu Val Val Thr Pro Gly Ser Arg Ala Cys Pro Arg Arg
               20                  25                  30
   Cys Ala Cys Tyr Val Pro Thr Glu Val His Cys Thr Phe Arg Tyr Leu
           35                  40                  45
   Thr Ser Ile Pro Asp Gly Ile Pro Ala Asn Val Glu Arg Ile Asn Leu
       50                  55                  60
   Gly Tyr Asn Ser Leu Thr Arg Leu Thr Glu Asn Asp Phe Asp Gly Leu
   65                  70                  75                  80
   Ser Lys Leu Glu Leu Leu Met Leu His Ser Asn Gly Ile His Arg Val
                   85                  90                  95
   Ser Asp Lys Thr Phe Ser Gly Leu Gln Ser Leu Gln Val Leu Lys Met
               100                 105                 110
   Ser Tyr Asn Lys Val Gln Ile Ile Arg Lys Asp Thr Phe Tyr Gly Leu
           115                 120                 125
   Gly Ser Leu Val Arg Leu His Leu Asp His Asn Asn Ile Glu Phe Ile
       130                 135                 140
   Asn Pro Glu Ala Phe Tyr Gly Leu Thr Ser Leu Arg Leu Val His Leu
   145                 150                 155                 160
   Glu Gly Asn Arg Leu Thr Lys Leu His Pro Asp Thr Phe Val Ser Leu
                   165                 170                 175
   Ser Tyr Leu Gln Ile Phe Lys Thr Ser Phe Ile Lys Tyr Leu Phe Leu
               180                 185                 190
   Ser Asp Asn Phe Leu Thr Ser Leu Pro Lys Glu Met Val Ser Tyr Met
           195                 200                 205
   Pro Asn Leu Glu Ser Leu Tyr Leu His Gly Asn Pro Trp Thr Cys Asp
       210                 215                 220
   Cys His Leu Lys Trp Leu Ser Glu Trp Met Gln Gly Asn Pro Asp Ile
   225                 230                 235                 240
   Ile Lys Cys Lys Lys Asp Arg Ser Ser Ser Ser Pro Gln Gln Cys Pro
                   245                 250                 255
   Leu Cys Met Asn Pro Arg Ile Ser Lys Gly Arg Pro Phe Ala Met Val
               260                 265                 270
   Pro Ser Gly Ala Phe Leu Cys Thr Lys Pro Thr Ile Asp Pro Ser Leu
           275                 280                 285
   Lys Ser Lys Ser Leu Val Thr Gln Glu Asp Asn Gly Ser Ala Ser Thr
       290                 295                 300
   Ser Pro Gln Asp Phe Ile Glu Pro Phe Gly Ser Leu Ser Leu Asn Met
   305                 310                 315                 320
   Thr Xaa Xaa Ser Gly Asn Lys Ala Asp Met Val Cys Ser Ile Gln Lys
                   325                 330                 335
   Pro Ser Arg Thr Ser Pro Thr Ala Phe Thr Glu Glu Asn Asp Tyr Ile
               340                 345                 350
   Met Leu Asn Ala Ser Phe Ser Thr Asn Leu Val Cys Ser Val Asp Tyr
           355                 360                 365
   Asn His Ile Gln Pro Val Trp Gln Leu Leu Ala Leu Tyr Ser Asp Ser
       370                 375                 380
   Pro Leu Ile Leu Glu Arg Lys Pro Gln Leu Thr Glu Thr Pro Ser Leu
   385                 390                 395                 400
   Ser Ser Arg Tyr Lys Gln Val Ala Leu Arg Pro Glu Asp Ile Phe Thr
                   405                 410                 415
   Ser Ile Glu Ala Asp Val Arg Ala Asp Pro Phe Trp Phe Gln Gln Glu
               420                 425                 430
   Lys Ile Val Leu Gln Leu Asn Arg Thr Ala Thr Thr Leu Ser Thr Leu
           435                 440                 445
   Gln Ile Gln Phe Ser Thr Asp Ala Gln Ile Ala Leu Pro Arg Ala Glu
       450                 455                 460
   Met Arg Ala Glu Arg Leu Lys Trp Thr Met Ile Leu Met Met Asn Asn
   465                 470                 475                 480
   Pro Lys Leu Glu Arg Thr Val Leu Val Gly Gly Thr Ile Ala Leu Ser
                   485                 490                 495
   Cys Pro Gly Lys Gly Asp Pro Ser Pro His Leu Glu Trp Leu Leu Ala
               500                 505                 510
   Asp Gly Ser Lys Val Arg Ala Pro Tyr Val Ser Glu Asp Gly Arg Ile
           515                 520                 525
   Leu Ile Asp Lys Asn Gly Lys Leu Glu Leu Gln Met Ala Asp Ser Phe
       530                 535                 540
   Asp Ala Gly Leu Tyr His Cys Ile Ser Thr Asn Asp Ala Asp Ala Asp
   545                 550                 555                 560
   Val Leu Thr Tyr Arg Ile Thr Val Val Glu Pro Tyr Gly Glu Ser Thr
                   565                 570                 575
   His Asp Ser Gly Val Gln His Thr Val Val Thr Gly Glu Thr Leu Asp
               580                 585                 590
   Leu Pro Cys Leu Ser Thr Gly Val Pro Asp Ala Ser Ile Ser Trp Ile
           595                 600                 605
   Leu Pro Gly Asn Thr Val Phe Ser Gln Pro Ser Arg Asp Arg Gln Ile
       610                 615                 620
   Leu Asn Asn Gly Thr Leu Arg Ile Leu Gln Val Thr Pro Lys Asp Gln
   625                 630                 635                 640
   Gly His Tyr Gln Cys Val Ala Ala Asn Pro Ser Gly Ala Asp Phe Ser
                   645                 650                 655
   Ser Phe Lys Val Ser Val Gln Lys Lys Gly Gln Arg Met Val Glu His
               660                 665                 670
   Asp Arg Glu Ala Gly Gly Ser Gly Leu Gly Glu Pro Asn Ser Ser Val
           675                 680                 685
   Ser Leu Lys Gln Pro Ala Ser Leu Lys Leu Ser Ala Ser Ala Leu Thr
       690                 695                 700
   Gly Ser Glu Ala Gly Lys Gln Val Ser Gly Val His Arg Lys Asn Lys
   705                 710                 715                 720
   His Arg Asp Leu Ile His Arg Arg Arg Gly Asp Ser Thr Leu Arg Arg
                   725                 730                 735
   Phe Arg Glu His Arg Arg Gln Leu Pro Leu Ser Ala Arg Arg Ile Asp
               740                 745                 750
   Pro Gln Arg Trp Ala Ala Leu Leu Glu Lys Ala Lys Lys Asn Ser Val
           755                 760                 765
   Pro Lys Lys Gln Glu Asn Thr Thr Val Lys Pro Val Pro Leu Ala Val
       770                 775                 780
   Pro Leu Val Glu Leu Thr Asp Glu Glu Lys Asp Ala Ser Gly Met Ile
   785                 790                 795                 800
   Pro Pro Asp Glu Glu Phe Met Val Leu Lys Thr Lys Ala Ser Gly Val
                   805                 810                 815
   Pro Gly Arg Ser Pro Thr Ala Asp Ser Gly Pro Val Asn His Gly Phe
               820                 825                 830
   Met Thr Ser Ile Ala Ser Gly Thr Glu Val Ser Thr Val Asn Pro Gln
           835                 840                 845
   Thr Leu Gln Ser Glu His Leu Pro Asp Phe Lys Leu Phe Ser Val Thr
       850                 855                 860
   Asn Gly Thr Ala Val Thr Lys Ser Met Asn Pro Ser Ile Ala Ser Lys
   865                 870                 875                 880
   Ile Glu Asp Thr Thr Asn Gln Asn Pro Ile Ile Ile Phe Pro Ser Val
                   885                 890                 895
   Ala Glu Ile Arg Asp Ser Ala Gln Ala Gly Arg Ala Ser Ser Gln Ser
               900                 905                 910
   Ala His Pro Val Thr Gly Gly Asn Met Ala Thr Tyr Gly His Thr Asn
           915                 920                 925
   Thr Tyr Ser Ser Phe Thr Ser Lys Ala Ser Thr Val Leu Gln Pro Ile
       930                 935                 940
   Asn Pro Thr Glu Ser Tyr Gly Pro Gln Ile Pro Ile Thr Gly Val Ser
   945                 950                 955                 960
   Arg Pro Ser Ser Ser Asp Ile Ser Ser His Thr Thr Ala Asp Pro Ser
                   965                 970                 975
   Phe Ser Ser His Pro Ser Gly Ser His Thr Thr Ala Ser Ser Leu Phe
               980                 985                 990
   His Ile Pro Arg Asn Asn Asn Thr Gly Asn Phe Pro Leu Ser Arg His
           995                 1000                1005
   Leu Gly Arg Glu Arg Thr Ile Trp Ser Arg Gly Arg Val Lys Asn Pro
      1010                1015                1020
   His Arg Thr Pro Val Leu Arg Arg His Arg His Arg Thr Val Arg Pro
   1025                1030                1035                1040
   Ala Ile Lys Gly Pro Ala Asn Lys Asn Val Ser Gln Val Pro Ala Thr
                   1045                1050                1055
   Glu Tyr Pro Gly Met Cys His Thr Cys Pro Ser Ala Glu Gly Leu Thr
               1060                1065                1070
   Val Ala Thr Ala Ala Leu Ser Val Pro Ser Ser Ser His Ser Ala Leu
           1075                1080                1085
   Pro Lys Thr Asn Asn Val Gly Val Ile Ala Glu Glu Ser Thr Thr Val
      1090                1095                1100
   Val Lys Lys Pro Leu Leu Leu Phe Lys Asp Lys Gln Asn Val Asp Ile
   1105                1110                1115                1120
   Glu Ile Ile Thr Thr Thr Thr Lys Tyr Ser Gly Gly Glu Ser Asn His
                   1125                1130                1135
   Val Ile Pro Thr Glu Ala Ser Met Thr Ser Ala Pro Thr Ser Val Ser
               1140                1145                1150
   Leu Gly Lys Ser Pro Val Asp Asn Ser Gly His Leu Ser Met Pro Gly
           1155                1160                1165
   Thr Ile Gln Thr Gly Lys Asp Ser Val Glu Thr Thr Pro Leu Pro Ser
      1170                1175                1180
   Pro Leu Ser Thr Pro Ser Ile Pro Thr Ser Thr Lys Phe Ser Lys Arg
   1185                1190                1195                1200
   Lys Thr Pro Leu His Gln Ile Phe Val Asn Asn Gln Lys Lys Glu Gly
                   1205                1210                1215
   Met Leu Lys Asn Pro Tyr Gln Phe Gly Leu Gln Lys Asn Pro Ala Ala
               1220                1225                1230
   Lys Leu Pro Lys Ile Ala Pro Leu Leu Pro Thr Gly Gln Ser Ser Pro
           1235                1240                1245
   Ser Asp Ser Thr Thr Leu Leu Thr Ser Pro Pro Pro Ala Leu Ser Thr
      1250                1255                1260
   Thr Met Ala Ala Thr Gln Asn Lys Gly Thr Glu Val Val Ser Gly Ala
   1265                1270                1275                1280
   Arg Ser Leu Ser Ala Gly Lys Lys Gln Pro Phe Thr Asn Ser Ser Pro
                   1285                1290                1295
   Val Leu Pro Ser Thr Ile Ser Lys Arg Ser Asn Thr Leu Asn Phe Leu
               1300                1305                1310
   Ser Thr Glu Thr Pro Thr Val Thr Ser Pro Thr Ala Thr Ala Ser Val
           1315                1320                1325
   Ile Met Ser Glu Thr Gln Arg Thr Arg Ser Lys Glu Ala Lys Asp Gln
      1330                1335                1340
   Ile Lys Gly Pro Arg Lys Asn Arg Asn Asn Ala Asn Thr Thr Pro Arg
   1345                1350                1355                1360
   Gln Val Ser Gly Tyr Ser Ala Tyr Ser Ala Leu Thr Thr Ala Asp Thr
                   1365                1370                1375
   Pro Leu Ala Phe Ser His Ser Pro Arg Gln Asp Asp Gly Gly Asn Val
               1380                1385                1390
   Ser Ala Val Ala Tyr His Ser Thr Thr Ser Leu Leu Ala Ile Thr Glu
           1395                1400                1405
   Leu Phe Glu Lys Tyr Thr Gln Thr Leu Gly Asn Thr Thr Ala Leu Glu
       1410                1415                1420
   Thr Thr Leu Leu Ser Lys Ser Gln Glu Ser Thr Thr Val Lys Arg Ala
   1425                1430                1435                1440
   Ser Asp Thr Pro Pro Pro Leu Leu Ser Ser Gly Ala Pro Pro Val Pro
                   1445                1450                1455
   Thr Pro Ser Pro Pro Pro Phe Thr Lys Gly Val Val Thr Asp Ser Lys
               1460                1465                1470
   Val Thr Ser Ala Phe Gln Met Thr Ser Asn Arg Val Val Thr Ile Tyr
           1475                1480                1485
   Glu Ser Ser Arg His Asn Thr Asp Leu Gln Gln Pro Ser Ala Glu Ala
       1490                1495                1500
   Ser Pro Asn Pro Glu Ile Ile Thr Gly Thr Thr Asp Ser Pro Ser Asn
   1505                1510                1515                1520
   Leu Phe Pro Ser Thr Ser Val Pro Ala Leu Arg Val Asp Lys Pro Gln
                   1525                1530                1535
   Asn Ser Lys Trp Lys Pro Ser Pro Trp Pro Glu His Lys Tyr Gln Leu
               1540                1545                1550
   Lys Ser Tyr Ser Glu Thr Ile Glu Lys Gly Lys Arg Pro Ala Val Ser
           1555                1560                1565
   Met Ser Pro His Leu Ser Leu Pro Glu Ala Ser Thr His Ala Ser His
       1570                1575                1580
   Trp Asn Thr Gln Lys His Ala Glu Lys Ser Val Phe Asp Lys Lys Pro
   1585                1590                1595                1600
   Gly Gln Asn Pro Thr Ser Lys His Leu Pro Tyr Val Ser Leu Pro Lys
                   1605                1610                1615
   Thr Leu Leu Lys Lys Pro Arg Ile Ile Gly Gly Lys Ala Ala Ser Phe
               1620                1625                1630
   Thr Val Pro Ala Asn Ser Asp Val Phe Leu Pro Cys Glu Ala Val Gly
           1635                1640                1645
   Asp Pro Leu Pro Ile Ile His Trp Thr Arg Val Ser Ser Gly Xaa Glu
       1650                1655                1660
   Ile Ser Gln Gly Thr Gln Lys Ser Arg Phe His Val Leu Pro Asn Gly
   1665                1670                1675                1680
   Thr Leu Ser Ile Gln Arg Val Ser Ile Gln Asp Arg Gly Gln Tyr Leu
                   1685                1690                1695
   Cys Ser Ala Phe Asn Pro Leu Gly Val Asp His Phe His Val Ser Leu
               1700                1705                1710
   Ser Val Val Phe Tyr Pro Ala Arg Ile Leu Asp Arg His Val Lys Glu
           1715                1720                1725
   Ile Thr Val His Phe Gly Ser Thr Val Glu Leu Lys Cys Arg Val Glu
       1730                1735                1740
   Gly Met Pro Arg Pro Thr Val Ser Trp Ile Leu Ala Asn Gln Thr Val
   1745                1750                1755                1760
   Val Ser Glu Thr Ala Lys Gly Ser Arg Lys Val Trp Val Thr Pro Asp
                   1765                1770                1775
   Gly Thr Leu Ile Ile Tyr Asn Leu Ser Leu Tyr Asp Arg Gly Phe Tyr
               1780                1785                1790
   Lys Cys Val Ala Ser Asn Pro Ser Gly Gln Asp Ser Leu Leu Val Lys
           1795                1800                1805
   Ile Gln Val Ile Thr Ala Pro Pro Val Ile Ile Glu Gln Lys Arg Gln
       1810                1815                1820
   Ala Ile Val Gly Val Leu Gly Gly Ser Leu Lys Leu Pro Cys Thr Ala
   1825                1830                1835                1840
   Lys Gly Thr Pro Gln Pro Ser Val His Trp Val Leu Tyr Asp Gly Thr
                   1845                1850                1855
   Glu Leu Lys Pro Leu Gln Leu Thr His Ser Arg Phe Phe Leu Tyr Pro
               1860                1865                1870
   Asn Gly Thr Leu Tyr Ile Arg Ser Ile Ala Pro Ser Val Arg Gly Thr
           1875                1880                1885
   Tyr Glu Cys Ile Ala Thr Ser Ser Ser Gly Ser Glu Arg Arg Val Val
       1890                1895                1900
   Ile Leu Thr Val Glu Glu Gly Glu Thr Ile Pro Arg Ile Glu Thr Ala
   1905                1910                1915                1920
   Ser Gln Lys Trp Thr Glu Val Asn Leu Gly Glu Lys Leu Leu Leu Asn
                   1925                1930                1935
   Cys Ser Ala Thr Gly Asp Pro Lys Pro Arg Ile Ile Trp Arg Leu Pro
               1940                1945                1950
   Ser Lys Ala Val Ile Asp Gln Trp His Arg Met Gly Ser Arg Ile His
           1955                1960                1965
   Val Tyr Pro Asn Gly Ser Leu Val Val Gly Ser Val Thr Glu Lys Asp
       1970                1975                1980
   Ala Gly Asp Tyr Leu Cys Val Ala Arg Asn Lys Met Gly Asp Asp Leu
   1985                1990                1995                2000
   Val Leu Met His Val Arg Leu Arg Leu Thr Pro Ala Lys Ile Glu Gln
                   2005                2010                2015
   Lys Gln Tyr Phe Lys Lys Gln Val Leu His Gly Lys Asp Phe Gln Val
               2020                2025                2030
   Asp Cys Lys Ala Ser Gly Ser Pro Val Pro Glu Val Ser Trp Ser Leu
           2035                2040                2045
   Pro Asp Gly Thr Val Leu Asn Asn Val Ala Gln Ala Asp Asp Ser Gly
       2050                2055                2060
   Tyr Arg Thr Lys Arg Tyr Thr Leu Phe His Asn Gly Thr Leu Tyr Phe
   2065                2070                2075                2080
   Asn Asn Val Gly Met Ala Glu Glu Gly Asp Tyr Ile Cys Ser Ala Gln
                   2085                2090                2095
   Asn Thr Leu Gly Lys Asp Glu Met Lys Val His Leu Thr Val Leu Thr
               2100                2105                2110
   Ala Ile Pro Arg Ile Arg Gln Ser Tyr Lys Thr Thr Met Arg Leu Arg
           2115                2120                2125
   Ala Gly Glu Thr Ala Val Leu Asp Cys Glu Val Thr Gly Glu Pro Lys
       2130                2135                2140
   Pro Asn Val Phe Trp Leu Leu Pro Ser Asn Asn Val Ile Ser Phe Ser
   2145                2150                2155                2160
   Asn Asp Arg Phe Thr Phe His Ala Asn Arg Thr Leu Ser Ile His Lys
                   2165                2170                2175
   Val Lys Pro Leu Asp Ser Gly Asp Tyr Val Cys Val Ala Gln Asn Pro
               2180                2185                2190
   Ser Gly Asp Asp Thr Lys Thr Tyr Lys Leu Asp Ile Val Ser Lys Pro
           2195                2200                2205
   Pro Leu Ile Asn Gly Leu Tyr Ala Asn Lys Thr Val Ile Lys Ala Thr
       2210                2215                2220
   Ala Ile Arg His Ser Lys Lys Tyr Phe Asp Cys Arg Ala Asp Gly Ile
   2225                2230                2235                2240
   Pro Ser Ser Gln Val Thr Trp Ile Met Pro Gly Asn Ile Phe Leu Pro
                   2245                2250                2255
   Ala Pro Tyr Phe Gly Ser Arg Val Thr Val His Pro Asn Gly Thr Leu
               2260                2265                2270
   Glu Met Arg Asn Ile Arg Leu Ser Asp Ser Ala Asp Phe Thr Cys Val
           2275                2280                2285
   Val Arg Ser Glu Gly Gly Glu Ser Val Leu Val Val Gln Leu Glu Val
       2290                2295                2300
   Leu Glu Met Leu Arg Arg Pro Thr Phe Arg Asn Pro Phe Asn Glu Lys
   2305                2310                2315                2320
   Val Ile Ala Gln Ala Gly Lys Pro Val Ala Leu Asn Cys Ser Val Asp
                   2325                2330                2335
   Gly Asn Pro Pro Pro Glu Ile Thr Trp Ile Leu Pro Asp Gly Thr Gln
               2340                2345                2350
   Phe Ala Asn Arg Pro His Asn Ser Pro Tyr Leu Met Ala Gly Asn Gly
           2355                2360                2365
   Ser Leu Ile Leu Tyr Lys Ala Thr Arg Asn Lys Ser Gly Lys Tyr Arg
       2370                2375                2380
   Cys Ala Ala Arg Asn Lys Val Gly Tyr Ile Glu Lys Leu Ile Leu Leu
   2385                2390                2395                2400
   Glu Ile Gly Gln Lys Pro Val Ile Leu Thr Tyr Glu Pro Gly Met Val
                   2405                2410                2415
   Lys Ser Val Ser Gly Glu Pro Leu Ser Leu His Cys Val Ser Asp Gly
               2420                2425                2430
   Ile Pro Lys Pro Asn Val Lys Trp Thr Thr Pro Gly Gly His Val Ile
           2435                2440                2445
   Asp Arg Pro Gln Val Asp Gly Lys Tyr Ile Leu His Glu Asn Gly Thr
       2450                2455                2460
   Leu Val Ile Lys Ala Thr Thr Ala His Asp Gln Gly Asn Tyr Ile Cys
   2465                2470                2475                2480
   Arg Ala Gln Asn Ser Val Gly Gln Ala Val Ile Ser Val Ser Val Met
                   2485                2490                2495
   Val Val Ala Tyr Pro Pro Arg Ile Ile Asn Tyr Leu Pro Arg Asn Met
               2500                2505                2510
   Leu Arg Arg Thr Gly Glu Ala Met Gln Leu His Cys Val Ala Leu Gly
           2515                2520                2525
   Ile Pro Lys Pro Lys Val Thr Trp Glu Thr Pro Arg His Ser Leu Leu
       2530                2535                2540
   Ser Lys Ala Thr Ala Arg Lys Pro His Arg Ser Glu Met Leu His Pro
   2545                2550                2555                2560
   Gln Gly Thr Leu Val Ile Gln Asn Leu Gln Thr Ser Asp Ser Gly Val
                   2565                2570                2575
   Tyr Lys Cys Arg Ala Gln Asn Leu Leu Gly Thr Asp Tyr Ala Thr Thr
               2580                2585                2590
   Tyr Ile Gln Val Leu
           2595
<210> 34
   <211> 7679
   <212> DNA
   <213> Rattus sp.
<220>
   <221> CDS
   <222> (1)..(7161)
   <223> coding sequences for inventive nucleic acid molecule 608
<220>
   <221> misc_feature
   <222> (4357)..(4357)
   <223> "n" can be a or g or c or t
<220>
   <221> misc_feature
   <222> (1453)..(1453)
   <223> "Xaa" can be any amino acid
<400> 34
   cta gaa agc ctg tat ttg cat gga aac cca tgg acc tgt gac tgc cat     48
   Leu Glu Ser Leu Tyr Leu His Gly Asn Pro Trp Thr Cys Asp Cys His
     1               5                  10                  15
   tta aag tgg ttg tct gag tgg atg cag gga aac cca gat ata ata aaa     96
   Leu Lys Trp Leu Ser Glu Trp Met Gln Gly Asn Pro Asp Ile Ile Lys
                20                  25                  30
   tgc aag aaa gac aga agc tct tcc agt cct cag caa tgt ccc ctt tgc    144
   Cys Lys Lys Asp Arg Ser Ser Ser Ser Pro Gln Gln Cys Pro Leu Cys
           35                  40                  45
   atg aac ccc agg atc tct aaa ggc aga ccc ttt gct atg gta cca tct    192
   Met Asn Pro Arg Ile Ser Lys Gly Arg Pro Phe Ala Met Val Pro Ser
       50                  55                  60
   gga gct ttc cta tgt aca aag cca acc att gat cca tca ctg aag tca    240
   Gly Ala Phe Leu Cys Thr Lys Pro Thr Ile Asp Pro Ser Leu Lys Ser
   65                  70                  75                  80
   aag agc ctg gtt act cag gag gac aat gga tct gcc tcc acc tca cct    288
   Lys Ser Leu Val Thr Gln Glu Asp Asn Gly Ser Ala Ser Thr Ser Pro
                   85                  90                  95
   caa gat ttc ata gaa ccc ttt ggc tcc ttg tct ttg aac atg aca gac    336
   Gln Asp Phe Ile Glu Pro Phe Gly Ser Leu Ser Leu Asn Met Thr Asp
               100                 105                 110
   ctg tct gga aat aag gcc gac atg gtc tgt agt atc caa aag cca tca    384
   Leu Ser Gly Asn Lys Ala Asp Met Val Cys Ser Ile Gln Lys Pro Ser
           115                 120                 125
   agg aca tca cca act gca ttc act gaa gaa aat gac tac atc atg cta    432
   Arg Thr Ser Pro Thr Ala Phe Thr Glu Glu Asn Asp Tyr Ile Met Leu
       130                 135                 140
   aat gcg tca ttt tcc aca aat ctt gtg tgc agt gta gat tat aat cac    480
   Asn Ala Ser Phe Ser Thr Asn Leu Val Cys Ser Val Asp Tyr Asn His
   145                 150                 155                 160
   atc cag cca gtg tgg caa ctt ctg gct tta tac agt gac tct cct ctg    528
   Ile Gln Pro Val Trp Gln Leu Leu Ala Leu Tyr Ser Asp Ser Pro Leu
                   165                 170                 175
   ata cta gaa agg aag ccc cag ctt acc gag act cct tca ctg tct tct    576
   Ile Leu Glu Arg Lys Pro Gln Leu Thr Glu Thr Pro Ser Leu Ser Ser
               180                 185                 190
   aga tat aaa cag gtg gct ctt agg cct gaa gac att ttt acc agc ata    624
   Arg Tyr Lys Gln Val Ala Leu Arg Pro Glu Asp Ile Phe Thr Ser Ile
           195                 200                 205
   gag gct gat gtc aga gca gac cct ttt tgg ttc caa caa gaa aaa att    672
   Glu Ala Asp Val Arg Ala Asp Pro Phe Trp Phe Gln Gln Glu Lys Ile
       210                 215                 220
   gtc ttg cag ctg aac aga act gcc acc aca ctt agc aca tta cag atc    720
   Val Leu Gln Leu Asn Arg Thr Ala Thr Thr Leu Ser Thr Leu Gln Ile
   225                 230                 235                 240
   cag ttt tcc act gat gct caa atc gct tta cca agg gcg gag atg aga    768
   Gln Phe Ser Thr Asp Ala Gln Ile Ala Leu Pro Arg Ala Glu Met Arg
                   245                 250                 255
   gcg gag aga ctc aaa tgg acc atg atc ctg atg atg aac aat ccc aaa    816
   Ala Glu Arg Leu Lys Trp Thr Met Ile Leu Met Met Asn Asn Pro Lys
               260                 265                 270
   ctg gaa cgc act gtc ctg gtt ggc ggc act att gcc ctg agc tgt cca    864
   Leu Glu Arg Thr Val Leu Val Gly Gly Thr Ile Ala Leu Ser Cys Pro
           275                 280                 285
   ggc aaa ggc gac cct tca cct cac ttg gaa tgg ctt cta gct gat ggg    912
   Gly Lys Gly Asp Pro Ser Pro His Leu Glu Trp Leu Leu Ala Asp Gly
       290                 295                 300
   agt aaa gtg aga gcc cct tac gtt agc gag gat ggg cga atc cta ata    960
   Ser Lys Val Arg Ala Pro Tyr Val Ser Glu Asp Gly Arg Ile Leu Ile
   305                 310                 315                 320
   gac aaa aat ggg aag ttg gaa ctg cag atg gct gac agc ttt gat gca   1008
   Asp Lys Asn Gly Lys Leu Glu Leu Gln Met Ala Asp Ser Phe Asp Ala
                   325                 330                 335
   ggt ctt tac cac tgc ata agc acc aat gat gca gat gcg gat gtt ctc   1056
   Gly Leu Tyr His Cys Ile Ser Thr Asn Asp Ala Asp Ala Asp Val Leu
               340                 345                 350
   aca tac agg ata act gtg gta gag ccc tat gga gaa agc aca cat gac   1104
   Thr Tyr Arg Ile Thr Val Val Glu Pro Tyr Gly Glu Ser Thr His Asp
           355                 360                 365
   agt gga gtc cag cac aca gtg gtt acg ggt gag acg ctc gac ctt cca   1152
   Ser Gly Val Gln His Thr Val Val Thr Gly Glu Thr Leu Asp Leu Pro
       370                 375                 380
   tgc ctt tcc acg ggt gtt cca gat gct tct att agc tgg att ctt cca   1200
   Cys Leu Ser Thr Gly Val Pro Asp Ala Ser Ile Ser Trp Ile Leu Pro
   385                 390                 395                 400
   ggg aac act gtg ttc tct cag cca tca aga gac agg caa att ctt aac   1248
   Gly Asn Thr Val Phe Ser Gln Pro Ser Arg Asp Arg Gln Ile Leu Asn
                   405                 410                 415
   aat ggg acc tta aga ata tta cag gtt acg cca aaa gat caa ggt cat   1296
   Asn Gly Thr Leu Arg Ile Leu Gln Val Thr Pro Lys Asp Gln Gly His
               420                 425                 430
   tac caa tgt gtg gct gcc aac cca tca ggg gcc gac ttt tcc agt ttt   1344
   Tyr Gln Cys Val Ala Ala Asn Pro Ser Gly Ala Asp Phe Ser Ser Phe
           435                 440                 445
   aaa gtt tca gtt caa aag aaa ggc caa agg atg gtt gag cat cac agg   1392
   Lys Val Ser Val Gln Lys Lys Gly Gln Arg Met Val Glu His His Arg
       450                 455                 460
   gag gca ggt gga tct gga ctt gga gaa ccc aac tcc agt gtt tcc ctt   1440
   Glu Ala Gly Gly Ser Gly Leu Gly Glu Pro Asn Ser Ser Val Ser Leu
   465                 470                 475                 480
   aag cag cca gca tct ttg aaa ctc tct gca tca gct ttg aca ggg tca   1488
   Lys Gln Pro Ala Ser Leu Lys Leu Ser Ala Ser Ala Leu Thr Gly Ser
                   485                 490                 495
   gag gct gga aaa caa gtc tcc ggt gta cat agg aag aac aaa cat aga   1536
   Glu Ala Gly Lys Gln Val Ser Gly Val His Arg Lys Asn Lys His Arg
               500                 505                 510
   gac tta ata cat cgg cgg cgt ggg gat tcc acg ctc cgg cga ttc agg   1584
   Asp Leu Ile His Arg Arg Arg Gly Asp Ser Thr Leu Arg Arg Phe Arg
           515                 520                 525
   gag cat agg agg cag ctc cct ctc tct gct cgg aga att gac ccg caa    1632
   Glu His Arg Arg Gln Leu Pro Leu Ser Ala Arg Arg Ile Asp Pro Gln
       530                 535                 540
   cgc tgg gca gca ctt cta gaa aaa gcc aaa aag aat tct gtg cca aaa    1680
   Arg Trp Ala Ala Leu Leu Glu Lys Ala Lys Lys Asn Ser Val Pro Lys
   545                 550                 555                 560
   aag caa gaa aat acc aca gta aag cca gtg cca ctg gct gtt ccc ctc    1728
   Lys Gln Glu Asn Thr Thr Val Lys Pro Val Pro Leu Ala Val Pro Leu
                   565                 570                 575
   gtg gaa ctc act gac gag gaa aag gat gcc tct ggc atg att cct cca    1776
   Val Glu Leu Thr Asp Glu Glu Lys Asp Ala Ser Gly Met Ile Pro Pro
               580                 585                 590
   gat gaa gaa ttc atg gtt ctg aaa act aag gct tct ggt gtc cca gga    1824
   Asp Glu Glu Phe Met Val Leu Lys Thr Lys Ala Ser Gly Val Pro Gly
           595                 600                 605
   agg tca cca act gct gac tct gga cca gta aat cat ggt ttt atg acg    1872
   Arg Ser Pro Thr Ala Asp Ser Gly Pro Val Asn His Gly Phe Met Thr
       610                 615                 620
   agt ata gct tct ggc aca gaa gtc tca act gtg aat cca caa aca cta    1920
   Ser Ile Ala Ser Gly Thr Glu Val Ser Thr Val Asn Pro Gln Thr Leu
   625                 630                 635                 640
   caa tct gag cac ctt cct gat ttc aaa tta ttt agt gta aca aac ggt    1968
   Gln Ser Glu His Leu Pro Asp Phe Lys Leu Phe Ser Val Thr Asn Gly
                   645                 650                 655
   aca gct gtg aca aag agt atg aac cca tcc ata gca agc aaa ata gaa    2016
   Thr Ala Val Thr Lys Ser Met Asn Pro Ser Ile Ala Ser Lys Ile Glu
               660                 665                 670
   gat aca acc aac caa aac cca atc att atc ttt cca tca gta gct gaa    2064
   Asp Thr Thr Asn Gln Asn Pro Ile Ile Ile Phe Pro Ser Val Ala Glu
           675                 680                 685
   att cga gat tct gct cag gca gga aga gca tct tcc caa agt gca cac    2112
   Ile Arg Asp Ser Ala Gln Ala Gly Arg Ala Ser Ser Gln Ser Ala His
       690                 695                 700
   cct gta aca ggg gga aac atg gct acc tat ggc cat acc aac aca tat    2160
   Pro Val Thr Gly Gly Asn Met Ala Thr Tyr Gly His Thr Asn Thr Tyr
   705                 710                 715                 720
   agt agc ttt acc agc aaa gcc agt aca gtc ttg cag cca ata aat cca    2208
   Ser Ser Phe Thr Ser Lys Ala Ser Thr Val Leu Gln Pro Ile Asn Pro
                   725                 730                 735
   aca gaa agt tat gga cct cag ata cct att aca gga gtc agc aga cct    2256
   Thr Glu Ser Tyr Gly Pro Gln Ile Pro Ile Thr Gly Val Ser Arg Pro
               740                 745                 750
   agc agt agt gac atc tct tct cac act act gca gac cct agc ttc tcc    2304
   Ser Ser Ser Asp Ile Ser Ser His Thr Thr Ala Asp Pro Ser Phe Ser
           755                 760                 765
   agt cac cct tca ggt tca cac acc act gcc tcg tct tta ttt cac att   2352
   Ser His Pro Ser Gly Ser His Thr Thr Ala Ser Ser Leu Phe His Ile
       770                 775                 780
   cct aga aac aac aat aca ggt aac ttc ccc ttg tcc agg cac ttg gga   2400
   Pro Arg Asn Asn Asn Thr Gly Asn Phe Pro Leu Ser Arg His Leu Gly
   785                 790                 795                 800
   aga gag agg aca att tgg agc aga ggg aga gtt aaa aac cca cat aga   2448
   Arg Glu Arg Thr Ile Trp Ser Arg Gly Arg Val Lys Asn Pro His Arg
                   805                 810                 815
   acc cca gtt ctc cga cgg cat aga cac agg act gtg agg cca gca atc   2496
   Thr Pro Val Leu Arg Arg His Arg His Arg Thr Val Arg Pro Ala Ile
               820                 825                 830
   aag gga cct gct aac aaa aat gtg agc caa gtt cca gcc aca gag tac   2544
   Lys Gly Pro Ala Asn Lys Asn Val Ser Gln Val Pro Ala Thr Glu Tyr
           835                 840                 845
   cct ggg atg tgc cac aca tgt cct tcc gca gag ggg ctc aca gtg gct   2592
   Pro Gly Met Cys His Thr Cys Pro Ser Ala Glu Gly Leu Thr Val Ala
       850                 855                 860
   act gca gca ctg tca gtt cca agt tca tcc cac agt gcc ctc ccc aaa   2640
   Thr Ala Ala Leu Ser Val Pro Ser Ser Ser His Ser Ala Leu Pro Lys
   865                 870                 875                 880
   act aat aat gtt ggg gtc ata gca gaa gag tct acc act gtg gtc aag   2688
   Thr Asn Asn Val Gly Val Ile Ala Glu Glu Ser Thr Thr Val Val Lys
                   885                 890                 895
   aaa cca ctg tta cta ttt aag gac aaa caa aat gta gat att gag ata   2736
   Lys Pro Leu Leu Leu Phe Lys Asp Lys Gln Asn Val Asp Ile Glu Ile
               900                 905                 910
   ata aca acc act aca aaa tat tcc gga ggg gaa agt aac cac gtg att   2784
   Ile Thr Thr Thr Thr Lys Tyr Ser Gly Gly Glu Ser Asn His Val Ile
           915                 920                 925
   cct acg gaa gca agc atg act tct gct cca aca tct gta tcc ctg ggg   2832
   Pro Thr Glu Ala Ser Met Thr Ser Ala Pro Thr Ser Val Ser Leu Gly
       930                 935                 940
   aaa tct cct gta gac aat agt ggt cac ctg agc atg cct ggg acc atc   2880
   Lys Ser Pro Val Asp Asn Ser Gly His Leu Ser Met Pro Gly Thr Ile
   945                 950                 955                 960
   caa act ggg aaa gat tca gtg gaa aca aca cca ctt ccc agc ccc ctc   2928
   Gln Thr Gly Lys Asp Ser Val Glu Thr Thr Pro Leu Pro Ser Pro Leu
                   965                 970                 975
   agc aca ccc tca ata cca aca agc aca aaa ttc tca aag agg aaa act   2976
   Ser Thr Pro Ser Ile Pro Thr Ser Thr Lys Phe Ser Lys Arg Lys Thr
               980                 985                 990
   ccc ttg cac cag atc ttt gta aat aac cag aag aag gag ggg atg tta   3024
   Pro Leu His Gln Ile Phe Val Asn Asn Gln Lys Lys Glu Gly Met Leu
           995                 1000                1005
   aag aat cca tat caa ttc ggt tta caa aag aac cca gcc gca aag ctt   3072
   Lys Asn Pro Tyr Gln Phe Gly Leu Gln Lys Asn Pro Ala Ala Lys Leu
      1010                1015                1020
   ccc aaa ata gct cct ctt tta ccc aca ggt cag agt tcc ccc tca gat    3120
   Pro Lys Ile Ala Pro Leu Leu Pro Thr Gly Gln Ser Ser Pro Ser Asp
   1025                1030                1035                1040
   tct aca act ctc ttg aca agt ccg cca cca gct ctg tct aca aca atg    3168
   Ser Thr Thr Leu Leu Thr Ser Pro Pro Pro Ala Leu Ser Thr Thr Met
                   1045                1050                1055
   gct gcc act cag aac aag ggc act gaa gta gta tca ggt gcc aga agt    3216
   Ala Ala Thr Gln Asn Lys Gly Thr Glu Val Val Ser Gly Ala Arg Ser
               1060                1065                1070
   ctc tca gca ggg aag aag cag ccc ttc acc aac tcc tct cca gtg ctt    3264
   Leu Ser Ala Gly Lys Lys Gln Pro Phe Thr Asn Ser Ser Pro Val Leu
           1075                1080                1085
   cct agc acc ata agc aag aga tct aat aca tta aac ttc ttg tca acg    3312
   Pro Ser Thr Ile Ser Lys Arg Ser Asn Thr Leu Asn Phe Leu Ser Thr
       1090                1095                1100
   gaa acc ccc aca gtg aca agt cct act gct act gca tct gtc att atg    3360
   Glu Thr Pro Thr Val Thr Ser Pro Thr Ala Thr Ala Ser Val Ile Met
   1105                1110                1115                1120
   tct gaa acc caa cga aca aga tcc aaa gaa gca aaa gac caa ata aag    3408
   Ser Glu Thr Gln Arg Thr Arg Ser Lys Glu Ala Lys Asp Gln Ile Lys
                   1125                1130                1135
   ggg cct cgg aag aac aga aac aac gca aac acc acc ccc agg cag gtt    3456
   Gly Pro Arg Lys Asn Arg Asn Asn Ala Asn Thr Thr Pro Arg Gln Val
               1140                1145                1150
   tct ggc tat agt gca tac tca gct cta aca aca gct gat acc ccc ttg    3504
   Ser Gly Tyr Ser Ala Tyr Ser Ala Leu Thr Thr Ala Asp Thr Pro Leu
           1155                1160                1165
   gct ttc agt cat tcc cca cga caa gat gat ggt gga aat gta agt gca    3552
   Ala Phe Ser His Ser Pro Arg Gln Asp Asp Gly Gly Asn Val Ser Ala
       1170                1175                1180
   gtt gct tat cac tca aca acc tct ctt ctg gcc ata act gaa ctg ttt    3600
   Val Ala Tyr His Ser Thr Thr Ser Leu Leu Ala Ile Thr Glu Leu Phe
   1185                1190                1195                1200
   gag aag tac acc cag act ttg gga aat aca aca gct ttg gaa aca acg    3648
   Glu Lys Tyr Thr Gln Thr Leu Gly Asn Thr Thr Ala Leu Glu Thr Thr
                   1205                1210                1215
   ttg ttg agc aaa tca cag gag agt acc aca gtg aaa aga gcc tca gac    3696
   Leu Leu Ser Lys Ser Gln Glu Ser Thr Thr Val Lys Arg Ala Ser Asp
               1220                1225                1230
   aca cca cca cca ctc ctc agc agt ggg gcg ccc cca gtg ccc act cct    3744
   Thr Pro Pro Pro Leu Leu Ser Ser Gly Ala Pro Pro Val Pro Thr Pro
           1235                1240                1245
   tcc cca cct cct ttt act aag ggt gtg gtt aca gac agc aaa gtc aca    3792
   Ser Pro Pro Pro Phe Thr Lys Gly Val Val Thr Asp Ser Lys Val Thr
   1250 1255 1260
   tca gct ttc cag atg acg tca aat aga gtg gtc acc ata tat gaa tct    3840
   Ser Ala Phe Gln Met Thr Ser Asn Arg Val Val Thr Ile Tyr Glu Ser
   1265                1270                1275                1280
   tca agg cac aat aca gat ctg cag caa ccc tca gca gag gct agc ccc    3888
   Ser Arg His Asn Thr Asp Leu Gln Gln Pro Ser Ala Glu Ala Ser Pro
                   1285                1290                1295
   aat cct gag atc ata act gga acc act gac tct ccc tct aat ctg ttt    3936
   Asn Pro Glu Ile Ile Thr Gly Thr Thr Asp Ser Pro Ser Asn Leu Phe
               1300                1305                1310
   cca tcc act tct gtg cca gca cta agg gta gat aaa cca cag aat tct    3984
   Pro Ser Thr Ser Val Pro Ala Leu Arg Val Asp Lys Pro Gln Asn Ser
           1315                1320                1325
   aaa tgg aag ccc tct ccc tgg cca gaa cac aaa tat cag ctc aag tca    4032
   Lys Trp Lys Pro Ser Pro Trp Pro Glu His Lys Tyr Gln Leu Lys Ser
       1330                1335                1340
   tac tcc gaa acc att gag aag ggc aaa agg cca gca gta agc atg tcc    4080
   Tyr Ser Glu Thr Ile Glu Lys Gly Lys Arg Pro Ala Val Ser Met Ser
   1345                1350                1355                1360
   ccc cac ctc agc ctt cca gag gcc agc act cat gcc tca cac tgg aat    4128
   Pro His Leu Ser Leu Pro Glu Ala Ser Thr His Ala Ser His Trp Asn
                   1365                1370                1375
   aca cag aag cat gca gaa aag agt gtt ttt gat aag aaa cct ggt caa    4176
   Thr Gln Lys His Ala Glu Lys Ser Val Phe Asp Lys Lys Pro Gly Gln
               1380                1385                1390
   aac cca act tcc aaa cat ctg cct tac gtc tct cta cct aag act cta    4224
   Asn Pro Thr Ser Lys His Leu Pro Tyr Val Ser Leu Pro Lys Thr Leu
           1395                1400                1405
   ttg aaa aag cca aga ata att gga gga aag gct gca agc ttt aca gtt    4272
   Leu Lys Lys Pro Arg Ile Ile Gly Gly Lys Ala Ala Ser Phe Thr Val
       1410                1415                1420
   cca gct aat tca gac gtt ttt ctt cct tgt gag gct gtt gga gac cca    4320
   Pro Ala Asn Ser Asp Val Phe Leu Pro Cys Glu Ala Val Gly Asp Pro
   1425                1430                1435                1440
   ctg ccc atc atc cac tgg acc aga gtt tca tca gga ntt gaa ata tcc    4368
   Leu Pro Ile Ile His Trp Thr Arg Val Ser Ser Gly Xaa Glu Ile Ser
                   1445                1450                1455
   caa ggg aca cag aaa agc cgg ttc cac gtg ctt ccc aat ggc acc ttg    4416
   Gln Gly Thr Gln Lys Ser Arg Phe His Val Leu Pro Asn Gly Thr Leu
               1460                1465                1470
   tcc atc cag agg gtc agt att cag gac cgt gga cag tac ctg tgc tct    4464
   Ser Ile Gln Arg Val Ser Ile Gln Asp Arg Gly Gln Tyr Leu Cys Ser
           1475                1480                1485
   gca ttt aat cca ctg ggc gta gac cat ttt cat gtc tct ttg tct gtg    4512
   Ala Phe Asn Pro Leu Gly Val Asp His Phe His Val Ser Leu Ser Val
       1490                1495                1500
   gtt ttt tac ccg gca agg att ttg gac aga cat gtc aag gag atc aca    4560
   Val Phe Tyr Pro Ala Arg Ile Leu Asp Arg His Val Lys Glu Ile Thr
   1505                1510                1515                1520
   gtt cac ttt gga agt act gtg gaa cta aag tgc aga gtg gag ggt atg    4608
   Val His Phe Gly Ser Thr Val Glu Leu Lys Cys Arg Val Glu Gly Met
                   1525                1530                1535
   ccg agg cct acg gtt tcc tgg ata ctt gca aac caa acg gtg gtc tca    4656
   Pro Arg Pro Thr Val Ser Trp Ile Leu Ala Asn Gln Thr Val Val Ser
               1540                1545                1550
   gaa acg gcc aag gga agc aga aag gtg tgg gta aca cct gat gga aca    4704
   Glu Thr Ala Lys Gly Ser Arg Lys Val Trp Val Thr Pro Asp Gly Thr
           1555                1560                1565
   ttg atc atc tat aat ctg agt ctt tat gat cgt ggt ttt tac aag tgt    4752
   Leu Ile Ile Tyr Asn Leu Ser Leu Tyr Asp Arg Gly Phe Tyr Lys Cys
       1570                1575                1580
   gtg gcc agc aac cca tct ggc cag gat tca ctg ttg gtt aag ata caa    4800
   Val Ala Ser Asn Pro Ser Gly Gln Asp Ser Leu Leu Val Lys Ile Gln
   1585                1590                1595                1600
   gtc atc aca gct ccc cct gtc att ata gag caa aag agg caa gcc atc    4848
   Val Ile Thr Ala Pro Pro Val Ile Ile Glu Gln Lys Arg Gln Ala Ile
                   1605                1610                1615
   gtt ggg gtt tta ggt gga agt ttg aaa ctg ccc tgc act gca aaa gga    4896
   Val Gly Val Leu Gly Gly Ser Leu Lys Leu Pro Cys Thr Ala Lys Gly
               1620                1625                1630
   act ccc cag cct agt gtt cac tgg gtc ctt tat gat ggg act gaa cta    4944
   Thr Pro Gln Pro Ser Val His Trp Val Leu Tyr Asp Gly Thr Glu Leu
           1635                1640                1645
   aaa cca ttg cag ttg act cat tcc aga ttt ttc ttg tat cca aat gga    4992
   Lys Pro Leu Gln Leu Thr His Ser Arg Phe Phe Leu Tyr Pro Asn Gly
       1650                1655                1660
   act ctg tat ata aga agc atc gct cct tca gtg agg ggc act tat gag    5040
   Thr Leu Tyr Ile Arg Ser Ile Ala Pro Ser Val Arg Gly Thr Tyr Glu
   1665                1670                1675                1680
   tgc att gcc acc agc tcc tca ggc tca gag aga agg gta gtg att ctt    5088
   Cys Ile Ala Thr Ser Ser Ser Gly Ser Glu Arg Arg Val Val Ile Leu
                   1685                1690                1695
   act gtg gaa gag gga gag aca atc ccc agg ata gaa act gcc tct cag    5136
   Thr Val Glu Glu Gly Glu Thr Ile Pro Arg Ile Glu Thr Ala Ser Gln
               1700                1705                1710
   aaa tgg act gag gtg aat ttg ggt gag aaa tta cta ctg aac tgc tca    5184
   Lys Trp Thr Glu Val Asn Leu Gly Glu Lys Leu Leu Leu Asn Cys Ser
           1715                1720                1725
   gct act ggg gat cca aag cct aga ata atc tgg agg ctg cca tcc aag    5232
   Ala Thr Gly Asp Pro Lys Pro Arg Ile Ile Trp Arg Leu Pro Ser Lys
       1730                1735                1740
   gct gtc atc gac cag tgg cac aga atg ggc agc cga atc cac gtc tac   5280
   Ala Val Ile Asp Gln Trp His Arg Met Gly Ser Arg Ile His Val Tyr
   1745                1750                1755                1760
   cca aat gga tcc ttg gtg gtt ggg tca gtg acg gaa aaa gac gct ggt   5328
   Pro Asn Gly Ser Leu Val Val Gly Ser Val Thr Glu Lys Asp Ala Gly
                   1765                1770                1775
   gac tac tta tgt gtg gca aga aac aaa atg gga gat gac cta gtc ctg   5376
   Asp Tyr Leu Cys Val Ala Arg Asn Lys Met Gly Asp Asp Leu Val Leu
               1780                1785                1790
   atg cat gtc cgc ctg aga ttg aca cct gcc aaa att gaa cag aag cag   5424
   Met His Val Arg Leu Arg Leu Thr Pro Ala Lys Ile Glu Gln Lys Gln
           1795                1800                1805
   tat ttt aag aag caa gtg ctc cat ggg aaa gat ttc caa gtt gac tgc   5472
   Tyr Phe Lys Lys Gln Val Leu His Gly Lys Asp Phe Gln Val Asp Cys
       1810                1815                1820
   aag gcc tct ggc tcc cct gtg cct gag gta tcc tgg agt ttg cct gat   5520
   Lys Ala Ser Gly Ser Pro Val Pro Glu Val Ser Trp Ser Leu Pro Asp
   1825                1830                1835                1840
   ggg aca gtg ctc aac aat gta gcc caa gct gat gac agt ggc tat agg   5568
   Gly Thr Val Leu Asn Asn Val Ala Gln Ala Asp Asp Ser Gly Tyr Arg
                   1845                1850                1855
   acc aag agg tac acc ctt ttc cac aat gga acc ttg tat ttc aac aac   5616
   Thr Lys Arg Tyr Thr Leu Phe His Asn Gly Thr Leu Tyr Phe Asn Asn
               1860                1865                1870
   gtt ggg atg gca gag gaa gga gat tat atc tgc tct gcc cag aac acc   5664
   Val Gly Met Ala Glu Glu Gly Asp Tyr Ile Cys Ser Ala Gln Asn Thr
           1875                1880                1885
   tta ggg aaa gat gag atg aaa gtc cac cta aca gtt cta aca gcc atc   5712
   Leu Gly Lys Asp Glu Met Lys Val His Leu Thr Val Leu Thr Ala Ile
       1890                1895                1900
   cca cgg ata agg caa agc tac aag acc acc atg agg ctc agg gct gga   5760
   Pro Arg Ile Arg Gln Ser Tyr Lys Thr Thr Met Arg Leu Arg Ala Gly
   1905                1910                1915                1920
   gaa aca gct gtc ctt gac tgc gag gtc act ggg gaa ccg aag ccc aat   5808
   Glu Thr Ala Val Leu Asp Cys Glu Val Thr Gly Glu Pro Lys Pro Asn
                   1925                1930                1935
   gta ttt tgg ttg ctg cct tcc aac aat gtc att tca ttc tcc aat gac   5856
   Val Phe Trp Leu Leu Pro Ser Asn Asn Val Ile Ser Phe Ser Asn Asp
               1940                1945                1950
   agg ttc aca ttt cat gcc aat aga act ttg tcc atc cat aaa gtg aaa   5904
   Arg Phe Thr Phe His Ala Asn Arg Thr Leu Ser Ile His Lys Val Lys
           1955                1960                1965
   cca ctt gac tct ggg gac tat gtg tgc gta gct cag aat cct agt ggg   5952
   Pro Leu Asp Ser Gly Asp Tyr Val Cys Val Ala Gln Asn Pro Ser Gly
       1970                1975                1980
   gat gac act aag aca tac aaa ctg gac att gtc tct aaa cct cca tta   6000
   Asp Asp Thr Lys Thr Tyr Lys Leu Asp Ile Val Ser Lys Pro Pro Leu
   1985                1990                1995                2000
   atc aat ggc ctg tat gca aac aag act gtt att aaa gcc aca gcc att     6048
   Ile Asn Gly Leu Tyr Ala Asn Lys Thr Val Ile Lys Ala Thr Ala Ile
               2005                2010                2015
   cgg cac tcc aaa aaa tac ttt gac tgc aga gca gat ggg atc cca tct     6096
   Arg His Ser Lys Lys Tyr Phe Asp Cys Arg Ala Asp Gly Ile Pro Ser
           2020                2025                2030
   tcc cag gtc acg tgg att atg cca ggc aat att ttc ctc cca gct cca     6144
   Ser Gln Val Thr Trp Ile Met Pro Gly Asn Ile Phe Leu Pro Ala Pro
       2035                2040                2045
   tac ttt gga agc aga gtc acg gtc cat cca aat gga acc ttg gag atg     6192
   Tyr Phe Gly Ser Arg Val Thr Val His Pro Asn Gly Thr Leu Glu Met
      2050                2055                2060
   agg aac atc cgg ctt tct gac tct gcg gac ttc acc tgt gtg gtt cgg     6240
   Arg Asn Ile Arg Leu Ser Asp Ser Ala Asp Phe Thr Cys Val Val Arg
   2065                2070                2075                2080
   agc gag gga gga gag agt gtg ttg gta gtg cag tta gaa gtc cta gaa     6288
   Ser Glu Gly Gly Glu Ser Val Leu Val Val Gln Leu Glu Val Leu Glu
               2085                2090                2095
   atg ctg aga aga cca aca ttc aga aac cca ttc aac gaa aaa gtc atc     6336
   Met Leu Arg Arg Pro Thr Phe Arg Asn Pro Phe Asn Glu Lys Val Ile
           2100                2105                2110
   gcc caa gct ggc aag ccc gta gca ctg aac tgc tct gtg gat ggg aac     6384
   Ala Gln Ala Gly Lys Pro Val Ala Leu Asn Cys Ser Val Asp Gly Asn
       2115                2120                2125
   ccc cca cct gaa att acc tgg atc tta cct gac ggc aca cag ttt gct     6432
   Pro Pro Pro Glu Ile Thr Trp Ile Leu Pro Asp Gly Thr Gln Phe Ala
      2130                2135                2140
   aac aga cca cac aat tcc ccg tat ctg atg gca ggc aat ggc tct ctc     6480
   Asn Arg Pro His Asn Ser Pro Tyr Leu Met Ala Gly Asn Gly Ser Leu
   2145                2150                2155                2160
   atc ctt tac aaa gca act cgg aac aag tca ggg aag tat cgc tgt gca     6528
   Ile Leu Tyr Lys Ala Thr Arg Asn Lys Ser Gly Lys Tyr Arg Cys Ala
               2165                2170                2175
   gcc agg aat aag gtt ggc tac atc gag aaa ctc atc ctg tta gag att     6576
   Ala Arg Asn Lys Val Gly Tyr Ile Glu Lys Leu Ile Leu Leu Glu Ile
           2180                2185                2190
   ggg cag aag cca gtc att ctg aca tac gaa cca ggg atg gtg aag agc     6624
   Gly Gln Lys Pro Val Ile Leu Thr Tyr Glu Pro Gly Met Val Lys Ser
       2195                2200                2205
   gtc agt ggg gaa ccg tta tca ctg cat tgt gtg tct gat ggg atc ccc     6672
   Val Ser Gly Glu Pro Leu Ser Leu His Cys Val Ser Asp Gly Ile Pro
      2210                2215                2220
   aag cca aat gtc aag tgg act aca ccg ggt ggc cat gta atc gac agg     6720
   Lys Pro Asn Val Lys Trp Thr Thr Pro Gly Gly His Val Ile Asp Arg
   2225                2230                2235                2240
   cct caa gtg gat gga aaa tac ata ctg cat gaa aat ggc acg ctg gtc   6768
   Pro Gln Val Asp Gly Lys Tyr Ile Leu His Glu Asn Gly Thr Leu Val
               2245                2250                2255
   atc aaa gca aca aca gct cac gac caa gga aat tat atc tgt agg gct   6816
   Ile Lys Ala Thr Thr Ala His Asp Gln Gly Asn Tyr Ile Cys Arg Ala
           2260                2265                2270
   caa aac agt gtt ggc cag gca gtt att agc gtg tca gtg atg gtt gtg   6864
   Gln Asn Ser Val Gly Gln Ala Val Ile Ser Val Ser Val Met Val Val
       2275                2280                2285
   gcc tac cct ccc cga atc ata aac tac cta ccc agg aac atg ctc agg   6912
   Ala Tyr Pro Pro Arg Ile Ile Asn Tyr Leu Pro Arg Asn Met Leu Arg
   2290                2295                2300
   agg aca ggg gaa gcc atg cag ctc cac tgt gtg gcc ttg gga atc ccc   6960
   Arg Thr Gly Glu Ala Met Gln Leu His Cys Val Ala Leu Gly Ile Pro
   2305                2310                2315                2320
   aag cca aaa gtc acc tgg gag acg cca aga cac tcc ctg ctc tca aaa   7008
   Lys Pro Lys Val Thr Trp Glu Thr Pro Arg His Ser Leu Leu Ser Lys
               2325                2330                2335
   gca aca gca aga aaa ccc cat aga agt gag atg ctt cac cca caa ggt   7056
   Ala Thr Ala Arg Lys Pro His Arg Ser Glu Met Leu His Pro Gln Gly
           2340                2345                2350
   acg ctg gtc att cag aat ctc caa acc tcg gat tcc gga gtc tat aag   7104
   Thr Leu Val Ile Gln Asn Leu Gln Thr Ser Asp Ser Gly Val Tyr Lys
       2355                2360                2365
   tgc aga gct cag aac cta ctt ggg act gat tac gca aca act tac atc   7152
   Cys Arg Ala Gln Asn Leu Leu Gly Thr Asp Tyr Ala Thr Thr Tyr Ile
   2370                2375                2380
   cag gta ctc tgacaggaag ggggagacta aaattcaaca gaagtccaca          7201
   Gln Val Leu
   2385
   tccacagggt ttattttttg gaagaagttt aatcaaaggc agccataggc atgtaaatga 7261
   gtctgaatac atttacagta ttaaatttac aatggacatg cgatgagact tgtaaatgaa 7321
   agcattgtga actgaaaccg agtctctgtg gatctcaaag caaactctta acttaaggca 7381
   ctttgatttt gccaacaaat aataacaaac attaagagaa aaaaatgatc cactacgaaa 7441
   taacaaacgg ctaatgcacc tgaattctca gtaaaaagac ctttctctcg ctaacagttg 7501
   ccagcagcct cgtgtctgtt tcctaccaat gtcacaaaca tcgcacacag ggtgaatgga 7561
   gtcaacggga aagattaagt ttgcggtctg tgtaaatctc aatgtacaaa tattctgtcn 7621
   ctggtttata aacattttga taaaaccgaa aaaaaaaaaa aaaaaaaaaa aaaaaaaa   7679
<210> 35
   <211> 2387
   <212> PRT
   <213> Rattus sp.
<220>
   <221> misc_feature
   <222> (1453)..(1453)
   <223> "Xaa" can be any amino acid
<400> 35
   Leu Glu Ser Leu Tyr Leu His Gly Asn Pro Trp Thr Cys Asp Cys His
    1               5                  10                  15
   Leu Lys Trp Leu Ser Glu Trp Met Gln Gly Asn Pro Asp Ile Ile Lys
               20                  25                  30
   Cys Lys Lys Asp Arg Ser Ser Ser Ser Pro Gln Gln Cys Pro Leu Cys
           35                  40                  45
   Met Asn Pro Arg Ile Ser Lys Gly Arg Pro Phe Ala Met Val Pro Ser
       50                  55                  60
   Gly Ala Phe Leu Cys Thr Lys Pro Thr Ile Asp Pro Ser Leu Lys Ser
   65                  70                  75                  80
   Lys Ser Leu Val Thr Gln Glu Asp Asn Gly Ser Ala Ser Thr Ser Pro
                   85                  90                  95
   Gln Asp Phe Ile Glu Pro Phe Gly Ser Leu Ser Leu Asn Met Thr Asp
               100                 105                 110
   Leu Ser Gly Asn Lys Ala Asp Met Val Cys Ser Ile Gln Lys Pro Ser
           115                 120                 125
   Arg Thr Ser Pro Thr Ala Phe Thr Glu Glu Asn Asp Tyr Ile Met Leu
       130                 135                 140
   Asn Ala Ser Phe Ser Thr Asn Leu Val Cys Ser Val Asp Tyr Asn His
   145                 150                 155                 160
   Ile Gln Pro Val Trp Gln Leu Leu Ala Leu Tyr Ser Asp Ser Pro Leu
                   165                 170                 175
   Ile Leu Glu Arg Lys Pro Gln Leu Thr Glu Thr Pro Ser Leu Ser Ser
               180                 185                 190
   Arg Tyr Lys Gln Val Ala Leu Arg Pro Glu Asp Ile Phe Thr Ser Ile
           195                 200                 205
   Glu Ala Asp Val Arg Ala Asp Pro Phe Trp Phe Gln Gln Glu Lys Ile
       210                 215                 220
   Val Leu Gln Leu Asn Arg Thr Ala Thr Thr Leu Ser Thr Leu Gln Ile
   225                 230                 235                 240
   Gln Phe Ser Thr Asp Ala Gln Ile Ala Leu Pro Arg Ala Glu Met Arg
                   245                 250                 255
   Ala Glu Arg Leu Lys Trp Thr Met Ile Leu Met Met Asn Asn Pro Lys
               260                 265                 270
   Leu Glu Arg Thr Val Leu Val Gly Gly Thr Ile Ala Leu Ser Cys Pro
           275                 280                 285
   Gly Lys Gly Asp Pro Ser Pro His Leu Glu Trp Leu Leu Ala Asp Gly
       290                 295                 300
   Ser Lys Val Arg Ala Pro Tyr Val Ser Glu Asp Gly Arg Ile Leu Ile
   305                 310                 315                 320
   Asp Lys Asn Gly Lys Leu Glu Leu Gln Met Ala Asp Ser Phe Asp Ala
                   325                 330                 335
   Gly Leu Tyr His Cys Ile Ser Thr Asn Asp Ala Asp Ala Asp Val Leu
               340                 345                 350
   Thr Tyr Arg Ile Thr Val Val Glu Pro Tyr Gly Glu Ser Thr His Asp
           355                 360                 365
   Ser Gly Val Gln His Thr Val Val Thr Gly Glu Thr Leu Asp Leu Pro
       370                 375                 380
   Cys Leu Ser Thr Gly Val Pro Asp Ala Ser Ile Ser Trp Ile Leu Pro
   385                 390                 395                 400
   Gly Asn Thr Val Phe Ser Gln Pro Ser Arg Asp Arg Gln Ile Leu Asn
                   405                 410                 415
   Asn Gly Thr Leu Arg Ile Leu Gln Val Thr Pro Lys Asp Gln Gly His
               420                 425                 430
   Tyr Gln Cys Val Ala Ala Asn Pro Ser Gly Ala Asp Phe Ser Ser Phe
           435                 440                 445
   Lys Val Ser Val Gln Lys Lys Gly Gln Arg Met Val Glu His His Arg
       450                 455                 460
   Glu Ala Gly Gly Ser Gly Leu Gly Glu Pro Asn Ser Ser Val Ser Leu
   465                 470                 475                 480
   Lys Gln Pro Ala Ser Leu Lys Leu Ser Ala Ser Ala Leu Thr Gly Ser
                   485                 490                 495
   Glu Ala Gly Lys Gln Val Ser Gly Val His Arg Lys Asn Lys His Arg
               500                 505                 510
   Asp Leu Ile His Arg Arg Arg Gly Asp Ser Thr Leu Arg Arg Phe Arg
           515                 520                 525
   Glu His Arg Arg Gln Leu Pro Leu Ser Ala Arg Arg Ile Asp Pro Gln
       530                 535                 540
   Arg Trp Ala Ala Leu Leu Glu Lys Ala Lys Lys Asn Ser Val Pro Lys
   545                 550                 555                 560
   Lys Gln Glu Asn Thr Thr Val Lys Pro Val Pro Leu Ala Val Pro Leu
                   565                 570                 575
   Val Glu Leu Thr Asp Glu Glu Lys Asp Ala Ser Gly Met Ile Pro Pro
               580                 585                 590
   Asp Glu Glu Phe Met Val Leu Lys Thr Lys Ala Ser Gly Val Pro Gly
           595                 600                 605
   Arg Ser Pro Thr Ala Asp Ser Gly Pro Val Asn His Gly Phe Met Thr
       610                 615                 620
   Ser Ile Ala Ser Gly Thr Glu Val Ser Thr Val Asn Pro Gln Thr Leu
   625                 630                 635                 640
   Gln Ser Glu His Leu Pro Asp Phe Lys Leu Phe Ser Val Thr Asn Gly
                   645                 650                 655
   Thr Ala Val Thr Lys Ser Met Asn Pro Ser Ile Ala Ser Lys Ile Glu
               660                 665                 670
   Asp Thr Thr Asn Gln Asn Pro Ile Ile Ile Phe Pro Ser Val Ala Glu
           675                 680                 685
   Ile Arg Asp Ser Ala Gln Ala Gly Arg Ala Ser Ser Gln Ser Ala His
       690                 695                 700
   Pro Val Thr Gly Gly Asn Met Ala Thr Tyr Gly His Thr Asn Thr Tyr
   705                 710                 715                 720
   Ser Ser Phe Thr Ser Lys Ala Ser Thr Val Leu Gln Pro Ile Asn Pro
                   725                 730                 735
   Thr Glu Ser Tyr Gly Pro Gln Ile Pro Ile Thr Gly Val Ser Arg Pro
               740                 745                 750
   Ser Ser Ser Asp Ile Ser Ser His Thr Thr Ala Asp Pro Ser Phe Ser
           755                 760                 765
   Ser His Pro Ser Gly Ser His Thr Thr Ala Ser Ser Leu Phe His Ile
       770                 775                 780
   Pro Arg Asn Asn Asn Thr Gly Asn Phe Pro Leu Ser Arg His Leu Gly
   785                 790                 795                 800
   Arg Glu Arg Thr Ile Trp Ser Arg Gly Arg Val Lys Asn Pro His Arg
                   805                 810                 815
   Thr Pro Val Leu Arg Arg His Arg His Arg Thr Val Arg Pro Ala Ile
               820                 825                 830
   Lys Gly Pro Ala Asn Lys Asn Val Ser Gln Val Pro Ala Thr Glu Tyr
           835                 840                 845
   Pro Gly Met Cys His Thr Cys Pro Ser Ala Glu Gly Leu Thr Val Ala
       850                 855                 860
   Thr Ala Ala Leu Ser Val Pro Ser Ser Ser His Ser Ala Leu Pro Lys
   865                 870                 875                 880
   Thr Asn Asn Val Gly Val Ile Ala Glu Glu Ser Thr Thr Val Val Lys
                   885                 890                 895
   Lys Pro Leu Leu Leu Phe Lys Asp Lys Gln Asn Val Asp Ile Glu Ile
               900                 905                 910
   Ile Thr Thr Thr Thr Lys Tyr Ser Gly Gly Glu Ser Asn His Val Ile
           915                 920                 925
   Pro Thr Glu Ala Ser Met Thr Ser Ala Pro Thr Ser Val Ser Leu Gly
   930 935 940
   Lys Ser Pro Val Asp Asn Ser Gly His Leu Ser Met Pro Gly Thr Ile
   945 950 955 960
   Gln Thr Gly Lys Asp Ser Val Glu Thr Thr Pro Leu Pro Ser Pro Leu
   965 970 975
   Ser Thr Pro Ser Ile Pro Thr Ser Thr Lys Phe Ser Lys Arg Lys Thr
   980 985 990
   Pro Leu His Gln Ile Phe Val Asn Asn Gln Lys Lys Glu Gly Met Leu
   995 1000 1005
   Lys Asn Pro Tyr Gln Phe Gly Leu Gln Lys Asn Pro Ala Ala Lys Leu
   1010 1015 1020
   Pro Lys Ile Ala Pro Leu Leu Pro Thr Gly Gln Ser Ser Pro Ser Asp
   1025 1030 1035 1040
   Ser Thr Thr Leu Leu Thr Ser Pro Pro Pro Ala Leu Ser Thr Thr Met
   1045 1050 1055
   Ala Ala Thr Gln Asn Lys Gly Thr Glu Val Val Ser Gly Ala Arg Ser
   1060 1065 1070
   Leu Ser Ala Gly Lys Lys Gln Pro Phe Thr Asn Ser Ser Pro Val Leu
   1075 1080 1085
   Pro Ser Thr Ile Ser Lys Arg Ser Asn Thr Leu Asn Phe Leu Ser Thr
   1090 1095 1100
   Glu Thr Pro Thr Val Thr Ser Pro Thr Ala Thr Ala Ser Val Ile Met
   1105 1110 1115 1120
   Ser Glu Thr Gln Arg Thr Arg Ser Lys Glu Ala Lys Asp Gln Ile Lys
   1125 1130 1135
   Gly Pro Arg Lys Asn Arg Asn Asn Ala Asn Thr Thr Pro Arg Gln Val
   1140 1145 1150
   Ser Gly Tyr Ser Ala Tyr Ser Ala Leu Thr Thr Ala Asp Thr Pro Leu
   1155 1160 1165
   Ala Phe Ser His Ser Pro Arg Gln Asp Asp Gly Gly Asn Val Ser Ala
   1170 1175 1180
   Val Ala Tyr His Ser Thr Thr Ser Leu Leu Ala Ile Thr Glu Leu Phe
   1185 1190 1195 1200
   Glu Lys Tyr Thr Gln Thr Leu Gly Asn Thr Thr Ala Leu Glu Thr Thr
   1205 1210 1215
   Leu Leu Ser Lys Ser Gln Glu Ser Thr Thr Val Lys Arg Ala Ser Asp
   1220 1225 1230
   Thr Pro Pro Pro Leu Leu Ser Ser Gly Ala Pro Pro Val Pro Thr Pro
   1235 1240 1245
   Ser Pro Pro Pro Phe Thr Lys Gly Val Val Thr Asp Ser Lys Val Thr
   1250 1255 1260
   Ser Ala Phe Gln Met Thr Ser Asn Arg Val Val Thr Ile Tyr Glu Ser
   1265 1270 1275 1280
   Ser Arg His Asn Thr Asp Leu Gln Gln Pro Ser Ala Glu Ala Ser Pro
   1285 1290 1295
   Asn Pro Glu Ile Ile Thr Gly Thr Thr Asp Ser Pro Ser Asn Leu Phe
   1300 1305 1310
   Pro Ser Thr Ser Val Pro Ala Leu Arg Val Asp Lys Pro Gln Asn Ser
   1315 1320 1325
   Lys Trp Lys Pro Ser Pro Trp Pro Glu His Lys Tyr Gln Leu Lys Ser
   1330 1335 1340
   Tyr Ser Glu Thr Ile Glu Lys Gly Lys Arg Pro Ala Val Ser Met Ser
   1345 1350 1355 1360
   Pro His Leu Ser Leu Pro Glu Ala Ser Thr His Ala Ser His Trp Asn
   1365 1370 1375
   Thr Gln Lys His Ala Glu Lys Ser Val Phe Asp Lys Lys Pro Gly Gln
   1380 1385 1390
   Asn Pro Thr Ser Lys His Leu Pro Tyr Val Ser Leu Pro Lys Thr Leu
   1395 1400 1405
   Leu Lys Lys Pro Arg Ile Ile Gly Gly Lys Ala Ala Ser Phe Thr Val
   1410 1415 1420
   Pro Ala Asn Ser Asp Val Phe Leu Pro Cys Glu Ala Val Gly Asp Pro
   1425 1430 1435 1440
   Leu Pro Ile Ile His Trp Thr Arg Val Ser Ser Gly Xaa Glu Ile Ser
   1445 1450 1455
   Gln Gly Thr Gln Lys Ser Arg Phe His Val Leu Pro Asn Gly Thr Leu
   1460 1465 1470
   Ser Ile Gln Arg Val Ser Ile Gln Asp Arg Gly Gln Tyr Leu Cys Ser
   1475 1480 1485
   Ala Phe Asn Pro Leu Gly Val Asp His Phe His Val Ser Leu Ser Val
   1490 1495 1500
   Val Phe Tyr Pro Ala Arg Ile Leu Asp Arg His Val Lys Glu Ile Thr
   1505 1510 1515 1520
   Val His Phe Gly Ser Thr Val Glu Leu Lys Cys Arg Val Glu Gly Met
   1525 1530 1535
   Pro Arg Pro Thr Val Ser Trp Ile Leu Ala Asn Gln Thr Val Val Ser
   1540 1545 1550
   Glu Thr Ala Lys Gly Ser Arg Lys Val Trp Val Thr Pro Asp Gly Thr
   1555 1560 1565
   Leu Ile Ile Tyr Asn Leu Ser Leu Tyr Asp Arg Gly Phe Tyr Lys Cys
   1570 1575 1580
   Val Ala Ser Asn Pro Ser Gly Gln Asp Ser Leu Leu Val Lys Ile Gln
   1585 1590 1595 1600
   Val Ile Thr Ala Pro Pro Val Ile Ile Glu Gln Lys Arg Gln Ala Ile
   1605 1610 1615
   Val Gly Val Leu Gly Gly Ser Leu Lys Leu Pro Cys Thr Ala Lys Gly
   1620 1625 1630
   Thr Pro Gln Pro Ser Val His Trp Val Leu Tyr Asp Gly Thr Glu Leu
   1635 1640 1645
   Lys Pro Leu Gln Leu Thr His Ser Arg Phe Phe Leu Tyr Pro Asn Gly
   1650 1655 1660
   Thr Leu Tyr Ile Arg Ser Ile Ala Pro Ser Val Arg Gly Thr Tyr Glu
   1665 1670 1675 1680
   Cys Ile Ala Thr Ser Ser Ser Gly Ser Glu Arg Arg Val Val Ile Leu
   1685 1690 1695
   Thr Val Glu Glu Gly Glu Thr Ile Pro Arg Ile Glu Thr Ala Ser Gln
   1700 1705 1710
   Lys Trp Thr Glu Val Asn Leu Gly Glu Lys Leu Leu Leu Asn Cys Ser
   1715 1720 1725
   Ala Thr Gly Asp Pro Lys Pro Arg Ile Ile Trp Arg Leu Pro Ser Lys
   1730 1735 1740
   Ala Val Ile Asp Gln Trp His Arg Met Gly Ser Arg Ile His Val Tyr
   1745 1750 1755 1760
   Pro Asn Gly Ser Leu Val Val Gly Ser Val Thr Glu Lys Asp Ala Gly
   1765 1770 1775
   Asp Tyr Leu Cys Val Ala Arg Asn Lys Met Gly Asp Asp Leu Val Leu
   1780 1785 1790
   Met His Val Arg Leu Arg Leu Thr Pro Ala Lys Ile Glu Gln Lys Gln
   1795 1800 1805
   Tyr Phe Lys Lys Gln Val Leu His Gly Lys Asp Phe Gln Val Asp Cys
   1810 1815 1820
   Lys Ala Ser Gly Ser Pro Val Pro Glu Val Ser Trp Ser Leu Pro Asp
   1825 1830 1835 1840
   Gly Thr Val Leu Asn Asn Val Ala Gln Ala Asp Asp Ser Gly Tyr Arg
   1845 1850 1855
   Thr Lys Arg Tyr Thr Leu Phe His Asn Gly Thr Leu Tyr Phe Asn Asn
   1860 1865 1870
   Val Gly Met Ala Glu Glu Gly Asp Tyr Ile Cys Ser Ala Gln Asn Thr
   1875 1880 1885
   Leu Gly Lys Asp Glu Met Lys Val His Leu Thr Val Leu Thr Ala Ile
   1890 1895 1900
   Pro Arg Ile Arg Gln Ser Tyr Lys Thr Thr Met Arg Leu Arg Ala Gly
   1905 1910 1915 1920
   Glu Thr Ala Val Leu Asp Cys Glu Val Thr Gly Glu Pro Lys Pro Asn
   1925 1930 1935
   Val Phe Trp Leu Leu Pro Ser Asn Asn Val Ile Ser Phe Ser Asn Asp
   1940 1945 1950
   Arg Phe Thr Phe His Ala Asn Arg Thr Leu Ser Ile His Lys Val Lys
   1955 1960 1965
   Pro Leu Asp Ser Gly Asp Tyr Val Cys Val Ala Gln Asn Pro Ser Gly
   1970 1975 1980
   Asp Asp Thr Lys Thr Tyr Lys Leu Asp Ile Val Ser Lys Pro Pro Leu
   1985 1990 1995 2000
   Ile Asn Gly Leu Tyr Ala Asn Lys Thr Val Ile Lys Ala Thr Ala Ile
   2005 2010 2015
   Arg His Ser Lys Lys Tyr Phe Asp Cys Arg Ala Asp Gly Ile Pro Ser
   2020 2025 2030
   Ser Gln Val Thr Trp Ile Met Pro Gly Asn Ile Phe Leu Pro Ala Pro
   2035 2040 2045
   Tyr Phe Gly Ser Arg Val Thr Val His Pro Asn Gly Thr Leu Glu Met
   2050 2055 2060
   Arg Asn Ile Arg Leu Ser Asp Ser Ala Asp Phe Thr Cys Val Val Arg
   2065 2070 2075 2080
   Ser Glu Gly Gly Glu Ser Val Leu Val Val Gln Leu Glu Val Leu Glu
   2085 2090 2095
   Met Leu Arg Arg Pro Thr Phe Arg Asn Pro Phe Asn Glu Lys Val Ile
   2100 2105 2110
   Ala Gln Ala Gly Lys Pro Val Ala Leu Asn Cys Ser Val Asp Gly Asn
   2115 2120 2125
   Pro Pro Pro Glu Ile Thr Trp Ile Leu Pro Asp Gly Thr Gln Phe Ala
   2130 2135 2140
   Asn Arg Pro His Asn Ser Pro Tyr Leu Met Ala Gly Asn Gly Ser Leu
   2145 2150 2155 2160
   Ile Leu Tyr Lys Ala Thr Arg Asn Lys Ser Gly Lys Tyr Arg Cys Ala
   2165 2170 2175
   Ala Arg Asn Lys Val Gly Tyr Ile Glu Lys Leu Ile Leu Leu Glu Ile
   2180 2185 2190
   Gly Gln Lys Pro Val Ile Leu Thr Tyr Glu Pro Gly Met Val Lys Ser
   2195 2200 2205
   Val Ser Gly Glu Pro Leu Ser Leu His Cys Val Ser Asp Gly Ile Pro
   2210 2215 2220
   Lys Pro Asn Val Lys Trp Thr Thr Pro Gly Gly His Val Ile Asp Arg
   2225                2230                2235                2240
   Pro Gln Val Asp Gly Lys Tyr Ile Leu His Glu Asn Gly Thr Leu Val
                   2245                2250                2255
   Ile Lys Ala Thr Thr Ala His Asp Gln Gly Asn Tyr Ile Cys Arg Ala
               2260                2265                2270
   Gln Asn Ser Val Gly Gln Ala Val Ile Ser Val Ser Val Met Val Val
           2275                2280                2285
   Ala Tyr Pro Pro Arg Ile Ile Asn Tyr Leu Pro Arg Asn Met Leu Arg
       2290                2295                2300
   Arg Thr Gly Glu Ala Met Gln Leu His Cys Val Ala Leu Gly Ile Pro
   2305                2310                2315                2320
   Lys Pro Lys Val Thr Trp Glu Thr Pro Arg His Ser Leu Leu Ser Lys
                   2325                2330                2335
   Ala Thr Ala Arg Lys Pro His Arg Ser Glu Met Leu His Pro Gln Gly
               2340                2345                2350
   Thr Leu Val Ile Gln Asn Leu Gln Thr Ser Asp Ser Gly Val Tyr Lys
           2355                2360                2365
   Cys Arg Ala Gln Asn Leu Leu Gly Thr Asp Tyr Ala Thr Thr Tyr Ile
       2370                2375                2380
   Gln Val Leu
   2385
<210> 36
   <211> 7962
   <212> PRT
   <213> homo sapiens
<220>
   <222> (1)..(7962)
   <223> elastic titin
<300>
   <301> Andersson, et al. Vogel, et al. Lbeit, et al. Ohara, O
   <302> Unknown protein [Homo sapiens].
   <308> GI 3005744
   <309> 1998-04-02
   <313> 1 TO 494
<400> 36
   Pro Asp Gln Glu Met Pro Val Tyr Pro Pro Ala Ile Ile Thr Pro Leu
    1               5                  10                  15
   Gln Asp Thr Val Thr Ser Glu Gly Gln Pro Ala Arg Phe Gln Cys Arg
               20                  25                  30
   Val Ser Gly Thr Asp Leu Lys Val Ser Trp Tyr Ser Lys Asp Lys Lys
                   35                  40                  45
   Ile Lys Pro Ser Arg Phe Phe Arg Met Thr Gln Phe Glu Asp Thr Tyr
       50                  55                  60
   Gln Leu Glu Ile Ala Glu Ala Tyr Pro Glu Asp Glu Gly Thr Tyr Thr
   65                  70                  75                  80
   Phe Val Ala Asn Asn Ala Val Gly Gln Val Ser Ser Thr Ala Asn Leu
                   85                  90                  95
   Ser Leu Glu Ala Pro Glu Ser Ile Leu His Glu Arg Ile Glu Gln Glu
               100                 105                 110
   Ile Glu Met Glu Met Lys Ala Ala Pro Val Ile Lys Arg Lys Ile Glu
           115                 120                 125
   Pro Leu Glu Val Ala Leu Gly His Leu Ala Lys Phe Thr Cys Glu Ile
       130                 135                 140
   Gln Ser Ala Pro Asn Val Arg Phe Gln Trp Phe Lys Ala Gly Arg Glu
   145                 150                 155                 160
   Ile Tyr Glu Ser Asp Lys Cys Ser Ile Arg Ser Ser Lys Tyr Ile Ser
                   165                 170                 175
   Ser Leu Glu Ile Leu Arg Thr Gln Val Val Asp Cys Gly Glu Tyr Thr
               180                 185                 190
   Cys Lys Ala Ser Asn Glu Tyr Gly Ser Val Ser Cys Thr Ala Thr Leu
           195                 200                 205
   Thr Val Thr Glu Ala Tyr Pro Pro Thr Phe Leu Ser Arg Pro Lys Ser
       210                 215                 220
   Leu Thr Thr Phe Val Gly Lys Ala Ala Lys Phe Ile Cys Thr Val Thr
   225                 230                 235                 240
   Gly Thr Pro Val Ile Glu Thr Ile Trp Gln Lys Asp Gly Ala Ala Leu
                   245                 250                 255
   Ser Pro Ser Pro Asn Trp Arg Ile Ser Asp Ala Glu Asn Lys His Ile
               260                 265                 270
   Leu Glu Leu Ser Asn Leu Thr Ile Gln Asp Arg Gly Val Tyr Ser Cys
           275                 280                 285
   Lys Ala Ser Asn Lys Phe Gly Ala Asp Ile Cys Gln Ala Glu Leu Ile
       290                 295                 300
   Ile Ile Asp Lys Pro His Phe Ile Lys Glu Leu Glu Pro Val Gln Ser
   305                 310                 315                 320
   Ala Ile Asn Lys Lys Val His Leu Glu Cys Gln Val Asp Glu Asp Arg
                   325                 330                 335
   Lys Val Thr Val Thr Trp Ser Lys Asp Gly Gln Lys Leu Pro Pro Gly
               340                 345                 350
   Lys Asp Tyr Lys Ile Cys Phe Glu Asp Lys Ile Ala Thr Leu Glu Ile
           355                 360                 365
   Pro Leu Ala Lys Leu Lys Asp Ser Gly Thr Tyr Val Cys Thr Ala Ser
   370 375 380
   Asn Glu Ala Gly Ser Ser Ser Cys Ser Ala Thr Val Thr Val Arg Glu
   385 390 395 400
   Pro Pro Ser Phe Val Lys Lys Val Asp Pro Ser Tyr Leu Met Leu Pro
   405 410 415
   Gly Glu Ser Ala Arg Leu His Cys Lys Leu Lys Gly Ser Pro Val Ile
   420 425 430
   Gln Val Thr Trp Phe Lys Asn Asn Lys Glu Leu Ser Glu Ser Asn Thr
   435 440 445
   Val Arg Met Tyr Phe Val Asn Ser Glu Ala Ile Leu Asp Ile Thr Asp
   450 455 460
   Val Lys Val Glu Asp Ser Gly Ser Tyr Ser Cys Glu Ala Val Asn Asp
   465 470 475 480
   Val Gly Ser Asp Ser Cys Ser Thr Glu Ile Val Ile Lys Glu Pro Pro
   485 490 495
   Ser Phe Ile Lys Thr Leu Glu Pro Ala Asp Ile Val Arg Gly Thr Asn
   500 505 510
   Ala Leu Leu Gln Cys Glu Val Ser Gly Thr Gly Pro Phe Glu Ile Ser
   515 520 525
   Trp Phe Lys Asp Lys Lys Gln Ile Arg Ser Ser Lys Lys Tyr Arg Leu
   530 535 540
   Phe Ser Gln Lys Ser Leu Val Cys Leu Glu Ile Phe Ser Phe Asn Ser
   545 550 555 560
   Ala Asp Val Gly Glu Tyr Glu Cys Val Val Ala Asn Glu Val Gly Lys
   565 570 575
   Cys Gly Cys Met Ala Thr His Leu Leu Lys Glu Pro Pro Thr Phe Val
   580 585 590
   Lys Lys Val Asp Asp Leu Ile Ala Leu Gly Gly Gln Thr Val Thr Leu
   595 600 605
   Gln Ala Ala Val Arg Gly Ser Glu Pro Ile Ser Val Thr Trp Met Lys
   610 615 620
   Gly Gln Glu Val Ile Arg Glu Asp Gly Lys Ile Lys Met Ser Phe Ser
   625 630 635 640
   Asn Gly Val Ala Val Leu Ile Ile Pro Asp Val Gln Ile Ser Phe Gly
   645 650 655
   Gly Lys Tyr Thr Cys Leu Ala Glu Asn Glu Ala Gly Ser Gln Thr Ser
   660 665 670
   Val Gly Glu Leu Ile Val Lys Glu Pro Ala Lys Ile Ile Glu Arg Ala
   675 680 685
   Glu Leu Ile Gln Val Thr Ala Gly Asp Pro Ala Thr Leu Glu Tyr Thr
       690                 695                 700
   Val Ala Gly Thr Pro Glu Leu Lys Pro Lys Trp Tyr Lys Asp Gly Arg
   705                 710                 715                 720
   Pro Leu Val Ala Ser Lys Lys Tyr Arg Ile Ser Phe Lys Asn Asn Val
                   725                 730                 735
   Ala Gln Leu Lys Phe Tyr Ser Ala Glu Leu His Asp Ser Gly Gln Tyr
               740                 745                 750
   Thr Phe Glu Ile Ser Asn Glu Val Gly Ser Ser Ser Cys Glu Thr Thr
           755                 760                 765
   Phe Thr Val Leu Asp Arg Asp Ile Ala Pro Phe Phe Thr Lys Pro Leu
       770                 775                 780
   Arg Asn Val Asp Ser Val Val Asn Gly Thr Cys Arg Leu Asp Cys Lys
   785                 790                 795                 800
   Ile Ala Gly Ser Leu Pro Met Arg Val Ser Trp Phe Lys Asp Gly Lys
                   805                 810                 815
   Glu Ile Ala Ala Ser Asp Arg Tyr Arg Ile Ala Phe Val Glu Gly Thr
               820                 825                 830
   Ala Ser Leu Glu Ile Ile Arg Val Asp Met Asn Asp Ala Gly Asn Phe
           835                 840                 845
   Thr Cys Arg Ala Thr Asn Ser Val Gly Ser Lys Asp Ser Ser Gly Ala
       850                 855                 860
   Leu Ile Val Gln Glu Pro Pro Ser Phe Val Thr Lys Pro Gly Ser Lys
   865                 870                 875                 880
   Asp Val Leu Pro Gly Ser Ala Val Cys Leu Lys Ser Thr Phe Gln Gly
                   885                 890                 895
   Ser Thr Pro Leu Thr Ile Arg Trp Phe Lys Gly Asn Lys Glu Leu Val
               900                 905                 910
   Ser Gly Gly Ser Cys Tyr Ile Thr Lys Glu Ala Leu Glu Ser Ser Leu
           915                 920                 925
   Glu Leu Tyr Leu Val Lys Thr Ser Asp Ser Gly Thr Tyr Thr Cys Lys
       930                 935                 940
   Val Ser Asn Val Ala Gly Gly Val Glu Cys Ser Ala Asn Leu Phe Val
   945                 950                 955                 960
   Lys Glu Pro Ala Thr Phe Val Glu Lys Leu Glu Pro Ser Gln Leu Leu
                   965                 970                 975
   Lys Lys Gly Asp Ala Thr Gln Leu Ala Cys Lys Val Thr Gly Thr Pro
               980                 985                 990
   Pro Ile Lys Ile Thr Trp Phe Ala Asn Asp Arg Glu Ile Lys Glu Ser
           995                 1000                1005
   Ser Lys His Arg Met Ser Phe Val Glu Ser Thr Ala Val Leu Arg Leu
   1010 1015 1020
   Thr Asp Val Gly Ile Glu Asp Ser Gly Glu Tyr Met Cys Glu Ala Gln
   1025 1030 1035 1040
   Asn Glu Ala Gly Ser Asp His Cys Ser Ser Ile Val Ile Val Lys Glu
   1045 1050 1055
   Ser Pro Tyr Phe Thr Lys Glu Phe Lys Pro Ile Glu Val Leu Lys Glu
   1060 1065 1070
   Tyr Asp Val Met Leu Leu Ala Glu Val Ala Gly Thr Pro Pro Phe Glu
   1075 1080 1085
   Ile Thr Trp Phe Lys Asp Asn Thr Ile Leu Arg Ser Gly Arg Lys Tyr
   1090 1095 1100
   Lys Thr Phe Ile Gln Asp His Leu Val Ser Leu Gln Ile Leu Lys Phe
   1105 1110 1115 1120
   Val Ala Ala Asp Ala Gly Glu Tyr Gln Cys Arg Val Thr Asn Glu Val
   1125 1130 1135
   Gly Ser Ser Ile Cys Ser Ala Arg Val Thr Leu Arg Glu Pro Pro Ser
   1140 1145 1150
   Phe Ile Lys Lys Ile Glu Ser Thr Ser Ser Leu Arg Gly Gly Thr Ala
   1155 1160 1165
   Ala Phe Gln Ala Thr Leu Lys Gly Ser Leu Pro Ile Thr Val Thr Trp
   1170 1175 1180
   Leu Lys Asp Ser Asp Glu Ile Thr Glu Asp Asp Asn Ile Arg Met Thr
   1185 1190 1195 1200
   Phe Glu Asn Asn Val Ala Ser Leu Tyr Leu Ser Gly Ile Glu Val Lys
   1205 1210 1215
   His Asp Gly Lys Tyr Val Cys Gln Ala Lys Asn Asp Ala Gly Ile Gln
   1220 1225 1230
   Arg Cys Ser Ala Leu Leu Ser Val Lys Glu Pro Ala Thr Ile Thr Glu
   1235 1240 1245
   Glu Ala Val Ser Ile Asp Val Thr Gln Gly Asp Pro Ala Thr Leu Gln
   1250 1255 1260
   Val Lys Phe Ser Gly Thr Lys Glu Ile Thr Ala Lys Trp Phe Lys Asp
   1265 1270 1275 1280
   Gly Gln Glu Leu Thr Leu Gly Ser Lys Tyr Lys Ile Ser Val Thr Asp
   1285 1290 1295
   Thr Val Ser Ile Leu Lys Ile Ile Ser Thr Glu Lys Lys Asp Ser Gly
   1300 1305 1310
   Glu Tyr Thr Phe Glu Val Gln Asn Asp Val Gly Arg Ser Ser Cys Lys
   1315 1320 1325
   Ala Arg Ile Asn Val Leu Asp Leu Ile Ile Pro Pro Ser Phe Thr Lys
   1330 1335 1340
   Lys Leu Lys Lys Met Asp Ser Ile Lys Gly Ser Phe Ile Asp Leu Glu
   1345                1350                1355                1360
   Cys Ile Val Ala Gly Ser His Pro Ile Ser Ile Gln Trp Phe Lys Asp
                   1365                1370                1375
   Asp Gln Glu Ile Ser Ala Ser Glu Lys Tyr Lys Phe Ser Phe His Asp
               1380                1385                1390
   Asn Thr Ala Phe Leu Glu Ile Ser Gln Leu Glu Gly Thr Asp Ser Gly
           1395                1400                1405
   Thr Tyr Thr Cys Ser Ala Thr Asn Lys Ala Gly His Asn Gln Cys Ser
       1410                1415                1420
   Gly His Leu Thr Val Lys Glu Pro Pro Tyr Phe Val Glu Lys Pro Gln
   1425                1430                1435                1440
   Ser Gln Asp Val Asn Pro Asn Thr Arg Val Gln Leu Lys Ala Leu Val
                   1445                1450                1455
   Gly Gly Thr Ala Pro Met Thr Ile Lys Trp Phe Lys Asp Asn Lys Glu
               1460                1465                1470
   Leu His Ser Gly Ala Ala Arg Ser Val Trp Lys Asp Asp Thr Ser Thr
           1475                1480                1485
   Ser Leu Glu Leu Phe Ala Ala Lys Ala Thr Asp Ser Gly Thr Tyr Ile
       1490                1495                1500
   Cys Gln Leu Ser Asn Asp Val Gly Thr Ala Thr Ser Lys Ala Thr Leu
   1505                1510                1515                1520
   Phe Val Lys Glu Pro Pro Gln Phe Ile Lys Lys Pro Ser Pro Val Leu
                   1525                1530                1535
   Val Leu Arg Asn Gly Gln Ser Thr Thr Phe Glu Cys Gln Ile Thr Gly
               1540                1545                1550
   Thr Pro Lys Ile Arg Val Ser Trp Tyr Leu Asp Gly Asn Glu Ile Thr
           1555                1560                1565
   Ala Ile Gln Lys His Gly Ile Ser Phe Ile Asp Gly Leu Ala Thr Phe
       1570                1575                1580
   Gln Ile Ser Gly Ala Arg Val Glu Asn Ser Gly Thr Tyr Val Cys Glu
   1585                1590                1595                1600
   Ala Arg Asn Asp Ala Gly Thr Ala Ser Cys Ser Ile Glu Leu Lys Val
                   1605                1610                1615
   Lys Glu Pro Pro Thr Phe Ile Arg Glu Leu Lys Pro Val Glu Val Val
               1620                1625                1630
   Lys Tyr Ser Asp Val Glu Leu Glu Cys Glu Val Thr Gly Thr Pro Pro
           1635                1640                1645
   Phe Glu Val Thr Trp Leu Lys Asn Asn Arg Glu Ile Arg Ser Ser Lys
       1650                1655                1660
   Lys Tyr Thr Leu Thr Asp Arg Val Ser Val Phe Asn Leu His Ile Thr
   1665 1670 1675 1680
   Lys Cys Asp Pro Ser Asp Thr Gly Glu Tyr Gln Cys Ile Val Ser Asn
   1685 1690 1695
   Glu Gly Gly Ser Cys Ser Cys Ser Thr Arg Val Ala Leu Lys Glu Pro
   1700 1705 1710
   Pro Ser Phe Ile Lys Lys Ile Glu Asn Thr Thr Thr Val Leu Lys Ser
   1715 1720 1725
   Ser Ala Thr Phe Gln Ser Thr Val Ala Gly Ser Pro Pro Ile Ser Ile
   1730 1735 1740
   Thr Trp Leu Lys Asp Asp Gln Ile Leu Asp Glu Asp Asp Asn Val Tyr
   1745 1750 1755 1760
   Ile Ser Phe Val Asp Ser Val Ala Thr Leu Gln Ile Arg Ser Val Asp
   1765 1770 1775
   Asn Gly His Ser Gly Arg Tyr Thr Cys Gln Ala Lys Asn Glu Ser Gly
   1780 1785 1790
   Val Glu Arg Cys Tyr Ala Phe Leu Leu Val Gln Glu Pro Ala Gln Ile
   1795 1800 1805
   Val Glu Lys Ala Lys Ser Val Asp Val Thr Glu Lys Asp Pro Met Thr
   1810 1815 1820
   Leu Glu Cys Val Val Ala Gly Thr Pro Glu Leu Lys Val Lys Trp Leu
   1825 1830 1835 1840
   Lys Asp Gly Lys Gln Ile Val Pro Ser Arg Tyr Phe Ser Met Ser Phe
   1845 1850 1855
   Glu Asn Asn Val Ala Ser Phe Arg Ile Gln Ser Val Met Lys Gln Asp
   1860 1865 1870
   Ser Gly Gln Tyr Thr Phe Lys Val Glu Asn Asp Phe Gly Ser Ser Ser
   1875 1880 1885
   Cys Asp Ala Tyr Leu Arg Val Leu Asp Gln Asn Ile Pro Pro Ser Phe
   1890 1895 1900
   Thr Lys Lys Leu Thr Lys Met Asp Lys Val Leu Gly Ser Ser Ile His
   1905 1910 1915 1920
   Met Glu Cys Lys Val Ser Gly Ser Leu Pro Ile Ser Ala Gln Trp Phe
   1925 1930 1935
   Lys Asp Gly Lys Glu Ile Ser Thr Ser Ala Lys Tyr Arg Leu Val Cys
   1940 1945 1950
   His Glu Arg Ser Val Ser Leu Glu Val Asn Asn Leu Glu Leu Glu Asp
   1955 1960 1965
   Thr Ala Asn Tyr Thr Cys Lys Val Ser Asn Val Ala Gly Asp Asp Ala
   1970 1975 1980
   Cys Ser Gly Ile Leu Thr Val Lys Glu Pro Pro Ser Phe Leu Val Lys
   1985 1990 1995 2000
   Pro Gly Arg Gln Gln Ala Ile Pro Asp Ser Thr Val Glu Phe Lys Ala
   2005 2010 2015
   Ile Leu Lys Gly Thr Pro Pro Phe Lys Ile Lys Trp Phe Lys Asp Asp
   2020 2025 2030
   Val Glu Leu Val Ser Gly Pro Lys Cys Phe Ile Gly Leu Glu Gly Ser
   2035 2040 2045
   Thr Ser Phe Leu Asn Leu Tyr Ser Val Asp Ala Ser Lys Thr Gly Gln
   2050 2055 2060
   Tyr Thr Cys His Val Thr Asn Asp Val Gly Ser Asp Ser Cys Thr Thr
   2065 2070 2075 2080
   Met Leu Leu Val Thr Glu Pro Pro Lys Phe Val Lys Lys Leu Glu Ala
   2085 2090 2095
   Ser Lys Ile Val Lys Ala Gly Asp Ser Ser Arg Leu Glu Cys Lys Ile
   2100 2105 2110
   Ala Gly Ser Pro Glu Ile Arg Val Val Trp Phe Arg Asn Glu His Glu
   2115 2120 2125
   Leu Pro Ala Ser Asp Lys Tyr Arg Met Thr Phe Ile Asp Ser Val Ala
   2130 2135 2140
   Val Ile Gln Met Asn Asn Leu Ser Thr Glu Asp Ser Gly Asp Phe Ile
   2145 2150 2155 2160
   Cys Glu Ala Gln Asn Pro Ala Gly Ser Thr Ser Cys Ser Thr Lys Val
   2165 2170 2175
   Ile Val Lys Glu Pro Pro Val Phe Ser Ser Phe Pro Pro Ile Val Glu
   2180 2185 2190
   Thr Leu Lys Asn Ala Glu Val Ser Leu Glu Cys Glu Leu Ser Gly Thr
   2195 2200 2205
   Pro Pro Phe Glu Val Val Trp Tyr Lys Asp Lys Arg Gln Leu Arg Ser
   2210 2215 2220
   Ser Lys Lys Tyr Lys Ile Ala Ser Lys Asn Phe His Thr Ser Ile His
   2225 2230 2235 2240
   Ile Leu Asn Val Asp Thr Ser Asp Ile Gly Glu Tyr His Cys Lys Ala
   2245 2250 2255
   Gln Asn Glu Val Gly Ser Asp Thr Cys Val Cys Thr Val Lys Leu Lys
   2260 2265 2270
   Glu Pro Pro Arg Phe Val Ser Lys Leu Asn Ser Leu Thr Val Val Ala
   2275 2280 2285
   Gly Glu Pro Ala Glu Leu Gln Ala Ser Ile Glu Gly Ala Gln Pro Ile
   2290 2295 2300
   Phe Val Gln Trp Leu Lys Glu Lys Glu Glu Val Ile Arg Glu Ser Glu
   2305 2310 2315 2320
   Asn Ile Arg Ile Thr Phe Val Glu Asn Val Ala Thr Leu Gln Phe Ala
   2325 2330 2335
   Lys Ala Glu Pro Ala Asn Ala Gly Lys Tyr Ile Cys Gln Ile Lys Asn
   2340 2345 2350
   Asp Gly Gly Met Arg Glu Asn Met Ala Thr Leu Met Val Leu Glu Pro
   2355 2360 2365
   Ala Val Ile Val Glu Lys Ala Gly Pro Met Thr Val Thr Val Gly Glu
   2370 2375 2380
   Thr Cys Thr Leu Glu Cys Lys Val Ala Gly Thr Pro Glu Leu Ser Val
   2385 2390 2395 2400
   Glu Trp Tyr Lys Asp Gly Lys Leu Leu Thr Ser Ser Gln Lys His Lys
   2405 2410 2415
   Phe Ser Phe Tyr Asn Lys Ile Ser Ser Leu Arg Ile Leu Ser Val Glu
   2420 2425 2430
   Arg Gln Asp Ala Gly Thr Tyr Thr Phe Gln Val Gln Asn Asn Val Gly
   2435 2440 2445
   Lys Ser Ser Cys Thr Ala Val Val Asp Val Ser Asp Arg Ala Val Pro
   2450 2455 2460
   Pro Ser Phe Thr Arg Arg Leu Lys Asn Thr Gly Gly Val Leu Gly Ala
   2465 2470 2475 2480
   Ser Cys Ile Leu Glu Cys Lys Val Ala Gly Ser Ser Pro Ile Ser Val
   2485 2490 2495
   Ala Trp Phe His Glu Lys Thr Lys Ile Val Ser Gly Ala Lys Tyr Gln
   2500 2505 2510
   Thr Thr Phe Ser Asp Asn Val Cys Thr Leu Gln Leu Asn Ser Leu Asp
   2515 2520 2525
   Ser Ser Asp Met Gly Asn Tyr Thr Cys Val Ala Ala Asn Val Ala Gly
   2530 2535 2540
   Ser Asp Glu Cys Arg Ala Val Leu Thr Val Gln Glu Pro Pro Ser Phe
   2545 2550 2555 2560
   Val Lys Glu Pro Glu Pro Leu Glu Val Leu Pro Gly Lys Asn Val Thr
   2565 2570 2575
   Phe Thr Ser Val Ile Arg Gly Thr Pro Pro Phe Lys Val Asn Trp Phe
   2580 2585 2590
   Arg Gly Ala Arg Glu Leu Val Lys Gly Asp Arg Cys Asn Ile Tyr Phe
   2595 2600 2605
   Glu Asp Thr Val Ala Glu Leu Glu Leu Phe Asn Ile Asp Ile Ser Gln
   2610 2615 2620
   Ser Gly Glu Tyr Thr Cys Val Val Ser Asn Asn Ala Gly Gln Ala Ser
   2625 2630 2635 2640
   Cys Thr Thr Arg Leu Phe Val Lys Glu Pro Ala Ala Phe Leu Lys Arg
   2645 2650 2655
   Leu Ser Asp His Ser Val Glu Pro Gly Lys Ser Ile Ile Leu Glu Ser
   2660 2665 2670
   Thr Tyr Thr Gly Thr Leu Pro Ile Ser Val Thr Trp Lys Lys Asp Gly
   2675 2680 2685
   Phe Asn Ile Thr Thr Ser Glu Lys Cys Asn Ile Val Thr Thr Glu Lys
   2690 2695 2700
   Thr Cys Ile Leu Glu Ile Leu Asn Ser Thr Lys Arg Asp Ala Gly Gln
   2705 2710 2715 2720
   Tyr Ser Cys Glu Ile Glu Asn Glu Ala Gly Arg Asp Val Cys Gly Ala
   2725 2730 2735
   Leu Val Ser Thr Leu Glu Pro Pro Tyr Phe Val Thr Glu Leu Glu Pro
   2740 2745 2750
   Leu Glu Ala Ala Val Gly Asp Ser Val Ser Leu Gln Cys Gln Val Ala
   2755 2760 2765
   Gly Thr Pro Glu Ile Thr Val Ser Trp Tyr Lys Gly Asp Thr Lys Leu
   2770 2775 2780
   Arg Pro Thr Pro Glu Tyr Arg Thr Tyr Phe Thr Asn Asn Val Ala Thr
   2785 2790 2795 2800
   Leu Val Phe Asn Lys Val Asn Ile Asn Asp Ser Gly Glu Tyr Thr Cys
   2805 2810 2815
   Lys Ala Glu Asn Ser Ile Gly Thr Ala Ser Ser Lys Thr Val Phe Arg
   2820 2825 2830
   Ile Gln Glu Arg Gln Leu Pro Pro Ser Phe Ala Arg Gln Leu Lys Asp
   2835 2840 2845
   Ile Glu Gln Thr Val Gly Leu Pro Val Thr Leu Thr Cys Arg Leu Asn
   2850 2855 2860
   Gly Ser Ala Pro Ile Gln Val Cys Trp Tyr Arg Asp Gly Val Leu Leu
   2865 2870 2875 2880
   Arg Asp His Glu Asn Leu Gln Thr Ser Phe Val Asp Asn Val Ala Thr
   2885 2890 2895
   Leu Lys Ile Leu Gln Thr Asp Leu Ser His Ser Gly Gln Tyr Ser Cys
   2900 2905 2910
   Ser Ala Ser Asn Pro Leu Gly Thr Ala Ser Ser Ser Ala Arg Leu Thr
   2915 2920 2925
   Ala Arg Glu Pro Lys Lys Ser Pro Phe Phe Asp Ile Lys Pro Val Ser
   2930 2935 2940
   Ile Asp Val Ile Ala Gly Glu Ser Ala Asp Phe Glu Cys His Val Thr
   2945 2950 2955 2960
   Gly Ala Gln Pro Met Arg Ile Thr Trp Ser Lys Asp Asn Lys Glu Ile
   2965 2970 2975
   Arg Pro Gly Gly Asn Tyr Thr Ile Thr Cys Val Gly Asn Thr Pro His
   2980 2985 2990
   Leu Arg Ile Leu Lys Val Gly Lys Gly Asp Ser Gly Gln Tyr Thr Cys
   2995 3000 3005
   Gln Ala Thr Asn Asp Val Gly Lys Asp Met Cys Ser Ala Gln Leu Ser
   3010 3015 3020
   Val Lys Glu Pro Pro Lys Phe Val Lys Lys Leu Glu Ala Ser Lys Val
   3025 3030 3035 3040
   Ala Lys Gln Gly Glu Ser Ile Gln Leu Glu Cys Lys Ile Ser Gly Ser
   3045 3050 3055
   Pro Glu Ile Lys Val Ser Trp Phe Arg Asn Asp Ser Glu Leu His Glu
   3060 3065 3070
   Ser Trp Lys Tyr Asn Met Ser Phe Ile Asn Ser Val Ala Leu Leu Thr
   3075 3080 3085
   Ile Asn Glu Ala Ser Ala Glu Asp Ser Gly Asp Tyr Ile Cys Glu Ala
   3090 3095 3100
   His Asn Gly Val Gly Asp Ala Ser Cys Ser Thr Ala Leu Thr Val Lys
   3105 3110 3115 3120
   Ala Pro Pro Val Phe Thr Gln Lys Pro Ser Pro Val Gly Ala Leu Lys
   3125 3130 3135
   Gly Ser Asp Val Ile Leu Gln Cys Glu Ile Ser Gly Thr Pro Pro Phe
   3140 3145 3150
   Glu Val Val Trp Val Lys Asp Arg Lys Gln Val Arg Asn Ser Lys Lys
   3155 3160 3165
   Phe Lys Ile Thr Ser Lys His Phe Asp Thr Asn Leu His Ile Leu Asn
   3170 3175 3180
   Leu Glu Ala Ser Asp Val Gly Glu Tyr His Cys Lys Ala Thr Asn Glu
   3185 3190 3195 3200
   Val Gly Ser Asp Thr Cys Ser Cys Ser Val Lys Phe Lys Glu Pro Pro
   3205 3210 3215
   Arg Phe Val Lys Lys Leu Ser Asp Thr Ser Thr Leu Ile Gly Asp Ala
   3220 3225 3230
   Val Glu Leu Arg Ala Ile Val Glu Gly Phe Gln Pro Ile Ser Val Val
   3235 3240 3245
   Trp Leu Lys Asp Arg Gly Glu Val Ile Arg Glu Ser Glu Asn Thr Arg
   3250 3255 3260
   Ile Ser Phe Ile Asp Asn Ile Ala Thr Leu Gln Leu Gly Ser Pro Glu
   3265 3270 3275 3280
   Ala Ser Asn Ser Gly Lys Tyr Ile Cys Gln Ile Lys Asn Asp Ala Gly
   3285 3290 3295
   Met Arg Glu Cys Ser Ala Val Leu Thr Val Leu Glu Pro Ala Arg Ile
   3300 3305 3310
   Ile Glu Lys Pro Glu Pro Met Thr Val Thr Thr Gly Asn Pro Phe Ala
   3315 3320 3325
   Leu Glu Cys Val Val Thr Gly Thr Pro Glu Leu Ser Ala Lys Trp Phe
   3330 3335 3340
   Lys Asp Gly Arg Glu Leu Ser Ala Asp Ser Lys His His Ile Thr Phe
   3345 3350 3355 3360
   Ile Asn Lys Val Ala Ser Leu Lys Ile Pro Cys Ala Glu Met Ser Asp
   3365 3370 3375
   Lys Gly Leu Tyr Ser Phe Glu Val Lys Asn Ser Val Gly Lys Ser Asn
   3380 3385 3390
   Cys Thr Val Ser Val His Val Ser Asp Arg Ile Val Pro Pro Ser Phe
   3395 3400 3405
   Ile Arg Lys Leu Lys Asp Val Asn Ala Ile Leu Gly Ala Ser Val Val
   3410 3415 3420
   Leu Glu Cys Arg Val Ser Gly Ser Ala Pro Ile Ser Val Gly Trp Phe
   3425 3430 3435 3440
   Gln Asp Gly Asn Glu Ile Val Ser Gly Pro Lys Cys Gln Ser Ser Phe
   3445 3450 3455
   Ser Glu Asn Val Cys Thr Leu Asn Leu Ser Leu Leu Glu Pro Ser Asp
   3460 3465 3470
   Thr Gly Ile Tyr Thr Cys Val Ala Ala Asn Val Ala Gly Ser Asp Glu
   3475 3480 3485
   Cys Ser Ala Val Leu Thr Val Gln Glu Pro Pro Ser Phe Glu Gln Thr
   3490 3495 3500
   Pro Asp Ser Val Glu Val Leu Pro Gly Met Ser Leu Thr Phe Thr Ser
   3505 3510 3515 3520
   Val Ile Arg Gly Thr Pro Pro Phe Lys Val Lys Trp Phe Lys Gly Ser
   3525 3530 3535
   Arg Glu Leu Val Pro Gly Glu Ser Cys Asn Ile Ser Leu Glu Asp Phe
   3540 3545 3550
   Val Thr Glu Leu Glu Leu Phe Glu Val Gln Pro Leu Glu Ser Gly Asp
   3555 3560 3565
   Tyr Ser Cys Leu Val Thr Asn Asp Ala Gly Ser Ala Ser Cys Thr Thr
   3570 3575 3580
   His Leu Phe Val Lys Glu Pro Ala Thr Phe Val Lys Arg Leu Ala Asp
   3585 3590 3595 3600
   Phe Ser Val Glu Thr Gly Ser Pro Ile Val Leu Glu Ala Thr Tyr Thr
   3605 3610 3615
   Gly Thr Pro Pro Ile Ser Val Ser Trp Ile Lys Asp Glu Tyr Leu Ile
   3620 3625 3630
   Ser Gln Ser Glu Arg Cys Ser Ile Thr Met Thr Glu Lys Ser Thr Ile
   3635 3640 3645
   Leu Glu Ile Leu Glu Ser Thr Ile Glu Asp Tyr Ala Gln Tyr Ser Cys
   3650 3655 3660
   Leu Ile Glu Asn Glu Ala Gly Gln Asp Ile Cys Glu Ala Leu Val Ser
   3665 3670 3675 3680
   Val Leu Glu Pro Pro Tyr Phe Ile Glu Pro Leu Glu His Val Glu Ala
   3685 3690 3695
   Val Ile Gly Glu Pro Ala Thr Leu Gln Cys Lys Val Asp Gly Thr Pro
   3700 3705 3710
   Glu Ile Arg Ile Ser Trp Tyr Lys Glu His Thr Lys Leu Arg Ser Ala
   3715 3720 3725
   Pro Ala Tyr Lys Met Gln Phe Lys Asn Asn Val Ala Ser Leu Val Ile
   3730 3735 3740
   Asn Lys Val Asp His Ser Asp Val Gly Glu Tyr Ser Cys Lys Ala Asp
   3745 3750 3755 3760
   Asn Ser Val Gly Ala Val Ala Ser Ser Ala Val Leu Val Ile Lys Ala
   3765 3770 3775
   Arg Lys Leu Pro Pro Phe Phe Ala Arg Lys Leu Lys Asp Val His Glu
   3780 3785 3790
   Thr Leu Gly Phe Pro Val Ala Phe Glu Cys Arg Ile Asn Gly Ser Glu
   3795 3800 3805
   Pro Leu Gln Val Ser Trp Tyr Lys Asp Gly Val Leu Leu Lys Asp Asp
   3810 3815 3820
   Ala Asn Leu Gln Thr Ser Phe Val His Asn Val Ala Thr Leu Gln Ile
   3825 3830 3835 3840
   Leu Gln Thr Asp Gln Ser His Ile Gly Gln Tyr Asn Cys Ser Ala Ser
   3845 3850 3855
   Asn Pro Leu Gly Thr Ala Ser Ser Ser Ala Lys Leu Ile Leu Ser Glu
   3860 3865 3870
   His Glu Val Pro Pro Phe Phe Asp Leu Lys Pro Val Ser Val Asp Leu
   3875 3880 3885
   Ala Leu Gly Glu Ser Gly Thr Phe Lys Cys His Val Thr Gly Thr Ala
   3890 3895 3900
   Pro Ile Lys Ile Thr Trp Ala Lys Asp Asn Arg Glu Ile Arg Pro Gly
   3905 3910 3915 3920
   Gly Asn Tyr Lys Met Thr Leu Val Glu Asn Thr Ala Thr Leu Thr Val
   3925 3930 3935
   Leu Lys Val Gly Lys Gly Asp Ala Gly Gln Tyr Thr Cys Tyr Ala Ser
   3940 3945 3950
   Asn Ile Ala Gly Lys Asp Ser Cys Ser Ala Gln Leu Gly Val Gln Glu
   3955 3960 3965
   Pro Pro Arg Phe Ile Lys Lys Leu Glu Pro Ser Arg Ile Val Lys Gln
   3970 3975 3980
   Asp Glu Phe Thr Arg Tyr Glu Cys Lys Ile Gly Gly Ser Pro Glu Ile
   3985 3990 3995 4000
   Lys Val Leu Trp Tyr Lys Asp Glu Thr Glu Ile Gln Glu Ser Ser Lys
   4005 4010 4015
   Phe Arg Met Ser Phe Val Asp Ser Val Ala Val Leu Glu Met His Asn
   4020 4025 4030
   Leu Ser Val Glu Asp Ser Gly Asp Tyr Thr Cys Glu Ala His Asn Ala
   4035 4040 4045
   Ala Gly Ser Ala Ser Ser Ser Thr Ser Leu Lys Val Lys Glu Pro Pro
   4050 4055 4060
   Ile Phe Arg Lys Lys Pro His Pro Ile Glu Thr Leu Lys Gly Ala Asp
   4065 4070 4075 4080
   Val His Leu Glu Cys Glu Leu Gln Gly Thr Pro Pro Phe His Val Ser
   4085 4090 4095
   Trp Tyr Lys Asp Lys Arg Glu Leu Arg Ser Gly Lys Lys Tyr Lys Ile
   4100 4105 4110
   Met Ser Glu Asn Phe Leu Thr Ser Ile His Ile Leu Asn Val Asp Ala
   4115 4120 4125
   Ala Asp Ile Gly Glu Tyr Gln Cys Lys Ala Thr Asn Asp Val Gly Ser
   4130 4135 4140
   Asp Thr Cys Val Gly Ser Ile Ala Leu Lys Ala Pro Pro Arg Phe Val
   4145 4150 4155 4160
   Lys Lys Leu Ser Asp Ile Ser Thr Val Val Gly Lys Glu Val Gln Leu
   4165 4170 4175
   Gln Thr Thr Ile Glu Gly Ala Glu Pro Ile Ser Val Val Trp Phe Lys
   4180 4185 4190
   Asp Lys Gly Glu Ile Val Arg Glu Ser Asp Asn Ile Trp Ile Ser Tyr
   4195 4200 4205
   Ser Glu Asn Ile Ala Thr Leu Gln Phe Ser Arg Val Glu Pro Ala Asn
   4210 4215 4220
   Ala Gly Lys Tyr Thr Cys Gln Ile Lys Asn Asp Ala Gly Met Gln Glu
   4225 4230 4235 4240
   Cys Phe Ala Thr Leu Ser Val Leu Glu Pro Ala Thr Ile Val Glu Lys
   4245 4250 4255
   Pro Glu Ser Ile Lys Val Thr Thr Gly Asp Thr Cys Thr Leu Glu Cys
   4260 4265 4270
   Thr Val Ala Gly Thr Pro Glu Leu Ser Thr Lys Trp Phe Lys Asp Gly
   4275 4280 4285
   Lys Glu Leu Thr Ser Asp Asn Lys Tyr Lys Ile Ser Phe Phe Asn Lys
   4290 4295 4300
   Val Ser Gly Leu Lys Ile Ile Asn Val Ala Pro Ser Asp Ser Gly Val
   4305 4310 4315 4320
   Tyr Ser Phe Glu Val Gln Asn Pro Val Gly Lys Asp Ser Cys Thr Ala
   4325 4330 4335
   Ser Leu Gln Val Ser Asp Arg Thr Val Pro Pro Ser Phe Thr Arg Lys
   4340 4345 4350
   Leu Lys Glu Thr Asn Gly Leu Ser Gly Ser Ser Val Val Met Glu Cys
   4355 4360 4365
   Lys Val Tyr Gly Ser Pro Pro Ile Ser Val Ser Trp Phe His Glu Gly
   4370 4375 4380
   Asn Glu Ile Ser Ser Gly Arg Lys Tyr Gln Thr Thr Leu Thr Asp Asn
   4385 4390 4395 4400
   Thr Cys Ala Leu Thr Val Asn Met Leu Glu Glu Ser Asp Ser Gly Asp
   4405 4410 4415
   Tyr Thr Cys Ile Ala Thr Asn Met Ala Gly Ser Asp Glu Cys Ser Ala
   4420 4425 4430
   Pro Leu Thr Val Arg Glu Pro Pro Ser Phe Val Gln Lys Pro Asp Pro
   4435 4440 4445
   Met Asp Val Leu Thr Gly Thr Asn Val Thr Phe Thr Ser Ile Val Lys
   4450 4455 4460
   Gly Thr Pro Pro Phe Ser Val Ser Trp Phe Lys Gly Ser Ser Glu Leu
   4465 4470 4475 4480
   Val Pro Gly Asp Arg Cys Asn Val Ser Leu Glu Asp Ser Val Ala Glu
   4485 4490 4495
   Leu Glu Leu Phe Asp Val Asp Thr Ser Gln Ser Gly Glu Tyr Thr Cys
   4500 4505 4510
   Ile Val Ser Asn Glu Ala Gly Lys Ala Ser Cys Thr Thr His Leu Tyr
   4515 4520 4525
   Ile Lys Ala Pro Ala Lys Phe Val Lys Arg Leu Asn Asp Tyr Ser Ile
   4530 4535 4540
   Glu Lys Gly Lys Pro Leu Ile Leu Glu Gly Thr Phe Thr Gly Thr Pro
   4545 4550 4555 4560
   Pro Ile Ser Val Thr Trp Lys Lys Asn Gly Ile Asn Val Thr Pro Ser
   4565 4570 4575
   Gln Arg Cys Asn Ile Thr Thr Thr Glu Lys Ser Pro Ile Leu Glu Ile
   4580 4585 4590
   Pro Ser Ser Thr Val Glu Asp Ala Gly Gln Tyr Asn Cys Tyr Ile Glu
   4595 4600 4605
   Asn Ala Ser Gly Lys Asp Ser Cys Ser Ala Gln Ile Leu Ile Leu Glu
   4610 4615 4620
   Pro Pro Tyr Phe Val Lys Gln Leu Glu Pro Val Lys Val Ser Val Gly
   4625 4630 4635 4640
   Asp Ser Ala Ser Leu Gln Cys Gln Leu Ala Gly Thr Pro Glu Ile Gly
   4645 4650 4655
   Val Ser Trp Tyr Lys Gly Asp Thr Lys Leu Arg Pro Thr Thr Thr Tyr
   4660 4665 4670
   Lys Met His Phe Arg Asn Asn Val Ala Thr Leu Val Phe Asn Gln Val
   4675 4680 4685
   Asp Ile Asn Asp Ser Gly Glu Tyr Ile Cys Lys Ala Glu Asn Ser Val
   4690 4695 4700
   Gly Glu Val Ser Ala Ser Thr Phe Leu Thr Val Gln Glu Gln Lys Leu
   4705 4710 4715 4720
   Pro Pro Ser Phe Ser Arg Gln Leu Arg Asp Val Gln Glu Thr Val Gly
   4725 4730 4735
   Leu Pro Val Val Phe Asp Cys Ala Ile Ser Gly Ser Glu Pro Ile Ser
   4740 4745 4750
   Val Ser Trp Tyr Lys Asp Gly Lys Pro Leu Lys Asp Ser Pro Asn Val
   4755 4760 4765
   Gln Thr Ser Phe Leu Asp Asn Thr Ala Thr Leu Asn Ile Phe Lys Thr
   4770 4775 4780
   Asp Arg Ser Leu Ala Gly Gln Tyr Ser Cys Thr Ala Thr Asn Pro Ile
   4785 4790 4795 4800
   Gly Ser Ala Ser Ser Ser Ala Arg Leu Ile Leu Thr Glu Gly Lys Asn
   4805 4810 4815
   Pro Pro Phe Phe Asp Ile Arg Leu Ala Pro Val Asp Ala Val Val Gly
   4820 4825 4830
   Glu Ser Ala Asp Phe Glu Cys His Val Thr Gly Thr Gln Pro Ile Lys
   4835 4840 4845
   Val Ser Trp Ala Lys Asp Ser Arg Glu Ile Arg Ser Gly Gly Lys Tyr
   4850 4855 4860
   Gln Ile Ser Tyr Leu Glu Asn Ser Ala His Leu Thr Val Leu Lys Val
   4865 4870 4875 4880
   Asp Lys Gly Asp Ser Gly Gln Tyr Thr Cys Tyr Ala Val Asn Glu Val
   4885 4890 4895
   Gly Lys Asp Ser Cys Thr Ala Gln Leu Asn Ile Lys Glu Arg Leu Ile
   4900 4905 4910
   Pro Pro Ser Phe Thr Lys Arg Leu Ser Glu Thr Val Glu Glu Thr Glu
   4915 4920 4925
   Gly Asn Ser Phe Lys Leu Glu Gly Arg Val Ala Gly Ser Gln Pro Ile
   4930 4935 4940
   Thr Val Ala Trp Tyr Lys Asn Asn Ile Glu Ile Gln Pro Thr Ser Asn
   4945 4950 4955 4960
   Cys Glu Ile Thr Phe Lys Asn Asn Thr Leu Val Leu Gln Val Arg Lys
   4965 4970 4975
   Ala Gly Met Asn Asp Ala Gly Leu Tyr Thr Cys Lys Val Ser Asn Asp
   4980 4985 4990
   Ala Gly Ser Ala Leu Cys Thr Ser Ser Ile Val Ile Lys Glu Pro Lys
   4995 5000 5005
   Lys Pro Pro Val Phe Asp Gln His Leu Thr Pro Val Thr Val Ser Glu
   5010 5015 5020
   Gly Glu Tyr Val Gln Leu Ser Cys His Val Gln Gly Ser Glu Pro Ile
   5025 5030 5035 5040
   Arg Ile Gln Trp Leu Lys Ala Gly Arg Glu Ile Lys Pro Ser Asp Arg
   5045 5050 5055
   Cys Ser Phe Ser Phe Ala Ser Gly Thr Ala Val Leu Glu Leu Arg Asp
   5060 5065 5070
   Val Ala Lys Ala Asp Ser Gly Asp Tyr Val Cys Lys Ala Ser Asn Val
   5075 5080 5085
   Ala Gly Ser Asp Thr Thr Lys Ser Lys Val Thr Ile Lys Asp Lys Pro
   5090 5095 5100
   Ala Val Ala Pro Ala Thr Lys Lys Ala Ala Val Asp Gly Arg Leu Phe
   5105 5110 5115 5120
   Phe Val Ser Glu Pro Gln Ser Ile Arg Val Val Glu Lys Thr Thr Ala
   5125 5130 5135
   Thr Phe Ile Ala Lys Val Gly Gly Asp Pro Ile Pro Asn Val Lys Trp
   5140 5145 5150
   Thr Lys Gly Lys Trp Arg Gln Leu Asn Gln Gly Gly Arg Val Phe Ile
   5155 5160 5165
   His Gln Lys Gly Asp Glu Ala Lys Leu Glu Ile Arg Asp Thr Thr Lys
   5170 5175 5180
   Thr Asp Ser Gly Leu Tyr Arg Cys Val Ala Phe Asn Glu His Gly Glu
   5185 5190 5195 5200
   Ile Glu Ser Asn Val Asn Leu Gln Val Asp Glu Arg Lys Lys Gln Glu
   5205 5210 5215
   Lys Ile Glu Gly Asp Leu Arg Ala Met Leu Lys Lys Thr Pro Ile Leu
   5220 5225 5230
   Lys Lys Gly Ala Gly Glu Glu Glu Glu Ile Asp Ile Met Glu Leu Leu
   5235 5240 5245
   Lys Asn Val Asp Pro Lys Glu Tyr Glu Lys Tyr Ala Arg Met Tyr Gly
      5250                5255                5260
   Ile Thr Asp Phe Arg Gly Leu Leu Gln Ala Phe Glu Leu Leu Lys Gln
   5265                5270                5275                5280
   Ser Gln Glu Glu Glu Thr His Arg Leu Glu Ile Glu Glu Ile Glu Arg
                   5285                5290                5295
   Ser Glu Arg Asp Glu Lys Glu Phe Glu Glu Leu Val Ser Phe Ile Gln
               5300                5305                5310
   Gln Arg Leu Ser Gln Thr Glu Pro Val Thr Leu Ile Lys Asp Ile Glu
           5315                5320                5325
   Asn Gln Thr Val Leu Lys Asp Asn Asp Ala Val Phe Glu Ile Asp Ile
      5330                5335                5340
   Lys Ile Asn Tyr Pro Glu Ile Lys Leu Ser Trp Tyr Lys Gly Thr Glu
   5345                5350                5355                5360
   Lys Leu Glu Pro Ser Asp Lys Phe Glu Ile Ser Ile Asp Gly Asp Arg
                   5365                5370                5375
   His Thr Leu Arg Val Lys Asn Cys Gln Leu Lys Asp Gln Gly Asn Tyr
               5380                5385                5390
   Arg Leu Val Cys Gly Pro His Ile Ala Ser Ala Lys Leu Thr Val Ile
           5395                5400                5405
   Glu Pro Ala Trp Glu Arg His Leu Gln Asp Val Thr Leu Lys Glu Gly
      5410                5415                5420
   Gln Thr Cys Thr Met Thr Val Gln Phe Ser Val Pro Asn Val Lys Ser
   5425                5430                5435                5440
   Glu Trp Phe Arg Asn Gly Arg Ile Leu Lys Pro Gln Gly Arg His Lys
                   5445                5450                5455
   Thr Glu Val Glu His Lys Val His Lys Leu Thr Ile Ala Asp Val Arg
               5460                5465                5470
   Ala Glu Asp Gln Gly Gln Tyr Thr Cys Lys Tyr Glu Asp Leu Glu Thr
           5475                5480                5485
   Ser Ala Glu Leu Arg Ile Glu Ala Glu Pro Ile Gln Phe Thr Lys Arg
      5490                5495                5500
   Ile Gln Asn Ile Val Val Ser Glu His Gln Ser Ala Thr Phe Glu Cys
   5505                5510                5515                5520
   Glu Val Ser Phe Asp Asp Ala Ile Val Thr Trp Tyr Lys Gly Pro Thr
                   5525                5530                5535
   Glu Leu Thr Glu Ser Gln Lys Tyr Asn Phe Arg Asn Asp Gly Arg Cys
               5540                5545                5550
   His Tyr Met Thr Ile His Asn Val Thr Pro Asp Asp Glu Gly Val Tyr
           5555                5560                5565
   Ser Val Ile Ala Arg Leu Glu Pro Arg Gly Glu Ala Arg Ser Thr Ala
   5570 5575 5580
   Glu Leu Tyr Leu Thr Thr Lys Glu Ile Lys Leu Glu Leu Lys Pro Pro
   5585 5590 5595 5600
   Asp Ile Pro Asp Ser Arg Val Pro Ile Pro Thr Met Pro Ile Arg Ala
   5605 5610 5615
   Val Pro Pro Glu Glu Ile Pro Pro Val Val Ala Pro Pro Val Pro Leu
   5620 5625 5630
   Leu Leu Pro Thr Pro Glu Glu Lys Lys Pro Pro Pro Lys Arg Ile Glu
   5635 5640 5645
   Val Thr Lys Lys Ala Val Lys Lys Asp Ala Lys Lys Val Val Ala Lys
   5650 5655 5660
   Pro Lys Glu Met Thr Pro Arg Glu Glu Ile Val Lys Lys Pro Pro Pro
   5665 5670 5675 5680
   Pro Thr Thr Leu Ile Pro Ala Lys Ala Pro Glu Ile Ile Asp Val Ser
   5685 5690 5695
   Ser Lys Ala Glu Glu Val Lys Ile Met Thr Ile Thr Arg Lys Lys Glu
   5700 5705 5710
   Val Gln Lys Glu Lys Glu Ala Val Tyr Glu Lys Lys Gln Ala Val His
   5715 5720 5725
   Lys Glu Lys Arg Val Phe Ile Glu Ser Phe Glu Glu Pro Tyr Asp Glu
   5730 5735 5740
   Leu Glu Val Glu Pro Tyr Thr Glu Pro Phe Glu Gln Pro Tyr Tyr Glu
   5745 5750 5755 5760
   Glu Pro Asp Glu Asp Tyr Glu Glu Ile Lys Val Glu Ala Lys Lys Glu
   5765 5770 5775
   Val His Glu Glu Trp Glu Glu Asp Phe Glu Glu Gly Gln Glu Tyr Tyr
   5780 5785 5790
   Glu Arg Glu Glu Gly Tyr Asp Glu Gly Glu Glu Glu Trp Glu Glu Ala
   5795 5800 5805
   Tyr Gln Glu Arg Glu Val Ile Gln Val Gln Lys Glu Val Tyr Glu Glu
   5810 5815 5820
   Ser His Glu Arg Lys Val Pro Ala Lys Val Pro Glu Lys Lys Ala Pro
   5825 5830 5835 5840
   Pro Pro Pro Lys Val Ile Lys Lys Pro Val Ile Glu Lys Ile Glu Lys
   5845 5850 5855
   Thr Ser Arg Arg Met Glu Glu Glu Lys Val Gln Val Thr Lys Val Pro
   5860 5865 5870
   Glu Val Ser Lys Lys Ile Val Pro Gln Lys Pro Ser Arg Thr Pro Val
   5875 5880 5885
   Gln Glu Glu Val Ile Glu Val Lys Val Pro Ala Val His Thr Lys Lys
   5890 5895 5900
   Met Val Ile Ser Glu Glu Lys Met Phe Phe Ala Ser His Thr Glu Glu
   5905 5910 5915 5920
   Glu Val Ser Val Thr Val Pro Glu Val Gln Lys Glu Ile Val Thr Glu
   5925 5930 5935
   Glu Lys Ile His Val Ala Val Ser Lys Arg Val Glu Pro Pro Pro Lys
   5940 5945 5950
   Val Pro Glu Leu Pro Glu Lys Pro Ala Pro Glu Glu Val Ala Pro Val
   5955 5960 5965
   Pro Ile Pro Lys Lys Val Glu Pro Pro Ala Pro Lys Val Pro Glu Val
   5970 5975 5980
   Pro Lys Lys Pro Val Pro Glu Glu Lys Lys Pro Val Pro Val Pro Lys
   5985 5990 5995 6000
   Lys Glu Pro Ala Ala Pro Pro Lys Val Pro Glu Val Pro Lys Lys Pro
   6005 6010 6015
   Val Pro Glu Glu Lys Ile Pro Val Pro Val Ala Lys Lys Lys Glu Ala
   6020 6025 6030
   Pro Pro Ala Lys Val Pro Glu Val Gln Lys Gly Val Val Thr Glu Glu
   6035 6040 6045
   Lys Ile Thr Ile Val Thr Gln Arg Glu Glu Ser Pro Pro Pro Ala Val
   6050 6055 6060
   Pro Glu Ile Pro Lys Lys Lys Val Pro Glu Glu Arg Lys Pro Val Pro
   6065 6070 6075 6080
   Arg Lys Glu Glu Glu Val Pro Pro Pro Pro Lys Val Pro Ala Leu Pro
   6085 6090 6095
   Lys Lys Pro Val Pro Glu Glu Lys Val Ala Val Pro Val Pro Val Ala
   6100 6105 6110
   Lys Lys Ala Pro Pro Pro Arg Ala Glu Val Ser Lys Lys Thr Val Val
   6115 6120 6125
   Glu Glu Lys Arg Phe Val Ala Glu Glu Lys Leu Ser Phe Ala Val Pro
   6130 6135 6140
   Gln Arg Val Glu Val Thr Arg His Glu Val Ser Ala Glu Glu Glu Trp
   6145 6150 6155 6160
   Ser Tyr Ser Glu Glu Glu Glu Gly Val Ser Ile Ser Val Tyr Arg Glu
   6165 6170 6175
   Glu Glu Arg Glu Glu Glu Glu Glu Ala Glu Val Thr Glu Tyr Glu Val
   6180 6185 6190
   Met Glu Glu Pro Glu Glu Tyr Val Val Glu Glu Lys Leu His Ile Ile
   6195 6200 6205
   Ser Lys Arg Val Glu Ala Glu Pro Ala Glu Val Thr Glu Arg Gln Glu
   6210 6215 6220
   Lys Lys Ile Val Leu Lys Pro Lys Ile Pro Ala Lys Ile Glu Glu Pro
   6225 6230 6235 6240
   Pro Pro Ala Lys Val Pro Glu Ala Pro Lys Lys Ile Val Pro Glu Lys
   6245 6250 6255
   Lys Val Pro Ala Pro Val Pro Lys Lys Glu Lys Val Pro Pro Pro Lys
   6260 6265 6270
   Val Pro Glu Glu Pro Lys Lys Pro Val Pro Glu Lys Lys Val Pro Pro
   6275 6280 6285
   Lys Val Ile Lys Met Glu Glu Pro Leu Pro Ala Lys Val Thr Glu Lys
   6290 6295 6300
   His Met Gln Ile Thr Gln Glu Glu Lys Val Leu Val Ala Val Thr Lys
   6305 6310 6315 6320
   Lys Glu Ala Pro Pro Lys Ala Arg Val Pro Glu Glu Pro Lys Arg Ala
   6325 6330 6335
   Val Pro Glu Glu Lys Val Leu Lys Leu Lys Pro Lys Arg Glu Glu Glu
   6340 6345 6350
   Pro Pro Ala Lys Val Thr Glu Phe Arg Lys Arg Val Val Lys Glu Glu
   6355 6360 6365
   Lys Val Ser Ile Glu Ala Pro Lys Arg Glu Pro Gln Pro Ile Lys Glu
   6370 6375 6380
   Val Thr Ile Met Glu Glu Lys Glu Arg Ala Tyr Thr Leu Glu Glu Glu
   6385 6390 6395 6400
   Ala Val Ser Val Gln Arg Glu Glu Glu Tyr Glu Glu Tyr Glu Glu Tyr
   6405 6410 6415
   Asp Tyr Lys Glu Phe Glu Glu Tyr Glu Pro Thr Glu Glu Tyr Asp Gln
   6420 6425 6430
   Tyr Glu Glu Tyr Glu Glu Arg Glu Tyr Glu Arg Tyr Glu Glu His Glu
   6435 6440 6445
   Glu Tyr Ile Thr Glu Pro Glu Lys Pro Ile Pro Val Lys Pro Val Pro
   6450 6455 6460
   Glu Glu Pro Val Pro Thr Lys Pro Lys Ala Pro Pro Ala Lys Val Leu
   6465 6470 6475 6480
   Lys Lys Ala Val Pro Glu Glu Lys Val Pro Val Pro Ile Pro Lys Lys
   6485 6490 6495
   Leu Lys Pro Pro Pro Pro Lys Val Pro Glu Glu Pro Lys Lys Val Phe
   6500 6505 6510
   Glu Glu Lys Ile His Ile Ser Ile Thr Lys Arg Glu Lys Glu Gln Val
   6515 6520 6525
   Thr Glu Pro Ala Ala Lys Val Pro Met Lys Pro Lys Arg Val Val Ala
   6530 6535 6540
   Glu Glu Lys Val Pro Val Pro Arg Lys Glu Val Ala Pro Pro Val Arg
   6545 6550 6555 6560
   Val Pro Glu Val Pro Lys Glu Leu Glu Pro Glu Glu Val Ala Phe Glu
   6565 6570 6575
   Glu Glu Val Val Thr His Val Glu Glu Tyr Leu Val Glu Glu Glu Glu
   6580 6585 6590
   Glu Tyr Ile His Glu Glu Glu Glu Phe Ile Thr Glu Glu Glu Val Val
   6595 6600 6605
   Pro Val Ile Pro Val Lys Val Pro Glu Val Pro Arg Lys Pro Val Pro
   6610 6615 6620
   Glu Glu Lys Lys Pro Val Pro Val Pro Lys Lys Lys Glu Ala Pro Pro
   6625 6630 6635 6640
   Ala Lys Val Pro Glu Val Pro Lys Lys Pro Glu Glu Lys Val Pro Val
   6645 6650 6655
   Leu Ile Pro Lys Lys Glu Lys Pro Pro Pro Ala Lys Val Pro Glu Val
   6660 6665 6670
   Pro Lys Lys Pro Val Pro Glu Glu Lys Val Pro Val Pro Val Pro Lys
   6675 6680 6685
   Lys Val Glu Ala Pro Pro Ala Lys Val Pro Glu Val Pro Lys Lys Pro
   6690 6695 6700
   Val Pro Glu Lys Lys Val Pro Val Pro Ala Pro Lys Lys Val Glu Ala
   6705 6710 6715 6720
   Pro Pro Ala Lys Val Pro Glu Val Pro Lys Lys Leu Ile Pro Glu Glu
   6725 6730 6735
   Lys Lys Pro Thr Pro Val Pro Lys Lys Val Glu Ala Pro Pro Pro Lys
   6740 6745 6750
   Val Pro Lys Lys Arg Glu Pro Val Pro Val Pro Val Ala Leu Pro Gln
   6755 6760 6765
   Glu Glu Glu Val Leu Phe Glu Glu Glu Ile Val Pro Glu Glu Glu Val
   6770 6775 6780
   Leu Pro Glu Glu Glu Glu Val Leu Pro Glu Glu Glu Glu Val Leu Pro
   6785 6790 6795 6800
   Glu Glu Glu Glu Val Leu Pro Glu Glu Glu Glu Ile Pro Pro Glu Glu
   6805 6810 6815
   Glu Glu Val Pro Pro Glu Glu Glu Tyr Val Pro Glu Glu Glu Glu Phe
   6820 6825 6830
   Val Pro Glu Glu Glu Val Leu Pro Glu Val Lys Pro Lys Val Pro Val
   6835 6840 6845
   Pro Ala Pro Val Pro Glu Ile Lys Lys Lys Val Thr Glu Lys Lys Val
   6850 6855 6860
   Val Ile Pro Lys Lys Glu Glu Ala Pro Pro Ala Lys Val Pro Glu Val
   6865                6870                6875                6880
   Pro Lys Lys Val Glu Glu Lys Arg Ile Ile Leu Pro Lys Glu Glu Glu
                   6885                6890                6895
   Val Leu Pro Val Glu Val Thr Glu Glu Pro Glu Glu Glu Pro Ile Ser
               6900                6905                6910
   Glu Glu Glu Ile Pro Glu Glu Pro Pro Ser Ile Glu Glu Val Glu Glu
           6915                6920                6925
   Val Ala Pro Pro Arg Val Pro Glu Val Ile Lys Lys Ala Val Pro Glu
       6930                6935                6940
   Ala Pro Thr Pro Val Pro Lys Lys Val Glu Ala Pro Pro Ala Lys Val
   6945                6950                6955                6960
   Ser Lys Lys Ile Pro Glu Glu Lys Val Pro Val Pro Val Gln Lys Lys
                   6965                6970                6975
   Glu Ala Pro Pro Ala Lys Val Pro Glu Val Pro Lys Lys Val Pro Glu
               6980                6985                6990
   Lys Lys Val Leu Val Pro Lys Lys Glu Ala Val Pro Pro Ala Lys Gly
           6995                7000                7005
   Arg Thr Val Leu Glu Glu Lys Val Ser Val Ala Phe Arg Gln Glu Val
       7010                7015                7020
   Val Val Lys Glu Arg Leu Glu Leu Glu Val Val Glu Ala Glu Val Glu
   7025                7030                7035                7040
   Glu Ile Pro Glu Glu Glu Glu Phe His Glu Val Glu Glu Tyr Phe Glu
                   7045                7050                7055
   Glu Gly Glu Phe His Glu Val Glu Glu Phe Ile Lys Leu Glu Gln His
               7060                7065                7070
   Arg Val Glu Glu Glu His Arg Val Glu Lys Val His Arg Val Ile Glu
           7075                7080                7085
   Val Phe Glu Ala Glu Glu Val Glu Val Phe Glu Lys Pro Lys Ala Pro
       7090                7095                7100
   Pro Lys Gly Pro Glu Ile Ser Glu Lys Ile Ile Pro Pro Lys Lys Pro
   7105                7110                7115                7120
   Pro Thr Lys Val Val Pro Arg Lys Glu Pro Pro Ala Lys Val Pro Glu
                   7125                7130                7135
   Val Pro Lys Lys Ile Val Val Glu Glu Lys Val Arg Val Pro Glu Glu
               7140                7145                7150
   Pro Arg Val Pro Pro Thr Lys Val Pro Glu Val Leu Pro Pro Lys Glu
           7155                7160                7165
   Val Val Pro Glu Lys Lys Val Pro Val Pro Pro Ala Lys Lys Pro Glu
       7170                7175                7180
   Ala Pro Pro Pro Lys Val Pro Glu Ala Pro Lys Glu Val Val Pro Glu
   7185                7190                7195                7200
   Lys Lys Val Pro Val Pro Pro Pro Lys Lys Pro Glu Val Pro Pro Thr
   7205 7210 7215
   Lys Val Pro Glu Val Pro Lys Ala Ala Val Pro Glu Lys Lys Val Pro
   7220 7225 7230
   Glu Ala Ile Pro Pro Lys Pro Glu Ser Pro Pro Pro Glu Val Phe Glu
   7235 7240 7245
   Glu Pro Glu Glu Ser Pro Ser Ala Pro Pro Lys Lys Pro Glu Val Pro
   7250 7255 7260
   Pro Val Arg Val Pro Glu Val Pro Lys Glu Val Val Pro Glu Lys Lys
   7265 7270 7275 7280
   Val Pro Ala Ala Pro Pro Lys Lys Pro Glu Val Thr Pro Val Lys Val
   7285 7290 7295
   Pro Glu Ala Pro Lys Glu Val Val Pro Glu Lys Lys Val Pro Val Pro
   7300 7305 7310
   Pro Pro Lys Lys Pro Glu Val Pro Pro Thr Lys Val Pro Glu Val Pro
   7315 7320 7325
   Lys Val Ala Val Pro Glu Lys Lys Val Pro Glu Ala Ile Pro Pro Lys
   7330 7335 7340
   Pro Glu Ser Pro Pro Pro Glu Val Phe Glu Glu Pro Glu Glu Val Ala
   7345 7350 7355 7360
   Leu Glu Glu Pro Pro Ala Glu Val Val Glu Glu Pro Glu Pro Ala Ala
   7365 7370 7375
   Pro Pro Gln Val Thr Val Pro Pro Lys Asn Pro Val Pro Glu Lys Lys
   7380 7385 7390
   Ala Pro Ala Val Val Ala Lys Lys Pro Glu Leu Pro Pro Val Lys Val
   7395 7400 7405
   Pro Glu Val Pro Lys Glu Val Val Pro Glu Lys Lys Val Pro Leu Val
   7410 7415 7420
   Val Pro Lys Lys Pro Glu Ala Pro Pro Ala Lys Val Pro Glu Val Pro
   7425 7430 7435 7440
   Lys Glu Val Val Pro Glu Lys Lys Val Ala Val Pro Lys Lys Pro Glu
   7445 7450 7455
   Val Pro Pro Ala Lys Val Pro Glu Val Pro Lys Lys Pro Val Leu Glu
   7460 7465 7470
   Glu Lys Pro Ala Val Pro Val Pro Glu Arg Ala Glu Ser Pro Pro Pro
   7475 7480 7485
   Glu Val Tyr Glu Glu Pro Glu Glu Ile Ala Pro Glu Glu Glu Ile Ala
   7490 7495 7500
   Pro Glu Glu Glu Lys Pro Val Pro Val Ala Glu Glu Glu Glu Pro Glu
   7505 7510 7515 7520
   Val Pro Pro Pro Ala Val Pro Glu Glu Pro Lys Lys Ile Ile Pro Glu
                   7525                7530                7535
   Lys Lys Val Pro Val Ile Lys Lys Pro Glu Ala Pro Pro Pro Lys Glu
               7540                7545                7550
   Pro Glu Pro Glu Lys Val Ile Glu Lys Pro Lys Leu Lys Pro Arg Pro
           7555                7560                7565
   Pro Pro Pro Pro Pro Ala Pro Pro Lys Glu Asp Val Lys Glu Lys Ile
       7570                7575                7580
   Phe Gln Leu Lys Ala Ile Pro Lys Lys Lys Val Pro Glu Asn Pro Gln
   7585                7590                7595                7600
   Val Pro Glu Lys Val Glu Leu Thr Pro Leu Lys Val Pro Gly Gly Glu
                   7605                7610                7615
   Lys Lys Val Arg Lys Leu Leu Pro Glu Arg Lys Pro Glu Pro Lys Glu
               7620                7625                7630
   Glu Val Val Leu Lys Ser Val Leu Arg Lys Arg Pro Glu Glu Glu Glu
           7635                7640                7645
   Pro Lys Val Glu Pro Lys Lys Leu Glu Lys Val Lys Lys Pro Ala Val
       7650                7655                7660
   Pro Glu Pro Pro Pro Pro Lys Pro Val Glu Glu Val Glu Val Pro Thr
   7665                7670                7675                7680
   Val Thr Lys Arg Glu Arg Lys Ile Pro Glu Pro Thr Lys Val Pro Glu
                   7685                7690                7695
   Ile Lys Pro Ala Ile Pro Leu Pro Ala Pro Glu Pro Lys Pro Lys Pro
               7700                7705                7710
   Glu Ala Glu Val Lys Thr Ile Lys Pro Pro Pro Val Glu Pro Glu Pro
           7715                7720                7725
   Thr Pro Ile Ala Ala Pro Val Thr Val Pro Val Val Gly Lys Lys Ala
       7730                7735                7740
   Glu Ala Lys Ala Pro Lys Glu Glu Ala Ala Lys Pro Lys Gly Pro Ile
   7745                7750                7755                7760
   Lys Gly Val Pro Lys Lys Thr Pro Ser Pro Ile Glu Ala Glu Arg Arg
                   7765                7770                7775
   Lys Leu Arg Pro Gly Ser Gly Gly Glu Lys Pro Pro Asp Glu Ala Pro
               7780                7785                7790
   Phe Thr Tyr Gln Leu Lys Ala Val Pro Leu Lys Phe Val Lys Glu Ile
           7795                7800                7805
   Lys Asp Ile Ile Leu Thr Glu Ser Glu Phe Val Gly Ser Ser Ala Ile
       7810                7815                7820
   Phe Glu Cys Leu Val Ser Pro Ser Thr Ala Ile Thr Thr Trp Met Lys
   7825                7830                7835                7840
   Asp Gly Ser Asn Ile Arg Glu Ser Pro Lys His Arg Phe Ile Ala Asp
   7845 7850 7855
   Gly Lys Asp Arg Lys Leu His Ile Ile Asp Val Gln Leu Ser Asp Ala
   7860 7865 7870
   Gly Glu Tyr Thr Cys Val Leu Arg Leu Gly Asn Lys Glu Lys Thr Ser
   7875 7880 7885
   Thr Ala Lys Leu Val Val Glu Glu Leu Pro Val Arg Phe Val Lys Thr
   7890 7895 7900
   Leu Glu Glu Glu Val Thr Val Val Lys Gly Gln Pro Leu Tyr Leu Ser
   7905 7910 7915 7920
   Cys Glu Leu Asn Lys Glu Arg Asp Val Val Trp Arg Lys Asp Gly Lys
   7925 7930 7935
   Ile Val Val Glu Lys Pro Gly Arg Ile Val Pro Gly Val Ile Gly Leu
   7940 7945 7950
   Met Arg Ala Leu Thr Ile Asn Asp Ala Asp
   7955 7960
<210> 37
   <211> 5198
   <212> PRT
   <213> Caenorhabditis elegans
<220>
   <222> (1)..(5198)
   <223> hemicentin precursor
<300>
   <301> Andersson, et al. Vogel, et al. Lbeit, et al. Ohara, O
   <302> Unknown protein [Homo sapiens].
   <308> GI 3005744
   <309> 1998-04-02
   <313> 1 TO 494
<400> 37
   Met Gly Arg Ser Pro Ser Trp Leu Tyr Gly Val Leu Gly Leu Leu Leu
    1               5                  10                  15
   Leu Ala Thr Thr Cys Ser Ser Val Asn Asp Asp Lys Asn Asp Pro Thr
               20                  25                  30
   Gly Lys Ser Ser Leu Ala Phe Val Phe Asp Ile Thr Gly Ser Met Phe
           35                  40                  45
   Asp Asp Leu Val Gln Val Arg Glu Gly Ala Ala Lys Ile Phe Lys Thr
       50                  55                  60
   Val Met Ala Gln Arg Glu Lys Leu Ile Tyr Asn Tyr Ile Met Val Pro
   65                  70                  75                  80
   Phe His Asp Pro Tyr Leu Gly Glu Ile Ile Asn Thr Thr Asp Ser Thr
                   85                  90                  95
   Tyr Phe Met Arg Gln Leu Ser Lys Val Tyr Val His Gly Gly Gly Asp
               100                 105                 110
   Cys Pro Glu Lys Thr Leu Thr Gly Ile Leu Lys Ala Leu Gln Ile Ser
           115                 120                 125
   Leu Pro Ser Ser Phe Ile Tyr Val Phe Thr Asp Ala Arg Ser Lys Asp
       130                 135                 140
   Tyr His Leu Glu Asp Glu Val Leu Asn Thr Ile Gln Glu Lys Gln Ser
   145                 150                 155                 160
   Ser Val Val Phe Val Met Thr Gly Asp Cys Gly Asn Arg Thr His Pro
                   165                 170                 175
   Gly Phe Arg Thr Tyr Glu Lys Ile Ala Ala Ala Ser Phe Gly Gln Val
               180                 185                 190
   Phe His Leu Glu Lys Ser Asp Val Ser Thr Val Leu Glu Tyr Val Arg
           195                 200                 205
   His Ala Val Lys Gln Lys Lys Val His Leu Met Tyr Glu Ala Arg Glu
       210                 215                 220
   Arg Gly Gly Thr Val Ser Arg Asn Ile Pro Val Asp Lys His Leu Ser
   225                 230                 235                 240
   Glu Leu Thr Ile Ser Leu Ser Gly Asp Lys Asp Asp Ser Asp Asn Leu
                   245                 250                 255
   Asp Ile Val Leu Arg Asp Pro Glu Gly Arg Thr Val Asp Lys Arg Leu
               260                 265                 270
   Tyr Ser Lys Glu Gly Gly Thr Ile Asp Leu Lys Asn Val Lys Leu Ile
           275                 280                 285
   Arg Leu Lys Asp Pro Ser Pro Gly Val Trp Thr Val Asn Thr Asn Ser
       290                 295                 300
   Arg Leu Lys His Thr Ile Arg Val Phe Gly His Gly Ala Val Asp Phe
   305                 310                 315                 320
   Lys Tyr Gly Phe Ala Ser Arg Pro Leu Asp Arg Ile Glu Leu Ala Arg
                   325                 330                 335
   Pro Arg Pro Val Leu Asn Gln Asp Thr Tyr Leu Leu Ile Asn Met Thr
               340                 345                 350
   Gly Leu Ile Pro Pro Gly Thr Val Gly Glu Ile Asp Leu Val Asp Tyr
           355                 360                 365
   His Gly His Ser Leu Tyr Lys Ala Val Ala Ser Pro His Arg Thr Asn
       370                 375                 380
   Pro Asn Met Tyr Phe Ala Gly Pro Phe Val Pro Pro Lys Gly Leu Phe
   385                 390                 395                 400
   Phe Val Arg Val Gln Gly Tyr Asp Glu Asp Asn Tyr Glu Phe Met Arg
                   405                 410                 415
   Ile Ala Pro Thr Ala Ile Gly Ser Val Ile Val Gly Gly Pro Arg Ala
               420                 425                 430
   Phe Met Ser Pro Ile His Gln Glu Phe Val Gly Arg Asp Leu Asn Leu
           435                 440                 445
   Ser Cys Thr Val Glu Ser Ala Ser Ala Tyr Thr Ile Tyr Trp Val Lys
       450                 455                 460
   Thr Gly Glu Asp Ile Ile Gly Gly Pro Leu Phe Tyr His Asn Thr Asp
   465                 470                 475                 480
   Thr Ser Val Trp Thr Ile Pro Glu Leu Ser Leu Lys Asp Ala Gly Glu
                   485                 490                 495
   Tyr Glu Cys Arg Val Ile Ser Asn Asn Gly Asn Tyr Ser Val Lys Thr
               500                 505                 510
   Arg Val Glu Thr Arg Glu Ser Pro Pro Glu Ile Phe Gly Val Arg Asn
           515                 520                 525
   Val Ser Val Pro Leu Gly Glu Ala Ala Phe Leu His Cys Ser Thr Arg
       530                 535                 540
   Ser Ala Gly Glu Val Glu Ile Arg Trp Thr Arg Tyr Gly Ala Thr Val
   545                 550                 555                 560
   Phe Asn Gly Pro Asn Thr Glu Arg Asn Pro Thr Asn Gly Thr Leu Lys
                   565                 570                 575
   Ile His His Val Thr Arg Ala Asp Ala Gly Val Tyr Glu Cys Met Ala
               580                 585                 590
   Arg Asn Ala Gly Gly Met Ser Thr Arg Lys Met Arg Leu Asp Ile Met
           595                 600                 605
   Glu Pro Pro Ser Val Lys Val Thr Pro Gln Asp Val Tyr Phe Asn Met
       610                 615                 620
   Arg Glu Gly Val Asn Leu Ser Cys Glu Ala Met Gly Asp Pro Lys Pro
   625                 630                 635                 640
   Glu Val His Trp Tyr Phe Lys Gly Arg His Leu Leu Asn Asp Tyr Lys
                   645                 650                 655
   Tyr Gln Val Gly Gln Asp Ser Lys Phe Leu Tyr Ile Arg Asp Ala Thr
               660                 665                 670
   His His Asp Glu Gly Thr Tyr Glu Cys Arg Ala Met Ser Gln Ala Gly
           675                 680                 685
   Gln Ala Arg Asp Thr Thr Asp Leu Met Leu Ala Thr Pro Pro Lys Val
       690                 695                 700
   Glu Ile Ile Gln Asn Lys Met Met Val Gly Arg Gly Asp Arg Val Ser
   705                 710                 715                 720
   Phe Glu Cys Lys Thr Ile Arg Gly Lys Pro His Pro Lys Ile Arg Trp
                   725                 730                 735
   Phe Lys Asn Gly Lys Asp Leu Ile Lys Pro Asp Asp Tyr Ile Lys Ile
   740 745 750
   Asn Glu Gly Gln Leu His Ile Met Gly Ala Lys Asp Glu Asp Ala Gly
   755 760 765
   Ala Tyr Ser Cys Val Gly Glu Asn Met Ala Gly Lys Asp Val Gln Val
   770 775 780
   Ala Asn Leu Ser Val Gly Arg Val Pro Thr Ile Ile Glu Ser Pro His
   785 790 795 800
   Thr Val Arg Val Asn Ile Glu Arg Gln Val Thr Leu Gln Cys Leu Ala
   805 810 815
   Val Gly Ile Pro Pro Pro Glu Ile Glu Trp Gln Lys Gly Asn Val Leu
   820 825 830
   Leu Ala Thr Leu Asn Asn Pro Arg Tyr Thr Gln Leu Ala Asp Gly Asn
   835 840 845
   Leu Leu Ile Thr Asp Ala Gln Ile Glu Asp Gln Gly Gln Phe Thr Cys
   850 855 860
   Ile Ala Arg Asn Thr Tyr Gly Gln Gln Ser Gln Ser Thr Thr Leu Met
   865 870 875 880
   Val Thr Gly Leu Val Ser Pro Val Leu Gly His Val Pro Pro Glu Glu
   885 890 895
   Gln Leu Ile Glu Gly Gln Asp Leu Thr Leu Ser Cys Val Val Val Leu
   900 905 910
   Gly Thr Pro Lys Pro Ser Ile Val Trp Ile Lys Asp Asp Lys Pro Val
   915 920 925
   Glu Glu Gly Pro Thr Ile Lys Ile Glu Gly Gly Gly Ser Leu Leu Arg
   930 935 940
   Leu Arg Gly Gly Asn Pro Lys Asp Glu Gly Lys Tyr Thr Cys Ile Ala
   945 950 955 960
   Val Ser Pro Ala Gly Asn Ser Thr Leu His Ile Asn Val Gln Leu Ile
   965 970 975
   Lys Lys Pro Glu Phe Val Tyr Lys Pro Glu Gly Gly Ile Val Phe Lys
   980 985 990
   Pro Thr Ile Ser Gly Met Asp Glu Lys His Val Ala Val Val Asn Ser
   995 1000 1005
   Thr His Asp Val Leu Asp Gly Glu Gly Phe Ala Ile Pro Cys Val Val
   1010 1015 1020
   Ser Gly Thr Pro Pro Pro Ile Ile Thr Trp Tyr Leu Asp Gly Arg Pro
   1025 1030 1035 1040
   Ile Thr Pro Asn Ser Arg Asp Phe Thr Val Thr Ala Asp Asn Thr Leu
   1045 1050 1055
   Ile Val Arg Lys Ala Asp Lys Ser Tyr Ser Gly Val Tyr Thr Cys Gln
   1060 1065 1070
   Ala Thr Asn Ser Ala Gly Asp Asn Glu Gln Lys Thr Thr Ile Arg Ile
         1075                1080                1085
   Met Asn Thr Pro Met Ile Ser Pro Gly Gln Ser Ser Phe Asn Met Val
      1090                1095                1100
   Val Asp Asp Leu Phe Thr Ile Pro Cys Asp Val Tyr Gly Asp Pro Lys
   1105                1110                1115                1120
   Pro Val Ile Thr Trp Leu Leu Asp Asp Lys Pro Phe Thr Glu Gly Val
                  1125                1130                1135
   Val Asn Glu Asp Gly Ser Leu Thr Ile Pro Asn Val Asn Glu Ala His
               1140                1145                1150
   Arg Gly Thr Phe Thr Cys His Ala Gln Asn Ala Ala Gly Asn Asp Thr
         1155                1160                1165
   Arg Thr Val Thr Leu Thr Val His Thr Thr Pro Thr Ile Asn Ala Glu
      1170                1175                1180
   Asn Gln Glu Lys Ile Ala Leu Gln Asn Asp Asp Ile Val Leu Glu Cys
   1185                1190                1195                1200
   Pro Ala Lys Ala Leu Pro Pro Pro Val Arg Leu Trp Thr Tyr Glu Gly
                  1205                1210                1215
   Glu Lys Ile Asp Ser Gln Leu Ile Pro His Thr Ile Arg Glu Asp Gly
               1220                1225                1230
   Ala Leu Val Leu Gln Asn Val Lys Leu Glu Asn Thr Gly Val Phe Val
         1235                1240                1245
   Cys Gln Val Ser Asn Leu Ala Gly Glu Asp Ser Leu Ser Tyr Thr Leu
      1250                1255                1260
   Thr Val His Glu Lys Pro Lys Ile Ile Ser Glu Val Pro Gly Val Val
   1265                1270                1275                1280
   Asp Val Val Lys Gly Phe Thr Ile Glu Ile Pro Cys Arg Ala Thr Gly
                  1285                1290                1295
   Val Pro Glu Val Ile Arg Thr Trp Asn Lys Asn Gly Ile Asp Leu Lys
               1300                1305                1310
   Met Asp Glu Lys Lys Phe Ser Val Asp Asn Leu Gly Thr Leu Arg Ile
         1315                1320                1325
   Tyr Glu Ala Asp Lys Asn Asp Ile Gly Asn Tyr Asn Cys Val Val Thr
      1330                1335                1340
   Asn Glu Ala Gly Thr Ser Gln Met Thr Thr His Val Asp Val Gln Glu
   1345                1350                1355                1360
   Pro Pro Ile Ile Leu Pro Ser Thr Gln Thr Asn Asn Thr Ala Val Val
                  1365                1370                1375
   Gly Asp Arg Val Glu Leu Lys Cys Tyr Val Glu Ala Ser Pro Pro Ala
               1380                1385                1390
   Ser Val Thr Trp Phe Arg Arg Gly Ile Ala Ile Gly Thr Asp Thr Lys
   1395 1400 1405
   Gly Tyr Val Val Glu Ser Asp Gly Thr Leu Val Ile Gln Ser Ala Ser
   1410 1415 1420
   Val Glu Asp Ala Thr Ile Tyr Thr Cys Lys Ala Ser Asn Pro Ala Gly
   1425 1430 1435 1440
   Lys Ala Glu Ala Asn Leu Gln Val Thr Val Ile Ala Ser Pro Asp Ile
   1445 1450 1455
   Lys Asp Pro Asp Val Val Thr Gln Glu Ser Ile Lys Glu Ser His Pro
   1460 1465 1470
   Phe Ser Leu Tyr Cys Pro Val Phe Ser Asn Pro Leu Pro Gln Ile Ser
   1475 1480 1485
   Trp Tyr Leu Asn Asp Lys Pro Leu Ile Asp Asp Lys Thr Ser Trp Lys
   1490 1495 1500
   Thr Ser Asp Asp Lys Arg Lys Leu His Val Phe Lys Ala Lys Ile Thr
   1505 1510 1515 1520
   Asp Ser Gly Val Tyr Lys Cys Val Ala Arg Asn Ala Ala Gly Glu Gly
   1525 1530 1535
   Ser Lys Ser Phe Gln Val Glu Val Ile Val Pro Leu Asn Leu Asp Glu
   1540 1545 1550
   Ser Lys Tyr Lys Lys Lys Val Phe Ala Lys Glu Gly Glu Glu Val Thr
   1555 1560 1565
   Leu Gly Cys Pro Val Ser Gly Phe Pro Val Pro Gln Ile Asn Trp Val
   1570 1575 1580
   Val Asp Gly Thr Val Val Glu Pro Gly Lys Lys Tyr Lys Gly Ala Thr
   1585 1590 1595 1600
   Leu Ser Asn Asp Gly Leu Thr Leu His Phe Asp Ser Val Ser Val Lys
   1605 1610 1615
   Gln Glu Gly Asn Tyr His Cys Val Ala Gln Ser Lys Gly Asn Ile Leu
   1620 1625 1630
   Asp Ile Asp Val Glu Leu Ser Val Leu Ala Val Pro Ile Val Gly Glu
   1635 1640 1645
   Asp Asp Asn Leu Glu Val Phe Leu Gly Lys Asp Ile Ser Leu Ser Cys
   1650 1655 1660
   Asp Leu Gln Thr Glu Ser Asp Asp Lys Thr Thr Phe Val Trp Ser Ile
   1665 1670 1675 1680
   Asn Gly Ser Glu Ser Asp Arg Pro Asp Asn Val Gln Ile Pro Ser Asp
   1685 1690 1695
   Gly His Arg Leu Tyr Ile Thr Asp Ala Lys Pro Glu Asn Asn Gly Lys
   1700 1705 1710
   Tyr Met Cys Arg Val Thr Asn Ser Ala Gly Lys Ala Glu Arg Thr Leu
   1715 1720 1725
   Thr Leu Asp Val Leu Glu Pro Pro Val Phe Val Glu Pro Val Phe Glu
   1730 1735 1740
   Ala Asn Gln Lys Leu Ile Gly Asn Asn Pro Ile Ile Leu Gln Cys Gln
   1745 1750 1755 1760
   Val Thr Gly Asn Pro Lys Pro Thr Val Ile Trp Lys Ile Asp Gly Asn
   1765 1770 1775
   Asp Val Asp Lys Ser Trp Leu Phe Asp Glu Ser Leu Ser Leu Leu Arg
   1780 1785 1790
   Ile Glu Lys Leu Thr Gly Lys Ser Ala Gln Ile Ser Cys Thr Ala Glu
   1795 1800 1805
   Asn Lys Ala Gly Thr Ala Ser Arg Asp Phe Phe Ile Gln Asn Ile Ala
   1810 1815 1820
   Ala Pro Thr Phe Lys Asn Glu Gly Asp Gln Glu Thr Ile Phe Arg Glu
   1825 1830 1835 1840
   Ser Glu Thr Ile Thr Leu Asp Cys Pro Val Ser Leu Gly Asp Phe Gln
   1845 1850 1855
   Ile Thr Trp Met Lys Gln Gly Leu Pro Leu Thr Glu Asn Asp Ala Ile
   1860 1865 1870
   Phe Thr Leu Asp Asn Thr Arg Leu Thr Ile Leu Asn Ala Asn Arg Asp
   1875 1880 1885
   His Glu Asp Ile Tyr Thr Cys Val Ala Asn Asn Thr Ala Gly Gln Val
   1890 1895 1900
   Ser Lys Asp Phe Asp Val Val Val Gln Val Leu Pro Lys Ile Lys Asn
   1905 1910 1915 1920
   Ala Val Val Thr Leu Glu Ile Asn Glu Gly Glu Glu Ile Ile Leu Thr
   1925 1930 1935
   Cys Asp Ala Glu Gly Asn Pro Thr Pro Thr Ala Lys Trp Asp Phe Asn
   1940 1945 1950
   Gln Gly Asp Leu Pro Lys Glu Ala Val Phe Val Asn Asn Asn His Thr
   1955 1960 1965
   Val Val Val Asn Asn Val Thr Lys Tyr His Thr Gly Val Tyr Lys Cys
   1970 1975 1980
   Tyr Ala Thr Asn Lys Val Gly Gln Ala Val Lys Thr Ile Asn Val His
   1985 1990 1995 2000
   Val Arg Thr Lys Pro Arg Phe Glu Ser Gly Leu Thr Glu Ser Glu Leu
   2005 2010 2015
   Thr Val Asn Leu Thr Arg Ser Ile Thr Leu Glu Cys Asp Val Asp Asp
   2020 2025 2030
   Ala Ile Gly Val Gly Ile Ser Trp Thr Val Asn Gly Lys Pro Phe Leu
   2035 2040 2045
   Ala Glu Thr Asp Gly Val Gln Thr Leu Ala Gly Gly Arg Phe Leu His
   2050 2055 2060
   Ile Val Ser Ala Lys Thr Asp Asp His Gly Ser Tyr Ala Cys Thr Val
   2065 2070 2075 2080
   Thr Asn Glu Ala Gly Val Ala Thr Lys Thr Phe Asn Leu Phe Val Gln
   2085 2090 2095
   Val Pro Pro Thr Ile Val Asn Glu Gly Gly Glu Tyr Thr Val Ile Glu
   2100 2105 2110
   Asn Asn Ser Leu Val Leu Pro Cys Glu Val Thr Gly Lys Pro Asn Pro
   2115 2120 2125
   Val Val Thr Trp Thr Lys Asp Gly Arg Pro Val Gly Asp Leu Lys Ser
   2130 2135 2140
   Val Gln Val Leu Ser Glu Gly Gln Gln Phe Lys Ile Val His Ala Glu
   2145 2150 2155 2160
   Ile Ala His Lys Gly Ser Tyr Ile Cys Met Ala Lys Asn Asp Val Gly
   2165 2170 2175
   Thr Ala Glu Ile Ser Phe Asp Val Asp Ile Ile Thr Arg Pro Met Ile
   2180 2185 2190
   Gln Lys Gly Ile Lys Asn Ile Val Thr Ala Ile Lys Gly Gly Ala Leu
   2195 2200 2205
   Pro Phe Lys Cys Pro Ile Asp Asp Asp Lys Asn Phe Lys Gly Gln Ile
   2210 2215 2220
   Ile Trp Leu Arg Asn Tyr Gln Pro Ile Asp Leu Glu Ala Glu Asp Ala
   2225 2230 2235 2240
   Arg Ile Thr Arg Leu Ser Asn Asp Arg Arg Leu Thr Ile Leu Asn Val
   2245 2250 2255
   Thr Glu Asn Asp Glu Gly Gln Tyr Ser Cys Arg Val Lys Asn Asp Ala
   2260 2265 2270
   Gly Glu Asn Ser Phe Asp Phe Lys Ala Thr Val Leu Val Pro Pro Thr
   2275 2280 2285
   Ile Ile Met Leu Asp Lys Asp Lys Asn Lys Thr Ala Val Glu His Ser
   2290 2295 2300
   Thr Val Thr Leu Ser Cys Pro Ala Thr Gly Lys Pro Glu Pro Asp Ile
   2305 2310 2315 2320
   Thr Trp Phe Lys Asp Gly Glu Ala Ile His Ile Glu Asn Ile Ala Asp
   2325 2330 2335
   Ile Ile Pro Asn Gly Glu Leu Asn Gly Asn Gln Leu Lys Ile Thr Arg
   2340 2345 2350
   Ile Lys Glu Gly Asp Ala Gly Lys Tyr Thr Cys Glu Ala Asp Asn Ser
   2355 2360 2365
   Ala Gly Ser Val Glu Gln Asp Val Asn Val Asn Val Ile Thr Ile Pro
   2370 2375 2380
   Lys Ile Glu Lys Asp Gly Ile Pro Ser Asp Tyr Glu Ser Gln Gln Asn
   2385 2390 2395 2400
   Glu Arg Val Val Ile Ser Cys Pro Val Tyr Ala Arg Pro Pro Ala Lys
   2405 2410 2415
   Ile Thr Trp Leu Lys Ala Gly Lys Pro Leu Gln Ser Asp Lys Phe Val
   2420 2425 2430
   Lys Thr Ser Ala Asn Gly Gln Lys Leu Tyr Leu Phe Lys Leu Arg Glu
   2435 2440 2445
   Thr Asp Ser Ser Lys Tyr Thr Cys Ile Ala Thr Asn Glu Ala Gly Thr
   2450 2455 2460
   Asp Lys Arg Asp Phe Lys Val Ser Met Leu Val Ala Pro Ser Phe Asp
   2465 2470 2475 2480
   Glu Pro Asn Ile Val Arg Arg Ile Thr Val Asn Ser Gly Asn Pro Ser
   2485 2490 2495
   Thr Leu His Cys Pro Ala Lys Gly Ser Pro Ser Pro Thr Ile Thr Trp
   2500 2505 2510
   Leu Lys Asp Gly Asn Ala Ile Glu Pro Asn Asp Arg Tyr Val Phe Phe
   2515 2520 2525
   Asp Ala Gly Arg Gln Leu Gln Ile Ser Lys Thr Glu Gly Ser Asp Gln
   2530 2535 2540
   Gly Arg Tyr Thr Cys Ile Ala Thr Asn Ser Val Gly Ser Asp Asp Leu
   2545 2550 2555 2560
   Glu Asn Thr Leu Glu Val Ile Ile Pro Pro Val Ile Asp Gly Glu Arg
   2565 2570 2575
   Arg Glu Ala Val Ala Val Ile Glu Gly Phe Ser Ser Glu Leu Phe Cys
   2580 2585 2590
   Asp Ser Asn Ser Thr Gly Val Asp Val Glu Trp Gln Lys Asp Gly Leu
   2595 2600 2605
   Thr Ile Asn Gln Asp Thr Leu Arg Gly Asp Ser Phe Ile Gln Ile Pro
   2610 2615 2620
   Ser Ser Gly Lys Lys Met Ser Phe Leu Ser Ala Arg Lys Ser Asp Ser
   2625 2630 2635 2640
   Gly Arg Tyr Thr Cys Ile Val Arg Asn Pro Ala Gly Glu Ala Arg Lys
   2645 2650 2655
   Leu Phe Asp Phe Ala Val Asn Asp Pro Pro Ser Ile Ser Asp Glu Leu
   2660 2665 2670
   Ser Ser Ala Asn Ile Gln Thr Ile Val Pro Tyr Tyr Pro Val Glu Ile
   2675 2680 2685
   Asn Cys Val Val Ser Gly Ser Pro His Pro Lys Val Tyr Trp Leu Phe
   2690 2695 2700
   Asp Asp Lys Pro Leu Glu Pro Asp Ser Ala Ala Tyr Glu Leu Thr Asn
   2705 2710 2715 2720
   Asn Gly Glu Thr Leu Lys Ile Val Arg Ser Gln Val Glu His Ala Gly
   2725 2730 2735
   Thr Tyr Thr Cys Glu Ala Gln Asn Asn Val Gly Lys Ala Arg Lys Asp
   2740 2745 2750
   Phe Leu Val Arg Val Thr Ala Pro Pro His Phe Glu Lys Glu Arg Glu
   2755 2760 2765
   Glu Val Val Ala Arg Val Gly Asp Thr Met Leu Leu Thr Cys Asn Ala
   2770 2775 2780
   Glu Ser Ser Val Pro Leu Ser Ser Val Tyr Trp His Ala His Asp Glu
   2785 2790 2795 2800
   Ser Val Gln Asn Gly Val Ile Thr Ser Lys Tyr Ala Ala Asn Glu Lys
   2805 2810 2815
   Thr Leu Asn Val Thr Asn Ile Gln Leu Asp Asp Glu Gly Phe Tyr Tyr
   2820 2825 2830
   Cys Thr Ala Val Asn Glu Ala Gly Ile Thr Lys Lys Phe Phe Lys Leu
   2835 2840 2845
   Ile Val Ile Glu Thr Pro Tyr Phe Leu Asp Gln Gln Lys Leu Tyr Pro
   2850 2855 2860
   Ile Ile Leu Gly Lys Arg Leu Thr Leu Asp Cys Ser Ala Thr Gly Thr
   2865 2870 2875 2880
   Pro Pro Pro Thr Ile Leu Phe Met Lys Asp Gly Lys Arg Leu Asn Glu
   2885 2890 2895
   Ser Asp Glu Val Asp Ile Ile Gly Ser Thr Leu Val Ile Asp Asn Pro
   2900 2905 2910
   Gln Lys Glu Val Glu Gly Arg Tyr Thr Cys Ile Ala Glu Asn Lys Ala
   2915 2920 2925
   Gly Arg Ser Glu Lys Asp Met Met Val Glu Val Leu Leu Pro Pro Lys
   2930 2935 2940
   Leu Ser Lys Glu Trp Ile Asn Val Glu Val Gln Ala Gly Asp Pro Leu
   2945 2950 2955 2960
   Thr Leu Glu Cys Pro Ile Glu Asp Thr Ser Gly Val His Ile Thr Trp
   2965 2970 2975
   Ser Arg Gln Phe Gly Lys Asp Gly Gln Leu Asp Met Arg Ala Gln Ser
   2980 2985 2990
   Ser Ser Asp Lys Ser Lys Leu Tyr Ile Met Gln Ala Thr Pro Glu Asp
   2995 3000 3005
   Ala Asp Ser Tyr Ser Cys Ile Ala Val Asn Asp Ala Gly Gly Ala Glu
   3010 3015 3020
   Ala Val Phe Gln Val Thr Val Asn Thr Pro Pro Lys Ile Phe Gly Asp
   3025 3030 3035 3040
   Ser Phe Ser Thr Thr Glu Ile Val Ala Asp Thr Thr Leu Glu Ile Pro
   3045 3050 3055
   Cys Arg Thr Glu Gly Ile Pro Pro Pro Glu Ile Ser Trp Phe Leu Asp
   3060 3065 3070
   Gly Lys Pro Ile Leu Glu Met Pro Gly Val Thr Tyr Lys Gln Gly Asp
   3075 3080 3085
   Leu Ser Leu Arg Ile Asp Asn Ile Lys Pro Asn Gln Glu Gly Arg Tyr
   3090 3095 3100
   Thr Cys Val Ala Glu Asn Lys Ala Gly Arg Ala Glu Gln Asp Thr Tyr
   3105 3110 3115 3120
   Val Glu Ile Ser Glu Pro Pro Arg Val Val Met Ala Ser Glu Val Met
   3125 3130 3135
   Arg Val Val Glu Gly Arg Gln Thr Thr Ile Arg Cys Glu Val Phe Gly
   3140 3145 3150
   Asn Pro Glu Pro Val Val Asn Trp Leu Lys Asp Gly Glu Pro Tyr Thr
   3155 3160 3165
   Ser Asp Leu Leu Gln Phe Ser Thr Lys Leu Ser Tyr Leu His Leu Arg
   3170 3175 3180
   Glu Thr Thr Leu Ala Asp Gly Gly Thr Tyr Thr Cys Ile Ala Thr Asn
   3185 3190 3195 3200
   Lys Ala Gly Glu Ser Gln Thr Thr Thr Asp Val Glu Val Leu Val Pro
   3205 3210 3215
   Pro Arg Ile Glu Asp Glu Glu Arg Val Leu Gln Gly Lys Glu Gly Asn
   3220 3225 3230
   Thr Tyr Met Val His Cys Gln Val Thr Gly Arg Pro Val Pro Tyr Val
   3235 3240 3245
   Thr Trp Lys Arg Asn Gly Lys Glu Ile Glu Gln Phe Asn Pro Val Leu
   3250 3255 3260
   His Ile Arg Asn Ala Thr Arg Ala Asp Glu Gly Lys Tyr Ser Cys Ile
   3265 3270 3275 3280
   Ala Ser Asn Glu Ala Gly Thr Ala Val Ala Asp Phe Leu Ile Asp Val
   3285 3290 3295
   Phe Thr Lys Pro Thr Phe Glu Thr His Glu Thr Thr Phe Asn Ile Val
   3300 3305 3310
   Glu Gly Glu Ser Ala Lys Ile Glu Cys Lys Ile Asp Gly His Pro Lys
   3315 3320 3325
   Pro Thr Ile Ser Trp Leu Lys Gly Gly Arg Pro Phe Asn Met Asp Asn
   3330 3335 3340
   Ile Ile Leu Ser Pro Arg Gly Asp Thr Leu Met Ile Leu Lys Ala Gln
   3345 3350 3355 3360
   Arg Phe Asp Gly Gly Leu Tyr Thr Cys Val Ala Thr Asn Ser Tyr Gly
   3365 3370 3375
   Asp Ser Glu Gln Asp Phe Lys Val Asn Val Tyr Thr Lys Pro Tyr Ile
   3380 3385 3390
   Asp Glu Thr Ile Asp Gln Thr Pro Lys Ala Val Ala Gly Gly Glu Ile
   3395 3400 3405
   Ile Leu Lys Cys Pro Val Leu Gly Asn Pro Thr Pro Thr Val Thr Trp
   3410 3415 3420
   Lys Arg Gly Asp Asp Ala Val Pro Asn Asp Ser Arg His Thr Ile Val
   3425 3430 3435 3440
   Asn Asn Tyr Asp Leu Lys Ile Asn Ser Val Thr Thr Glu Asp Ala Gly
   3445 3450 3455
   Gln Tyr Ser Cys Ile Ala Val Asn Glu Ala Gly Asn Leu Thr Thr His
   3460 3465 3470
   Tyr Ala Ala Glu Val Ile Gly Lys Pro Thr Phe Val Arg Lys Gly Gly
   3475 3480 3485
   Asn Leu Tyr Glu Val Ile Glu Asn Asp Thr Ile Thr Met Asp Cys Gly
   3490 3495 3500
   Val Thr Ser Arg Pro Leu Pro Ser Ile Ser Trp Phe Arg Gly Asp Lys
   3505 3510 3515 3520
   Pro Val Tyr Leu Tyr Asp Arg Tyr Ser Ile Ser Pro Asp Gly Ser His
   3525 3530 3535
   Ile Thr Ile Asn Lys Ala Lys Leu Ser Asp Gly Gly Lys Tyr Ile Cys
   3540 3545 3550
   Arg Ala Ser Asn Glu Ala Gly Thr Ser Asp Ile Asp Leu Ile Leu Lys
   3555 3560 3565
   Ile Leu Val Pro Pro Lys Ile Asp Lys Ser Asn Ile Ile Gly Asn Pro
   3570 3575 3580
   Leu Ala Ile Val Ala Arg Thr Ile Tyr Leu Glu Cys Pro Ile Ser Gly
   3585 3590 3595 3600
   Ile Pro Gln Pro Asp Val Ile Trp Thr Lys Asn Gly Met Asp Ile Asn
   3605 3610 3615
   Met Thr Asp Ser Arg Val Ile Leu Ala Gln Asn Asn Glu Thr Phe Gly
   3620 3625 3630
   Ile Glu Asn Val Gln Val Thr Asp Gln Gly Arg Tyr Thr Cys Thr Ala
   3635 3640 3645
   Thr Asn Arg Gly Gly Lys Ala Ser His Asp Phe Ser Leu Asp Val Leu
   3650 3655 3660
   Ser Pro Pro Glu Phe Asp Ile His Gly Thr Gln Pro Thr Ile Lys Arg
   3665 3670 3675 3680
   Glu Gly Asp Thr Ile Thr Leu Thr Cys Pro Ile Lys Leu Ala Glu Asp
   3685 3690 3695
   Ile Ala Asp Gln Val Met Asp Val Ser Trp Thr Lys Asp Ser Arg Ala
   3700 3705 3710
   Leu Asp Gly Asp Leu Thr Asp Asn Val Asp Ile Ser Asp Asp Gly Arg
   3715 3720 3725
   Lys Leu Thr Ile Ser Gln Ala Ser Leu Glu Asn Ala Gly Leu Tyr Thr
   3730 3735 3740
   Cys Ile Ala Leu Asn Arg Ala Gly Glu Ala Ser Leu Glu Phe Lys Val
   3745 3750 3755 3760
   Glu Ile Leu Ser Pro Pro Val Ile Asp Ile Ser Arg Asn Asp Val Gln
   3765 3770 3775
   Pro Gln Val Ala Val Asn Gln Pro Thr Ile Met Arg Cys Ala Val Thr
   3780 3785 3790
   Gly His Pro Phe Pro Ser Ile Lys Trp Leu Lys Asn Gly Lys Glu Val
   3795 3800 3805
   Thr Asp Asp Glu Asn Ile Arg Ile Val Glu Gln Gly Gln Val Leu Gln
   3810 3815 3820
   Ile Leu Arg Thr Asp Ser Asp His Ala Gly Lys Trp Ser Cys Val Ala
   3825 3830 3835 3840
   Glu Asn Asp Ala Gly Val Lys Glu Leu Glu Met Val Leu Asp Val Phe
   3845 3850 3855
   Thr Pro Pro Val Val Ser Val Lys Ser Asp Asn Pro Ile Lys Ala Leu
   3860 3865 3870
   Gly Glu Thr Ile Thr Leu Phe Cys Asn Ala Ser Gly Asn Pro Tyr Pro
   3875 3880 3885
   Gln Leu Lys Trp Ala Lys Gly Gly Ser Leu Ile Phe Asp Ser Pro Asp
   3890 3895 3900
   Gly Ala Arg Ile Ser Leu Lys Gly Ala Arg Leu Asp Ile Pro His Leu
   3905 3910 3915 3920
   Lys Lys Thr Asp Val Gly Asp Tyr Thr Cys Gln Ala Leu Asn Ala Ala
   3925 3930 3935
   Gly Thr Ser Glu Ala Ser Val Ser Val Asp Val Leu Val Pro Pro Glu
   3940 3945 3950
   Ile Asn Arg Asp Gly Ile Asp Met Ser Pro Arg Leu Pro Ala Gln Gln
   3955 3960 3965
   Ser Leu Thr Leu Gln Cys Leu Ala Gln Gly Lys Pro Val Pro Gln Met
   3970 3975 3980
   Arg Trp Thr Leu Asn Gly Thr Ala Leu Thr His Ser Thr Pro Gly Ile
   3985 3990 3995 4000
   Thr Val Ala Ser Asp Ser Thr Phe Ile Gln Ile Asn Asn Val Ser Leu
   4005 4010 4015
   Ser Asp Lys Gly Val Tyr Thr Cys Tyr Ala Glu Asn Val Ala Gly Ser
   4020 4025 4030
   Asp Asn Leu Met Tyr Asn Val Asp Val Val Gln Ala Pro Val Ile Ser
   4035 4040 4045
   Asn Gly Gly Thr Lys Gln Val Ile Glu Gly Glu Leu Ala Val Ile Glu
   4050 4055 4060
   Cys Leu Val Glu Gly Tyr Pro Ala Pro Gln Val Ser Trp Leu Arg Asn
   4065 4070 4075 4080
   Gly Asn Arg Val Glu Thr Gly Val Gln Gly Val Arg Tyr Val Thr Asp
   4085 4090 4095
   Gly Arg Met Leu Thr Ile Ile Glu Ala Arg Ser Leu Asp Ser Gly Ile
   4100 4105 4110
   Tyr Leu Cys Ser Ala Thr Asn Glu Ala Gly Ser Ala Gln Gln Ala Tyr
   4115 4120 4125
   Thr Leu Glu Val Leu Val Ser Pro Lys Ile Ile Thr Ser Thr Pro Gly
   4130 4135 4140
   Val Leu Thr Pro Ser Ser Gly Ser Lys Phe Ser Leu Pro Cys Ala Val
   4145 4150 4155 4160
   Arg Gly Tyr Pro Asp Pro Ile Ile Ser Trp Thr Leu Asn Gly Asn Asp
   4165 4170 4175
   Ile Lys Asp Gly Glu Asn Gly His Thr Ile Gly Ala Asp Gly Thr Leu
   4180 4185 4190
   His Ile Glu Lys Ala Glu Glu Arg His Leu Ile Tyr Glu Cys Thr Ala
   4195 4200 4205
   Lys Asn Asp Ala Gly Ala Asp Thr Leu Glu Phe Pro Val Gln Thr Ile
   4210 4215 4220
   Val Ala Pro Lys Ile Ser Thr Ser Gly Asn Arg Tyr Ile Asn Gly Ser
   4225 4230 4235 4240
   Glu Gly Thr Glu Thr Val Ile Lys Cys Glu Ile Glu Ser Glu Ser Ser
   4245 4250 4255
   Glu Phe Ser Trp Ser Lys Asn Gly Val Pro Leu Leu Pro Ser Asn Asn
   4260 4265 4270
   Leu Ile Phe Ser Glu Asp Tyr Lys Leu Ile Lys Ile Leu Ser Thr Arg
   4275 4280 4285
   Leu Ser Asp Gln Gly Glu Tyr Ser Cys Thr Ala Ala Asn Lys Ala Gly
   4290 4295 4300
   Asn Ala Thr Gln Lys Thr Asn Leu Asn Val Gly Val Ala Pro Lys Ile
   4305 4310 4315 4320
   Met Glu Arg Pro Arg Thr Gln Val Val His Lys Gly Asp Gln Val Thr
   4325 4330 4335
   Leu Trp Cys Glu Ala Ser Gly Val Pro Gln Pro Ala Ile Thr Trp Tyr
   4340 4345 4350
   Lys Asp Asn Glu Leu Leu Thr Asn Thr Gly Val Asp Glu Thr Ala Thr
   4355 4360 4365
   Thr Lys Lys Lys Ser Val Ile Phe Ser Ser Ile Ser Pro Ser Gln Ala
   4370 4375 4380
   Gly Val Tyr Thr Cys Lys Ala Glu Asn Trp Val Ala Ser Thr Glu Glu
   4385 4390 4395 4400
   Asp Ile Asp Leu Ile Val Met Ile Pro Pro Glu Val Val Pro Glu Arg
   4405 4410 4415
   Met Asn Val Ser Thr Asn Pro Arg Gln Thr Val Phe Leu Ser Cys Asn
   4420 4425 4430
   Ala Thr Gly Ile Pro Glu Pro Val Ile Ser Trp Met Arg Asp Ser Asn
   4435 4440 4445
   Ile Ala Ile Gln Asn Asn Glu Lys Tyr Gln Ile Leu Gly Thr Thr Leu
   4450 4455 4460
   Ala Ile Arg Asn Val Leu Pro Asp Asp Asp Gly Phe Tyr His Cys Ile
   4465 4470 4475 4480
   Ala Lys Ser Asp Ala Gly Gln Lys Ile Ala Thr Arg Lys Leu Ile Val
   4485 4490 4495
   Asn Lys Pro Ser Asp Arg Pro Ala Pro Ile Trp Val Glu Cys Asp Glu
   4500 4505 4510
   Lys Gly Lys Pro Lys Lys Thr Glu Tyr Met Ile Asp Arg Gly Asp Thr
   4515 4520 4525
   Pro Asp Asp Asn Pro Gln Leu Leu Pro Trp Lys Asp Val Glu Asp Ser
   4530 4535 4540
   Ser Leu Asn Gly Ser Ile Ala Tyr Arg Cys Met Pro Gly Pro Arg Ser
   4545 4550 4555 4560
   Ser Arg Thr Val Leu Leu His Ala Ala Pro Gln Phe Ile Val Lys Pro
   4565 4570 4575
   Lys Asn Thr Thr Ala Ala Ile Gly Ala Ile Val Glu Leu Arg Cys Ser
   4580 4585 4590
   Ala Ala Gly Pro Pro His Pro Thr Ile Thr Trp Ala Lys Asp Gly Lys
   4595 4600 4605
   Leu Ile Glu Asp Ser Lys Phe Glu Ile Ala Tyr Ser His Leu Lys Val
   4610 4615 4620
   Thr Leu Asn Ser Thr Ser Asp Ser Gly Glu Tyr Thr Cys Met Ala Gln
   4625 4630 4635 4640
   Asn Ser Val Gly Ser Ser Thr Val Ser Ala Phe Ile Asn Val Asp Asn
   4645 4650 4655
   Asn Ile Leu Pro Thr Pro Lys Pro Ser Ser Asn Gln Lys Asn Val Ala
   4660 4665 4670
   Val Ile Thr Cys Tyr Glu Arg Asn Gln Ala Tyr Ser Arg Gly Leu Thr
   4675 4680 4685
   Trp Glu Tyr Asn Gly Val Pro Met Pro Lys Asn Leu Ala Gly Ile His
   4690 4695 4700
   Phe Met Asn Asn Gly Ser Leu Val Ile Leu Asp Thr Ser Ser Leu Lys
   4705 4710 4715 4720
   Glu Gly Asp Leu Glu Leu Tyr Thr Cys Lys Val Arg Asn Arg Arg Arg
   4725 4730 4735
   His Ser Ile Pro His Leu Thr Ser Ala Phe Glu Gly Val Pro Glu Val
   4740 4745 4750
   Lys Thr Ile Asp Lys Val Glu Val Asn Asn Gly Asp Ser Val Val Leu
   4755 4760 4765
   Asp Cys Glu Val Thr Ser Asp Pro Leu Thr Thr His Val Val Trp Thr
   4770 4775 4780
   Lys Asn Asp Gln Lys Met Leu Asp Asp Asp Ala Ile Tyr Val Leu Pro
   4785 4790 4795 4800
   Asn Asn Ser Leu Val Leu Leu Asn Val Glu Lys Tyr Asp Glu Gly Val
   4805 4810 4815
   Tyr Lys Cys Val Ala Ser Asn Ser Ile Gly Lys Ala Phe Asp Asp Thr
   4820 4825 4830
   Gln Leu Asn Val Tyr Glu Gly Asp Phe Leu Pro Leu Thr Gly Phe Glu
   4835 4840 4845
   Gly Ser Gly Ile Asn Ile Asp Asp Ser Ser Asn Ala Gly Gly Ser Ser
   4850 4855 4860
   Arg Arg Glu Ala Tyr Lys Lys Glu Asn Glu Asp Ala Ser Thr Thr Thr
   4865 4870 4875 4880
   Ile Thr Thr Thr Ser Pro Thr Thr Thr Thr Thr Glu Thr Pro Leu Thr
   4885 4890 4895
   Thr Thr Ile Ile Pro Ala Leu Ile Thr Leu Pro Ala Lys Gln Tyr Pro
   4900 4905 4910
   Thr Asp Asp Tyr His Glu Gly Ser Ala Asn Asp Asp Gly Phe Gly Pro
   4915 4920 4925
   Thr Thr Gln Asp Ser Leu Phe Glu Phe Asn Pro Pro Leu His Pro Glu
   4930 4935 4940
   Ile Ser Val Val Asn Thr Asp Cys Ala Gly Thr Ile Asn Glu Asn Gly
   4945 4950 4955 4960
   Asp Cys Val Asp Lys Asp Gly Lys Thr His Asn Leu Lys Ile Leu Thr
   4965 4970 4975
   Gly Glu Asn His Cys Pro Glu Gly Phe Ala Met Asn Pro His Thr Arg
               4980                4985                4990
   Ile Cys Glu Asp Leu Asp Glu Cys Ala Phe Tyr Gln Pro Cys Asp Phe
           4995                5000                5005
   Glu Cys Ile Asn Tyr Asp Gly Gly Phe Gln Cys Asn Cys Pro Leu Gly
       5010                5015                5020
   Tyr Glu Leu Ala Glu Glu Gly Cys Arg Asp Val Asn Glu Cys Glu Ser
   5025                5030                5035                5040
   Val Arg Cys Glu Asp Gly Lys Ala Cys Phe Asn Gln Leu Gly Gly Tyr
                   5045                5050                5055
   Glu Cys Ile Asp Asp Pro Cys Pro Ala Asn Tyr Ser Leu Val Asp Asp
               5060                5065                5070
   Arg Tyr Cys Glu Pro Glu Cys Glu Asn Cys Thr Ser Thr Pro Ile Gln
           5075                5080                5085
   Val His Met Leu Ala Ile Pro Ser Gly Leu Pro Ile Ser His Ile Ala
       5090                5095                5100
   Thr Leu Thr Ala Tyr Asp Lys Ser Gly Arg Val Leu Asn Asp Thr Thr
   5105                5110                5115                5120
   Tyr Ala Ile Ser Asp Thr Gly Ala Pro Leu Ala Arg Gly Arg Met Thr
                   5125                5130                5135
   Ser Gly Pro Phe Thr Ile Lys Ala Val Lys Arg Gly His Ala Gln Val
               5140                5145                5150
   Trp Thr Asn Arg Val Leu Ala Ala Gly Asp His His Lys Val Arg Val
           5155                5160                5165
   Arg Ala His Ser Asp His Ala Thr Asn Glu Leu His Ala Pro Lys Glu
       5170                5175                5180
   Thr Asn Phe Leu Val Leu Ile Asn Val Gly Gln Tyr Pro Phe
   5185                5190                5195
<210> 38
   <211> 3720
   <212> DNA
   <213> Rattus sp.
<220>
   <221> CDS
   <222> (46)..(3174)
   <223> coding sequence for inventive nucleic acid product 405
<400> 38
<210> 39
   <211> 1043
   <212> PRT
   <213> Rattus sp.
<400> 39
   Met Leu Gly Tyr Leu Ala Ala Leu Ala Lys Ala Cys Phe Gly Gly Asn
    1               5                  10                  15
   Ile Glu Leu Phe Val Phe Phe Asn Gly Ala Leu Glu Lys Ala Arg Leu
               20                  25                  30
   His Glu Trp Val Lys Arg Gln Gly Asn Glu Arg Gln Thr Ala Gln Gln
           35                  40                  45
   Ile Val Ser His Val Gln Asn Lys Gly Thr Pro Pro Pro Lys Val Trp
       50                  55                  60
   Phe Leu Pro Pro Val Cys Met Ala His Cys Ile Arg Leu Ala Leu Ile
   65                  70                  75                  80
   Arg Phe His Val Lys Val Ala Gln Ser Ile Glu Asp His His Gln Glu
                   85                  90                  95
   Val Ile Gly Phe Cys Arg Glu Asn Gly Phe His Gly Leu Val Ala Tyr
   100 105 110
   Asp Ser Asp Tyr Ala Leu Cys Asn Ile Pro Tyr Tyr Phe Ser Ala His
   115 120 125
   Ala Leu Lys Leu Ser Arg Asn Gly Lys Ser Leu Thr Thr Ser Gln Tyr
   130 135 140
   Leu Met His Glu Val Ala Lys Gln Leu Asp Leu Asn Pro Asn Arg Phe
   145 150 155 160
   Pro Ile Phe Ala Ala Leu Leu Gly Asn His Ile Leu Pro Asp Glu Asp
   165 170 175
   Leu Ala Ser Phe His Trp Ser Leu Leu Gly Pro Glu His Pro Leu Ala
   180 185 190
   Ser Leu Lys Val Arg Ala His Gln Leu Val Leu Pro Pro Cys Asp Val
   195 200 205
   Val Ile Lys Ala Val Ala Asp Tyr Val Arg Asn Ile His Asp Thr Ser
   210 215 220
   Asp Leu Asp Ala Ile Ala Lys Asp Val Phe Gln His Ser Gln Ser Arg
   225 230 235 240
   Thr Asp Asp Lys Val Ile Arg Phe Lys Arg Ala Val Gly Tyr Tyr Ser
   245 250 255
   Ala Thr Ser Lys Pro Met Pro Phe His Pro Pro His Tyr Leu Pro Arg
   260 265 270
   Pro Asn Pro Phe Gly Met Pro Gly Met Val Pro Pro Tyr Val Pro Pro
   275 280 285
   Gln Met Leu Asn Ile Pro Gln Thr Ser Leu Gln Ala Lys Pro Ala Val
   290 295 300
   Pro Gln Val Pro Ser Pro Gly Gly Thr Pro Gly Gln Ala Pro Tyr Pro
   305 310 315 320
   Tyr Ser Leu Pro Glu Pro Ala Leu Ala Leu Asp Ser Ser Gly Lys Asn
   325 330 335
   Leu Thr Glu Gln Asn Ser Tyr Ser Asn Ile Pro His Glu Gly Lys His
   340 345 350
   Thr Pro Leu Tyr Glu Arg Ser Ser Pro Ile Asn Leu Ala Gln Ser Gly
   355 360 365
   Ser Pro Asn His Val Asp Ser Ala Tyr Phe Pro Gly Ser Ser Thr Ser
   370 375 380
   Ser Ser Ser Asp Asn Asp Glu Gly Ser Gly Gly Ala Thr Asn His Ile
   385 390 395 400
   Ser Gly Asn Lys Ile Gly Trp Glu Lys Thr Gly Ser His Ala Glu Pro
   405 410 415
   Leu Ala Arg Gly Asp Pro Gly Asp Gln Val Lys Val Glu Gly Ser Ser
   420 425 430
   Thr Ala Ser Ser Gly Ser Gln Leu Ala Glu Gly Lys Gly Ser His Met
   435 440 445
   Gly Thr Val Gln Pro Ile Pro Cys Leu Leu Ser Met Pro Thr Arg Asn
   450 455 460
   His Met Asp Ile Thr Thr Pro Pro Leu Pro Pro Val Ala Pro Glu Val
   465 470 475 480
   Leu Arg Val Ala Glu His Arg His Lys Lys Gly Leu Met Tyr Pro Tyr
   485 490 495
   Ile Phe His Val Leu Thr Lys Gly Glu Ile Lys Ile Ala Val Ser Ile
   500 505 510
   Glu Asp Glu Ala Asn Lys Asp Leu Pro Pro Ala Ala Leu Leu Tyr Arg
   515 520 525
   Pro Val Arg Gln Tyr Val Tyr Gly Val Leu Phe Ser Leu Ala Glu Ser
   530 535 540
   Arg Lys Lys Thr Glu Arg Leu Ala Phe Arg Lys Asn Arg Leu Pro Pro
   545 550 555 560
   Glu Phe Ser Pro Leu Ile Ile Lys Glu Trp Ala Ala Tyr Lys Gly Lys
   565 570 575
   Ser Pro Gln Thr Pro Glu Leu Val Glu Ala Leu Ala Phe Arg Glu Trp
   580 585 590
   Thr Cys Pro Asn Leu Lys Arg Leu Trp Leu Gly Lys Ala Val Glu Asp
   595 600 605
   Lys Asn Arg Arg Met Arg Ala Phe Leu Ala Cys Met Arg Ser Asp Thr
   610 615 620
   Pro Ala Met Leu Asn Pro Ala Asn Val Pro Thr His Leu Met Val Leu
   625 630 635 640
   Cys Cys Val Leu Arg Tyr Met Val Gln Trp Pro Gly Ala Arg Ile Leu
   645 650 655
   Arg Arg Gln Glu Leu Asp Ala Phe Leu Ala Gln Ala Leu Ser Pro Lys
   660 665 670
   Leu Tyr Glu Pro Asp Gln Leu Gln Glu Leu Lys Ile Glu Asn Leu Asp
   675 680 685
   Pro Arg Gly Ile Gln Leu Ser Ala Leu Phe Met Ser Gly Val Asp Met
   690 695 700
   Ala Leu Phe Ala Asn Asp Ala Cys Gly Gln Pro Ile Pro Trp Glu His
   705 710 715 720
   Cys Cys Pro Trp Met Tyr Phe Asp Gly Lys Leu Phe Gln Ser Lys Leu
   725 730 735
   Leu Lys Ala Ser Arg Glu Lys Thr Pro Leu Ile Asp Leu Cys Asp Gly
   740 745 750
   Gln Ala Glu Gln Ala Ala Lys Val Glu Lys Met Arg Gln Ser Ile Leu
           755                 760                 765
   Glu Gly Leu Asn Phe Ser Arg Gln Asn His Pro Leu Pro Phe Pro Pro
       770                 775                 780
   Pro Pro Ala Leu Pro Phe Tyr Pro Ala Ser Val Tyr Pro Arg His Phe
   785                 790                 795                 800
   Gly Pro Val Pro Pro Ser Gln Gly Arg Gly Arg Gly Phe Ala Gly Val
                   805                 810                 815
   Cys Gly Phe Gly Gly His Tyr Gly Glu Thr Val Ala Thr Gly Pro Tyr
               820                 825                 830
   Arg Ala Phe Arg Val Thr Ala Ala Ser Gly His Cys Gly Ala Phe Ser
           835                 840                 845
   Gly Ser Asp Ser Asn Arg Thr Ser Lys Ser Gln Gly Gly Val Gln Pro
       850                 855                 860
   Ile Pro Ser Gln Gly Gly Lys Leu Glu Ile Ala Gly Thr Val Val Gly
   865                 870                 875                 880
   His Trp Ala Gly Ser Arg Arg Gly Arg Gly Gly Arg Gly Pro Phe Pro
                   885                 890                 895
   Leu Gln Val Val Ser Val Gly Gly Pro Ala Arg Gly Arg Pro Arg Gly
               900                 905                 910
   Val Ile Ser Thr Pro Val Ile Arg Thr Phe Gly Arg Gly Gly Arg Tyr
           915                 920                 925
   Tyr Gly Arg Gly Tyr Lys Ser Gln Gly Ala Ile Gln Gly Arg Pro Pro
       930                 935                 940
   Tyr Ala Ala Ser Ala Glu Glu Val Ala Lys Glu Leu Lys Ser Lys Ser
   945                 950                 955                 960
   Gly Glu Ser Lys Ser Ser Ala Val Ser Ser Glu Gly Ser Leu Ala Glu
                   965                 970                 975
   Asn Gly Val Met Ala Glu Glu Lys Pro Val Pro Gln Leu Asn Gly Ser
               980                 985                 990
   Thr Ala Asp Thr Arg Ala Pro Ser His Ser Glu Ser Ala Leu Asn Asn
           995                 1000                1005
   Asp Ser Lys Thr Cys Asn Thr Asn Pro His Leu Asn Ala Leu Ser Thr
       1010                1015                1020
   Asp Ser Ala Cys Arg Arg Glu Ala Ala Leu Glu Ala Ala Val Leu Asn
   1025                1030                1035                1040
   Lys Glu Glu
<210> 40
   <211> 4679
   <212> DNA
   <213> homo sapiens
<220>
   <221> CDS
   <222> (151)..(3504)
<400> 40
   ccgcggcggc catgagcgcg cccccgaccc gccccagtcc cccctagagg ccgccgcccc 60
   cgcccgccag cccgcccgcg cgccacggcc ccaccacccc cggccccgcc gccccccgcc 120
   cgcacccgcg cccgcgcccc cgccgccgcc atg ggc gtg cag ggc ttc cag gac  174
                                    Met Gly Val Gln Gly Phe Gln Asp
                                     1               5
   tac atc gag aag cac tgc ccg agc gcc gtg gtg ccg gtg gag ctg cag   222
   Tyr Ile Glu Lys His Cys Pro Ser Ala Val Val Pro Val Glu Leu Gln
       10                  15                  20
   aag ctg gcc cgg ggc agc ctg gtg ggc ggc ggg cgg cag cgg ccc ccg   270
   Lys Leu Ala Arg Gly Ser Leu Val Gly Gly Gly Arg Gln Arg Pro Pro
    25                  30                  35                  40
   cag acc ccg ctg cgc ctg ctg gtg gac gcc gac aac tgc ctg cac cgc   318
   Gln Thr Pro Leu Arg Leu Leu Val Asp Ala Asp Asn Cys Leu His Arg
                    45                  50                  55
   ctc tac ggc ggc ttc tac acc gac tgg gtc agc ggc ggc cag tgg aac   366
   Leu Tyr Gly Gly Phe Tyr Thr Asp Trp Val Ser Gly Gly Gln Trp Asn
               60                  65                  70
   cac atg ctt ggc tac ctg gcg gcg ctg gcc aag gcc tgc ttc ggc ggc   414
   His Met Leu Gly Tyr Leu Ala Ala Leu Ala Lys Ala Cys Phe Gly Gly
           75                  80                  85
   aac atc gag ctc ttc gtc ttc ttc aac ggc gcg ctc gag aag gcc cgg   462
   Asn Ile Glu Leu Phe Val Phe Phe Asn Gly Ala Leu Glu Lys Ala Arg
       90                  95                 100
   ctg cac gag tgg gtc aag cgg cag ggc aac gag cgc cag acg gca cag   510
   Leu His Glu Trp Val Lys Arg Gln Gly Asn Glu Arg Gln Thr Ala Gln
   105                 110                 115                 120
   cag atc gtc agc cat gtc cag aac aag ggc acc ccg ccg cca aag gtc   558
   Gln Ile Val Ser His Val Gln Asn Lys Gly Thr Pro Pro Pro Lys Val
                   125                 130                 135
   tgg ttc ctg ccg ccc gtc tgc atg gcc cac tgc atc cgc ctg gcg ctc   606
   Trp Phe Leu Pro Pro Val Cys Met Ala His Cys Ile Arg Leu Ala Leu
               140                 145                 150
   atc cgc ttc cac gtc aag gtt gca cag agc att gag gat cac cat cag   654
   Ile Arg Phe His Val Lys Val Ala Gln Ser Ile Glu Asp His His Gln
           155                 160                 165
   gaa gtg att ggt ttc tgc aga gag aat ggt ttc cat ggc ttg gtt gcg   702
   Glu Val Ile Gly Phe Cys Arg Glu Asn Gly Phe His Gly Leu Val Ala
       170                 175                 180
   tat gac tct gat tat gca ctg tgc aac atc ccc tac tat ttc agt gcc   750
   Tyr Asp Ser Asp Tyr Ala Leu Cys Asn Ile Pro Tyr Tyr Phe Ser Ala
   185                 190                 195                 200
   cat gcc cta aaa ctg agc cgg aac ggg aaa agt ctc acc aca agc caa      798
   His Ala Leu Lys Leu Ser Arg Asn Gly Lys Ser Leu Thr Thr Ser Gln
                   205                 210                 215
   tat ctg atg cat gaa gtt gcc aag caa ctg gac ctg aat cca aat cgt      846
   Tyr Leu Met His Glu Val Ala Lys Gln Leu Asp Leu Asn Pro Asn Arg
               220                 225                 230
   ttt cct att ttt gct gct ctc tta gga aat cac att ctg cct gat gaa      894
   Phe Pro Ile Phe Ala Ala Leu Leu Gly Asn His Ile Leu Pro Asp Glu
           235                 240                 245
   gat ctg gct tcc ttt cat tgg agt tta ctt ggt cca gaa cat cca cta      942
   Asp Leu Ala Ser Phe His Trp Ser Leu Leu Gly Pro Glu His Pro Leu
       250                 255                 260
   gcc tca cta aag gtc cgg gcc cac cag ctg gtc ttg cca cct tgc gac      990
   Ala Ser Leu Lys Val Arg Ala His Gln Leu Val Leu Pro Pro Cys Asp
   265                 270                 275                 280
   gta gtg atc aaa gcc gtt gct gac tat gta cgc aac att cag gac acc      1038
   Val Val Ile Lys Ala Val Ala Asp Tyr Val Arg Asn Ile Gln Asp Thr
                   285                 290                 295
   tct gac ttg gat gcc ata gct aaa gat gtt ttc cag cat tca cag tct      1086
   Ser Asp Leu Asp Ala Ile Ala Lys Asp Val Phe Gln His Ser Gln Ser
               300                 305                 310
   aga aca gat gac aaa gtt att cga ttt aag aga gca att gga tat tat      1134
   Arg Thr Asp Asp Lys Val Ile Arg Phe Lys Arg Ala Ile Gly Tyr Tyr
           315                 320                 325
   tca gcg act agt aag cct atg tca ttt cat cca cca cat tac tta gca      1182
   Ser Ala Thr Ser Lys Pro Met Ser Phe His Pro Pro His Tyr Leu Ala
       330                 335                 340
   gcc aga ccc ggt ccg ttt gga atg cct ggg atg gtg ccg ccg cat gtt      1230
   Ala Arg Pro Gly Pro Phe Gly Met Pro Gly Met Val Pro Pro His Val
   345                 350                 355                 360
   cct cct cag atg ctc aac att ccg cag acc tct ctg caa gca aag ccc      1278
   Pro Pro Gln Met Leu Asn Ile Pro Gln Thr Ser Leu Gln Ala Lys Pro
                   365                 370                 375
   gtg gcc cca cag gtg ccc agc cca ggg ggc gcc ccg ggc cag ggt cca      1326
   Val Ala Pro Gln Val Pro Ser Pro Gly Gly Ala Pro Gly Gln Gly Pro
               380                 385                 390
   tac ccg tac agc ctc tct gag cca gca cct ctc act ttg gac acg agc      1374
   Tyr Pro Tyr Ser Leu Ser Glu Pro Ala Pro Leu Thr Leu Asp Thr Ser
           395                 400                 405
   ggg aag aat ctg acg gag cag aac agc tac agc aac att cct cac gaa      1422
   Gly Lys Asn Leu Thr Glu Gln Asn Ser Tyr Ser Asn Ile Pro His Glu
       410                 415                 420
   ggg aag cac acg ccg ctg tat gag cgg tcc tcg ccc atc aac cgg gcc      1470
   Gly Lys His Thr Pro Leu Tyr Glu Arg Ser Ser Pro Ile Asn Arg Ala
   425                 430                 435                 440
   cag agc ggc agc ccc aac cac gtg gat tcc gcc tac ttc cct ggc tct     1518
   Gln Ser Gly Ser Pro Asn His Val Asp Ser Ala Tyr Phe Pro Gly Ser
                   445                 450                 455
   tct aca tcg tca tct tcc gac aac gac gag ggc agc gga ggg gcg aca     1566
   Ser Thr Ser Ser Ser Ser Asp Asn Asp Glu Gly Ser Gly Gly Ala Thr
               460                 465                 470
   aac cat atc agc ggg aac aag att ggc tgg gag aag acg gga agc cac     1614
   Asn His Ile Ser Gly Asn Lys Ile Gly Trp Glu Lys Thr Gly Ser His
           475                 480                 485
   tca gag cct cag gca cga gga gac cca gga gac caa aca aag gca gaa     1662
   Ser Glu Pro Gln Ala Arg Gly Asp Pro Gly Asp Gln Thr Lys Ala Glu
       490                 495                 500
   ggc tcg tcc act gcc tct tca gga agc caa cta gcc gaa ggc aag gga     1710
   Gly Ser Ser Thr Ala Ser Ser Gly Ser Gln Leu Ala Glu Gly Lys Gly
   505                 510                 515                 520
   agc cag atg ggc act gtc cag cca atc ccg tgc ctc ctg tcg atg ccc     1758
   Ser Gln Met Gly Thr Val Gln Pro Ile Pro Cys Leu Leu Ser Met Pro
                   525                 530                 535
   acc agg aac cac atg gac atc acc aca cct ccc ctg ccc ccc gtc gca     1806
   Thr Arg Asn His Met Asp Ile Thr Thr Pro Pro Leu Pro Pro Val Ala
               540                 545                 550
   cct gag gtg ctg aga gtg gcc gag cac agg cac aag aag ggg ctg atg     1854
   Pro Glu Val Leu Arg Val Ala Glu His Arg His Lys Lys Gly Leu Met
           555                 560                 565
   tac ccc tac atc ttc cat gtc ctg acg aag ggt gaa atc aaa att gct     1902
   Tyr Pro Tyr Ile Phe His Val Leu Thr Lys Gly Glu Ile Lys Ile Ala
       570                 575                 580
   gtt tct att gaa gat gaa gcc aac aag gac ctg cct ccg gcc gct ctg     1950
   Val Ser Ile Glu Asp Glu Ala Asn Lys Asp Leu Pro Pro Ala Ala Leu
   585                 590                 595                 600
   ctc tat agg cca gtt cgt cag tat gtt tac gga gtc ctg ttt agt ttg     1998
   Leu Tyr Arg Pro Val Arg Gln Tyr Val Tyr Gly Val Leu Phe Ser Leu
                   605                 610                 615
   gca gaa agc aga aag aaa act gag aga ctt gct ttt aga aag aac aga     2046
   Ala Glu Ser Arg Lys Lys Thr Glu Arg Leu Ala Phe Arg Lys Asn Arg
               620                 625                 630
   ctt cca cca gaa ttt tca cca gtg atc att aaa gaa tgg gca gct tac     2094
   Leu Pro Pro Glu Phe Ser Pro Val Ile Ile Lys Glu Trp Ala Ala Tyr
           635                 640                 645
   aaa gga aag tct cct caa acc ccg gaa ctg gtt gaa gct ctt gcc ttc     2142
   Lys Gly Lys Ser Pro Gln Thr Pro Glu Leu Val Glu Ala Leu Ala Phe
       650                 655                 660
   agg gag tgg acc tgc ccc aac ctg aag agg ctg tgg ttg ggt aag gcg     2190
   Arg Glu Trp Thr Cys Pro Asn Leu Lys Arg Leu Trp Leu Gly Lys Ala
   665                 670                 675                 680
   gta gag gac aag aac cgc agg atg agg gcc ttc ctg gcc tgc atg agg    2238
   Val Glu Asp Lys Asn Arg Arg Met Arg Ala Phe Leu Ala Cys Met Arg
                   685                 690                 695
   tcg gac acc cca gcc atg ctc aac cct gcc aac gtg ccc act cac ctc    2286
   Ser Asp Thr Pro Ala Met Leu Asn Pro Ala Asn Val Pro Thr His Leu
               700                 705                 710
   atg gtg ctc tgc tgc gtc tta cgg tac atg gtg cag tgg ccg gga gca    2334
   Met Val Leu Cys Cys Val Leu Arg Tyr Met Val Gln Trp Pro Gly Ala
           715                 720                 725
   tgc atc ctt cgg cgt cag gag cta gat gcc ttc ctg gct cag gcg ctg    2382
   Cys Ile Leu Arg Arg Gln Glu Leu Asp Ala Phe Leu Ala Gln Ala Leu
       730                 735                 740
   tcc ccc aaa ctc tac gag cct gat cag ctc cag gag ctc aag att gag    2430
   Ser Pro Lys Leu Tyr Glu Pro Asp Gln Leu Gln Glu Leu Lys Ile Glu
   745                 750                 755                 760
   aac cta gat ccc cga gga att cag cta tca gct ctc ttc atg agt gga    2478
   Asn Leu Asp Pro Arg Gly Ile Gln Leu Ser Ala Leu Phe Met Ser Gly
                   765                 770                 775
   gta gac atg gcc ttg ttt gca aat gat gca tgc gga cag cca atc ccc    2526
   Val Asp Met Ala Leu Phe Ala Asn Asp Ala Cys Gly Gln Pro Ile Pro
               780                 785                 790
   tgg gaa cac tgt tgt cct tgg atg tat ttt gat ggg aag ctc ttc caa    2574
   Trp Glu His Cys Cys Pro Trp Met Tyr Phe Asp Gly Lys Leu Phe Gln
           795                 800                 805
   tcc aaa ctc ctc aaa gcc agc cgg gaa aag acc cca ctc att gac ctc    2622
   Ser Lys Leu Leu Lys Ala Ser Arg Glu Lys Thr Pro Leu Ile Asp Leu
       810                 815                 820
   tgt gat ggt cag gct gat cag gct gcc aag gta gag aag atg cgc cag    2670
   Cys Asp Gly Gln Ala Asp Gln Ala Ala Lys Val Glu Lys Met Arg Gln
   825                 830                 835                 840
   agc gtc ctc gag ggg ctc agc ttc tcc agg cag agc cac acg ctc cct    2718
   Ser Val Leu Glu Gly Leu Ser Phe Ser Arg Gln Ser His Thr Leu Pro
                   845                 850                 855
   ttc ccg ccg cca cct gcc ctg ccc ttc tac cct gcc tct gcg tac ccc    2766
   Phe Pro Pro Pro Pro Ala Leu Pro Phe Tyr Pro Ala Ser Ala Tyr Pro
               860                 865                 870
   cgg cac ttt ggg cct gtc cca ccc tct cag ggc agg ggc aga ggc ttt    2814
   Arg His Phe Gly Pro Val Pro Pro Ser Gln Gly Arg Gly Arg Gly Phe
           875                 880                 885
   gca ggc gtc tgt ggc ttt gga ggc ccc tat ggg gaa acg gta gca aca    2862
   Ala Gly Val Cys Gly Phe Gly Gly Pro Tyr Gly Glu Thr Val Ala Thr
       890                 895                 900
   ggc cct tac cgt gcc ttc cgt gtg gcg gca gca tcg gga cac tgc gga    2910
   Gly Pro Tyr Arg Ala Phe Arg Val Ala Ala Ala Ser Gly His Cys Gly
   905                 910                 915                 920
   gcc ttc tca ggc agt gac agc agc agg act agc aag tcc cag ggc gga     2958
   Ala Phe Ser Gly Ser Asp Ser Ser Arg Thr Ser Lys Ser Gln Gly Gly
                   925                 930                 935
   gtc caa cct ata cct tct cag gga ggc aaa cta gaa ata gct ggc act     3006
   Val Gln Pro Ile Pro Ser Gln Gly Gly Lys Leu Glu Ile Ala Gly Thr
               940                 945                 950
   gtg gtt ggc cat tgg gct ggg agc agg cgg ggc cgt ggg ggc cgg ggg     3054
   Val Val Gly His Trp Ala Gly Ser Arg Arg Gly Arg Gly Gly Arg Gly
           955                 960                 965
   cct ttc ccc ctg cag gtg gtt tct gtc gga gga cca gct aga ggg cgt     3102
   Pro Phe Pro Leu Gln Val Val Ser Val Gly Gly Pro Ala Arg Gly Arg
       970                 975                 980
   cca aga gga gtt att tcc acc cca gtg att aga aca ttt gga aga ggt     3150
   Pro Arg Gly Val Ile Ser Thr Pro Val Ile Arg Thr Phe Gly Arg Gly
   985                 990                 995                 1000
   gga agg tac tat ggc aga ggt tac aaa aac cag gca gca att cag ggc     3198
   Gly Arg Tyr Tyr Gly Arg Gly Tyr Lys Asn Gln Ala Ala Ile Gln Gly
                   1005                1010                1015
   aga cct cct tat gct gct tca gca gaa gaa gtg gcc aaa gaa ctt aag     3246
   Arg Pro Pro Tyr Ala Ala Ser Ala Glu Glu Val Ala Lys Glu Leu Lys
               1020                1025                1030
   tca aaa tct ggg gaa tcg aag tcc tct gct atg tct tca gac ggg tcc     3294
   Ser Lys Ser Gly Glu Ser Lys Ser Ser Ala Met Ser Ser Asp Gly Ser
           1035                1040                1045
   ctg gct gaa aac gga gtg atg gcc gag gag aag ccg gct ccc cag atg     3342
   Leu Ala Glu Asn Gly Val Met Ala Glu Glu Lys Pro Ala Pro Gln Met
       1050                1055                1060
   aac ggg agc acg ggt gac gcc agg gcc ccc agc cac tct gaa agt gcc     3390
   Asn Gly Ser Thr Gly Asp Ala Arg Ala Pro Ser His Ser Glu Ser Ala
   1065                1070                1075                1080
   ttg aat aat gac tct aaa acg tgc aat aca aat cct cat tta aat gca     3438
   Leu Asn Asn Asp Ser Lys Thr Cys Asn Thr Asn Pro His Leu Asn Ala
                   1085                1090                1095
   cta agt aca gac agc gct tgc cgc aga gaa gct gct ctg gag gca gct     3486
   Leu Ser Thr Asp Ser Ala Cys Arg Arg Glu Ala Ala Leu Glu Ala Ala
               1100                1105                1110
   gtc tta aat aaa gaa gag taaacttatt ttttatagag ggtgaaggat            3534
   Val Leu Asn Lys Glu Glu
           1115
   gctggaaggg taaggattta ggaatatctg gagagaaaga gagcctgcag ttatgtacat 3594
   tttgtccttt ccgtaagaga aaaatgagga ctttggaaat tcagatccct ctttgatatc 3654
   agagatttaa acaacacatt tttagtttta accagttgta gtcaaaatgc tacaataaaa 3714
   caaaaaagag aaagaaaatg aagagcattt gactcccgca cttaaaatga agtacacata 3774
   aagtttaaac tggttatgac aaaagcctat agttgtgttt cttgaactat aaagaaaaca 3834
   aattttggca gtctttaagt atatatagct taaaatataa tttttagcat ttggcaccat 3894
   atgtatgcca ttatatttga ttttgcatta ctgtttcaca atgaagcttt ctttaaggct 3954
   ttgattttta tgattatgaa agaaataagg cacaaccaca gtttttcttt cttaaatttc 4014
   atcactgttg atgtggttct tttgtgttaa aaaaaaaaag tgcaactatc aaaactaaaa 4074
   aattatagag taatattgcc gttctgctga ttttaaatat acaatacatc atacatactt 4134
   tacaagcaag ttaaatggag ataaagttga aatcatagaa gatgcaaatg acctttcaaa 4194
   atcaacacaa tgtgttctga aactttcgtg actaatacca tgcatctgtg atcaatgaac 4254
   tatgtggttt tgaatcggat gtagaccatt agtactacta cttgagctaa acttctgcat 4314
   ggttcataat ttttaaagtg tgtagttaat atgcatgtta tcgtcctttc ttccattctt 4374
   aacagtatgt gcccatttgc aaaacaaaaa tgctaataat cagtaatagt cctataaaag 4434
   atgttaactc tgtttagtca ttgactgatc ttgctctaac cttaaaattt tgtgattatt 4494
   gacctctgtt gcatttattc taaagccccc caaaaattat ctagccgttt cgaatatcaa 4554
   cattaccctg gtgtattcac tgctgtatgc attattgttc tttgttgctg ttttatgcct 4614
   tcatattagc aaatatgaaa ttctgtgaaa aaaaaccctt tgatcttaaa aaaaaaaaaa 4674
   aaaaa 4679
<210> 41
   <211> 1118
   <212> PRT
   <213> homo sapiens
<400> 41
   Met Gly Val Gln Gly Phe Gln Asp Tyr Ile Glu Lys His Cys Pro Ser
    1               5                  10                  15
   Ala Val Val Pro Val Glu Leu Gln Lys Leu Ala Arg Gly Ser Leu Val
               20                  25                  30
   Gly Gly Gly Arg Gln Arg Pro Pro Gln Thr Pro Leu Arg Leu Leu Val
           35                  40                  45
   Asp Ala Asp Asn Cys Leu His Arg Leu Tyr Gly Gly Phe Tyr Thr Asp
       50                  55                  60
   Trp Val Ser Gly Gly Gln Trp Asn His Met Leu Gly Tyr Leu Ala Ala
   65                  70                  75                  80
   Leu Ala Lys Ala Cys Phe Gly Gly Asn Ile Glu Leu Phe Val Phe Phe
                   85                  90                  95
   Asn Gly Ala Leu Glu Lys Ala Arg Leu His Glu Trp Val Lys Arg Gln
               100                 105                 110
   Gly Asn Glu Arg Gln Thr Ala Gln Gln Ile Val Ser His Val Gln Asn
           115                 120                 125
   Lys Gly Thr Pro Pro Pro Lys Val Trp Phe Leu Pro Pro Val Cys Met
       130                 135                 140
   Ala His Cys Ile Arg Leu Ala Leu Ile Arg Phe His Val Lys Val Ala
   145                 150                 155                 160
   Gln Ser Ile Glu Asp His His Gln Glu Val Ile Gly Phe Cys Arg Glu
                   165                 170                 175
   Asn Gly Phe His Gly Leu Val Ala Tyr Asp Ser Asp Tyr Ala Leu Cys
               180                 185                 190
   Asn Ile Pro Tyr Tyr Phe Ser Ala His Ala Leu Lys Leu Ser Arg Asn
           195                 200                 205
   Gly Lys Ser Leu Thr Thr Ser Gln Tyr Leu Met His Glu Val Ala Lys
       210                 215                 220
   Gln Leu Asp Leu Asn Pro Asn Arg Phe Pro Ile Phe Ala Ala Leu Leu
   225                 230                 235                 240
   Gly Asn His Ile Leu Pro Asp Glu Asp Leu Ala Ser Phe His Trp Ser
                   245                 250                 255
   Leu Leu Gly Pro Glu His Pro Leu Ala Ser Leu Lys Val Arg Ala His
               260                 265                 270
   Gln Leu Val Leu Pro Pro Cys Asp Val Val Ile Lys Ala Val Ala Asp
           275                 280                 285
   Tyr Val Arg Asn Ile Gln Asp Thr Ser Asp Leu Asp Ala Ile Ala Lys
       290                 295                 300
   Asp Val Phe Gln His Ser Gln Ser Arg Thr Asp Asp Lys Val Ile Arg
   305                 310                 315                 320
   Phe Lys Arg Ala Ile Gly Tyr Tyr Ser Ala Thr Ser Lys Pro Met Ser
                   325                 330                 335
   Phe His Pro Pro His Tyr Leu Ala Ala Arg Pro Gly Pro Phe Gly Met
               340                 345                 350
   Pro Gly Met Val Pro Pro His Val Pro Pro Gln Met Leu Asn Ile Pro
           355                 360                 365
   Gln Thr Ser Leu Gln Ala Lys Pro Val Ala Pro Gln Val Pro Ser Pro
       370                 375                 380
   Gly Gly Ala Pro Gly Gln Gly Pro Tyr Pro Tyr Ser Leu Ser Glu Pro
   385                 390                 395                 400
   Ala Pro Leu Thr Leu Asp Thr Ser Gly Lys Asn Leu Thr Glu Gln Asn
                   405                 410                 415
   Ser Tyr Ser Asn Ile Pro His Glu Gly Lys His Thr Pro Leu Tyr Glu
               420                 425                 430
   Arg Ser Ser Pro Ile Asn Arg Ala Gln Ser Gly Ser Pro Asn His Val
           435                 440                 445
   Asp Ser Ala Tyr Phe Pro Gly Ser Ser Thr Ser Ser Ser Ser Asp Asn
       450                 455                 460
   Asp Glu Gly Ser Gly Gly Ala Thr Asn His Ile Ser Gly Asn Lys Ile
   465                 470                 475                 480
   Gly Trp Glu Lys Thr Gly Ser His Ser Glu Pro Gln Ala Arg Gly Asp
                   485                 490                 495
   Pro Gly Asp Gln Thr Lys Ala Glu Gly Ser Ser Thr Ala Ser Ser Gly
               500                 505                 510
   Ser Gln Leu Ala Glu Gly Lys Gly Ser Gln Met Gly Thr Val Gln Pro
           515                 520                 525
   Ile Pro Cys Leu Leu Ser Met Pro Thr Arg Asn His Met Asp Ile Thr
       530                 535                 540
   Thr Pro Pro Leu Pro Pro Val Ala Pro Glu Val Leu Arg Val Ala Glu
   545                 550                 555                 560
   His Arg His Lys Lys Gly Leu Met Tyr Pro Tyr Ile Phe His Val Leu
                   565                 570                 575
   Thr Lys Gly Glu Ile Lys Ile Ala Val Ser Ile Glu Asp Glu Ala Asn
               580                 585                 590
   Lys Asp Leu Pro Pro Ala Ala Leu Leu Tyr Arg Pro Val Arg Gln Tyr
           595                 600                 605
   Val Tyr Gly Val Leu Phe Ser Leu Ala Glu Ser Arg Lys Lys Thr Glu
       610                 615                 620
   Arg Leu Ala Phe Arg Lys Asn Arg Leu Pro Pro Glu Phe Ser Pro Val
   625                 630                 635                 640
   Ile Ile Lys Glu Trp Ala Ala Tyr Lys Gly Lys Ser Pro Gln Thr Pro
                   645                 650                 655
   Glu Leu Val Glu Ala Leu Ala Phe Arg Glu Trp Thr Cys Pro Asn Leu
               660                 665                 670
   Lys Arg Leu Trp Leu Gly Lys Ala Val Glu Asp Lys Asn Arg Arg Met
           675                 680                 685
   Arg Ala Phe Leu Ala Cys Met Arg Ser Asp Thr Pro Ala Met Leu Asn
       690                 695                 700
   Pro Ala Asn Val Pro Thr His Leu Met Val Leu Cys Cys Val Leu Arg
   705                 710                 715                 720
   Tyr Met Val Gln Trp Pro Gly Ala Cys Ile Leu Arg Arg Gln Glu Leu
                   725                 730                 735
   Asp Ala Phe Leu Ala Gln Ala Leu Ser Pro Lys Leu Tyr Glu Pro Asp
               740                 745                 750
   Gln Leu Gln Glu Leu Lys Ile Glu Asn Leu Asp Pro Arg Gly Ile Gln
           755                 760                 765
   Leu Ser Ala Leu Phe Met Ser Gly Val Asp Met Ala Leu Phe Ala Asn
   770 775 780
   Asp Ala Cys Gly Gln Pro Ile Pro Trp Glu His Cys Cys Pro Trp Met
   785 790 795 800
   Tyr Phe Asp Gly Lys Leu Phe Gln Ser Lys Leu Leu Lys Ala Ser Arg
   805 810 815
   Glu Lys Thr Pro Leu Ile Asp Leu Cys Asp Gly Gln Ala Asp Gln Ala
   820 825 830
   Ala Lys Val Glu Lys Met Arg Gln Ser Val Leu Glu Gly Leu Ser Phe
   835 840 845
   Ser Arg Gln Ser His Thr Leu Pro Phe Pro Pro Pro Pro Ala Leu Pro
   850 855 860
   Phe Tyr Pro Ala Ser Ala Tyr Pro Arg His Phe Gly Pro Val Pro Pro
   865 870 875 880
   Ser Gln Gly Arg Gly Arg Gly Phe Ala Gly Val Cys Gly Phe Gly Gly
   885 890 895
   Pro Tyr Gly Glu Thr Val Ala Thr Gly Pro Tyr Arg Ala Phe Arg Val
   900 905 910
   Ala Ala Ala Ser Gly His Cys Gly Ala Phe Ser Gly Ser Asp Ser Ser
   915 920 925
   Arg Thr Ser Lys Ser Gln Gly Gly Val Gln Pro Ile Pro Ser Gln Gly
   930 935 940
   Gly Lys Leu Glu Ile Ala Gly Thr Val Val Gly His Trp Ala Gly Ser
   945 950 955 960
   Arg Arg Gly Arg Gly Gly Arg Gly Pro Phe Pro Leu Gln Val Val Ser
   965 970 975
   Val Gly Gly Pro Ala Arg Gly Arg Pro Arg Gly Val Ile Ser Thr Pro
   980 985 990
   Val Ile Arg Thr Phe Gly Arg Gly Gly Arg Tyr Tyr Gly Arg Gly Tyr
   995 1000 1005
   Lys Asn Gln Ala Ala Ile Gln Gly Arg Pro Pro Tyr Ala Ala Ser Ala
   1010 1015 1020
   Glu Glu Val Ala Lys Glu Leu Lys Ser Lys Ser Gly Glu Ser Lys Ser
   1025 1030 1035 1040
   Ser Ala Met Ser Ser Asp Gly Ser Leu Ala Glu Asn Gly Val Met Ala
   1045 1050 1055
   Glu Glu Lys Pro Ala Pro Gln Met Asn Gly Ser Thr Gly Asp Ala Arg
   1060 1065 1070
   Ala Pro Ser His Ser Glu Ser Ala Leu Asn Asn Asp Ser Lys Thr Cys
   1075 1080 1085
   Asn Thr Asn Pro His Leu Asn Ala Leu Ser Thr Asp Ser Ala Cys Arg
   1090 1095 1100
   Arg Glu Ala Ala Leu Glu Ala Ala Val Leu Asn Lys Glu Glu
   1105 1110 1115
<210> 42
   <211> 494
   <212> PRT
   <213> homo sapiens
<220>
   <222> (1)..(494)
   <223> unknown [Homo sapiens].
<300>
   <301> Andersson, et al. Vogel, et al. Lbeit, et al. Ohara, O
   <302> Unknown protein [Homo sapiens].
   <308> GI 3005744
   <309> 1998-04-02
   <313> 1 TO 494
<400> 42
   Gly Glu Ile Lys Ile Ala Val Ser Ile Glu Asp Glu Ala Asn Lys Asp
   1 5 10 15
   Leu Pro Pro Ala Ala Leu Leu Tyr Arg Pro Val Arg Gln Tyr Val Tyr
   20 25 30
   Gly Val Leu Phe Ser Leu Ala Glu Ser Arg Lys Lys Thr Glu Arg Leu
   35 40 45
   Ala Phe Arg Lys Asn Arg Leu Pro Pro Glu Phe Ser Pro Val Ile Ile
   50 55 60
   Lys Glu Trp Ala Ala Tyr Lys Gly Lys Ser Pro Gln Thr Pro Glu Leu
   65 70 75 80
   Val Glu Ala Leu Ala Phe Arg Glu Trp Thr Cys Pro Asn Leu Lys Arg
   85 90 95
   Leu Trp Leu Gly Lys Ala Val Glu Asp Lys Asn Arg Arg Met Arg Ala
   100 105 110
   Phe Leu Ala Cys Met Arg Ser Asp Thr Pro Ala Met Leu Asn Pro Ala
   115 120 125
   Asn Val Pro Thr His Leu Met Val Leu Cys Cys Val Leu Arg Tyr Met
   130 135 140
   Val Gln Trp Pro Gly Ala Arg Ile Leu Arg Arg Gln Glu Leu Asp Ala
   145 150 155 160
   Phe Leu Ala Gln Ala Leu Ser Pro Lys Leu Tyr Glu Pro Asp Gln Leu
   165 170 175
   Gln Glu Leu Lys Ile Glu Asn Leu Asp Pro Arg Gly Ile Gln Leu Ser
   180 185 190
   Ala Leu Phe Met Ser Gly Val Asp Met Ala Leu Phe Ala Asn Asp Ala
           195                 200                 205
   Cys Gly Gln Pro Ile Pro Trp Glu His Cys Cys Pro Trp Met Tyr Phe
       210                 215                 220
   Asp Gly Lys Leu Phe Gln Ser Lys Leu Leu Lys Ala Ser Arg Glu Lys
   225                 230                 235                 240
   Thr Pro Leu Ile Asp Leu Cys Asp Gly Gln Ala Asp Gln Ala Ala Lys
                   245                 250                 255
   Val Glu Lys Met Arg Gln Ser Val Leu Glu Gly Leu Ser Phe Ser Arg
               260                 265                 270
   Gln Ser His Thr Leu Pro Phe Pro Pro Pro Pro Ala Leu Pro Phe Tyr
           275                 280                 285
   Pro Ala Ser Ala Tyr Pro Arg His Phe Gly Pro Val Pro Pro Ser Gln
       290                 295                 300
   Gly Arg Gly Arg Gly Phe Ala Gly Val Gln Pro Ile Pro Ser Gln Gly
   305                 310                 315                 320
   Gly Lys Leu Glu Ile Ala Gly Thr Val Val Gly His Trp Ala Gly Ser
                   325                 330                 335
   Arg Arg Gly Arg Gly Gly Arg Gly Pro Phe Pro Leu Gln Val Val Ser
               340                 345                 350
   Val Gly Gly Pro Ala Arg Gly Arg Pro Arg Gly Val Ile Ser Thr Pro
           355                 360                 365
   Val Ile Arg Thr Phe Gly Arg Gly Gly Arg Tyr Tyr Gly Arg Gly Tyr
       370                 375                 380
   Lys Asn Gln Ala Ala Ile Gln Gly Arg Pro Pro Tyr Ala Ala Ser Ala
   385                 390                 395                 400
   Glu Glu Val Ala Lys Glu Leu Lys Ser Lys Ser Gly Glu Ser Lys Ser
                   405                 410                 415
   Ser Ala Met Ser Ser Asp Gly Ser Leu Ala Glu Asn Gly Val Met Ala
               420                 425                 430
   Glu Glu Lys Pro Ala Pro Gln Met Asn Gly Ser Thr Gly Asp Ala Arg
           435                 440                 445
   Ala Pro Ser His Ser Glu Ser Ala Leu Asn Asn Asp Ser Lys Thr Cys
       450                 455                 460
   Asn Thr Asn Pro His Leu Asn Ala Leu Ser Thr Asp Ser Ala Cys Arg
   465                 470                 475                 480
   Arg Glu Ala Ala Leu Glu Ala Ala Val Leu Asn Lys Glu Glu
                   485                 490
<210> 43
   <211> 1063
   <212> PRT
   <213> Rattus sp.
<400> 43
   Asp Tyr Ile Glu Lys His Cys Pro Ser Ala Val Val Pro Val Glu Leu
    1               5                  10                  15
   Gln Lys Leu Ala Arg Gly Ser Leu Val Gly Gly Gly Arg Gln Arg Pro
               20                  25                  30
   Pro Gln Thr Pro Leu Arg Leu Leu Val Asp Ala Asp Asn Cys Leu His
           35                  40                  45
   Arg Leu Tyr Gly Gly Phe Tyr Thr Asp Trp Val Ser Gly Gly Gln Trp
       50                  55                  60
   Asn His Met Leu Gly Tyr Leu Ala Ala Leu Ala Lys Ala Cys Phe Gly
    65                 70                  75                  80
   Gly Asn Ile Glu Leu Phe Val Phe Phe Asn Gly Ala Leu Glu Lys Ala
                   85                  90                  95
   Arg Leu His Glu Trp Val Lys Arg Gln Gly Asn Glu Arg Gln Thr Ala
               100                 105                 110
   Gln Gln Ile Val Ser His Val Gln Asn Lys Gly Thr Pro Pro Pro Lys
           115                 120                 125
   Val Trp Phe Leu Pro Pro Val Cys Met Ala His Cys Ile Arg Leu Ala
       130                 135                 140
   Leu Ile Arg Phe His Val Lys Val Ala Gln Ser Ile Glu Asp His His
   145                 150                 155                 160
   Gln Glu Val Ile Gly Phe Cys Arg Glu Asn Gly Phe His Gly Leu Val
                   165                 170                 175
   Ala Tyr Asp Ser Asp Tyr Ala Leu Cys Asn Ile Pro Tyr Tyr Phe Ser
               180                 185                 190
   Ala His Ala Leu Lys Leu Ser Arg Asn Gly Lys Ser Leu Thr Thr Ser
           195                 200                 205
   Gln Tyr Leu Met His Glu Val Ala Lys Gln Leu Asp Leu Asn Pro Asn
       210                 215                 220
   Arg Phe Pro Ile Phe Ala Ala Leu Leu Gly Asn His Ile Leu Pro Asp
   225                 230                 235                 240
   Glu Asp Leu Ala Ser Phe His Trp Ser Leu Leu Gly Pro Glu His Pro
                   245                 250                 255
   Leu Ala Ser Leu Lys Val Arg Ala His Gln Leu Val Leu Pro Pro Cys
               260                 265                 270
   Asp Val Val Ile Lys Ala Val Ala Asp Tyr Val Arg Asn Ile Gln Asp
           275                 280                 285
   Thr Ser Asp Leu Asp Ala Ile Ala Lys Asp Val Phe Gln His Ser Gln
       290                 295                 300
   Ser Arg Thr Asp Asp Lys Val Ile Arg Phe Lys Arg Ala Ile Gly Tyr
   305                 310                 315                 320
   Tyr Ser Ala Thr Ser Lys Pro Met Ser Phe His Pro Pro His Tyr Leu
                   325                 330                 335
   Ala Ala Arg Pro Gly Pro Phe Gly Met Pro Gly Met Val Pro Pro His
               340                 345                 350
   Val Pro Pro Gln Met Leu Asn Ile Pro Gln Thr Ser Leu Gln Ala Lys
           355                 360                 365
   Pro Val Ala Pro Gln Val Pro Ser Pro Gly Gly Ala Pro Gly Gln Gly
       370                 375                 380
   Pro Tyr Pro Tyr Ser Leu Ser Glu Pro Ala Pro Leu Thr Leu Asp Thr
   385                 390                 395                 400
   Ser Gly Lys Asn Leu Thr Glu Gln Asn Ser Tyr Ser Asn Ile Pro His
                   405                 410                 415
   Glu Gly Lys His Thr Pro Leu Tyr Glu Arg Ser Ser Pro Ile Asn Pro
               420                 425                 430
   Ala Gln Ser Gly Ser Pro Asn His Val Asp Ser Ala Tyr Phe Pro Gly
           435                 440                 445
   Ser Ser Thr Ser Ser Ser Ser Asp Asn Asp Glu Gly Ser Gly Gly Ala
       450                 455                 460
   Thr Asn His Ile Ser Gly Asn Lys Ile Gly Trp Glu Lys Thr Gly Ser
   465                 470                 475                 480
   His Ser Glu Pro Gln Ala Arg Gly Asp Pro Gly Asp Gln Thr Lys Ala
                   485                 490                 495
   Glu Gly Ser Ser Thr Ala Ser Ser Gly Ser Gln Leu Ala Glu Gly Lys
               500                 505                 510
   Gly Ser Gln Met Gly Thr Val Gln Pro Ile Pro Cys Leu Leu Ser Met
           515                 520                 525
   Pro Thr Arg Asn His Met Asp Ile Thr Thr Pro Pro Leu Pro Pro Val
       530                 535                 540
   Ala Pro Glu Val Leu Arg Val Ala Glu His Arg His Lys Lys Gly Leu
   545                 550                 555                 560
   Met Tyr Pro Tyr Ile Phe His Val Leu Thr Lys Gly Glu Ile Lys Ile
                   565                 570                 575
   Ala Val Ser Ile Glu Asp Glu Ala Asn Lys Asp Leu Pro Pro Ala Ala
               580                 585                 590
   Leu Leu Tyr Arg Pro Val Arg Gln Tyr Val Tyr Gly Val Leu Phe Ser
           595                 600                 605
   Leu Ala Glu Ser Arg Lys Lys Thr Glu Arg Leu Ala Phe Arg Lys Asn
       610                 615                 620
   Arg Leu Pro Pro Glu Phe Ser Pro Val Ile Ile Lys Glu Trp Ala Ala
   625                 630                 635                 640
   Tyr Lys Gly Lys Ser Pro Gln Thr Pro Glu Leu Val Glu Ala Leu Ala
                   645                 650                 655
   Phe Arg Glu Trp Thr Cys Pro Asn Leu Lys Arg Leu Trp Leu Gly Lys
               660                 665                 670
   Ala Val Glu Asp Lys Asn Arg Arg Met Arg Ala Phe Leu Ala Cys Met
           675                 680                 685
   Arg Ser Asp Thr Pro Ala Met Leu Asn Pro Ala Asn Val Pro Thr His
       690                 695                 700
   Leu Met Val Leu Cys Cys Val Leu Arg Tyr Met Val Gln Trp Pro Gly
   705                 710                 715                 720
   Ala Arg Ile Leu Arg Arg Gln Glu Leu Asp Ala Phe Leu Ala Gln Ala
                   725                 730                 735
   Leu Ser Pro Lys Leu Tyr Glu Pro Asp Gln Leu Gln Glu Leu Lys Ile
               740                 745                 750
   Glu Asn Leu Asp Pro Arg Gly Ile Gln Leu Ser Ala Leu Phe Met Ser
           755                 760                 765
   Gly Val Asp Met Ala Leu Phe Ala Asn Asp Ala Cys Gly Gln Pro Ile
       770                 775                 780
   Pro Trp Glu His Cys Cys Pro Trp Met Tyr Phe Asp Gly Lys Leu Phe
   785                 790                 795                 800
   Gln Ser Lys Leu Leu Lys Ala Ser Arg Glu Lys Thr Pro Leu Ile Asp
                   805                 810                 815
   Leu Cys Asp Gly Gln Ala Asp Gln Ala Ala Lys Val Glu Lys Met Arg
               820                 825                 830
   Gln Ser Val Leu Glu Gly Leu Ser Phe Ser Arg Gln Ser His Thr Leu
           835                 840                 845
   Pro Phe Pro Pro Pro Pro Pro Ala Leu Pro Phe Tyr Pro Ala Ser Ala
       850                 855                 860
   Tyr Pro Arg His Phe Gly Pro Val Pro Pro Ser Gln Gly Arg Gly Arg
   865                 870                 875                 880
   Gly Phe Ala Gly Val Cys Gly Phe Gly Gly Pro Tyr Gly Glu Thr Val
                   885                 890                 895
   Ala Thr Gly Pro Tyr Arg Ala Phe Arg Val Ala Ala Ala Ser Gly His
               900                 905                 910
   Cys Gly Ala Phe Ser Gly Ser Asp Ser Ser Arg Thr Ser Lys Ser Gln
           915                 920                 925
   Gly Gly Val Gln Pro Ile Pro Ser Gln Gly Gly Lys Leu Glu Ile Ala
       930                 935                 940
   Gly Thr Val Val Gly His Trp Ala Gly Ser Arg Arg Gly Arg Gly Gly
   945                 950                 955                 960
   Arg Gly Pro Phe Pro Leu Gln Val Val Ser Val Gly Gly Pro Ala Arg
                   965                 970                 975
   Gly Arg Pro Arg Gly Val Ile Ser Thr Pro Val Ile Arg Thr Phe Gly
               980                 985                 990
   Arg Gly Gly Arg Tyr Tyr Gly Arg Gly Tyr Lys Asn Gln Ala Ala Ile
           995                 1000                1005
   Gln Gly Arg Pro Pro Tyr Ala Ala Ser Ala Glu Glu Val Ala Lys Glu
       1010                1015                1020
   Leu Lys Ser Lys Ser Gly Glu Ser Lys Ser Ser Ala Met Ser Ser Asp
   1025                1030                1035                1040
   Gly Ser Leu Ala Glu Asn Gly Val Ile Gly Arg Gly Glu Ala Gly Ser
                   1045                1050                1055
   Pro Asp Glu Arg Glu His Gly
               1060
<210> 44
   <211> 10427
   <212> DNA
   <213> Rattus sp.
<220>
   <221> CDS
   <222> (211)..(9996)
<220>
   <221> misc_feature
   <222> (115)..(115)
   <223> "Xaa" can be any amino acid
<400> 44
   cgacaaaggc agcctgtgcc gcacagttga ggggtgccgt gaggagctgc agaatcaggc 60
   caatttctcc ttcgctcctc tcgtgttaga catgctcaat ttcctcatgg atgccattca 120
   gaccaacttt cacaggcttc cgccgtgggg agcagcagcc gggcacagca ggccctcagt 180
   gaactgcaca cagtggacaa ggttgtggag atg aca gac cag ctg atg gtt ccc  234
                                    Met Thr Asp Gln Leu Met Val Pro
                                     1               5
   acc tta ggc tca cag gaa ggt gcc ttt gag aat gtt cgg atg aat tac   282
   Thr Leu Gly Ser Gln Glu Gly Ala Phe Glu Asn Val Arg Met Asn Tyr
        10                  15                  20
   agc gga gac cag ggc cag act ata cgg cag ctg atc agt gcc cac gtg   330
   Ser Gly Asp Gln Gly Gln Thr Ile Arg Gln Leu Ile Ser Ala His Val
    25                  30                  35                  40
   ctc agg cgg gtg gct atg tgt gtg ctt tct tcc ccc cat ggg cgc cgc   378
   Leu Arg Arg Val Ala Met Cys Val Leu Ser Ser Pro His Gly Arg Arg
                   45                  50                  55
   caa cat ttg gct gtc agc cac gag aag ggc aag atc acg gtg ctg cag   426
   Gln His Leu Ala Val Ser His Glu Lys Gly Lys Ile Thr Val Leu Gln
               60                  65                  70
   ctc tcg gcg ctc ctg aag caa gca gat tcc agt aag agg aag ttg act      474
   Leu Ser Ala Leu Leu Lys Gln Ala Asp Ser Ser Lys Arg Lys Leu Thr
           75                  80                  85
   cta acc cgc ttg gct tct gct cca gtt ccc ttc acc gtg ctg agt ctt      522
   Leu Thr Arg Leu Ala Ser Ala Pro Val Pro Phe Thr Val Leu Ser Leu
       90                  95                  100
   act gga aac ccc tgc aag gag gac tac ctg sca gtg tgt ggg ctg aag      570
   Thr Gly Asn Pro Cys Lys Glu Asp Tyr Leu Xaa Val Cys Gly Leu Lys
   105                 110                 115                 120
   gac tgc cat gtg ctc acc ttc agt agc tcg ggc tct gtc tcc gat cac      618
   Asp Cys His Val Leu Thr Phe Ser Ser Ser Gly Ser Val Ser Asp His
                   125                 130                 135
   tta gtg tta cat ccc cag ttg gca aca ggg aac ttc atc atc aaa gct      666
   Leu Val Leu His Pro Gln Leu Ala Thr Gly Asn Phe Ile Ile Lys Ala
               140                 145                 150
   gtg tgg ctc cct ggc tct cag acc gag cta gcc att gtt act gca gac      714
   Val Trp Leu Pro Gly Ser Gln Thr Glu Leu Ala Ile Val Thr Ala Asp
           155                 160                 165
   ttc gtc aag att tac gac ctg tct gtt gat gcc ttg agc ccc acc ttc      762
   Phe Val Lys Ile Tyr Asp Leu Ser Val Asp Ala Leu Ser Pro Thr Phe
       170                 175                 180
   tac ttc ctc ctg cca agc tca aag ata agg gat gtc acc ttc ctt ttc      810
   Tyr Phe Leu Leu Pro Ser Ser Lys Ile Arg Asp Val Thr Phe Leu Phe
   185                 190                 195                 200
   aac gag gaa ggc aag aac atc att gtc atc atg tcc tct gct ggg tac      858
   Asn Glu Glu Gly Lys Asn Ile Ile Val Ile Met Ser Ser Ala Gly Tyr
                   205                 210                 215
   atg tac aca cag ctc atg gag gag gcc agc agt gcc cag cag ggg ccc      906
   Met Tyr Thr Gln Leu Met Glu Glu Ala Ser Ser Ala Gln Gln Gly Pro
               220                 225                 230
   ttc tat gtc act aat gtg ctg gaa ata aat cac gag gat ctg aag gac      954
   Phe Tyr Val Thr Asn Val Leu Glu Ile Asn His Glu Asp Leu Lys Asp
           235                 240                 245
   agt aac agc cag gtg gca ggt ggt ggc gtg tct gtg tac tat tca cac     1002
   Ser Asn Ser Gln Val Ala Gly Gly Gly Val Ser Val Tyr Tyr Ser His
       250                 255                 260
   gtg ttg cag atg ctt ttc ttc agc tac agt cag ggc aag tcc ttt gca     1050
   Val Leu Gln Met Leu Phe Phe Ser Tyr Ser Gln Gly Lys Ser Phe Ala
   265                 270                 275                 280
   gcc acc gtc agc agg agc act ctg gag gtg ctg cag ctc ttc ccc atc     1098
   Ala Thr Val Ser Arg Ser Thr Leu Glu Val Leu Gln Leu Phe Pro Ile
                   285                 290                 295
   aac atc aaa agc tcc aat ggt ggc agt aag act tct ccc gcc ctt tgc     1146
   Asn Ile Lys Ser Ser Asn Gly Gly Ser Lys Thr Ser Pro Ala Leu Cys
               300                 305                 310
   cag tgg tct gag gtg atg aac cac cct ggc ttg gtg tgt tgt gtt cag    1194
   Gln Trp Ser Glu Val Met Asn His Pro Gly Leu Val Cys Cys Val Gln
           315                 320                 325
   caa act act ggt gtg cct ctg gta gtc atg gtg aaa cca gac act ttc    1242
   Gln Thr Thr Gly Val Pro Leu Val Val Met Val Lys Pro Asp Thr Phe
       330                 335                 340
   ctc atc cag gag att aag act ctt ccc gcc aaa gca aag atc cag gac    1290
   Leu Ile Gln Glu Ile Lys Thr Leu Pro Ala Lys Ala Lys Ile Gln Asp
   345                 350                 355                 360
   atg gtt gct att aga cac act gcc tgt aat gag cag cag cgc acc acc    1338
   Met Val Ala Ile Arg His Thr Ala Cys Asn Glu Gln Gln Arg Thr Thr
                   365                 370                 375
   atg atc ctg ctg tgt gag gat ggc agc ctg cga att tat atg gcc aac    1386
   Met Ile Leu Leu Cys Glu Asp Gly Ser Leu Arg Ile Tyr Met Ala Asn
               380                 385                 390
   gtg gag aac acc tct tac tgg ctc cag ccg tct ctg cag ccc agc agc    1434
   Val Glu Asn Thr Ser Tyr Trp Leu Gln Pro Ser Leu Gln Pro Ser Ser
           395                 400                 405
   gtc atc agc atc atg aag cct gtg cga aag cgc aaa aca gct aca atc    1482
   Val Ile Ser Ile Met Lys Pro Val Arg Lys Arg Lys Thr Ala Thr Ile
       410                 415                 420
   aca gcc cgc aca tcc agc cag gtg acc ttc ccc att gac ttc ttt gaa    1530
   Thr Ala Arg Thr Ser Ser Gln Val Thr Phe Pro Ile Asp Phe Phe Glu
   425                 430                 435                 440
   cac aac cag cag cta acg gat gtg gag ttt ggt ggt aat gac ctc ctg    1578
   His Asn Gln Gln Leu Thr Asp Val Glu Phe Gly Gly Asn Asp Leu Leu
                   445                 450                 455
   caa gtc tac aat gcg caa cag ata aag cac agg ctc aac tcc act ggc    1626
   Gln Val Tyr Asn Ala Gln Gln Ile Lys His Arg Leu Asn Ser Thr Gly
               460                 465                 470
   atg tat gtg gcg aac acc aag cct gga ggc ttc acc atg gag atc agt    1674
   Met Tyr Val Ala Asn Thr Lys Pro Gly Gly Phe Thr Met Glu Ile Ser
           475                 480                 485
   aac aac agt agc acc atg gtg atg acg ggc atg cgg atc cag att ggc    1722
   Asn Asn Ser Ser Thr Met Val Met Thr Gly Met Arg Ile Gln Ile Gly
       490                 495                 500
   aca cag gca atc gag cga gca ccg tcc tac atc gag atc ttt ggc aga    1770
   Thr Gln Ala Ile Glu Arg Ala Pro Ser Tyr Ile Glu Ile Phe Gly Arg
   505                 510                 515                 520
   acc atg cag ctt aac ctg agc cgc tcc cgc tgg ttt gac ttc ccc ttc    1818
   Thr Met Gln Leu Asn Leu Ser Arg Ser Arg Trp Phe Asp Phe Pro Phe
                   525                 530                 535
   acc aga gag gaa gcc ctg cag gct gac cgg aag ctg aac ctc ttc atc    1866
   Thr Arg Glu Glu Ala Leu Gln Ala Asp Arg Lys Leu Asn Leu Phe Ile
               540                 545                 550
   ggt gcc tct gtg gat cca gct ggc gtc acc atg ata gat gct gta aaa    1914
   Gly Ala Ser Val Asp Pro Ala Gly Val Thr Met Ile Asp Ala Val Lys
           555                 560                 565
   att tat ggc aag act aaa gag cag ttt ggc tgg cct gat gag ccc cca    1962
   Ile Tyr Gly Lys Thr Lys Glu Gln Phe Gly Trp Pro Asp Glu Pro Pro
       570                 575                 580
   gaa gac ttc cct tct gcc tct gtt agc agc gtc tgc cct cct aac ctg    2010
   Glu Asp Phe Pro Ser Ala Ser Val Ser Ser Val Cys Pro Pro Asn Leu
   585                 590                 595                 600
   aac cag agc aac ggc act gga gac agt gac tca gct gct cca gct aca    2058
   Asn Gln Ser Asn Gly Thr Gly Asp Ser Asp Ser Ala Ala Pro Ala Thr
                   605                 610                 615
   acc agt ggc act gtc ctg gag agg ctg gtt gtg agt tct ttg gaa gcc    2106
   Thr Ser Gly Thr Val Leu Glu Arg Leu Val Val Ser Ser Leu Glu Ala
               620                 625                 630
   ctg gaa agc tgc ttt gct gtc ggc cca atc att gag aag gag aga aac    2154
   Leu Glu Ser Cys Phe Ala Val Gly Pro Ile Ile Glu Lys Glu Arg Asn
           635                 640                 645
   aag cac gca gcc cag gag ctg gcc act ttg ttg ctc tcc ctg cca gcg    2202
   Lys His Ala Ala Gln Glu Leu Ala Thr Leu Leu Leu Ser Leu Pro Ala
       650                 655                 660
   cct gcc agc gtc caa cag caa tca aag agc ctc ctg gcc agc ctg cac    2250
   Pro Ala Ser Val Gln Gln Gln Ser Lys Ser Leu Leu Ala Ser Leu His
   665                 670                 675                 680
   agc agt cgc tcg gcc tac cac agc cac aag gac cag gcc ttg ttg agc    2298
   Ser Ser Arg Ser Ala Tyr His Ser His Lys Asp Gln Ala Leu Leu Ser
                   685                 690                 695
   aaa gct gtg cag tgt ctc aac act tcc agc aaa gaa ggc aag gac ttg    2346
   Lys Ala Val Gln Cys Leu Asn Thr Ser Ser Lys Glu Gly Lys Asp Leu
               700                 705                 710
   gac ccc gag gtg ttc cag cgt cta gta atc aca gct cgc tct att gcc    2394
   Asp Pro Glu Val Phe Gln Arg Leu Val Ile Thr Ala Arg Ser Ile Ala
           715                 720                 725
   gtc aca cgt ccc aac aac ctt gtg cac ttt acg gag tcc aag ctg ccc    2442
   Val Thr Arg Pro Asn Asn Leu Val His Phe Thr Glu Ser Lys Leu Pro
       730                 735                 740
   cag atg gaa aca gaa gga gcg gag gag ggg aaa gag ccg cag aag cag    2490
   Gln Met Glu Thr Glu Gly Ala Glu Glu Gly Lys Glu Pro Gln Lys Gln
   745                 750                 755                 760
   gtg gaa gga gac ggc tgt agt ttc atc act cag ctt gta aac cac ttc    2538
   Val Glu Gly Asp Gly Cys Ser Phe Ile Thr Gln Leu Val Asn His Phe
                   765                 770                 775
   tgg aaa ctc cat gca tct aag ccc aag aat gcc ttc ctg gca cct gcc    2586
   Trp Lys Leu His Ala Ser Lys Pro Lys Asn Ala Phe Leu Ala Pro Ala
               780                 785                 790
   tgc ctg cca ggc ctt act cat att gaa gct acg gtt aat gcg ctg gta     2634
   Cys Leu Pro Gly Leu Thr His Ile Glu Ala Thr Val Asn Ala Leu Val
           795                 800                 805
   gac att atc cat ggc tat tgt acc tgc gag ctg gac tgt atc aac aca     2682
   Asp Ile Ile His Gly Tyr Cys Thr Cys Glu Leu Asp Cys Ile Asn Thr
       810                 815                 820
   gca tcc aag atc tac atg cag atg ctg ctg tgt cct gac cct gct gtg     2730
   Ala Ser Lys Ile Tyr Met Gln Met Leu Leu Cys Pro Asp Pro Ala Val
   825                 830                 835                 840
   agc ttc tcc tgt aaa caa gct cta att cga gtc cta agg ccc agg aac     2778
   Ser Phe Ser Cys Lys Gln Ala Leu Ile Arg Val Leu Arg Pro Arg Asn
                   845                 850                 855
   aag cgg aga cac gtg aca ttg ccc tcc tcc ccc cga agc aac act cca     2826
   Lys Arg Arg His Val Thr Leu Pro Ser Ser Pro Arg Ser Asn Thr Pro
               860                 865                 870
   atg gga gac aag gat gac gat gat gat gat gat gcg gat gag aaa atg     2874
   Met Gly Asp Lys Asp Asp Asp Asp Asp Asp Asp Ala Asp Glu Lys Met
           875                 880                 885
   cag tca tcg ggc atc cct gat ggt ggt cac atc cgt cag gaa agc cag     2922
   Gln Ser Ser Gly Ile Pro Asp Gly Gly His Ile Arg Gln Glu Ser Gln
       890                 895                 900
   gaa cag agt gag gtg gac cat gga gat ttt gag atg gtg tct gag tcg     2970
   Glu Gln Ser Glu Val Asp His Gly Asp Phe Glu Met Val Ser Glu Ser
   905                 910                 915                 920
   atg gtc ctg gaa aca gct gaa aat gtc aac aat ggc aac ccc tct ccc     3018
   Met Val Leu Glu Thr Ala Glu Asn Val Asn Asn Gly Asn Pro Ser Pro
                   925                 930                 935
   cgg aaa gcc ctg ctg gcc ggt gct gag ggc ttt cct ccc atg ctg gac     3066
   Arg Lys Ala Leu Leu Ala Gly Ala Glu Gly Phe Pro Pro Met Leu Asp
               940                 945                 950
   atc cca cct gat gca gac gac gag acc atg gtt gaa cta gcc att gcc     3114
   Ile Pro Pro Asp Ala Asp Asp Glu Thr Met Val Glu Leu Ala Ile Ala
           955                 960                 965
   ctc agc ctg cag cag gac cag caa ggc agc agc agc agt gcc ctg ggc     3162
   Leu Ser Leu Gln Gln Asp Gln Gln Gly Ser Ser Ser Ser Ala Leu Gly
       970                 975                 980
   ctt cag agc ctg gga ctg tcg ggc cag gca ccc agc tct tcc tct ctg     3210
   Leu Gln Ser Leu Gly Leu Ser Gly Gln Ala Pro Ser Ser Ser Ser Leu
   985                 990                 995                 1000
   gac gca gga acc ctc tct gac acc aca gca tca gct cca gcc tca gat     3258
   Asp Ala Gly Thr Leu Ser Asp Thr Thr Ala Ser Ala Pro Ala Ser Asp
                   1005                1010                1015
   gac gag ggc agc act gca gca act gat ggc tcc acc ctg cgg acc tcg     3306
   Asp Glu Gly Ser Thr Ala Ala Thr Asp Gly Ser Thr Leu Arg Thr Ser
               1020                1025                1030
   cca gcc gac cat ggc ggt agt gtg ggc tca gag agc ggg gga agt gca     3354
   Pro Ala Asp His Gly Gly Ser Val Gly Ser Glu Ser Gly Gly Ser Ala
         1035                1040                1045
   gtg gac tcg gtg gct ggc gag cac agt gtg tct ggc cgg agc agt gca    3402
   Val Asp Ser Val Ala Gly Glu His Ser Val Ser Gly Arg Ser Ser Ala
      1050                1055                1060
   tat ggt gac gcc aca gct gag ggg cac ccg gct gga cca ggg agc gtc    3450
   Tyr Gly Asp Ala Thr Ala Glu Gly His Pro Ala Gly Pro Gly Ser Val
   1065                1070                1075                1080
   agc tct agc aca ggc gcc atc agc acc act act ggg cac cag gag gga    3498
   Ser Ser Ser Thr Gly Ala Ile Ser Thr Thr Thr Gly His Gln Glu Gly
                   1085                1090                1095
   gat gga tct gag gga gaa gga gaa ggg gaa gct gaa gga gat gtg cac    3546
   Asp Gly Ser Glu Gly Glu Gly Glu Gly Glu Ala Glu Gly Asp Val His
               1100                1105                1110
   act agc aac agg cta cac atg gtt cgt cta atg ctg ttg gaa aga tta    3594
   Thr Ser Asn Arg Leu His Met Val Arg Leu Met Leu Leu Glu Arg Leu
         1115                1120                1125
   ctg cag aca ctg ccc cag tta cgg aat gtc gga ggt gtc cgg gcc atc    3642
   Leu Gln Thr Leu Pro Gln Leu Arg Asn Val Gly Gly Val Arg Ala Ile
      1130                1135                1140
   cca tac atg cag gtc att ctc atg ctc act aca gat ctg gat gga gaa    3690
   Pro Tyr Met Gln Val Ile Leu Met Leu Thr Thr Asp Leu Asp Gly Glu
   1145                1150                1155                1160
   gat gag aaa gac aag gga gcc ctg gac aac ctg ctt gca cag ctc att    3738
   Asp Glu Lys Asp Lys Gly Ala Leu Asp Asn Leu Leu Ala Gln Leu Ile
                   1165                1170                1175
   gct gag ctg gga atg gac aaa aag gat gtc tcc aag aag aat gaa cgc    3786
   Ala Glu Leu Gly Met Asp Lys Lys Asp Val Ser Lys Lys Asn Glu Arg
               1180                1185                1190
   agt gca ttg aac gaa gtc cat ttg gtc gtg atg aga ctc ctg agt gtc    3834
   Ser Ala Leu Asn Glu Val His Leu Val Val Met Arg Leu Leu Ser Val
         1195                1200                1205
   ttc atg tcc cgc acc aag tct gga tcc aaa tct tcc atc tgt gag tcg    3882
   Phe Met Ser Arg Thr Lys Ser Gly Ser Lys Ser Ser Ile Cys Glu Ser
      1210                1215                1220
   tct tcc ctc atc tcc agt gcc acg gca gca gcc ctg ctg agc tcc ggg    3930
   Ser Ser Leu Ile Ser Ser Ala Thr Ala Ala Ala Leu Leu Ser Ser Gly
   1225                1230                1235                1240
   gcc gtg gac tat tgc ctc cat gtg ctc aag tct ctg ctg gaa tac tgg    3978
   Ala Val Asp Tyr Cys Leu His Val Leu Lys Ser Leu Leu Glu Tyr Trp
                   1245                1250                1255
   aag ggc cag cag agt gag gag gag cct gtg acc acc agc caa ttg ctg    4026
   Lys Gly Gln Gln Ser Glu Glu Glu Pro Val Thr Thr Ser Gln Leu Leu
               1260                1265                1270
   aaa cca cac acg acc tca tct cca cca gat atg agc ccg ttc ttt ctc    4074
   Lys Pro His Thr Thr Ser Ser Pro Pro Asp Met Ser Pro Phe Phe Leu
           1275                1280                1285
   cga caa tat gtg aag ggt cat gct gct gat gtg ttc gaa gcc tat acc    4122
   Arg Gln Tyr Val Lys Gly His Ala Ala Asp Val Phe Glu Ala Tyr Thr
       1290                1295                1300
   cag ctg ctc aca gaa atg gta ctg agg ctt ccc tac cag atc aag aag    4170
   Gln Leu Leu Thr Glu Met Val Leu Arg Leu Pro Tyr Gln Ile Lys Lys
   1305                1310                1315                1320
   atc gca gat acc agc tct cga atc cct cct cct gtc ttt gat cac tcc    4218
   Ile Ala Asp Thr Ser Ser Arg Ile Pro Pro Pro Val Phe Asp His Ser
                   1325                1330                1335
   tgg ttc tac ttt ctc tct gag tac cta atg atc cag cag acc cct ttt    4266
   Trp Phe Tyr Phe Leu Ser Glu Tyr Leu Met Ile Gln Gln Thr Pro Phe
               1340                1345                1350
   gtc cgc cgc caa gtc cgg aaa ctt ctg ctc ttc atc tgt gga tca aag    4314
   Val Arg Arg Gln Val Arg Lys Leu Leu Leu Phe Ile Cys Gly Ser Lys
           1355                1360                1365
   gag aaa tac cgt cag ctc cga gac ctg cac acc ctg gac tcc cat gtg    4362
   Glu Lys Tyr Arg Gln Leu Arg Asp Leu His Thr Leu Asp Ser His Val
       1370                1375                1380
   cgt ggg atc aag aag ctg ctg gag gaa caa ggc atc ttc ctc agg gcc    4410
   Arg Gly Ile Lys Lys Leu Leu Glu Glu Gln Gly Ile Phe Leu Arg Ala
   1385                1390                1395                1400
   agc gtg gtt aca gcc agt tcc ggc tcc gct ttg cag tat gac acg ctc    4458
   Ser Val Val Thr Ala Ser Ser Gly Ser Ala Leu Gln Tyr Asp Thr Leu
                   1405                1410                1415
   atc agc ctg atg gag cac ctg aag gcc tgt gca gag att gcc gcc cag    4506
   Ile Ser Leu Met Glu His Leu Lys Ala Cys Ala Glu Ile Ala Ala Gln
               1420                1425                1430
   cgc acc atc aac tgg cag aag ttc tgc atc aaa gat gac tct gtc ctg    4554
   Arg Thr Ile Asn Trp Gln Lys Phe Cys Ile Lys Asp Asp Ser Val Leu
           1435                1440                1445
   tac ttc ctc ctg caa gtg agt ttc ctg gta gat gag ggg gtg tcc cct    4602
   Tyr Phe Leu Leu Gln Val Ser Phe Leu Val Asp Glu Gly Val Ser Pro
       1450                1455                1460
   gtg cta ctg cag ctg ctt tcc tgt gct ctt tgc ggc agc aaa gtg ctc    4650
   Val Leu Leu Gln Leu Leu Ser Cys Ala Leu Cys Gly Ser Lys Val Leu
   1465                1470                1475                1480
   gca gcc ctg gcg gcc tcc acg ggc tcc tcc agt gtg gct tcc tca gcc    4698
   Ala Ala Leu Ala Ala Ser Thr Gly Ser Ser Ser Val Ala Ser Ser Ala
                   1485                1490                1495
   cct cct gca gcc agt tct gga caa acc aca act cag tcc aaa tcc tcc    4746
   Pro Pro Ala Ala Ser Ser Gly Gln Thr Thr Thr Gln Ser Lys Ser Ser
               1500                1505                1510
   act aaa aag agc aag aaa gaa gaa aag gag aaa gag aaa gag ggt gag    4794
   Thr Lys Lys Ser Lys Lys Glu Glu Lys Glu Lys Glu Lys Glu Gly Glu
           1515                1520                1525
   agc tcg ggt agc cag gaa gac cag ctg tgt aca gct ctc gtg aac cag    4842
   Ser Ser Gly Ser Gln Glu Asp Gln Leu Cys Thr Ala Leu Val Asn Gln
       1530                1535                1540
   ctg aac aga ttt gca gac aag gag act ctg atc cag ttt ctg cgc tgc    4890
   Leu Asn Arg Phe Ala Asp Lys Glu Thr Leu Ile Gln Phe Leu Arg Cys
   1545                1550                1555                1560
   ttc cta ttg gag tcc aac tct tcg tca gta cgc tgg cag gcc cac tgc    4938
   Phe Leu Leu Glu Ser Asn Ser Ser Ser Val Arg Trp Gln Ala His Cys
                   1565                1570                1575
   ctg aca ctg cac atc tac aga aac tcc aac aag gct cag cag gag ctc    4986
   Leu Thr Leu His Ile Tyr Arg Asn Ser Asn Lys Ala Gln Gln Glu Leu
               1580                1585                1590
   ttg cta gat ctc atg tgg tca atc tgg cca gaa ctc cca gct tat ggt    5034
   Leu Leu Asp Leu Met Trp Ser Ile Trp Pro Glu Leu Pro Ala Tyr Gly
           1595                1600                1605
   cgg aag gct gcc cag ttt gtg gat ctc ctg ggc tat ttc tcc ctg aaa    5082
   Arg Lys Ala Ala Gln Phe Val Asp Leu Leu Gly Tyr Phe Ser Leu Lys
       1610                1615                1620
   act gca cag aca gag aag aag ttg aag gag tac tca cag aag gcc gtg    5130
   Thr Ala Gln Thr Glu Lys Lys Leu Lys Glu Tyr Ser Gln Lys Ala Val
   1625                1630                1635                1640
   gag att ctg agg act cag aac cac att ctc acc aat cat ccc aac tcc    5178
   Glu Ile Leu Arg Thr Gln Asn His Ile Leu Thr Asn His Pro Asn Ser
                   1645                1650                1655
   aac att tac aat acc ttg tct ggc tta gtg gag ttt gat ggc tac tac    5226
   Asn Ile Tyr Asn Thr Leu Ser Gly Leu Val Glu Phe Asp Gly Tyr Tyr
               1660                1665                1670
   cta gag agt gat ccc tgc ctg gtg tgt aat aac cct gaa gtg ccg ttt    5274
   Leu Glu Ser Asp Pro Cys Leu Val Cys Asn Asn Pro Glu Val Pro Phe
           1675                1680                1685
   tgt tat atc aag ctg tct tcc att aaa gtg gac aca cgg tac acc acc    5322
   Cys Tyr Ile Lys Leu Ser Ser Ile Lys Val Asp Thr Arg Tyr Thr Thr
       1690                1695                1700
   acc cag cag gtg gta aag ctc att ggt agt cac acc atc agc aag gtg    5370
   Thr Gln Gln Val Val Lys Leu Ile Gly Ser His Thr Ile Ser Lys Val
   1705                1710                1715                1720
   aca gtg aaa att ggg gac ctg aag cgg acc aag atg gtg cgg aca atc    5418
   Thr Val Lys Ile Gly Asp Leu Lys Arg Thr Lys Met Val Arg Thr Ile
                   1725                1730                1735
   aac ctg tat tac aac aac cga act gtg cag gcc att gtg gag ttg aaa    5466
   Asn Leu Tyr Tyr Asn Asn Arg Thr Val Gln Ala Ile Val Glu Leu Lys
               1740                1745                1750
   aat aag cca gct cgc tgg cac aaa gcc aag aaa gtt cag ctg acg cct    5514
   Asn Lys Pro Ala Arg Trp His Lys Ala Lys Lys Val Gln Leu Thr Pro
           1755                1760                1765
   gga cag aca gaa gtc aag att gac ctg cct ctg ccc att gtg gcc tct    5562
   Gly Gln Thr Glu Val Lys Ile Asp Leu Pro Leu Pro Ile Val Ala Ser
      1770                1775                1780
   aac ctg atg atc gag ttt gca gac ttc tat gag aac tac cag gct tcc    5610
   Asn Leu Met Ile Glu Phe Ala Asp Phe Tyr Glu Asn Tyr Gln Ala Ser
   1785                1790                1795                1800
   aca gag acc ctt cag tgt cct cgc tgc agt gcc tct gtc ccc gcc aat    5658
   Thr Glu Thr Leu Gln Cys Pro Arg Cys Ser Ala Ser Val Pro Ala Asn
                   1805                1810                1815
   ccc ggc gtc tgt ggc aac tgt ggc gag aat gta tac cag tgt cac aag    5706
   Pro Gly Val Cys Gly Asn Cys Gly Glu Asn Val Tyr Gln Cys His Lys
               1820                1825                1830
   tgc agg tcc atc aac tat gat gag aag gat ccc ttc ctc tgc aat gct    5754
   Cys Arg Ser Ile Asn Tyr Asp Glu Lys Asp Pro Phe Leu Cys Asn Ala
           1835                1840                1845
   tgt ggc ttt tgt aaa tat gcc cgc ttt gac ttc atg ctt tat gcc aaa    5802
   Cys Gly Phe Cys Lys Tyr Ala Arg Phe Asp Phe Met Leu Tyr Ala Lys
       1850                1855                1860
   cct tgc tgc cca gtg gat ccc atc gag aac gag gaa gat cgg aag aag    5850
   Pro Cys Cys Pro Val Asp Pro Ile Glu Asn Glu Glu Asp Arg Lys Lys
   1865                1870                1875                1880
   gcc gtt tcc aac atc aac aca ctt ctg gac aaa gct gac cgc gtg tac    5898
   Ala Val Ser Asn Ile Asn Thr Leu Leu Asp Lys Ala Asp Arg Val Tyr
                   1885                1890                1895
   cat cag ctc atg gga cac cgg ccc cag ctg gag aac ctg ctc tgc aaa    5946
   His Gln Leu Met Gly His Arg Pro Gln Leu Glu Asn Leu Leu Cys Lys
               1900                1905                1910
   gtg aat gaa gca gct cca gaa aag cca cag gaa gac tcg gga aca gcg    5994
   Val Asn Glu Ala Ala Pro Glu Lys Pro Gln Glu Asp Ser Gly Thr Ala
           1915                1920                1925
   gga ggc atc agc tcc act tca gcc agt gtg aat cgc tac atc cta cag    6042
   Gly Gly Ile Ser Ser Thr Ser Ala Ser Val Asn Arg Tyr Ile Leu Gln
       1930                1935                1940
   ctg gcg cag gag tat tgt gga gac tgt aag aac tca ttt gac gag ctc    6090
   Leu Ala Gln Glu Tyr Cys Gly Asp Cys Lys Asn Ser Phe Asp Glu Leu
   1945                1950                1955                1960
   tcc aaa atc atc cag aaa gtc ttc gct tcg cgc aaa gag ctg tta gag    6138
   Ser Lys Ile Ile Gln Lys Val Phe Ala Ser Arg Lys Glu Leu Leu Glu
                   1965                1970                1975
   tat gac ctg cag cag aga gaa gcg gcc acc aag tca tcc cgg aca tcc    6186
   Tyr Asp Leu Gln Gln Arg Glu Ala Ala Thr Lys Ser Ser Arg Thr Ser
               1980                1985                1990
   gtg cag ccc acg ttt act gcc agc cag tac cgt gcg ttg tct gtc tta    6234
   Val Gln Pro Thr Phe Thr Ala Ser Gln Tyr Arg Ala Leu Ser Val Leu
           1995                2000                2005
   ggc tgt ggc cac acc tcc tcc acc aag tgc tat ggc tgt gcc tca gct    6282
   Gly Cys Gly His Thr Ser Ser Thr Lys Cys Tyr Gly Cys Ala Ser Ala
   2010 2015 2020
   gtc aca gag cat tgt atc aca ctg cta agg gcc ctg gcc acc aat cca 6330
   Val Thr Glu His Cys Ile Thr Leu Leu Arg Ala Leu Ala Thr Asn Pro
   2025 2030 2035 2040
   gcc ctg aga cac atc ctc gtc tcc cag ggc ctc atc cga gag ctc ttt 6378
   Ala Leu Arg His Ile Leu Val Ser Gln Gly Leu Ile Arg Glu Leu Phe
   2045 2050 2055
   gat tac aac ctc cgc aga ggc gcg gca gcc atc cgc gag gag gtc cgc 6426
   Asp Tyr Asn Leu Arg Arg Gly Ala Ala Ala Ile Arg Glu Glu Val Arg
   2060 2065 2070
   cag ctc atg tgc ctc ctg act aga gac aac cca gag gcc acc cag caa 6474
   Gln Leu Met Cys Leu Leu Thr Arg Asp Asn Pro Glu Ala Thr Gln Gln
   2075 2080 2085
   atg aat gac ctg atc att ggc aaa gtc tcc act gca ctg aag ggc cac 6522
   Met Asn Asp Leu Ile Ile Gly Lys Val Ser Thr Ala Leu Lys Gly His
   2090 2095 2100
   tgg gct aat cct gac ctg gct agc agc ctt cag tat gag atg ctg ctg 6570
   Trp Ala Asn Pro Asp Leu Ala Ser Ser Leu Gln Tyr Glu Met Leu Leu
   2105 2110 2115 2120
   ctg aca gac tcc atc tcc aag gag gac agc tgc tgg gaa ctc cgc tta 6618
   Leu Thr Asp Ser Ile Ser Lys Glu Asp Ser Cys Trp Glu Leu Arg Leu
   2125 2130 2135
   cgc tgt gca ctc agc ctt ttc ctc atg gct gtg aac atc aaa acg cca 6666
   Arg Cys Ala Leu Ser Leu Phe Leu Met Ala Val Asn Ile Lys Thr Pro
   2140 2145 2150
   gtg gtt gtt gag aac atc acc ctc atg tgt ctg cgg atc tta cag aag 6714
   Val Val Val Glu Asn Ile Thr Leu Met Cys Leu Arg Ile Leu Gln Lys
   2155 2160 2165
   ctg att aaa cca cct gcc cca acc agc aag aag aac aag gac gtc cct 6762
   Leu Ile Lys Pro Pro Ala Pro Thr Ser Lys Lys Asn Lys Asp Val Pro
   2170 2175 2180
   gtg gag gcc ctc acc acg gtg aag ccg tac tgc aat gag atc cac gcg 6810
   Val Glu Ala Leu Thr Thr Val Lys Pro Tyr Cys Asn Glu Ile His Ala
   2185 2190 2195 2200
   cag gct cag ctg tgg ctc aag cga gat cct aag gca tcc tac gaa gcc 6858
   Gln Ala Gln Leu Trp Leu Lys Arg Asp Pro Lys Ala Ser Tyr Glu Ala
   2205 2210 2215
   tgg aag aag tgc ctg cct atc cga ggg gta gat ggc aac ggg aaa tcc 6906
   Trp Lys Lys Cys Leu Pro Ile Arg Gly Val Asp Gly Asn Gly Lys Ser
   2220 2225 2230
   ccc agc aag tcc gag ctc cac cgg ctc tac ttg act gag aag tat gtg 6954
   Pro Ser Lys Ser Glu Leu His Arg Leu Tyr Leu Thr Glu Lys Tyr Val
   2235 2240 2245
   tgg agg tgg aaa cag ttc ctg agt cgg cgg ggg aag agg acc acc cca 7002
   Trp Arg Trp Lys Gln Phe Leu Ser Arg Arg Gly Lys Arg Thr Thr Pro
   2250 2255 2260
   ctt gac ctc aag ctg ggc cac aac aac tgg ctg cgg cag gta ctc ttt 7050
   Leu Asp Leu Lys Leu Gly His Asn Asn Trp Leu Arg Gln Val Leu Phe
   2265 2270 2275 2280
   acc ccg gca aca cag gca gca cgg cag gca gcc tgt act att gtg gaa 7098
   Thr Pro Ala Thr Gln Ala Ala Arg Gln Ala Ala Cys Thr Ile Val Glu
   2285 2290 2295
   gct ctt gct act gtc ccc agc cgc aag cag cag gtc ctt gac ctc ctc 7146
   Ala Leu Ala Thr Val Pro Ser Arg Lys Gln Gln Val Leu Asp Leu Leu
   2300 2305 2310
   acc agt tac ctg gac gag ctg agt gtg gct ggg gag tgt gct gca gag 7194
   Thr Ser Tyr Leu Asp Glu Leu Ser Val Ala Gly Glu Cys Ala Ala Glu
   2315 2320 2325
   tac ctg gct ctc tac cag aag ctc atc gcc tcc tgc cac tgg aaa gtc 7242
   Tyr Leu Ala Leu Tyr Gln Lys Leu Ile Ala Ser Cys His Trp Lys Val
   2330 2335 2340
   tac ctg gct gct cgg gga gtc ctg ccc tac gtg ggc aac ctc atc acc 7290
   Tyr Leu Ala Ala Arg Gly Val Leu Pro Tyr Val Gly Asn Leu Ile Thr
   2345 2350 2355 2360
   aag gaa atc gcc cgc ctg ctg gcc ttg gag gag gcc aca ctg agc aca 7338
   Lys Glu Ile Ala Arg Leu Leu Ala Leu Glu Glu Ala Thr Leu Ser Thr
   2365 2370 2375
   gac ctg cag cag gga tat gcc ctc aag agt ctc aca ggc ctc ctc tcc 7386
   Asp Leu Gln Gln Gly Tyr Ala Leu Lys Ser Leu Thr Gly Leu Leu Ser
   2380 2385 2390
   tcc ttt gta gag gtg gag tcc atc aag cgt cat ttc aag agc cgc ttg 7434
   Ser Phe Val Glu Val Glu Ser Ile Lys Arg His Phe Lys Ser Arg Leu
   2395 2400 2405
   gtg ggc act gtg ctg aat ggg tac ctg tgc ttg cgg aaa cta gtg ctg 7482
   Val Gly Thr Val Leu Asn Gly Tyr Leu Cys Leu Arg Lys Leu Val Leu
   2410 2415 2420
   cag agg acc aag ctc atc gat gag acc cag gac atg ctg ctg gag atg 7530
   Gln Arg Thr Lys Leu Ile Asp Glu Thr Gln Asp Met Leu Leu Glu Met
   2425 2430 2435 2440
   ctg gag gac atg acc aca ggt aca gag tct gaa acc aag gcc ttc atg 7578
   Leu Glu Asp Met Thr Thr Gly Thr Glu Ser Glu Thr Lys Ala Phe Met
   2445 2450 2455
   gct gtg tgc att gag aca gcc aag cgc tac aat ctg gat gac tac cgg 7626
   Ala Val Cys Ile Glu Thr Ala Lys Arg Tyr Asn Leu Asp Asp Tyr Arg
   2460 2465 2470
   act cct gtg ttc atc ttt gag agg ctg tgc agc atc atc tac cct gag 7674
   Thr Pro Val Phe Ile Phe Glu Arg Leu Cys Ser Ile Ile Tyr Pro Glu
   2475 2480 2485
   gaa aat gaa gtc act gag ttc ttt gtg acc ctg gag aag gac ccc cag 7722
   Glu Asn Glu Val Thr Glu Phe Phe Val Thr Leu Glu Lys Asp Pro Gln
   2490 2495 2500
   caa gag gac ttt cta cag ggc agg atg ccc gga aac cca tat agc agc    7770
   Gln Glu Asp Phe Leu Gln Gly Arg Met Pro Gly Asn Pro Tyr Ser Ser
   2505                2510                2515                2520
   aat gaa cca ggc att ggg cct ctt atg agg gac ata aag aac aag atc    7818
   Asn Glu Pro Gly Ile Gly Pro Leu Met Arg Asp Ile Lys Asn Lys Ile
                   2525                2530                2535
   tgc cag gac tgt gac ctg gtg gct ctt ctg gag gac gac agt ggg atg    7866
   Cys Gln Asp Cys Asp Leu Val Ala Leu Leu Glu Asp Asp Ser Gly Met
               2540                2545                2550
   gag ctt cta gtg aac aat aaa att atc agt ctg gat ctt cct gtg gct    7914
   Glu Leu Leu Val Asn Asn Lys Ile Ile Ser Leu Asp Leu Pro Val Ala
           2555                2560                2565
   gag gtt tac aag aag gtc tgg tgt acg acc aat gag gga gag ccc atg    7962
   Glu Val Tyr Lys Lys Val Trp Cys Thr Thr Asn Glu Gly Glu Pro Met
       2570                2575                2580
   agg att gtt tat cga atg cgg ggg cta ctg ggc gat gcc acc gag gag    8010
   Arg Ile Val Tyr Arg Met Arg Gly Leu Leu Gly Asp Ala Thr Glu Glu
   2585                2590                2595                2600
   ttt atc gag tcc ctg gac tcc acc aca gat gaa gaa gag gac gaa gag    8058
   Phe Ile Glu Ser Leu Asp Ser Thr Thr Asp Glu Glu Glu Asp Glu Glu
                   2605                2610                2615
   gaa gtg tac aga atg gcc ggt gtg atg gcc cag tgc ggg ggt ctg cag    8106
   Glu Val Tyr Arg Met Ala Gly Val Met Ala Gln Cys Gly Gly Leu Gln
               2620                2625                2630
   tgc atg ctg aac aga ctg gca gga gtc aaa gat ttt aag cag ggg cgc    8154
   Cys Met Leu Asn Arg Leu Ala Gly Val Lys Asp Phe Lys Gln Gly Arg
           2635                2640                2645
   cac ctt cta aca gtg ctc ctg aag ctg ttc agt tac tgt gtg aag gtg    8202
   His Leu Leu Thr Val Leu Leu Lys Leu Phe Ser Tyr Cys Val Lys Val
       2650                2655                2660
   aaa gtc aat cgg caa cag ttg gtc aag ctg gaa atg aac acc ttg aat    8250
   Lys Val Asn Arg Gln Gln Leu Val Lys Leu Glu Met Asn Thr Leu Asn
   2665                2670                2675                2680
   gtc atg ttg ggg act tta aac ttg gct ctg gta gct gag caa gag agc    8298
   Val Met Leu Gly Thr Leu Asn Leu Ala Leu Val Ala Glu Gln Glu Ser
                   2685                2690                2695
   aag gac agc gga gga gca gcg gtg gct gag cag gtg ctg agc atc atg    8346
   Lys Asp Ser Gly Gly Ala Ala Val Ala Glu Gln Val Leu Ser Ile Met
               2700                2705                2710
   gag atc att cta gat gag tcc aat gca gag ccc ctg agt gag gac aag    8394
   Glu Ile Ile Leu Asp Glu Ser Asn Ala Glu Pro Leu Ser Glu Asp Lys
           2715                2720                2725
   ggc aac ctc ctc ctc aca ggt gac aag gat caa ctg gtg atg ctc ttg    8442
   Gly Asn Leu Leu Leu Thr Gly Asp Lys Asp Gln Leu Val Met Leu Leu
       2730                2735                2740
   gac cag atc aac agc acc ttc gtt cgt tcc aat ccc agc gtg ctg cag     8490
   Asp Gln Ile Asn Ser Thr Phe Val Arg Ser Asn Pro Ser Val Leu Gln
   2745                2750                2755                2760
   ggt ttg ctg cgt atc atc cca tac ctg tcc ttc gga gag gtg gag aag     8538
   Gly Leu Leu Arg Ile Ile Pro Tyr Leu Ser Phe Gly Glu Val Glu Lys
                   2765                2770                2775
   atg cag atc ctg gtg gag cgg ttc aag cca tac tgc agc ttc gat aag     8586
   Met Gln Ile Leu Val Glu Arg Phe Lys Pro Tyr Cys Ser Phe Asp Lys
               2780                2785                2790
   tac gat gaa gac cat agt ggg gat gac aaa gtc ttc ctg gat tgc ttc     8634
   Tyr Asp Glu Asp His Ser Gly Asp Asp Lys Val Phe Leu Asp Cys Phe
           2795                2800                2805
   tgc aag att gct gct ggt atc aag aac aac agc aat ggt cat cag ctg     8682
   Cys Lys Ile Ala Ala Gly Ile Lys Asn Asn Ser Asn Gly His Gln Leu
       2810                2815                2820
   aag gac ctc atc ctg cag aag gga atc acc cag agt gcc ctg gac tac     8730
   Lys Asp Leu Ile Leu Gln Lys Gly Ile Thr Gln Ser Ala Leu Asp Tyr
   2825                2830                2835                2840
   atg aaa aag cat atc ccc agt gcc aag aat ttg gat gct gac atc tgg     8778
   Met Lys Lys His Ile Pro Ser Ala Lys Asn Leu Asp Ala Asp Ile Trp
                   2845                2850                2855
   aaa aag ttt ttg tct cgg cct gct ctg cca ttt att ttg agg ctt ctt     8826
   Lys Lys Phe Leu Ser Arg Pro Ala Leu Pro Phe Ile Leu Arg Leu Leu
               2860                2865                2870
   cgg ggt ctg gcc atg caa cac cct gcc acc cag gtt ctg att gga acg     8874
   Arg Gly Leu Ala Met Gln His Pro Ala Thr Gln Val Leu Ile Gly Thr
           2875                2880                2885
   gac tcc atc aca agc ctg cat aag cta gag cag gtg tcc agt gat gag     8922
   Asp Ser Ile Thr Ser Leu His Lys Leu Glu Gln Val Ser Ser Asp Glu
       2890                2895                2900
   ggc att ggg acc ttg gcc gag aac ctc ctg gag gcc cta aga gaa cac     8970
   Gly Ile Gly Thr Leu Ala Glu Asn Leu Leu Glu Ala Leu Arg Glu His
   2905                2910                2915                2920
   cca gat gtg aac aag aag att gac gca gcc cgc aga gag acc cgg gca     9018
   Pro Asp Val Asn Lys Lys Ile Asp Ala Ala Arg Arg Glu Thr Arg Ala
                   2925                2930                2935
   gag aag aag cgc atg gcc atg gcc atg agg cag aag gct ctg gga act     9066
   Glu Lys Lys Arg Met Ala Met Ala Met Arg Gln Lys Ala Leu Gly Thr
               2940                2945                2950
   ctg ggc atg acg aca aac gag aag ggt cag gtg gtg acc aag act gcg     9114
   Leu Gly Met Thr Thr Asn Glu Lys Gly Gln Val Val Thr Lys Thr Ala
           2955                2960                2965
   ctc ctg aag caa atg gaa gag ctg att gag gag ccc gga ctc acg tgc     9162
   Leu Leu Lys Gln Met Glu Glu Leu Ile Glu Glu Pro Gly Leu Thr Cys
       2970                2975                2980
   tgc atc tgc agg gaa ggg ggt acc aag ttc cag ccc acg aag gtc tta     9210
   Cys Ile Cys Arg Glu Gly Gly Thr Lys Phe Gln Pro Thr Lys Val Leu
   2985                2990                2995                3000
   ggc att tac act ttc acc aag cgg gtg gcc ttg gag gag atg gag aac     9258
   Gly Ile Tyr Thr Phe Thr Lys Arg Val Ala Leu Glu Glu Met Glu Asn
               3005                3010                3015
   aag cct cgg aaa cag cag ggc tac agc act gtg tcc cat ttc aac atc     9306
   Lys Pro Arg Lys Gln Gln Gly Tyr Ser Thr Val Ser His Phe Asn Ile
           3020                3025                3030
   gtg cac tat gac tgt cac ctg gct gct gtc agg ttg gct aga ggc cgg     9354
   Val His Tyr Asp Cys His Leu Ala Ala Val Arg Leu Ala Arg Gly Arg
       3035                3040                3045
   gaa gag tgg gaa agt gct gcc ctg cag aac gcc aac acg aag tgc aat     9402
   Glu Glu Trp Glu Ser Ala Ala Leu Gln Asn Ala Asn Thr Lys Cys Asn
       3050                3055                3060
   gga ctc ctt ccg gtc tgg ggc ccc cat gtc cct gag tca gct ttt gcc     9450
   Gly Leu Leu Pro Val Trp Gly Pro His Val Pro Glu Ser Ala Phe Ala
   3065                3070                3075                3080
   act tgt tta gct agg cac aac act tac ctc cag gag tgt acc ggc cag     9498
   Thr Cys Leu Ala Arg His Asn Thr Tyr Leu Gln Glu Cys Thr Gly Gln
               3085                3090                3095
   cgg gag ccc acg tac cag ctc aac atc cac gac atc aaa ctg ctt ttc     9546
   Arg Glu Pro Thr Tyr Gln Leu Asn Ile His Asp Ile Lys Leu Leu Phe
           3100                3105                3110
   ctg cgc ttc gcc atg gag cag tca ttc agt gca gac acc ggt ggg ggc     9594
   Leu Arg Phe Ala Met Glu Gln Ser Phe Ser Ala Asp Thr Gly Gly Gly
       3115                3120                3125
   ggt cgg gag agc aac atc cac ctg atc ccg tac atc att cac act gtg     9642
   Gly Arg Glu Ser Asn Ile His Leu Ile Pro Tyr Ile Ile His Thr Val
       3130                3135                3140
   ctt tac gtc ctg aac acg acc cga gca acg tcc cgg gag gag aag aac     9690
   Leu Tyr Val Leu Asn Thr Thr Arg Ala Thr Ser Arg Glu Glu Lys Asn
   3145                3150                3155                3160
   ctc caa ggc ttc ctg gag cag ccc aga gag aag tgg aca gag agt gcc     9738
   Leu Gln Gly Phe Leu Glu Gln Pro Arg Glu Lys Trp Thr Glu Ser Ala
               3165                3170                3175
   ttt gat gta gat ggg ccc cac tac ttc acc atc tta gcc ctg cat gtc     9786
   Phe Asp Val Asp Gly Pro His Tyr Phe Thr Ile Leu Ala Leu His Val
           3180                3185                3190
   ctc ccc cct gag cag tgg aaa gcc act cga gtg gag atc cta cgc agg     9834
   Leu Pro Pro Glu Gln Trp Lys Ala Thr Arg Val Glu Ile Leu Arg Arg
       3195                3200                3205
   ctg ctg gtg gcc tcg cat gcc cgg gca gtg gct cca gga gga gcc acc     9882
   Leu Leu Val Ala Ser His Ala Arg Ala Val Ala Pro Gly Gly Ala Thr
       3210                3215                3220
   agg ttg aca gat aag gcg gtg aaa gac tat tct gcc tac cgt tct tcc     9930
   Arg Leu Thr Asp Lys Ala Val Lys Asp Tyr Ser Ala Tyr Arg Ser Ser
   3225                3230                3235                3240
   ttg ctg ttc tgg gct ctc gtt gat ctc att tat aac atg ttt aag aag   9978
   Leu Leu Phe Trp Ala Leu Val Asp Leu Ile Tyr Asn Met Phe Lys Lys
                   3245                3250                3255
   gtg ccc act agt aac act tgagggcggg ctggtcctng ctctctggct         10026
   Val Pro Thr Ser Asn Thr
               3260
   gagtacattc cgccacaaat gacatgccca ttctacgaag ctgccgacaa agcccctgaa 10086
   aancttccag gaggagttca tgccagtgga gaccttctca gagttcctcg acgcagcagg 10146
   tcttctatca gaaatcaccg acccagagag cttcctaaag gacctgttga actcagtccc 10206
   ctgaccatcc ccatggaaat gagatcgcgg tgacgagatt gaagctagct tgcattctcc 10266
   cctcattcat ctctccgtgt gcactcattt cccccacgga tgctgcatta gcaccccctc 10326
   ctccctcagt tttcttggag tggcttgggg tttgtaggct tcctgttcta tcacgcgcgc 10386
   gtgtgtgtgt gtgtgtgtgt gtgtgtgtgt gtgtgtgtgt g                   10427
<210> 45
   <211> 3262
   <212> PRT
   <213> Rattus sp.
<400> 45
   Met Thr Asp Gln Leu Met Val Pro Thr Leu Gly Ser Gln Glu Gly Ala
    1               5                  10                  15
   Phe Glu Asn Val Arg Met Asn Tyr Ser Gly Asp Gln Gly Gln Thr Ile
               20                  25                  30
   Arg Gln Leu Ile Ser Ala His Val Leu Arg Arg Val Ala Met Cys Val
           35                  40                  45
   Leu Ser Ser Pro His Gly Arg Arg Gln His Leu Ala Val Ser His Glu
       50                  55                  60
   Lys Gly Lys Ile Thr Val Leu Gln Leu Ser Ala Leu Leu Lys Gln Ala
   65                  70                  75                  80
   Asp Ser Ser Lys Arg Lys Leu Thr Leu Thr Arg Leu Ala Ser Ala Pro
                   85                  90                  95
   Val Pro Phe Thr Val Leu Ser Leu Thr Gly Asn Pro Cys Lys Glu Asp
               100                 105                 110
   Tyr Leu Xaa Val Cys Gly Leu Lys Asp Cys His Val Leu Thr Phe Ser
           115                 120                 125
   Ser Ser Gly Ser Val Ser Asp His Leu Val Leu His Pro Gln Leu Ala
       130                 135                 140
   Thr Gly Asn Phe Ile Ile Lys Ala Val Trp Leu Pro Gly Ser Gln Thr
   145                 150                 155                 160
   Glu Leu Ala Ile Val Thr Ala Asp Phe Val Lys Ile Tyr Asp Leu Ser
                   165                 170                 175
   Val Asp Ala Leu Ser Pro Thr Phe Tyr Phe Leu Leu Pro Ser Ser Lys
               180                 185                 190
   Ile Arg Asp Val Thr Phe Leu Phe Asn Glu Glu Gly Lys Asn Ile Ile
           195                 200                 205
   Val Ile Met Ser Ser Ala Gly Tyr Met Tyr Thr Gln Leu Met Glu Glu
       210                 215                 220
   Ala Ser Ser Ala Gln Gln Gly Pro Phe Tyr Val Thr Asn Val Leu Glu
   225                 230                 235                 240
   Ile Asn His Glu Asp Leu Lys Asp Ser Asn Ser Gln Val Ala Gly Gly
                   245                 250                 255
   Gly Val Ser Val Tyr Tyr Ser His Val Leu Gln Met Leu Phe Phe Ser
               260                 265                 270
   Tyr Ser Gln Gly Lys Ser Phe Ala Ala Thr Val Ser Arg Ser Thr Leu
           275                 280                 285
   Glu Val Leu Gln Leu Phe Pro Ile Asn Ile Lys Ser Ser Asn Gly Gly
       290                 295                 300
   Ser Lys Thr Ser Pro Ala Leu Cys Gln Trp Ser Glu Val Met Asn His
   305                 310                 315                 320
   Pro Gly Leu Val Cys Cys Val Gln Gln Thr Thr Gly Val Pro Leu Val
                   325                 330                 335
   Val Met Val Lys Pro Asp Thr Phe Leu Ile Gln Glu Ile Lys Thr Leu
               340                 345                 350
   Pro Ala Lys Ala Lys Ile Gln Asp Met Val Ala Ile Arg His Thr Ala
           355                 360                 365
   Cys Asn Glu Gln Gln Arg Thr Thr Met Ile Leu Leu Cys Glu Asp Gly
       370                 375                 380
   Ser Leu Arg Ile Tyr Met Ala Asn Val Glu Asn Thr Ser Tyr Trp Leu
   385                 390                 395                 400
   Gln Pro Ser Leu Gln Pro Ser Ser Val Ile Ser Ile Met Lys Pro Val
                   405                 410                 415
   Arg Lys Arg Lys Thr Ala Thr Ile Thr Ala Arg Thr Ser Ser Gln Val
               420                 425                 430
   Thr Phe Pro Ile Asp Phe Phe Glu His Asn Gln Gln Leu Thr Asp Val
           435                 440                 445
   Glu Phe Gly Gly Asn Asp Leu Leu Gln Val Tyr Asn Ala Gln Gln Ile
       450                 455                 460
   Lys His Arg Leu Asn Ser Thr Gly Met Tyr Val Ala Asn Thr Lys Pro
   465                 470                 475                 480
   Gly Gly Phe Thr Met Glu Ile Ser Asn Asn Ser Ser Thr Met Val Met
   485 490 495
   Thr Gly Met Arg Ile Gln Ile Gly Thr Gln Ala Ile Glu Arg Ala Pro
   500 505 510
   Ser Tyr Ile Glu Ile Phe Gly Arg Thr Met Gln Leu Asn Leu Ser Arg
   515 520 525
   Ser Arg Trp Phe Asp Phe Pro Phe Thr Arg Glu Glu Ala Leu Gln Ala
   530 535 540
   Asp Arg Lys Leu Asn Leu Phe Ile Gly Ala Ser Val Asp Pro Ala Gly
   545 550 555 560
   Val Thr Met Ile Asp Ala Val Lys Ile Tyr Gly Lys Thr Lys Glu Gln
   565 570 575
   Phe Gly Trp Pro Asp Glu Pro Pro Glu Asp Phe Pro Ser Ala Ser Val
   580 585 590
   Ser Ser Val Cys Pro Pro Asn Leu Asn Gln Ser Asn Gly Thr Gly Asp
   595 600 605
   Ser Asp Ser Ala Ala Pro Ala Thr Thr Ser Gly Thr Val Leu Glu Arg
   610 615 620
   Leu Val Val Ser Ser Leu Glu Ala Leu Glu Ser Cys Phe Ala Val Gly
   625 630 635 640
   Pro Ile Ile Glu Lys Glu Arg Asn Lys His Ala Ala Gln Glu Leu Ala
   645 650 655
   Thr Leu Leu Leu Ser Leu Pro Ala Pro Ala Ser Val Gln Gln Gln Ser
   660 665 670
   Lys Ser Leu Leu Ala Ser Leu His Ser Ser Arg Ser Ala Tyr His Ser
   675 680 685
   His Lys Asp Gln Ala Leu Leu Ser Lys Ala Val Gln Cys Leu Asn Thr
   690 695 700
   Ser Ser Lys Glu Gly Lys Asp Leu Asp Pro Glu Val Phe Gln Arg Leu
   705 710 715 720
   Val Ile Thr Ala Arg Ser Ile Ala Val Thr Arg Pro Asn Asn Leu Val
   725 730 735
   His Phe Thr Glu Ser Lys Leu Pro Gln Met Glu Thr Glu Gly Ala Glu
   740 745 750
   Glu Gly Lys Glu Pro Gln Lys Gln Val Glu Gly Asp Gly Cys Ser Phe
   755 760 765
   Ile Thr Gln Leu Val Asn His Phe Trp Lys Leu His Ala Ser Lys Pro
   770 775 780
   Lys Asn Ala Phe Leu Ala Pro Ala Cys Leu Pro Gly Leu Thr His Ile
   785 790 795 800
   Glu Ala Thr Val Asn Ala Leu Val Asp Ile Ile His Gly Tyr Cys Thr
   805 810 815
   Cys Glu Leu Asp Cys Ile Asn Thr Ala Ser Lys Ile Tyr Met Gln Met
               820                 825                 830
   Leu Leu Cys Pro Asp Pro Ala Val Ser Phe Ser Cys Lys Gln Ala Leu
           835                 840                 845
   Ile Arg Val Leu Arg Pro Arg Asn Lys Arg Arg His Val Thr Leu Pro
       850                 855                 860
   Ser Ser Pro Arg Ser Asn Thr Pro Met Gly Asp Lys Asp Asp Asp Asp
   865                 870                 875                 880
   Asp Asp Asp Ala Asp Glu Lys Met Gln Ser Ser Gly Ile Pro Asp Gly
                   885                 890                 895
   Gly His Ile Arg Gln Glu Ser Gln Glu Gln Ser Glu Val Asp His Gly
               900                 905                 910
   Asp Phe Glu Met Val Ser Glu Ser Met Val Leu Glu Thr Ala Glu Asn
           915                 920                 925
   Val Asn Asn Gly Asn Pro Ser Pro Arg Lys Ala Leu Leu Ala Gly Ala
       930                 935                 940
   Glu Gly Phe Pro Pro Met Leu Asp Ile Pro Pro Asp Ala Asp Asp Glu
   945                 950                 955                 960
   Thr Met Val Glu Leu Ala Ile Ala Leu Ser Leu Gln Gln Asp Gln Gln
                   965                 970                 975
   Gly Ser Ser Ser Ser Ala Leu Gly Leu Gln Ser Leu Gly Leu Ser Gly
               980                 985                 990
   Gln Ala Pro Ser Ser Ser Ser Leu Asp Ala Gly Thr Leu Ser Asp Thr
           995                 1000                1005
   Thr Ala Ser Ala Pro Ala Ser Asp Asp Glu Gly Ser Thr Ala Ala Thr
      1010                 1015                1020
   Asp Gly Ser Thr Leu Arg Thr Ser Pro Ala Asp His Gly Gly Ser Val
   1025                1030                1035                1040
   Gly Ser Glu Ser Gly Gly Ser Ala Val Asp Ser Val Ala Gly Glu His
                   1045                1050                1055
   Ser Val Ser Gly Arg Ser Ser Ala Tyr Gly Asp Ala Thr Ala Glu Gly
               1060                1065                1070
   His Pro Ala Gly Pro Gly Ser Val Ser Ser Ser Thr Gly Ala Ile Ser
          1075                 1080                1085
   Thr Thr Thr Gly His Gln Glu Gly Asp Gly Ser Glu Gly Glu Gly Glu
      1090                 1095                1100
   Gly Glu Ala Glu Gly Asp Val His Thr Ser Asn Arg Leu His Met Val
   1105                1110                1115                1120
   Arg Leu Met Leu Leu Glu Arg Leu Leu Gln Thr Leu Pro Gln Leu Arg
                   1125                1130                1135
   Asn Val Gly Gly Val Arg Ala Ile Pro Tyr Met Gln Val Ile Leu Met
   1140 1145 1150
   Leu Thr Thr Asp Leu Asp Gly Glu Asp Glu Lys Asp Lys Gly Ala Leu
   1155 1160 1165
   Asp Asn Leu Leu Ala Gln Leu Ile Ala Glu Leu Gly Met Asp Lys Lys
   1170 1175 1180
   Asp Val Ser Lys Lys Asn Glu Arg Ser Ala Leu Asn Glu Val His Leu
   1185 1190 1195 1200
   Val Val Met Arg Leu Leu Ser Val Phe Met Ser Arg Thr Lys Ser Gly
   1205 1210 1215
   Ser Lys Ser Ser Ile Cys Glu Ser Ser Ser Leu Ile Ser Ser Ala Thr
   1220 1225 1230
   Ala Ala Ala Leu Leu Ser Ser Gly Ala Val Asp Tyr Cys Leu His Val
   1235 1240 1245
   Leu Lys Ser Leu Leu Glu Tyr Trp Lys Gly Gln Gln Ser Glu Glu Glu
   1250 1255 1260
   Pro Val Thr Thr Ser Gln Leu Leu Lys Pro His Thr Thr Ser Ser Pro
   1265 1270 1275 1280
   Pro Asp Met Ser Pro Phe Phe Leu Arg Gln Tyr Val Lys Gly His Ala
   1285 1290 1295
   Ala Asp Val Phe Glu Ala Tyr Thr Gln Leu Leu Thr Glu Met Val Leu
   1300 1305 1310
   Arg Leu Pro Tyr Gln Ile Lys Lys Ile Ala Asp Thr Ser Ser Arg Ile
   1315 1320 1325
   Pro Pro Pro Val Phe Asp His Ser Trp Phe Tyr Phe Leu Ser Glu Tyr
   1330 1335 1340
   Leu Met Ile Gln Gln Thr Pro Phe Val Arg Arg Gln Val Arg Lys Leu
   1345 1350 1355 1360
   Leu Leu Phe Ile Cys Gly Ser Lys Glu Lys Tyr Arg Gln Leu Arg Asp
   1365 1370 1375
   Leu His Thr Leu Asp Ser His Val Arg Gly Ile Lys Lys Leu Leu Glu
   1380 1385 1390
   Glu Gln Gly Ile Phe Leu Arg Ala Ser Val Val Thr Ala Ser Ser Gly
   1395 1400 1405
   Ser Ala Leu Gln Tyr Asp Thr Leu Ile Ser Leu Met Glu His Leu Lys
   1410 1415 1420
   Ala Cys Ala Glu Ile Ala Ala Gln Arg Thr Ile Asn Trp Gln Lys Phe
   1425 1430 1435 1440
   Cys Ile Lys Asp Asp Ser Val Leu Tyr Phe Leu Leu Gln Val Ser Phe
   1445 1450 1455
   Leu Val Asp Glu Gly Val Ser Pro Val Leu Leu Gln Leu Leu Ser Cys
   1460 1465 1470
   Ala Leu Cys Gly Ser Lys Val Leu Ala Ala Leu Ala Ala Ser Thr Gly
   1475 1480 1485
   Ser Ser Ser Val Ala Ser Ser Ala Pro Pro Ala Ala Ser Ser Gly Gln
   1490 1495 1500
   Thr Thr Thr Gln Ser Lys Ser Ser Thr Lys Lys Ser Lys Lys Glu Glu
   1505 1510 1515 1520
   Lys Glu Lys Glu Lys Glu Gly Glu Ser Ser Gly Ser Gln Glu Asp Gln
   1525 1530 1535
   Leu Cys Thr Ala Leu Val Asn Gln Leu Asn Arg Phe Ala Asp Lys Glu
   1540 1545 1550
   Thr Leu Ile Gln Phe Leu Arg Cys Phe Leu Leu Glu Ser Asn Ser Ser
   1555 1560 1565
   Ser Val Arg Trp Gln Ala His Cys Leu Thr Leu His Ile Tyr Arg Asn
   1570 1575 1580
   Ser Asn Lys Ala Gln Gln Glu Leu Leu Leu Asp Leu Met Trp Ser Ile
   1585 1590 1595 1600
   Trp Pro Glu Leu Pro Ala Tyr Gly Arg Lys Ala Ala Gln Phe Val Asp
   1605 1610 1615
   Leu Leu Gly Tyr Phe Ser Leu Lys Thr Ala Gln Thr Glu Lys Lys Leu
   1620 1625 1630
   Lys Glu Tyr Ser Gln Lys Ala Val Glu Ile Leu Arg Thr Gln Asn His
   1635 1640 1645
   Ile Leu Thr Asn His Pro Asn Ser Asn Ile Tyr Asn Thr Leu Ser Gly
   1650 1655 1660
   Leu Val Glu Phe Asp Gly Tyr Tyr Leu Glu Ser Asp Pro Cys Leu Val
   1665 1670 1675 1680
   Cys Asn Asn Pro Glu Val Pro Phe Cys Tyr Ile Lys Leu Ser Ser Ile
   1685 1690 1695
   Lys Val Asp Thr Arg Tyr Thr Thr Thr Gln Gln Val Val Lys Leu Ile
   1700 1705 1710
   Gly Ser His Thr Ile Ser Lys Val Thr Val Lys Ile Gly Asp Leu Lys
   1715 1720 1725
   Arg Thr Lys Met Val Arg Thr Ile Asn Leu Tyr Tyr Asn Asn Arg Thr
   1730 1735 1740
   Val Gln Ala Ile Val Glu Leu Lys Asn Lys Pro Ala Arg Trp His Lys
   1745 1750 1755 1760
   Ala Lys Lys Val Gln Leu Thr Pro Gly Gln Thr Glu Val Lys Ile Asp
   1765 1770 1775
   Leu Pro Leu Pro Ile Val Ala Ser Asn Leu Met Ile Glu Phe Ala Asp
   1780 1785 1790
   Phe Tyr Glu Asn Tyr Gln Ala Ser Thr Glu Thr Leu Gln Cys Pro Arg
   1795 1800 1805
   Cys Ser Ala Ser Val Pro Ala Asn Pro Gly Val Cys Gly Asn Cys Gly
   1810 1815 1820
   Glu Asn Val Tyr Gln Cys His Lys Cys Arg Ser Ile Asn Tyr Asp Glu
   1825 1830 1835 1840
   Lys Asp Pro Phe Leu Cys Asn Ala Cys Gly Phe Cys Lys Tyr Ala Arg
   1845 1850 1855
   Phe Asp Phe Met Leu Tyr Ala Lys Pro Cys Cys Pro Val Asp Pro Ile
   1860 1865 1870
   Glu Asn Glu Glu Asp Arg Lys Lys Ala Val Ser Asn Ile Asn Thr Leu
   1875 1880 1885
   Leu Asp Lys Ala Asp Arg Val Tyr His Gln Leu Met Gly His Arg Pro
   1890 1895 1900
   Gln Leu Glu Asn Leu Leu Cys Lys Val Asn Glu Ala Ala Pro Glu Lys
   1905 1910 1915 1920
   Pro Gln Glu Asp Ser Gly Thr Ala Gly Gly Ile Ser Ser Thr Ser Ala
   1925 1930 1935
   Ser Val Asn Arg Tyr Ile Leu Gln Leu Ala Gln Glu Tyr Cys Gly Asp
   1940 1945 1950
   Cys Lys Asn Ser Phe Asp Glu Leu Ser Lys Ile Ile Gln Lys Val Phe
   1955 1960 1965
   Ala Ser Arg Lys Glu Leu Leu Glu Tyr Asp Leu Gln Gln Arg Glu Ala
   1970 1975 1980
   Ala Thr Lys Ser Ser Arg Thr Ser Val Gln Pro Thr Phe Thr Ala Ser
   1985 1990 1995 2000
   Gln Tyr Arg Ala Leu Ser Val Leu Gly Cys Gly His Thr Ser Ser Thr
   2005 2010 2015
   Lys Cys Tyr Gly Cys Ala Ser Ala Val Thr Glu His Cys Ile Thr Leu
   2020 2025 2030
   Leu Arg Ala Leu Ala Thr Asn Pro Ala Leu Arg His Ile Leu Val Ser
   2035 2040 2045
   Gln Gly Leu Ile Arg Glu Leu Phe Asp Tyr Asn Leu Arg Arg Gly Ala
   2050 2055 2060
   Ala Ala Ile Arg Glu Glu Val Arg Gln Leu Met Cys Leu Leu Thr Arg
   2065 2070 2075 2080
   Asp Asn Pro Glu Ala Thr Gln Gln Met Asn Asp Leu Ile Ile Gly Lys
   2085 2090 2095
   Val Ser Thr Ala Leu Lys Gly His Trp Ala Asn Pro Asp Leu Ala Ser
   2100 2105 2110
   Ser Leu Gln Tyr Glu Met Leu Leu Leu Thr Asp Ser Ile Ser Lys Glu
   2115 2120 2125
   Asp Ser Cys Trp Glu Leu Arg Leu Arg Cys Ala Leu Ser Leu Phe Leu
   2130 2135 2140
   Met Ala Val Asn Ile Lys Thr Pro Val Val Val Glu Asn Ile Thr Leu
   2145 2150 2155 2160
   Met Cys Leu Arg Ile Leu Gln Lys Leu Ile Lys Pro Pro Ala Pro Thr
   2165 2170 2175
   Ser Lys Lys Asn Lys Asp Val Pro Val Glu Ala Leu Thr Thr Val Lys
   2180 2185 2190
   Pro Tyr Cys Asn Glu Ile His Ala Gln Ala Gln Leu Trp Leu Lys Arg
   2195 2200 2205
   Asp Pro Lys Ala Ser Tyr Glu Ala Trp Lys Lys Cys Leu Pro Ile Arg
   2210 2215 2220
   Gly Val Asp Gly Asn Gly Lys Ser Pro Ser Lys Ser Glu Leu His Arg
   2225 2230 2235 2240
   Leu Tyr Leu Thr Glu Lys Tyr Val Trp Arg Trp Lys Gln Phe Leu Ser
   2245 2250 2255
   Arg Arg Gly Lys Arg Thr Thr Pro Leu Asp Leu Lys Leu Gly His Asn
   2260 2265 2270
   Asn Trp Leu Arg Gln Val Leu Phe Thr Pro Ala Thr Gln Ala Ala Arg
   2275 2280 2285
   Gln Ala Ala Cys Thr Ile Val Glu Ala Leu Ala Thr Val Pro Ser Arg
   2290 2295 2300
   Lys Gln Gln Val Leu Asp Leu Leu Thr Ser Tyr Leu Asp Glu Leu Ser
   2305 2310 2315 2320
   Val Ala Gly Glu Cys Ala Ala Glu Tyr Leu Ala Leu Tyr Gln Lys Leu
   2325 2330 2335
   Ile Ala Ser Cys His Trp Lys Val Tyr Leu Ala Ala Arg Gly Val Leu
   2340 2345 2350
   Pro Tyr Val Gly Asn Leu Ile Thr Lys Glu Ile Ala Arg Leu Leu Ala
   2355 2360 2365
   Leu Glu Glu Ala Thr Leu Ser Thr Asp Leu Gln Gln Gly Tyr Ala Leu
   2370 2375 2380
   Lys Ser Leu Thr Gly Leu Leu Ser Ser Phe Val Glu Val Glu Ser Ile
   2385 2390 2395 2400
   Lys Arg His Phe Lys Ser Arg Leu Val Gly Thr Val Leu Asn Gly Tyr
   2405 2410 2415
   Leu Cys Leu Arg Lys Leu Val Leu Gln Arg Thr Lys Leu Ile Asp Glu
   2420 2425 2430
   Thr Gln Asp Met Leu Leu Glu Met Leu Glu Asp Met Thr Thr Gly Thr
   2435 2440 2445
   Glu Ser Glu Thr Lys Ala Phe Met Ala Val Cys Ile Glu Thr Ala Lys
   2450 2455 2460
   Arg Tyr Asn Leu Asp Asp Tyr Arg Thr Pro Val Phe Ile Phe Glu Arg
   2465 2470 2475 2480
   Leu Cys Ser Ile Ile Tyr Pro Glu Glu Asn Glu Val Thr Glu Phe Phe
   2485 2490 2495
   Val Thr Leu Glu Lys Asp Pro Gln Gln Glu Asp Phe Leu Gln Gly Arg
   2500 2505 2510
   Met Pro Gly Asn Pro Tyr Ser Ser Asn Glu Pro Gly Ile Gly Pro Leu
   2515 2520 2525
   Met Arg Asp Ile Lys Asn Lys Ile Cys Gln Asp Cys Asp Leu Val Ala
   2530 2535 2540
   Leu Leu Glu Asp Asp Ser Gly Met Glu Leu Leu Val Asn Asn Lys Ile
   2545 2550 2555 2560
   Ile Ser Leu Asp Leu Pro Val Ala Glu Val Tyr Lys Lys Val Trp Cys
   2565 2570 2575
   Thr Thr Asn Glu Gly Glu Pro Met Arg Ile Val Tyr Arg Met Arg Gly
   2580 2585 2590
   Leu Leu Gly Asp Ala Thr Glu Glu Phe Ile Glu Ser Leu Asp Ser Thr
   2595 2600 2605
   Thr Asp Glu Glu Glu Asp Glu Glu Glu Val Tyr Arg Met Ala Gly Val
   2610 2615 2620
   Met Ala Gln Cys Gly Gly Leu Gln Cys Met Leu Asn Arg Leu Ala Gly
   2625 2630 2635 2640
   Val Lys Asp Phe Lys Gln Gly Arg His Leu Leu Thr Val Leu Leu Lys
   2645 2650 2655
   Leu Phe Ser Tyr Cys Val Lys Val Lys Val Asn Arg Gln Gln Leu Val
   2660 2665 2670
   Lys Leu Glu Met Asn Thr Leu Asn Val Met Leu Gly Thr Leu Asn Leu
   2675 2680 2685
   Ala Leu Val Ala Glu Gln Glu Ser Lys Asp Ser Gly Gly Ala Ala Val
   2690 2695 2700
   Ala Glu Gln Val Leu Ser Ile Met Glu Ile Ile Leu Asp Glu Ser Asn
   2705 2710 2715 2720
   Ala Glu Pro Leu Ser Glu Asp Lys Gly Asn Leu Leu Leu Thr Gly Asp
   2725 2730 2735
   Lys Asp Gln Leu Val Met Leu Leu Asp Gln Ile Asn Ser Thr Phe Val
   2740 2745 2750
   Arg Ser Asn Pro Ser Val Leu Gln Gly Leu Leu Arg Ile Ile Pro Tyr
   2755 2760 2765
   Leu Ser Phe Gly Glu Val Glu Lys Met Gln Ile Leu Val Glu Arg Phe
   2770 2775 2780
   Lys Pro Tyr Cys Ser Phe Asp Lys Tyr Asp Glu Asp His Ser Gly Asp
   2785 2790 2795 2800
   Asp Lys Val Phe Leu Asp Cys Phe Cys Lys Ile Ala Ala Gly Ile Lys
   2805 2810 2815
   Asn Asn Ser Asn Gly His Gln Leu Lys Asp Leu Ile Leu Gln Lys Gly
   2820 2825 2830
   Ile Thr Gln Ser Ala Leu Asp Tyr Met Lys Lys His Ile Pro Ser Ala
   2835 2840 2845
   Lys Asn Leu Asp Ala Asp Ile Trp Lys Lys Phe Leu Ser Arg Pro Ala
   2850 2855 2860
   Leu Pro Phe Ile Leu Arg Leu Leu Arg Gly Leu Ala Met Gln His Pro
   2865 2870 2875 2880
   Ala Thr Gln Val Leu Ile Gly Thr Asp Ser Ile Thr Ser Leu His Lys
   2885 2890 2895
   Leu Glu Gln Val Ser Ser Asp Glu Gly Ile Gly Thr Leu Ala Glu Asn
   2900 2905 2910
   Leu Leu Glu Ala Leu Arg Glu His Pro Asp Val Asn Lys Lys Ile Asp
   2915 2920 2925
   Ala Ala Arg Arg Glu Thr Arg Ala Glu Lys Lys Arg Met Ala Met Ala
   2930 2935 2940
   Met Arg Gln Lys Ala Leu Gly Thr Leu Gly Met Thr Thr Asn Glu Lys
   2945 2950 2955 2960
   Gly Gln Val Val Thr Lys Thr Ala Leu Leu Lys Gln Met Glu Glu Leu
   2965 2970 2975
   Ile Glu Glu Pro Gly Leu Thr Cys Cys Ile Cys Arg Glu Gly Gly Thr
   2980 2985 2990
   Lys Phe Gln Pro Thr Lys Val Leu Gly Ile Tyr Thr Phe Thr Lys Arg
   2995 3000 3005
   Val Ala Leu Glu Glu Met Glu Asn Lys Pro Arg Lys Gln Gln Gly Tyr
   3010 3015 3020
   Ser Thr Val Ser His Phe Asn Ile Val His Tyr Asp Cys His Leu Ala
   3025 3030 3035 3040
   Ala Val Arg Leu Ala Arg Gly Arg Glu Glu Trp Glu Ser Ala Ala Leu
   3045 3050 3055
   Gln Asn Ala Asn Thr Lys Cys Asn Gly Leu Leu Pro Val Trp Gly Pro
   3060 3065 3070
   His Val Pro Glu Ser Ala Phe Ala Thr Cys Leu Ala Arg His Asn Thr
   3075 3080 3085
   Tyr Leu Gln Glu Cys Thr Gly Gln Arg Glu Pro Thr Tyr Gln Leu Asn
      3090                3095                3100
   Ile His Asp Ile Lys Leu Leu Phe Leu Arg Phe Ala Met Glu Gln Ser
   3105               3110                3115                3120
   Phe Ser Ala Asp Thr Gly Gly Gly Gly Arg Glu Ser Asn Ile His Leu
                  3125                3130                3135
   Ile Pro Tyr Ile Ile His Thr Val Leu Tyr Val Leu Asn Thr Thr Arg
              3140                3145                3150
   Ala Thr Ser Arg Glu Glu Lys Asn Leu Gln Gly Phe Leu Glu Gln Pro
          3155                3160                3165
   Arg Glu Lys Trp Thr Glu Ser Ala Phe Asp Val Asp Gly Pro His Tyr
      3170                3175                3180
   Phe Thr Ile Leu Ala Leu His Val Leu Pro Pro Glu Gln Trp Lys Ala
   3185               3190                3195                3200
   Thr Arg Val Glu Ile Leu Arg Arg Leu Leu Val Ala Ser His Ala Arg
                  3205                3210                3215
   Ala Val Ala Pro Gly Gly Ala Thr Arg Leu Thr Asp Lys Ala Val Lys
              3220                3225                3230
   Asp Tyr Ser Ala Tyr Arg Ser Ser Leu Leu Phe Trp Ala Leu Val Asp
          3235                3240                3245
   Leu Ile Tyr Asn Met Phe Lys Lys Val Pro Thr Ser Asn Thr
      3250                3255                3260
<210> 46
   <211> 2276
   <212> PRT
   <213> homo sapiens
<220>
   <222> (1)..(2276)
   <223> KIAA0462 protein
<300>
   <301> Andersson, et al. Vogel, et al. Lbeit, et al. Ohara, 0
   <302> Unknown protein [Homo sapiens].
   <308> GI 3005744
   <309> 1998-04-02
   <313> 1 TO 494
<400> 46
   Glu His Ser Val Ser Gly Arg Ser Ser Ala Tyr Gly Asp Ala Thr Ala
   1               5                   10                  15
   Glu Gly His Pro Ala Gly Pro Gly Ser Val Ser Ser Ser Thr Gly Ala
               20                  25                  30
   Ile Ser Thr Thr Thr Gly His Gln Glu Gly Asp Gly Ser Glu Gly Glu
           35                  40                  45
   Gly Glu Gly Glu Thr Glu Gly Asp Val His Thr Ser Asn Arg Leu His
       50                  55                  60
   Met Val Arg Leu Met Leu Leu Glu Arg Leu Leu Gln Thr Leu Pro Gln
   65                  70                  75                  80
   Leu Arg Asn Val Gly Gly Val Arg Ala Ile Pro Tyr Met Gln Val Ile
                   85                  90                  95
   Leu Met Leu Thr Thr Asp Leu Asp Gly Glu Asp Glu Lys Asp Lys Gly
               100                 105                 110
   Ala Leu Asp Asn Leu Leu Ser Gln Leu Ile Ala Glu Leu Gly Met Asp
           115                 120                 125
   Lys Lys Asp Val Ser Lys Lys Asn Glu Arg Ser Ala Leu Asn Glu Val
       130                 135                 140
   His Leu Val Val Met Arg Leu Leu Ser Val Phe Met Ser Arg Thr Lys
   145                 150                 155                 160
   Ser Gly Ser Lys Ser Ser Ile Cys Glu Ser Ser Ser Leu Ile Ser Ser
                   165                 170                 175
   Ala Thr Ala Ala Ala Leu Leu Ser Ser Gly Ala Val Asp Tyr Cys Leu
               180                 185                 190
   His Val Leu Lys Ser Leu Leu Glu Tyr Trp Lys Ser Gln Gln Asn Asp
           195                 200                 205
   Glu Glu Pro Val Ala Thr Ser Gln Leu Leu Lys Pro His Thr Thr Ser
       210                 215                 220
   Ser Pro Pro Asp Met Ser Pro Phe Phe Leu Arg Gln Tyr Val Lys Gly
   225                 230                 235                 240
   His Ala Ala Asp Val Phe Glu Ala Tyr Thr Gln Leu Leu Thr Glu Met
                   245                 250                 255
   Val Leu Arg Leu Pro Tyr Gln Ile Lys Lys Ile Thr Asp Thr Asn Ser
               260                 265                 270
   Arg Ile Pro Pro Pro Val Phe Asp His Ser Trp Phe Tyr Phe Leu Ser
           275                 280                 285
   Glu Tyr Leu Met Ile Gln Gln Thr Pro Phe Val Arg Arg Gln Val Arg
       290                 295                 300
   Lys Leu Leu Leu Phe Ile Cys Gly Ser Lys Glu Lys Tyr Arg Gln Leu
   305                 310                 315                 320
   Arg Asp Leu His Thr Leu Asp Ser His Val Arg Gly Ile Lys Lys Leu
                   325                 330                 335
   Leu Glu Glu Gln Gly Ile Phe Leu Arg Ala Ser Val Val Thr Ala Ser
               340                 345                 350
   Ser Gly Ser Ala Leu Gln Tyr Asp Thr Leu Ile Ser Leu Met Glu His
   355 360 365
   Leu Lys Ala Cys Ala Glu Ile Ala Ala Gln Arg Thr Ile Asn Trp Gln
   370 375 380
   Lys Phe Cys Ile Lys Asp Asp Ser Val Leu Tyr Phe Leu Leu Gln Val
   385 390 395 400
   Ser Phe Leu Val Asp Glu Gly Val Ser Pro Val Leu Leu Gln Leu Leu
   405 410 415
   Ser Cys Ala Leu Cys Gly Ser Lys Val Leu Ala Ala Leu Ala Ala Ser
   420 425 430
   Ser Gly Ser Ser Ser Ala Ser Ser Ser Ser Ala Pro Val Ala Ala Ser
   435 440 445
   Ser Gly Gln Ala Thr Thr Gln Ser Lys Ser Ser Thr Lys Lys Ser Lys
   450 455 460
   Lys Glu Glu Lys Glu Lys Glu Lys Asp Gly Glu Thr Ser Gly Ser Gln
   465 470 475 480
   Glu Asp Gln Leu Cys Thr Ala Leu Val Asn Gln Leu Asn Lys Phe Ala
   485 490 495
   Asp Lys Glu Thr Leu Ile Gln Phe Leu Arg Cys Phe Leu Leu Glu Ser
   500 505 510
   Asn Ser Ser Ser Val Arg Trp Gln Ala His Cys Leu Thr Leu His Ile
   515 520 525
   Tyr Arg Asn Ser Ser Lys Ser Gln Gln Glu Leu Leu Leu Asp Leu Met
   530 535 540
   Trp Ser Ile Trp Pro Glu Leu Pro Ala Tyr Gly Arg Lys Ala Ala Gln
   545 550 555 560
   Phe Val Asp Leu Leu Gly Tyr Phe Ser Leu Lys Thr Pro Gln Thr Glu
   565 570 575
   Lys Lys Leu Lys Glu Tyr Ser Gln Lys Ala Val Glu Ile Leu Arg Thr
   580 585 590
   Gln Asn His Ile Leu Thr Asn His Pro Asn Ser Asn Ile Tyr Asn Thr
   595 600 605
   Leu Ser Gly Leu Val Glu Phe Asp Gly Tyr Tyr Leu Glu Ser Asp Pro
   610 615 620
   Cys Leu Val Cys Asn Asn Pro Glu Val Pro Phe Cys Tyr Ile Lys Leu
   625 630 635 640
   Ser Ser Ile Lys Val Asp Thr Arg Tyr Thr Thr Thr Gln Gln Val Val
   645 650 655
   Lys Leu Ile Gly Ser His Thr Ile Ser Lys Val Thr Val Lys Ile Gly
   660 665 670
   Asp Leu Lys Arg Thr Lys Met Val Arg Thr Ile Asn Leu Tyr Tyr Asn
   675 680 685
   Asn Arg Thr Val Gln Ala Ile Val Glu Leu Lys Asn Lys Pro Ala Arg
   690 695 700
   Trp His Lys Ala Lys Lys Val Gln Leu Thr Pro Gly Gln Thr Glu Val
   705 710 715 720
   Lys Ile Asp Leu Pro Leu Pro Ile Val Ala Ser Asn Leu Met Ile Glu
   725 730 735
   Phe Ala Asp Phe Tyr Glu Asn Tyr Gln Ala Ser Thr Glu Thr Leu Gln
   740 745 750
   Cys Pro Arg Cys Ser Ala Ser Val Pro Ala Asn Pro Gly Val Cys Gly
   755 760 765
   Asn Cys Gly Glu Asn Val Tyr Gln Cys His Lys Cys Arg Ser Ile Asn
   770 775 780
   Tyr Asp Glu Lys Asp Pro Phe Leu Cys Asn Ala Cys Gly Phe Cys Lys
   785 790 795 800
   Tyr Ala Arg Phe Asp Phe Met Leu Tyr Ala Lys Pro Cys Cys Ala Val
   805 810 815
   Asp Pro Ile Glu Asn Glu Glu Asp Arg Lys Lys Ala Val Ser Asn Ile
   820 825 830
   Asn Thr Leu Leu Asp Lys Ala Asp Arg Val Tyr His Gln Leu Met Gly
   835 840 845
   His Arg Pro Gln Leu Glu Asn Leu Leu Cys Lys Val Asn Glu Ala Ala
   850 855 860
   Pro Glu Lys Pro Gln Asp Asp Ser Gly Thr Ala Gly Gly Ile Ser Ser
   865 870 875 880
   Thr Ser Ala Ser Val Asn Arg Tyr Ile Leu Gln Leu Ala Gln Glu Tyr
   885 890 895
   Cys Gly Asp Cys Lys Asn Ser Phe Asp Glu Leu Ser Lys Ile Ile Gln
   900 905 910
   Lys Val Phe Ala Ser Arg Lys Glu Leu Leu Glu Tyr Asp Leu Gln Gln
   915 920 925
   Arg Glu Ala Ala Thr Lys Ser Ser Arg Thr Ser Val Gln Pro Thr Phe
   930 935 940
   Thr Ala Ser Gln Tyr Arg Ala Leu Ser Val Leu Gly Cys Gly His Thr
   945 950 955 960
   Ser Ser Thr Lys Cys Tyr Gly Cys Ala Ser Ala Val Thr Glu His Cys
   965 970 975
   Ile Thr Leu Leu Arg Ala Leu Ala Thr Asn Pro Ala Leu Arg His Ile
   980 985 990
   Leu Val Ser Gln Gly Leu Ile Arg Glu Leu Phe Asp Tyr Asn Leu Arg
   995 1000 1005
   Arg Gly Ala Ala Ala Met Arg Glu Glu Val Arg Gln Leu Met Cys Leu
   1010 1015 1020
   Leu Thr Arg Asp Asn Pro Glu Ala Thr Gln Gln Met Asn Asp Leu Ile
   1025 1030 1035 1040
   Ile Gly Lys Val Ser Thr Ala Leu Lys Gly His Trp Ala Asn Pro Asp
   1045 1050 1055
   Leu Ala Ser Ser Leu Gln Tyr Glu Met Leu Leu Leu Thr Asp Ser Ile
   1060 1065 1070
   Ser Lys Glu Asp Ser Cys Trp Glu Leu Arg Leu Arg Cys Ala Leu Ser
   1075 1080 1085
   Leu Phe Leu Met Ala Val Asn Ile Lys Thr Pro Val Val Val Glu Asn
   1090 1095 1100
   Ile Thr Leu Met Cys Leu Arg Ile Leu Gln Lys Leu Ile Lys Pro Pro
   1105 1110 1115 1120
   Ala Pro Thr Ser Lys Lys Asn Lys Asp Val Pro Val Glu Ala Leu Thr
   1125 1130 1135
   Thr Val Lys Pro Tyr Cys Asn Glu Ile His Ala Gln Ala Gln Leu Trp
   1140 1145 1150
   Leu Lys Arg Asp Pro Lys Ala Ser Tyr Asp Ala Trp Lys Lys Cys Leu
   1155 1160 1165
   Pro Ile Arg Gly Ile Asp Gly Asn Gly Lys Ala Pro Ser Lys Ser Glu
   1170 1175 1180
   Leu Arg His Leu Tyr Leu Thr Glu Lys Tyr Val Trp Arg Trp Lys Gln
   1185 1190 1195 1200
   Phe Leu Ser Arg Arg Gly Lys Arg Thr Ser Pro Leu Asp Leu Lys Leu
   1205 1210 1215
   Gly His Asn Asn Trp Leu Arg Gln Val Leu Phe Thr Pro Ala Thr Gln
   1220 1225 1230
   Ala Ala Arg Gln Ala Ala Cys Thr Ile Val Glu Ala Leu Ala Thr Ile
   1235 1240 1245
   Pro Ser Arg Lys Gln Gln Val Leu Asp Leu Leu Thr Ser Tyr Leu Asp
   1250 1255 1260
   Glu Leu Ser Ile Ala Gly Glu Cys Ala Ala Glu Tyr Leu Ala Leu Tyr
   1265 1270 1275 1280
   Gln Lys Leu Ile Thr Ser Ala His Trp Lys Val Tyr Leu Ala Ala Arg
   1285 1290 1295
   Gly Val Leu Pro Tyr Val Gly Asn Leu Ile Thr Lys Glu Ile Ala Arg
   1300 1305 1310
   Leu Leu Ala Leu Glu Glu Ala Thr Leu Ser Thr Asp Leu Gln Gln Gly
   1315 1320 1325
   Tyr Ala Leu Lys Ser Leu Thr Gly Leu Leu Ser Ser Phe Val Glu Val
   1330 1335 1340
   Glu Ser Ile Lys Arg His Phe Lys Ser Arg Leu Val Gly Thr Val Leu
   1345 1350 1355 1360
   Asn Gly Tyr Leu Cys Leu Arg Lys Leu Val Val Gln Arg Thr Lys Leu
   1365 1370 1375
   Ile Asp Glu Thr Gln Asp Met Leu Leu Glu Met Leu Glu Asp Met Thr
   1380 1385 1390
   Thr Gly Thr Glu Ser Glu Thr Lys Ala Phe Met Ala Val Cys Ile Glu
   1395 1400 1405
   Thr Ala Lys Arg Tyr Asn Leu Asp Asp Tyr Arg Thr Pro Val Phe Ile
   1410 1415 1420
   Phe Glu Arg Leu Cys Ser Ile Ile Tyr Pro Glu Glu Asn Glu Val Thr
   1425 1430 1435 1440
   Glu Phe Phe Val Thr Leu Glu Lys Asp Pro Gln Gln Glu Asp Phe Leu
   1445 1450 1455
   Gln Gly Arg Met Pro Gly Asn Pro Tyr Ser Ser Asn Glu Pro Gly Ile
   1460 1465 1470
   Gly Pro Leu Met Arg Asp Ile Lys Asn Lys Ile Cys Gln Asp Cys Asp
   1475 1480 1485
   Leu Val Ala Leu Leu Glu Asp Asp Ser Gly Met Glu Leu Leu Val Asn
   1490 1495 1500
   Asn Lys Ile Ile Ser Leu Asp Leu Pro Val Ala Glu Val Tyr Lys Lys
   1505 1510 1515 1520
   Val Trp Cys Thr Thr Asn Glu Gly Glu Pro Met Arg Ile Val Tyr Arg
   1525 1530 1535
   Met Arg Gly Leu Leu Gly Asp Ala Thr Glu Glu Phe Ile Glu Ser Leu
   1540 1545 1550
   Asp Ser Thr Thr Asp Glu Glu Glu Asp Glu Glu Glu Val Tyr Lys Met
   1555 1560 1565
   Ala Gly Val Met Ala Gln Cys Gly Gly Leu Glu Cys Met Leu Asn Arg
   1570 1575 1580
   Leu Ala Gly Ile Arg Asp Phe Lys Gln Gly Arg His Leu Leu Thr Val
   1585 1590 1595 1600
   Leu Leu Lys Leu Phe Ser Tyr Cys Val Lys Val Lys Val Asn Arg Gln
   1605 1610 1615
   Gln Leu Val Lys Leu Glu Met Asn Thr Leu Asn Val Met Leu Gly Thr
   1620 1625 1630
   Leu Asn Leu Ala Leu Val Ala Glu Gln Glu Ser Lys Asp Ser Gly Gly
   1635 1640 1645
   Ala Ala Val Ala Glu Gln Val Leu Ser Ile Met Glu Ile Ile Leu Asp
   1650 1655 1660
   Glu Ser Asn Ala Glu Pro Leu Ser Glu Asp Lys Gly Asn Leu Leu Leu
   1665 1670 1675 1680
   Thr Gly Asp Lys Asp Gln Leu Val Met Leu Leu Asp Gln Ile Asn Ser
   1685 1690 1695
   Thr Phe Val Arg Ser Asn Pro Ser Val Leu Gln Gly Leu Leu Arg Ile
   1700 1705 1710
   Ile Pro Tyr Leu Ser Phe Gly Glu Val Glu Lys Met Gln Ile Leu Val
   1715 1720 1725
   Glu Arg Phe Lys Pro Tyr Cys Asn Phe Asp Lys Tyr Asp Glu Asp His
   1730 1735 1740
   Ser Gly Asp Asp Lys Val Phe Leu Asp Cys Phe Cys Lys Ile Ala Ala
   1745 1750 1755 1760
   Gly Ile Lys Asn Asn Ser Asn Gly His Gln Leu Lys Asp Leu Ile Leu
   1765 1770 1775
   Gln Lys Gly Ile Thr Gln Asn Ala Leu Asp Tyr Met Lys Lys His Ile
   1780 1785 1790
   Pro Ser Ala Lys Asn Leu Asp Ala Asp Ile Trp Lys Lys Phe Leu Ser
   1795 1800 1805
   Arg Pro Ala Leu Pro Phe Ile Leu Arg Leu Leu Arg Gly Leu Ala Ile
   1810 1815 1820
   Gln His Pro Gly Thr Gln Val Leu Ile Gly Thr Asp Ser Ile Pro Asn
   1825 1830 1835 1840
   Leu His Lys Leu Glu Gln Val Ser Ser Asp Glu Gly Ile Gly Thr Leu
   1845 1850 1855
   Ala Glu Asn Leu Leu Glu Ala Leu Arg Glu His Pro Asp Val Asn Lys
   1860 1865 1870
   Lys Ile Asp Ala Ala Arg Arg Glu Thr Arg Ala Glu Lys Lys Arg Met
   1875 1880 1885
   Ala Met Ala Met Arg Gln Lys Ala Leu Gly Thr Leu Gly Met Thr Thr
   1890 1895 1900
   Asn Glu Lys Gly Gln Val Val Thr Lys Thr Ala Leu Leu Lys Gln Met
   1905 1910 1915 1920
   Glu Glu Leu Ile Glu Glu Pro Gly Leu Thr Cys Cys Ile Cys Arg Glu
   1925 1930 1935
   Gly Tyr Lys Phe Gln Pro Thr Lys Val Leu Gly Ile Tyr Thr Phe Thr
   1940 1945 1950
   Lys Arg Val Ala Leu Glu Glu Met Glu Asn Lys Pro Arg Lys Gln Gln
   1955 1960 1965
   Gly Tyr Ser Thr Val Ser His Phe Asn Ile Val His Tyr Asp Cys His
   1970 1975 1980
   Leu Ala Ala Val Arg Leu Ala Arg Gly Arg Glu Glu Trp Glu Ser Ala
   1985 1990 1995 2000
   Ala Leu Gln Asn Ala Asn Thr Lys Cys Asn Gly Leu Leu Pro Val Trp
   2005 2010 2015
   Gly Pro His Val Pro Glu Ser Ala Phe Ala Thr Cys Leu Ala Arg His
   2020 2025 2030
   Asn Thr Tyr Leu Gln Glu Cys Thr Gly Gln Arg Glu Pro Thr Tyr Gln
   2035 2040 2045
   Leu Asn Ile His Asp Ile Lys Leu Leu Phe Leu Arg Phe Ala Met Glu
   2050 2055 2060
   Gln Ser Phe Ser Ala Asp Thr Gly Gly Gly Gly Arg Glu Ser Asn Ile
   2065 2070 2075 2080
   His Leu Ile Pro Tyr Ile Ile His Thr Val Leu Tyr Val Leu Asn Thr
   2085 2090 2095
   Thr Arg Ala Thr Ser Arg Glu Glu Lys Asn Leu Gln Gly Phe Leu Glu
   2100 2105 2110
   Gln Pro Lys Glu Lys Trp Val Glu Ser Ala Phe Glu Val Asp Gly Pro
   2115 2120 2125
   Tyr Tyr Phe Thr Val Leu Ala Leu His Ile Leu Pro Pro Glu Gln Trp
   2130 2135 2140
   Arg Ala Thr Arg Val Glu Ile Leu Arg Arg Leu Leu Val Thr Ser Gln
   2145 2150 2155 2160
   Ala Arg Ala Val Ala Pro Gly Gly Ala Thr Arg Leu Thr Asp Lys Ala
   2165 2170 2175
   Val Lys Asp Tyr Ser Ala Tyr Arg Ser Ser Leu Leu Phe Trp Ala Leu
   2180 2185 2190
   Val Asp Leu Ile Tyr Asn Met Phe Lys Lys Val Pro Thr Ser Asn Thr
   2195 2200 2205
   Glu Gly Gly Trp Ser Cys Ser Leu Ala Glu Tyr Ile Arg His Asn Asp
   2210 2215 2220
   Met Pro Ile Tyr Glu Ala Ala Asp Lys Ala Leu Lys Thr Phe Gln Glu
   2225 2230 2235 2240
   Glu Phe Met Pro Val Glu Thr Phe Ser Glu Phe Leu Asp Val Ala Gly
   2245 2250 2255
   Leu Leu Ser Glu Ile Thr Asp Pro Glu Ser Phe Leu Lys Asp Leu Leu
   2260 2265 2270
   Asn Ser Val Pro
   2275
<210> 47
   <211> 5322
   <212> PRT
   <213> Drosophila melanogastor
<400> 47
   Met Ser Ala His Ser Gly Gly Thr Asp Trp Asn Ser Val Val Lys Ala
    1               5                  10                  15
   Leu Ile Leu Asn Arg Thr Gly Ala Leu Asn Lys Asn Glu Val Val Asn
               20                  25                  30
   Leu Leu Lys Ala Ile Thr Arg Cys Glu His Asp Phe Phe Glu Glu Glu
           35                  40                  45
   Ser Asn Phe Thr Gln Phe Tyr Thr Ala Phe Ala Ala Leu Ala Ala Asp
       50                  55                  60
   Lys Leu Met Gln Ile Lys Thr Ile Cys Gln Thr Gln Ile Cys Gln Leu
   65                  70                  75                  80
   His Asp Ala Thr Ala Val Leu Ile Arg Phe Ile Ile Tyr Arg Leu Pro
                   85                  90                  95
   Arg Val Ser Val Tyr Glu Thr Lys Trp Leu Leu Gly Ala Leu Lys Met
               100                 105                 110
   Leu Cys Glu Gly Arg Glu Cys Pro Ala Ser Ala Ser Ser Ser Met Phe
           115                 120                 125
   Asp Tyr Asn Ala Val Ala Asn Val Leu Lys Ser Cys Lys His Pro Glu
       130                 135                 140
   Ser Thr Thr Lys Ser Ile Met Pro Ser Ser Ser Ser Ser Gly Ser Gly
   145                 150                 155                 160
   Ala Ser Asn Asp Lys Glu Ser Pro Lys Ser Glu Ile Lys Arg Ser Arg
                   165                 170                 175
   Ser Asp Leu Ser Ser Val Ile Leu Gln Gln Leu Ile Ala Pro Leu Glu
               180                 185                 190
   Pro Gly Lys Met Thr Trp Val Pro Leu Ser Glu Glu Val Thr Asp Cys
           195                 200                 205
   Thr Glu Gln Ile Leu Ala Ala Asn Val Glu Tyr Phe Gln Glu Gln Asn
       210                 215                 220
   Gly Val Asp Thr Leu Leu Asp Val Cys Val Ser Leu Pro Ile Leu Asn
   225                 230                 235                 240
   Arg Tyr Arg Ser Lys Tyr Met Glu Thr Ile Asn Gly Gly Lys Ser Leu
                   245                 250                 255
   Tyr Leu Pro Leu Thr Gln Val Glu Ala Thr Ala Val Lys Ser Ser Met
               260                 265                 270
   Asn His Met Leu Thr Asp Leu Thr Ile Leu Ser Gln Ala Gln Ala Leu
           275                 280                 285
   Ile Glu Met Gln Pro Leu Thr Pro Ser Arg Ile Glu Arg Leu Ser Met
       290                 295                 300
   Cys Gly Ile Ala Ala Leu Tyr Asn Ala Val Leu Thr Ser Ile Ala Thr
   305                 310                 315                 320
   Ser Val Leu Gly Met Ser Gln Ala Ser Ser Ser Gln Lys Gln Thr Ala
                   325                 330                 335
   Ser Thr Ser Gln Gly Ser Gly Val Gly Gly Ser Ser Gly Gly Gln Ser
               340                 345                 350
   Asn Lys Asp His Asp Asp Phe Glu Asp Gln Ala Cys Ser Ile Val Asn
           355                 360                 365
   Lys Ala Leu Glu Ile Tyr Ser Asn Ile Gly His Met Phe Lys Thr Ser
       370                 375                 380
   Ala Arg Ile His Val Tyr Gln Asn His Leu Cys Tyr Gly Ser Trp Leu
   385                 390                 395                 400
   Leu Ile Ser Gly Ile Gln Gly Ala Met Gly Ala Ser Gly Ser Gly Gly
                   405                 410                 415
   Ser Ser Ser Asp Ser Ala Ser Lys Ser Ala Ser Lys Ala Thr Lys Ser
               420                 425                 430
   Gly Ser Glu Ala Gly Thr Ala Pro Thr Thr Pro Ile Ala Arg Val Asn
           435                 440                 445
   Leu Phe Lys Val Gln Gln Gly Phe Gly Glu Leu Asn Ala Ala Ile Ala
       450                 455                 460
   Asn His Ser Ile Lys Leu Leu Ser Glu Leu Ile Glu Asp Leu Lys Val
   465                 470                 475                 480
   Glu Ala Ala Cys Gly Gln Ser Leu Glu Ser Thr Glu Leu Pro Glu Pro
                   485                 490                 495
   Ala Gln Phe Asp Ile Leu Gln Asn Tyr Ser Ser Leu Glu Arg Ile Val
               500                 505                 510
   Arg Val Leu Asn Thr Ala Thr Leu His Gln Leu Phe Thr Phe Leu Ala
           515                 520                 525
   Thr Val Ala Tyr Arg Lys Ala Cys Thr Leu Lys Arg Ala Ser Ala Lys
       530                 535                 540
   Asp Arg Thr Glu Cys Glu Pro Ile Ser Tyr Ser Asp Ser Thr Thr Tyr
   545                 550                 555                 560
   Phe Asn Asp Ser Leu Ser Cys Ser Asp Asn Ser Glu Glu Asp Asp Ser
                   565                 570                 575
   Glu Ser Tyr Leu Gly His Trp Phe Lys Glu Thr Leu Ser Pro Glu Thr
               580                 585                 590
   His Asp Asp Asn Ala Asn Thr Ser Thr Gln Glu Arg Ala Glu Gln Lys
           595                 600                 605
   Ser Ala Leu Val Pro Lys Leu Asp Glu Pro His Glu Tyr Leu Asp Leu
       610                 615                 620
   Ala Ala Asp Ile Phe Cys Phe Leu Asp Gln Phe Leu Ala Asn Arg His
   625                 630                 635                 640
   Ala Tyr Met Gln Arg Tyr Val Lys Ala Gly Val Ser Asp Gln Gln Met
                   645                 650                 655
   Leu Leu Met Ala Asn Ile Ile Lys Asp Phe Asp Arg Asp Val Met Arg
               660                 665                 670
   Asn Glu Thr Asp Gln Gly Ser Gly Asn Ala Pro Ala Ala Ser Ala Gly
           675                 680                 685
   Ala Gly Thr Ser Ala Gly Ala Ser Thr Lys Trp Gln Thr Ser Met Ile
       690                 695                 700
   Arg Phe Ser Gly Ala Ala Gly Arg Tyr Ile His Asn Leu Ile Ser Thr
   705                 710                 715                 720
   Ser Leu Leu Ser Glu Gln Leu Gln Ser Asn Leu Leu Gln His Leu Ser
                   725                 730                 735
   Ile Ser Pro Trp Ser Thr Asp Thr Asn Thr Trp Pro Leu Gln Val Tyr
               740                 745                 750
   Pro Ser Thr Leu Ser Val Leu Val Gln Ile Leu Leu Leu Lys Pro Thr
           755                 760                 765
   Gln Glu Lys Glu Ala Ala Cys Leu Ser Val Trp His Arg Leu Ile Asn
       770                 775                 780
   Thr Leu Val Glu Gly Val Cys Ser Ser Asn Thr Ala Ser Asp Ser Asp
   785                 790                 795                 800
   Tyr Glu Asp Leu Asn Ile Glu His Ala Gln Leu Leu Leu Phe Leu Phe
                   805                 810                 815
   His Ser Leu Asn Leu Met Gln Lys Lys Ser Ile Leu Leu Leu Thr Ala
               820                 825                 830
   Gly Gly Val Ile Arg Cys Ala Glu Val Cys Arg Gly Ile Ser Glu Asp
           835                 840                 845
   Arg Pro Val Lys Asn Ser Gln Ile Met Leu Leu Ser Arg Leu Leu Leu
       850                 855                 860
   Phe Leu Glu Tyr Leu Met Lys His Leu Tyr Asn Ala Pro Pro Glu Leu
   865                 870                 875                 880
   Leu Asp Gln Val Arg Trp Asn Leu Phe Ser Val Ser Ser Met Pro Asp
                   885                 890                 895
   Thr Gln Lys Ile Thr Asp Leu Leu Asn Cys Arg Thr Lys Leu Asn Ser
               900                 905                 910
   Tyr Cys Arg His Asp Ile Glu Glu Asn Phe Arg Lys Ser Ala Gly Glu
           915                 920                 925
   Tyr Gly Ser Ser Ile Arg Pro Thr Phe Tyr Ser Leu Val Met Gly Asp
       930                 935                 940
   Pro Glu Ile Ser Tyr Trp Ala Gln Glu Phe Lys Leu Asp Gly Leu Ala
   945                 950                 955                 960
   Trp Asn Phe Ile Leu Cys Thr Pro Asp Lys Leu Lys Tyr Pro Leu Leu
   965 970 975
   Val Asp Ala Leu Thr Asp Ile Leu Ser Ile Thr Asp Met Ser Met Tyr
   980 985 990
   Ser Lys Glu Lys Asp Lys Glu Ala Ser Met His Asn Leu Cys Ala Ile
   995 1000 1005
   Gln Tyr Cys Phe Thr Ile Ala Trp Lys Leu Asn Leu Gly Leu Pro Pro
   1010 1015 1020
   Ser Thr Ser His Val Glu Ser Leu Lys Ala Glu Arg Ser Pro Asn Leu
   1025 1030 1035 1040
   His Ser Leu Met Trp Ser Ile Arg Leu Pro Leu Ala Ser Ser His Tyr
   1045 1050 1055
   Leu Val Val Ser Ser Leu Ile Lys Gln Gly Met Tyr Thr Gln Tyr Ala
   1060 1065 1070
   Glu Thr Leu Trp Thr His Val Gly Asp Ile Gly Ala Asp Ile Lys Tyr
   1075 1080 1085
   Ser Leu Lys Gln Thr Ile Leu Gly Val Glu Ala Phe Asn Ser Gln Met
   1090 1095 1100
   Asn Gly Asn Gly Thr Pro Arg Leu Ser Asp Leu Ile Leu Phe Asp Ser
   1105 1110 1115 1120
   Leu Val Ala His Met Gln Ala Val Ala Trp Ala Asn Lys Glu Gly Leu
   1125 1130 1135
   Lys Trp Pro Arg Lys Glu Ser Glu Asp Ala Ala Gly Glu Gln Ser Ala
   1140 1145 1150
   Gly Thr Ser Leu Thr Ser Ser Ser Asn Asp Pro Glu Leu Tyr Ser Ser
   1155 1160 1165
   Asn Glu Ser Ile Asp Asp Gln Lys Leu Lys Gln Asp Asp Asp Gly Lys
   1170 1175 1180
   Leu Ser Ser Asp Leu Gln Lys Tyr Asn Gln Leu Val Asn Glu Leu Met
   1185 1190 1195 1200
   Val Lys Leu Met Asn Ser Tyr Gln Leu Leu Ser Val Ile Val Arg Gly
   1205 1210 1215
   Gln Met Leu Lys Gln Leu Ser Ser Ser Thr Pro Glu Lys Ala Leu Asn
   1220 1225 1230
   Leu Ile Val Pro Ile Val Ser Asp Lys Pro Ala Ile Met Leu Glu Leu
   1235 1240 1245
   His Ala Ala Phe Leu Lys Leu Leu Pro Asn Glu Asp Lys Gln Leu Ile
   1250 1255 1260
   Ala Asn Glu Trp Pro Lys Cys Leu Met Val Asn Asp Phe Ala Phe Asn
   1265 1270 1275 1280
   Gly Lys Gln His Pro Val Glu Pro Tyr Ile Leu Asn Val Ile Asp Ala
   1285 1290 1295
   His Ile Ile Glu Leu Thr Arg Gly Ser Asn Tyr Ser Thr Leu His Thr
   1300 1305 1310
   Leu Lys His Cys Leu Lys Ser Ile Leu Gln Leu Phe Glu Leu Leu Leu
   1315 1320 1325
   Pro His Arg Thr Ala Asn Ala Glu Val Glu Thr Gln Leu Lys Gln Leu
   1330 1335 1340
   Leu Ile Ser Ser Met Leu Asp Met Arg Thr Asp Tyr Leu Gln Gly His
   1345 1350 1355 1360
   Ser Glu His Cys Leu Arg Glu Ile Leu Ser Gly Leu Thr Gln Glu Ala
   1365 1370 1375
   Gln Lys Leu Leu Leu Tyr Glu His Met Val Gly Tyr Cys Tyr Arg Met
   1380 1385 1390
   Leu Lys Glu Phe Ala Ala Asp Leu Arg Gln Pro Gln Ala Ala Gly Pro
   1395 1400 1405
   Ser Gly Gly Ala Pro Leu Asp Gln Asp Arg Ala Met Phe Asn Glu Ser
   1410 1415 1420
   Met Leu Phe Ala Val Leu Lys Thr Met Phe Lys Met Leu Glu Lys Pro
   1425 1430 1435 1440
   Val Ala Val Gln Ala Met Arg Gln Phe Phe Lys Asp Gln Arg Ser Gly
   1445 1450 1455
   Ser Leu Thr Thr Leu Leu Leu Ser Phe Thr Gly Thr Ser Leu Pro Val
   1460 1465 1470
   Ser Tyr Ala Arg Lys Met Leu Gln Phe Val Asn Arg Leu Phe Gln Leu
   1475 1480 1485
   Ser Leu Gln Ala Asp Ser Gln Phe Gln His Glu Asp Leu Val Glu Cys
   1490 1495 1500
   Phe Ser Glu Leu Ala Thr Val Asp Val Ala Arg Leu Lys Gln Trp Leu
   1505 1510 1515 1520
   Gly His Ile Ile Tyr Gly Pro Asn Val Ser Thr Asp Val Ser Thr Ser
   1525 1530 1535
   Glu Ala Leu Asp Thr Thr Cys Arg Met Leu Thr Ser Ile Leu Gln Pro
   1540 1545 1550
   Ser Ser Ser Ser Ser Ser Asn Ala Gln Thr Pro Thr Asn Met Ala Thr
   1555 1560 1565
   Val Ser Ala Met Pro Ser Ile Ser Asp Gln Leu Asp Pro Met Glu Ile
   1570 1575 1580
   Glu Tyr Asp Cys Gly Thr Ala Ala Gly Gly Gly Glu Ser Asn Pro Gly
   1585 1590 1595 1600
   Thr Gly Gly Ala Ala Ala Asn Thr Ser Gln Ile Leu Ser Leu Trp Gln
   1605 1610 1615
   Ala Ala Gln Pro Asp Pro Ser Glu Glu Ser Ser Gln Ala Cys Asp His
   1620 1625 1630
   Ser Asp Ser Glu Arg Asn Gly Ala Leu Leu Leu Ser Phe Val Lys Ser
   1635 1640 1645
   Leu Val Lys Asp Gln Ser Lys Ala Ser Gln Ile Ala Pro Pro Leu Phe
   1650 1655 1660
   Gln Ala Leu Leu Gln Leu Gly Gln Thr Leu Ile Ser Pro Pro Gln Glu
   1665 1670 1675 1680
   Gly Cys Asp Phe Ala Asp Val Leu Gln Ile Met Ile Thr Leu Ala Asp
   1685 1690 1695
   Ala Ser Pro Ala Arg Gly His Val Ala Leu Phe Asn Thr Thr Leu Leu
   1700 1705 1710
   Trp Leu Glu Leu Ala Lys Leu Gln Leu Pro Asp Lys His Leu Lys His
   1715 1720 1725
   Ala Glu Asn Val Ser Ala Gln Leu Arg Tyr Leu Ser Glu Leu Leu Gln
   1730 1735 1740
   Ser Ile Gly Phe Arg Gly Ser Arg Gln His Asn Pro Pro Trp Asp Asp
   1745 1750 1755 1760
   Glu Leu Gln Thr Asp Ile Asp Glu Leu Tyr Asp Glu Leu Ala Glu Glu
   1765 1770 1775
   Gly Glu Gln Asp Ser Leu Leu Asp Asp Ser Asp Glu Asp Thr Leu Asn
   1780 1785 1790
   Asn Lys Leu Cys Thr Phe Ser Gln Thr Gln Lys Glu Phe Met Asn Gln
   1795 1800 1805
   His Trp Tyr His Cys His Thr Cys Asn Met Ile Asn Thr Val Gly Val
   1810 1815 1820
   Cys Ser Val Cys Ala Arg Val Cys His Lys Gly His Asp Val Ser Tyr
   1825 1830 1835 1840
   Ala Lys Tyr Gly Asn Phe Phe Cys Asp Cys Gly Ala Lys Glu Asp Gly
   1845 1850 1855
   Ser Cys Gln Ala Leu Ser Arg Arg Ile Gly Ser Asn Glu Val Arg Asp
   1860 1865 1870
   Ser Ala Gly Ile Gly Ser Tyr Leu Pro Ser His Met Ser Leu Leu Ala
   1875 1880 1885
   Gly Lys Lys Arg Ser Ser Leu Pro Val Gly Gln Pro Val Leu Thr Arg
   1890 1895 1900
   Lys Asp Ser Leu Thr Asn Glu Arg Ile Ala Val Leu Thr Lys Leu Leu
   1905 1910 1915 1920
   Glu Pro Tyr Arg Glu Thr Leu Gln His Gln Asp Gln Trp Leu Leu Val
   1925 1930 1935
   Val Arg Cys Ile Leu Glu Tyr Phe Asp Leu Leu Leu Pro Ser Ile Asn
   1940 1945 1950
   Glu Asn Cys Met Leu Tyr Ser Ile Val Gly Cys His Lys Arg Ala Thr
   1955 1960 1965
   Ala Ala Leu Glu Arg Leu His Leu Leu Glu Gln Ser Phe Gln Val Thr
   1970 1975 1980
   Asp Gln Leu Met Phe Ala Thr Leu Gly Ser Gln Glu Gly Ala Phe Glu
   1985 1990 1995 2000
   Asn Val Arg Met Asn Tyr Ser Gly Asp Gln Gly Gln Thr Ile Lys His
   2005 2010 2015
   Leu Leu Ser Ser Gly Val Val Arg Arg Val Ala Phe Cys Cys Leu Ser
   2020 2025 2030
   Ser Pro His Gly Arg Arg Gln Gln Leu Ala Val Ser His Glu Lys Gly
   2035 2040 2045
   Lys Val Thr Ile Leu Gln Leu Ser Ala Leu Leu Lys Gln Ala Asp Ala
   2050 2055 2060
   Ser Lys Arg Lys Leu Thr Leu Thr Gln Leu Ser Ser Ala Pro Ile Ala
   2065 2070 2075 2080
   Cys Thr Val Ile Ser Leu Ala Ala Asn Pro Cys Asn Glu Asp Cys Leu
   2085 2090 2095
   Ala Val Cys Gly Leu Lys Glu Cys His Ile Leu Thr Phe Ser Ser Ser
   2100 2105 2110
   Gly Ser Thr Asn Glu His Ile Val Val Asn Pro Gln Leu Glu Asn Gly
   2115 2120 2125
   Asn Tyr Ile Lys Lys Ala Val Trp Leu Pro Gly Ser Gln Thr Leu Leu
   2130 2135 2140
   Ala Val Val Thr Ser Asp Tyr Val Lys Ile Tyr Asp Leu Ala Val Asp
   2145 2150 2155 2160
   Thr Tyr Ser Pro Lys Tyr Tyr Tyr Leu Val Ala Val Gly Lys Ile Arg
   2165 2170 2175
   Asp Cys Thr Phe Val Tyr Gln Asp Gly Asn Tyr Asn Met Leu Thr Phe
   2180 2185 2190
   Ala Ser Ser Gly Tyr Ile Tyr Thr Gln Gln Leu Asp Gln Gln Ser Leu
   2195 2200 2205
   Ala Val His Gly Asp Phe Tyr Val Thr Asn Thr Leu Glu Leu Ser His
   2210 2215 2220
   Gln His Ile Lys Asp Ile Ala Gly His Ile Gly Gly Gly Gly Val Ser
   2225 2230 2235 2240
   Ile Tyr Tyr Ser His Thr Leu Gln Leu Leu Phe Tyr Ser Tyr Ser Cys
   2245 2250 2255
   Gly Arg Ser Phe Phe Ser Pro Leu Thr Asn Val Ser Glu Gly Val Lys
   2260 2265 2270
   Gly Ile Tyr His Leu Asp Thr Asn Ser Ala Ser Lys Ser Ala Ser Lys
   2275 2280 2285
   Gly Pro Leu Gln Pro Leu Val Gln Trp Thr Glu Val Thr Gly His Pro
   2290 2295 2300
   Gly Leu Val Tyr Ala Ser Met Gln Thr Ser Asn Asn Pro Ile Ile Leu
   2305 2310 2315 2320
   Met Ile Thr Pro Glu Arg Ile Tyr Leu Gln Glu Ile Lys Ala Gln Ser
   2325 2330 2335
   Ala Lys Ser Arg Ile Met Asp Val Val Gly Ile Arg His Ala Val Ala
   2340 2345 2350
   Gly Val Glu Lys Thr Thr Leu Leu Leu Leu Cys Glu Asp Gly Ser Leu
   2355 2360 2365
   Arg Ile Phe Ser Ala Gln Pro Glu Tyr Thr Ser Phe Trp Leu Ser Pro
   2370 2375 2380
   Gln Val Gln Pro Phe Gly Asn Gln Leu Tyr Ser Ser Thr Leu Met Ala
   2385 2390 2395 2400
   Lys Gly Gly Gly Ser Gly Ser Thr Ser Lys Ser Lys Ser Asn Thr Ala
   2405 2410 2415
   Ser Gly Lys Met Thr Ser Arg Lys Ala Ser Gln Gln Gln Lys Gln Pro
   2420 2425 2430
   Thr Ala Gly Gly Gln Pro Val Phe Pro Ile Asp Phe Phe Glu His Cys
   2435 2440 2445
   Asn Met Leu Ala Asp Val Glu Phe Gly Gly Asn Asp Leu Leu Gln Ile
   2450 2455 2460
   Tyr Asn Lys Gln Lys Leu Lys Thr Arg Leu Phe Ser Thr Gly Met Phe
   2465 2470 2475 2480
   Val Ala Ser Thr Arg Ser Asn Gly Phe Thr Leu Glu Val Ile Asn Asn
   2485 2490 2495
   Asp Pro Asn Val Val Ile Val Gly Ile Arg Val Leu Ile Gly Thr Gln
   2500 2505 2510
   Asp Val Gln Arg Ala Pro Gln Ser Val Thr Ile Leu Gly Arg Thr Ile
   2515 2520 2525
   Pro Thr Pro Val Arg Arg Ala Arg Trp Phe Asp Ile Pro Leu Thr Arg
   2530 2535 2540
   Glu Glu Met Leu Gln Ser Asp Lys Leu Leu Lys Val Val Phe Ala Lys
   2545 2550 2555 2560
   Ala Pro Asp Pro Glu His Val Thr Leu Leu Asp Cys Ile Glu Val Tyr
   2565 2570 2575
   Gly Lys Ser Lys Glu Leu Val Gly Trp Pro Asp Glu Ser Glu Asp Val
   2580 2585 2590
   Thr Val Pro Gly Ser Ser Ala Pro Ala Val Ser Ser Ser Gln Ala Ser
   2595 2600 2605
   Ser Ala Asn Phe Gly Glu Gly Phe Asn Cys Ile Thr Gln Leu Asp Arg
   2610 2615 2620
   Met Ala Asn His Leu Leu Glu Val Met Asp Cys Ala Leu His Leu Leu
   2625 2630 2635 2640
   Gly Ser Gly Val Pro Ala Ser Met Arg Gln Lys Ala Val Lys Thr Ala
   2645 2650 2655
   Ser Ala Leu Leu Leu Leu Pro Thr Pro Asn Pro Val Gln Thr Gln Ala
   2660 2665 2670
   Arg Tyr Val Leu Ala Thr Leu Tyr Gly Thr Arg Ala Leu Tyr His Asn
   2675 2680 2685
   Tyr Lys Asp Gly Val Leu Leu Gln Phe Val Asn Arg Glu Leu Gln Ser
   2690 2695 2700
   Met Gln Pro Lys Leu Glu Lys Leu Glu Thr Leu Arg Glu Ile Asp Pro
   2705 2710 2715 2720
   Glu Ala Phe Tyr Arg Leu Val Leu Met Val Arg Gly Ile Ala Asn Ala
   2725 2730 2735
   Arg Pro Gln Ser Leu Ala Lys Ile Cys Val Glu Asn Asn Tyr Asp Ile
   2740 2745 2750
   Val Pro Thr Leu Met Gly Ile Val Leu Glu Leu His Lys Val Thr Pro
   2755 2760 2765
   Thr Leu Asp Glu Pro Val Asn Ile Val Lys Arg Gly Leu Cys Gln Pro
   2770 2775 2780
   Glu Thr Ile Val His Cys Leu Val Glu Ile Met Tyr Gly Phe Ala Leu
   2785 2790 2795 2800
   Ala Asp Pro Gly Gln Val Gly Arg Met Thr Lys Tyr Phe Ile Asp Leu
   2805 2810 2815
   Leu Lys His Asp Ala Ser Val Ile Ser His Ser Ala Lys Glu Ala Leu
   2820 2825 2830
   Ile Leu Leu Leu Ser Pro Arg Met Lys Arg Arg Lys Val Ala Ile Val
   2835 2840 2845
   Thr Pro Pro Ala Cys Ser Thr Pro Thr Pro Ser Thr Ser Thr Met Gln
   2850 2855 2860
   Ala Leu Gln Ala Ala Ala Ser Ser Ala Ala Ser Asp Ile Ile Glu Glu
   2865 2870 2875 2880
   Ala Ala Gly Val Val Val Asp Gly Ser Val Gly Gly Gly Gly Leu Pro
   2885 2890 2895
   Glu Pro Asn Ala Asp Ala Glu Gly Ala Ala Val Gly Val Gly Gly Val
   2900 2905 2910
   Gly Gln Gln Gln Met Leu Asn Leu Glu Ala Phe Met Gly Gly Gly Phe
   2915 2920 2925
   Pro Arg Leu Leu Gly Leu Pro Glu Asp Ala Asp Asp Glu Ala Ile Met
   2930 2935 2940
   Asp Ile Ala Ile Ala Leu Ser Leu Gln Gln His Gly Gly Asp Ala Asn
   2945 2950 2955 2960
   Ala Leu Gln Ser Leu Gln Gln Gly Leu Ala Asn Leu Gln Gly Ile Arg
   2965 2970 2975
   Gln Ala Thr Ala Met Ala Ala Ala Val Asn Ala Ala Ala Asn Val Ser
   2980 2985 2990
   Leu Gly Gly Ser Asp Asp Asp Glu Gly Ser Asn Val Ala Thr Asp Gly
   2995 3000 3005
   Ser Thr Leu Arg Thr Ser Pro Ala Glu Pro Ala Gly Ser Gly Gly Ser
   3010 3015 3020
   Glu Ser Gly Gly Ser Gly Val Glu Ser Ile Gly Gly Thr Ser Ala Arg
   3025 3030 3035 3040
   Ser Ser Asn Phe Gly Asp His Ala Asn Ala Ser Pro Pro Arg Gln Gly
   3045 3050 3055
   Ser Thr Lys Asp Asp Gln Glu Gln Pro Gly Pro Ser Gly Val Ala Gly
   3060 3065 3070
   Ser Gly Gly Val Ala Val Leu Ser Ala Met Ser Ser Ser Glu Asp Asn
   3075 3080 3085
   Glu Ala Asn Glu Asp Asp Lys Leu Ser Lys Leu His Asp Leu Arg Ile
   3090 3095 3100
   Thr Val Leu Glu Ser Ile Ile Gln His Leu Gly Thr Phe Asp Leu Cys
   3105 3110 3115 3120
   Asn Gly Leu Gln Ala Ile Pro Leu Ile Gln Val Ile Leu Met Leu Thr
   3125 3130 3135
   Thr Asp Leu Asn Gly Asn Asn Glu Arg Asp Gln Gln Val Leu His Asp
   3140 3145 3150
   Leu Leu Thr Ala Leu Val Gly Leu Cys Gly Asn Trp Lys Arg Gly Ala
   3155 3160 3165
   Ala Ala Arg Met Glu Thr Lys Cys Pro Gly Asn Glu Val Arg Leu Ala
   3170 3175 3180
   Leu Leu Ser Leu Phe Gly Val Leu Met Gly Lys Thr Lys Ser Lys Gln
   3185 3190 3195 3200
   Thr Gly Thr Thr Ser Pro Pro His Gln Phe Lys Asp Asn Ser Ser Phe
   3205 3210 3215
   Val Ala Ser Thr Thr Ala Asn Val Leu Ser Lys Ser Gly Ala Phe Val
   3220 3225 3230
   Tyr Ala Leu Glu Ala Leu Asn Thr Leu Leu Val His Trp Lys Asn Val
   3235 3240 3245
   Leu Gly Asp Pro Tyr Ala Ala Gly Gly Gly Leu Ala Ser Gln Ser Ala
   3250 3255 3260
   Gln Ala Ser Gly Gly Ala Ser Gly Pro Gly Val Gln Leu Leu Lys Pro
   3265 3270 3275 3280
   Ile Lys His Gly Pro Lys Pro Asp Ile Ser Ile Leu Ile Pro His Asn
   3285 3290 3295
   Tyr Leu Lys Asn Tyr Pro Asp Ile Phe Glu Ser Tyr Asp Gly Leu Leu
   3300 3305 3310
   Thr Glu Ile Ile Val Arg Leu Pro Tyr Gln Ile Leu Arg Leu Ser Ser
   3315 3320 3325
   Ala His Pro Asp Asn Tyr Asp Ser Gly Phe Cys Glu Ala Met Thr Phe
   3330 3335 3340
   Thr Leu Cys Glu Tyr Met Met Leu Asn Leu Asn Thr Leu Leu Arg Arg
   3345 3350 3355 3360
   Gln Val Arg Lys Leu Leu Met Tyr Ile Cys Gly Ser Lys Glu Lys Phe
   3365 3370 3375
   Arg Met Tyr Arg Asp Gly His Ser Leu Asp Ala His Phe Arg Val Val
   3380 3385 3390
   Lys Arg Val Cys Asn Ile Val Ser Ser Lys Thr Gly Ala Pro Tyr Asn
   3395 3400 3405
   Ala Asn Pro Pro Met Leu Ser Tyr Asp Ala Leu Val Glu Leu Thr Glu
   3410 3415 3420
   His Leu Arg Thr Cys Gln Glu Ile Ser Gln Met Arg Thr Gly Asn Trp
   3425 3430 3435 3440
   Gln Lys Phe Cys Val Val His Glu Asp Ala Leu Ala Met Leu Met Glu
   3445 3450 3455
   Ile Ala Cys Tyr Gln Leu Asp Asp Gly Val Ser Pro Ile Ile Ile Gln
   3460 3465 3470
   Leu Leu Gln Ala Ala Val Cys Asn Leu Pro Pro Pro Ser Gly Ser Lys
   3475 3480 3485
   Gln Ala Gln Pro Gln Pro Ser Thr Ser Ser Ala Ser Gly Lys Leu Arg
   3490 3495 3500
   Thr Asp Arg Glu Lys Ser Glu Asp Thr Asp Ala Tyr Tyr Ser Lys Phe
   3505 3510 3515 3520
   Asp Pro Ala Gln Cys Gly Thr Phe Val His Gln Ile Phe Arg Tyr Ala
   3525 3530 3535
   Cys Asp Ala Leu Ile Ile Arg Phe Val Arg Ile Phe Leu Leu Glu Asn
   3540 3545 3550
   Asn Ile Thr Gln Leu Arg Trp Gln Ala His Ser Phe Met Thr Gly Leu
   3555 3560 3565
   Phe Glu His Ala Asn Glu Arg Gln Arg Glu Lys Leu Leu Asn Ile Phe
   3570 3575 3580
   Trp Asn Leu Trp Pro Leu Val Pro Thr Tyr Gly Arg Arg Thr Ala Gln
   3585 3590 3595 3600
   Phe Val Asp Leu Leu Gly Tyr Leu Thr Leu Ser Thr Arg Ser Ile Thr
   3605 3610 3615
   Glu Arg Leu Pro Glu Phe Val Ser Arg Ala Val Asp Val Leu Arg Gln
   3620 3625 3630
   Gln Asn Glu Leu Leu Cys Lys His Pro Asn Ala Pro Ile Tyr Thr Thr
   3635 3640 3645
   Leu Glu Ser Ile Leu Gln Val Asn Gly Tyr Tyr Leu Glu Ser Glu Pro
   3650 3655 3660
   Cys Leu Val Cys Asn Asn Pro Glu Val Pro Met Ala Asn Ile Lys Leu
   3665 3670 3675 3680
   Pro Ser Val Lys Ser Asp Ser Lys Tyr Thr Thr Thr Thr Met Ile Tyr
   3685 3690 3695
   Lys Leu Val Gln Cys His Thr Ile Ser Lys Leu Ile Val Arg Ile Ala
   3700 3705 3710
   Asp Leu Lys Arg Thr Lys Met Val Arg Thr Ile Asn Val Tyr Tyr Asn
   3715 3720 3725
   Asn Arg Ser Val Gln Ala Val Val Glu Leu Lys Asn Arg Pro Ala Leu
   3730 3735 3740
   Trp His Lys Ala Arg Ser Val Ser Leu Gln Ser Ala Gln Thr Glu Leu
   3745 3750 3755 3760
   Lys Ile Asp Phe Pro Leu Pro Ile Thr Ala Cys Asn Leu Met Ile Glu
   3765 3770 3775
   Phe Ala Asp Phe Phe Glu Thr Val Ser Gly Ser Ser Glu Asn Leu Gln
   3780 3785 3790
   Cys Pro Arg Cys Ser Ala Ala Val Pro Ala Tyr Pro Gly Val Cys Gly
   3795 3800 3805
   Asn Cys Gly Glu Asn Val Phe Gln Cys His Lys Cys Arg Ala Ile Asn
   3810 3815 3820
   Tyr Asp Glu Lys Asp Pro Phe Leu Cys His Ser Cys Gly Phe Cys Lys
   3825 3830 3835 3840
   Tyr Ala Lys Phe Asp Phe Ser Met Tyr Ala Arg Val Cys Cys Ala Val
   3845 3850 3855
   Asp Pro Ile Glu Ser Ala Glu Asp Arg Val Lys Thr Val Ser Leu Ile
   3860 3865 3870
   His Ser Ser Leu Glu Arg Ala Asp Arg Asn Tyr Arg Gln Leu Leu Thr
   3875 3880 3885
   Asn Lys Gln Met Leu Glu Leu Leu Ile Gln Lys Val Ala Glu His Arg
   3890 3895 3900
   Ser Ser Asp Arg Met Val Glu Asp Asn Met Ala Ser Val His Ser Thr
   3905 3910 3915 3920
   Ser Gln Val Asn Lys Ile Ile Gln Leu Leu Ala Gln Lys Tyr Cys Val
   3925 3930 3935
   Glu Ser Arg Thr Ser Phe Glu Glu Leu Ser Lys Ile Val Gln Lys Val
   3940 3945 3950
   Lys Ala Cys Arg Ser Glu Leu Val Ala Tyr Asp Arg Gln Gln Gln Asp
   3955 3960 3965
   Gln Pro Pro Val Asn Pro Gly Ser Thr Thr Gly Ala Glu Asn Pro Thr
   3970 3975 3980
   Thr Asn Arg Cys Tyr Gly Cys Ala Leu Ala Ser Thr Glu Gln Cys Leu
   3985 3990 3995 4000
   Thr Leu Leu Arg Ala Met Ala Tyr Asn Tyr Asp Cys Arg Val Cys Leu
   4005 4010 4015
   Tyr Ser Gln Gly Leu Val Ser Glu Leu Ala Glu His Asn Leu Arg Arg
   4020 4025 4030
   Gly Thr Pro Leu Ile Gln Glu Glu Val Arg Asn Leu Leu Val Val Leu
   4035 4040 4045
   Thr Lys Asp Asn Ala Glu Ala Cys Met His Leu Leu Gln Leu Val Thr
   4050 4055 4060
   Thr Arg Val Lys Asn Ala Leu Met Gly Ser Ile Pro Leu Ile Ser Leu
   4065 4070 4075 4080
   Glu Ala Ala Val His Gln Glu Met Thr Leu Leu Glu Val Leu Leu Gly
   4085 4090 4095
   Gln Asp Asp Ile Cys Trp Glu Tyr Lys Leu Lys Val Ile Phe Glu Leu
   4100 4105 4110
   Phe Ile Ser Asn Cys Arg Leu Pro Arg Gly Pro Val Thr Ala Val Leu
   4115 4120 4125
   His Pro Cys Leu Arg Ile Met Gln Asn Leu Ile Cys Pro Val Leu Pro
   4130 4135 4140
   Gly Ser Lys Pro Asn Gln Lys Val Ala Thr Thr Asp Leu Cys Ser Met
   4145 4150 4155 4160
   Lys Met Phe Glu Gly Asn Thr Val Asp Tyr Arg Ala Trp Leu Asn Ser
   4165 4170 4175
   Asp Arg Asn His Glu Tyr Ala Ala Trp Ser Lys Arg Met Pro Ser Asn
   4180 4185 4190
   Asn Gln Ala Lys Leu Lys Asn Ala Lys Asp Gln Asn Val Ala Ala Ser
   4195 4200 4205
   Gly Gly Ser Asp Ala Pro Pro Lys Ser Arg Arg Glu Val Arg Val Ala
   4210 4215 4220
   Phe Leu Ser Glu Lys Tyr Gly Lys Arg Trp Arg Glu Arg Val Leu Asp
   4225 4230 4235 4240
   Lys Gln Arg Val Ile Lys Pro Leu Val Phe Asn Ala Lys Trp Ile Gln
   4245 4250 4255
   Pro Leu Leu Phe Asn Ala Asn Ser Arg Phe Gly Arg Gln Leu Ala Cys
   4260 4265 4270
   Ser Leu Leu Ser Ser Leu Ser Arg Thr Asn Glu Arg Arg Gln Gln Ala
   4275 4280 4285
   Leu Asn Met Leu Thr Ser Phe Leu Lys His Val Gly Glu Ala Gly Glu
   4290 4295 4300
   Ala Ser Ala Glu Tyr Leu Met Leu Tyr Lys Asn Met Ala Thr Glu Gln
   4305 4310 4315 4320
   Pro Trp Leu Gln Tyr Leu Val Leu Lys Gly Val Leu Ser Gln Ile Ser
   4325 4330 4335
   Gln Leu Leu Ala Ile Glu Ile Ser Lys Val His Arg Met Glu Glu Tyr
   4340 4345 4350
   Ser Leu Ser Ser Asp Leu Ser Leu Gly Tyr Ala Leu Arg Gln Tyr Val
   4355 4360 4365
   Glu Leu Leu Trp Leu Leu Leu Glu Cys Pro Asn Ile Arg Arg Thr Tyr
   4370 4375 4380
   Lys Thr Arg Met Leu Gly Pro Val Leu Glu Ser Tyr Leu Ala Leu Arg
   4385 4390 4395 4400
   Ser Leu Val Val Gln Arg Thr Arg Leu Ile Asp Asp Ala Gln Glu Lys
   4405 4410 4415
   Leu Leu Glu Met Leu Glu Asp Met Thr Ser Gly Thr Glu Glu Glu Thr
   4420 4425 4430
   Arg Ala Phe Met Glu Ile Leu Ile Asp Thr Val Glu Lys Thr Arg Met
   4435 4440 4445
   Asn Asp Ile Lys Thr Pro Val Phe Val Phe Glu Arg Leu Tyr Ser Ile
   4450 4455 4460
   Ile His Pro Glu Glu His Asp Glu Ser Glu Phe Tyr Met Thr Leu Glu
   4465 4470 4475 4480
   Lys Asp Pro Gln Gln Glu Asp Phe Leu Gln Gly Arg Met Leu Gly Asn
   4485 4490 4495
   Pro Tyr Pro Ser Ser Glu Met Gly Leu Gly Pro Leu Met Arg Asp Val
   4500 4505 4510
   Lys Asn Lys Ile Cys Thr Asp Cys Glu Leu Ile Ala Leu Leu Glu Asp
   4515 4520 4525
   Asp Asn Gly Met Glu Leu Leu Val Asn Asn Lys Ile Ile Ser Leu Asp
   4530 4535 4540
   Leu Pro Val Lys Asp Val Tyr Lys Lys Val Trp Leu Ala Glu Gly Gly
   4545 4550 4555 4560
   Asp Arg Asp Ala Met Arg Ile Val Tyr Arg Met Arg Gly Leu Leu Gly
   4565 4570 4575
   Asp Ala Thr Glu Glu Phe Val Glu Thr Leu Asn Asn Lys Ser Gln Glu
   4580 4585 4590
   Gln Val Asp Thr Glu Gln Leu Tyr Arg Met Ala Asn Val Leu Ala Asp
   4595 4600 4605
   Cys Asn Gly Leu Arg Val Met Leu Glu Arg Ile Gly Ser Leu Gln Arg
   4610 4615 4620
   Ile Ser Arg Asn Arg Glu Leu Ile Gln Val Leu Leu Lys Leu Phe Leu
   4625 4630 4635 4640
   Ile Cys Val Lys Val Arg Arg Cys Gln Glu Val Leu Cys Gln Pro Glu
   4645 4650 4655
   Ile Gly Ala Ile Asn Thr Leu Leu Lys Val Leu Gln Met Cys Leu Gln
   4660 4665 4670
   Ser Glu Asn Asp Ser Ile Gln Ser Ala Val Thr Glu Gln Leu Leu Glu
   4675 4680 4685
   Ile Met Glu Thr Ile Leu Ser Lys Ala Ala Ser Asp Thr Leu Asp Ser
   4690 4695 4700
   Phe Leu Gln Phe Ser Leu Thr Phe Gly Gly Pro Glu Tyr Val Ser Ala
   4705 4710 4715 4720
   Leu Ile Ser Cys Thr Asp Cys Pro Asn Val Arg Asn Asn Pro Ser Val
   4725 4730 4735
   Leu Arg His Leu Ile Arg Val Leu Ala Ala Leu Val Tyr Gly Asn Glu
   4740 4745 4750
   Val Lys Met Ala Leu Leu Cys Glu His Phe Lys Asp Thr Leu Asn Phe
   4755 4760 4765
   Asn Arg Phe Asp Asn Glu Arg Thr Pro Glu Glu Glu Phe Lys Leu Glu
   4770 4775 4780
   Leu Phe Cys Val Leu Thr Asn Gln Ile Glu His Asn Cys Ile Gly Gly
   4785 4790 4795 4800
   Thr Leu Lys Asp Tyr Ile Val Ser Leu Gly Ile Val Glu Arg Ser Leu
   4805 4810 4815
   Ala Tyr Ile Thr Glu His Ala Pro Cys Val Lys Pro Thr Leu Leu Arg
   4820 4825 4830
   Thr Asp Ser Asp Glu Leu Lys Glu Phe Ile Ser Arg Pro Ser Leu Lys
   4835 4840 4845
   Tyr Ile Leu Arg Phe Leu Thr Gly Leu Ser Asn His His Glu Ala Thr
   4850 4855 4860
   Gln Val Ala Ile Ser Lys Asp Ile Ile Pro Ile Ile His Arg Leu Glu
   4865                4870                4875                4880
   Gln Val Ser Ser Asp Glu His Val Gly Ser Leu Ala Glu Asn Leu Leu
                   4885                4890                4895
   Glu Ala Leu Ser Thr Asp Ser Ala Thr Ala Ala Arg Val Gln Gln Val
               4900                4905                4910
   Arg Asp Phe Thr Arg Ala Glu Lys Lys Arg Leu Ala Met Ala Thr Arg
           4915                4920                4925
   Glu Lys Gln Leu Asp Ala Leu Gly Met Arg Thr Asn Glu Lys Gly Gln
       4930                4935                4940
   Val Thr Ala Lys Gly Ser Ile Leu Gln Lys Ile Glu Lys Leu Arg Asp
   4945                4950                4955                4960
   Glu Thr Gly Leu Thr Cys Phe Ile Cys Arg Glu Gly Tyr Ala Cys Gln
                   4965                4970                4975
   Pro Asp Lys Val Leu Gly Ile Tyr Thr Phe Thr Lys Arg Cys Asn Val
               4980                4985                4990
   Glu Glu Phe Glu Leu Lys Ser Arg Lys Thr Ile Gly Tyr Thr Thr Val
           4995                5000                5005
   Thr His Phe Asn Val Val His Val Asp Cys His Thr Ser Ala Ile Arg
       5010                5015                5020
   Leu Thr Arg Gly Arg Asp Glu Trp Glu Arg Ala Ser Leu Gln Asn Ala
   5025                5030                5035                5040
   Asn Thr Arg Cys Asn Gly Leu Leu Pro Leu Trp Gly Pro Ala Val Gly
                   5045                5050                5055
   Glu Ala Ala Phe Ser Ala Cys Met Thr Arg His Ser Ser Tyr Met Gln
               5060                5065                5070
   Glu Ser Thr Gln Arg Cys Asp Ile Ser Tyr Thr Ser Ser Val His Asp
           5075                5080                5085
   Leu Lys Leu Leu Leu Val Arg Phe Ala Trp Glu Arg Ser Phe His Asp
       5090                5095                5100
   Asp Ala Gly Gly Gly Gly Pro Gln Ser Asn Met His Phe Val Pro Tyr
   5105                5110                5115                5120
   Leu Leu Phe Tyr Ser Val Tyr Leu Leu Leu Ser Ser Arg Ser Ala Ala
                   5125                5130                5135
   Arg Asp Ser Lys Thr Leu Leu Thr Tyr Leu Gln Ala Pro Pro Ser Glu
               5140                5145                5150
   Lys Trp Leu Glu Cys Gly Tyr Glu Val Asp Gly Pro Leu Phe Met Ala
           5155                5160                5165
   Thr Ile Ser Leu Ser Leu His Ser Arg Glu Leu Trp Asn Lys His Lys
       5170                5175                5180
   Leu Ala His Leu Lys Arg Met Ile Ala Val Ala Gln Gly Arg His Val
   5185                5190                5195                5200
   Ser Pro Ala Val Leu Cys Lys Ala Leu Leu Ala Pro Ala Asp Arg Gln
                   5205                5210                5215
   Val Lys Asp Tyr Thr Val Tyr Lys Pro Phe Leu Met Met Trp Ala Leu
               5220                5225                5230
   Val Asp Leu Ile Tyr Asp Asn Leu Phe Lys Thr Val Ser Thr Pro Lys
           5235                5240                5245
   Glu Glu Asp Trp Pro Ile Ser Leu Phe Asp Tyr Leu Arg Lys Asn Asp
       5250                5255                5260
   Glu Ala Leu Leu Lys Ser Thr Asp Ser Ile Leu Gln Thr Leu Thr Glu
   5265                5270                5275                5280
   Glu Phe Leu Pro Cys Thr Ser Phe Val Glu Phe Cys Asp Val Ala Gly
                   5285                5290                5295
   Leu Leu His Leu Ile Glu His Pro Asp Asn Phe Ile Glu Glu Ile Leu
               5300                5305                5310
   Ala Ala Leu Pro Ser Thr Ser Ser Ser Asn
           5315                5320
<210> 48
   <211> 29
   <212> DNA
   <213> Rattus sp.
<400> 48
   cattatgctg agtgatatct ttttttttvv 30
<210> 49
   <211> 25
   <212> DNA
   <213> Rattus sp.
<400> 49
   attaaccctc actaaatgct gggga 25
<210> 50
   <211> 12
   <212> DNA
   <213> Rattus sp.
<400> 50
   gatctgcggt ga 12
<210> 51
   <211> 24
   <212> DNA
   <213> Rattus sp.
<400> 51
   agcactctcc agcctctcac cgca 24
<210> 52
   <211> 12
   <212> DNA
   <213> Rattus sp.
<400> 52
   gatctgttca tg 12
<210> 53
   <211> 24
   <212> DNA
   <213> Rattus sp.
<400> 53
   accgacgtcg actatccatg aaca 24
<210> 54
   <211> 12
   <212> DNA
   <213> Rattus sp.
<400> 54
   gatcttccct cg 12
<210> 55
   <211> 24
   <212> DNA
   <213> Rattus sp.
<400> 55
   aggcaactgt gctatccgag ggaa 24
   1
   1